# EUROPEAN PATENT SPECIFICATION

(11) **EP 2 281 834 B1**
(45) Date of publication and mention of the grant of the patent: **04.03.2015**
(21) Application number: 10181686.6
(22) Date of filing: 06.04.2000
(51) Int. Cl.: C07K 14/285, C07K 16/12, A61K 39/102, A61K 35/74, C12N 1/21, C12N 15/31

(54) **Attenuated Pasteurellaceae bacterium having a mutation in a virulence gene**
Attenuiertes Bakterium der Familie Pasteurellaceae welches ein mutiertes Virulenzgen aufweist
Bactéries atténuées de la Famille Pasteurellaceae comprenant un gène muté de virulence

(30) Priority: 09.04.1999 US 128689 P; 10.09.1999 US 153453 P
(43) Date of publication of application: 09.02.2011
(62) Divisional of application: 07001710.8
(73) Proprietor: Zoetis P&U LLC, Florham Park, NJ 07932 (US)
(72) Inventor: Lowery, David E., Stokesdale, NC 27357 (US); Kennedy, Michael J., Galesburg, MI 49053 (US); Fuller, Troy E., Ceresco, MI 49033 (US)
(74) Representative: Mannion, Sally Kim

(56) References cited:
- WO-A-94/11024
- WO-A-97/41823
- FERNANDEZ DE HENESTROSA A R ET AL: "Importance of the galE gene on the virulence of Pasteurella multocida", FEMS MICROBIOLOGY LETTERS, AMSTERDAM, NL, vol. 154, 1 January 1997 (1997-01-01), pages 311-316, XP000864738, ISSN: 0378-1097
- WARD ET AL: "Haemophilus ducreyi secretes a filamentous hemagglutinin-like protein.", JOURNAL OF BACTERIOLOGY, vol. 180, no. 22, 1 November 1998 (1998-11-01), pages 6013-6022, XP55008336, ISSN: 0021-9193 -& DATABASE EMBL [Online] 30 November 1998 (1998-11-30), "Haemophilus ducreyi strain 35000 LspB (lspB) gene, partial cds; large supernatant protein 2 (lspA2) gene, complete cds; and putative anaerobic glycerol-3-phosphate dehydrogenase GlpA (glpA) gene, partial cds.", XP002660855, retrieved from EBI accession no. EM_PRO:AF057696 Database accession no. AF057696
- ADLER B ET AL: "Candidate vaccine antigens and genes in Pasteurella multocida", JOURNAL OF BIOTECHNOLOGY, ELSEVIER SCIENCE PUBLISHERS, AMSTERDAM, NL, vol. 73, no. 2-3, 20 August 1999 (1999-08-20), pages 83-90, XP004180171, ISSN: 0168-1656, DOI: 10.1016/S0168-1656(99)00111-X
- FULLER TROY E ET AL: "Identification of Pasteurella multocida virulence genes in a septicemic mouse model using signature-tagged mutagenesis", MICROBIAL PATHOGENESIS, ACADEMIC PRESS LIMITED, NEW YORK, NY, US, vol. 29, no. 1, 1 July 2000 (2000-07-01), pages 25-38, XP002202784, ISSN: 0882-4010, DOI: 10.1006/MPAT.2000.0365

## Description

### FIELD OF THE INVENTION

The present invention relates generally to the identification of genes responsible for virulence of *Pasteurella multocida* and *Actillobacillus pleuropneumoniae* bacteria, thereby allowing for production of novel attenuated mutant strains useful in vaccines and identification of new anti-bacterial agents that target the virulence genes and their products.

### BACKGROUND OF THE INVENTION

The family *Pasteurellaceae* encompasses several significant pathogens that infect a wide variety of animals. In addition to *P. multocida,* prominent members of the family include *Pasteurella haemolytica, Actinobacillus pleuropneumoniae* and *Haemophilus somnus. P. multocida* is a gram-negative, nonmotile coccobacillus which is found in the normal flora of many wild and domestic animals and is known to cause disease in numerous animal species worldwide [Biberstein, In M. Kilian, W. Frederickson, and E. L. Biberstein (ed.), Haemophilus, Pasteurella, and Actinobacillus. Academic Press, London, p. 61-73 (1981)]. The disease manifestations following infection include septicemias, bronchopneumonias, rhinitis, and wound infections [Reviewed in Shewen, et al., In C. L. Gyles and C. O. Thoen (ed.), Pathogenesis of Bacterial Infections in Animals. Iowa State University Press, Ames, p. 216-225 (1993), incorporated herein by reference].

Infection by *P. multocida* generally results from invasion during periods of stress, but transmission may also occur by aerosol or contact exposure, or via flea and tick vectors. In fowl, *P. multocida* infection gives rise to acute to peracute septicemia, particularly prevalent in domestic turkeys and wild waterfowl under stress conditions associated with overcrowding, laying, molting, or severe climatic change. In cattle, a similar hemorrhagic septicemia follows infection and manifests conditions including high fever and depression, generally followed by quick death. Transmission is most likely through aerosol contact, but infection can also arise during periods of significant climatic change. In rabbits, infection gives rise to recurring purulent rhinitis, generally followed by conjunctivitis, otitis media, sinusitis, subcutaneous abscesses, and chronic bronchopneumonia. In severe infections, rabbit mortality arises from acute fibrinous bronchopneumonia, septicemia, or endotoxemia. Disease states normally arise during periods of stress. In pigs, common *P. multocida* disease states include atrophic rhinitis and bacterial pneumonia. Similar pneumonia conditions are also detected in dogs, cats, goats, and sheep. *P. multocida* is commonly detected in oral flora of many animals and is therefore a common contaminant in bite and scratch wounds.

*P. multocida* strains are normally designated by capsular serogroup and somatic serotype. Five capsular serogroups (A, B, D, E, and F) and 16 somatic serotypes are distinguished by expression of characteristic heat-stable antigens. Most strains are host specific and rarely infect more than one or two animals. The existence of different serotypes presents a problem for vaccination because traditional killed whole cell bacteria normally provide only serotype-specific protection. However, it has been demonstrated that natural infection with one serotype can lead to immunological protection against multiple serotypes [Shewen, et al., In C. L. Gyles and C. O. Thoen (Ed.), Pathogenesis of Bacterial Infections in Animals. Iowa State University Press, Ames, p. 216-225 (1993)] and cross protection can also be stimulated by using inactivated bacteria grown *in vivo* [Rimler, et al., Am J Vet Res. 42:2117-2121 (1981)]. One live spontaneous mutant *P. multocida* strain has been utilized as a vaccine and has been shown to stimulate a strong immune response [Davis, Poultry Digest. 20:430-434 (1987), Schlink, et al., Avian Dis. 31(1):13-21 (1987)]. This attenuated strain, however, has been shown to revert to a virulent state or cause mortality if the vaccine recipient is stressed [Davis, Poultry Digest. 20:430-434 (1987), Schlink, et al., Avian Dis. 31(1):13-21 (1987)].

*Another member of the Pasteurella* family, *A. pleuropneumoniae* exhibits strict host specificity for swine and is the causative agent of highly contagious porcine pleuropneumonia. Infection normally arises in intensive breeding conditions, and is believed to occur by a direct mode of transmission. The disease is often fatal and, as a result, leads to severe economic loss in the swine producing industry. *A*. *pleuropneumoniae* infection may be chronic or acute, and infection is characterized by a hemorrhagic, necrotic bronchopneumonia with accompanying fibrinous pleuritis. To date, bacterial virulence has been attributed to structural proteins, including serotype-specific capsular polysaccharides, lipopolysaccharides, and surface proteins, as well as extracellular cytolytic toxins. Despite purification and, in some instances cloning, of these virulence factors, the exact role of these virulence factors in *A. pleuropneumoniae* infection is poorly understood.

Twelve serotypes of *A. pleuropneumoniae* have been identified based on antigenic di fferences in capsular polysaccharides and production of extracellular toxins. Serotypes 1, 5, and 7 are most relevant to *A. pleuropneumoniae* infection in the United States, while serotypes 1, 2, 5, 7, and 9 are predominant in Europe. There are at least three significant extracellular toxins of *A. pleuropneumoniae* that are members of the haemolysin family and are referred to as RTX toxins. RTX toxins are produced by many Gram negative bacteria, including *E. coli, Proteus vulgarisa,* and *Pasteurella haemolytica,* and the proteins generally share structural and functional characteristics. Toxins from the various serotypes differ, however, in host specificity, target cells, and biological activities.

The major *A. pleuropneumoniae* RTX toxins include ApxI, ApxII, and ApxIII. ApxI and ApxII have haemolytic activity, with ApxI being more potent. ApxIII shows no haemolytic activity, but is cytotoxic for alveolar macrophages and neutrophils. Most *A. pleuropneumoniae* serotypes produce two of these three toxins. For example, serotypes 1, 5, 9, and 11 express ApxI and ApxII, and serotypes 2, 3, 4, 6, and 8 express ApxII and ApxIII. Serotype 10, however, produces only ApxI, and serotypes 7 and 12 express only ApxII. Those *A. pleuropneumoniae* serotypes that produce both ApxI and ApxII are the most virulent strains of the bacteria.

The Apx toxins were demonstrated to be virulence factors in murine models and swine infection using randomly mutated wild type bacteria [Tascon, et al., Mol. Microbiol. 14:207-216 (1994)]. Other *A. pleuropneumoniae mutants* have also been generated with targeted mutagenesis to inactivate the gene encoding the AopA outer membrane virulence protein [Mulks and Buysee, Gene 165:61-66 (1995)].

In attempts to produce vaccine compositions, traditional killed whole cell bacteria have provided only serotype-specific protection [MacInnes and Smart, *supra*], however, it has been demonstrated that natural infection with a highly virulent serotype can stimulate strong protective immunity against multiple serotypes [Nielsen, Nord Vet Med. 31:407-13 (1979), Nielsen, Nord Vet Med. 36:221-234 (1984), Nielsen, Can J Vet Res. 29:580-582 (1988); Nielsen, ACTA Vet Scand. 15:80-89 (1994)]. One defined live attenuated vaccine strain producing an inactive form of the Apxll toxin has shown promise for cross-protection in swine [Prideaux at al., Infection & Immunity 67:1962-1966 (1999)], while other undefined live attenuated mutants have also shown promise [Inzana et al., Infect Immun. 61:1682-6 (1993); Paltineanu et al., in International Pig Veterinary Society, 1992, p. 214; Utrera et al., in International Pig Veterinary Society, 1992, p. 213].

Because of the problems associated with vaccine formulations comprising bacterial strains with undefined, spontaneous mutations, there exists a need in the art for rational construction of live attenuated bacterial strains for use in vaccines that will safely stimulate protective immunity against homologous and heterologous *P. multocida* and *A. pleuropneumoniae* serotypes. There further exists a need to identify attenuated bacterial strains and genes required for bacterial virulence, thereby facilitating development of methods to identify anti-bacterial agents.

WO94/11024 discloses vaccines for protecting an animal against *P. multocida*-associated pasteurellosis by providing a mutant having at least one virulence gene genetically modified by transposon mutagenesis. The virulence gene is located in 9.4 kb EcoRV fragment of the *P. multocida* genome.

WO97/41823 discloses genetically engineered *Pasteurellaceae* useful as vaccines.

Fernandez De Henestroda et al, FEMS Microbiology Letters, Amsterda, NL, Vol. 154, 1 January 1997, pages 311-316, shows that galE is important for the virulence of *P. mutlicida* and that a galE-defective mutant is highly attenuated.

Ward et al, J. Bacteriology, Vol. 180, no. 22, 1 November 1998, pages 6013-6022, discloses an ORF coding sequence for secreted proteins from *Haemophilus ducreyi* (LspA and LspB). The nucleotide sequence coding for LspB is 65% identical to SEQ ID NO: 19 in a 597 nt overlap. The protein is 53% identical to SEQ ID NO: 20.

### SUMMARY OF THE INVENTION

A first aspect of the present invention is an attenuated *Pasteurella muttocida* bacterium comprising a mutation in a gene represented by a polynucleotide sequence that encodes a *fhaC* polypeptide
(i) wherein the polynucleotide sequence hybridises to the complement of SEQ ID NO:19 under stringent conditions, such conditions comprising a final wash in buffer comprising 2X SSC/0.1 % SDS, at 35°C to 45°C, or
(ii) wherein the polypeptide comprises an amino acid sequence at least 80% (preferably at least 90%, more preferably at least 95%, most preferably at least 99%) identical to SEQ ID NO:20;
wherein the attenuation of the bacterium is caused by said mutation.

A second aspect of the invention is an immunogenic composition comprising a bacterium of the invention.

A third aspect of the invention is a vaccine composition comprising an immunogenic composition of the invention and a pharmaceutically acceptable carrier.

A fourth aspect of the invention is a purified and isolated polynucleotide comprising a nucleotide sequence encoding a polypeptide having SEQ ID NO:19, or the sequence of SEQ ID NO: 20, e.g. as DNA.

A fifth aspect of the invention is a purified polypeptide comprising SEQ ID NO: 20.

In an attenuated bacterium of the invention, the mutation inhibits or abolishes expression and/or biological activity of an encoded gene product (i.e. the polypeptide encoded by a gene), the functional mutation resulting in attenuated virulence of the bacterial strain. Functional mutations that modulate (i.e. increase or decrease) expression and/or biological activity of a gene product include insertions or deletions in the protein coding region of the gene itself or in sequences responsible for, or involved in, control of gene expression. Deletion mutants include those wherein all or part of a specific gene sequence is deleted. In order for a modified strain to be effective in a vaccine formulation, the attenuation must be significant enough to prevent the pathogen from evoking severe clinical symptoms, but also insignificant enough to allow limited replication and growth of the bacteria in the host.

Polynucleotides of the invention include DNA, such as complementary DNA, genomic DNA including complementary or anti-sense DNA, and wholly or partially synthesized DNA; RNA, including sense and anti-sense strands; and peptide nucleic acids as described, for example in Corey TIBTECH 15:224-229 (1997).

A polypeptide encoded by a polynucleotide of the invention may be produced by growing a host cell of the invention under conditions that permit, and preferably promote, expression of a gene product encoded by the polynucleotide, and isolating the gene product from the host cell or the medium of its growth.

### DETAILED DESCRIPTION OF THE INVENTION

"Virulence genes," as used herein, are genes whose function or products are required for successful establishment and/or maintenance of bacterial infection in a host animal. Thus, virulence genes and/or the proteins encoded thereby are involved in pathogenesis in the host organism, but may not be necessary for growth.

"Signature-tagged mutagenesis (STM)," as used herein, is a method generally described in International Patent Publication No. WO 96/17951, and includes, for example, a method for identifying bacterial genes required for virulence in a murine model of bacteremia. In this method, bacterial strains that each have a random mutation in the genome are produced using transposon integration; each insertional mutation carries a different DNA signature tag which allows mutants to be differentiated from each other. The tags comprise 40 bp variable central regions flanked by invariant "arms" of 20 bp which allow the central portions to be co-amplified by polymerase chain reaction (PCR). Tagged mutant strains are assembled in microtiter dishes, then combined to form the "inoculum pool" for infection studies. At an appropriate time after inoculation, bacteria are isolated from the animal and pooled to form the "recovered pool." The tags in the recovered pool and the tags in the inoculum pool are separately amplified, labeled, and then used to probe filters arrayed with all of the different tags representing the mutants in the inoculum. Mutant strains with attenuated virulence are those which cannot be recovered from the infected animal, i.e., strains with tags that give hybridization signals when probed with tags from the inoculum pool but not when probed with tags from the recovered pool. In a variation of this method, non-radioactive detection methods such as chemiluminescence can be used

Signature-tagged mutagenesis allows a large number of insertional mutant strains to be screened simultaneously in a single animal for loss of virulence. Screening nineteen pools of mutant *P. multocida* strains resulted in the identification of more than 60 strains with reduced virulence, many of which were confirmed to be attenuated in virulence by subsequent determination of an approximate LD₅₀ for the individual mutants. The nucleotide sequence of the open reading frame disrupted by the transposon insertion was determined by sequencing both strands and an encoded amino acid sequence was deduced. Novelty of both the polynucleotide and amino acid sequences was determined by comparison of the sequences with DNA and protein database sequences.

The identification of bacterial, and more particularly *P. multocida* virulence genes provides for microorganisms exhibiting reduced virulence (*i.e*., attenuated strains), which are useful in vaccines. Such microorganisms include *Pasteurellaceae* mutants containing at least one functional mutation inactivating a gene represented by any one of SEQ ID NO: 19. The worker of ordinary skill in the art will realize that a "functional mutation" may occur in protein coding regions of a gene of the invention, as well as in regulatory regions that modulate transcription of the virulence gene RNA.

The worker of ordinary skill will also appreciate that attenuated *P*. *multocida* strains of the invention include those bearing more than one functional mutation. More than one mutation may result in additive or synergistic degrees of attenuation. Multiple mutations can be prepared by design or may fortuitously arise from a deletion event originally intended to introduce a single mutation. An example of an attenuated strain with multiple deletions is a *Salmonella typhimurium* strain wherein the *cya* and *crp* genes are functionally deleted. This mutant *S*. *typhimurium* strain has shown promise as a live vaccine.

Identification of virulence genes in *P. multocida* can provide information regarding similar genes, *i.e.,* species homologs, in other pathogenic species. As an example, identification of the *aroA* gene led to identification of conserved genes in a diverse number of pathogens, including *P. haemolytica, Aeromonas hydrophila, Aeromonas salmonicida, Salmonella typhimurium, Salmonella enteritulis, Salmonella dublin, Salmonella gallanerum, Bordella pertussis, Yersinia entericolitica, Neisseria gonorrhoeae, and Bacillus anthracis.* In many of these species, attenuated bacterial strains bearing mutations in the *aroA* gene have proven to be effective in vaccine formulations. Using the virulence genes sequences identified in *P. multocida,* similar or homologous genes can be identified in other organisms, particularly within the *Pasteurella* family, as well as *A. pleuropneumoniae* and *Haemophilus somnus.* Southern hybridization using the *P. multocida* genes as probes can identify these related genes in chromosomal libraries derived from other organisms. Alternatively, PCR can be equally effective in gene identification across species boundaries. As still another alternative, complementation of, for example, a *P. multocida* mutant with a chromosomal library from other species can also be used to identify genes having the same or related virulence activity. Identification of related virulence genes can therefore lead to production of an attenuated strain of the other organism which can be useful as still another vaccine formulation. Examples of *P. multocida* genes that have been demonstrated to exist in other species (*e.g. P. haemolytica, A. pleuropneumoniae* and *H. somnus*) include genes *exbB, atpG. and pnp*

Attenuated *P. multocida* strains identified using STM are insertional mutants wherein a virulence gene has been rendered non-functional through insertion of transposon sequences in either the open reading frame or regulatory DNA sequences. In one aspect, therefore, the attenuated *P. multocida* strains, as well as other gram-negative mutant bacterial strains of the invention can bear one or more mutations which result in an insertion in the gene, with the insertion causing decreased expression of a gene product encoded by the mutated gene and/or expression of an inactive gene product encoded by the mutated gene. These insertional mutants still contain all of the genetic information required for bacterial virulence and can possibly revert to a pathogenic state by deletion of the inserted transposon. Therefore, in preparing a vaccine formulation, it is desirable to take the information gleaned from the attenuated strain and create a deletion mutant strain wherein some, most, or all of the virulence gene sequence is removed, thereby precluding the possibility that the bacteria will revert to a virulent state. The attenuated *P. multocida* strains, as well as other gram-negative mutant bacterial strains of the invention therefore include those bearing one or more mutation which results in deletion of at least about 10%, at least about 20%, at least about 30%, at least about 40% at least about 50%, at least about 60%, at least about 70%, at least about 80%, at least about 90%, at least about 95%, at least about 98%, or at least about 99% of the virulence gene.

The vaccine properties of an attenuated insertional mutant identified using STM are expected to be the same or similar to those of a bacteria bearing a deletion in the same gene. However, it is possible that an insertion mutation may exert "polar" effects on adjoining gene sequences, and as a result, the insertion mutant may possess characteristic distinct from a mutant strain with a deletion in the same gene sequence. Deletion mutants can be constructed using any of a number of techniques well known and routinely practiced in the art.

In one example, a strategy using counterselectable markers can be employed which has commonly been utilized to delete genes in many bacteria. For a review, see, for example, Reyrat, et al., Infection and Immunity 66:4011-4017 (1998), incorporated herein by reference. In this technique, a double selection strategy is often employed wherein a plasmid is constructed encoding both a selectable and counterselectable marker, with flanking DNA sequences derived from both sides of the desired deletion. The selectable marker is used to select for bacteria in which the plasmid has integrated into the genome in the appropriate location and manner. The counterselecteable marker is used to select for the very small percentage of bacteria that have spontaneously eliminated the integrated plasmid. A fraction of these bacteria will then contain only the desired deletion with no other foreign DNA present. The key to the use of this technique is the availability of a suitable counterselectable marker.

In another technique, the *cre-lox* system is used for site specific recombination of DNA. The system consists of 34 base pair *lox* sequences that are recognized by the bacterial *cre* recombinase gene. If the *lox* sites are present in the DNA in an appropriate orientation, DNA flanked by the *lox* sites will be excised by the *cre* recombinase, resulting in the deletion of all sequences except for one remaining copy of the *lox* sequence. Using standard recombination techniques, it is possible to delete the targeted gene of interest in the *P. multocida* genome and to replace it with a selectable marker (*e.g*., a gene coding for kanamycin resistance) that is flanked by the *lox* sites. Transient expression (by electroporation of a suicide plasmid containing the *cre* gene under control of a promoter that functions in *P. multocida*) of the *cre* recombinase should result in efficient elimination of the *lox* flanked marker. This process would result in a mutant containing the desired deletion mutation and one copy of the *lox* sequences.

In another approach, it is possible to directly replace a desired deleted sequence in the *P. multocida* genome with a marker gene, such as green fluorescent protein (GFP), β-galactosidase, or luciferase. In this technique. DNA segments flanking a desired deletion are prepared by PCR and cloned into a suicide (non-replicating) vector for *P. multocida* An expression cassette, containing a promoter active in *P. multocida* and the appropriate marker gene, is cloned between the flanking sequences. The plasmid is introduced into wild-type *P. multocida.* Bacteria that incorporate and express the marker gene (probably at a very low frequency) are isolated and examined for the appropriate recombination event (*i.e*., replacement of the wild type gene with the marker gene).

The reduced virulence of these organisms and their immunogenicity may be confirmed by administration to a subject animal. While it is possible for an avirulent microorganism of the invention to be administered alone, one or more of such mutant microorganisms are preferably administered in a vaccine composition containing suitable adjuvant(s) and pharmaceutically acceptable diluent(s) or carrier(s). The carrier(s) must be "acceptable" in the sense of being compatible with the avirulent microorganism of the invention and not deleterious to the subject to be immunized. Typically, the carriers will be water or saline which will be sterile and pyrogen free. The subject to be immunized is a subject needing protection from a disease caused by a virulent form of *P. multocida,* or other pathogenic microorganisms.

It will be appreciated that the vaccine of the invention may be useful in the fields of human medicine and veterinary medicine. Thus, the subject to be immunized may be a human or other animal, for example, farm animals including cows, sheep, pigs, horses, goats and poultry (*e.g.*, chickens, turkeys, ducks and geese) companion animals such as dogs and cats; exotic and/or zoo animals; and laboratory animals including mice, rats, rabbits, guinea pigs, and hamsters.

DNA sequences encoding virulence polypeptides which would hybridize thereto but for the degeneracy of the genetic code are contemplated by the invention. Exemplary high stringency conditions include a final wash in buffer comprising 0.2X SSC/0.1% SDS, at 65°C to 75°C, while exemplary moderate stringency conditions include a final wash in buffer comprising 2X SSC/0.1% SDS, at 35°C to 45°C. It is understood in the art that conditions of equivalent stringency can be achieved through variation of temperature and buffer, or salt concentration as described in Ausubel, et al. (Eds.), Protocols in Molecular Biology, John Wiley & Sons (1994), pp. 6.0.3 to 6.4.10. Modifications in hybridization conditions can be empirically determined or precisely calculated based on the length and the percentage of guanosine/cytosine (GC) base pairing of the probe. The hybridization conditions can be calculated as described in Sambrook, et al., (Eds.), Molecular Cloning: A Laboratory Manual, Cold Spring Harbor Laboratory Press: Cold Spring Harbor, New York (1989), pp. 9.47 to 9.51.

Autonomously replicating recombinant expression constructions such as plasmid and viral DNA vectors incorporating virulence gene sequences are also provided. Expression constructs wherein virulence polypeptide-encoding polynucleotides are operatively linked to an endogenous or exogenous expression control DNA sequence and a transcription terminator are also provided. The virulence genes may be cloned by PCR, using *P. multocida* genomic DNA as the template. For ease of inserting the gene into expression vectors, PCR primers are chosen so that the PCR-amplified gene has a restriction enzyme site at the 5' end preceding the initiation codon ATG, and a restriction enzyme site at the 3' end after the termination codon TAG, TGA or TAA. If desirable, the codons in the gene are changed, without changing the amino acids, according to *E*. *coli* codon preference described by Grosjean and Fiers, Gene, 18:199-209 (1982), and Konigsberg and Godson, Proc. Natl. Acad. Sci. (USA), 80:687-691 (1983). Optimization of codon usage may lead to an increase in the expression of the gene product when produced in *E. coli.* If the gene product is to be produced extracellularly, either in the periplasm of *E. coli* or other bacteria, or into the cell culture medium, the gene is cloned without its initiation codon and placed into an expression vector behind a signal sequence.

According to another aspect of the invention, host cells are provided, including procaryotic and eukaryotic cells, either stably or transiently transformed, transfected, or electroporated with polynucleotide sequences of the invention in a manner which permits expression of virulence polypeptides of the invention. Expression systems of the invention include bacterial, yeast, fungal, viral, invertebrate, and mammalian cells systems. Host cells of the invention are a valuable source of immunogen for development of antibodies specifically immunoreactive with the virulence gene product. Host cells of the invention are conspicuously useful in methods for large scale production of virulence polypeptides wherein the cells are grown in a suitable culture medium and the desired polypeptide products are isolated from the cells or from the medium in which the cells are grown by, for example, immunoaffinity purification or any of the multitude of purification techniques well known and routinely practiced in the art. Any suitable host cell may be used for expression of the gene product, such as *E. coli,* other bacteria, including *P. multocida, Bacillus* and *S*. *aureus,* yeast, including *Pichia pastoris* and *Saccharomyces cerevisiae,* insect cells, or mammalian cells, including CHO cells, utilizing suitable vectors known in the art. Proteins may be produced directly or fused to a peptide or polypeptide, and either intracellularly or extracellularly by secretion into the periplasmic space of a bacterial cell or into the cell culture medium. Secretion of a protein requires a signal peptide (also known as pre-sequence); a number of signal sequences from prokaryotes and eukaryotes are known to function for the secretion of recombinant proteins. During the protein secretion process, the signal peptide is removed by signal peptidase to yield the mature protein.

To simplify the protein purification process, a purification tag may be added either at the 5' or 3' end of the gene coding sequence. Commonly used purification tags include a stretch of six histidine residues (U.S. Patent Nos. 5,284,933 and 5,310,663), a streptavidin-affinity tag described by Schmidt and Skerra, Protein Engineering, 6:109-122 (1993), a FLAG peptide [Hopp et al., Biotechnology, 6:1205-1210 (1988)], glutathione S-transferase [Smith and Johnson, Gene. 67:31-40 (1988)], and thioredoxin [LaVallie et al., Bio/Technology, 11:187-193 (1993)]. To remove these peptide or polypeptides, a proteolytic cleavage recognition site may be inserted at the fusion junction. Commonly used proteases are factor Xa, thrombin, and enterokinase.

The invention also embraces polypeptides, i.e., species homologs and orthologs, that have at least about 99%, at least about 95%, at least about 90%, at least about 85%, at least about 80%, at least about 75%, at least about 70%, at least about 65%, at least about 60%, at least about 55%, and at least about 50% identity and/or. Percent amino acid sequence "identity" with respect to the preferred polypeptides of the invention is defined herein as the percentage of amino acid residues in the candidate sequence that are identical with the residues in the virulence gene product sequence after aligning both sequences and introducing gaps, ifnecessary, to achieve the maximum percent sequence identity, and not considering any conservative substitutions as part of the sequence identity. Percent sequence "homology" with respect to the preferred polypeptides of the invention is defined herein as the percentage of amino acid residues in the candidate sequence that are identical with the residues in one of the virulence polypeptide sequences after aligning the sequences and introducing gaps, if necessary, to achieve the maximum percent sequence identity, and also considering any conservative substitutions as part of the sequence identity. Conservative substitutions can be defined as set out in Tables A and B.

**Table A**

| Conservative Substitutions I | | |
|---|---|---|
| SIDE CHAIN CHARACTERISTIC | | AMINO ACID |
| Aliphatic | Non-polar | G A P |
| | | I L V |
| | Polar - uncharged | C S T M |
| | | N Q |
| | Polar - charged | D E |
| | | K R |
| Aromatic | | H F W Y |
| Other | | N Q D E |

Polypeptides of the invention may be isolated from natural bacterial cell sources or may be chemically synthesized, but are preferably produced by recombinant procedures involving host cells of the invention. Virulence gene products of the invention may be full length polypeptides, biologically active fragments, or variants thereof which retain specific biological or immunological activity. Variants may comprise virulence polypeptide analogs wherein one or more of the specified (i.e., naturally encoded) amino acids is deleted or replaced or wherein one or more non-specified amino acids are added: (1) without loss of one or more of the biological activities or immunological characteristics specific for the virulence gene product; or (2) with specific disablement of a particular biological activity of the virulence gene product. Deletion variants contemplated also include fragments lacking portions of the polypeptide not essential for biological activity, and insertion variants include fusion polypeptides in which the wild-type polypeptide or fragment thereof have been fused to another polypeptide.

Variant virulence polypeptides include those wherein conservative substitutions have been introduced by modification of polynucleotides encoding polypeptides of the invention. Conservative substitutions are recognized in the art to classify amino acids according to their related physical properties and can be defined as set out in Table A (from WO 97/09433, page 10, published March 13, 1997 (PCT/GB96/02197, filed 9/6/96). Alternatively, conservative amino acids can be grouped as defined in Lehninger, [Biochemistry, Second Edition; Worth Publishers, Inc. NY:NY (1975), pp.71-77] as set out in Table B.

**Table B**

| Conservative Substitutions II | |
|---|---|
| SIDE CHAIN CHARACTERISTIC | AMINO ACID |
| Non-polar (hydrophobic) | |
| A. Aliphatic: | A L I V P |
| B. Aromatic: | F W |
| C. Sulfur-containing: | M |
| D. Borderline: | G |
| Uncharged-polar | |
| A. Hydroxyl: | S T Y |
| B. Amides: | N Q |
| C. Sulfhydryl: | C |
| D. Borderline: | G |
| Positively Charged (Basic): | K R H |
| Negatively Charged (Acidic): | D E |

Variant virulence products of the invention include mature virulence gene products, i.e., wherein leader or signal sequences are removed, having additional amino terminal residues. Virulence gene products having an additional methionine residue at position - 1 are contemplated, as are virulence products having additional methionine and lysine residues at positions -2 and -1. Variants of these types are particularly useful for recombinant protein production in bacterial cell types. Variants of the invention.also include gene products wherein amino terminal sequences derived from other proteins have been introduced, as well as variants comprising amino terminal sequences that are not found in naturally occurring proteins.

The invention also embraces variant polypeptides having additional amino acid residues which result from use of specific expression systems. For example, use of commercially available vectors that express a desired polypeptide as a fusion protein with glutathione-S-transferase (GST) provide the desired polypeptide having an additional glycine residue at position - 1 following cleavage of the GST component from the desired polypeptide. Variants which result from expression using other vector systems are also contemplated.

The DNA and amino acid sequence information provided by the present invention also makes possible the systematic analysis of the structure and function of the virulence genes and their encoded gene products. Knowledge of a polynucleotide encoding a virulence gene product of the invention also makes available anti-sense polynucleotides which recognize and hybridize to polynucleotides encoding a virulence polypeptide of the invention. Full length and fragment anti-sense polynucleotides are provided. The worker of ordinary skill will appreciate that fragment anti-sense molecules of the invention include (i) those which specifically recognize and hybridize to a specific RNA (as determined by sequence comparison of DNA encoding a virulence polypeptide of the invention to DNA encoding other known molecules) as well as (ii) those which recognize and hybridize to RNA encoding variants of the family of virulence proteins. Antisense polynucleotides that hybridize to RNA encoding other members of the virulence familyofproteins are also identifiable through sequence comparison to identify characteristic, or signature, sequences for the family of molecules.

Any adjuvant known in the art may be used in the vaccine composition, including oil-based adjuvants such as Freund's Complete Adjuvant and Freund's Incomplete Adjuvant, mycolate-based adjuvants (*e.g*., trehalose dimycolate), bacterial lipopolysaccharide (LPS), peptidoglycans (*i.e*., mureins, mucopeptides, or glycoproteins such as N-Opaca, muramyl dipeptide [MDP], or MDP analogs), proteoglycans (*e.g.*, extracted from *Klebsiella pneumoniae*), streptococcal preparations (*e.g.,* OK432), Biostim™ (*e.g*., 01K2), the "Iscoms" of EP 109 942, EP 180 564 and EP 231 039, aluminum hydroxide, saponin, DEAE-dextran, neutral oils (such as miglyol), vegetable oils (such as arachis oil), liposomcs, Pluronic® polyols, the Ribi adjuvant system (see, for example GB-A-2 189 141), or interleukins, particularly those that stimulate cell mediated immunity. An alternative adjuvant consisting of extracts of *Amycolata,* a bacterial genus in the order Actinomycetales, has been described in U.S. Patent No. 4,877,612. Additionally, proprietary adjuvant mixtures are commercially available. The adjuvant used will depend, in part, on the recipient organism. The amount of adjuvant to administer will depend on the type and size of animal. Optimal dosages may be readily determined by routine methods.

The vaccine compositions optionally may include vaccine-compatible pharmaceutically acceptable (*i.e*., sterile and non-toxic) liquid, semisolid, or solid diluents that serve as pharmaceutical vehicles, excipients, or media. Any diluent known in the art may be used. Exemplary diluents include, but are not limited to, polyoxyethylene sorbitan monolaurate, magnesium stearate, methyl- and propylhydroxybenzoate, talc, alginates, starches, lactose, sucrose, dextrose, sorbitol, mannitol, gum acacia, calcium phosphate, mineral oil, cocoa butter, and oil of the theobroma.

The vaccine compositions can be packaged in forms convenient for delivery. The compositions can be enclosed within a capsule, caplet, sachet, cachet, gelatin, paper, or other container. These delivery forms are preferred when compatible with entry of the immunogenic composition into the recipient organism and, particularly, when the immunogenic composition is being delivered in unit dose form. The dosage units can be packaged, *e.g*., in tablets, capsules, suppositories or cachets.

The vaccine compositions may be introduced into the subject to be immunized by any conventional method including, *e.g*., by intravenous, intradermal, intramuscular, intramammary, intraperitoneal, or subcutaneous injection, by oral, sublingual, nasal, anal, or vaginal, delivery. The treatment may consist of a single dose or a plurality of doses over a period of time.

The invention also comprehends use of an attenuated bacterial strain of the invention for manufacture of a vaccine medicament to prevent or alleviate bacterial infection and/or symptoms associated therewith.

The present invention is illustrated by the following examples. Example 1 describes constructions of *P*. *multocida* mutants. Example 2 relates to screening for *P*. *multocida* mutants. Example 3 addresses methods to determine virulence of the *P*. *multocida* mutants. Example 4 describes cloning of *P*. *multocida* virulence genes.

### Example 1

### Construction of a Library of Tagged-Transposon P. multocida Mutants

A library of tagged-transposon mutants was constructed in parental vector pLOF/Km [Herrero, et al.. J Bactertol. 172:6557-67 (1990)] which has previously been demonstrated to be functional and random in *P*. *multocida* [Lee, et al., Vet Microbiol. 50:143-8 (1996)]. Plasmid pLOF/Km was constructed as a modification of suicide vector pGP704 and included a transposase gene under control of the *Tac* promoter as well as the mini-Tn10 transposable element encoding kanamycin resistance. Plasmid pTEF-1 was constructed as described below by modifying pLOF/Km to accept sequence tags which contained a semi-random [NK]₃₅ sequence.

Plasmid pLOF/Km was first modified to eliminate the unique *Kpn*I restriction site in the multiple cloning region and then to introduce a new *Kpn*I site in the mini-Tn10 region. The plasmid was digested with *Kpn*I and the resulting overhanging ends were filled in with Klenow polymerase according to manufacturer's suggested protocol. Restriction digests and ligations described herein were performed according to manufacturer's suggested protocols (Gibco BRL, Gaithersburg, MD and Boehringer Mannheim, Indianapolis, IN). The blunt end product was self-ligated to produce a plasmid designated pLOF/Km--*Kpn*I which was transformed into *E.coli* DH5α:λpir for amplification. *E.coli* DH5α: (λpir φ80dlacZΔM15, recA1, endA1, gyrA96, thi-1, hsdR17(rₖ, mₖ, supE44, relA1, deoR, Δ(lacZYA-argF)U169, was propagated at 37°C in Luria-Bertani (LB) medium. Plasmids were prepared using QIAGEN SpinPreps from QIAGEN Inc. (Santa Clarita, CA) and digested with *Sfi*I which cuts at a unique site within the mini-Tn10 transposable element. A *SfiI-Kpn*I*-Sfi*I adaptor was prepared by annealing oligonucleotides TEF1 (SEQ ID NO: 86) and TEF3 (SEQ ID NO: 87) and the resulting double-stranded adapter was ligated into the *Sfi*I site to create plasmid pTEF-1. Oligonucleotides TEF1 and TEF3 (as well as all other oligonucleotides described herein) were synthesized by Genosys Biotechnologies (The Woodlands, TX).
TEF1 5'-AGGCCGGTACCGGCCGCCT SEQ ID NO: 86
TEF3 5'-CGGCCGGTACCGGCCTAGG SEQ ID NO: 87

Unique sequence tags for insertion into the *Kpn*I site of pTEF-1 were prepared as follows. PCR was carried out to generate double stranded DNA tags using a GeneAmp XL PCR Kit (PE Applied Biosystems, Foster City, CA) under conditions including 250 µM each dNTP, 1.5 mM Mg(OAc)₂, 100 pmol each primer TEF14 (SEQ ID NO: 88) and TEF15 (SEQ ID NO: 89), 1 ng TEF26 (SEQ ID NO: 90) as template DNA and 2.5 units recombinant *Tth* DNA Polymerase XL.
TEF14 5'-CATGGTACCCATTCTAAC SEQ ID NO: 88
TEF15 5'-CTAGGTACCTACAACCTC SEQ ID NO: 89

Reaction conditions included an initial incubation at 95°C for one minute, followed by thirty cycles of 30 seconds at 95°C, 45 seconds at 45°C, and 15 seconds at 72°C, followed by a final incubation at 72°C for two minutes. The PCR products were digested with *Kpn*I and purified using a QIAGEN Nucleotide Removal Kit (QIAGEN, Inc., Chatsworth, GA) according to the manufacturer's suggested protocol. The unique tag sequences were ligated into the mini-Tn 10 element of linearized pTEF-1, previously digested with *KpnI* and dephosphorylated with calf intestinal alkaline phosphatase (Boehringer Mannheim) using standard procedures. The resulting plasmid library was transformed into *E.coli* DH5α:λpir. Colony blot analysis was performed according to the DIG User's Guide (Boehringer-Mannheim) with hybridization and detection performed as follows.

Hybridizations were essentially performed according to the Genius Non-Radioactive User's Guide (Boehringer Mannheim Biochemicals), the product sheet for the DIG-PCR labeling kit (Boehringer Mannheim Biochemicals), and the product sheet for CSPD (Boehringer Mannheim Biochemicals). For preparation of probes, a 100 µl primary PCR reaction was set up using Amplitaq PCR buffer (PE Applied Biosystems), 200 µM dNTPs, 140 pmol each of primers TEF5 (SEQ ID NO: 91) and TEF6 (SEQ ID NO: 92), 2 mM MgCl₂, 2.5 units Amplitaq (PE Applied Biosystems) and I ng of plasmid DNA.
TEF5 5'-TACCTACAACCTGAAGCT SEQ ID NO: 91
TEF6 5'-TACCCATTCTAACCAAGC SEQ ID NO: 92

Cycle conditions included an initial incubation at 95°C for two minutes, followed by 35 cycles of 95°C for 30 seconds, 50°C for 45 seconds, 72°C for 15 seconds and a final incubation at 72°C for three minutes. The amplification products were separated using electrophoresis on a 2% - 3:1 NuSieve GTG (FMC BioProducts, Rockland, ME, USA):Agarose gel and the 109 bp product was excised and purified. Gel extractions were carried out using a QIAGEN Gel Extraction kit (QIAGEN). Approximately 15 ng of the primary product was labeled in a 50 µl PCR reaction using the DIG PCR Kit, 50 pmol each of primers TEF24 and TEF25, and a 1:1 mix of DIG Probe Synthesis Mix with 2 mM dNTP stock solution.
TEF24 5'-TACCTACAACCTCAAGCTT SEQ ID NO: 93
TEF25 5'-TACCCATTCTAACCAAGCTT SEQ ID NO: 94

PCR conditions included an initial incubation at 95°C for four minutes, followed by 25 cycles of 95°C for 30 seconds, 50°C for 45 seconds, 72°C for 15 seconds and a final incubation at 72°C for three minutes. The labeled PCR product was digested with *Hind*III in a total reaction volume of 90 µl and purified from the constant primer arms using a 2% - 3:1 NuSieve GTG (FMC BioProducts):Agarose gel. The region containing the labeled variable tag was excised and the entire gel slice was dissolved and denatured in 10 ml of DIG EasyHyb at 95°C for ten minutes.

Dot blots were prepared using a Hybond^{®}-N⁺ membrane (Amersham-Pharmacia Biotech). Target DNA for each tag was prepared in 96 well plates using approximately 30 ng of PCR product. An equal volume of 0.1 N NaOH was added to denature the sample and each sample was applied to the membrane with minimal vacuum using a Minifold I™ Dot-Blot Apparatus from Schleicher and Schuell (Keene, NH, USA). Each well was washed with 150 µl of Neutralization Solution (0.5 M Tris /3 M NaCl, pH 7.5) and 150 µl of 2X SSC. Membranes were UV-crosslinked in a Stratalinker (Stratagene, La Jolla, CA, USA) and prehybridized for one hour in 20 mls DIG EasyHyb Buffer at 42°C. The denatured probe was added and hybridization carried out overnight at 42°C. The membrane was washed two times in 2X SSC containing 0.1 % SDS for five minutes each wash. Two high stringency washes were performed in 50 ml of pre-warmed 0.1X SSC buffer containing 0.1 % SDS at 68°C for 15 minutes before proceeding with standard Genius Detection protocols (Genius Manual).

It is desirable to use a non-radioactive detection system for safety, lower cost, ease of use, and reduction of hazardous materials. In initial experiments using similar procedures previously described [Mei, et al., Mol Microbiol. 26:399-407 (1997)], unacceptable background levels of hybridization were obtained in negative controls. In order to decrease background, tag length was increased by 30 bp to a total of 70, amplification primers were lengthened to include all sequence flanking the variable region, a lower concentration of dig-dUTP was used, and the conserved sequences flanking the sequence tag region were removed by gel purification. Most significantly, PCR was used to generate [NK]₃₅ sequence tags as the target DNA in dot blots rather than the entire plasmids containing the tagged transposons after detecting background hybridization from the transposon itself. Using these modifications background was eliminated making chemiluminescent/non-radioactive screening more effective.

Approximately four hundred different transformants resulting from the ligation of pTEF-1 with the PCR generated sequence tags were screened by colony blot and the 96 strongest hybridizing colonies were assembled into microtiter plates for further use. Even though the likelihood of duplicated tags was very low, half of the plate of master tags was probed against the other to confirm that no tags were duplicated. The plasmids containing these tags were purified and transformed into *E.coli* S17-1:λpir (pir, *recA, thi, pro, hsd,* (r-m+), RP4-2, (Tc::Mu), (Km::Tn7), [TmpR], [SmR]), and the transformed bacteria propagated at 37°C in Luria-Bertani (LB) medium. Each of the 96 *E.coli* S17-1:λpir transformants containing the tagged plasmid pTEF-1 was used in conjugative matings to generate transposon mutants of *P. multocida. P. multocida* strain TF5 is a spontaneous nalidixic acid resistant mutant derived from UC6731, a bovine clinical isolate. *P. multicida* strains were grown on brain heart infusion (BHI) media (Difco Laboratories, Detroit, MI, USA) at 37°C and in 5% CO₂ when grown on plates. Matings were set up by growing each *E.coli* S17-1:λpir /pTEF1:[NK]₃₅ clone and the TF5 strain to late log phase. Fifty µl of culture for each tagged-pTEF-1 clone was mixed with 200 µl of the TF5 culture and 50 µl of each mating mixture was spotted onto 0.22 TM filters previously placed on BHI plates containing 100 mM IPTG and 10 mM MgSO₄. Following overnight incubation at 37°C with 5% CO₂, mating mixtures were washed off of each filter into 3 ml of PBS and 25 µl of each was plated onto BHIN⁵⁰K¹⁰⁰ plates. Following selective overnight growth, colonies were assembled into microtiter plates by toothpick transfer into 200 µl BHIN⁵⁰K⁵⁰ making sure that each well in a microtiter plate always contained a transposon mutant with the same sequence tag. Following overnight growth, 50 µl of 75% glycerol was added to each well and plates were stored frozen at -80°C.

Nineteen pools were assembled by transferring the transposon mutants to microtiter plates making sure that each well contained a transposon mutant with the appropriate tag for that well. In other words, a specific well in each microtiter plate always contained a transposon mutant with the same sequence tag even though the location of the transposon within those mutants may be different.

### Example 2

### Murine Screening for Attenuated P. multocida Mutants

Nineteen pools of *Pasteurella multocida* transposon mutants were screened using a murine model of septicemia. Frozen plates of pooled *P. multocida* transposon mutants were removed from -80°C storage and subcultured by transferring 10 µl from each well to a new 96 well round bottom plate (Coming Costar, Cambridge, MA, USA) containing 200 µl of brain heart infusion (DIFCO) with 50 µg/ml nalidixic acid (Sigma) and 50 µg/ml kanamycin (Sigma) (BHIN⁵⁰K⁵⁰). Plates were incubated without shaking overnight at 37°C in 5% CO₂. Overnight plates were subcultured by transferring 10 µl from each well to a new flat bottomed 96-well plate (Coming Costar) containing 100 µl of BHI per well and incubating at 37°C with shaking at approximately 150 rpm. The OD₅₄₀ was monitored using a micro-titer plate reader. At an OD₅₄₀ of approximately 0.2 to 0.25, each plate was pooled to form the "input pool" by combining 100 µl from each of the wells of the micro-titer plate. The culture was diluted appropriately in BHI to doses of approximately 10⁴, 10⁵, 10⁶ CFU/ml and 0.2 ml of each dilution was used to infect female 14-16 g BALB/c mice by intraperitoneal administration. At two days post-infection, one or two surviving mice were euthanized and the spleens harvested. The entire spleen was homogenized in 1.0 ml sterile 0.9 % saline. Dilutions of the homogenate from 10⁻² to 10⁻⁵ were prepared and plated onto BHIN⁵⁰K⁵⁰ plates. Following overnight growth, at least 20,000 colonies were pooled in 10 mls BHI broth to form the "recovered pool" and 0.5 ml of the recovered pool was centrifuged at 3,500 X g and the pellet used to prepare genomic DNA according to a previously described protocol [Wilson, In F. M. Ausubel, et al.,(ed.), Current Protocols in Molecular Biology, vol. 1. John Wiley and Sons, New York, p. 2.4.1-2.4.5. (1997)].

Initial experiments with virulent wild-type *P*. *multocida* indicated that organisms could be recovered from the spleen, lungs, kidneys, and liver indicating a truly septicemic model of infection. Dot blots for both the "input" and "recovered" pools were performed as described in Example I and evaluated both by visual inspection and by semi-quantitative analysis. Hybridization was carried out as described in Example 1 except that 5 µg of genomic DNA from input and recovered pools was used as template. Semi-quantitative analysis indicates whether a significant reduction in a single clone has occurred. If a mutant is unable to survive within the host, then the recovered signal should be very low compared to the input signal yielding a high input/recovered ratio. Most mutants will grow as well *in vivo* as *in vitro* and therefore a ratio of their signals should be approximately equal to 1. Clones selected by quantitative analysis as being highly reduced in the recovered pool were selected for further study. Additional clones with questionable input/recovered ratios were also selected after visually evaluating films made from the dot blots.

### Example 3

### Determination of Virulence for P. multocida Candidate Mutants

Each potential mutant which exhibited reduced recovery from splenic tissue was isolated from the original pool plate and used individually in a challenge experiment to verify and roughly estimate the attenuation caused by the transposon mutation. Individual candidate mutants from *in vivo* screens were grown on Sheep Blood Agar plates overnight in 5% CO₂ at 37°C. Approximately six colonies of each mutant were inoculated into BHI broth and allowed to grow for six hours. Dilutions were prepared and five mice each were infected as described above with 10²*,* 10³, 10⁴ and 10⁵ CFU each. Attenuation was determined by comparing mortality after six days relative to the wild type. Surviving mice were presumed to be protected and then challenged with a dose of wild type *P*. *multocida* at a concentration approximately 200-fold greater than the LD₅₀ for the wild type strain. Survival rate was then determined for each challenged group of mice.

Results indicated that 62 of 120 potential transposon mutants were attenuated, having an approximate LD₅₀ of at least 10 fold higher than the wild type strain. The clones and their approximate LD₅₀ values are listed in Table 1. A control experiment with the wild type strain was run in parallel with each set of challenges and in all cases mortality in wild type-challenged groups was 100%.

In addition to LD₅₀ values, Table 1 also provides data from vaccination and challenge experiments. Briefly, groups of mice (n = 5 to 10) were vaccinated by intraperitoneal injection with the individual *P. multocida* strains shown in Table 1 at a dose that was approximately 200 times greater than the LD₅₀ of the virulent, wild type strain. Animals were observed for 28 days after which mortality figures were calculated.

**Table 1**

| *P. multocida* Virulence Genes | | | | | |
|---|---|---|---|---|---|
| **Nucleotide SEQ ID NO:** | **Representative isolate** | **PossibleGene Function** | **Vaccination # survivors/total** | **Challenge # survivors/total** | **LD₅₀** |
| - | wild type | - | 0/10 | - | <10 |
| 23 | PM1B1 | guaB | 10/10, 10/10, 10/10 | 9/10, 9/10 | 4.3 x 106 |
| 11 | PM1D1 | dsbB | 10/10, 5/10 | 10/10, 5/5 | 8.4 x 104 |
| 3 | PM1BD7 | atpG | 5/5, 10/10 | 10/10 | >3 x 105 |
| 74 | PM1BE11 | yhcJ (H10145) | 10/10 | 5/10 | >2 x 105 |
| 70 | PM1BF6 | yabK (HI1020) | 3/5, 8/10 | 9/9 | >2 x 105 |
| 19 | PM2G8 | fhaC | 4/5. 9/10 | 9/9 | >4 x 105 |
| 76 | PM3C9 | viaO(H10146) | 3/5 | | >6 x 105 |
| 118 | PM3G11 | UnkO | 4/5, 10/10 | 10/10 | >3 x 105 |
| 31 | PM7B4 | iroA (UnkB) | 0/5 | | |
| 17 | PM4C6 | fhaB (fhaB2) | 2/5, 10/10, 9/10 | 10/10, 9/9 | >3 x 106 |
| 9 | PM4G10-T9 | dnaA | 4/5 | | >5 x 105 |
| 1 | PM4D5-T5 | atpB | 5/5 | | >4 x 105 |
| 53 | PM4D5-T1 | UnkC2 | 5/5 | | >4 x 105 |
| 15 | PM4F2 | fhaB (fhaB1) | 3/5, 6/10, 10/10 | 6/6, 10/10 | >3 x 105 |
| 41 | PM5F7 | mreB | 4/5 | | 1 x 103 |
| 7 | PM5E2 | devB | 0/5, 3/10 | 2/3 | ? |
| 68 | PM6H5-T1 | xyla | 5/5 | | >3 x 105 |
| 78 | PM6H8 | vigF (H10719) | 5/5, 9/10 | 9/9 | >3 x 105 |
| 108 | PM7D12 | pnp | 5/5, 9/10 | 9/9 | |
| 51 | PM8C1R1-T2 | UnkCl | 5/5 | | -6 x 105 |
| 37 | PM8C1-T3 | mglB | 5/5 | | -6 x 105 |
| 58 | PM8C1R1-T6 | UnkDI | 5/5 | | -6 x 10⁵ |
| 45 | PM10H7 | purF (H11207) | 3/5, 8/10, 8/10 | 8/8, 8/8 | >3 x 105 |
| 25 | PM10H10-T2 | H11501 | 5/5 | | >1 x 104 |
| 72 | PM11G8-T2 | vgiK | 5/5 | | >2.4 x 103 |
| 21 | PM11G8-T4 | greA | 5/5 | | >2.4 x 103 |
| 84 | PM12H6 | yyam (H10687) | 3/5, 0/10 | | -2.2 x 103 |
| 33 | PM15G8-T2 | kdtB | 5/5 | | >1.2 x 105 |
| 116 | PM15G8-T1 | UnkK | 5/5 | | >1.2 x 105 |
| 104 | PM16G11-T1 | hmbR | 3/5 | | >1.9 x 105 |
| 29 | PM16G11-T2 | hxuC | 3/5 | | >1.9 x 105 |
| 35 | PM16H8 | IgtC | 5/5, 10/10 | 10/10 | >2.4 x 105 |
| 80 | PM16H3 | ylea (H10019) | 5/5, 10/10 | | > 2.0 x 105 |
| 49 | PM17H6-T1 | sopE | 4/5 | | -6 x 105 |
| 120 | PM17H6 | UnkP | 4/5 | | -8 x 105 |
| 5 | PM 18F5-T8 | cap5E | 5/5 | | >2.4 x 105 |
| 82 | PM18F5-T10 | yojB (H10345) | 5/5 | | >2.4 x 105 |
| 13 | PM19A1 | exbB | 5/5, 10/10 | 10/10 | >1.2 x 105 |
| 112 | PM19D4 | rci | 5/5, 8/10 | 8/8 | -1.6 x 105 |
| 39 | PM20A12 | mioC (H10669) | 3/5, 8/10 | 8/8 | -2 x 104 |
| 60 | PM20C2 | UnkD2 | 5/5, 10/10 | 10/10 | >8.2 x 106 |

### Example 4

### Cloning and Identification of Genes Required for P. multocida Virulence

Each transposon mutant which was verified to be attenuated was analyzed further to determine the identity of the disrupted open reading frame. DNA from each mutant was amplified, purified, and digested with restriction enzymes that were known not to cut within the transposon and generally produced 4-8 kb fragments that hybridized with the transposon. Using selection for kanamycin resistance encoded by the transposon, at least one fragment for each transposon mutant was cloned.

Southern hybridization with multiple restriction enzymes was performed for each attenuated mutant using a labeled 1.8 kb *Mlu*I fragment from pLOF/Km as a probe to identify a suitably sized fragment for cloning. The mini-Tn10 element and flanking DNA from each mutant was cloned into pUC19 and the flanking sequence determined using internal primers TEF32 and TEF40, primer walking and in some cases universal pUC-19 primers.
TEF-32 GGCAGAGCATTACGCTGAC SEQ ID NO: 95
TEF-40 GTACCGGCCAGGCGGCCACGCGTATTC SEQ ID NO:96
Sequencing reactions were performed using the BigDye™ Dye Terminator Chemistry kit from PE Applied Biosystems (Foster City, CA) and run on an ABI Prism 377 DNA Sequencer. Double stranded sequence for putative interrupted open reading frames was obtained for each clone. Sequencer 3.0 software (Genecodes, Corp., Ann Arbor, MI) was used to assemble and analyze sequence data. GCG programs [Devereux, et al., 1997. Wisconsin Package Version 9.0, 9.0 ed. Genetics Computer Group, Inc., Madison] were used to search for homologous sequences in currently available databases.

In 37% of the clones that were identified as being attenuated, there were multiple insertions of the mini-Tn10 transposable element. Each insertion including its flanking sequence was cloned individually into pGP704 and mated into the wild-type strain to produce new mutants of *P. multocida,* each carrying only one of the multiple original insertions. Individual mutants were retested individually to determine the insertion responsible for the attenuated phenotype. The nucleotide sequence of the disrupted, predicted open reading frame was determined by sequencing both strands, and the predicted amino acid sequence was used to search currently available databases for similar sequences. Sequences either matched known genes, unknown genes, and hypothetical open reading frames previously sequenced or did not match any previously identified sequence. For those genes having homology to previously identified sequences, potential functions were assigned as set out in Table 1.

## Claims

1. An attenuated *Pasteurella multocida* bacterium comprising a mutation in a gene represented by a polynucleotide sequence that encodes a *fhaC* polypeptide
(i) wherein the polynucleotide sequence hybridises to the complement of SEQ ID NO:19 under stringent conditions, such conditions comprising a final wash in buffer comprising 2X SSC/0.1% SDS, at 35°C to 45°C, or
(ii) wherein the polypeptide comprises an amino acid sequence at least 80% identical to SEQ ID NO:20;
wherein the attenuation of the bacterium is caused by said mutation.

2. The bacterium of claim 1, wherein said mutation results in decreased expression of a gene product encoded by the mutated gene.

3. The bacterium of claim 1, wherein said mutation results in expression of an inactive gene product encoded by the gene.

4. The bacterium of claim 1, wherein said mutation results in deletion of all or part of said gene.

5. The bacterium of claim 1, wherein said mutation results in an insertion in said gene.

6. The bacterium of claim 1, wherein the mutation is in the polynucleotide sequence of SEQ ID NO:19.

7. An immunogenic composition comprising the bacterium of any of claims 1 to 6.

8. A vaccine composition comprising an immunogenic composition of claim 7 and a pharmaceutically acceptable carrier.

9. The vaccine composition of claim 8, further comprising an adjuvant.

10. A purified and isolated polynucleotide comprising a nucleotide sequence encoding a polypeptide having SEQ ID NO: 20.

11. The polynucleotide of claim 10, comprising SEQ ID NO:19.

12. A purified polypeptide comprising SEQ ID NO: 20.

## Patentansprüche

1. Attenuiertes *Pasteurella multocida*-Bakterium, umfassend eine Mutation in einem Gen, das durch eine Polynucleotidsequenz dargestellt wird, welche ein *fhaC*-Polypeptid codiert,
(i) wobei die Polynucleotidsequenz an das Komplement von SEQ ID NO:19 unter stringenten Bedingungen hybridisiert, wobei solche Bedingungen einen Endwaschgang in Puffer, umfassend 2X SSC/0,1% SDS, bei 35°C bis 45°C umfassen, oder
(ii) wobei das Polypeptid eine Aminosäuresequenz umfasst, die zu wenigstens 80% identisch zu SEQ ID NO:20 ist;
wobei die Attenuierung des Bakteriums durch die genannte Mutation verursacht ist.

2. Bakterium gemäß Anspruch 1, wobei die Mutation in einer verringerten Expression eines Genproduktes, das durch das mutierte Gen codiert wird, resultiert.

3. Bakterium gemäß Anspruch 1, wobei die Mutation in einer Expression eines inaktiven Genprodukts, das durch das Gen codiert wird, resultiert.

4. Bakterium gemäß Anspruch 1, wobei die Mutation in einer Deletion des gesamten Gens oder eines Teils des Gens resultiert.

5. Bakterium gemäß Anspruch 1, wobei die Mutation in einer Insertion in das Gen resultiert.

6. Bakterium gemäß Anspruch 1, wobei die Mutation in der Polynucleotidsequenz von SEQ ID NO:19 ist.

7. Immunogene Zusammensetzung, die das Bakterium gemäß einem der Ansprüche 1 bis 6 umfasst.

8. Impfstoffzusammensetzung, die eine immunogene Zusammensetzung gemäß Anspruch 7 und einen pharmazeutisch annehmbaren Träger umfasst.

9. Impfstoffzusammensetzung gemäß Anspruch 8, die außerdem ein Adjuvans umfasst.

10. Gereinigtes und isoliertes Polynucleotid, das eine Nucleotidsequenz umfasst, die ein Polypeptid mit SEQ ID NO:20 codiert.

11. Polynucleotid gemäß Anspruch 10, das SEQ ID NO:19 umfasst.

12. Gereinigtes Polypeptid, das SEQ ID NO:20 umfasst.

## Revendications

1. Bactérie *Pasteurella multocida* atténuée comprenant une mutation dans un gène représentée par une séquence polynucléotidique qui code pour un polypeptide *fhaC*
(i) dans laquelle la séquence polynucléotidique s'hybridant au complément de SEQ ID NO:19 dans des conditions stringentes, de telles conditions comprenant un lavage final dans du tampon comprenant 2X SSC/0,1 % SDS, à 35°C-45°C, ou
(ii) dans laquelle le polypeptide comprenant une séquence d'acides aminés identique à raison d'au moins 80 % à SEQ ID NO:20,
dans laquelle l'atténuation de la bactérie étant causée par ladite mutation.

2. Bactérie selon la revendication 1, dans laquelle ladite mutation aboutit à une diminution de l'expression d'un produit génique codé par le gène muté.

3. Bactérie selon la revendication 1, dans laquelle ladite mutation aboutit à l'expression d'un produit génique inactif codé par le gène.

4. Bactérie selon la revendication 1, dans laquelle ladite mutation aboutit à la délétion de tout ou partie dudit gène.

5. Bactérie selon la revendication 1, dans laquelle ladite mutation aboutit à une insertion dans ledit gène.

6. Bactérie selon la revendication 1, dans laquelle la mutation est dans la séquence polynucléotidique de SEQ ID NO:19.

7. Composition immunogène comprenant la bactérie selon l'une quelconque des revendications 1 à 6.

8. Composition vaccinale comprenant une composition immunogène selon la revendication 7 et un support pharmaceutiquement acceptable.

9. Composition vaccinale selon la revendication 8, comprenant en outre un adjuvant.

10. Polynucléotide purifié et isolé comprenant une séquence de nucléotides codant pour un polypeptide ayant SEQ ID NO:20.

11. Polynucléotide selon la revendication 10, comprenant SEQ ID NO:19.

12. Polypeptide purifié comprenant SEQ ID NO:20.


### SEQUENCE LISTING

<110> Lowery, David E Fuller, Troy E Kennedy, Michael J
<120> ANTI-BACTERIAL VACCINE COMPOSITIONS
<130> PC29940A Div3
<140> 09/545,199
   <141> 2000-04-06
<150> 60/153,453
   <151> 1999-09-10
<150> 60/128,689
   <151> 1999-04-09
<160> 168
<170> PatentIn version 3.4
<210> 1
   <211> 1112
   <212> DNA
   <213> Pasteurella Multocida
<220>
   <221> misc_feature
   <223> atpB
<220>
   <221> CDS
   <222> (210)..(1001)
<220>
   <221> misc_feature
   <222> (1099)..(1099)
   <223> n is a, c, g, or t
<220>
   <221> misc_feature
   <222> (1104)..(1104)
   <223> n is a, c, g, or t
<400> 1
   gtcaacaaca ttttatggtg gagaggccgt taaatttata tccacaattt ttttgattgt      60
   acttgctttt aaactgttca attcaatgca ttttattgca ttttttgttg gatattttat     120
   aacaatagtt ttaaacaata ttcttccatt ttttataagt aagtacttaa atataaagca     180
   ttttcataaa tatcaataaa ggattagtt atg gca gca gag ctt aca aca gcg       233
                                   Met Ala Ala Glu Leu Thr Thr Ala
                                   1               5
   gga tat att ggg cac cat tta gca ttc ttg aaa aca ggg gat tct ttc       281
   Gly Tyr Ile Gly His His Leu Ala Phe Leu Lys Thr Gly Asp Ser Phe
       10                  15                  20
   tgg cat gtt cat tta gat acc ctt cta ttt tca att att tca ggt gca      329
   Trp His Val His Leu Asp Thr Leu Leu Phe Ser Ile Ile Ser Gly Ala
   25                  30                  35                  40
   att ttt ctt ttt gtt ttt tca aaa gtt gca aaa aaa gca acg ccg ggt      377
   Ile Phe Leu Phe Val Phe Ser Lys Val Ala Lys Lys Ala Thr Pro Gly
                   45                  50                  55
   gtg cct agc aag atg caa tgt ttt gtt gag ata atg gtt gat tgg att      425
   Val Pro Ser Lys Met Gln Cys Phe Val Glu Ile Met Val Asp Trp Ile
               60                  65                  70
   gat ggg atc gta aaa gaa aat ttc cat ggt cct cgt cat gct gtt gga      473
   Asp Gly Ile Val Lys Glu Asn Phe His Gly Pro Arg His Ala Val Gly
           75                  80                  85
   cca tta gca tta act att ttc tgc tgg gta ttc att atg aat gct atc      521
   Pro Leu Ala Leu Thr Ile Phe Cys Trp Val Phe Ile Met Asn Ala Ile
       90                  95                  100
   gat ttg atc cca gta gat ttc cta cct caa tta gcc cat tta ttt ggt      569
   Asp Leu Ile Pro Val Asp Phe Leu Pro Gln Leu Ala His Leu Phe Gly
   105                 110                 115                 120
   att gaa tac tta aga gct gtt cca aca gca gat atc agt gga aca tta      617
   Ile Glu Tyr Leu Arg Ala Val Pro Thr Ala Asp Ile Ser Gly Thr Leu
                   125                 130                 135
   ggc tta tca att ggt gtc ttc ttc tta att att ttc tat aca atc aaa      665
   Gly Leu Ser Ile Gly Val Phe Phe Leu Ile Ile Phe Tyr Thr Ile Lys
               140                 145                 150
   tca aaa ggt atg agt ggc ttt gtt aaa gaa tat acg ctt cat cct ttt      713
   Ser Lys Gly Met Ser Gly Phe Val Lys Glu Tyr Thr Leu His Pro Phe
           155                 160                 165
   aat cat cct ttg tta att ccg gtt aac tta gcg ctt gaa tca gtc aca      761
   Asn His Pro Leu Leu Ile Pro Val Asn Leu Ala Leu Glu Ser Val Thr
       170                 175                 180
   tta tta gca aaa cct gtt tct ttg gcg ttc cgt ctt ttc ggg aat atg      809
   Leu Leu Ala Lys Pro Val Ser Leu Ala Phe Arg Leu Phe Gly Asn Met
   185                 190                 195                 200
   tat gca ggt gaa ctt atc ttt att ctt att gca gtg atg tac atg gca      857
   Tyr Ala Gly Glu Leu Ile Phe Ile Leu Ile Ala Val Met Tyr Met Ala
                   205                 210                 215
   aat aat ttt gca ctt aat tca atg ggt att ttc atg cat ttg gct tgg      905
   Asn Asn Phe Ala Leu Asn Ser Met Gly Ile Phe Met His Leu Ala Trp
               220                 225                 230
   gct att ttc cat att ctt gtg att acc tta caa gca ttt att ttt atg      953
   Ala Ile Phe His Ile Leu Val Ile Thr Leu Gln Ala Phe Ile Phe Met
           235                 240                 245
   atg ctt aca gtg gtt tat ttg agt atg ggt tat aac aaa gca gaa cac     1001
   Met Leu Thr Val Val Tyr Leu Ser Met Gly Tyr Asn Lys Ala Glu His
       250                 255                 260
   taatttttta taaacaaaac cagaccttgg gtctaaattt caatcttatg gagaacatta    1061
   tggaacactg taattactac aacaatcatc gcatctgnaa ttnttcttgc t            1112
<210> 2
   <211> 264
   <212> PRT
   <213> Pasteurella Multocida
<400> 2
   Met Ala Ala Glu Leu Thr Thr Ala Gly Tyr Ile Gly His His Leu Ala
   1               5                   10                  15
   Phe Leu Lys Thr Gly Asp Ser Phe Trp His Val His Leu Asp Thr Leu
               20                  25                  30
   Leu Phe Ser Ile Ile Ser Gly Ala Ile Phe Leu Phe Val Phe Ser Lys
           35                  40                  45
   Val Ala Lys Lys Ala Thr Pro Gly Val Pro Ser Lys Met Gln Cys Phe
       50                  55                  60
   Val Glu Ile Met Val Asp Trp Ile Asp Gly Ile Val Lys Glu Asn Phe
   65                  70                  75                  80
   His Gly Pro Arg His Ala Val Gly Pro Leu Ala Leu Thr Ile Phe Cys
                   85                  90                  95
   Trp Val Phe Ile Met Asn Ala Ile Asp Leu Ile Pro Val Asp Phe Leu
               100                 105                 110
   Pro Gln Leu Ala His Leu Phe Gly Ile Glu Tyr Leu Arg Ala Val Pro
           115                 120                 125
   Thr Ala Asp Ile Ser Gly Thr Leu Gly Leu Ser Ile Gly Val Phe Phe
       130                 135                 140
   Leu Ile Ile Phe Tyr Thr Ile Lys Ser Lys Gly Met Ser Gly Phe Val
   145                 150                 155                 160
   Lys Glu Tyr Thr Leu His Pro Phe Asn His Pro Leu Leu Ile Pro Val
                   165                 170                 175
   Asn Leu Ala Leu Glu Ser Val Thr Leu Leu Ala Lys Pro Val Ser Leu
               180                 185                 190
   Ala Phe Arg Leu Phe Gly Asn Met Tyr Ala Gly Glu Leu Ile Phe Ile
                   195                 200                 205
   Leu Ile Ala Val Met Tyr Met Ala Asn Asn Phe Ala Leu Asn Ser Met
       210                 215                 220
   Gly Ile Phe Met His Leu Ala Trp Ala Ile Phe His Ile Leu Val Ile
   225                 230                 235                 240
   Thr Leu Gln Ala Phe Ile Phe Met Met Leu Thr Val Val Tyr Leu Ser
                   245                 250                 255
   Met Gly Tyr Asn Lys Ala Glu His
               260
<210> 3
   <211> 1972
   <212> DNA
   <213> Pasteurella Multocida
<220>
   <221> misc_feature
   <223> atpG
<220>
   <221> CDS
   <222> (364)..(1230)
<400> 3
   agcgggccat ttggctcagt tcggcttcgg attcttatga tgcacacgta agcaattatc      60
   acatggtcaa aaagtaactg aattattgaa acaaaaccaa tactctccgt tatctgtagc     120
   acaacaagca ttagtgttat ttgcagtaga gtttggttac ttagaagaag tggacttaga     180
   tcgtattggt tcatttgaat cagcactttt agagtatgct aaccataact atgctgattt     240
   tatgcgtgag ttaacccaat ctggcaatta caatgatgaa attaaagagt cattaaaagg     300
   cattttggat agcttcaaag caaacagtgc gtggtaagtt aacactttaa atggagagac     360
   aaa atg gca ggt gct aaa gag ata aga acc aaa atc gcg agt gta aaa       408
       Met Ala Gly Ala Lys Glu Ile Arg Thr Lys Ile Ala Ser Val Lys
       1               5                   10                  15
   agt aca caa aaa att act aaa gcg atg gaa atg gtt gct gcc tcg aaa       456
   Ser Thr Gln Lys Ile Thr Lys Ala Met Glu Met Val Ala Ala Ser Lys
                   20                  25                  30
   atg cgt aaa acg caa gaa cgc atg tct tct tca cgc cct tat tca gaa       504
   Met Arg Lys Thr Gln Glu Arg Met Ser Ser Ser Arg Pro Tyr Ser Glu
               35                  40                  45
   aca ata cgt aac gtg att agc cac gtt tcc aaa gca acg att ggt tac       552
   Thr Ile Arg Asn Val Ile Ser His Val Ser Lys Ala Thr Ile Gly Tyr
           50                  55                  60
   aag cat cca ttt tta gtg gat cgc gaa gta aaa aaa gtg ggc atg att      600
   Lys His Pro Phe Leu Val Asp Arg Glu Val Lys Lys Val Gly Met Ile
       65                  70                  75
   gtt gtg tcc aca gat cgt ggt ctt tgt ggt ggc tta aac gtg aac ttg      648
   Val Val Ser Thr Asp Arg Gly Leu Cys Gly Gly Leu Asn Val Asn Leu
   80                  85                  90                  95
   ttt aaa act gta tta aat gaa atg aaa gaa tgg aaa gaa aaa gat gtt      696
   Phe Lys Thr Val Leu Asn Glu Met Lys Glu Trp Lys Glu Lys Asp Val
                   100                 105                 110
   tcc gtt caa ttg agt tta atc ggt tct aaa tct atc aac ttt ttc caa      744
   Ser Val Gln Leu Ser Leu Ile Gly Ser Lys Ser Ile Asn Phe Phe Gln
               115                 120                 125
   tct ttg gga att aaa att tta acc caa gat tca ggt att ggt gat act      792
   Ser Leu Gly Ile Lys Ile Leu Thr Gln Asp Ser Gly Ile Gly Asp Thr
           130                 135                 140
   ccc tct gtt gag cag tta att ggt tca gtc aat tct atg att gat gct      840
   Pro Ser Val Glu Gln Leu Ile Gly Ser Val Asn Ser Met Ile Asp Ala
       145                 150                 155
   tat aaa aaa ggg gaa gta gat gtt gtg tat tta gtt tat aac aaa ttt      888
   Tyr Lys Lys Gly Glu Val Asp Val Val Tyr Leu Val Tyr Asn Lys Phe
   160                 165                 170                 175
   att aac acg atg tcg caa aag cca gta ttg gaa aaa tta att cca tta      936
   Ile Asn Thr Met Ser Gln Lys Pro Val Leu Glu Lys Leu Ile Pro Leu
                   180                 185                 190
   cca gaa tta gat aat gat gaa tta ggc gaa aga aaa caa gtt tgg gat      984
   Pro Glu Leu Asp Asn Asp Glu Leu Gly Glu Arg Lys Gln Val Trp Asp
               195                 200                 205
   tat att tac gaa cct gat gcg aaa gta tta tta gat aat tta ttg gtt     1032
   Tyr Ile Tyr Glu Pro Asp Ala Lys Val Leu Leu Asp Asn Leu Leu Val
           210                 215                 220
   cgt tat tta gaa tct cag gtt tat caa gca gca gtt gaa aac ctt gct     1080
   Arg Tyr Leu Glu Ser Gln Val Tyr Gln Ala Ala Val Glu Asn Leu Ala
       225                 230                 235
   tct gag caa gcc gct cga atg gtc gcc atg aaa gca gca aca gat aac     1128
   Ser Glu Gln Ala Ala Arg Met Val Ala Met Lys Ala Ala Thr Asp Asn
   240                 245                 250                 255
   gca ggt aac tta att aat gag tta cag tta gtc tat aac aaa gct cgt     1176
   Ala Gly Asn Leu Ile Asn Glu Leu Gln Leu Val Tyr Asn Lys Ala Arg
                   260                 265                 270
   caa gca agt att aca aat gaa tta aat gaa att gtt gcc ggt gca gca     1224
   Gln Ala Ser Ile Thr Asn Glu Leu Asn Glu Ile Val Ala Gly Ala Ala
               275                 280                 285
   gca att taacaaatag aggatcggta atggcaactg gaaaaattgt acaaatcatc      1280
   Ala Ile
   ggtgcggtta ttgacgttga attcccacaa gatgcagtac caaaagtata tgatgcctta 1340
   aatgttgaaa caggtttagt acttgaagtt caacaacaat taggtggtgg tgtagttcgc 1400
   tgtatcgcaa tgggatcatc tgatggatta aaacgcggtt taagcgtaac aaatacgaat 1460
   aacccaattt ctgttccagt gggaacgaaa acattgggtc gtatcatgaa cgtattgggt 1520
   gaaccaatcg atgagcaagg tgaaatcggt gcagaagaga attggtctat tcaccgtgcg 1580
   ccaccaagtt atgaagaaca atctaacagt actgaacttt tagaaacggg aattaaagtt 1640
   atcgacttag tttgtccgtt tgcgaaaggg ggtaaagtag gtttattcgg tggtgcgggt 1700
   gtcggtaaaa ccgtcaatat gatggaatta atccgtaaca tcgcaattga gcactcaggt 1760
   tactctgtct ttgcgggggt aggtgagcgt acgcgtgaag gtaacgactt ctatcatgag 1820
   atgaaagact ctaacgtatt agataaagtg tctcttgttt atggtcaaat gaacgagcca 1880
   ccaggtaacc gtttacgtgt ggcattaaca ggcttaacta tggcggaaaa attccgtgat 1940
   gaaggtcgtg atgtcttatt cttcgttgat aa 1972
<210> 4
   <211> 289
   <212> PRT
   <213> Pasteurella Multocida
<400> 4
   Met Ala Gly Ala Lys Glu Ile Arg Thr Lys Ile Ala Ser Val Lys Ser
   1 5 10 15
   Thr Gln Lys Ile Thr Lys Ala Met Glu Met Val Ala Ala Ser Lys Met
   20 25 30
   Arg Lys Thr Gln Glu Arg Met Ser Ser Ser Arg Pro Tyr Ser Glu Thr
   35 40 45
   Ile Arg Asn Val Ile Ser His Val Ser Lys Ala Thr Ile Gly Tyr Lys
   50 55 60
   His Pro Phe Leu Val Asp Arg Glu Val Lys Lys Val Gly Met Ile Val
   65 70 75 80
   Val Ser Thr Asp Arg Gly Leu Cys Gly Gly Leu Asn Val Asn Leu Phe
   85 90 95
   Lys Thr Val Leu Asn Glu Met Lys Glu Trp Lys Glu Lys Asp Val Ser
   100 105 110
   Val Gln Leu Ser Leu Ile Gly Ser Lys Ser Ile Asn Phe Phe Gln Ser
                   115                 120                 125
   Leu Gly Ile Lys Ile Leu Thr Gln Asp Ser Gly Ile Gly Asp Thr Pro
       130                 135                 140
   Ser Val Glu Gln Leu Ile Gly Ser Val Asn Ser Met Ile Asp Ala Tyr
   145                 150                 155                 160
   Lys Lys Gly Glu Val Asp Val Val Tyr Leu Val Tyr Asn Lys Phe Ile
                   165                 170                 175
   Asn Thr Met Ser Gln Lys Pro Val Leu Glu Lys Leu Ile Pro Leu Pro
               180                 185                 190
   Glu Leu Asp Asn Asp Glu Leu Gly Glu Arg Lys Gln Val Trp Asp Tyr
           195                 200                 205
   Ile Tyr Glu Pro Asp Ala Lys Val Leu Leu Asp Asn Leu Leu Val Arg
       210                 215                 220
   Tyr Leu Glu Ser Gln Val Tyr Gln Ala Ala Val Glu Asn Leu Ala Ser
   225                 230                 235                 240
   Glu Gln Ala Ala Arg Met Val Ala Met Lys Ala Ala Thr Asp Asn Ala
                   245                 250                 255
   Gly Asn Leu Ile Asn Glu Leu Gln Leu Val Tyr Asn Lys Ala Arg Gln
               260                 265                 270
   Ala Ser Ile Thr Asn Glu Leu Asn Glu Ile Val Ala Gly Ala Ala Ala
           275                 280                 285
   Ile
<210> 5
   <211> 1357
   <212> DNA
   <213> Pasteurella Multocida
<220>
   <221> misc_feature
   <223> cap5E
<220>
   <221> CDS
   <222> (1)..(813)
<400> 5
   gtc gac tat att tat cat gct gcc gca tta aag caa gtg cct tca tgc        48
   Val Asp Tyr Ile Tyr His Ala Ala Ala Leu Lys Gln Val Pro Ser Cys
   1               5                   10                  15
   gag ttt tat ccg tta gag gca gtg aaa acc aat att tta ggt acg gca        96
   Glu Phe Tyr Pro Leu Glu Ala Val Lys Thr Asn Ile Leu Gly Thr Ala
               20                  25                  30
   aat gtc tta gaa gcc gcc atc caa aac cag ata aaa cgc gtc gtc tgt       144
   Asn Val Leu Glu Ala Ala Ile Gln Asn Gln Ile Lys Arg Val Val Cys
           35                  40                  45
   ctt agc aca gat aaa gcg gtg tac cca att aat gcg atg ggc att tct       192
   Leu Ser Thr Asp Lys Ala Val Tyr Pro Ile Asn Ala Met Gly Ile Ser
       50                  55                  60
   aaa gca atg atg gaa aaa gtc atc atc gca aaa tcg cgt aac cta gaa       240
   Lys Ala Met Met Glu Lys Val Ile Ile Ala Lys Ser Arg Asn Leu Glu
   65                  70                  75                  80
   ggc aca cca acg aca atc tgt tgt act cgc tat ggc aat gtc atg gca       288
   Gly Thr Pro Thr Thr Ile Cys Cys Thr Arg Tyr Gly Asn Val Met Ala
                   85                  90                  95
   tcg cgt ggt tcg gtt atc cca tta ttt gtc gat caa ata cgt caa ggc       336
   Ser Arg Gly Ser Val Ile Pro Leu Phe Val Asp Gln Ile Arg Gln Gly
               100                 105                 110
   aag cct ttt act att act gat cct gag atg aca cgc ttt atg atg aca       384
   Lys Pro Phe Thr Ile Thr Asp Pro Glu Met Thr Arg Phe Met Met Thr
           115                 120                 125
   ttg gaa gat gct gtg gat tta gtc cta tat gca ttt aaa aat ggt caa       432
   Leu Glu Asp Ala Val Asp Leu Val Leu Tyr Ala Phe Lys Asn Gly Gln
       130                 135                 140
   aat ggt gat gtt ttt gta caa aaa gcc ccc gca gca acc att ggt acc       480
   Asn Gly Asp Val Phe Val Gln Lys Ala Pro Ala Ala Thr Ile Gly Thr
   145                 150                 155                 160
   ctt gcc aaa gca att acc gaa tta tta tct gtc cca aat cac cct att       528
   Leu Ala Lys Ala Ile Thr Glu Leu Leu Ser Val Pro Asn His Pro Ile
                   165                 170                 175
   tcc att ata ggt acg cgt cat gga gag aaa gca ttc gaa gct tta tta       576
   Ser Ile Ile Gly Thr Arg His Gly Glu Lys Ala Phe Glu Ala Leu Leu
               180                 185                 190
   agc cgt gaa gaa atg gtt cat gca att aat gaa ggt aat tat tat cgc       624
   Ser Arg Glu Glu Met Val His Ala Ile Asn Glu Gly Asn Tyr Tyr Arg
           195                 200                 205
   atc cca gcc gat caa cgc agt tta aat tac agt aaa tat gtc gaa aaa       672
   Ile Pro Ala Asp Gln Arg Ser Leu Asn Tyr Ser Lys Tyr Val Glu Lys
       210                 215                 220
   ggg gaa cca aaa att acc gaa gtc acc gac tac aac tca cat aat act       720
   Gly Glu Pro Lys Ile Thr Glu Val Thr Asp Tyr Asn Ser His Asn Thr
   225                 230                 235                 240
   gag cgt ttg act gtc aag gaa atg aag cag tta ctg ctt aaa ctt gaa       768
   Glu Arg Leu Thr Val Lys Glu Met Lys Gln Leu Leu Leu Lys Leu Glu
                   245                 250                 255
   ttc ata cag aaa atg att gag ggt gaa tac atc tca ccg gag gta           813
   Phe Ile Gln Lys Met Ile Glu Gly Glu Tyr Ile Ser Pro Glu Val
               260                 265                 270
   taaaaatgaa agtcttagta actggttcaa atggttttat tgcgaaaaat ctgattcagt     873
   ctttatctga ggaacaagat attgagattt tatgttatca ccgtcaatcc tctgagaaaa     933
   cgcttattca tcatgtattg agtgctgatt ggattattca tcttgcgggt gcgaatcgtc     993
   cacctgaaga acaagaattt atgacatcaa atacacaatt gacggaaaaa atttgccgta    1053
   ttttacagcg tcatcagaaa aaaacgcctt tgttatattc ctctagcatt caagtagaaa    1113
   gtcccaaaat aagtacttat tcgcaaacca aattagaaag tgaatatcat gttcatcaat    1173
   tacataaaga aaatggtaat ccgatttata tctgccgttt agctaatgtc tttggcaaat    1233
   ggtcacgacc tcactataac tcggtagtcg ccacattttg ccataactta attcatgatt    1293
   tacccatcga aattcatgat catactgcag aaataaggct catttatatt gatgatgtcg    1353
   ttga                                                                 1357
<210> 6
   <211> 271
   <212> PRT
   <213> Pasteurella Multocida
<400> 6
   Val Asp Tyr Ile Tyr His Ala Ala Ala Leu Lys Gln Val Pro Ser Cys
   1               5                   10                  15
   Glu Phe Tyr Pro Leu Glu Ala Val Lys Thr Asn Ile Leu Gly Thr Ala
               20                  25                  30
   Asn Val Leu Glu Ala Ala Ile Gln Asn Gln Ile Lys Arg Val Val Cys
           35                  40                  45
   Leu Ser Thr Asp Lys Ala Val Tyr Pro Ile Asn Ala Met Gly Ile Ser
       50                  55                  60
   Lys Ala Met Met Glu Lys Val Ile Ile Ala Lys Ser Arg Asn Leu Glu
   65                  70                  75                  80
   Gly Thr Pro Thr Thr Ile Cys Cys Thr Arg Tyr Gly Asn Val Met Ala
                   85                  90                  95
   Ser Arg Gly Ser Val Ile Pro Leu Phe Val Asp Gln Ile Arg Gln Gly
                   100                 105                 110
   Lys Pro Phe Thr Ile Thr Asp Pro Glu Met Thr Arg Phe Met Met Thr
           115                 120                 125
   Leu Glu Asp Ala Val Asp Leu Val Leu Tyr Ala Phe Lys Asn Gly Gln
       130                 135                 140
   Asn Gly Asp Val Phe Val Gln Lys Ala Pro Ala Ala Thr Ile Gly Thr
   145                 150                 155                 160
   Leu Ala Lys Ala Ile Thr Glu Leu Leu Ser Val Pro Asn His Pro Ile
                   165                 170                 175
   Ser Ile Ile Gly Thr Arg His Gly Glu Lys Ala Phe Glu Ala Leu Leu
               180                 185                 190
   Ser Arg Glu Glu Met Val His Ala Ile Asn Glu Gly Asn Tyr Tyr Arg
           195                 200                 205
   Ile Pro Ala Asp Gln Arg Ser Leu Asn Tyr Ser Lys Tyr Val Glu Lys
       210                 215                 220
   Gly Glu Pro Lys Ile Thr Glu Val Thr Asp Tyr Asn Ser His Asn Thr
   225                 230                 235                 240
   Glu Arg Leu Thr Val Lys Glu Met Lys Gln Leu Leu Leu Lys Leu Glu
                   245                 250                 255
   Phe Ile Gln Lys Met Ile Glu Gly Glu Tyr Ile Ser Pro Glu Val
               260                 265                 270
<210> 7
   <211> 6132
   <212> DNA
   <213> Pasteurella Multocida
<220>
   <221> misc_feature
   <223> devB
<220>
   <221> CDS
   <222> (4032)..(4727)
<400> 7
   gtcaacaatt accgcacttt agtggagtaa ataacaatgg cgaaaaagaa taacgggcat      60
   gaaaaagagg atgatgttcg cctagataaa tggctttggg ctgcccgttt ttataaaaca     120
   cgtactttag caaaagacat gattgatggc ggtaaagtgc attataatgg gcagcgcacg 180
   aaacccaata aaacggttga aattggtggt gtgatcaaac ttcgtcaagg taatgacgaa 240
   aaagaagtgg aagtgcttgc gctttctacg caacgtcgtg gggcgccaga agcacaattg 300
   ttgtatcaag aaacagaaaa aagccttgaa caacgtgcga aaatggcgat tgcacgtaag 360
   attaatgcat taacgatgcc gcatcctgat cgtcgcccga ataaaaaaga gcggcgtgat 420
   ttattgaaat ttaaacatca agatagcttt tcatcttgat gatgtgattt acctactttt 480
   cttattaaag aaaggaatat ggggaagctg tgtgcttgcc cttaacctga ataaaggctt 540
   tttatgacag acaacacaga caatgacaaa ctgtatcgct accttttcca agatcgcgcg 600
   gtgcgcggtg aatgggtacg gttaaaccaa acgtttactg atacgttaaa tacacatcaa 660
   tatccgaaag tcatccaaaa cttgctcggt gaaatgatgg tggcgaccag tttattgacg 720
   gcgacgttaa aatttgaagg ggatattact gttcaagtac aaggtgatgg accattaaaa 780
   ttagcattag ttaatggcaa tcatcagcag caaattcgcg cattagcgcg tttacaagcg 840
   gatgtcagtg atgatatgag tttggcgcaa ttagtcggga aaggggtatt agtgattacg 900
   attgcaccga cagaaggcga gcgttaccaa ggcgtgattg cgttagataa gccaaccatt 960
   actgcctgtc ttgaagatta ttttgtgcgt tcagaacaat tgcaaaccca gcttattatt 1020
   cgtgctggcg aatttgaagg acaacctgtg gcagccggta tgttgttaca aattatgccg 1080
   gacggttcag gttctccaga ggattttgaa cacttagcaa cattggcagc gacggtgaaa 1140
   gaggaagaac tatttggttt aacagcagaa gaattattgt accgtttata tcatgaagag 1200
   cgtgttgaaa ttttcccttc acagccgatc tcatttttct gtggctgctc acaagaacgt 1260
   tctggtgccg cattgttgtt gatttctgat gaagaattgg atgaagtctt ggcagagcat 1320
   aacggtacca ttgatatgca gtgtgaatgt tgtgggacgc attatttctt caataaagca 1380
   gcgattatgc aattgaaagt agaaaaataa gtcttggtat tatggatttt gttgcaggct 1440
   atcaagatat ttgatagcct gtttttcctt tgcagcaaac gattttatga gaaaaacgcc 1500
   gtcttctcac acagtagttt aggtattgca tagcatgaag cgggaaacta tgtttggcgc 1560
   ttgtctgttt aaaaaggtta tctatgttag cactttcacc atcattactt gaaaaaacac 1620
   ttgaaatcgc ggatcaagca ggagattttt taacgcgatt ttataccgac tcagggcaaa 1680
   atgcgttagc aattcaaacc aaacaagata acacgccagt gactgccgtg gatttatttt 1740
   tgagtcaatt tttaattgaa aaattgaccg cactgacacc tgaggtcccg attctttccg 1800
   aagagagttg taaaatcccc ttacaagatc gtgcacattg ggcagaatat tggttaattg 1860
   acccactcga tggaacccaa caatttatta atcgcaccga tcaattttcc attttgatta 1920
   cacttgtcca acacaatcaa cccgtcttaa gcattaccca tgcccctatt ttacaaacga 1980
   cctattatgc tatgcagggc tttggtgctt acagaaggca gggcaatcaa caagaaaagc 2040
   taaacaacca agcgcgacct gagcagcgaa aaatcaaaat tgctgtggga gtcgggggcg 2100
   ttgaacggaa aattcagccc ttgttaaatc cagcttatca atatgaattt ttggtttatg 2160
   gttcaagtgg cttaaaaggc ggcttagtgg ccgatggcac ttgtgattgc tatattcgag 2220
   taggaaaaac gggtgaatgg gatacggggg cggctgaaat cctcttacgt gaaatgggag 2280
   gtgctgtgtt tgactttgct tttcaaccgc ttagctataa tcagagagaa agttttatta 2340
   atcccaattt tgtgatggta gcgaatacag aatttgattg gcagaaaatt tttcaatttc 2400
   attcgcacta ggcattattt attataagat gcgatatctt atgacctcta tttttaacaa 2460
   acggatttca ggaaaaaaat gaaaattgaa gcagacaaca attgtattgt aatttttggt 2520
   gcatcaggtg atttaactta tcgtaaattg attcctgcac tgtataactt atataaaatc 2580
   ggtcgtttga ctgagcattt ctccgtgtta ggtgtggcaa gaacggaatt aagtgatgag 2640
   ggtttccgtg aaaaaatgcg ccaagcgttg atcaaaagtg aaaaagcgaa tggcgaaaca 2700
   ctcgatcaat tttgtagcca cctttattat caggcattaa ataccgcgga tgctgccgat 2760
   tatggcaagt taattcctcg tcttgatgac ttacatgata aatatcaaac ttgtggtaac 2820
   acactttact atttatctac gccgccaagc ctttatggcg tgattccaga atgccttgcg 2880
   gcacatgggt taaatactga agagtttggc tggaagcggt taattgtgga aaaaccgttt 2940
   ggttatgata tacgcacggc aaaagaactc gatattcaaa ttcaccgttt ctttgatgaa 3000
   caccaaattt atcgtattga ccactatctt ggtaaagaaa cggttcaaaa tctgcttgtg 3060
   ttgcgttttt ctaatggatg gtttgaacca ctctggaacc gtaatttcat tgattatatt 3120
   gaaatcacgg gcgcagaatc tatcggtgta gaagagcgtg gtggttatta cgatgattct 3180
   ggcgcaatgc gtgatatgtt ccaaaaccat ttgttgcaag tgttagccat ggttgcgatg 3240
   gagccaccag caattattaa tgccgactca atgcgtgatg aagtggcaaa agtcttgtat 3300
   tgtttacatc cattaagtga ggatgactta gaaaatcatt tagtcttagg gcaatatacg 3360
   gcaggcacag ttgaaggtga agcggttaag ggctacttac aggaaaaagg tgtgccggca 3420
   gagtctaata cggaaactta catggcatta cgttgtgaaa ttgacaactg gcgttgggcg 3480
   ggtgtgccat tttatgtgcg tactgggaaa cggttaccaa gtcgagtgac cgaaattgtg 3540
   attcatttca aaaccacacc acatccggta tttagccaaa aagcaccaga aaacaaatta 3600
   attatccgta ttcaacccga tgaagcgatt tcgatgcgtt ttggtttgaa aaaaccggga 3660
   gcaggttttg aagcaaaaga agtgtcgatg gatttccgtt atgcggatct tgcctcacca 3720
   agcttactga ccgcttatga gcgtttatta ttggattcta tgaaaggcga tgccactttg 3780
   tttgcgcgta ctgatgcggt acatgcctgt tggcagtttg tggagccgat tttacaatat   3840
   aaagcacaaa atgggcgtgt ttatgagtat gaagccggta cttggggacc gacagaagcc   3900
   gacaaactga tcgcgaaaac gggtcgtgtt tggcgtaaac caagtggatt aatgaaaaag   3960
   aaagtgtaat gtccgcctct ttcgtaagaa atgcgagggg ctaatgtgag cagattgagt   4020
   aaggaaagat c atg aat aca atc att ttt gac agt gca cag cat gcc gta    4070
                Met Asn Thr Ile Ile Phe Asp Ser Ala Gln His Ala Val
                1               5                   10
   gag aaa att gca caa gaa ttg tta gcg tat agc tta gaa ggt cgc cct     4118
   Glu Lys Ile Ala Gln Glu Leu Leu Ala Tyr Ser Leu Glu Gly Arg Pro
       15                  20                  25
   gtg cat att tcc tta tcc gga ggc tca acg ccg aaa ttg tta ttt aaa     4166
   Val His Ile Ser Leu Ser Gly Gly Ser Thr Pro Lys Leu Leu Phe Lys
   30                  35                  40                  45
   act tta gct caa gca ccg tat aac acc gag att caa tgg aaa aat ttg     4214
   Thr Leu Ala Gln Ala Pro Tyr Asn Thr Glu Ile Gln Trp Lys Asn Leu
                   50                  55                  60
   cat ttt tgg tgg ggc gat gat cgt atg gtg cca cca acc gat cca gaa     4262
   His Phe Trp Trp Gly Asp Asp Arg Met Val Pro Pro Thr Asp Pro Glu
               65                  70                  75
   agt aat tac ggc gag gtg caa aaa ttg tta ttc gat cat att cag atc     4310
   Ser Asn Tyr Gly Glu Val Gln Lys Leu Leu Phe Asp His Ile Gln Ile
           80                  85                  90
   cct gca gaa aat att cac cgc att cgt ggt gaa gcc ccc gtt gag agt     4358
   Pro Ala Glu Asn Ile His Arg Ile Arg Gly Glu Ala Pro Val Glu Ser
       95                  100                 105
   gaa ctt cac cgt ttt gaa caa gcg cta agt gcg gtc att cct ggg caa     4406
   Glu Leu His Arg Phe Glu Gln Ala Leu Ser Ala Val Ile Pro Gly Gln
   110                 115                 120                 125
   gtt ttt gat tgg att att ttg ggc atg gga acg gac ggg cac acg gcc     4454
   Val Phe Asp Trp Ile Ile Leu Gly Met Gly Thr Asp Gly His Thr Ala
                   130                 135                 140
   tca tta ttc ccg cat caa acc gat ttt gac gat cct cat ttc gcc gtg     4502
   Ser Leu Phe Pro His Gln Thr Asp Phe Asp Asp Pro His Phe Ala Val
               145                 150                 155
   atc gcg aaa cac cct gaa aca ggg caa att cgt att tca aaa aca gcg     4550
   Ile Ala Lys His Pro Glu Thr Gly Gln Ile Arg Ile Ser Lys Thr Ala
           160                 165                 170
   aaa ttg att gaa caa gca aag cgg gtg acc tat ttg gtg aca ggt agc     4598
   Lys Leu Ile Glu Gln Ala Lys Arg Val Thr Tyr Leu Val Thr Gly Ser
       175                 180                 185
   agt aaa gcc gag atc tta aaa gaa att caa act act ccg gca gaa caa     4646
   Ser Lys Ala Glu Ile Leu Lys Glu Ile Gln Thr Thr Pro Ala Glu Gln
   190                 195                 200                 205
   ctg cct tat cct gct gcg aaa atc aaa gcg aag cat ggg gtg acg gaa     4694
   Leu Pro Tyr Pro Ala Ala Lys Ile Lys Ala Lys His Gly Val Thr Glu
                   210                 215                 220
   tgg tat ttg gat aag gat gcg gca aaa tta ctg taatgcgtcg tgagattttt   4747
   Trp Tyr Leu Asp Lys Asp Ala Ala Lys Leu Leu
               225                 230
   caacattttt gcaaagagac ttgaaacaaa atagaccata gcgttcgttt tcaacgagtg   4807
   ctgaaaatga aggctctcgt tgaaaatggc gccatttagt gggtaagctt aaggttcgct   4867
   cagacagcgc tatcaaaagg gtaaaagaat gtatcaactc tattttaatc caccaaaatc   4927
   ttcatggtca ttacgcgttt gggtgttatt gaaagaactg gcaattcctt ttgagcctaa   4987
   aattgtacgt tatttggatg atttaagtga acaacgccaa caatttaagg cgttttcgcc   5047
   gacttcaaaa atcccagtat tgcatgctga tggtgttgtc atttgggaca gtttagcgat   5107
   tatcgagttt ttggcagaaa gttatccgca cgtgtgggcg caagataagg cgacaagagc   5167
   gtggtcacgt tctgcttgtg ctgaaatgca ctctggcttt gaaaatttgc gtgaaatgtg   5227
   tgatttcgcc cctttagctc gcaaaccgtt acaagaaatg cccgctgtgt taagccaaga   5287
   gctaacaagg cttaatcaat tattagaaga agggttaaca aatcacactg ggcgatttaa   5347
   tgcaaacctc cagtgctttt ggtcgagtac aatcctcgtt gtcttttttc cgtaattcct   5407
   atgatgactt tgccgcttat cgtgcggtgt taaatcgttt aactggcttt catagcgcga   5467
   ttaaagcggc taatcgccca agcaaattag tgctcacaga aagtcgtcat gatattggtt   5527
   ttcatgatct caacgtagag accccgcaag gaaaaacctt aattgacaag ctcaacctac   5587
   aatttcctct cggtacatgg ttattaattc aaggacattc tggtgtaggg aaaacaacct   5647
   tgttaagaac cattgcggga ctatggcctt atgctagtgg gacaattcaa cgtccacaac   5707
   aagatactct gtttctttct caaaaacctt atttgccaca aggtcgccta cttgatgccc   5767
   tattttatcc tgaactggcg cctgaagacg tgaatgagca acaagttata gacatactcg   5827
   cgaaagtaca actcgggcat ttaagcgata aactagaaca agaaaatgat tggacacggg   5887
   tactctcttt aggtgaacaa caacgtctgt cgtttgctcg cattttattg cataaaccta   5947
   ctgttgtttt cttagatgaa gccactgcca gcatggatga aggactggaa gatgcgatgt   6007
   accgcttact gaaagatgaa ctgcctcaga ttactgtgat cagtgttgga caccgttcga   6067
   cgttaattcc gcaccattca cagcaattac acattcaata acaagacagg gcgttgtgga   6127
   gtcga                                                               6132
<210> 8
   <211> 232
   <212> PRT
   <213> Pasteurella Multocida
<400> 8
   Met Asn Thr Ile Ile Phe Asp Ser Ala Gln His Ala Val Glu Lys Ile
   1               5                   10                  15
   Ala Gln Glu Leu Leu Ala Tyr Ser Leu Glu Gly Arg Pro Val His Ile
               20                  25                  30
   Ser Leu Ser Gly Gly Ser Thr Pro Lys Leu Leu Phe Lys Thr Leu Ala
           35                  40                  45
   Gln Ala Pro Tyr Asn Thr Glu Ile Gln Trp Lys Asn Leu His Phe Trp
       50                  55                  60
   Trp Gly Asp Asp Arg Met Val Pro Pro Thr Asp Pro Glu Ser Asn Tyr
   65                  70                  75                  80
   Gly Glu Val Gln Lys Leu Leu Phe Asp His Ile Gln Ile Pro Ala Glu
                   85                  90                  95
   Asn Ile His Arg Ile Arg Gly Glu Ala Pro Val Glu Ser Glu Leu His
               100                 105                 110
   Arg Phe Glu Gln Ala Leu Ser Ala Val Ile Pro Gly Gln Val Phe Asp
           115                 120                 125
   Trp Ile Ile Leu Gly Met Gly Thr Asp Gly His Thr Ala Ser Leu Phe
       130                 135                 140
   Pro His Gln Thr Asp Phe Asp Asp Pro His Phe Ala Val Ile Ala Lys
   145                 150                 155                 160
   His Pro Glu Thr Gly Gln Ile Arg Ile Ser Lys Thr Ala Lys Leu Ile
                   165                 170                 175
   Glu Gln Ala Lys Arg Val Thr Tyr Leu Val Thr Gly Ser Ser Lys Ala
               180                 185                 190
   Glu Ile Leu Lys Glu Ile Gln Thr Thr Pro Ala Glu Gln Leu Pro Tyr
           195                 200                 205
   Pro Ala Ala Lys Ile Lys Ala Lys His Gly Val Thr Glu Trp Tyr Leu
       210                 215                 220
   Asp Lys Asp Ala Ala Lys Leu Leu
   225                 230
<210> 9
   <211> 2438
   <212> DNA
   <213> Pasteurella Multocida
<220>
   <221> misc_feature
   <223> dnaA
<220>
   <221> CDS
   <222> (1635)..(2396)
<400> 9
   gacccaatgc ttgccaccgg cggctctatg attgcgacaa tcgatcttct aaaagcgaaa     60
   ggctgtaaac acattaaagt gctcgtgtta gtcgccgcgc ctgaaggcat taaagcatta    120
   gaagctgcgc accctgatat cgaattatat accgcatcag ttgatagtca cttaaatgaa    180
   caaggctata ttattccagg tcttggtgat gccggtgata aaatttttgg cactaaataa    240
   tcccaacaca agcggcatct tatgccgctt ttttccgttc aatttatagc gcttacaatc    300
   ttaacagctt gaacactata aaatgaaaag ttaattcaga cagagagttg aaacttaaca    360
   tgacaaatca aaatccccct gttcttctag aacaaaatca cgcaaaacaa gccttcgttg    420
   ggctacaaat gctttttgtt gccttcggtg ctttagtcct tgttcccctg attacgggtt    480
   taaatgccaa tactgcctta ttgaccgcag ggattgggac actcttattc caactttgta    540
   ctggacgcca agtcccaatt ttcttagcct cttcctttgc ttttattgca ccaattcaat    600
   atggcgtggc aacatggggc attgctacta ctatgggggg gctggtgttt actggactgg    660
   tttattttgc cctcagtacg ttagtcaaaa ttaaaggtgc tggtgcttta caaaaagtct    720
   ttccgccagt agttgttggt cccgttatta tcatcatcgg tatgggactt gcccctgttg    780
   ccgtggacat ggcattaggt aaaaacagca cttatcaata taacgatgcc gtattcgttt    840
   cgatggcaac attattgaca acgttaggtg ttgcggtgtt tgctaaaggc atgatgaaat    900
   taattcctat catgtttggt attgtcgtcg gctatatcct ctgcttattc ttaggcttaa    960
   ttaatttcca acctgtcatt gatgcacctt ggtttagtgt gccagaaatt actacccctg   1020
   aatttaaatt agaagctatt ctttatttat taccgattgc tatcgcccca gcagttgagc   1080
   atgtcggtgg gatcatggca atcagttcgg tgacagggaa agacttccta caaaaaccag   1140
   gattacatcg cactttatta ggggatggta ttgcaacgag tgccgcctca ttcttaggag   1200
   gaccacctaa tacaacttat gctgaagtca ctggtgctgt catgcttacg cgcaacttta   1260
   accctaaaat catgacatgg gcagccgttt gggcaattgc gatttccttc tgtggtaaag   1320
   tcggggcttt cctctctacc attccaacta ttgtcatggg tggcattatg atgttagtgt   1380
   ttggctctat cgccgtagtc ggtatgagta cactgatccg tggtaaagtg gatgtaacag   1440
   aagcgcgtaa tctgtgtatt atttccgttg tgatgacgtt tggcatcggt ggtatgtttg   1500
   tgaactttgg agaagtctcc ttaaaaggga ttagtttatg cgccgttgtc gcgattttac   1560
   tcaacttaat cttacccaaa gccaaaaaca ccccaataga agaaaatcga taagagaaaa   1620
   ttaagggtta agtc ttg ctt aac cct tca ttt ttc gtt tat cct tat tct     1670
                   Leu Leu Asn Pro Ser Phe Phe Val Tyr Pro Tyr Ser
                   1               5                   10
   cct ttt ttc gat ttt gta ggt tgc ttt ttg tta gaa aat ttc caa tta     1718
   Pro Phe Phe Asp Phe Val Gly Cys Phe Leu Leu Glu Asn Phe Gln Leu
           15                  20                  25
   cct ttg cct att cat caa ctc gat gat gaa acg ctg gat aat ttc tat     1766
   Pro Leu Pro Ile His Gln Leu Asp Asp Glu Thr Leu Asp Asn Phe Tyr
       30                  35                  40
   ccc gac aat aat tta ttg ttg ctc aat tcg cta cgc aaa aat ttt act     1814
   Pro Asp Asn Asn Leu Leu Leu Leu Asn Ser Leu Arg Lys Asn Phe Thr
   45                  50                  55                  60
   tgt cta aca caa caa ttt ttt tat att tgg ggc gag caa agc agt ggt     1862
   Cys Leu Thr Gln Gln Phe Phe Tyr Ile Trp Gly Glu Gln Ser Ser Gly
                   65                  70                  75
   aaa agt cac ctc tta aaa ggc att act cat cat ttt ttc ctt tta cag     1910
   Lys Ser His Leu Leu Lys Gly Ile Thr His His Phe Phe Leu Leu Gln
               80                  85                  90
   cgc ccc gct atc tat gtg ccc tta gaa aaa tcc caa tat ttc tca ccg     1958
   Arg Pro Ala Ile Tyr Val Pro Leu Glu Lys Ser Gln Tyr Phe Ser Pro
           95                  100                 105
   gcg gta ctc gaa aac tta gaa caa caa caa ttg gtt tgt tta gat aat     2006
   Ala Val Leu Glu Asn Leu Glu Gln Gln Gln Leu Val Cys Leu Asp Asn
       110                 115                 120
   tta cag gca att ata ggc aat act gaa tgg gaa tta gcg att ttt gat     2054
   Leu Gln Ala Ile Ile Gly Asn Thr Glu Trp Glu Leu Ala Ile Phe Asp
   125                 130                 135                 140
   tta ttt aat cgc ata aaa tct gtt gaa aat aca ctg ctt gtg atc agt     2102
   Leu Phe Asn Arg Ile Lys Ser Val Glu Asn Thr Leu Leu Val Ile Ser
                   145                 150                 155
   gca aat caa tcc cca act gca tta cct gta agt tta cct gac tta gct     2150
   Ala Asn Gln Ser Pro Thr Ala Leu Pro Val Ser Leu Pro Asp Leu Ala
               160                 165                 170
   tca cgt tta cgc tgg gga gaa agc tat cag ctg gtc ccc tta aat gat     2198
   Ser Arg Leu Arg Trp Gly Glu Ser Tyr Gln Leu Val Pro Leu Asn Asp
           175                 180                 185
   caa caa aaa atc cat gta ttg caa aaa aat gca cat caa cgt ggt atc     2246
   Gln Gln Lys Ile His Val Leu Gln Lys Asn Ala His Gln Arg Gly Ile
       190                 195                 200
   gaa ctc ccc gat gaa gta gct aat ttt ctt ttg aaa cgc tta gag cgc      2294
   Glu Leu Pro Asp Glu Val Ala Asn Phe Leu Leu Lys Arg Leu Glu Arg
   205                 210                 215                 220
   gat atg aaa acg tta ttt gaa gca cta agt aaa tta gat aaa gca tca      2342
   Asp Met Lys Thr Leu Phe Glu Ala Leu Ser Lys Leu Asp Lys Ala Ser
               225                 230                 235
   tta caa gcc caa cgt aaa tta acg att ccc ttt gta aaa gaa att tta      2390
   Leu Gln Ala Gln Arg Lys Leu Thr Ile Pro Phe Val Lys Glu Ile Leu
           240                 245                 250
   aag cta taaaaaaaga ccacctcttt atcaggtgat ctttcttgtc ga               2438
   Lys Leu
<210> 10
   <211> 254
   <212> PRT
   <213> Pasteurella Multocida
<400> 10
   Leu Leu Asn Pro Ser Phe Phe Val Tyr Pro Tyr Ser Pro Phe Phe Asp
   1               5                   10                  15
   Phe Val Gly Cys Phe Leu Leu Glu Asn Phe Gln Leu Pro Leu Pro Ile
               20                  25                  30
   His Gln Leu Asp Asp Glu Thr Leu Asp Asn Phe Tyr Pro Asp Asn Asn
           35                  40                  45
   Leu Leu Leu Leu Asn Ser Leu Arg Lys Asn Phe Thr Cys Leu Thr Gln
       50                  55                  60
   Gln Phe Phe Tyr Ile Trp Gly Glu Gln Ser Ser Gly Lys Ser His Leu
   65                  70                  75                  80
   Leu Lys Gly Ile Thr His His Phe Phe Leu Leu Gln Arg Pro Ala Ile
                   85                  90                  95
   Tyr Val Pro Leu Glu Lys Ser Gln Tyr Phe Ser Pro Ala Val Leu Glu
               100                 105                 110
   Asn Leu Glu Gln Gln Gln Leu Val Cys Leu Asp Asn Leu Gln Ala Ile
           115                 120                 125
   Ile Gly Asn Thr Glu Trp Glu Leu Ala Ile Phe Asp Leu Phe Asn Arg
       130                 135                 140
   Ile Lys Ser Val Glu Asn Thr Leu Leu Val Ile Ser Ala Asn Gln Ser
   145                 150                 155                 160
   Pro Thr Ala Leu Pro Val Ser Leu Pro Asp Leu Ala Ser Arg Leu Arg
                   165                 170                 175
   Trp Gly Glu Ser Tyr Gln Leu Val Pro Leu Asn Asp Gln Gln Lys Ile
               180                 185                 190
   His Val Leu Gln Lys Asn Ala His Gln Arg Gly Ile Glu Leu Pro Asp
           195                 200                 205
   Glu Val Ala Asn Phe Leu Leu Lys Arg Leu Glu Arg Asp Met Lys Thr
       210                 215                 220
   Leu Phe Glu Ala Leu Ser Lys Leu Asp Lys Ala Ser Leu Gln Ala Gln
   225                 230                 235                 240
   Arg Lys Leu Thr Ile Pro Phe Val Lys Glu Ile Leu Lys Leu
                   245                 250
<210> 11
   <211> 2060
   <212> DNA
   <213> Pasteurella Multocida
<220>
   <221> misc_feature
   <223> dsbB
<220>
   <221> CDS
   <222> (856)..(1389)
<400> 11
   gaattcttct tacgtatgct cccagtcacg ttgccagttc tcatttgtgg tttagtgacc      60
   tgcttcttag tggaaaaatt tggtgtattt ggctatggcg ccaaattgcc acgtaaagta     120
   tggggcatct tggcaaagtt tgatcgcaat aatcaacaaa aaatgtcacg acaagatcgt     180
   ttgaaacttt ttgtgcaagc tttaattggt atttggttgg ttgttggact cgcattccat     240
   ctcgccgccg tcggtatcat tggtttaacg gtgattattt tggctacttc attttgtggt     300
   gtcaccagcg agcatgctat tggtaaagcc tttcaggaat ccttaccctt cacagcattg     360
   ttagtggtgt tcttctcggt tgttgccgtc atcattgacc aacatctgtt tgcgccaatt     420
   attcagtttg tgctggctgc cagtgaacat actcagcttg ctcttttcta tatttttaac     480
   ggtttgttat ccgccatttc agataatgtg tttgtggcca cagtttatat caatgaaacc     540
   aaagcggcat tagaggctgg cttaattgct caaccacaat atgaattact ggcagtagca     600
   attaataccg gtaccaatct tccttctgtt gcaaccccaa atggtcaagc cgcattctta     660
   tttttattga cctcatcact ggcaccatta attcgtcttt cttatggtag aatggtttat     720
   atggcattgc cttataccat cgtattatcc tgtattggtt tattgactgt ggaatatatt     780
   ttgcctggcg caaccaatgt gctcattcaa attggtttat taaaaccaat gtaatgacaa     840
   gtaaaaggag gaaac atg cta agc ttt ttt aag aca ctc tca aca aaa cga      891
                    Met Leu Ser Phe Phe Lys Thr Leu Ser Thr Lys Arg
                    1               5                   10
   agt gca tgg ttt cta ttg ttt agc tca gct tta cta tta gag gct atc      939
   Ser Ala Trp Phe Leu Leu Phe Ser Ser Ala Leu Leu Leu Glu Ala Ile
           15                  20                  25
   gct ctt tat ttt caa cat ggc atg ggg ctc gcc cct tgt gtc atg tgt      987
   Ala Leu Tyr Phe Gln His Gly Met Gly Leu Ala Pro Cys Val Met Cys
       30                  35                  40
   att tac gag agg gta gct att ctt ggc att gct ttc tcc ggt tta ttg     1035
   Ile Tyr Glu Arg Val Ala Ile Leu Gly Ile Ala Phe Ser Gly Leu Leu
   45                  50                  55                  60
   ggg tta ctc tac ccg agt tcg atg ctt ttg cgc ctt gtg gcg tta tta     1083
   Gly Leu Leu Tyr Pro Ser Ser Met Leu Leu Arg Leu Val Ala Leu Leu
                   65                  70                  75
   att ggt tta agc agt gca atc aaa ggc tta atg att agc atc acc cat     1131
   Ile Gly Leu Ser Ser Ala Ile Lys Gly Leu Met Ile Ser Ile Thr His
               80                  85                  90
   tta gat cta caa ctt tac cct gca cct tgg aaa caa tgt tca gcg gtg     1179
   Leu Asp Leu Gln Leu Tyr Pro Ala Pro Trp Lys Gln Cys Ser Ala Val
           95                  100                 105
   gca gaa ttt ccc gag act tta ccc tta gat cag tgg ttt cct gca ctc     1227
   Ala Glu Phe Pro Glu Thr Leu Pro Leu Asp Gln Trp Phe Pro Ala Leu
       110                 115                 120
   ttc ctc cct tca ggc tca tgc agt gaa gta aca tgg caa ttt ctc ggc     1275
   Phe Leu Pro Ser Gly Ser Cys Ser Glu Val Thr Trp Gln Phe Leu Gly
   125                 130                 135                 140
   ttt tct atg gtg caa tgg atc gtc gtc att ttt gca ctc tat acc tta     1323
   Phe Ser Met Val Gln Trp Ile Val Val Ile Phe Ala Leu Tyr Thr Leu
                   145                 150                 155
   tta ctt gct ctc att ttc atc agc caa gtc aaa cgt cta aaa ccc aag     1371
   Leu Leu Ala Leu Ile Phe Ile Ser Gln Val Lys Arg Leu Lys Pro Lys
               160                 165                 170
   cag cgc aga ctc ttt cat taagtcataa aaaatggtgc gataaagcac            1419
   Gln Arg Arg Leu Phe His
           175
   catttttcat ttctcgttcg gtatagatta aatttcttgc acgacaaact gcagggaatg  1479
   tcttgctaat aatcttgctt tacgctgctt taagcgtttt aaacgtaatg cgcgtccttg   1539
   tgataaacga tataattttt ttgtcggctt caaaaagctt ttacgtctcg ccatactctt   1599
   cctcttattg tgtcgttacg gttgtcgcta acgtatccgt ttctttcttg atttcgttga   1659
   tgttttctat ttcggcggct tgtgtatcta atgtttcttc aatttgtgct tgctgtaccc   1719
   gataataatg cacaatgctg ttcatataac gacggatatt ttccacatag tgatatgctt   1779
   catagcctcg cgcatacccg tattttaacc ccgtgtaata gcgtttttct gctaatagtg   1839
   gcaaattctt cttcacatct aaccaattat cgggatcacc acctaggctc ttggttaaac   1899
   ggcgcgcatc taacaaatgc cctaatccca tattatacgc cgctaaggca aaccaaatac   1959
   gctcatcttc tttaatcgta tcaggcattt gcgtaataag ccaatgtaaa tattctgaac   2019
   cggctttaat actttgttcc ggatccgttc tgtcttgaat t                        2060
<210> 12
   <211> 178
   <212> PRT
   <213> Pasteurella Multocida
<400> 12
   Met Leu Ser Phe Phe Lys Thr Leu Ser Thr Lys Arg Ser Ala Trp Phe
   1               5                   10                  15
   Leu Leu Phe Ser Ser Ala Leu Leu Leu Glu Ala Ile Ala Leu Tyr Phe
               20                  25                  30
   Gln His Gly Met Gly Leu Ala Pro Cys Val Met Cys Ile Tyr Glu Arg
           35                  40                  45
   Val Ala Ile Leu Gly Ile Ala Phe Ser Gly Leu Leu Gly Leu Leu Tyr
       50                  55                  60
   Pro Ser Ser Met Leu Leu Arg Leu Val Ala Leu Leu Ile Gly Leu Ser
   65                  70                  75                  80
   Ser Ala Ile Lys Gly Leu Met Ile Ser Ile Thr His Leu Asp Leu Gln
                   85                  90                  95
   Leu Tyr Pro Ala Pro Trp Lys Gln Cys Ser Ala Val Ala Glu Phe Pro
               100                 105                 110
   Glu Thr Leu Pro Leu Asp Gln Trp Phe Pro Ala Leu Phe Leu Pro Ser
           115                 120                 125
   Gly Ser Cys Ser Glu Val Thr Trp Gln Phe Leu Gly Phe Ser Met Val
       130                 135                 140
   Gln Trp Ile Val Val Ile Phe Ala Leu Tyr Thr Leu Leu Leu Ala Leu
   145 150 155 160
   Ile Phe Ile Ser Gln Val Lys Arg Leu Lys Pro Lys Gln Arg Arg Leu
   165 170 175
   Phe His
<210> 13
   <211> 4426
   <212> DNA
   <213> Pasteurella Multocida
<220>
   <221> misc_feature
   <223> exbB
<220>
   <221> CDS
   <222> (2756)..(3211)
<400> 13
   gaattcttga cctggtgtga ggcttttatt gacttccacc ccaattgcgc gttctttcac 60
   tttattaata aagtcacgta ccactggtaa cgccacatcc gcttctaata atgccatgcg 120
   gacttcacgt agcgtttcct taatattgtc atcagttaaa cgtccacgtc cactaatgtt 180
   acgtagcgtt tttgacaagc gatccgacaa attctcaaac atgcaatctt cctatttaat 240
   ctcgaaaaaa ttgccctaat tatactgaaa tcccatagat tttcatcctt tgtaataaat 300
   ttatagcgat ttcacagtga gaaagttaga atggacggag aacaaaggtg agggaatttc 360
   aatgtggctt tcaatttttt ccgtgttttt ttatctattg agtgtcttac tcattacccc 420
   tatgttgtta aaaattcaag cgggcgaacc cgcatcggtt cccaatagga tgcctttttt 480
   aacggcagca ttactcgcca ttcttctgca ctttatcaat cttattcctc tttttaccga 540
   tttaaccagc ggtcaaaatt tctcggttat cgaaatcagt tccttaatca gtgttatgac 600
   agcaacgatc gccacattag cgtttttatt tcgcatacac actttatggt ttttattacc 660
   gattatttat tctttcgcca tcattaatct ggtcctcagt accttaatgc cggcgcattt 720
   tttatatcat cttaatcaag atatgggctt atttattcat gtaggcttat cactgttgac 780
   ttattcggtc tgttcgattg tcgcgttata ttccattcag ttagtttgga tcgatcgcgc 840
   cttaaaaaat aaaaaactgc ctttttcacc aatgattcca ccgttaatga ccgtagagcg 900
   ccattttttt cgtttaatgc tgataggtga aatattactc acgattgtcc tcatttcagg 960
   gagctaccat ttatcgaaaa cctttgcccc acaagacatc cagaaagctg ttttttcttt   1020
   cttggcttgg atagtctttg ggagtgcact ggttggtcat tggaagctcc attggcgtgg   1080
   aaaaaaagtg gtgtattatg cgcttttagg tatcattctc ctgactatcg cctattttgg   1140
   tagtcgtgta atgcttgaaa tataaacaag atttatttat gctacgccat agtaaaagtg   1200
   cggttcaaaa aacgaaaaaa tgaccgcact tttcgatttt tgattaactc gcaaaaggat   1260
   gcattttgga cagtattccc cttagtaccc tctttattac actcatcatc ttattgatca   1320
   tctctgctta tttttcaggc tctgaaaccg gtttgttgtc tgccaatcgt tatcgactac   1380
   gccatttagc cgaaaaaggg catagaggtg cgaaaaaagc ggaaaaatta ctgaaaaaaa   1440
   cagatgtttt gcttagcctg attttaattt gtaataactt agtcaatatt actgcttctg   1500
   ccattaccac gattatcggc atgcgcttat atggcgatgc gggagtcgcc attgcaactg   1560
   gggcattaac ctttgtgatg cttatttttg ccgaaatttt gccgaaaact atcgccgccc   1620
   gttatccaga aaaagtggca ttcacgtcca gtcatttgtt gtccgttttt ctgcgacttt   1680
   ttaccccgct ggtctattta atgaatttaa ttattcaggg gattttggca ctattacgtc   1740
   taaaatcaga taataaatca acctcattaa gcccagagga attacgttcc atcgtaaatg   1800
   aatcaggtaa atttattcct tccgcccacc aagaaatgct gttatctatt ttggatttag   1860
   aaggggtcac cgtagatgac attatggtgc cacgtaatga cattgggggt attgatattg   1920
   acgatgattg gaaagccatt atgcgtcaac ttaaccatgc agcgcacggg cgtgttgtac   1980
   tgtataaagg aaatatggat gaaaatattt tggggatgtt acgtgtacgt gaagcctatc   2040
   gcttaatgct cgataaaaat gaatttaaca aagaaacttt aatccgtgcc gccgatgaag   2100
   tgtattttat tcctgaaggt acgccactga atagccaatt attaaatttc cgcaacaata   2160
   aagaaaggat tggtttagtt gtagatgaat atggtgatat taaaggctta gtcaccttag   2220
   aagatatctt agaagagatt gtcggtgaat ttaccacttc aacagcccca tcaattaacg   2280
   atgaagttat cccacaatca gacggttcgc ttatcattga gggatccgcc aatttacgtg   2340
   atttgaataa attatttgac tggaatctcg ataccgaaga tgcacgtacc ttcaacggct   2400
   taattttaga gcatttagaa gaaattccag aagaaggaac ggtatgtgaa attaatgggc   2460
   tacaaatcac gattctagaa gtgaatgaca acatgattaa acaagccaaa gtcattaaac   2520
   tttaattcaa catctggcta agcgatgtca tcaagacatc gcttttttat tccgtacatg   2580
   aatgtttgat ccaacacaac atttattcaa cattggataa ataatcatcc taaatcgcac   2640
   gaatttctta tttaccccgt tttggctttt tgctagaatc ttgcaattga aattaattct   2700
   caataccgta taatgttcaa cattattttg cgatacaaat taaaggatta ttaaa atg    2758
                                                                Met
                                                                1
   cca caa tta ttc caa ttc tta caa caa tat att gat tac att att cta      2806
   Pro Gln Leu Phe Gln Phe Leu Gln Gln Tyr Ile Asp Tyr Ile Ile Leu
                   5                   10                  15
   ggc tta ctt gcc ttc atg agc ttt att atg gtt tgg ctt gtg att gaa      2854
   Gly Leu Leu Ala Phe Met Ser Phe Ile Met Val Trp Leu Val Ile Glu
               20                  25                  30
   cgc ttt ctt ttc tta agt cgc gtc aac gtg gca tct tat gaa agc ata      2902
   Arg Phe Leu Phe Leu Ser Arg Val Asn Val Ala Ser Tyr Glu Ser Ile
           35                  40                  45
   cat gaa tta gac att gac tta caa cgc cac ctc aca gct atc tct aca      2950
   His Glu Leu Asp Ile Asp Leu Gln Arg His Leu Thr Ala Ile Ser Thr
   50                  55                  60                  65
   atc ggt tct aat gca cct tat gta ggt ttg ctt ggt acc gtc att ggt      2998
   Ile Gly Ser Asn Ala Pro Tyr Val Gly Leu Leu Gly Thr Val Ile Gly
                   70                  75                  80
   att ctc tta act ttc tat gaa tta ggt cac tcc ggt ggc gat att gat      3046
   Ile Leu Leu Thr Phe Tyr Glu Leu Gly His Ser Gly Gly Asp Ile Asp
                   85                  90                  95
   gcg gcg gcc att atg gtg cac tta tca tta gcc tta aaa gcc aca gca      3094
   Ala Ala Ala Ile Met Val His Leu Ser Leu Ala Leu Lys Ala Thr Ala
               100                 105                 110
   gta ggt att tta gtc gcc att cct gca atg gtg tgt tac aac ggt tta      3142
   Val Gly Ile Leu Val Ala Ile Pro Ala Met Val Cys Tyr Asn Gly Leu
           115                 120                 125
   gga cgt aaa gtc gaa gtt aat cgt ttg aaa tgg ttt gcc tta aat gag      3190
   Gly Arg Lys Val Glu Val Asn Arg Leu Lys Trp Phe Ala Leu Asn Glu
   130                 135                 140                 145
   aaa aaa gcc aaa caa caa gca tagggagccg tcatgaaaaa gtttgatgaa         3241
   Lys Lys Ala Lys Gln Gln Ala
                   150
   atcaacatta tcccttttat tgacatcatg ttggtactat tggctatcgt tctgattaca   3301
   gcctctttta tttcacaagg taaaatccaa gtgaatgtac caaaagcaag ttcaacagtt   3361
   gcgtttcgtt cagatgattt agccaaattg ctgactatta cggaaagcgg tgaaattttt   3421
   tatcacgata aaccgattac gatagaggca ttggaacaag aaatcagtaa ttgggaaaaa   3481
   gatcaaaaag tcaccttgaa ggtagatgca aaatccagtt tccaagattt cgtttctatc   3541
   actgatatta tggctaaaaa tgaaattaaa aatgtcgcta tcgtgacggt taaagaaaag   3601
   gcatctcaat gatagataaa agtcgttctt gcatcgggtt tgcaatttca ttgctttttc    3661
   acgcaagttt tgtctctttc ctgtattgga ttgtacaaaa agacgatgac agcgcgaatg   3721
   gatttgctgc cgatatcatc tcaactcata tttccatgga aatgctggcg gctaccgttt   3781
   tagaagaacc agagccggaa ccagagccgg cgcctccggt agtagaacct gaactgccaa   3841
   aagaagtagt cgcagatccg acggtaaaac ctgagccacc aaaagaaccc gaaaaaccaa 3901
   aagagcctga aaagccaaaa gagaaaccga aagaaaaacc aaaagaaaag ccgaaaaaac 3961
   cgaagaaaga acaacgtgat ttaccaaagt cagatcgcca aattgattct aattcatcga 4021
   tcaatcaaca agcgaccaca acaggcaaca tcacaaccaa taatccgaat ctggtcggta 4081
   aaggtaatag cacagatgaa gtcaatgctt atcgctcggc tttacgcaga gaaattgaaa 4141
   aacataaacg ctatccaaac cgtgcacgca tgatgcgcaa acaaggtgtg gtaacaatca 4201
   cgttccatct taataatgcc ggcgtaatta gtaatgcgcg aatcagcaaa tcttccggct 4261
   cagaagaatt agataacgct gcactggtag ctgtcaataa tgcccgtcca attggtccac 4321
   tgcctgttgg tatgccaaat gaagtgagcg ttcctgtcag tttcagaatc acaaattaaa 4381
   aaagtgcggt aaaatttacc gcactttttt ctctctatta gaatt 4426
<210> 14
   <211> 152
   <212> PRT
   <213> Pasteurella Multocida
<400> 14
   Met Pro Gln Leu Phe Gln Phe Leu Gln Gln Tyr Ile Asp Tyr Ile Ile
   1 5 10 15
   Leu Gly Leu Leu Ala Phe Met Ser Phe Ile Met Val Trp Leu Val Ile
   20 25 30
   Glu Arg Phe Leu Phe Leu Ser Arg Val Asn Val Ala Ser Tyr Glu Ser
   35 40 45
   Ile His Glu Leu Asp Ile Asp Leu Gln Arg His Leu Thr Ala Ile Ser
   50 55 60
   Thr Ile Gly Ser Asn Ala Pro Tyr Val Gly Leu Leu Gly Thr Val Ile
   65 70 75 80
   Gly Ile Leu Leu Thr Phe Tyr Glu Leu Gly His Ser Gly Gly Asp Ile
   85 90 95
   Asp Ala Ala Ala Ile Met Val His Leu Ser Leu Ala Leu Lys Ala Thr
   100 105 110
   Ala Val Gly Ile Leu Val Ala Ile Pro Ala Met Val Cys Tyr Asn Gly
   115 120 125
   Leu Gly Arg Lys Val Glu Val Asn Arg Leu Lys Trp Phe Ala Leu Asn
                   130                 135                 140
   Glu Lys Lys Ala Lys Gln Gln Ala
   145                 150
<210> 15
   <211> 6876
   <212> DNA
   <213> Pasteurella Multocida
<220>
   <221> CDS
   <222> (534)..(6863)
   <223> fhaB1
<400> 15
   agatgcgtga tctgatcctt caactcagca aaagttcgat ttattcaaca aagccgccgt      60
   cccgtcaagt cagcgtaatg tctgccagtg ttacaccaat taaccaattc tgattagaaa     120
   aactcatcga gcatcaaatg aaactgcaat ttattcatat caggattatc aataccatat     180
   ttttgaaaaa gccgtttctg taatgaagga gaaaactcac cgaggcagtt ccataggatg     240
   gcaagatcct ggtatcggtc tgcgattccg actcgtccaa catcaataca acctattaat     300
   ttcccctcgt caaaaataag gttatcaagt gagaaatcac catgagtgac gactgaatcc     360
   ggtgagaatg gcaaaagctt atgcatttct ttccagactt gttcaacagg ccagccatta     420
   cgctcgtcat caaaatcact cgcatcaacc aaaccgttat tcattcgtga ttgcgcctga     480
   gcgagacgaa atacgcgatc gctgttaaaa ggacaattac aaacaggaat cga atg       536
                                                              Met
                                                              1
   caa ccg gcg cag gaa cac tgc cag cgc atc aac aat att gtt aac caa       584
   Gln Pro Ala Gln Glu His Cys Gln Arg Ile Asn Asn Ile Val Asn Gln
               5                   10                  15
   gaa aac ggt tta ttc cat aca ctc ggt aat atg atg tta gaa gca gag       632
   Glu Asn Gly Leu Phe His Thr Leu Gly Asn Met Met Leu Glu Ala Glu
           20                  25                  30
   cgt tct gtt tat aat att ggc gat att tat gcg agt aaa aaa tta aca       680
   Arg Ser Val Tyr Asn Ile Gly Asp Ile Tyr Ala Ser Lys Lys Leu Thr
       35                  40                  45
   gtt cat act cat aat ttg att aat gat gtg cgt tta tct ggc aat gtg       728
   Val His Thr His Asn Leu Ile Asn Asp Val Arg Leu Ser Gly Asn Val
   50                  55                  60                  65
   agt tat aag cct atc ggt tca agt cgt gat tat gat atc agt cgt gtt       776
   Ser Tyr Lys Pro Ile Gly Ser Ser Arg Asp Tyr Asp Ile Ser Arg Val
                   70                  75                  80
   gcg gta cat ggt tgg cac aat aat gtt tat aag ctc aac tta aat ctg       824
   Ala Val His Gly Trp His Asn Asn Val Tyr Lys Leu Asn Leu Asn Leu
               85                  90                  95
   caa gaa caa gat aaa acc gat att aaa gtt gtg aaa atg ggg gct atc      872
   Gln Glu Gln Asp Lys Thr Asp Ile Lys Val Val Lys Met Gly Ala Ile
           100                 105                 110
   cgt tct gat ggt gat ttt gac ttt aag gga ata aag gcg aca tca tca      920
   Arg Ser Asp Gly Asp Phe Asp Phe Lys Gly Ile Lys Ala Thr Ser Ser
       115                 120                 125
   gaa tca aaa ccg cag tta att aat cat gga tta att aat gtc aaa gga      968
   Glu Ser Lys Pro Gln Leu Ile Asn His Gly Leu Ile Asn Val Lys Gly
   130                 135                 140                 145
   aca ttt aat gcg gaa gct gat caa gtg gtg aac caa atg aaa gcg ttt      1016
   Thr Phe Asn Ala Glu Ala Asp Gln Val Val Asn Gln Met Lys Ala Phe
                   150                 155                 160
   aac caa aat gca tta gca agc gtg ttt aag aat cca gcg aaa atc acg      1064
   Asn Gln Asn Ala Leu Ala Ser Val Phe Lys Asn Pro Ala Lys Ile Thr
               165                 170                 175
   atg tac tat caa cca ctt act cgt tat att tgg aca cca tta tcg ggt      1112
   Met Tyr Tyr Gln Pro Leu Thr Arg Tyr Ile Trp Thr Pro Leu Ser Gly
           180                 185                 190
   aat gca tcg cgt gaa ttt aac aat tta gag tct ttc ctc gat gcc ttg      1160
   Asn Ala Ser Arg Glu Phe Asn Asn Leu Glu Ser Phe Leu Asp Ala Leu
       195                 200                 205
   ttt ggc tca aca aca atc tta aaa tca agt ttc tat agt acg gaa aat      1208
   Phe Gly Ser Thr Thr Ile Leu Lys Ser Ser Phe Tyr Ser Thr Glu Asn
   210                 215                 220                 225
   ttt agt gct tat cag ctt cta tct cat att cag cat tca cca atg tac      1256
   Phe Ser Ala Tyr Gln Leu Leu Ser His Ile Gln His Ser Pro Met Tyr
                   230                 235                 240
   caa aaa gcg atg gca caa gtg ttt ggt gca gag tgg cat agt aaa tcc      1304
   Gln Lys Ala Met Ala Gln Val Phe Gly Ala Glu Trp His Ser Lys Ser
               245                 250                 255
   tat gat gag atg cga aac aaa tgg aaa agc ttt aaa gaa aat cca aca      1352
   Tyr Asp Glu Met Arg Asn Lys Trp Lys Ser Phe Lys Glu Asn Pro Thr
           260                 265                 270
   gat ttc att tat tac cca tca gaa aaa gca aaa atc cta gcg gga aaa      1400
   Asp Phe Ile Tyr Tyr Pro Ser Glu Lys Ala Lys Ile Leu Ala Gly Lys
       275                 280                 285
   cta gaa ggt aag ctt aca acg cta caa aat ggt gaa tat gcc gaa cgt      1448
   Leu Glu Gly Lys Leu Thr Thr Leu Gln Asn Gly Glu Tyr Ala Glu Arg
   290                 295                 300                 305
   ggt aag ttt gat gag agt atc caa att ggt aaa cac caa tta tcg cta      1496
   Gly Lys Phe Asp Glu Ser Ile Gln Ile Gly Lys His Gln Leu Ser Leu
                   310                 315                 320
   cca tca gta gag ctt aaa gcg gag ttt agt gat aaa gaa cgt ttg gaa      1544
   Pro Ser Val Glu Leu Lys Ala Glu Phe Ser Asp Lys Glu Arg Leu Glu
               325                 330                 335
   gag gac ggg gta gat tta tcc tcg atc gcc gaa ctc tta gaa atg cca      1592
   Glu Asp Gly Val Asp Leu Ser Ser Ile Ala Glu Leu Leu Glu Met Pro
           340                 345                 350
   aac tta ttt att gat aat agt atc caa tta gaa aag aaa aag ttg tct      1640
   Asn Leu Phe Ile Asp Asn Ser Ile Gln Leu Glu Lys Lys Lys Leu Ser
       355                 360                 365
   cct att gag gat cta gat gaa gaa cca cgt aaa aat ctg gat ata gaa      1688
   Pro Ile Glu Asp Leu Asp Glu Glu Pro Arg Lys Asn Leu Asp Ile Glu
   370                 375                 380                 385
   gaa agc cat tct aat tca tcg gat gac gtg ctt agc atg aat gat gat      1736
   Glu Ser His Ser Asn Ser Ser Asp Asp Val Leu Ser Met Asn Asp Asp
                   390                 395                 400
   gag tct gat aca gac gat agc aag tgg agt atg ggc aat gat gag aaa      1784
   Glu Ser Asp Thr Asp Asp Ser Lys Trp Ser Met Gly Asn Asp Glu Lys
               405                 410                 415
   gag atg ccc gat gat aag ctg ggt ata agt cgt gat gat cgt gga aat      1832
   Glu Met Pro Asp Asp Lys Leu Gly Ile Ser Arg Asp Asp Arg Gly Asn
           420                 425                 430
   aaa cca cct cgt act gat cct aca gtt gat tat ctt aac cct gat gaa      1880
   Lys Pro Pro Arg Thr Asp Pro Thr Val Asp Tyr Leu Asn Pro Asp Glu
       435                 440                 445
   ttc ttt gaa aat ggt tat ctc ttg aat gag cta cta cag gag ctt gga      1928
   Phe Phe Glu Asn Gly Tyr Leu Leu Asn Glu Leu Leu Gln Glu Leu Gly
   450                 455                 460                 465
   gaa gag ccg tta cta aaa gaa ggg gaa gat cat ttt aaa cgt tct acc      1976
   Glu Glu Pro Leu Leu Lys Glu Gly Glu Asp His Phe Lys Arg Ser Thr
                   470                 475                 480
   aat cta gtc cgt cta ggc gag aga gat agg caa aat aga gaa aag aga      2024
   Asn Leu Val Arg Leu Gly Glu Arg Asp Arg Gln Asn Arg Glu Lys Arg
               485                 490                 495
   gaa aaa gag ggg tat ttt gat ctg cct ggt aca tta gat atg aaa ctg      2072
   Glu Lys Glu Gly Tyr Phe Asp Leu Pro Gly Thr Leu Asp Met Lys Leu
           500                 505                 510
   cag gag tta ttc gaa aaa aga aaa caa aaa cac gaa gca gaa cag aaa      2120
   Gln Glu Leu Phe Glu Lys Arg Lys Gln Lys His Glu Ala Glu Gln Lys
       515                 520                 525
   gca aga ata gaa aaa gca ctt cta caa aaa tca gaa caa caa gaa aaa      2168
   Ala Arg Ile Glu Lys Ala Leu Leu Gln Lys Ser Glu Gln Gln Glu Lys
   530                 535                 540                 545
   cgt gtt gaa gaa cgt aag caa gag gaa aaa cgt caa gcg caa gat aaa      2216
   Arg Val Glu Glu Arg Lys Gln Glu Glu Lys Arg Gln Ala Gln Asp Lys
                   550                 555                 560
   att gct aag caa gta gaa att gca aaa gaa atg caa cgg gta gaa gaa      2264
   Ile Ala Lys Gln Val Glu Ile Ala Lys Glu Met Gln Arg Val Glu Glu
               565                 570                 575
   att cgc cag aga gaa aaa caa ctt gcg atc caa ctg caa gaa gaa gag     2312
   Ile Arg Gln Arg Glu Lys Gln Leu Ala Ile Gln Leu Gln Glu Glu Glu
           580                 585                 590
   aag aaa caa caa gaa gaa aaa cat tta tcc gag gag aaa aaa caa gct     2360
   Lys Lys Gln Gln Glu Glu Lys His Leu Ser Glu Glu Lys Lys Gln Ala
       595                 600                 605
   gaa cag aaa caa aaa gct gag gag aaa gtt gca caa gaa aga tta gac     2408
   Glu Gln Lys Gln Lys Ala Glu Glu Lys Val Ala Gln Glu Arg Leu Asp
   610                 615                 620                 625
   att gaa caa cag aaa gcg tat gaa gaa atg gcg aag cga gag gca gag     2456
   Ile Glu Gln Gln Lys Ala Tyr Glu Glu Met Ala Lys Arg Glu Ala Glu
                   630                 635                 640
   gca tca aaa aat gtt tta ttg aaa gcg att gat gaa gaa cgt cca aaa     2504
   Ala Ser Lys Asn Val Leu Leu Lys Ala Ile Asp Glu Glu Arg Pro Lys
               645                 650                 655
   gtg gaa act gat cca ctt ttc cgt aca aaa ttg aaa tat atc aat caa     2552
   Val Glu Thr Asp Pro Leu Phe Arg Thr Lys Leu Lys Tyr Ile Asn Gln
           660                 665                 670
   gat gac tat gct ggt gca aat tat ttc ttc aat aaa gtt ggt tta aat     2600
   Asp Asp Tyr Ala Gly Ala Asn Tyr Phe Phe Asn Lys Val Gly Leu Asn
       675                 680                 685
   aca aaa ggt cat caa aaa gta aat gtg tta ggg gat aac tat ttt gat     2648
   Thr Lys Gly His Gln Lys Val Asn Val Leu Gly Asp Asn Tyr Phe Asp
   690                 695                 700                 705
   cat caa gtg att act cgc tcg att gag aaa aaa gta gat aac cac ctt     2696
   His Gln Val Ile Thr Arg Ser Ile Glu Lys Lys Val Asp Asn His Leu
                   710                 715                 720
   aac caa aaa tac aat ctc agc gat gtg gaa tta gtt aaa cag ctg atg     2744
   Asn Gln Lys Tyr Asn Leu Ser Asp Val Glu Leu Val Lys Gln Leu Met
               725                 730                 735
   gac aat tcc aca aca caa gcg cag gag ttg gat ttg aaa cta ggt gcg     2792
   Asp Asn Ser Thr Thr Gln Ala Gln Glu Leu Asp Leu Lys Leu Gly Ala
           740                 745                 750
   gca tta act aaa gaa caa caa gct aac ttg acc caa gat atc gtt tgg     2840
   Ala Leu Thr Lys Glu Gln Gln Ala Asn Leu Thr Gln Asp Ile Val Trp
       755                 760                 765
   tat gtc aaa acg aag gta aag ggc aaa gat gtg ttt gtt cca aag gtt     2888
   Tyr Val Lys Thr Lys Val Lys Gly Lys Asp Val Phe Val Pro Lys Val
   770                 775                 780                 785
   tat ttc gct tct gaa acg ctc gta gaa gcc caa aaa tta caa ggt tta     2936
   Tyr Phe Ala Ser Glu Thr Leu Val Glu Ala Gln Lys Leu Gln Gly Leu
                   790                 795                 800
   ggc act ggg act atc aga gtt ggt gaa gct aag att aaa gcc aaa gat     2984
   Gly Thr Gly Thr Ile Arg Val Gly Glu Ala Lys Ile Lys Ala Lys Asp
               805                 810                 815
   gtg gtg aat acc ggg aca tta gct ggg aga aaa ctc aat gtt gaa gcg     3032
   Val Val Asn Thr Gly Thr Leu Ala Gly Arg Lys Leu Asn Val Glu Ala
           820                 825                 830
   agt aat aaa atc aaa aat caa ggg agt atc tta agt act caa gaa aca      3080
   Ser Asn Lys Ile Lys Asn Gln Gly Ser Ile Leu Ser Thr Gln Glu Thr
       835                 840                 845
   cgt tta gtc ggg cgt aaa ggt att gaa aac gta tct cgt tca ttt gca      3128
   Arg Leu Val Gly Arg Lys Gly Ile Glu Asn Val Ser Arg Ser Phe Ala
   850                 855                 860                 865
   aat gat gaa tta gga gtc act gca caa cgc tca gaa atc aaa acg gaa      3176
   Asn Asp Glu Leu Gly Val Thr Ala Gln Arg Ser Glu Ile Lys Thr Glu
                   870                 875                 880
   ggt cat tta cat ctt gaa aca gat aag gat tca act att gat gta caa      3224
   Gly His Leu His Leu Glu Thr Asp Lys Asp Ser Thr Ile Asp Val Gln
               885                 890                 895
   gca tcg gat att aaa gca aaa aca agc ttt gtg aag act ggt gat gtg      3272
   Ala Ser Asp Ile Lys Ala Lys Thr Ser Phe Val Lys Thr Gly Asp Val
           900                 905                 910
   aat ctc aaa aat aca tac aat act aaa cat gcc tac cgt gag aaa ttc      3320
   Asn Leu Lys Asn Thr Tyr Asn Thr Lys His Ala Tyr Arg Glu Lys Phe
       915                 920                 925
   tcg ccg agt gca cta caa gtt gca gaa ctt gat gtg gca ggg ctt aaa      3368
   Ser Pro Ser Ala Leu Gln Val Ala Glu Leu Asp Val Ala Gly Leu Lys
   930                 935                 940                 945
   gtc cca ctt tta ggc gtg tcg tct cca tcc agt tat tca gag cat act      3416
   Val Pro Leu Leu Gly Val Ser Ser Pro Ser Ser Tyr Ser Glu His Thr
                   950                 955                 960
   agt gag gca act tca gag gga tca atc ttc gaa gta ggg cac tta cat      3464
   Ser Glu Ala Thr Ser Glu Gly Ser Ile Phe Glu Val Gly His Leu His
               965                 970                 975
   ctt gcg gta gac aga gat gtg aac caa gcg ggg agt aaa att aag gct      3512
   Leu Ala Val Asp Arg Asp Val Asn Gln Ala Gly Ser Lys Ile Lys Ala
           980                 985                 990
   aag tat acc act ggt gtt gtg  aaa ggg aac ttt aat  act gaa gcg ggc    3560
   Lys Tyr Thr Thr Gly Val Val  Lys Gly Asn Phe Asn  Thr Glu Ala Gly
       995                 1000                1005
   aag  aat att aaa cat gtc  gaa aaa gaa gaa tat  agt tca cag cta      3605
   Lys  Asn Ile Lys His Val  Glu Lys Glu Glu Tyr  Ser Ser Gln Leu
   1010                1015                1020
   ttt  gct tca gca cac gcg  agt ggt ggt ggc acc  tca gtt cgt tat      3650
   Phe  Ala Ser Ala His Ala  Ser Gly Gly Gly Thr  Ser Val Arg Tyr
   1025                1030                1035
   gac  tat aac agc caa gat  ggt ggc aat gcc tct  gtt ggt gtt ccg      3695
   Asp  Tyr Asn Ser Gln Asp  Gly Gly Asn Ala Ser  Val Gly Val Pro
   1040                1045                1050
   aca  aac cat act gga gtt  ggg gca gag gca gga  atg tca ttc acc      3740
   Thr  Asn His Thr Gly Val  Gly Ala Glu Ala Gly  Met Ser Phe Thr
   1055                1060                1065
   cat acc aaa gac aaa gaa aca ctg ctc act cac acc aat agt gaa      3785
   His Thr Lys Asp Lys Glu Thr Leu Leu Thr His Thr Asn Ser Glu
   1070                1075                1080
   tta caa gtc aaa cat ggg aaa tta cat gtg ctt ggt tat gcc gat      3830
   Leu Gln Val Lys His Gly Lys Leu His Val Leu Gly Tyr Ala Asp
   1085                1090                1095
   att ggt gga gta gat att aat act aaa cta cca gaa gat gca caa      3875
   Ile Gly Gly Val Asp Ile Asn Thr Lys Leu Pro Glu Asp Ala Gln
   1100                1105                1110
   agc aaa gca cag aaa gag ata gcg gct agc aag cca gag aag acc      3920
   Ser Lys Ala Gln Lys Glu Ile Ala Ala Ser Lys Pro Glu Lys Thr
   1115                1120                1125
   gag caa tct gca cag gat gtg gct caa gct caa tca aat gcc aat      3965
   Glu Gln Ser Ala Gln Asp Val Ala Gln Ala Gln Ser Asn Ala Asn
   1130                1135                1140
   aag gat aag gaa aat aaa gcc cca gaa ata aaa gaa tta tca gag      4010
   Lys Asp Lys Glu Asn Lys Ala Pro Glu Ile Lys Glu Leu Ser Glu
   1145                1150                1155
   gct gaa atc gcg gat ctc atg tca gaa aaa tca aaa gcg tat ttt      4055
   Ala Glu Ile Ala Asp Leu Met Ser Glu Lys Ser Lys Ala Tyr Phe
   1160                1165                1170
   gat gat ttt gca gag caa gcg aaa aaa gca cct gaa aac aat cga      4100
   Asp Asp Phe Ala Glu Gln Ala Lys Lys Ala Pro Glu Asn Asn Arg
   1175                1180                1185
   ttt gaa ttg tct gcg aaa gaa att aag tcg agc aaa caa aaa gac      4145
   Phe Glu Leu Ser Ala Lys Glu Ile Lys Ser Ser Lys Gln Lys Asp
   1190                1195                1200
   caa tat gat cat gag tct gaa cgg acg act ttt aaa gtt gga cct      4190
   Gln Tyr Asp His Glu Ser Glu Arg Thr Thr Phe Lys Val Gly Pro
   1205                1210                1215
   gaa gcg gag gct cat tct gcg gtt gcc gat atg gtg agc cat tta      4235
   Glu Ala Glu Ala His Ser Ala Val Ala Asp Met Val Ser His Leu
   1220                1225                1230
   gtg aaa gaa tat aga gat gca caa aat ggg act aaa caa gac ggt      4280
   Val Lys Glu Tyr Arg Asp Ala Gln Asn Gly Thr Lys Gln Asp Gly
   1235                1240                1245
   aca gta gca tta caa cat gct agt gat gtc tta aat att gtg acg      4325
   Thr Val Ala Leu Gln His Ala Ser Asp Val Leu Asn Ile Val Thr
   1250                1255                1260
   ggg gat tta gcg ggg agt tca gct aaa ttg tct gtt gaa aga aca      4370
   Gly Asp Leu Ala Gly Ser Ser Ala Lys Leu Ser Val Glu Arg Thr
   1265                1270                1275
   cat gag aca aaa cga acg aca gaa acg ggg gat att gtt act aag      4415
   His Glu Thr Lys Arg Thr Thr Glu Thr Gly Asp Ile Val Thr Lys
   1280                1285                1290
   att  ggt ggc aat gtc aca  ctg tca gca cgc agt  ggt agt gtg aac        4460
   Ile  Gly Gly Asn Val Thr  Leu Ser Ala Arg Ser  Gly Ser Val Asn
   1295                 1300                 1305
   ctt  aaa aat gta caa agt  gat gaa caa gct aat  ttg acc tta aga        4505
   Leu  Lys Asn Val Gln Ser  Asp Glu Gln Ala Asn  Leu Thr Leu Arg
   1310                 1315                 1320
   gca  aaa gaa gat gtg aat  gtg ctg tct ggt gaa  aaa aca cga gaa        4550
   Ala  Lys Glu Asp Val Asn  Val Leu Ser Gly Glu  Lys Thr Arg Glu
   1325                 1330                 1335
   acc  aca gaa aca gta tca  aga cag aaa ctt tct  cat ggt gtg aac        4595
   Thr  Thr Glu Thr Val Ser  Arg Gln Lys Leu Ser  His Gly Val Asn
   1340                 1345                 1350
   gca  ggt tgc agt atg atg  agt ggc gcc tgt act  gcc ggt gtt agc        4640
   Ala  Gly Cys Ser Met Met  Ser Gly Ala Cys Thr  Ala Gly Val Ser
   1355                 1360                 1365
   aca  tca ctt gaa gga aat  gaa agc tat acg tca  gaa cgt gaa acg        4685
   Thr  Ser Leu Glu Gly Asn  Glu Ser Tyr Thr Ser  Glu Arg Glu Thr
   1370                 1375                 1380
   gct  caa aat aac agt ttc  tta aaa gca cgc aac  atg aaa gtt gaa        4730
   Ala  Gln Asn Asn Ser Phe  Leu Lys Ala Arg Asn  Met Lys Val Glu
   1385                 1390                 1395
   gca  ggt cgc gat ttt aat  gtt gtc agt tcg aat  att gat gca gat        4775
   Ala  Gly Arg Asp Phe Asn  Val Val Ser Ser Asn  Ile Asp Ala Asp
   1400                 1405                 1410
   aag  ctc gat ctc cac gtt  aaa gga aaa acg aat  gtg gta tcc aaa        4820
   Lys  Leu Asp Leu His Val  Lys Gly Lys Thr Asn  Val Val Ser Lys
   1415                 1420                 1425
   caa  gat acg tta caa aaa  gtg acg cat gga gtt  gac tat aat ctt        4865
   Gln  Asp Thr Leu Gln Lys  Val Thr His Gly Val  Asp Tyr Asn Leu
   1430                 1435                 1440
   tcc  gct ggc gtt gca ctt  tct agt gca aca att  gct acc cca acc        4910
   Ser  Ala Gly Val Ala Leu  Ser Ser Ala Thr Ile  Ala Thr Pro Thr
   1445                 1450                 1455
   ggt  aat gtt ggt ttc ggt  tat acc aat gag acc  gaa agc aag cgg        4955
   Gly  Asn Val Gly Phe Gly  Tyr Thr Asn Glu Thr  Glu Ser Lys Arg
   1460                 1465                 1470
   acg  gtt aat caa caa gca  ggg att aaa gcg aat  aaa att aca ggg        5000
   Thr  Val Asn Gln Gln Ala  Gly Ile Lys Ala Asn  Lys Ile Thr Gly
   1475                 1480                 1485
   caa  acg cat gac tta aat  ctt gag ggg gga tat  ctt gtc agc aac        5045
   Gln  Thr His Asp Leu Asn  Leu Glu Gly Gly Tyr  Leu Val Ser Asn
   1490                 1495                 1500
   gat  aag gat aat cag ctg  aaa gtt acc ggc gat  gtc aca act aaa        5090
   Asp  Lys Asp Asn Gln Leu  Lys Val Thr Gly Asp  Val Thr Thr Lys
   1505                 1510                 1515
   gcc ctt cac gat caa cat gat aaa gat ggt gga aca ttt ggt tta      5135
   Ala Leu His Asp Gln His Asp Lys Asp Gly Gly Thr Phe Gly Leu
   1520                1525                1530
   tct gtg ggt atc agt gaa cgt ggt act acc gcc ttt aat gta cga      5180
   Ser Val Gly Ile Ser Glu Arg Gly Thr Thr Ala Phe Asn Val Arg
   1535                1540                1545
   ggt ggg cga gct gaa cag aaa cac tat aat gca acg cag aaa tcc      5225
   Gly Gly Arg Ala Glu Gln Lys His Tyr Asn Ala Thr Gln Lys Ser
   1550                1555                1560
   act ctt tct ggc gtg gat acc tct caa gcg aat gta tca ggt caa      5270
   Thr Leu Ser Gly Val Asp Thr Ser Gln Ala Asn Val Ser Gly Gln
   1565                1570                1575
   gtg aat aca gat tta acc aag gca aaa gct gtc aca cgt gat gat      5315
   Val Asn Thr Asp Leu Thr Lys Ala Lys Ala Val Thr Arg Asp Asp
   1580                1585                1590
   act tac gca agt acg caa ttt agt ttt gaa gtg gca gat att gtg      5360
   Thr Tyr Ala Ser Thr Gln Phe Ser Phe Glu Val Ala Asp Ile Val
   1595                1600                1605
   gaa tta gga cag aga gcg aaa aac aag ctg tca gca cca aac aat      5405
   Glu Leu Gly Gln Arg Ala Lys Asn Lys Leu Ser Ala Pro Asn Asn
   1610                1615                1620
   gac acc gat atg gcg tca ggc tcc aca tta cgc tcg cgt tct act      5450
   Asp Thr Asp Met Ala Ser Gly Ser Thr Leu Arg Ser Arg Ser Thr
   1625                1630                1635
   aca gaa gaa gca gat gta cca aca aca aga tcg cgt gta acg gat      5495
   Thr Glu Glu Ala Asp Val Pro Thr Thr Arg Ser Arg Val Thr Asp
   1640                1645                1650
   gag gcg gat tct gta tcc gtg aaa aat ccg att tat gaa agt gca      5540
   Glu Ala Asp Ser Val Ser Val Lys Asn Pro Ile Tyr Glu Ser Ala
   1655                1660                1665
   gat gct gtt gta cca aca cca cgt agt aga aat gtg gac agt acc      5585
   Asp Ala Val Val Pro Thr Pro Arg Ser Arg Asn Val Asp Ser Thr
   1670                1675                1680
   gat ctt gtg gac aat cca ctg tat gct agt gcc act aca aaa gca      5630
   Asp Leu Val Asp Asn Pro Leu Tyr Ala Ser Ala Thr Thr Lys Ala
   1685                1690                1695
   aac atc cat gat tat gaa gaa att cca gcc gtt tat agc aaa gtc      5675
   Asn Ile His Asp Tyr Glu Glu Ile Pro Ala Val Tyr Ser Lys Val
   1700                1705                1710
   ggt gat aac aat gct gat ctt gtt cgt cat aaa acg gca act agt      5720
   Gly Asp Asn Asn Ala Asp Leu Val Arg His Lys Thr Ala Thr Ser
   1715                1720                1725
   gat gag cat ctt tat gca gag att aat gaa cca aca tat agc cgt      5765
   Asp Glu His Leu Tyr Ala Glu Ile Asn Glu Pro Thr Tyr Ser Arg
   1730                1735                1740
   gtt ggt gat aaa aat gca gat atg aga cgt cat aac gcg gca ggt      5810
   Val Gly Asp Lys Asn Ala Asp Met Arg Arg His Asn Ala Ala Gly
   1745                1750                1755
   aca aca gac tat gcc gat gtc gtg caa gca cat aca aga aag gca      5855
   Thr Thr Asp Tyr Ala Asp Val Val Gln Ala His Thr Arg Lys Ala
   1760                1765                1770
   gat gat cca cta cca gca tta ccg aat cag ggt aaa gca aga acg      5900
   Asp Asp Pro Leu Pro Ala Leu Pro Asn Gln Gly Lys Ala Arg Thr
   1775                1780                1785
   gta aac gac ggt tca gag cat att tat act gat att agc gac gtg      5945
   Val Asn Asp Gly Ser Glu His Ile Tyr Thr Asp Ile Ser Asp Val
   1790                1795                1800
   ggc act caa act aaa gct att gat agt act tat gca aca gta ggc      5990
   Gly Thr Gln Thr Lys Ala Ile Asp Ser Thr Tyr Ala Thr Val Gly
   1805                1810                1815
   atg ccg aaa gcg aat gcc gtt aac ttg ata ggg caa aat ggc tta      6035
   Met Pro Lys Ala Asn Ala Val Asn Leu Ile Gly Gln Asn Gly Leu
   1820                1825                1830
   ggc agc att tat cac agc cca gac agt gct tat aaa aca tgg caa      6080
   Gly Ser Ile Tyr His Ser Pro Asp Ser Ala Tyr Lys Thr Trp Gln
   1835                1840                1845
   ttg ctt gat caa ttt gcc aac aaa ggc gga gat gcg gtc ttc tta      6125
   Leu Leu Asp Gln Phe Ala Asn Lys Gly Gly Asp Ala Val Phe Leu
   1850                1855                1860
   cgc cct gca aca gaa atg aaa tgt gca ggt gca cct tta aaa tat      6170
   Arg Pro Ala Thr Glu Met Lys Cys Ala Gly Ala Pro Leu Lys Tyr
   1865                1870                1875
   acc ttt atc gtg cgt gat tat ttg ctc aga cgc cat acc tta gat      6215
   Thr Phe Ile Val Arg Asp Tyr Leu Leu Arg Arg His Thr Leu Asp
   1880                1885                1890
   aaa tca aga tta ttt tat aac gca cat aat aaa acc tta ttt agc      6260
   Lys Ser Arg Leu Phe Tyr Asn Ala His Asn Lys Thr Leu Phe Ser
   1895                1900                1905
   gtg cct atc gtt gat gca aaa gtc aaa atg ctg ttt gct gaa aaa      6305
   Val Pro Ile Val Asp Ala Lys Val Lys Met Leu Phe Ala Glu Lys
   1910                1915                1920
   aat atc caa gtc aat tac gat cgt agc ctt aca gcc att gat ctg      6350
   Asn Ile Gln Val Asn Tyr Asp Arg Ser Leu Thr Ala Ile Asp Leu
   1925                1930                1935
   agt aaa cgt att gcg acc ttt aat agc cca gaa gga gtt gta gaa      6395
   Ser Lys Arg Ile Ala Thr Phe Asn Ser Pro Glu Gly Val Val Glu
   1940                1945                1950
   gtc cct tat gat ttt att aat gtg gta cct cca atg cga gca cct      6440
   Val Pro Tyr Asp Phe Ile Asn Val Val Pro Pro Met Arg Ala Pro
   1955                1960                1965
   gat gcc gtt cgt caa tca gca ctc gcg tgg caa gaa gga aaa tgg      6485
   Asp Ala Val Arg Gln Ser Ala Leu Ala Trp Gln Glu Gly Lys Trp
   1970                1975                1980
   gct  aac gat ggt tgg gtt  gaa gta gaa aaa cat  acc ttg cgt cac       6530
   Ala  Asn Asp Gly Trp Val  Glu Val Glu Lys His  Thr Leu Arg His
   1985                1990                1995
   cgt  cgt tat gcc aat gtg  ttt gct gtg ggt gat  gtg gca ggg gtc       6575
   Arg  Arg Tyr Ala Asn Val  Phe Ala Val Gly Asp  Val Ala Gly Val
   2000                2005                2010
   cca  aaa ggc aaa acg gct  gcc agt gtc aaa tgg  caa gtt cct gtt       6620
   Pro  Lys Gly Lys Thr Ala  Ala Ser Val Lys Trp  Gln Val Pro Val
   2015                2020                2025
   gca  gtg gca cat tta ctc  gca gaa tta gag ggc  aaa cct tgt gat       6665
   Ala  Val Ala His Leu Leu  Ala Glu Leu Glu Gly  Lys Pro Cys Asp
   2030                2035                2040
   gaa  att tac aac ggt tat  aca tct tgt cca tta  att act caa tta       6710
   Glu  Ile Tyr Asn Gly Tyr  Thr Ser Cys Pro Leu  Ile Thr Gln Leu
   2045                2050                2055
   gga  aag ggg atg cta gta  gaa ttt gat tat aac  aac cac tta aca       6755
   Gly  Lys Gly Met Leu Val  Glu Phe Asp Tyr Asn  Asn His Leu Thr
   2060                2065                2070
   cct  tct ttc ccc ggt gta  ata gcg cca tta gaa  gaa ctg tgg gca       6800
   Pro  Ser Phe Pro Gly Val  Ile Ala Pro Leu Glu  Glu Leu Trp Ala
   2075                2080                2085
   aca  tgg gca att aaa aca  tta ggt tta aaa ccc  act tat tta ggt       6845
   Thr  Trp Ala Ile Lys Thr  Leu Gly Leu Lys Pro  Thr Tyr Leu Gly
   2090                2095                2100
   atg  tta cgt gga tta gct  taaggagcgt tga                            6876
   Met  Leu Arg Gly Leu Ala
   2105                2110
<210> 16
   <211> 2110
   <212> PRT
   <213> Pasteurella Multocida
<400> 16
   Met Gln Pro Ala Gln Glu His Cys Gln Arg Ile Asn Asn Ile Val Asn
   1               5                   10                  15
   Gln Glu Asn Gly Leu Phe His Thr Leu Gly Asn Met Met Leu Glu Ala
               20                  25                  30
   Glu Arg Ser Val Tyr Asn Ile Gly Asp Ile Tyr Ala Ser Lys Lys Leu
           35                  40                  45
   Thr Val His Thr His Asn Leu Ile Asn Asp Val Arg Leu Ser Gly Asn
       50                  55                  60
   Val Ser Tyr Lys Pro Ile Gly Ser Ser Arg Asp Tyr Asp Ile Ser Arg
   65                  70                  75                  80
   Val Ala Val His Gly Trp His Asn Asn Val Tyr Lys Leu Asn Leu Asn
                   85                  90                  95
   Leu Gln Glu Gln Asp Lys Thr Asp Ile Lys Val Val Lys Met Gly Ala
               100                 105                 110
   Ile Arg Ser Asp Gly Asp Phe Asp Phe Lys Gly Ile Lys Ala Thr Ser
           115                 120                 125
   Ser Glu Ser Lys Pro Gln Leu Ile Asn His Gly Leu Ile Asn Val Lys
       130                 135                 140
   Gly Thr Phe Asn Ala Glu Ala Asp Gln Val Val Asn Gln Met Lys Ala
   145                 150                 155                 160
   Phe Asn Gln Asn Ala Leu Ala Ser Val Phe Lys Asn Pro Ala Lys Ile
                   165                 170                 175
   Thr Met Tyr Tyr Gln Pro Leu Thr Arg Tyr Ile Trp Thr Pro Leu Ser
               180                 185                 190
   Gly Asn Ala Ser Arg Glu Phe Asn Asn Leu Glu Ser Phe Leu Asp Ala
           195                 200                 205
   Leu Phe Gly Ser Thr Thr Ile Leu Lys Ser Ser Phe Tyr Ser Thr Glu
       210                 215                 220
   Asn Phe Ser Ala Tyr Gln Leu Leu Ser His Ile Gln His Ser Pro Met
   225                 230                 235                 240
   Tyr Gln Lys Ala Met Ala Gln Val Phe Gly Ala Glu Trp His Ser Lys
                   245                 250                 255
   Ser Tyr Asp Glu Met Arg Asn Lys Trp Lys Ser Phe Lys Glu Asn Pro
               260                 265                 270
   Thr Asp Phe Ile Tyr Tyr Pro Ser Glu Lys Ala Lys Ile Leu Ala Gly
           275                 280                 285
   Lys Leu Glu Gly Lys Leu Thr Thr Leu Gln Asn Gly Glu Tyr Ala Glu
       290                 295                 300
   Arg Gly Lys Phe Asp Glu Ser Ile Gln Ile Gly Lys His Gln Leu Ser
   305                 310                 315                 320
   Leu Pro Ser Val Glu Leu Lys Ala Glu Phe Ser Asp Lys Glu Arg Leu
                   325                 330                 335
   Glu Glu Asp Gly Val Asp Leu Ser Ser Ile Ala Glu Leu Leu Glu Met
               340                 345                 350
   Pro Asn Leu Phe Ile Asp Asn Ser Ile Gln Leu Glu Lys Lys Lys Leu
           355                 360                 365
   Ser Pro Ile Glu Asp Leu Asp Glu Glu Pro Arg Lys Asn Leu Asp Ile
       370                 375                 380
   Glu Glu Ser His Ser Asn Ser Ser Asp Asp Val Leu Ser Met Asn Asp
   385                 390                 395                 400
   Asp Glu Ser Asp Thr Asp Asp Ser Lys Trp Ser Met Gly Asn Asp Glu
                   405                 410                 415
   Lys Glu Met Pro Asp Asp Lys Leu Gly Ile Ser Arg Asp Asp Arg Gly
               420                 425                 430
   Asn Lys Pro Pro Arg Thr Asp Pro Thr Val Asp Tyr Leu Asn Pro Asp
           435                 440                 445
   Glu Phe Phe Glu Asn Gly Tyr Leu Leu Asn Glu Leu Leu Gln Glu Leu
       450                 455                 460
   Gly Glu Glu Pro Leu Leu Lys Glu Gly Glu Asp His Phe Lys Arg Ser
   465                 470                 475                 480
   Thr Asn Leu Val Arg Leu Gly Glu Arg Asp Arg Gln Asn Arg Glu Lys
                   485                 490                 495
   Arg Glu Lys Glu Gly Tyr Phe Asp Leu Pro Gly Thr Leu Asp Met Lys
               500                 505                 510
   Leu Gln Glu Leu Phe Glu Lys Arg Lys Gln Lys His Glu Ala Glu Gln
           515                 520                 525
   Lys Ala Arg Ile Glu Lys Ala Leu Leu Gln Lys Ser Glu Gln Gln Glu
       530                 535                 540
   Lys Arg Val Glu Glu Arg Lys Gln Glu Glu Lys Arg Gln Ala Gln Asp
   545 550 555 560
   Lys Ile Ala Lys Gln Val Glu Ile Ala Lys Glu Met Gln Arg Val Glu
   565 570 575
   Glu Ile Arg Gln Arg Glu Lys Gln Leu Ala Ile Gln Leu Gln Glu Glu
   580 585 590
   Glu Lys Lys Gln Gln Glu Glu Lys His Leu Ser Glu Glu Lys Lys Gln
   595 600 605
   Ala Glu Gln Lys Gln Lys Ala Glu Glu Lys Val Ala Gln Glu Arg Leu
   610 615 620
   Asp Ile Glu Gln Gln Lys Ala Tyr Glu Glu Met Ala Lys Arg Glu Ala
   625 630 635 640
   Glu Ala Ser Lys Asn Val Leu Leu Lys Ala Ile Asp Glu Glu Arg Pro
   645 650 655
   Lys Val Glu Thr Asp Pro Leu Phe Arg Thr Lys Leu Lys Tyr Ile Asn
   660 665 670
   Gln Asp Asp Tyr Ala Gly Ala Asn Tyr Phe Phe Asn Lys Val Gly Leu
   675 680 685
   Asn Thr Lys Gly His Gln Lys Val Asn Val Leu Gly Asp Asn Tyr Phe
   690 695 700
   Asp His Gln Val Ile Thr Arg Ser Ile Glu Lys Lys Val Asp Asn His
   705 710 715 720
   Leu Asn Gln Lys Tyr Asn Leu Ser Asp Val Glu Leu Val Lys Gln Leu
   725 730 735
   Met Asp Asn Ser Thr Thr Gln Ala Gln Glu Leu Asp Leu Lys Leu Gly
   740 745 750
   Ala Ala Leu Thr Lys Glu Gln Gln Ala Asn Leu Thr Gln Asp Ile Val
   755 760 765
   Trp Tyr Val Lys Thr Lys Val Lys Gly Lys Asp Val Phe Val Pro Lys
   770 775 780
   Val Tyr Phe Ala Ser Glu Thr Leu Val Glu Ala Gln Lys Leu Gln Gly
   785 790 795 800
   Leu Gly Thr Gly Thr Ile Arg Val Gly Glu Ala Lys Ile Lys Ala Lys
                   805                 810                 815
   Asp Val Val Asn Thr Gly Thr Leu Ala Gly Arg Lys Leu Asn Val Glu
               820                 825                 830
   Ala Ser Asn Lys Ile Lys Asn Gln Gly Ser Ile Leu Ser Thr Gln Glu
           835                 840                 845
   Thr Arg Leu Val Gly Arg Lys Gly Ile Glu Asn Val Ser Arg Ser Phe
       850                 855                 860
   Ala Asn Asp Glu Leu Gly Val Thr Ala Gln Arg Ser Glu Ile Lys Thr
   865                 870                 875                 880
   Glu Gly His Leu His Leu Glu Thr Asp Lys Asp Ser Thr Ile Asp Val
                   885                 890                 895
   Gln Ala Ser Asp Ile Lys Ala Lys Thr Ser Phe Val Lys Thr Gly Asp
               900                 905                 910
   Val Asn Leu Lys Asn Thr Tyr Asn Thr Lys His Ala Tyr Arg Glu Lys
           915                 920                 925
   Phe Ser Pro Ser Ala Leu Gln Val Ala Glu Leu Asp Val Ala Gly Leu
       930                 935                 940
   Lys Val Pro Leu Leu Gly Val Ser Ser Pro Ser Ser Tyr Ser Glu His
   945                 950                 955                 960
   Thr Ser Glu Ala Thr Ser Glu Gly Ser Ile Phe Glu Val Gly His Leu
                   965                 970                 975
   His Leu Ala Val Asp Arg Asp Val Asn Gln Ala Gly Ser Lys Ile Lys
               980                 985                 990
   Ala Lys Tyr Thr Thr Gly Val Val  Lys Gly Asn Phe Asn  Thr Glu Ala
           995                 1000                 1005
   Gly Lys  Asn Ile Lys His Val  Glu Lys Glu Glu Tyr  Ser Ser Gln
       1010                 1015                 1020
   Leu Phe  Ala Ser Ala His Ala  Ser Gly Gly Gly Thr  Ser Val Arg
       1025                 1030                 1035
   Tyr Asp  Tyr Asn Ser Gln Asp  Gly Gly Asn Ala Ser  Val Gly Val
       1040                 1045                 1050
   Pro Thr  Asn His Thr Gly Val  Gly Ala Glu Ala Gly  Met Ser Phe
       1055                 1060                 1065
   Thr His  Thr Lys Asp Lys Glu  Thr Leu Leu Thr His  Thr Asn Ser
       1070                 1075                 1080
   Glu Leu  Gln Val Lys His Gly  Lys Leu His Val Leu  Gly Tyr Ala
       1085                 1090                 1095
   Asp Ile  Gly Gly Val Asp Ile  Asn Thr Lys Leu Pro  Glu Asp Ala
       1100                 1105                 1110
   Gln Ser  Lys Ala Gln Lys Glu  Ile Ala Ala Ser Lys  Pro Glu Lys
       1115                 1120                 1125
   Thr Glu  Gln Ser Ala Gln Asp  Val Ala Gln Ala Gln  Ser Asn Ala
       1130                 1135                 1140
   Asn Lys  Asp Lys Glu Asn Lys  Ala Pro Glu Ile Lys  Glu Leu Ser
       1145                 1150                 1155
   Glu Ala  Glu Ile Ala Asp Leu  Met Ser Glu Lys Ser  Lys Ala Tyr
       1160                 1165                 1170
   Phe Asp  Asp Phe Ala Glu Gln  Ala Lys Lys Ala Pro  Glu Asn Asn
       1175                 1180                 1185
   Arg Phe  Glu Leu Ser Ala Lys  Glu Ile Lys Ser Ser  Lys Gln Lys
       1190                 1195                 1200
   Asp Gln  Tyr Asp His Glu Ser  Glu Arg Thr Thr Phe  Lys Val Gly
       1205                 1210                 1215
   Pro Glu  Ala Glu Ala His Ser  Ala Val Ala Asp Met  Val Ser His
       1220                 1225                 1230
   Leu Val  Lys Glu Tyr Arg Asp  Ala Gln Asn Gly Thr  Lys Gln Asp
       1235                 1240                 1245
   Gly Thr  Val Ala Leu Gln His  Ala Ser Asp Val Leu  Asn Ile Val
       1250                 1255                 1260
   Thr Gly Asp Leu Ala Gly Ser Ser Ala Lys Leu Ser Val Glu Arg
   1265 1270 1275
   Thr His Glu Thr Lys Arg Thr Thr Glu Thr Gly Asp Ile Val Thr
   1280 1285 1290
   Lys Ile Gly Gly Asn Val Thr Leu Ser Ala Arg Ser Gly Ser Val
   1295 1300 1305
   Asn Leu Lys Asn Val Gln Ser Asp Glu Gln Ala Asn Leu Thr Leu
   1310 1315 1320
   Arg Ala Lys Glu Asp Val Asn Val Leu Ser Gly Glu Lys Thr Arg
   1325 1330 1335
   Glu Thr Thr Glu Thr Val Ser Arg Gln Lys Leu Ser His Gly Val
   1340 1345 1350
   Asn Ala Gly Cys Ser Met Met Ser Gly Ala Cys Thr Ala Gly Val
   1355 1360 1365
   Ser Thr Ser Leu Glu Gly Asn Glu Ser Tyr Thr Ser Glu Arg Glu
   1370 1375 1380
   Thr Ala Gln Asn Asn Ser Phe Leu Lys Ala Arg Asn Met Lys Val
   1385 1390 1395
   Glu Ala Gly Arg Asp Phe Asn Val Val Ser Ser Asn Ile Asp Ala
   1400 1405 1410
   Asp Lys Leu Asp Leu His Val Lys Gly Lys Thr Asn Val Val Ser
   1415 1420 1425
   Lys Gln Asp Thr Leu Gln Lys Val Thr His Gly Val Asp Tyr Asn
   1430 1435 1440
   Leu Ser Ala Gly Val Ala Leu Ser Ser Ala Thr Ile Ala Thr Pro
   1445 1450 1455
   Thr Gly Asn Val Gly Phe Gly Tyr Thr Asn Glu Thr Glu Ser Lys
   1460 1465 1470
   Arg Thr Val Asn Gln Gln Ala Gly Ile Lys Ala Asn Lys Ile Thr
   1475 1480 1485
   Gly Gln Thr His Asp Leu Asn Leu Glu Gly Gly Tyr Leu Val Ser
   1490 1495 1500
   Asn Asp Lys Asp Asn Gln Leu Lys Val Thr Gly Asp Val Thr Thr
   1505 1510 1515
   Lys Ala Leu His Asp Gln His Asp Lys Asp Gly Gly Thr Phe Gly
   1520 1525 1530
   Leu Ser Val Gly Ile Ser Glu Arg Gly Thr Thr Ala Phe Asn Val
   1535 1540 1545
   Arg Gly Gly Arg Ala Glu Gln Lys His Tyr Asn Ala Thr Gln Lys
   1550 1555 1560
   Ser Thr Leu Ser Gly Val Asp Thr Ser Gln Ala Asn Val Ser Gly
   1565 1570 1575
   Gln Val Asn Thr Asp Leu Thr Lys Ala Lys Ala Val Thr Arg Asp
   1580 1585 1590
   Asp Thr Tyr Ala Ser Thr Gln Phe Ser Phe Glu Val Ala Asp Ile
   1595 1600 1605
   Val Glu Leu Gly Gln Arg Ala Lys Asn Lys Leu Ser Ala Pro Asn
   1610 1615 1620
   Asn Asp Thr Asp Met Ala Ser Gly Ser Thr Leu Arg Ser Arg Ser
   1625 1630 1635
   Thr Thr Glu Glu Ala Asp Val Pro Thr Thr Arg Ser Arg Val Thr
   1640 1645 1650
   Asp Glu Ala Asp Ser Val Ser Val Lys Asn Pro Ile Tyr Glu Ser
   1655 1660 1665
   Ala Asp Ala Val Val Pro Thr Pro Arg Ser Arg Asn Val Asp Ser
   1670 1675 1680
   Thr Asp Leu Val Asp Asn Pro Leu Tyr Ala Ser Ala Thr Thr Lys
   1685 1690 1695
   Ala Asn Ile His Asp Tyr Glu Glu Ile Pro Ala Val Tyr Ser Lys
   1700 1705 1710
   Val Gly Asp Asn Asn Ala Asp Leu Val Arg His Lys Thr Ala Thr
   1715 1720 1725
   Ser Asp  Glu His Leu Tyr Ala  Glu Ile Asn Glu Pro  Thr Tyr Ser
       1730                 1735                 1740
   Arg Val  Gly Asp Lys Asn Ala  Asp Met Arg Arg His  Asn Ala Ala
       1745                 1750                 1755
   Gly Thr  Thr Asp Tyr Ala Asp  Val Val Gln Ala His  Thr Arg Lys
       1760                 1765                 1770
   Ala Asp  Asp Pro Leu Pro Ala  Leu Pro Asn Gln Gly  Lys Ala Arg
       1775                 1780                 1785
   Thr Val  Asn Asp Gly Ser Glu  His Ile Tyr Thr Asp  Ile Ser Asp
       1790                 1795                 1800
   Val Gly  Thr Gln Thr Lys Ala  Ile Asp Ser Thr Tyr  Ala Thr Val
       1805                 1810                 1815
   Gly Met  Pro Lys Ala Asn Ala  Val Asn Leu Ile Gly  Gln Asn Gly
       1820                 1825                 1830
   Leu Gly  Ser Ile Tyr His Ser  Pro Asp Ser Ala Tyr  Lys Thr Trp
       1835                 1840                 1845
   Gln Leu  Leu Asp Gln Phe Ala  Asn Lys Gly Gly Asp  Ala Val Phe
       1850                 1855                 1860
   Leu Arg  Pro Ala Thr Glu Met  Lys Cys Ala Gly Ala  Pro Leu Lys
       1865                 1870                 1875
   Tyr Thr  Phe Ile Val Arg Asp  Tyr Leu Leu Arg Arg  His Thr Leu
       1880                 1885                 1890
   Asp Lys  Ser Arg Leu Phe Tyr  Asn Ala His Asn Lys  Thr Leu Phe
       1895                 1900                 1905
   Ser Val  Pro Ile Val Asp Ala  Lys Val Lys Met Leu  Phe Ala Glu
       1910                 1915                 1920
   Lys Asn  Ile Gln Val Asn Tyr  Asp Arg Ser Leu Thr  Ala Ile Asp
       1925                 1930                 1935
   Leu Ser  Lys Arg Ile Ala Thr  Phe Asn Ser Pro Glu  Gly Val Val
       1940                 1945                 1950
   Glu Val  Pro Tyr Asp Phe Ile  Asn Val Val Pro Pro  Met Arg Ala
       1955                 1960                 1965
   Pro Asp  Ala Val Arg Gln Ser  Ala Leu Ala Trp Gln  Glu Gly Lys
       1970                 1975                 1980
   Trp Ala  Asn Asp Gly Trp Val  Glu Val Glu Lys His  Thr Leu Arg
       1985                 1990                 1995
   His Arg  Arg Tyr Ala Asn Val  Phe Ala Val Gly Asp  Val Ala Gly
       2000                 2005                 2010
   Val Pro  Lys Gly Lys Thr Ala  Ala Ser Val Lys Trp  Gln Val Pro
       2015                 2020                 2025
   Val Ala  Val Ala His Leu Leu  Ala Glu Leu Glu Gly  Lys Pro Cys
       2030                 2035                 2040
   Asp Glu  Ile Tyr Asn Gly Tyr  Thr Ser Cys Pro Leu  Ile Thr Gln
       2045                 2050                 2055
   Leu Gly  Lys Gly Met Leu Val  Glu Phe Asp Tyr Asn  Asn His Leu
       2060                 2065                 2070
   Thr Pro  Ser Phe Pro Gly Val  Ile Ala Pro Leu Glu  Glu Leu Trp
       2075                 2080                 2085
   Ala Thr  Trp Ala Ile Lys Thr  Leu Gly Leu Lys Pro  Thr Tyr Leu
       2090                 2095                 2100
   Gly Met  Leu Arg Gly Leu Ala
       2105                 2110
<210> 17
   <211> 3247
   <212> DNA
   <213> Pasteurella Multocida
<220>
   <221> misc_feature
   <223> fhaB2
<220>
   <221> CDS
   <222> (1479) .. (3245)
<400> 17
   gtcgaccttg cgggtgaaaa ggtatctcta aattttggcg atatcattca tgcttaccaa     60
   aaccagcccc tatcaacaaa agttgttttt caattagtga aagatttgac ggaagtttta    120
   taccgttctg gctacgtgac aagtgcaatt ggtttaaaaa attcaaaaat cagcaatggc    180
   gatcttgaat ttattgtact gtggggaaga actcgcgatc tgtttgtgaa tggggagaaa    240
   ccaacccgtt ttagagataa aacaatgtta tcagtcctac ccaatttaat cggaaatcgc    300
   ttaagtattc acgacattga ccagttgatc gaaatcttaa atactacgaa taaaaaagcc    360
   acagtgaatg tggttgcaag tgaagaaaaa ggcagctcaa atctaaatat tgaaagacaa    420
   tatgatgttt ttccgcaagt gagtgtcgga ttcaataatt caggtgctgg caataatgcc    480
   aatgggcgta atcaagctac attgaatatt gcttggagtg atctattagg cacgaatgat    540
   cgttggagtt tctcgagtag ttaccgttta tataaaaatc atcatgctaa ccagcaacgc    600
   aattatactt tgtcttacag tcagcctata ggcttttcta cagtagaaat taaagcatcg    660
   gaatctacgt atgaaaaaga acttcgcggt ataaatactc attcttctca tgggaaaacc    720
   caaagcttag ctgtcaagct gatgcatgtg ttattgcgta ataaggagag tattttatct    780
   acatataccg agttcgagtt taaaaaacgg attagttatt tttctgatat tttgattggg    840
   aaatatcaca ataataaagt gagcgtaggg ttatcttaca tgactaattt tgcttacggg    900
   aagctttaca gcgacattgc ttacgcgaat gggttgagat ggtttggggc gaattattca    960
   gcatatgatg caaatcgtga aaaaacctta aaattattgt caggaagtat taattggcag   1020
   cgtccaatat ccctgtttga acgtgcgatg aattatcaat tacgtattgg tgcccaatat   1080
   ggttttgata gtttgtattc tgaaaatcaa ttttcaattg gtgatgaata tacagtaaga   1140
   ggatttaaag gtggtgcggt ttctggtgat agtggtgcgt atttatcaca aacactgacg   1200
   gttccttttt atccacaaaa agcatattta tctcaggtat ccccttttat tggatttgat   1260
   atgggtaaag tacatattaa atcaaagcat aaaacaacca ctttagtcgg ttttgcccta   1320
   ggcttgaaaa cgcaaataaa gttattttca ttatcattaa cctatgcaca accaatgaat   1380
   ggtgtgagtg gtgttacgca acatcgtcaa aaaccgattt attatttctc aggatcactt   1440
   tctttttaat ctcttttaag tttaaggatt aacttaat atg aac aaa aat cgt tac   1496
                                             Met Asn Lys Asn Arg Tyr
                                             1               5
   aaa ctc att ttt agt caa gtc aaa ggt tgt ctc gtt cct gtg gca gaa     1544
   Lys Leu Ile Phe Ser Gln Val Lys Gly Cys Leu Val Pro Val Ala Glu
               10                  15                  20
   tgt att aac tca gct att agc aat ggt tca tct gat tca aca tcc aca     1592
   Cys Ile Asn Ser Ala Ile Ser Asn Gly Ser Ser Asp Ser Thr Ser Thr
           25                  30                  35
   tca gaa caa gtt gaa gag gaa cct ttc ctt cta gaa caa tat tca ctt     1640
   Ser Glu Gln Val Glu Glu Glu Pro Phe Leu Leu Glu Gln Tyr Ser Leu
       40                   45                   50
   tcc tcc gtg tct tta tta gta aaa agc acg ttc aat cct gtt tcg tat        1688
   Ser Ser Val Ser Leu Leu Val Lys Ser Thr Phe Asn Pro Val Ser Tyr
   55                  60                  65                  70
   gca atg caa ttg act tgg aaa cag ctt tct att tta ttt tta act gtg        1736
   Ala Met Gln Leu Thr Trp Lys Gln Leu Ser Ile Leu Phe Leu Thr Val
                   75                  80                  85
   att tct gtt cct gtt ttg gct gag gga aaa ggg gat gaa aga aat caa        1784
   Ile Ser Val Pro Val Leu Ala Glu Gly Lys Gly Asp Glu Arg Asn Gln
               90                  95                  100
   tta aca gtg att gat aat agc gat cat att aaa tta gat gca tct aat        1832
   Leu Thr Val Ile Asp Asn Ser Asp His Ile Lys Leu Asp Ala Ser Asn
           105                 110                 115
   ctt gct ggt aat gat aaa aca aaa atc tat caa gca gaa aat aaa gtt        1880
   Leu Ala Gly Asn Asp Lys Thr Lys Ile Tyr Gln Ala Glu Asn Lys Val
       120                 125                 130
   ctg gtt att gat att gct aaa cca aat ggg aaa ggg att tca gat aac        1928
   Leu Val Ile Asp Ile Ala Lys Pro Asn Gly Lys Gly Ile Ser Asp Asn
   135                 140                 145                 150
   cgt ttt gaa aaa ttt aat att cca aat agc gcg gtg ttt aat aat aat        1976
   Arg Phe Glu Lys Phe Asn Ile Pro Asn Ser Ala Val Phe Asn Asn Asn
                   155                 160                 165
   ggg act gaa gcg cag gca aga tca aca tta att ggt tac att ccg caa        2024
   Gly Thr Glu Ala Gln Ala Arg Ser Thr Leu Ile Gly Tyr Ile Pro Gln
               170                 175                 180
   aat caa aat tta agg gga ggg aaa gaa gct gat gtt ata tta aat caa        2072
   Asn Gln Asn Leu Arg Gly Gly Lys Glu Ala Asp Val Ile Leu Asn Gln
           185                 190                 195
   gtg aca ggt cct caa gaa agt aaa att gtt ggc gcg ctt gaa gta tta        2120
   Val Thr Gly Pro Gln Glu Ser Lys Ile Val Gly Ala Leu Glu Val Leu
       200                 205                 210
   ggt aaa aaa gct gat atc gtc att gca aac caa aat ggt att acc tta        2168
   Gly Lys Lys Ala Asp Ile Val Ile Ala Asn Gln Asn Gly Ile Thr Leu
   215                 220                 225                 230
   aat ggt gta aga aca ata aat tca gat cgt ttt gtt gcc act acg agt        2216
   Asn Gly Val Arg Thr Ile Asn Ser Asp Arg Phe Val Ala Thr Thr Ser
                   235                 240                 245
   gag ctt ata gat ccg aat cag atg atg tta aag gtt aca aaa gga aat        2264
   Glu Leu Ile Asp Pro Asn Gln Met Met Leu Lys Val Thr Lys Gly Asn
               250                 255                 260
   gtg atc att gat att gat ggt ttt tcg aca gat gga tta aag tat tta        2312
   Val Ile Ile Asp Ile Asp Gly Phe Ser Thr Asp Gly Leu Lys Tyr Leu
           265                 270                 275
   gat att att gct aaa aaa att gaa caa aag caa tca att aca tca ggg        2360
   Asp Ile Ile Ala Lys Lys Ile Glu Gln Lys Gln Ser Ile Thr Ser Gly
       280                 285                 290
   gat aat tca gaa gca aaa aca gat gtc act ctt att gcg ggt tcc agt      2408
   Asp Asn Ser Glu Ala Lys Thr Asp Val Thr Leu Ile Ala Gly Ser Ser
   295                 300                 305                 310
   gaa tat gat tta agc aaa cat gag ctg aaa aaa acg agc ggt gaa aat      2456
   Glu Tyr Asp Leu Ser Lys His Glu Leu Lys Lys Thr Ser Gly Glu Asn
                   315                 320                 325
   gta tct aat gat gtt att gct atc acg gga tct agt aca ggc gca atg      2504
   Val Ser Asn Asp Val Ile Ala Ile Thr Gly Ser Ser Thr Gly Ala Met
               330                 335                 340
   cat ggt aaa aat att aag ttg att gtg aca gat aaa ggt gca ggc gta      2552
   His Gly Lys Asn Ile Lys Leu Ile Val Thr Asp Lys Gly Ala Gly Val
           345                 350                 355
   aaa cat gat gga att att ttg tct gaa aat gat att cag att gaa atg      2600
   Lys His Asp Gly Ile Ile Leu Ser Glu Asn Asp Ile Gln Ile Glu Met
       360                 365                 370
   aat gaa ggt gac tta gaa ctt ggc aat acg att cag caa aca gtg gta      2648
   Asn Glu Gly Asp Leu Glu Leu Gly Asn Thr Ile Gln Gln Thr Val Val
   375                 380                 385                 390
   aaa aaa gac cga aat att cga gcc aag aaa aaa att gaa gtg aaa aac      2696
   Lys Lys Asp Arg Asn Ile Arg Ala Lys Lys Lys Ile Glu Val Lys Asn
                   395                 400                 405
   gct aat cgt gtt ttt gtt ggt agt caa acg aaa tca gat gaa att tcg      2744
   Ala Asn Arg Val Phe Val Gly Ser Gln Thr Lys Ser Asp Glu Ile Ser
               410                 415                 420
   tta gag gcg aaa caa gtt aaa atc aga aaa aac gca gag att agg agt      2792
   Leu Glu Ala Lys Gln Val Lys Ile Arg Lys Asn Ala Glu Ile Arg Ser
           425                 430                 435
   acg aca caa gcc aaa atc gta gca aag ggt gcc ctg tct att gag caa      2840
   Thr Thr Gln Ala Lys Ile Val Ala Lys Gly Ala Leu Ser Ile Glu Gln
       440                 445                 450
   aat gcg aag ctc gtc gct aaa aag ata gat gtg gca aca gaa act cta      2888
   Asn Ala Lys Leu Val Ala Lys Lys Ile Asp Val Ala Thr Glu Thr Leu
   455                 460                 465                 470
   act aat gct ggg cgt att tat ggt cga gag gtt aag ctt gac act aat      2936
   Thr Asn Ala Gly Arg Ile Tyr Gly Arg Glu Val Lys Leu Asp Thr Asn
                   475                 480                 485
   aat ttg att aat gat aaa gaa att tat gct gaa cgg aaa ttg agt att      2984
   Asn Leu Ile Asn Asp Lys Glu Ile Tyr Ala Glu Arg Lys Leu Ser Ile
               490                 495                 500
   ttg acg aaa gga aaa gat ctt gaa att att caa gat aga tat ttg tct      3032
   Leu Thr Lys Gly Lys Asp Leu Glu Ile Ile Gln Asp Arg Tyr Leu Ser
           505                 510                 515
   cca ctg atg cgc gta aaa agt agt gtc cgc ttt tta ggc tct ccg ttt      3080
   Pro Leu Met Arg Val Lys Ser Ser Val Arg Phe Leu Gly Ser Pro Phe
       520                 525                 530
   ttc tca ata tct ccg tcg atg ctc gca agc ctt agt gca cag ttt aag      3128
   Phe Ser Ile Ser Pro Ser Met Leu Ala Ser Leu Ser Ala Gln Phe Lys
   535                 540                 545                 550
   cct ggt ttt gtg aat aag gga ctc att gaa agt gcg ggg agt gca gaa      3176
   Pro Gly Phe Val Asn Lys Gly Leu Ile Glu Ser Ala Gly Ser Ala Glu
                   555                 560                 565
   tta act ttt aaa gaa aaa acc agt ttt tta aca gag ggc aat aat ttt      3224
   Leu Thr Phe Lys Glu Lys Thr Ser Phe Leu Thr Glu Gly Asn Asn Phe
               570                 575                 580
   att aga gct aaa gat gcg tta ac                                       3247
   Ile Arg Ala Lys Asp Ala Leu
           585
<210> 18
   <211> 589
   <212> PRT
   <213> Pasteurella Multocida
<400> 18
   Met Asn Lys Asn Arg Tyr Lys Leu Ile Phe Ser Gln Val Lys Gly Cys
   1               5                   10                  15
   Leu Val Pro Val Ala Glu Cys Ile Asn Ser Ala Ile Ser Asn Gly Ser
               20                  25                  30
   Ser Asp Ser Thr Ser Thr Ser Glu Gln Val Glu Glu Glu Pro Phe Leu
           35                  40                  45
   Leu Glu Gln Tyr Ser Leu Ser Ser Val Ser Leu Leu Val Lys Ser Thr
       50                  55                  60
   Phe Asn Pro Val Ser Tyr Ala Met Gln Leu Thr Trp Lys Gln Leu Ser
   65                  70                  75                  80
   Ile Leu Phe Leu Thr Val Ile Ser Val Pro Val Leu Ala Glu Gly Lys
                   85                  90                  95
   Gly Asp Glu Arg Asn Gln Leu Thr Val Ile Asp Asn Ser Asp His Ile
               100                 105                 110
   Lys Leu Asp Ala Ser Asn Leu Ala Gly Asn Asp Lys Thr Lys Ile Tyr
           115                 120                 125
   Gln Ala Glu Asn Lys Val Leu Val Ile Asp Ile Ala Lys Pro Asn Gly
       130                 135                 140
   Lys Gly Ile Ser Asp Asn Arg Phe Glu Lys Phe Asn Ile Pro Asn Ser
   145 150 155 160
   Ala Val Phe Asn Asn Asn Gly Thr Glu Ala Gln Ala Arg Ser Thr Leu
   165 170 175
   Ile Gly Tyr Ile Pro Gln Asn Gln Asn Leu Arg Gly Gly Lys Glu Ala
   180 185 190
   Asp Val Ile Leu Asn Gln Val Thr Gly Pro Gln Glu Ser Lys Ile Val
   195 200 205
   Gly Ala Leu Glu Val Leu Gly Lys Lys Ala Asp Ile Val Ile Ala Asn
   210 215 220
   Gln Asn Gly Ile Thr Leu Asn Gly Val Arg Thr Ile Asn Ser Asp Arg
   225 230 235 240
   Phe Val Ala Thr Thr Ser Glu Leu Ile Asp Pro Asn Gln Met Met Leu
   245 250 255
   Lys Val Thr Lys Gly Asn Val Ile Ile Asp Ile Asp Gly Phe Ser Thr
   260 265 270
   Asp Gly Leu Lys Tyr Leu Asp Ile Ile Ala Lys Lys Ile Glu Gln Lys
   275 280 285
   Gln Ser Ile Thr Ser Gly Asp Asn Ser Glu Ala Lys Thr Asp Val Thr
   290 295 300
   Leu Ile Ala Gly Ser Ser Glu Tyr Asp Leu Ser Lys His Glu Leu Lys
   305 310 315 320
   Lys Thr Ser Gly Glu Asn Val Ser Asn Asp Val Ile Ala Ile Thr Gly
   325 330 335
   Ser Ser Thr Gly Ala Met His Gly Lys Asn Ile Lys Leu Ile Val Thr
   340 345 350
   Asp Lys Gly Ala Gly Val Lys His Asp Gly Ile Ile Leu Ser Glu Asn
   355 360 365
   Asp Ile Gln Ile Glu Met Asn Glu Gly Asp Leu Glu Leu Gly Asn Thr
   370 375 380
   Ile Gln Gln Thr Val Val Lys Lys Asp Arg Asn Ile Arg Ala Lys Lys
   385 390 395 400
   Lys Ile Glu Val Lys Asn Ala Asn Arg Val Phe Val Gly Ser Gln Thr
                   405                 410                 415
   Lys Ser Asp Glu Ile Ser Leu Glu Ala Lys Gln Val Lys Ile Arg Lys
               420                 425                 430
   Asn Ala Glu Ile Arg Ser Thr Thr Gln Ala Lys Ile Val Ala Lys Gly
           435                 440                 445
   Ala Leu Ser Ile Glu Gln Asn Ala Lys Leu Val Ala Lys Lys Ile Asp
       450                 455                 460
   Val Ala Thr Glu Thr Leu Thr Asn Ala Gly Arg Ile Tyr Gly Arg Glu
   465                 470                 475                 480
   Val Lys Leu Asp Thr Asn Asn Leu Ile Asn Asp Lys Glu Ile Tyr Ala
                   485                 490                 495
   Glu Arg Lys Leu Ser Ile Leu Thr Lys Gly Lys Asp Leu Glu Ile Ile
               500                 505                 510
   Gln Asp Arg Tyr Leu Ser Pro Leu Met Arg Val Lys Ser Ser Val Arg
           515                 520                 525
   Phe Leu Gly Ser Pro Phe Phe Ser Ile Ser Pro Ser Met Leu Ala Ser
       530                 535                 540
   Leu Ser Ala Gln Phe Lys Pro Gly Phe Val Asn Lys Gly Leu Ile Glu
   545                 550                 555                 560
   Ser Ala Gly Ser Ala Glu Leu Thr Phe Lys Glu Lys Thr Ser Phe Leu
                   565                 570                 575
   Thr Glu Gly Asn Asn Phe Ile Arg Ala Lys Asp Ala Leu
               580                 585
<210> 19
   <211> 3247
   <212> DNA
   <213> Pasteurella Multocida
<220>
   <221> misc_feature
   <223> fhaC
<220>
   <221> CDS
   <222> (1) .. (1446)
<400> 19
   gtc gac ctt gcg ggt gaa aag gta tct cta aat ttt ggc gat atc att      48
   Val Asp Leu Ala Gly Glu Lys Val Ser Leu Asn Phe Gly Asp Ile Ile
   1               5                   10                  15
   cat gct tac caa aac cag ccc cta tca aca aaa gtt gtt ttt caa tta      96
   His Ala Tyr Gln Asn Gln Pro Leu Ser Thr Lys Val Val Phe Gln Leu
               20                  25                  30
   gtg aaa gat ttg acg gaa gtt tta tac cgt tct ggc tac gtg aca agt     144
   Val Lys Asp Leu Thr Glu Val Leu Tyr Arg Ser Gly Tyr Val Thr Ser
           35                  40                  45
   gca att ggt tta aaa aat tca aaa atc agc aat ggc gat ctt gaa ttt     192
   Ala Ile Gly Leu Lys Asn Ser Lys Ile Ser Asn Gly Asp Leu Glu Phe
       50                  55                  60
   att gta ctg tgg gga aga act cgc gat ctg ttt gtg aat ggg gag aaa     240
   Ile Val Leu Trp Gly Arg Thr Arg Asp Leu Phe Val Asn Gly Glu Lys
   65                  70                  75                  80
   cca acc cgt ttt aga gat aaa aca atg tta tca gtc cta ccc aat tta     288
   Pro Thr Arg Phe Arg Asp Lys Thr Met Leu Ser Val Leu Pro Asn Leu
                   85                  90                  95
   atc gga aat cgc tta agt att cac gac att gac cag ttg atc gaa atc     336
   Ile Gly Asn Arg Leu Ser Ile His Asp Ile Asp Gln Leu Ile Glu Ile
               100                 105                 110
   tta aat act acg aat aaa aaa gcc aca gtg aat gtg gtt gca agt gaa     384
   Leu Asn Thr Thr Asn Lys Lys Ala Thr Val Asn Val Val Ala Ser Glu
           115                 120                 125
   gaa aaa ggc agc tca aat cta aat att gaa aga caa tat gat gtt ttt     432
   Glu Lys Gly Ser Ser Asn Leu Asn Ile Glu Arg Gln Tyr Asp Val Phe
       130                 135                 140
   ccg caa gtg agt gtc gga ttc aat aat tca ggt gct ggc aat aat gcc     480
   Pro Gln Val Ser Val Gly Phe Asn Asn Ser Gly Ala Gly Asn Asn Ala
   145                 150                 155                 160
   aat ggg cgt aat caa gct aca ttg aat att gct tgg agt gat cta tta     528
   Asn Gly Arg Asn Gln Ala Thr Leu Asn Ile Ala Trp Ser Asp Leu Leu
                   165                 170                 175
   ggc acg aat gat cgt tgg agt ttc tcg agt agt tac cgt tta tat aaa     576
   Gly Thr Asn Asp Arg Trp Ser Phe Ser Ser Ser Tyr Arg Leu Tyr Lys
               180                 185                 190
   aat cat cat gct aac cag caa cgc aat tat act ttg tct tac agt cag     624
   Asn His His Ala Asn Gln Gln Arg Asn Tyr Thr Leu Ser Tyr Ser Gln
           195                 200                 205
   cct ata ggc ttt tct aca gta gaa att aaa gca tcg gaa tct acg tat     672
   Pro Ile Gly Phe Ser Thr Val Glu Ile Lys Ala Ser Glu Ser Thr Tyr
       210                 215                 220
   gaa aaa gaa ctt cgc ggt ata aat act cat tct tct cat ggg aaa acc     720
   Glu Lys Glu Leu Arg Gly Ile Asn Thr His Ser Ser His Gly Lys Thr
   225                 230                 235                 240
   caa agc tta gct gtc aag ctg atg cat gtg tta ttg cgt aat aag gag      768
   Gln Ser Leu Ala Val Lys Leu Met His Val Leu Leu Arg Asn Lys Glu
                   245                 250                 255
   agt att tta tct aca tat acc gag ttc gag ttt aaa aaa cgg att agt      816
   Ser Ile Leu Ser Thr Tyr Thr Glu Phe Glu Phe Lys Lys Arg Ile Ser
               260                 265                 270
   tat ttt tct gat att ttg att ggg aaa tat cac aat aat aaa gtg agc      864
   Tyr Phe Ser Asp Ile Leu Ile Gly Lys Tyr His Asn Asn Lys Val Ser
           275                 280                 285
   gta ggg tta tct tac atg act aat ttt gct tac ggg aag ctt tac agc      912
   Val Gly Leu Ser Tyr Met Thr Asn Phe Ala Tyr Gly Lys Leu Tyr Ser
       290                 295                 300
   gac att gct tac gcg aat ggg ttg aga tgg ttt ggg gcg aat tat tca      960
   Asp Ile Ala Tyr Ala Asn Gly Leu Arg Trp Phe Gly Ala Asn Tyr Ser
   305                 310                 315                 320
   gca tat gat gca aat cgt gaa aaa acc tta aaa tta ttg tca gga agt     1008
   Ala Tyr Asp Ala Asn Arg Glu Lys Thr Leu Lys Leu Leu Ser Gly Ser
                   325                 330                 335
   att aat tgg cag cgt cca ata tcc ctg ttt gaa cgt gcg atg aat tat     1056
   Ile Asn Trp Gln Arg Pro Ile Ser Leu Phe Glu Arg Ala Met Asn Tyr
               340                 345                 350
   caa tta cgt att ggt gcc caa tat ggt ttt gat agt ttg tat tct gaa     1104
   Gln Leu Arg Ile Gly Ala Gln Tyr Gly Phe Asp Ser Leu Tyr Ser Glu
           355                 360                 365
   aat caa ttt tca att ggt gat gaa tat aca gta aga gga ttt aaa ggt     1152
   Asn Gln Phe Ser Ile Gly Asp Glu Tyr Thr Val Arg Gly Phe Lys Gly
       370                 375                 380
   ggt gcg gtt tct ggt gat agt ggt gcg tat tta tca caa aca ctg acg     1200
   Gly Ala Val Ser Gly Asp Ser Gly Ala Tyr Leu Ser Gln Thr Leu Thr
   385                 390                 395                 400
   gtt cct ttt tat cca caa aaa gca tat tta tct cag gta tcc cct ttt     1248
   Val Pro Phe Tyr Pro Gln Lys Ala Tyr Leu Ser Gln Val Ser Pro Phe
                   405                 410                 415
   att gga ttt gat atg ggt aaa gta cat att aaa tca aag cat aaa aca     1296
   Ile Gly Phe Asp Met Gly Lys Val His Ile Lys Ser Lys His Lys Thr
               420                 425                 430
   acc act tta gtc ggt ttt gcc cta ggc ttg aaa acg caa ata aag tta     1344
   Thr Thr Leu Val Gly Phe Ala Leu Gly Leu Lys Thr Gln Ile Lys Leu
           435                 440                 445
   ttt tca tta tca tta acc tat gca caa cca atg aat ggt gtg agt ggt     1392
   Phe Ser Leu Ser Leu Thr Tyr Ala Gln Pro Met Asn Gly Val Ser Gly
       450                 455                 460
   gtt acg caa cat cgt caa aaa ccg att tat tat ttc tca gga tca ctt     1440
   Val Thr Gln His Arg Gln Lys Pro Ile Tyr Tyr Phe Ser Gly Ser Leu
   465 470 475 480
   tct ttt taatctcttt taagtttaag gattaactta atatgaacaa aaatcgttac 1496
   Ser Phe
   aaactcattt ttagtcaagt caaaggttgt ctcgttcctg tggcagaatg tattaactca 1556
   gctattagca atggttcatc tgattcaaca tccacatcag aacaagttga agaggaacct 1616
   ttccttctag aacaatattc actttcctcc gtgtctttat tagtaaaaag cacgttcaat 1676
   cctgtttcgt atgcaatgca attgacttgg aaacagcttt ctattttatt tttaactgtg 1736
   atttctgttc ctgttttggc tgagggaaaa ggggatgaaa gaaatcaatt aacagtgatt 1796
   gataatagcg atcatattaa attagatgca tctaatcttg ctggtaatga taaaacaaaa 1856
   atctatcaag cagaaaataa agttctggtt attgatattg ctaaaccaaa tgggaaaggg 1916
   atttcagata accgttttga aaaatttaat attccaaata gcgcggtgtt taataataat 1976
   gggactgaag cgcaggcaag atcaacatta attggttaca ttccgcaaaa tcaaaattta 2036
   aggggaggga aagaagctga tgttatatta aatcaagtga caggtcctca agaaagtaaa 2096
   attgttggcg cgcttgaagt attaggtaaa aaagctgata tcgtcattgc aaaccaaaat 2156
   ggtattacct taaatggtgt aagaacaata aattcagatc gttttgttgc cactacgagt 2216
   gagcttatag atccgaatca gatgatgtta aaggttacaa aaggaaatgt gatcattgat 2276
   attgatggtt tttcgacaga tggattaaag tatttagata ttattgctaa aaaaattgaa 2336
   caaaagcaat caattacatc aggggataat tcagaagcaa aaacagatgt cactcttatt 2396
   gcgggttcca gtgaatatga tttaagcaaa catgagctga aaaaaacgag cggtgaaaat 2456
   gtatctaatg atgttattgc tatcacggga tctagtacag gcgcaatgca tggtaaaaat 2516
   attaagttga ttgtgacaga taaaggtgca ggcgtaaaac atgatggaat tattttgtct 2576
   gaaaatgata ttcagattga aatgaatgaa ggtgacttag aacttggcaa tacgattcag 2636
   caaacagtgg taaaaaaaga ccgaaatatt cgagccaaga aaaaaattga agtgaaaaac 2696
   gctaatcgtg tttttgttgg tagtcaaacg aaatcagatg aaatttcgtt agaggcgaaa 2756
   caagttaaaa tcagaaaaaa cgcagagatt aggagtacga cacaagccaa aatcgtagca 2816
   aagggtgccc tgtctattga gcaaaatgcg aagctcgtcg ctaaaaagat agatgtggca 2876
   acagaaactc taactaatgc tgggcgtatt tatggtcgag aggttaagct tgacactaat 2936
   aatttgatta atgataaaga aatttatgct gaacggaaat tgagtatttt gacgaaagga 2996
   aaagatcttg aaattattca agatagatat ttgtctccac tgatgcgcgt aaaaagtagt 3056
   gtccgctttt taggctctcc gtttttctca atatctccgt cgatgctcgc aagccttagt 3116
   gcacagttta agcctggttt tgtgaataag ggactcattg aaagtgcggg gagtgcagaa 3176
   ttaactttta aagaaaaaac cagtttttta acagagggca ataattttat tagagctaaa 3236
   gatgcgttaa c 3247
<210> 20
   <211> 482
   <212> PRT
   <213> Pasteurella Multocida
<400> 20
   Val Asp Leu Ala Gly Glu Lys Val Ser Leu Asn Phe Gly Asp Ile Ile
   1               5                   10                  15
   His Ala Tyr Gln Asn Gln Pro Leu Ser Thr Lys Val Val Phe Gln Leu
               20                  25                  30
   Val Lys Asp Leu Thr Glu Val Leu Tyr Arg Ser Gly Tyr Val Thr Ser
           35                  40                  45
   Ala Ile Gly Leu Lys Asn Ser Lys Ile Ser Asn Gly Asp Leu Glu Phe
       50                  55                  60
   Ile Val Leu Trp Gly Arg Thr Arg Asp Leu Phe Val Asn Gly Glu Lys
   65                  70                  75                  80
   Pro Thr Arg Phe Arg Asp Lys Thr Met Leu Ser Val Leu Pro Asn Leu
                   85                  90                  95
   Ile Gly Asn Arg Leu Ser Ile His Asp Ile Asp Gln Leu Ile Glu Ile
               100                 105                 110
   Leu Asn Thr Thr Asn Lys Lys Ala Thr Val Asn Val Val Ala Ser Glu
           115                 120                 125
   Glu Lys Gly Ser Ser Asn Leu Asn Ile Glu Arg Gln Tyr Asp Val Phe
       130                 135                 140
   Pro Gln Val Ser Val Gly Phe Asn Asn Ser Gly Ala Gly Asn Asn Ala
   145                 150                 155                 160
   Asn Gly Arg Asn Gln Ala Thr Leu Asn Ile Ala Trp Ser Asp Leu Leu
                   165                 170                 175
   Gly Thr Asn Asp Arg Trp Ser Phe Ser Ser Ser Tyr Arg Leu Tyr Lys
               180                 185                 190
   Asn His His Ala Asn Gln Gln Arg Asn Tyr Thr Leu Ser Tyr Ser Gln
           195                 200                 205
   Pro Ile Gly Phe Ser Thr Val Glu Ile Lys Ala Ser Glu Ser Thr Tyr
       210                 215                 220
   Glu Lys Glu Leu Arg Gly Ile Asn Thr His Ser Ser His Gly Lys Thr
   225                 230                 235                 240
   Gln Ser Leu Ala Val Lys Leu Met His Val Leu Leu Arg Asn Lys Glu
                   245                 250                 255
   Ser Ile Leu Ser Thr Tyr Thr Glu Phe Glu Phe Lys Lys Arg Ile Ser
               260                 265                 270
   Tyr Phe Ser Asp Ile Leu Ile Gly Lys Tyr His Asn Asn Lys Val Ser
           275                 280                 285
   Val Gly Leu Ser Tyr Met Thr Asn Phe Ala Tyr Gly Lys Leu Tyr Ser
       290                 295                 300
   Asp Ile Ala Tyr Ala Asn Gly Leu Arg Trp Phe Gly Ala Asn Tyr Ser
   305                 310                 315                 320
   Ala Tyr Asp Ala Asn Arg Glu Lys Thr Leu Lys Leu Leu Ser Gly Ser
                   325                 330                 335
   Ile Asn Trp Gln Arg Pro Ile Ser Leu Phe Glu Arg Ala Met Asn Tyr
               340                 345                 350
   Gln Leu Arg Ile Gly Ala Gln Tyr Gly Phe Asp Ser Leu Tyr Ser Glu
           355                 360                 365
   Asn Gln Phe Ser Ile Gly Asp Glu Tyr Thr Val Arg Gly Phe Lys Gly
       370                 375                 380
   Gly Ala Val Ser Gly Asp Ser Gly Ala Tyr Leu Ser Gln Thr Leu Thr
   385                 390                 395                 400
   Val Pro Phe Tyr Pro Gln Lys Ala Tyr Leu Ser Gln Val Ser Pro Phe
                   405                 410                 415
   Ile Gly Phe Asp Met Gly Lys Val His Ile Lys Ser Lys His Lys Thr
               420                 425                 430
   Thr Thr Leu Val Gly Phe Ala Leu Gly Leu Lys Thr Gln Ile Lys Leu
               435                 440                 445
   Phe Ser Leu Ser Leu Thr Tyr Ala Gln Pro Met Asn Gly Val Ser Gly
       450                 455                 460
   Val Thr Gln His Arg Gln Lys Pro Ile Tyr Tyr Phe Ser Gly Ser Leu
   465                 470                 475                 480
   Ser Phe
<210> 21
   <211> 1170
   <212> DNA
   <213> Pasteurella Multocida
<220>
   <221> misc_feature
   <223> greA
<220>
   <221> CDS
   <222> (639) .. (1022)
<400> 21
   gtcaacaaac ggcaaccact tcggcaaaaa gcgattgcgc ttgtgttctg ctctaagctc      60
   aagcgttggg atcgcgcgaa ttgcgttgac cactggtgag atcggggtca cctgtaaaac     120
   gtacgataag atcgccatgc atttcattgt tttttatttt tccattggtt aatagactgg     180
   tttcaaattg aaattggtca cttagtacga gtttggcggt taaggcggtg agcacttttt     240
   gtgtactggc gggtaacata aaggtactgg cttggtgcgc tacaattttt tcattacgat     300
   ttaagttttt agccacaaaa cctaggctgg tcccttcggg taaatgagcg ttgatttcag     360
   caagatcaat ctcagcataa ctgaaatgac tgacgagtaa actacatata agtatcgttc     420
   gtttgaaaag gcgtaaaagc gtggcagtaa aaaaagaaga tattttatac ataattggct     480
   cgagcagttg ctattttttt attgtcgaac aataatagta tttgaaccct cgagagtaaa     540
   tccttttctc gttaaacact tattttttta ttcaactacg gcattgtttt tacaatgttg     600
   tggttttgtt tttatctaaa aaggaagaaa aaacgatt atg aaa cag att cca atg     656
                                             Met Lys Gln Ile Pro Met
                                             1               5
   act ata cgt ggt gcg gaa caa tta aga caa gaa ctc gat ttt ttg aaa     704
   Thr Ile Arg Gly Ala Glu Gln Leu Arg Gln Glu Leu Asp Phe Leu Lys
               10                  15                  20
   aac act cgt cgc cca gaa att att aat gct atc gca gaa gct cgt gaa     752
   Asn Thr Arg Arg Pro Glu Ile Ile Asn Ala Ile Ala Glu Ala Arg Glu
           25                  30                  35
   cat ggc gat cta aaa gaa aat gca gaa tac cat gct gcg cgt gaa cag      800
   His Gly Asp Leu Lys Glu Asn Ala Glu Tyr His Ala Ala Arg Glu Gln
       40                  45                  50
   caa gga ttt tgt gaa gga cga atc caa gaa att gaa ggg aaa tta gcg      848
   Gln Gly Phe Cys Glu Gly Arg Ile Gln Glu Ile Glu Gly Lys Leu Ala
   55                  60                  65                  70
   aat agt caa att att gat gtc aca aag atc cca aat aat ggc aaa gtg      896
   Asn Ser Gln Ile Ile Asp Val Thr Lys Ile Pro Asn Asn Gly Lys Val
                   75                  80                  85
   att ttt ggt gcc aca att ttg tta ctg aat att gac acg gaa gaa gaa      944
   Ile Phe Gly Ala Thr Ile Leu Leu Leu Asn Ile Asp Thr Glu Glu Glu
               90                  95                  100
   gtc tcg tac caa att gta ggc gat gat gaa gcc aat att aaa gca ggg      992
   Val Ser Tyr Gln Ile Val Gly Asp Asp Glu Ala Asn Ile Lys Ala Gly
           105                 110                 115
   cta att tca gtt aac gcc acg cga ttg aat tagagaaagc taaatggatt       1042
   Leu Ile Ser Val Asn Ala Thr Arg Leu Asn
       120                 125
   gcccaagatc ttggcgtcaa acaaacgtta attgacactt ccgtcattaa agcgattacg   1102
   caaaatgcct taatggacga acaggcaaga attgagcaac atggcagtac accgaatact   1162
   ttcgttga                                                             1170
<210> 22
   <211> 128
   <212> PRT
   <213> Pasteurella Multocida
<400> 22
   Met Lys Gln Ile Pro Met Thr Ile Arg Gly Ala Glu Gln Leu Arg Gln
   1               5                   10                  15
   Glu Leu Asp Phe Leu Lys Asn Thr Arg Arg Pro Glu Ile Ile Asn Ala
               20                  25                  30
   Ile Ala Glu Ala Arg Glu His Gly Asp Leu Lys Glu Asn Ala Glu Tyr
           35                  40                  45
   His Ala Ala Arg Glu Gln Gln Gly Phe Cys Glu Gly Arg Ile Gln Glu
       50                  55                  60
   Ile Glu Gly Lys Leu Ala Asn Ser Gln Ile Ile Asp Val Thr Lys Ile
   65                  70                  75                  80
   Pro Asn Asn Gly Lys Val Ile Phe Gly Ala Thr Ile Leu Leu Leu Asn
                   85                  90                  95
   Ile Asp Thr Glu Glu Glu Val Ser Tyr Gln Ile Val Gly Asp Asp Glu
   100 105 110
   Ala Asn Ile Lys Ala Gly Leu Ile Ser Val Asn Ala Thr Arg Leu Asn
   115 120 125
<210> 23
   <211> 4666
   <212> DNA
   <213> Pasteurella Multocida
<220>
   <221> misc_feature
   <223> guaB
<220>
   <221> CDS
   <222> (980) .. (2440)
<400> 23
   aacacatgaa cctcttcccc aaaatacgca tccatatctc gccaatcttg gcgaaatgct 60
   tcatcaattc ctgcttgttc aaaatgttgt aaacgtgcaa tcaacttttt acctaattct 120
   gcgatcaatt tattccgatc aatcgttggt aatacttcaa tcagctctgc ccaaggttga 180
   tcaatttgct gtgtttgttt tgggaaagac aaattaatgc caaagccaat cacgagatta 240
   tgttgattat tctgacgatt ggcgatttcg accaaaatcc ctgctaattt gcgcccatgt 300
   aatagcacat catttggcca ttttaatcca atgttcaaag cacctgcttg ctttagcgtt 360
   tctgcgattg ccatacccac tactaaactc aagccttcta aattgacctt ttggtcacat 420
   gcccaataca aactcataat cacttgtcca gcaaaaggag aaagccattg acgaccacgt 480
   cgtccacgtc ccgcagtttg atattctgct aagcaaatag cgcctttttc caaatgtgca 540
   atattgtcaa gcaagaattg attggtcgag ttaataatcg gcttaatata aagtgggtaa 600
   ggtgctaacg cttgcgtcaa ataagattca tttaagcgac ttaattgagg tatgagacga 660
   aaatgttgga cttgctgttc tatttgtatc ccttgttgtt tcaatttttc gatattgtgt 720
   aagatatctt gttctgaata acctaaaagt gcagtcaatt ctgctaaaga aagttgttga 780
   tagctagcga gtaatgcaag tacgttttgc ataaaaatcc ttatttatat aaccaaagag 840
   aggcaactta ttatagacaa tgattttctc gaaaatcgat aaaaaaatcc attttcaaac 900
   agcaacgaaa tctgtataat gcgaccgcaa tattttttac ccttttattt ttcatatcaa 960
   cctaagagag aatattgca atg tta cga gta ata aaa gaa gca tta acc ttc 1012
   Met Leu Arg Val Ile Lys Glu Ala Leu Thr Phe
   1 5 10
   gat gat gtt ttg ctt gtc cca gca cat tct act gtg ctc cca aat acc      1060
   Asp Asp Val Leu Leu Val Pro Ala His Ser Thr Val Leu Pro Asn Thr
               15                  20                  25
   gca gac ctt tcc act caa ctc acc aaa act atc cgc ctc aat atc cca      1108
   Ala Asp Leu Ser Thr Gln Leu Thr Lys Thr Ile Arg Leu Asn Ile Pro
           30                  35                  40
   atg tta tcc gcc gcc atg gat acc gtg aca gaa act aaa ctg gca atc      1156
   Met Leu Ser Ala Ala Met Asp Thr Val Thr Glu Thr Lys Leu Ala Ile
       45                  50                  55
   tct ctt gca caa gaa ggt ggc atc ggg ttt att cat aaa aat atg tct      1204
   Ser Leu Ala Gln Glu Gly Gly Ile Gly Phe Ile His Lys Asn Met Ser
   60                  65                  70                  75
   att gag cgt caa gcg gaa cgt gtc cgc aaa gtg aaa aaa ttt gag agc      1252
   Ile Glu Arg Gln Ala Glu Arg Val Arg Lys Val Lys Lys Phe Glu Ser
                   80                  85                  90
   ggt att gta tcc gat cct gtc acc gtt tca cca acc tta tct tta gca      1300
   Gly Ile Val Ser Asp Pro Val Thr Val Ser Pro Thr Leu Ser Leu Ala
               95                  100                 105
   gaa tta agt gaa tta gtg aag aaa aat ggt ttt gcg agt ttc cct gtt      1348
   Glu Leu Ser Glu Leu Val Lys Lys Asn Gly Phe Ala Ser Phe Pro Val
           110                 115                 120
   gtt gat gat gaa aaa aat ctt gtc ggt atc att act ggt cgt gat aca      1396
   Val Asp Asp Glu Lys Asn Leu Val Gly Ile Ile Thr Gly Arg Asp Thr
       125                 130                 135
   cgc ttt gtc acg gat tta aat aaa aca gtg gcg gac ttt atg acc cct      1444
   Arg Phe Val Thr Asp Leu Asn Lys Thr Val Ala Asp Phe Met Thr Pro
   140                 145                 150                 155
   aaa gct cgt ctt gtc acg gtg aaa cgc aat gca agt cgc gat gaa att      1492
   Lys Ala Arg Leu Val Thr Val Lys Arg Asn Ala Ser Arg Asp Glu Ile
                   160                 165                 170
   ttt ggt cta atg cat aca cac cgt gta gaa aaa gtc ctt gtt gtc agc      1540
   Phe Gly Leu Met His Thr His Arg Val Glu Lys Val Leu Val Val Ser
               175                 180                 185
   gac gat ttc aaa tta aaa ggc atg atc acc tta aaa gac tac caa aaa      1588
   Asp Asp Phe Lys Leu Lys Gly Met Ile Thr Leu Lys Asp Tyr Gln Lys
           190                 195                 200
   tcc gag caa aaa cca caa gcc tgt aaa gat gaa ttt ggt cgt tta cgt      1636
   Ser Glu Gln Lys Pro Gln Ala Cys Lys Asp Glu Phe Gly Arg Leu Arg
       205                 210                 215
   gtc ggt gct gca gta gga gca gga cct ggt aat gaa gaa cgt att gat      1684
   Val Gly Ala Ala Val Gly Ala Gly Pro Gly Asn Glu Glu Arg Ile Asp
   220                 225                 230                 235
   gca tta gtg aaa gca ggg gtc gat gtg tta ttg att gac tca tca cac      1732
   Ala Leu Val Lys Ala Gly Val Asp Val Leu Leu Ile Asp Ser Ser His
                   240                 245                 250
   ggt cat tca gaa ggt gtg tta caa cgt gtg cgt gaa act cgt gcg aaa      1780
   Gly His Ser Glu Gly Val Leu Gln Arg Val Arg Glu Thr Arg Ala Lys
               255                 260                 265
   tac cca gat ttg cca att gtt gca ggt aat gtg gca acc gct gaa ggc      1828
   Tyr Pro Asp Leu Pro Ile Val Ala Gly Asn Val Ala Thr Ala Glu Gly
           270                 275                 280
   gca att gcg ttg gct gat gca ggg gca agt gca gtg aaa gtg ggg att      1876
   Ala Ile Ala Leu Ala Asp Ala Gly Ala Ser Ala Val Lys Val Gly Ile
       285                 290                 295
   ggt cct ggt tca att tgt aca aca cgt att gtc aca ggc gtg ggc gtt      1924
   Gly Pro Gly Ser Ile Cys Thr Thr Arg Ile Val Thr Gly Val Gly Val
   300                 305                 310                 315
   cca caa att aca gcg att gcc gat gcg gca gaa gca cta aaa gat cgg      1972
   Pro Gln Ile Thr Ala Ile Ala Asp Ala Ala Glu Ala Leu Lys Asp Arg
                   320                 325                 330
   ggt att cct gtg att gca gat ggc ggt atc cgt ttc tct ggt gat att      2020
   Gly Ile Pro Val Ile Ala Asp Gly Gly Ile Arg Phe Ser Gly Asp Ile
               335                 340                 345
   tcg aaa gcc att gcg gcg ggc gcc tct tgt gtt atg gtg ggt tcc atg      2068
   Ser Lys Ala Ile Ala Ala Gly Ala Ser Cys Val Met Val Gly Ser Met
           350                 355                 360
   ttt gca ggt aca gaa gaa gca cca ggt gaa atc gaa ctt tat caa ggt      2116
   Phe Ala Gly Thr Glu Glu Ala Pro Gly Glu Ile Glu Leu Tyr Gln Gly
       365                 370                 375
   cgt gcc ttt aaa tct tat cga ggt atg gga tcg tta ggt gcg atg agc      2164
   Arg Ala Phe Lys Ser Tyr Arg Gly Met Gly Ser Leu Gly Ala Met Ser
   380                 385                 390                 395
   aaa ggc tca agc gac cgc tat ttc cag tcc gat aat gca gct gac aaa      2212
   Lys Gly Ser Ser Asp Arg Tyr Phe Gln Ser Asp Asn Ala Ala Asp Lys
                   400                 405                 410
   tta gta cca gaa ggt att gaa gga cgt att cca tat aaa gga ttc tta      2260
   Leu Val Pro Glu Gly Ile Glu Gly Arg Ile Pro Tyr Lys Gly Phe Leu
               415                 420                 425
   aaa gaa att atc cat caa caa atg ggt gga ttg cgt tct tgt atg ggc      2308
   Lys Glu Ile Ile His Gln Gln Met Gly Gly Leu Arg Ser Cys Met Gly
           430                 435                 440
   tta acg ggt tgt gca acc att gat gaa ctc cgt acc aaa gcg cag ttt      2356
   Leu Thr Gly Cys Ala Thr Ile Asp Glu Leu Arg Thr Lys Ala Gln Phe
       445                 450                 455
   gtg cgc att agt ggt gca ggg atc caa gaa agc cat gtg cat gat gtg      2404
   Val Arg Ile Ser Gly Ala Gly Ile Gln Glu Ser His Val His Asp Val
   460                 465                 470                 475
   act atc aca aaa gaa gcc cct aat tat cgt atg ggt taaacattgc           2450
   Thr Ile Thr Lys Glu Ala Pro Asn Tyr Arg Met Gly
                   480                 485
   ttaggtgggg attatcccca cctaagttta ttttaaataa caacgttaat agagaagctt    2510
   atttttatgt atggcattaa aattaaaaat gttattaaac tttttctatt aaagttttta 2570
   agaaataaat atcgatataa aatcaatatt caacatcatc tcattagtat tgaaggaaaa 2630
   tgcggtgagt ttgatttaag ccagctaaac tatgtttatt tggtgaaaga tcctgagata 2690
   agaaataatc gactgacact gtacttgaat gattttttca aaattggggt aaattatcat 2750
   ggatttactc aaatgtatca gacactatca tccaagtatg gttttgatga cgcaacgttt 2810
   tttgaatatc tttgtaagaa agggcctttt tctattcaaa tttggcgtaa aaaacaaact 2870
   caaaattatg tgattcttga tgaagcctat actgactata cacaaggttt tgaaattcaa 2930
   tctcctgaaa aaatatttat tccttggggg actacttatg aagccttatt tcagcagaca 2990
   caatttaaag aaaaaggaat ctcttatgac tttatcttcc ctattcggat agggcgttta 3050
   ttactcaagg atgtgtggat cacaccaagt gtccgaaaag atgtaccgat tttagcgtta 3110
   tacactgagt gctatcatga atccgcgaca gaaaaaagtt atcaggaatt aaccgccgca 3170
   ttacgtgaga accaacagtt aatcagatca tgtgttgaag aacgagccga tccgaaatta 3230
   tataagtctg ttttacgcct taacgcgaca gaatttgaat tgcgttatta tcgacatata 3290
   cgagacgatt ttgatagggg atacactaaa ttcagcatta gagatacgac agactattta 3350
   gattatgtga tcaacgagcc ttatgaaaat caattagtga taacggatta tttagtgatt 3410
   gaggcgcaaa atttaataaa aatggattat accgataatt ccattattaa acgccgacca 3470
   ccaaaaataa aagaaaagtt tcgtgatgca caaagcctga tttggacaga tgatctcaat 3530
   cataaaatcg gttttaccag tgatgaccgc gctatcgtct ttgataaagc ggacattgaa 3590
   tcctttactc tggcaaatat agagacaacc cgaagacata atcgcagttc actcagcatc 3650
   tgttttgtgg ataaaaataa ggaagccatc actgtatttc tagctgaaca tcattttctg 3710
   ataccctatg tggataaaat aaaaacactg acacaaaaag aggtgttgtt tcttgaagaa 3770
   tatatagagg atgtttaaaa aaccgataac atcttgatgt catcgcaaat tcaaactcct 3830
   tttacacaat atatttaaac tttaacccga tttaatattt acgtaaaaac aactaagaga 3890
   acttaaatga acaacattca caaccataaa attttaattt tggacttcgg ttcacaatat 3950
   acccagttga ttgcccgccg tgtacgtgaa attggcgtgt actgcgaact ttgggcatgg 4010
   gatgtatccg aagccgatat tcgtgagttt aatccaactg ggattattct ttctggtggt 4070
   cctgaaagta ccactgaaga aaacagccca cgagctcccg aatacgtatt caacgccggt 4130
   gtacccgtat tagggatctg ttatggtatg caaaccatgg cgatgcaact aggtggttta 4190
   actgaaactt ctactcaccg agaatttggt tatgcatcag tgaatctaaa agccgcggac 4250
   gcgttatttg ctcaattaaa tgatgatgtc gcaagttcac agcccaaatt agacgtttgg 4310
   atgagccatg gcgataaagt gacgcgtttg ccagatcatt tccaagtcac cgcaatgacc 4370
   tcaacctgtc caatcgcagc tatgtcggat gaacgtcgtc gtttttatgg tgtccaattc    4430
   cacccagaag tgactcatac aaaaagcggg cttgaattat taacgaattt tgtggtgaag    4490
   atctgtggtt gtgaacgtaa ctggacacca gaaaatatca ttgaagatgc cgttgctcgt    4550
   cttaaagcac aagtgggcga tgatgaagtg attttaggct tatctggtgg cgttaactgc    4610
   cgtataggca gcttagaaaa agtgtccgcg ctcacgttaa tccccgtaag cgttga        4666
<210> 24
   <211> 487
   <212> PRT
   <213> Pasteurella Multocida
<400> 24
   Met Leu Arg Val Ile Lys Glu Ala Leu Thr Phe Asp Asp Val Leu Leu
   1               5                   10                  15
   Val Pro Ala His Ser Thr Val Leu Pro Asn Thr Ala Asp Leu Ser Thr
               20                  25                  30
   Gln Leu Thr Lys Thr Ile Arg Leu Asn Ile Pro Met Leu Ser Ala Ala
           35                  40                  45
   Met Asp Thr Val Thr Glu Thr Lys Leu Ala Ile Ser Leu Ala Gln Glu
       50                  55                  60
   Gly Gly Ile Gly Phe Ile His Lys Asn Met Ser Ile Glu Arg Gln Ala
   65                  70                  75                  80
   Glu Arg Val Arg Lys Val Lys Lys Phe Glu Ser Gly Ile Val Ser Asp
                   85                  90                  95
   Pro Val Thr Val Ser Pro Thr Leu Ser Leu Ala Glu Leu Ser Glu Leu
               100                 105                 110
   Val Lys Lys Asn Gly Phe Ala Ser Phe Pro Val Val Asp Asp Glu Lys
           115                 120                 125
   Asn Leu Val Gly Ile Ile Thr Gly Arg Asp Thr Arg Phe Val Thr Asp
       130                 135                 140
   Leu Asn Lys Thr Val Ala Asp Phe Met Thr Pro Lys Ala Arg Leu Val
   145                 150                 155                 160
   Thr Val Lys Arg Asn Ala Ser Arg Asp Glu Ile Phe Gly Leu Met His
                   165                 170                 175
   Thr His Arg Val Glu Lys Val Leu Val Val Ser Asp Asp Phe Lys Leu
   180 185 190
   Lys Gly Met Ile Thr Leu Lys Asp Tyr Gln Lys Ser Glu Gln Lys Pro
   195 200 205
   Gln Ala Cys Lys Asp Glu Phe Gly Arg Leu Arg Val Gly Ala Ala Val
   210 215 220
   Gly Ala Gly Pro Gly Asn Glu Glu Arg Ile Asp Ala Leu Val Lys Ala
   225 230 235 240
   Gly Val Asp Val Leu Leu Ile Asp Ser Ser His Gly His Ser Glu Gly
   245 250 255
   Val Leu Gln Arg Val Arg Glu Thr Arg Ala Lys Tyr Pro Asp Leu Pro
   260 265 270
   Ile Val Ala Gly Asn Val Ala Thr Ala Glu Gly Ala Ile Ala Leu Ala
   275 280 285
   Asp Ala Gly Ala Ser Ala Val Lys Val Gly Ile Gly Pro Gly Ser Ile
   290 295 300
   Cys Thr Thr Arg Ile Val Thr Gly Val Gly Val Pro Gln Ile Thr Ala
   305 310 315 320
   Ile Ala Asp Ala Ala Glu Ala Leu Lys Asp Arg Gly Ile Pro Val Ile
   325 330 335
   Ala Asp Gly Gly Ile Arg Phe Ser Gly Asp Ile Ser Lys Ala Ile Ala
   340 345 350
   Ala Gly Ala Ser Cys Val Met Val Gly Ser Met Phe Ala Gly Thr Glu
   355 360 365
   Glu Ala Pro Gly Glu Ile Glu Leu Tyr Gln Gly Arg Ala Phe Lys Ser
   370 375 380
   Tyr Arg Gly Met Gly Ser Leu Gly Ala Met Ser Lys Gly Ser Ser Asp
   385 390 395 400
   Arg Tyr Phe Gln Ser Asp Asn Ala Ala Asp Lys Leu Val Pro Glu Gly
   405 410 415
   Ile Glu Gly Arg Ile Pro Tyr Lys Gly Phe Leu Lys Glu Ile Ile His
               420                 425                 430
   Gln Gln Met Gly Gly Leu Arg Ser Cys Met Gly Leu Thr Gly Cys Ala
           435                 440                 445
   Thr Ile Asp Glu Leu Arg Thr Lys Ala Gln Phe Val Arg Ile Ser Gly
       450                 455                 460
   Ala Gly Ile Gln Glu Ser His Val His Asp Val Thr Ile Thr Lys Glu
   465                 470                 475                 480
   Ala Pro Asn Tyr Arg Met Gly
                   485
<210> 25
   <211> 2364
   <212> DNA
   <213> Pasteurella Multocida
<220>
   <221> misc_feature
   <223> Hi1501
<220>
   <221> CDS
   <222> (191) .. (1828)
<400> 25
   gtcaacactc atcgcacagc tgaggcattt cccgaaagct gatcatgatg atggacctga      60
   tgcgctagag atgctgtgga aaaatgcggt aagcagctct gccccgattg agttcatgac     120
   aattgacggc gatttagggc gtgatgaatt tgatgacggc gatttataca gtatttggcg     180
   gagataaaaa atg gcg aag aaa aag aaa aaa tta caa caa gcg aaa aaa       229
              Met Ala Lys Lys Lys Lys Lys Leu Gln Gln Ala Lys Lys
              1               5                   10
   gta caa gtt ggc tta gat aca caa aca aat gag gcg cgt gtc acg gag      277
   Val Gln Val Gly Leu Asp Thr Gln Thr Asn Glu Ala Arg Val Thr Glu
       15                  20                  25
   aca gga aga att att tct gat cac cca agc aat aaa att acc ccc gca      325
   Thr Gly Arg Ile Ile Ser Asp His Pro Ser Asn Lys Ile Thr Pro Ala
   30                  35                  40                  45
   aag tta aaa ggg att tta gaa gat gct gaa ggt ggt gat att acc gcg      373
   Lys Leu Lys Gly Ile Leu Glu Asp Ala Glu Gly Gly Asp Ile Thr Ala
                   50                  55                  60
   caa cat gag ctt ttc atg gat att gaa gaa cgc gac agt tgc atc ggg      421
   Gln His Glu Leu Phe Met Asp Ile Glu Glu Arg Asp Ser Cys Ile Gly
               65                  70                  75
   gca aat att caa acc cgt aag cgt gcg att tta acc ctt gac tgg cgc      469
   Ala Asn Ile Gln Thr Arg Lys Arg Ala Ile Leu Thr Leu Asp Trp Arg
           80                  85                  90
   att gca gag cca cgt aat gcc aca ccg caa gaa gaa aaa ctg caa gtc      517
   Ile Ala Glu Pro Arg Asn Ala Thr Pro Gln Glu Glu Lys Leu Gln Val
       95                  100                 105
   gaa att gac gag ctt ttc tat caa ttc cca atg cta gaa gat tta atg      565
   Glu Ile Asp Glu Leu Phe Tyr Gln Phe Pro Met Leu Glu Asp Leu Met
   110                 115                 120                 125
   gtg gat atg atg gat gcg gta gga cat ggt ttt tcg gcg tta gaa att      613
   Val Asp Met Met Asp Ala Val Gly His Gly Phe Ser Ala Leu Glu Ile
                   130                 135                 140
   gaa tgg aag caa gct gaa agt aaa tgg att cca gtt aat ttt atc gca      661
   Glu Trp Lys Gln Ala Glu Ser Lys Trp Ile Pro Val Asn Phe Ile Ala
               145                 150                 155
   cgt ccg cag tcg tgg ttt aaa cta gac aag gat gat aat tta ctg ctt      709
   Arg Pro Gln Ser Trp Phe Lys Leu Asp Lys Asp Asp Asn Leu Leu Leu
           160                 165                 170
   aaa acg cca gat aat caa gac ggt gag ccg ttg aga caa tat ggc tgg      757
   Lys Thr Pro Asp Asn Gln Asp Gly Glu Pro Leu Arg Gln Tyr Gly Trp
       175                 180                 185
   gta gtg cat acc cac aaa tca aga aca gta cag ctt gct cgt atg ggt      805
   Val Val His Thr His Lys Ser Arg Thr Val Gln Leu Ala Arg Met Gly
   190                 195                 200                 205
   tta ttt aga acg ctc gca tgg ctt tat atg ttt aaa cac tac tcg gtg      853
   Leu Phe Arg Thr Leu Ala Trp Leu Tyr Met Phe Lys His Tyr Ser Val
                   210                 215                 220
   cat gat ttt gcc gaa ttt cta gag ctt tat ggt atg ccg att cgt att      901
   His Asp Phe Ala Glu Phe Leu Glu Leu Tyr Gly Met Pro Ile Arg Ile
               225                 230                 235
   ggt aaa tac cca ttt ggg gca acg aat gac gaa aag cgc aca tta ttg      949
   Gly Lys Tyr Pro Phe Gly Ala Thr Asn Asp Glu Lys Arg Thr Leu Leu
           240                 245                 250
   cgt gca ctt gct caa atc gga cat aac gca gca ggg att atg cca gaa      997
   Arg Ala Leu Ala Gln Ile Gly His Asn Ala Ala Gly Ile Met Pro Glu
       255                 260                 265
   gga atg aat gtt gag ttg cat aat gtg aca aac act act ggc tcg gct     1045
   Gly Met Asn Val Glu Leu His Asn Val Thr Asn Thr Thr Gly Ser Ala
   270                 275                 280                 285
   gga agc aac ccg ttt ttg caa atg gtg gac tgg tgt gaa aag tcc gcc     1093
   Gly Ser Asn Pro Phe Leu Gln Met Val Asp Trp Cys Glu Lys Ser Ala
                   290                 295                 300
   gca cgt ttg att cta ggg caa aca tta aca agc ggt gca gat ggt aaa     1141
   Ala Arg Leu Ile Leu Gly Gln Thr Leu Thr Ser Gly Ala Asp Gly Lys
               305                 310                 315
   act tca act aat gcc ctt gga caa gtg cat aat gaa gtc aga cgt gac      1189
   Thr Ser Thr Asn Ala Leu Gly Gln Val His Asn Glu Val Arg Arg Asp
           320                 325                 330
   ttg ctt gtg tct gat gct aaa cag att gca caa act att aca caa cag      1237
   Leu Leu Val Ser Asp Ala Lys Gln Ile Ala Gln Thr Ile Thr Gln Gln
       335                 340                 345
   att att ctg cca tat ctt caa att aac att gat ccg aat att ttg cct      1285
   Ile Ile Leu Pro Tyr Leu Gln Ile Asn Ile Asp Pro Asn Ile Leu Pro
   350                 355                 360                 365
   tct cgt gtg ccg tat ttc gag ttt gac acg aaa gaa tat gct gat tta      1333
   Ser Arg Val Pro Tyr Phe Glu Phe Asp Thr Lys Glu Tyr Ala Asp Leu
                   370                 375                 380
   agt gtc cta gcg gat gct att cct aag ctt gtg agc gta gga gtg cgc      1381
   Ser Val Leu Ala Asp Ala Ile Pro Lys Leu Val Ser Val Gly Val Arg
               385                 390                 395
   att cct gaa aat tgg gtg cgt gat aaa gcg ggc att cca gaa ccg cag      1429
   Ile Pro Glu Asn Trp Val Arg Asp Lys Ala Gly Ile Pro Glu Pro Gln
           400                 405                 410
   gaa aat gaa acg att tta agt gcg gtt caa cat gat ttt aaa aca gat      1477
   Glu Asn Glu Thr Ile Leu Ser Ala Val Gln His Asp Phe Lys Thr Asp
       415                 420                 425
   tta aac gat gtt gaa aat ccg aaa aaa cag acc gca ctt tct gta caa      1525
   Leu Asn Asp Val Glu Asn Pro Lys Lys Gln Thr Ala Leu Ser Val Gln
   430                 435                 440                 445
   aat cac gtg aca ggt tgt cag tgt gat ggc tgt cgt ggt gtt gca tta      1573
   Asn His Val Thr Gly Cys Gln Cys Asp Gly Cys Arg Gly Val Ala Leu
                   450                 455                 460
   tct gcg aat aat aac agt tct act gcg cag ggc gtg cta gat ggt gga      1621
   Ser Ala Asn Asn Asn Ser Ser Thr Ala Gln Gly Val Leu Asp Gly Gly
               465                 470                 475
   ctt gcg caa gca ttt aat gag cct gat ttt aat aaa caa tta aat cca      1669
   Leu Ala Gln Ala Phe Asn Glu Pro Asp Phe Asn Lys Gln Leu Asn Pro
           480                 485                 490
   atg gta aag aaa gct gtt gcg gta ctc atg gca tgt gac tct tac gat      1717
   Met Val Lys Lys Ala Val Ala Val Leu Met Ala Cys Asp Ser Tyr Asp
       495                 500                 505
   gag gcg gca gaa aaa ctc gct gaa gca tac cca gaa att tca agt cac      1765
   Glu Ala Ala Glu Lys Leu Ala Glu Ala Tyr Pro Glu Ile Ser Ser His
   510                 515                 520                 525
   gaa cac gaa cag tat ctc tca aat gcg ctg ttt tta gct gat tta ctt      1813
   Glu His Glu Gln Tyr Leu Ser Asn Ala Leu Phe Leu Ala Asp Leu Leu
                   530                 535                 540
   gga gga act aat gtc taaaccgctt agttttctat tcggacttga accaacgcaa      1868
   Gly Gly Thr Asn Val
               545
   gccattgagt ttttacataa taaaaaatta cttgcaacga aagtgtttaa aaaatcactg   1928
   catgatagtg ccatcgcaag agctacaaca atcgcgagat tatctagtct tgagatgacg   1988
   aatgatattt ataaatcaat ggaagttgcc aaaaaagagg gtaagagctt tacacaatgg   2048
   aaaaaagact tggtaagtga gtttgagaaa aaaggctggg tattcgggca tgataaatct   2108
   atcagtcgcg gtatcgacgg aaaactgttg gctgatccga aaacaggcga atattttggt   2168
   acaccgcgtc ggctgaatac aatttatcgt acaaacgtgc aagccgcata ttctgcggcg   2228
   cgctatcagc gcatgatgga taatattgat catcgcccct attggcaata ttccgctgtc   2288
   agcgatgagc gtacacgacc ctctcatctt gcactaaacg gtcgaattta tcgctatgat   2348
   gacccgtttt ggtcga                                                   2364
<210> 26
   <211> 546
   <212> PRT
   <213> Pasteurella Multocida
<400> 26
   Met Ala Lys Lys Lys Lys Lys Leu Gln Gln Ala Lys Lys Val Gln Val
   1               5                   10                  15
   Gly Leu Asp Thr Gln Thr Asn Glu Ala Arg Val Thr Glu Thr Gly Arg
               20                  25                  30
   Ile Ile Ser Asp His Pro Ser Asn Lys Ile Thr Pro Ala Lys Leu Lys
           35                  40                  45
   Gly Ile Leu Glu Asp Ala Glu Gly Gly Asp Ile Thr Ala Gln His Glu
       50                  55                  60
   Leu Phe Met Asp Ile Glu Glu Arg Asp Ser Cys Ile Gly Ala Asn Ile
   65                  70                  75                  80
   Gln Thr Arg Lys Arg Ala Ile Leu Thr Leu Asp Trp Arg Ile Ala Glu
                   85                  90                  95
   Pro Arg Asn Ala Thr Pro Gln Glu Glu Lys Leu Gln Val Glu Ile Asp
               100                 105                 110
   Glu Leu Phe Tyr Gln Phe Pro Met Leu Glu Asp Leu Met Val Asp Met
           115                 120                 125
   Met Asp Ala Val Gly His Gly Phe Ser Ala Leu Glu Ile Glu Trp Lys
       130                 135                 140
   Gln Ala Glu Ser Lys Trp Ile Pro Val Asn Phe Ile Ala Arg Pro Gln
   145 150 155 160
   Ser Trp Phe Lys Leu Asp Lys Asp Asp Asn Leu Leu Leu Lys Thr Pro
   165 170 175
   Asp Asn Gln Asp Gly Glu Pro Leu Arg Gln Tyr Gly Trp Val Val His
   180 185 190
   Thr His Lys Ser Arg Thr Val Gln Leu Ala Arg Met Gly Leu Phe Arg
   195 200 205
   Thr Leu Ala Trp Leu Tyr Met Phe Lys His Tyr Ser Val His Asp Phe
   210 215 220
   Ala Glu Phe Leu Glu Leu Tyr Gly Met Pro Ile Arg Ile Gly Lys Tyr
   225 230 235 240
   Pro Phe Gly Ala Thr Asn Asp Glu Lys Arg Thr Leu Leu Arg Ala Leu
   245 250 255
   Ala Gln Ile Gly His Asn Ala Ala Gly Ile Met Pro Glu Gly Met Asn
   260 265 270
   Val Glu Leu His Asn Val Thr Asn Thr Thr Gly Ser Ala Gly Ser Asn
   275 280 285
   Pro Phe Leu Gln Met Val Asp Trp Cys Glu Lys Ser Ala Ala Arg Leu
   290 295 300
   Ile Leu Gly Gln Thr Leu Thr Ser Gly Ala Asp Gly Lys Thr Ser Thr
   305 310 315 320
   Asn Ala Leu Gly Gln Val His Asn Glu Val Arg Arg Asp Leu Leu Val
   325 330 335
   Ser Asp Ala Lys Gln Ile Ala Gln Thr Ile Thr Gln Gln Ile Ile Leu
   340 345 350
   Pro Tyr Leu Gln Ile Asn Ile Asp Pro Asn Ile Leu Pro Ser Arg Val
   355 360 365
   Pro Tyr Phe Glu Phe Asp Thr Lys Glu Tyr Ala Asp Leu Ser Val Leu
   370 375 380
   Ala Asp Ala Ile Pro Lys Leu Val Ser Val Gly Val Arg Ile Pro Glu
   385                 390                 395                 400
   Asn Trp Val Arg Asp Lys Ala Gly Ile Pro Glu Pro Gln Glu Asn Glu
                   405                 410                 415
   Thr Ile Leu Ser Ala Val Gln His Asp Phe Lys Thr Asp Leu Asn Asp
               420                 425                 430
   Val Glu Asn Pro Lys Lys Gln Thr Ala Leu Ser Val Gln Asn His Val
           435                 440                 445
   Thr Gly Cys Gln Cys Asp Gly Cys Arg Gly Val Ala Leu Ser Ala Asn
       450                 455                 460
   Asn Asn Ser Ser Thr Ala Gln Gly Val Leu Asp Gly Gly Leu Ala Gln
   465                 470                 475                 480
   Ala Phe Asn Glu Pro Asp Phe Asn Lys Gln Leu Asn Pro Met Val Lys
                   485                 490                 495
   Lys Ala Val Ala Val Leu Met Ala Cys Asp Ser Tyr Asp Glu Ala Ala
               500                 505                 510
   Glu Lys Leu Ala Glu Ala Tyr Pro Glu Ile Ser Ser His Glu His Glu
           515                 520                 525
   Gln Tyr Leu Ser Asn Ala Leu Phe Leu Ala Asp Leu Leu Gly Gly Thr
       530                 535                 540
   Asn Val
   545
<210> 27
   <211> 1353
   <212> DNA
   <213> Pasteurella Multocida
<220>
   <221> misc_feature
   <223> hmbR
<220>
   <221> CDS
   <222> (2)..(1351)
<220>
   <221> misc_feature
   <222> (375) .. (375)
   <223> n is a, c, g, or t
<220>
   <221> misc_feature
   <222> (399) .. (399)
   <223> n is a, c, g, or t
<220>
   <221> misc_feature
   <222> (423) .. (423)
   <223> n is a, c, g, or t
<220>
   <221> misc_feature
   <222> (453) .. (453)
   <223> n is a, c, g, or t
<400> 27
   g tca acg aaa gtc ggt tac gat att aat aac act cat cgt ttt aca ctg      49
     Ser Thr Lys Val Gly Tyr Asp Ile Asn Asn Thr His Arg Phe Thr Leu
     1               5                   10                  15
   ttt tta gaa gat cgc cgt gaa aag aag ctt acc gaa gaa aaa aca tta        97
   Phe Leu Glu Asp Arg Arg Glu Lys Lys Leu Thr Glu Glu Lys Thr Leu
               20                  25                  30
   ggg ctt agt gat gca gtg cgt ttt gct aat gat caa acc cct tat ctc       145
   Gly Leu Ser Asp Ala Val Arg Phe Ala Asn Asp Gln Thr Pro Tyr Leu
           35                  40                  45
   cgt tat ggt att gaa tat cga tat aac ggc ttg tct tgg ttg gaa acg       193
   Arg Tyr Gly Ile Glu Tyr Arg Tyr Asn Gly Leu Ser Trp Leu Glu Thr
       50                  55                  60
   gta aag ctt ttt ttg gca aag cag aaa atc gaa caa cgt tct gct ctc       241
   Val Lys Leu Phe Leu Ala Lys Gln Lys Ile Glu Gln Arg Ser Ala Leu
   65                  70                  75                  80
   caa gag ttt gat att aat aat agg aat aaa ttg gat tcg act atg tcg       289
   Gln Glu Phe Asp Ile Asn Asn Arg Asn Lys Leu Asp Ser Thr Met Ser
                   85                  90                  95
   ttt gta tat tta caa aga cag aat ata gct cgg gga gaa ttt tca acg       337
   Phe Val Tyr Leu Gln Arg Gln Asn Ile Ala Arg Gly Glu Phe Ser Thr
               100                 105                 110
   agt cct tta tat tgg ggg ccg agt cgc cat cgt tta tnt gcg aaa ttc       385
   Ser Pro Leu Tyr Trp Gly Pro Ser Arg His Arg Leu Xaa Ala Lys Phe
           115                 120                 125
   gaa ttt cgt gat ang ttt tta gaa aat atg aat aag cnt ttt acg ttt       433
   Glu Phe Arg Asp Xaa Phe Leu Glu Asn Met Asn Lys Xaa Phe Thr Phe
       130                 135                 140
   cgg ccg tgg caa atc aat ana ttc aga caa caa ggt cga aat aac tat       481
   Arg Pro Trp Gln Ile Asn Xaa Phe Arg Gln Gln Gly Arg Asn Asn Tyr
   145                 150                 155                 160
   aca gaa gtg ttt ccc gtt aaa tcc cga gag ttt tct ttt tct ctt atg       529
   Thr Glu Val Phe Pro Val Lys Ser Arg Glu Phe Ser Phe Ser Leu Met
                   165                 170                 175
   gac gac att aag att ggc gaa ttg cta cat ctc gga ttg ggc ggt cgg      577
   Asp Asp Ile Lys Ile Gly Glu Leu Leu His Leu Gly Leu Gly Gly Arg
               180                 185                 190
   tgg gat cac tat aac tat aag cca tta tta aat tct cag cat aat atc      625
   Trp Asp His Tyr Asn Tyr Lys Pro Leu Leu Asn Ser Gln His Asn Ile
           195                 200                 205
   aac agg aca cag aga tta cct tat cca aaa aca tca tcc aaa ttt tcg      673
   Asn Arg Thr Gln Arg Leu Pro Tyr Pro Lys Thr Ser Ser Lys Phe Ser
       210                 215                 220
   tat caa ttg agt tta gag tat caa tta cat cca tca cat caa att gca      721
   Tyr Gln Leu Ser Leu Glu Tyr Gln Leu His Pro Ser His Gln Ile Ala
   225                 230                 235                 240
   tac cgt tta agt acc ggt ttt agg gtt ccc cgt gtt gaa gat ctt tat      769
   Tyr Arg Leu Ser Thr Gly Phe Arg Val Pro Arg Val Glu Asp Leu Tyr
                   245                 250                 255
   ttt gaa gac cga gga aaa agt tct tca caa ttt ctt cct aac ccc gat      817
   Phe Glu Asp Arg Gly Lys Ser Ser Ser Gln Phe Leu Pro Asn Pro Asp
               260                 265                 270
   cta caa ccg gaa act gca ctg aat cat gaa ata agt tac cgt ttc caa      865
   Leu Gln Pro Glu Thr Ala Leu Asn His Glu Ile Ser Tyr Arg Phe Gln
           275                 280                 285
   aat caa tat gcc cat ttc agc gtc ggg ctt ttc cgt aca cgt tat cat      913
   Asn Gln Tyr Ala His Phe Ser Val Gly Leu Phe Arg Thr Arg Tyr His
       290                 295                 300
   aac ttt att caa gaa cgt gag atg acc tgt gat aaa att cca tat gag      961
   Asn Phe Ile Gln Glu Arg Glu Met Thr Cys Asp Lys Ile Pro Tyr Glu
   305                 310                 315                 320
   tat aat agg act tat gga tat tgc acg cat aat act tat gta atg ttt     1009
   Tyr Asn Arg Thr Tyr Gly Tyr Cys Thr His Asn Thr Tyr Val Met Phe
                   325                 330                 335
   gtt aat gaa cct gaa gcc gtg att aaa ggg gtt gaa gta agc ggt gct     1057
   Val Asn Glu Pro Glu Ala Val Ile Lys Gly Val Glu Val Ser Gly Ala
               340                 345                 350
   tta aat ggg tcg gca ttc gga ctt tcc gac ggt tta act ttc cgt ctc     1105
   Leu Asn Gly Ser Ala Phe Gly Leu Ser Asp Gly Leu Thr Phe Arg Leu
           355                 360                 365
   aaa ggg agc tac agc aaa ggt caa aat cat gac ggc gat ccg tta aaa     1153
   Lys Gly Ser Tyr Ser Lys Gly Gln Asn His Asp Gly Asp Pro Leu Lys
       370                 375                 380
   tct att caa cca tgg aca gtg gta acc ggt att gat tac gaa act gaa     1201
   Ser Ile Gln Pro Trp Thr Val Val Thr Gly Ile Asp Tyr Glu Thr Glu
   385                 390                 395                 400
   ggg tgg agc gtg agt ttg agc ggg cgt tat agt gcg gct aaa aaa gcc     1249
   Gly Trp Ser Val Ser Leu Ser Gly Arg Tyr Ser Ala Ala Lys Lys Ala
                   405                 410                 415
   aaa gat gcg ata gaa acg gaa tac aca cat gat aaa aag gtt gtc aaa     1297
   Lys Asp Ala Ile Glu Thr Glu Tyr Thr His Asp Lys Lys Val Val Lys
               420                 425                 430
   caa tgg ccg cat tta agt cca tcc tac ttt gtt gtt gat ttt acg ggg      1345
   Gln Trp Pro His Leu Ser Pro Ser Tyr Phe Val Val Asp Phe Thr Gly
           435                 440                 445
   caa gtt ga                                                           1353
   Gln Val
       450
<210> 28
   <211> 450
   <212> PRT
   <213> Pasteurella Multocida
<220>
   <221> misc_feature
   <222> (125) .. (125)
   <223> The 'Xaa' at location 125 stands for Tyr, Cys, Ser, or Phe.
<220>
   <221> misc_feature
   <222> (133) .. (133)
   <223> The 'Xaa' at location 133 stands for Lys, Arg, Thr, or Met.
<220>
   <221> misc_feature
   <222> (141) .. (141)
   <223> The 'Xaa' at location 141 stands for His, Arg, Pro, or Leu.
<220>
   <221> misc_feature
   <222> (151) .. (151)
   <223> The 'Xaa' at location 151 stands for Lys, Arg, Thr, or Ile.
<400> 28
   Ser Thr Lys Val Gly Tyr Asp Ile Asn Asn Thr His Arg Phe Thr Leu
   1               5                   10                  15
   Phe Leu Glu Asp Arg Arg Glu Lys Lys Leu Thr Glu Glu Lys Thr Leu
               20                  25                  30
   Gly Leu Ser Asp Ala Val Arg Phe Ala Asn Asp Gln Thr Pro Tyr Leu
           35                  40                  45
   Arg Tyr Gly Ile Glu Tyr Arg Tyr Asn Gly Leu Ser Trp Leu Glu Thr
       50                  55                  60
   Val Lys Leu Phe Leu Ala Lys Gln Lys Ile Glu Gln Arg Ser Ala Leu
   65                  70                  75                  80
   Gln Glu Phe Asp Ile Asn Asn Arg Asn Lys Leu Asp Ser Thr Met Ser
                   85                  90                  95
   Phe Val Tyr Leu Gln Arg Gln Asn Ile Ala Arg Gly Glu Phe Ser Thr
               100                 105                 110
   Ser Pro Leu Tyr Trp Gly Pro Ser Arg His Arg Leu Xaa Ala Lys Phe
           115                 120                 125
   Glu Phe Arg Asp Xaa Phe Leu Glu Asn Met Asn Lys Xaa Phe Thr Phe
       130                 135                 140
   Arg Pro Trp Gln Ile Asn Xaa Phe Arg Gln Gln Gly Arg Asn Asn Tyr
   145                 150                 155                 160
   Thr Glu Val Phe Pro Val Lys Ser Arg Glu Phe Ser Phe Ser Leu Met
                   165                 170                 175
   Asp Asp Ile Lys Ile Gly Glu Leu Leu His Leu Gly Leu Gly Gly Arg
               180                 185                 190
   Trp Asp His Tyr Asn Tyr Lys Pro Leu Leu Asn Ser Gln His Asn Ile
           195                 200                 205
   Asn Arg Thr Gln Arg Leu Pro Tyr Pro Lys Thr Ser Ser Lys Phe Ser
       210                 215                 220
   Tyr Gln Leu Ser Leu Glu Tyr Gln Leu His Pro Ser His Gln Ile Ala
   225                 230                 235                 240
   Tyr Arg Leu Ser Thr Gly Phe Arg Val Pro Arg Val Glu Asp Leu Tyr
                   245                 250                 255
   Phe Glu Asp Arg Gly Lys Ser Ser Ser Gln Phe Leu Pro Asn Pro Asp
               260                 265                 270
   Leu Gln Pro Glu Thr Ala Leu Asn His Glu Ile Ser Tyr Arg Phe Gln
           275                 280                 285
   Asn Gln Tyr Ala His Phe Ser Val Gly Leu Phe Arg Thr Arg Tyr His
       290                 295                 300
   Asn Phe Ile Gln Glu Arg Glu Met Thr Cys Asp Lys Ile Pro Tyr Glu
   305                 310                 315                 320
   Tyr Asn Arg Thr Tyr Gly Tyr Cys Thr His Asn Thr Tyr Val Met Phe
                   325                 330                 335
   Val Asn Glu Pro Glu Ala Val Ile Lys Gly Val Glu Val Ser Gly Ala
           340                 345                 350
   Leu Asn Gly Ser Ala Phe Gly Leu Ser Asp Gly Leu Thr Phe Arg Leu
       355                 360                 365
   Lys Gly Ser Tyr Ser Lys Gly Gln Asn His Asp Gly Asp Pro Leu Lys
       370                 375                 380
   Ser Ile Gln Pro Trp Thr Val Val Thr Gly Ile Asp Tyr Glu Thr Glu
   385                 390                 395                 400
   Gly Trp Ser Val Ser Leu Ser Gly Arg Tyr Ser Ala Ala Lys Lys Ala
               405                 410                 415
   Lys Asp Ala Ile Glu Thr Glu Tyr Thr His Asp Lys Lys Val Val Lys
           420                 425                 430
   Gln Trp Pro His Leu Ser Pro Ser Tyr Phe Val Val Asp Phe Thr Gly
       435                 440                 445
   Gln Val
       450
<210> 29
   <211> 4936
   <212> DNA
   <213> Pasteurella Multocida
<220>
   <221> misc_feature
   <223> hxuC
<220>
   <221> CDS
   <222> (1078) .. (2769)
<400> 29
   gtcaacaaca aagcgcacag gcattacttc atgccacaca catcatacag aaagtacgta      60
   ccgatttaac gcaaattaat gccgtcaaca ttcatctttt cctatcataa agcgtttcat     120
   catggctagc attctagcaa aaattagttg aggaaaatag cggtcttgtt ttgcttaaaa     180
   aacaacccac cccgtagggc acggctgttt ctttttgaga aattacgctt cttcatcttg     240
   atcttttttc aagatctcat cttcattgag ttttaaaaga cgggcaatcg cattgcggta     300
   ggagatttca aggctttctc gactagtagc aatgacacct tgatcgatta agaaaccgtc     360
   attgacatca taaacccaac catgtaatga gagttttttc ccatttttcc acgcggattt      420
   aatgattgac gagcgaccta agttataaac ttgctctgcg acgttaattt tcgtcagcat     480
   atcagcccgt ttttcaggcg gtaaattgcc aagtaaatga ctatgcttat accaaatatc      540
   gcgtaagtgg agtaaccagt tattaattaa acctaaatct tgatccgcca ttgcggcttt     600
   aattccacca cagtttgtat gtccacaaat aataatgtgt tcaatattta agacctcaac     660
   ggcatattgc acaacagata aacagtttaa atcggtgtga atgacttgat ttgcaacatt     720
   acgatgcaca aacagctcac ccggtcctaa atttgttaat ttttctgcag gaacacggct     780
   atccgagcaa ccaatccaaa gatagctcgg ggtttgatga tcagccaatt ctttaaagta     840
   agaggagttt tcctctttca tccgtaacgc ccagctataa ttattggcaa aaagttgttc     900
   aatttttttc attagagtga ttcctatacc gcaaaaataa gggggctagt atagcttaga      960
   aatagacagt gggtaaagaa aggcaaaaaa ttgtatagga taacttgttt tttattgcca    1020
   tttatttaga attagaatct ttaataataa aaataattat cattaaggtt aatagtt       1077
   atg gat aaa aat tta atg aag gga tgt gta ttc tta tca ata gtc ggt      1125
   Met Asp Lys Asn Leu Met Lys Gly Cys Val Phe Leu Ser Ile Val Gly
   1               5                   10                  15
   tgc ggt atc caa ata ggg cta gca tca aat cca aat cct cca gat gtg      1173
   Cys Gly Ile Gln Ile Gly Leu Ala Ser Asn Pro Asn Pro Pro Asp Val
               20                  25                  30
   gat gag tta tta cct att att gtg aat gct gat gaa gat aat aaa tta      1221
   Asp Glu Leu Leu Pro Ile Ile Val Asn Ala Asp Glu Asp Asn Lys Leu
           35                  40                  45
   cca ggt cgt tct gta tta aaa cag aaa aat atc gat caa caa caa gca      1269
   Pro Gly Arg Ser Val Leu Lys Gln Lys Asn Ile Asp Gln Gln Gln Ala
       50                  55                  60
   gat aat gcc gct gac tta ata aat att tta cct ggg gta aat atg gcg      1317
   Asp Asn Ala Ala Asp Leu Ile Asn Ile Leu Pro Gly Val Asn Met Ala
   65                  70                  75                  80
   gga gga ttt cgc cct ggt ggt caa aca tta aat att aat gga atg ggt      1365
   Gly Gly Phe Arg Pro Gly Gly Gln Thr Leu Asn Ile Asn Gly Met Gly
                   85                  90                  95
   gat gct gaa gat gtt aga gtt caa cta gac ggc gca aca aaa agt ttc      1413
   Asp Ala Glu Asp Val Arg Val Gln Leu Asp Gly Ala Thr Lys Ser Phe
               100                 105                 110
   gaa aaa tat caa caa ggc tct att ttt att gaa cct gag tta tta aga      1461
   Glu Lys Tyr Gln Gln Gly Ser Ile Phe Ile Glu Pro Glu Leu Leu Arg
           115                 120                 125
   aag gtg aca gta gac aaa gga aat tat tct cct caa tat ggc aat ggt      1509
   Lys Val Thr Val Asp Lys Gly Asn Tyr Ser Pro Gln Tyr Gly Asn Gly
       130                 135                 140
   ggc ttt gct ggt act gta aaa ttt gaa aca aaa gat gca act gat ttt      1557
   Gly Phe Ala Gly Thr Val Lys Phe Glu Thr Lys Asp Ala Thr Asp Phe
   145                 150                 155                 160
   ttg aaa gaa aat cag aaa ata ggt gga tta ttt aaa tat gga aat aat      1605
   Leu Lys Glu Asn Gln Lys Ile Gly Gly Leu Phe Lys Tyr Gly Asn Asn
                   165                 170                 175
   agc aat aat aac caa aaa act tat agt aca gcc cta gtt tta cag aat      1653
   Ser Asn Asn Asn Gln Lys Thr Tyr Ser Thr Ala Leu Val Leu Gln Asn
               180                 185                 190
   gaa caa aaa aat att gat ttg tta tta ttt ggt tct gta aga aat gca      1701
   Glu Gln Lys Asn Ile Asp Leu Leu Leu Phe Gly Ser Val Arg Asn Ala
           195                 200                 205
   agc aat tat aca aga cct gat aaa agt aaa att ctt ttt tca aaa aac      1749
   Ser Asn Tyr Thr Arg Pro Asp Lys Ser Lys Ile Leu Phe Ser Lys Asn
       210                 215                 220
   aat caa aaa agt gga tta ata aaa gta aat tgg caa att act cct gaa      1797
   Asn Gln Lys Ser Gly Leu Ile Lys Val Asn Trp Gln Ile Thr Pro Glu
   225                 230                 235                 240
   cat tta tta act tta tcc agt gtt tat ggc att cat aaa ggg tgg gaa      1845
   His Leu Leu Thr Leu Ser Ser Val Tyr Gly Ile His Lys Gly Trp Glu
                   245                 250                 255
   cct tgg gca gca aaa aga gat gtg atg tcg aga cca aca gaa aca gaa      1893
   Pro Trp Ala Ala Lys Arg Asp Val Met Ser Arg Pro Thr Glu Thr Glu
               260                 265                 270
   ata aaa cac tat ggg att gat gtt gcg tgg aaa cgt aaa ctt gtt tat      1941
   Ile Lys His Tyr Gly Ile Asp Val Ala Trp Lys Arg Lys Leu Val Tyr
           275                 280                 285
   cga gat caa aaa gat gaa agt tat tca ttg aaa tat cgc tat tta cct      1989
   Arg Asp Gln Lys Asp Glu Ser Tyr Ser Leu Lys Tyr Arg Tyr Leu Pro
       290                 295                 300
   gaa aat aat aag tgg att aat ttg tct gtt cag ctg agt tat agt aaa      2037
   Glu Asn Asn Lys Trp Ile Asn Leu Ser Val Gln Leu Ser Tyr Ser Lys
   305                 310                 315                 320
   aca gag cag aat gat act cgc cat gag aaa gtc act tct tca ttc cta      2085
   Thr Glu Gln Asn Asp Thr Arg His Glu Lys Val Thr Ser Ser Phe Leu
                   325                 330                 335
   ggt aca tta gga aat aaa agt tgg ata act tat tca gat ctt act ttt      2133
   Gly Thr Leu Gly Asn Lys Ser Trp Ile Thr Tyr Ser Asp Leu Thr Phe
               340                 345                 350
   gat ata agt aac aca agt act cta aat att ggg cgt gct gag cat gaa      2181
   Asp Ile Ser Asn Thr Ser Thr Leu Asn Ile Gly Arg Ala Glu His Glu
           355                 360                 365
   cta cta ttt ggt tta cag tgg tta aaa aat aaa aga aat acc ctt atg      2229
   Leu Leu Phe Gly Leu Gln Trp Leu Lys Asn Lys Arg Asn Thr Leu Met
       370                 375                 380
   tat cat aaa ggg gga gtc aag aag gca gac tat aat tat ggc tat ttt      2277
   Tyr His Lys Gly Gly Val Lys Lys Ala Asp Tyr Asn Tyr Gly Tyr Phe
   385                 390                 395                 400
   cag cct tat tat atg cct tct gga cgc cag tat aca caa gca ttt tat      2325
   Gln Pro Tyr Tyr Met Pro Ser Gly Arg Gln Tyr Thr Gln Ala Phe Tyr
                   405                 410                 415
   tta caa gat caa ata aaa tgg cag aat ttc ctc ttt aca gga ggg ata      2373
   Leu Gln Asp Gln Ile Lys Trp Gln Asn Phe Leu Phe Thr Gly Gly Ile
               420                 425                 430
   aga tat gac cat atc aat aat ata ggg cag aaa aat tta gcg cca cga      2421
   Arg Tyr Asp His Ile Asn Asn Ile Gly Gln Lys Asn Leu Ala Pro Arg
           435                 440                 445
   tat aat gat atc tct gca gga cat gat tat agc cag aaa aat tat aat      2469
   Tyr Asn Asp Ile Ser Ala Gly His Asp Tyr Ser Gln Lys Asn Tyr Asn
       450                 455                 460
   ggt tgg tct tat tat tta ggt ctt aag tat gat gta aat cat tat tta      2517
   Gly Trp Ser Tyr Tyr Leu Gly Leu Lys Tyr Asp Val Asn His Tyr Leu
   465                 470                 475                 480
   agt tta ttt acg aat ttt agt aaa act tgg cga gcc cct gtt att gat      2565
   Ser Leu Phe Thr Asn Phe Ser Lys Thr Trp Arg Ala Pro Val Ile Asp
                   485                 490                 495
   gaa cag tat gag aca caa tat agt caa gct tct gta tct gcg act tct      2613
   Glu Gln Tyr Glu Thr Gln Tyr Ser Gln Ala Ser Val Ser Ala Thr Ser
               500                 505                 510
   tta aat tta gaa aaa gaa atg att aat caa acc aga gtg ggt gga att      2661
   Leu Asn Leu Glu Lys Glu Met Ile Asn Gln Thr Arg Val Gly Gly Ile
           515                 520                 525
   att act ctc aat cat cta ttt cag gaa aat gat gct ttt caa ttt aga      2709
   Ile Thr Leu Asn His Leu Phe Gln Glu Asn Asp Ala Phe Gln Phe Arg
       530                 535                 540
   act act tat ttt tac aat cgc ggc aag aat gaa atc ttc aaa acg aga      2757
   Thr Thr Tyr Phe Tyr Asn Arg Gly Lys Asn Glu Ile Phe Lys Thr Arg
   545                 550                 555                 560
   ggg gtt aac cgt tagagttggt tgaaatgact gaaaaattag acctatacgt          2809
   Gly Val Asn Arg
   tactgttaaa ggtggcggta tttctggtca agcgggtgca atccgtcacg gtatcactcg    2869
   tgcattaatc gaatatgatg agagtttacg ctctgtatta cgcgcagctg gtttcgttac    2929
   tcgcgatgca cgtcaagttg aacgtaaaaa agtgggttta cgcaaagcgc gtcgtcgtcc    2989
   acaattctca aaacgttaat ttttctttta cgttttatat tcagattgca agcccaaaag    3049
   gcttgcaatt tttttatctc aataaaattt acgataatct ttggaaatca gtgggcgatt    3109
   tgtggtagaa taaacgccca ttttttatat aaaatcatgc cagaatcagg caaagtttaa    3169
   taaattttaa ttcattttag agctgtcgga ggaatagatg acaagcgctg caaataaacg    3229
   ttcaataatg acactttttt cagataaaac agatatttat tgccaccaag taaggattgt    3289
   tttggctgaa aagggtgttg cttatgaaac ggaagttgta gatcctcaag tcgtatcaga 3349
   agatttaatg gaattaaatc cgtatggcac gttgccgaca ttagttgatc gtgatttagt 3409
   gttatttaat tcacgtatta ttatggaata tcttgatgag cgtttccctc atccaccttt 3469
   gatgcctgtt tatccagtgg cacgtgggaa aagccgttta ttaatgttac gtattgagca 3529
   agattggtac ccagtattag caaaagctga aaaaggcacg gacgcagaac gtgctgtcgc 3589
   attaaaacaa ttaagagaag agattttagc gattgcgcct attttcacgc agatgcctta 3649
   ttttatgagc gaagagttta gtttagtaga ttgttatatc gccccattat tatggcgtat 3709
   gcaagaactg ggtgtggatt tcagtggggc gggtagcaaa gcaattaaag cttacatggc 3769
   acgtgttttt gaacgcgatt catttatgca atctttaggc gtgtcggctc cgaaaaactt 3829
   aatggatgag aaataatcag tatgctacat aaatcatcac caaagcgtcc ttacttgtta 3889
   agagcgtatt atgattggtt agtggataat gatttcaccc cttatttagt ggtggacgcg 3949
   acttatgttg gtgtgaaagt ccctgtggaa tatgtcaaag atgggcaaat tgtcctcaat 4009
   ttatctgcga atgcgacagg taatctggta ctaagcaatg aaagtattca gtttagcgcg 4069
   cgttttcgtg gtatttcaca agatattttt attcctatgg gggctgcgtt agccatttat 4129
   gctcgtgaaa atggtgatgg tgtactgttt gaacctgaag cgatttatga tgagctcgca 4189
   acacaaaata ttggtattga gcagccactg agctttgttg aggctgtcga taaaccaaaa 4249
   accagtgaga atactcaaaa aagcacaaac aaagacaaaa cgacggaaaa aaaagcgact 4309
   tctcatttaa gaattattaa ataaaagagg ttttctttct tcataaaaaa acacgctttt 4369
   acgcgtgttt ttttgttgcg gacagtttat tgtgccattt tttttgcggc ttttaagaag 4429
   ccttgcgcac tcgtgtagat gtcactttta ttctgtgccg ctaaaatcat atccgacatt 4489
   tcacgaaagg ccccttgtcc acctttcagg cttagtacgt gatcagcgtg cattttgata 4549
   taagcggcgc atcttgtact gcaaaggcaa caccacagcg gcaaaagcag gcagatcaac 4609
   gctgtcatca ccaatataag ccgtttcttg cgcacagaca ttggcttgtt gtatcaattc 4669
   aagacaggcg ctttcttttt ccaatttgcc gaggaaaaag tgttggatgc ctagatctgc 4729
   aatacgtttg cgtagaatcg gggaatctcg ccccgagagt accgcgactt gaatgccaga 4789
   ttccattaac attctgatcc ccaagccatc acgaacatga aaggttttga aagcttcacc 4849
   atgggcatcg taatgcaaag agccgtcggt cagtacaccg tcgatatctg taatcacaaa 4909
   tttaattttt ttgagttttt ccgttga 4936
<210> 30
   <211> 564
   <212> PRT
   <213> Pasteurella Multocida
<400> 30
   Met Asp Lys Asn Leu Met Lys Gly Cys Val Phe Leu Ser Ile Val Gly
   1 5 10 15
   Cys Gly Ile Gln Ile Gly Leu Ala Ser Asn Pro Asn Pro Pro Asp Val
   20 25 30
   Asp Glu Leu Leu Pro Ile Ile Val Asn Ala Asp Glu Asp Asn Lys Leu
   35 40 45
   Pro Gly Arg Ser Val Leu Lys Gln Lys Asn Ile Asp Gln Gln Gln Ala
   50 55 60
   Asp Asn Ala Ala Asp Leu Ile Asn Ile Leu Pro Gly Val Asn Met Ala
   65 70 75 80
   Gly Gly Phe Arg Pro Gly Gly Gln Thr Leu Asn Ile Asn Gly Met Gly
   85 90 95
   Asp Ala Glu Asp Val Arg Val Gln Leu Asp Gly Ala Thr Lys Ser Phe
   100 105 110
   Glu Lys Tyr Gln Gln Gly Ser Ile Phe Ile Glu Pro Glu Leu Leu Arg
   115 120 125
   Lys Val Thr Val Asp Lys Gly Asn Tyr Ser Pro Gln Tyr Gly Asn Gly
   130 135 140
   Gly Phe Ala Gly Thr Val Lys Phe Glu Thr Lys Asp Ala Thr Asp Phe
   145 150 155 160
   Leu Lys Glu Asn Gln Lys Ile Gly Gly Leu Phe Lys Tyr Gly Asn Asn
   165 170 175
   Ser Asn Asn Asn Gln Lys Thr Tyr Ser Thr Ala Leu Val Leu Gln Asn
   180 185 190
   Glu Gln Lys Asn Ile Asp Leu Leu Leu Phe Gly Ser Val Arg Asn Ala
   195 200 205
   Ser Asn Tyr Thr Arg Pro Asp Lys Ser Lys Ile Leu Phe Ser Lys Asn
   210 215 220
   Asn Gln Lys Ser Gly Leu Ile Lys Val Asn Trp Gln Ile Thr Pro Glu
   225 230 235 240
   His Leu Leu Thr Leu Ser Ser Val Tyr Gly Ile His Lys Gly Trp Glu
                   245                 250                 255
   Pro Trp Ala Ala Lys Arg Asp Val Met Ser Arg Pro Thr Glu Thr Glu
               260                 265                 270
   Ile Lys His Tyr Gly Ile Asp Val Ala Trp Lys Arg Lys Leu Val Tyr
           275                 280                 285
   Arg Asp Gln Lys Asp Glu Ser Tyr Ser Leu Lys Tyr Arg Tyr Leu Pro
       290                 295                 300
   Glu Asn Asn Lys Trp Ile Asn Leu Ser Val Gln Leu Ser Tyr Ser Lys
   305                 310                 315                 320
   Thr Glu Gln Asn Asp Thr Arg His Glu Lys Val Thr Ser Ser Phe Leu
                   325                 330                 335
   Gly Thr Leu Gly Asn Lys Ser Trp Ile Thr Tyr Ser Asp Leu Thr Phe
               340                 345                 350
   Asp Ile Ser Asn Thr Ser Thr Leu Asn Ile Gly Arg Ala Glu His Glu
           355                 360                 365
   Leu Leu Phe Gly Leu Gln Trp Leu Lys Asn Lys Arg Asn Thr Leu Met
       370                 375                 380
   Tyr His Lys Gly Gly Val Lys Lys Ala Asp Tyr Asn Tyr Gly Tyr Phe
   385                 390                 395                 400
   Gln Pro Tyr Tyr Met Pro Ser Gly Arg Gln Tyr Thr Gln Ala Phe Tyr
                   405                 410                 415
   Leu Gln Asp Gln Ile Lys Trp Gln Asn Phe Leu Phe Thr Gly Gly Ile
               420                 425                 430
   Arg Tyr Asp His Ile Asn Asn Ile Gly Gln Lys Asn Leu Ala Pro Arg
           435                 440                 445
   Tyr Asn Asp Ile Ser Ala Gly His Asp Tyr Ser Gln Lys Asn Tyr Asn
       450                 455                 460
   Gly Trp Ser Tyr Tyr Leu Gly Leu Lys Tyr Asp Val Asn His Tyr Leu
   465                 470                 475                 480
   Ser Leu Phe Thr Asn Phe Ser Lys Thr Trp Arg Ala Pro Val Ile Asp
   485 490 495
   Glu Gln Tyr Glu Thr Gln Tyr Ser Gln Ala Ser Val Ser Ala Thr Ser
   500 505 510
   Leu Asn Leu Glu Lys Glu Met Ile Asn Gln Thr Arg Val Gly Gly Ile
   515 520 525
   Ile Thr Leu Asn His Leu Phe Gln Glu Asn Asp Ala Phe Gln Phe Arg
   530 535 540
   Thr Thr Tyr Phe Tyr Asn Arg Gly Lys Asn Glu Ile Phe Lys Thr Arg
   545 550 555 560
   Gly Val Asn Arg
<210> 31
   <211> 9814
   <212> DNA
   <213> Pasteurella Multocida
<220>
   <221> misc_feature
   <223> iroA
<220>
   <221> CDS
   <222> (4762) .. (7662)
<400> 31
<210> 32
   <211> 967
   <212> PRT
   <213> Pasteurella Multocida
<400> 32
   Met Arg Thr Thr Thr Ile Lys Phe Ser Ala Ile Thr Leu Ala Leu Leu
   1               5                   10                  15
   Ser Tyr Cys Gly Thr Ile Leu Ala Asp Ser His Gln Glu Ala Thr Glu
               20                  25                  30
   Leu Asp Thr Ile Thr Val Ser Ser Gln Gln Asp Glu Met Asn Ile Lys
           35                  40                  45
   Glu Lys Lys Ile Gly Glu Thr Val Lys Thr Ala Ser Gln Leu Lys Arg
       50                  55                  60
   Gln Gln Val Gln Asp Ser Arg Asp Leu Val Arg Tyr Glu Thr Gly Val
   65                  70                  75                  80
   Thr Val Val Glu Ala Gly Arg Phe Gly Ser Ser Gly Tyr Ala Ile Arg
                   85                  90                  95
   Gly Val Asp Glu Asn Arg Val Ala Ile Thr Val Asp Gly Leu His Gln
               100                 105                 110
   Ala Glu Thr Leu Ser Ser Gln Gly Phe Lys Glu Leu Phe Glu Gly Tyr
   115 120 125
   Gly Asn Phe Asn Asn Thr Arg Asn Ser Val Glu Ile Glu Thr Leu Lys
   130 135 140
   Val Ala Lys Ile Ala Lys Gly Ala Asp Ser Val Lys Val Gly Ser Gly
   145 150 155 160
   Ser Leu Gly Gly Ala Val Leu Phe Glu Thr Lys Asp Ala Arg Asp Phe
   165 170 175
   Leu Thr Glu Lys Asp Trp His Ile Gly Tyr Lys Ala Gly Tyr Ser Thr
   180 185 190
   Ala Asp Asn Gln Gly Leu Asn Ala Val Thr Leu Ala Gly Arg Tyr Gln
   195 200 205
   Met Phe Asp Ala Leu Ile Met His Ser Lys Arg His Gly His Glu Leu
   210 215 220
   Glu Asn Tyr Asp Tyr Lys Asn Gly Arg Asp Ile Gln Gly Lys Glu Arg
   225 230 235 240
   Glu Lys Ala Asp Pro Tyr Thr Ile Thr Lys Glu Ser Thr Leu Val Lys
   245 250 255
   Phe Ser Phe Ser Pro Thr Glu Asn His Arg Phe Thr Val Ala Ser Asp
   260 265 270
   Thr Tyr Leu Gln His Ser Arg Gly His Asp Leu Ser Tyr Asn Leu Val
   275 280 285
   Ala Thr Thr His Ile Gln Leu Asp Glu Lys Glu Ser Arg His Ala Asn
   290 295 300
   Asp Leu Thr Lys Arg Lys Asn Val Ser Phe Thr Tyr Glu Asn Tyr Thr
   305 310 315 320
   Val Thr Pro Phe Trp Asp Thr Leu Lys Leu Ser Tyr Ser Gln Gln Arg
   325 330 335
   Ile Thr Thr Arg Ala Arg Thr Glu Asp Tyr Cys Asp Gly Asn Glu Leu
   340 345 350
   Cys Asp Ser Tyr Lys Asn Pro Leu Gly Leu Gln Phe Lys Asp Gly Gln
   355 360 365
   Ile Leu Asp Pro Ala Gly Asn Lys Ile Lys Leu Gln Gly Ser Gly Leu
   370 375 380
   Ser Thr Gln Ile Val Asp Glu Asn Gly Lys Pro Phe Pro Thr Thr Thr
   385 390 395 400
   Gly Thr Asn Asn Ala Ala Phe Ser Asn Asn Leu Arg Leu Arg Pro Thr
   405 410 415
   Gly Phe Trp Leu Asp Cys Ser Val Phe Asp Cys Asn Lys Pro Phe Thr
   420 425 430
   Val Tyr Asn Ile Ser Asn Gly Thr Tyr Gln Ala Arg Glu Val Leu Leu
   435 440 445
   Ser Glu Glu Ile Thr Val Asp Gly Lys Leu Tyr Lys Thr Ala Lys Glu
   450 455 460
   Glu Gly Gly Leu Pro Asn Tyr Leu Ile Leu Pro Asn Ser Lys Gly Tyr
   465 470 475 480
   Leu Pro Tyr Asp Tyr Lys Glu Arg Asp Leu Asn Thr Asn Thr Lys Gln
   485 490 495
   Ile Asn Leu Asp Leu Thr Lys Thr Phe Leu Thr Phe Asn Ile Glu Asn
   500 505 510
   Asn Leu Ser Tyr Gly Gly Val Tyr Ser Arg Ile Glu Lys Glu Met Ile
   515 520 525
   Asn Lys Ala Gly Tyr Glu Gly Arg Asn Pro Thr Trp Trp Ala Asp Arg
   530 535 540
   Ile Leu Gly Gln Ser Ser Tyr Cys Gly Tyr Asn Ala Leu Lys Cys Pro
   545 550 555 560
   Lys His Glu Pro Leu Thr Ser Phe Leu Ile Pro Val Glu Ala Thr Thr
   565 570 575
   Gln Ser Leu Tyr Phe Ala Asn Ile Leu Lys Val His Asn Met Ile Ser
   580 585 590
   Ile Asp Leu Gly Tyr Arg Tyr Asp His Ile Lys Tyr Asn Pro Glu Tyr
   595 600 605
   Thr Pro Gly Val Thr Pro Lys Ile Pro Asp Asp Met Val Lys Gly Leu
   610 615 620
   Phe Ile Pro Met Pro Lys Glu Pro Gln Leu Lys Asp Phe Asp Tyr Asn
   625 630 635 640
   Tyr Ala Lys Phe Gly Glu Ala Tyr Lys Lys Trp Lys Glu Tyr Leu Pro
   645 650 655
   Lys Asn Ala Glu Glu Asn Ile Ala Tyr Ile Ala Gln Asp Lys Thr Phe
   660 665 670
   Lys Lys His Ser Tyr Ser Leu Gly Ala Thr Phe Asp Pro Leu Asn Phe
   675 680 685
   Leu Arg Val Gln Val Lys Tyr Ser Lys Gly Phe Arg Ala Pro Thr Ser
   690 695 700
   Asp Glu Leu Tyr Phe Thr Phe Lys His Pro Asp Phe Thr Ile Leu Pro
   705 710 715 720
   Asn Pro Val Leu Lys Pro Glu Glu Ala Lys Asn Gln Glu Ile Ala Leu
   725 730 735
   Thr Val His Asp Asn Trp Gly Phe Val Ser Thr Ser Val Phe Gln Thr
   740 745 750
   Lys Tyr Arg His Phe Ile Asp Leu Ala Tyr Leu Gly Ser Arg Asn Leu
   755 760 765
   Ser Asn Ser Val Gly Gly Gln Ala Gln Ala Arg Asp Phe Gln Val Tyr
   770 775 780
   Gln Asn Val Asn Val Asp Asn Ala Lys Val Lys Gly Leu Glu Ile Asn
   785 790 795 800
   Ala Arg Leu Asn Leu Gly Tyr Phe Trp His Val Leu Asp Gly Phe Asn
   805 810 815
   Thr Ser Tyr Lys Phe Thr Tyr Gln Arg Gly Arg Leu Asp Gly Asp Arg
   820 825 830
   Pro Met Asn Ala Ile Gln Pro Lys Ala Ser Val Phe Gly Leu Gly Tyr
   835 840 845
   Asp His Lys Glu Asn Lys Phe Gly Ala Asp Leu Tyr Ile Thr Arg Val
   850 855 860
   Ser Glu Lys Lys Ala Lys Asp Thr Tyr Asn Met Phe Tyr Lys Glu Gln
   865 870 875 880
   Gly Tyr Lys Asp Ser Ala Val Arg Trp Arg Ser Asp Asp Tyr Thr Leu
   885 890 895
   Val Asp Ala Val Gly Tyr Ile Lys Pro Ile Lys Asn Leu Thr Leu Gln
   900 905 910
   Phe Gly Val Tyr Asn Leu Thr Asp Arg Lys Tyr Leu Thr Trp Glu Ser
   915 920 925
   Ala Arg Ser Ile Lys Pro Phe Gly Thr Ser Asn Leu Ile Asn Gln Lys
   930 935 940
   Thr Gly Ala Gly Ile Asn Arg Phe Tyr Ser Pro Gly Arg Asn Phe Lys
   945 950 955 960
   Leu Ser Ala Glu Ile Thr Phe
   965
<210> 33
   <211> 2990
   <212> DNA
   <213> Pasteurella Multocida
<220>
   <221> misc_feature
   <223> kdtB
<220>
   <221> CDS
   <222> (1106) .. (1564)
<400> 33
   gtcaacccct ttggctttga gttgtgctaa taaagcatcg tcaaaatgca agccggcagt 60
   tggtgccgca accgcgccag ggactttgtt ataaacggtt tgataacgtt ctttatccgc 120
   ttcttcgtca gggcgatcaa tataaggggg caatggcata tgcccaattt gctgtaacac 180
   gtctaaaagt gcggtctgtt tttgcgcgat ttctaattca aataaggtat catggcgcgc 240
   aaccatgatc attttgacac catgatgttc acctaactta tcttcgccta accacagttc 300
   tgccccttct ttcggtgctt ttgaggagcg cacatgggct aaaaagcgtg tgtcggataa 360
   aatccgctcg accaacactt ccaccttacc gccactggct ttacgtccaa acatccttgc    420
   aggaatcacg cgcgtgttat taaaaattaa taagtcgcct tcatgaattt gatcaaggat    480
   atcagcaaaa gtgcggtggg taatctcacc attttcgccg ttaagttgta ataagcgact    540
   agcggtgcga tccggttttg ggtaacgagc aatcagctca tcgggtaaat caaaataaaa    600
   gtcagaaaca cgcataaata gggttataaa aagttatcta aaaaatcgtg ggcgtaagtc    660
   tagtgtgaat tccgctcttg cacaaggaaa aatccagatt ttgttgttta gtatcgaatt    720
   gagatgattt tggacaaaaa aaaagccctt tcaagaaaga cgaaagggcg aaaatatatt    780
   tggagtcata ctttttaggg tatgtgtcgg attatacaca caaaaataac aaatgcaaca    840
   tttttttaac aatcatatgt aagcgtattg tgtgagaacg agcgtaaaaa tgaacgcatt    900
   ctaaaggatg atttatttag cctattaaaa aaacacatga gatgagagtt tgcgagagcg    960
   gtaataaaag tgcggtgggt tttagaaaag ttttgaatag gatcacaaat taaacaaagt   1020
   ttgtgaaata ccaagtagta gtttttaagt atatgatgaa tcatatgcta aagtttaaac   1080
   ccgttaaata accaagaggt ggaag atg aca gaa gaa aat aaa gga aag aga    1132
                               Met Thr Glu Glu Asn Lys Gly Lys Arg
                               1               5
   tat ttt tta tgg ttc ata ttg ttt atc ctt tca atc tat tta ttt att    1180
   Tyr Phe Leu Trp Phe Ile Leu Phe Ile Leu Ser Ile Tyr Leu Phe Ile
   10                  15                  20                  25
   acc ata caa gaa aga cga ggt tat tgt ttt gac aaa cgt gca tat att    1228
   Thr Ile Gln Glu Arg Arg Gly Tyr Cys Phe Asp Lys Arg Ala Tyr Ile
                   30                  35                  40
   cat gag ctt tat act gag caa gag tta att gat cgg ggg att gaa tat    1276
   His Glu Leu Tyr Thr Glu Gln Glu Leu Ile Asp Arg Gly Ile Glu Tyr
               45                  50                  55
   gtg gta tcc acc atg ccg tca ggt gtt att aaa cca gat ggc aca ata    1324
   Val Val Ser Thr Met Pro Ser Gly Val Ile Lys Pro Asp Gly Thr Ile
           60                  65                  70
   aaa gaa gta aag cgt tac acg agt gtc gag gag ttt aaa cag atg aac    1372
   Lys Glu Val Lys Arg Tyr Thr Ser Val Glu Glu Phe Lys Gln Met Asn
       75                  80                  85
   cca gct tgt tgt aca tta acc acc ttt att gat gaa gga ggc gat ggc    1420
   Pro Ala Cys Cys Thr Leu Thr Thr Phe Ile Asp Glu Gly Gly Asp Gly
   90                  95                  100                 105
   tat cca gat gat gat gga tat ggt tat gtc aga att gaa tat tta aga    1468
   Tyr Pro Asp Asp Asp Gly Tyr Gly Tyr Val Arg Ile Glu Tyr Leu Arg
                   110                 115                 120
   cat tat gtt gag aat cta aaa cct tat cat aga gtg att tat ctt gaa    1516
   His Tyr Val Glu Asn Leu Lys Pro Tyr His Arg Val Ile Tyr Leu Glu
               125                 130                 135
   tat acg ccc tgt gga gag tta agg gaa gag gcg gct ttt tca aaa aat    1564
   Tyr Thr Pro Cys Gly Glu Leu Arg Glu Glu Ala Ala Phe Ser Lys Asn
           140                 145                 150
   taagagtgag gtgaagaaat ggcattacca acagcaacaa taatgaggaa tttatcttta   1624
   tctaaaaatc aattcactct gaaagggatg gaatgcgtag attccctatt tcaagcatgc   1684
   agtaatatgg atcatgggta ctgaggtgga agatggcaga agaaaataaa ggaaagagat   1744
   attttttatg gttcatattg tttatccttt caatctattt atttattacc atacaagaaa   1804
   gacgaggtta ttgttttgac aaatgggaat atatccataa cctttatacc gagcaagagt   1864
   tgatcgatag aggggttgaa tatgtggtat ccaccatgcc gtcaggtgtt tttgaaccag   1924
   atggcacaac aaccgaaata aaacgttatg ctagtgttga ggagtttaaa cagatgaacc   1984
   ctgattgttg taaattaaca agatttatta atgaaggaat agatggctat ccagatgatg   2044
   atggatatgg ttatataaga attgaatatt taagacatta tgttgggaat tttaaacctg   2104
   atcatagagt gctttatctc gaatatacgc cttgtggaga attaagggaa gaggtttctt   2164
   tttaaaaaat aaataatagt gaggtgaaga aatggcatta ccaacagcaa cagaaatcac   2224
   aaatgcatat ttatataaaa ataaattaac tcctaaagcg gaggaaagag tagattcaat   2284
   acaaattctt gaaaaaggag atgaacattt cgaagtaaat tttaattgat caaagtactc   2344
   tattgattga aggaaaaaca gtggaattaa tggcaggtat ggcagtttct gcggaaatta   2404
   aaacaggtaa acgcagtgta ttagattact tatttagccc attaaaaacc acaaaataat   2464
   attaaggaga ataatatgtc gtataataaa tatactgttg ctttgattac gttctcaaca   2524
   gggatctgta ttccggcaat atgctacgct ctaaattcgc tgggatacag atcctgtttg   2584
   agactatgta gaaaagacta aactttgtgt ggttaactgg gcttcggtaa aattctggaa   2644
   acaaatgggc ttaacccgcg tgatcttatc ccgtgagctt tcgcttgatg aaattgccga   2704
   aattcgtcag caagtgccag aaatggaaat tgaagtgttc gtgcatgggg cattatgcat   2764
   ggcgtattct ggacgttgtt tattatcagg ctatattaat aaacgtgatc caaatcaagg   2824
   cacctgtacc aatgcgtgcc gttgggaata cagtgtaacc gaagccaaag aagatgagat   2884
   cggcaacatt gtgaatgtgg gtgaagaaat tccagtgaaa aatgtagcac cgacacttgg   2944
   cgaaggcgac accaccagta aagtattttt attagcagaa agtcga                  2990
<210> 34
   <211> 153
   <212> PRT
   <213> Pasteurella Multocida
<400> 34
   Met Thr Glu Glu Asn Lys Gly Lys Arg Tyr Phe Leu Trp Phe Ile Leu
   1               5                   10                  15
   Phe Ile Leu Ser Ile Tyr Leu Phe Ile Thr Ile Gln Glu Arg Arg Gly
               20                  25                  30
   Tyr Cys Phe Asp Lys Arg Ala Tyr Ile His Glu Leu Tyr Thr Glu Gln
           35                  40                  45
   Glu Leu Ile Asp Arg Gly Ile Glu Tyr Val Val Ser Thr Met Pro Ser
       50                  55                  60
   Gly Val Ile Lys Pro Asp Gly Thr Ile Lys Glu Val Lys Arg Tyr Thr
   65                  70                  75                  80
   Ser Val Glu Glu Phe Lys Gln Met Asn Pro Ala Cys Cys Thr Leu Thr
                   85                  90                  95
   Thr Phe Ile Asp Glu Gly Gly Asp Gly Tyr Pro Asp Asp Asp Gly Tyr
               100                 105                 110
   Gly Tyr Val Arg Ile Glu Tyr Leu Arg His Tyr Val Glu Asn Leu Lys
           115                 120                 125
   Pro Tyr His Arg Val Ile Tyr Leu Glu Tyr Thr Pro Cys Gly Glu Leu
       130                 135                 140
   Arg Glu Glu Ala Ala Phe Ser Lys Asn
   145                 150
<210> 35
   <211> 1683
   <212> DNA
   <213> Pasteurella Multocida
<220>
   <221> misc_feature
   <223> lgtC
<220>
   <221> CDS
   <222> (325) .. (1230)
<220>
   <221> misc_feature
   <222> (981) .. (981)
   <223> n is a, c, g, or t
<220>
   <221> misc_feature
   <222> (1000) .. (1000)
   <223> n is a, c, g, or t
<220>
   <221> misc_feature
   <222> (1129)..(1129)
   <223> n is a, c, g, or t
<220>
   <221> misc_feature
   <222> (1134) .. (1134)
   <223> n is a, c, g, or t
<220>
   <221> misc_feature
   <222> (1144) .. (1144)
   <223> n is a, c, g, or t
<220>
   <221> misc_feature
   <222> (1423) .. (1423)
   <223> n is a, c, g, or t
<220>
   <221> misc_feature
   <222> (1665)..(1665)
   <223> n is a, c, g, or t
<220>
   <221> misc_feature
   <222> (1667) .. (1667)
   <223> n is a, c, g, or t
<400> 35
   atatcaaagt ctcatggcaa gaaaattaga aaagagcgat caattattat ttgcaagatt      60
   tgggtattat tcataggcta ggtgaaagat atatttttcc atgatattaa aacgattcag     120
   gcagaactgg ctagcttatc acttttagat aattgtatta ttaaaagaag ctgtatgatt     180
   gttattctat cattagtgga taataaatat tctttatttt ttgagagata aaaacaattc     240
   atatttcaat agaaaacaga aaataaagat tatcaaaaga attatccgtc cttataaata     300
   tgagtctgta ttgtgagatg atat atg aat att tta ttt gtt tct gat gat       351
                              Met Asn Ile Leu Phe Val Ser Asp Asp
                              1               5
   gtt tat gct aaa cat ctg gtg gtt gcg att aaa agc att ata aat cat      399
   Val Tyr Ala Lys His Leu Val Val Ala Ile Lys Ser Ile Ile Asn His
   10                  15                  20                  25
   aat gaa aaa ggt att tca ttt tat att ttt gat ttg ggt ata aag gat      447
   Asn Glu Lys Gly Ile Ser Phe Tyr Ile Phe Asp Leu Gly Ile Lys Asp
                   30                  35                  40
   gaa aat aag aga aat att aat gat att gtt tct tct tat gga agt gaa      495
   Glu Asn Lys Arg Asn Ile Asn Asp Ile Val Ser Ser Tyr Gly Ser Glu
               45                  50                  55
   gtc aac ttt att gct gtg aat gag aaa gaa ttt gag agt ttt cct gtt      543
   Val Asn Phe Ile Ala Val Asn Glu Lys Glu Phe Glu Ser Phe Pro Val
           60                  65                  70
   caa att agt tat att tct tta gca aca tat gca agg cta aaa gcg gca      591
   Gln Ile Ser Tyr Ile Ser Leu Ala Thr Tyr Ala Arg Leu Lys Ala Ala
       75                  80                  85
   gag tat ttg ccg gat aat tta aat aaa att att tat tta gat gtt gat      639
   Glu Tyr Leu Pro Asp Asn Leu Asn Lys Ile Ile Tyr Leu Asp Val Asp
   90                  95                  100                 105
   gtt ttg gtt ttt aac tca tta gaa atg tta tgg aat gtt gat gtt aat      687
   Val Leu Val Phe Asn Ser Leu Glu Met Leu Trp Asn Val Asp Val Asn
                   110                 115                 120
   aat ttt ctt acc gca gcc tgt tat gat tct ttc atc gaa aat gaa aag      735
   Asn Phe Leu Thr Ala Ala Cys Tyr Asp Ser Phe Ile Glu Asn Glu Lys
               125                 130                 135
   tct gag cat aaa aaa tcg att tca atg tca gat aag gaa tat tat ttt      783
   Ser Glu His Lys Lys Ser Ile Ser Met Ser Asp Lys Glu Tyr Tyr Phe
           140                 145                 150
   aat gca gga gta atg cta ttt aat tta gat gaa tgg cgg aag atg gat      831
   Asn Ala Gly Val Met Leu Phe Asn Leu Asp Glu Trp Arg Lys Met Asp
       155                 160                 165
   gta ttc tca aga gct tta gac ctg tta gct atg tat cct aat caa atg      879
   Val Phe Ser Arg Ala Leu Asp Leu Leu Ala Met Tyr Pro Asn Gln Met
   170                 175                 180                 185
   att tat cag gat caa gat ata ttg aat atc ctt ttt agg aat aaa gtc      927
   Ile Tyr Gln Asp Gln Asp Ile Leu Asn Ile Leu Phe Arg Asn Lys Val
                   190                 195                 200
   tgt tat tta gat tgc aga ttt aat ttc atg cca aat caa ctt gaa aga      975
   Cys Tyr Leu Asp Cys Arg Phe Asn Phe Met Pro Asn Gln Leu Glu Arg
               205                 210                 215
   ata aan caa tac cat aaa gga aaa ntg agc aac tta cat tct tta gaa     1023
   Ile Xaa Gln Tyr His Lys Gly Lys Xaa Ser Asn Leu His Ser Leu Glu
           220                 225                 230
   aaa aca acg atg cct gtc gtt att tca cat tat tgt ggt cca gaa aaa     1071
   Lys Thr Thr Met Pro Val Val Ile Ser His Tyr Cys Gly Pro Glu Lys
       235                 240                 245
   gcg tgg cat gcg gat tgt aaa cat ttt aat gta tat ttc tat cag aaa     1119
   Ala Trp His Ala Asp Cys Lys His Phe Asn Val Tyr Phe Tyr Gln Lys
   250                 255                 260                 265
   ata tta gca naa atn tcg aga ggc ncg gat aaa gaa cgc gta tta tct     1167
   Ile Leu Ala Xaa Xaa Ser Arg Gly Xaa Asp Lys Glu Arg Val Leu Ser
                   270                 275                 280
   ata aaa act tat ctc aag gcc ttg att aga agg att aga tat aaa ttc     1215
   Ile Lys Thr Tyr Leu Lys Ala Leu Ile Arg Arg Ile Arg Tyr Lys Phe
               285                 290                 295
   aaa tat caa gtc tat taactattga atttttgcaa atgagataag agtatagtgc     1270
   Lys Tyr Gln Val Tyr
           300
   tgatttcttc aaagcgaaaa ggaggaaata gcttgttcta atttattaca ataatggttg   1330
   tattcatctt gattttgaag gaaagagagt gttttttgta taaaagcatt ttcgtcacct   1390
   aaatttacta atcctccaaa ttctcctcct cgnagaattt ctttcggacc ggtagggcag   1450
   tccatggata ttacaggtgt accgcaagcc atgctttcta ggataactgt cggtaacccc   1510
   tctttcaaag aggtgtgtaa aaatagctta gcatttttta ttaatggata cggattatct   1570
   ttatttccta aaagaaaaca atcttcttgt agattgagtg attctatttg tttctctaat   1630
   ttttctcgac actcaccatc ccaaacaata tatancnttt cttggatacc tcc          1683
<210> 36
   <211> 302
   <212> PRT
   <213> Pasteurella Multocida
<220>
   <221> misc_feature
   <222> (219) .. (219)
   <223> The 'Xaa' at location 219 stands for Lys, or Asn.
<220>
   <221> misc_feature
   <222> (226)..(226)
   <223> The 'Xaa' at location 226 stands for Met, Val, or Leu.
<220>
   <221> misc_feature
   <222> (269) .. (269)
   <223> The 'Xaa' at location 269 stands for Lys, Glu, or Gln.
<220>
   <221> misc_feature
   <222> (270)..(270)
   <223> The 'Xaa' at location 270 stands for Ile, or Met.
<220>
   <221> misc_feature
   <222> (274) .. (274)
   <223> The 'Xaa' at location 274 stands for Thr, Ala, Pro, or Ser.
<400> 36
   Met Asn Ile Leu Phe Val Ser Asp Asp Val Tyr Ala Lys His Leu Val
   1               5                   10                  15
   Val Ala Ile Lys Ser Ile Ile Asn His Asn Glu Lys Gly Ile Ser Phe
               20                  25                  30
   Tyr Ile Phe Asp Leu Gly Ile Lys Asp Glu Asn Lys Arg Asn Ile Asn
           35                  40                  45
   Asp Ile Val Ser Ser Tyr Gly Ser Glu Val Asn Phe Ile Ala Val Asn
       50                  55                  60
   Glu Lys Glu Phe Glu Ser Phe Pro Val Gln Ile Ser Tyr Ile Ser Leu
   65                  70                  75                  80
   Ala Thr Tyr Ala Arg Leu Lys Ala Ala Glu Tyr Leu Pro Asp Asn Leu
                   85                  90                  95
   Asn Lys Ile Ile Tyr Leu Asp Val Asp Val Leu Val Phe Asn Ser Leu
               100                 105                 110
   Glu Met Leu Trp Asn Val Asp Val Asn Asn Phe Leu Thr Ala Ala Cys
           115                 120                 125
   Tyr Asp Ser Phe Ile Glu Asn Glu Lys Ser Glu His Lys Lys Ser Ile
       130                 135                 140
   Ser Met Ser Asp Lys Glu Tyr Tyr Phe Asn Ala Gly Val Met Leu Phe
   145                 150                 155                 160
   Asn Leu Asp Glu Trp Arg Lys Met Asp Val Phe Ser Arg Ala Leu Asp
                   165                 170                 175
   Leu Leu Ala Met Tyr Pro Asn Gln Met Ile Tyr Gln Asp Gln Asp Ile
               180                 185                 190
   Leu Asn Ile Leu Phe Arg Asn Lys Val Cys Tyr Leu Asp Cys Arg Phe
           195                 200                 205
   Asn Phe Met Pro Asn Gln Leu Glu Arg Ile Xaa Gln Tyr His Lys Gly
       210                 215                 220
   Lys Xaa Ser Asn Leu His Ser Leu Glu Lys Thr Thr Met Pro Val Val
   225                 230                 235                 240
   Ile Ser His Tyr Cys Gly Pro Glu Lys Ala Trp His Ala Asp Cys Lys
                   245                 250                 255
   His Phe Asn Val Tyr Phe Tyr Gln Lys Ile Leu Ala Xaa Xaa Ser Arg
               260                 265                 270
   Gly Xaa Asp Lys Glu Arg Val Leu Ser Ile Lys Thr Tyr Leu Lys Ala
           275                 280                 285
   Leu Ile Arg Arg Ile Arg Tyr Lys Phe Lys Tyr Gln Val Tyr
       290                 295                 300
<210> 37
   <211> 2029
   <212> DNA
   <213> Pasteurella Multocida
<220>
   <221> misc_feature
   <223> mglB
<220>
   <221> CDS
   <222> (2) .. (499)
<220>
   <221> misc_feature
   <222> (98) .. (98)
   <223> n is a, c, g, or t
<220>
   <221> misc_feature
   <222> (296) .. (296)
   <223> n is a, c, g, or t
<220>
   <221> misc_feature
   <222> (302) .. (302)
   <223> n is a, c, g, or t
<220>
   <221> misc_feature
   <222> (928) .. (928)
   <223> n is a, c, g, or t
<220>
   <221> misc_feature
   <222> (1007) .. (1007)
   <223> n is a, c, g, or t
<220>
   <221> misc_feature
   <222> (1740) .. (1740)
   <223> n is a, c, g, or t
<220>
   <221> misc_feature
   <222> (1808) .. (1808)
   <223> n is a, c, g, or t
<220>
   <221> misc_feature
   <222> (1816) .. (1816)
   <223> n is a, c, g, or t
<220>
   <221> misc_feature
   <222> (1820) .. (1820)
   <223> n is a, c, g, or t
<400> 37
   c tta aat aaa gcc ggt aaa att caa tac gtt tta tta aaa ggt aac caa      49
     Leu Asn Lys Ala Gly Lys Ile Gln Tyr Val Leu Leu Lys Gly Asn Gln
     1               5                   10                  15
   gga cac cca gat gca gaa gct cgt aca aaa ttc gtc att aaa gaa tta        97
   Gly His Pro Asp Ala Glu Ala Arg Thr Lys Phe Val Ile Lys Glu Leu
               20                  25                  30
   nat aat aaa ggc att caa gat gag caa tta ttc atc gac acg ggg atg       145
   Xaa Asn Lys Gly Ile Gln Asp Glu Gln Leu Phe Ile Asp Thr Gly Met
           35                  40                  45
   tgg gat gcc gct tta gcg aaa gat aaa atg gat gca tgg tta tct agc       193
   Trp Asp Ala Ala Leu Ala Lys Asp Lys Met Asp Ala Trp Leu Ser Ser
       50                  55                  60
   tct aaa gca aat caa att gaa gtg atc atc gct aac aac gat ggt atg       241
   Ser Lys Ala Asn Gln Ile Glu Val Ile Ile Ala Asn Asn Asp Gly Met
   65                  70                  75                  80
   gcg atg ggg gca ttg gaa gcc acg aaa gca cat ggt aaa aaa tta cca       289
   Ala Met Gly Ala Leu Glu Ala Thr Lys Ala His Gly Lys Lys Leu Pro
                   85                  90                  95
   atc ttc ngt gta nat gcg tta cca gaa gtc ctc caa tta atc aaa aaa       337
   Ile Phe Xaa Val Xaa Ala Leu Pro Glu Val Leu Gln Leu Ile Lys Lys
               100                 105                 110
   ggt gaa att gca ggt acg gtg tta aat gac ggt gtg aac caa ggt aaa       385
   Gly Glu Ile Ala Gly Thr Val Leu Asn Asp Gly Val Asn Gln Gly Lys
           115                 120                 125
   gcc gtt gtt caa tta agt aat aat ctt gca aaa gga aaa cct gcc act       433
   Ala Val Val Gln Leu Ser Asn Asn Leu Ala Lys Gly Lys Pro Ala Thr
       130                 135                 140
   gaa ggc aca aaa tgg cag tta aaa cga tcg tgt cct acg tat ccc tta       481
   Glu Gly Thr Lys Trp Gln Leu Lys Arg Ser Cys Pro Thr Tyr Pro Leu
   145                 150                 155                 160
   tgt tgg tgt gga tgc gga taacttaaac gagttcctaa aataataaac              529
   Cys Trp Cys Gly Cys Gly
                   165
   tataacaaaa caagamgttg taattctcgg ggaggtatac cctccccctt tttatgtgag     589
   gttggatatg acaactcaaa ttccaaatca agacagtgaa atactgctca caatgaccaa     649
   cgtctgtaaa tcctttcccg gtgttaaagc gttagacaat gcaaacctaa ctgtgcgctc     709
   gcattctgtc catgccttaa tgggcgaaaa tggggcgggc aaatcgacct tattaaaatg     769
   cttatttggt atttacagta aagatgaagg tgacatcctt ttcttaggca agccagtcaa     829
   ctttaaaacg tcgaaagaag ccttagagaa cgggattttc atggtgcacc aagaacttaa     889
   cttggttaaa caatgtactg taatggataa tcctttggnt aggacgttat ccattaaaag     949
   caggctttgt cgatcacggc aaaatgtatc gtgataccaa agcagatttt tgaagaanta    1009
   gatatcgata tcgatccaaa agaaaaagtg gccaaattgt cagtgtcaca aatgcaaatg    1069
   atcgagatcg caaaggcctt ttcatacaat gccaaaatcg taatcatgga cgaaccgact   1129
   tcttcgcttt cagaaaaaga agttgaacac ctatttaaaa ttatcgcgaa gctaaaacaa   1189
   cgtggctgtg gcattattta tatttcgcac aaaatggacg aaatcttcaa aatttgtgac   1249
   gaaattacga ttttacgcga tggtaaatgg atcaatacgg tcgctgttaa aggcaccaca   1309
   atggatcaga ttgtatccat gatggttggg cgtgaactca cgcaacgttt cccaccaaaa   1369
   accaataccc caaaagaaac catcttaacg gtggaaaatc tgaccgcact taatcagcca   1429
   tctattcaag atgttagttt tgaattacgc aaaggcgaag tgctcggcat tgcgggactg   1489
   gttggggcaa aacggtaccg atattgtgga aacgatcttc ggggtgcgtg aacgtaaatc   1549
   tggtgtgatt aaactacacg ataaggaaat gaaaaaccgg aatgcgttcg aagccattaa   1609
   caatggtttt gccttggtca cggaagaacg tcgctctaca gggatttatg cgaatctcag   1669
   tattgagttt aactcattaa tttctaacat gaagaaatcc tatatcagca agttaggttt   1729
   attgagtaac ncaaaaatga aaagcgacac gcaatggggt cattgattcc atgaatgtga   1789
   aaacgccatc acaaaaccna tattggntca ntatctgggg tggtaaccaa caaaaagtgg   1849
   tcattggtcg ttggttatta acccaccctg aaatcttgat gttagacgaa ccaacacgtg   1909
   gtatcgacat tggtgcgaaa tatgaaattt atcagctgat tatggagtta gccaaaaaag   1969
   ataaaggtat catcatgatt tcatctaaag gccagagtta ttaggggtac tgaccgaatt   2029
<210> 38
   <211> 166
   <212> PRT
   <213> Pasteurella Multocida
<220>
   <221> misc_feature
   <222> (33) .. (33)
   <223> The 'Xaa' at location 33 stands for Asn, Asp, His, or Tyr.
<220>
   <221> misc_feature
   <222> (99) .. (99)
   <223> The 'Xaa' at location 99 stands for Ser, Gly, Arg, or Cys.
<220>
   <221> misc_feature
   <222> (101) .. (101)
   <223> The 'Xaa' at location 101 stands for Asn, Asp, His, or Tyr.
<400> 38
   Leu Asn Lys Ala Gly Lys Ile Gln Tyr Val Leu Leu Lys Gly Asn Gln
   1               5                   10                  15
   Gly His Pro Asp Ala Glu Ala Arg Thr Lys Phe Val Ile Lys Glu Leu
                   20                  25                  30
   Xaa Asn Lys Gly Ile Gln Asp Glu Gln Leu Phe Ile Asp Thr Gly Met
           35                  40                  45
   Trp Asp Ala Ala Leu Ala Lys Asp Lys Met Asp Ala Trp Leu Ser Ser
       50                  55                  60
   Ser Lys Ala Asn Gln Ile Glu Val Ile Ile Ala Asn Asn Asp Gly Met
   65                  70                  75                  80
   Ala Met Gly Ala Leu Glu Ala Thr Lys Ala His Gly Lys Lys Leu Pro
                   85                  90                  95
   Ile Phe Xaa Val Xaa Ala Leu Pro Glu Val Leu Gln Leu Ile Lys Lys
               100                 105                 110
   Gly Glu Ile Ala Gly Thr Val Leu Asn Asp Gly Val Asn Gln Gly Lys
           115                 120                 125
   Ala Val Val Gln Leu Ser Asn Asn Leu Ala Lys Gly Lys Pro Ala Thr
       130                 135                 140
   Glu Gly Thr Lys Trp Gln Leu Lys Arg Ser Cys Pro Thr Tyr Pro Leu
   145                 150                 155                 160
   Cys Trp Cys Gly Cys Gly
                   165
<210> 39
   <211> 2628
   <212> DNA
   <213> Pasteurella Multocida
<220>
   <221> misc_feature
   <223> mioC
<220>
   <221> CDS
   <222> (326) .. (766)
<220>
   <221> misc_feature
   <222> (1839) .. (1839)
   <223> n is a, c, g, or t
<400> 39
   gtcaactaga gtaaaataga cacacttaat tacattgtag aggaatcctt ttatgtcttt      60
   agaaatttta gatcagttag aagaaaaaat taaacaagcg gttgaaacta tccaattact     120
   tcaattggaa attgatgaat taaaagaaaa aaataaccaa tctcaacaag caaatgacgc     180
   attacgcagt gaaaatgaac aactaaagag tgagcaccaa aactggcaag aacgtttacg     240
   ctcattatta ggcaaaattg ataacgtata attcacttct tattaaggct tagtttttct     300
   aagccttatt ttttaggaga aatta atg aaa aca aaa att tgt att atc act       352
                               Met Lys Thr Lys Ile Cys Ile Ile Thr
                               1               5
   ggc agt acg ctt ggt ggt gca gaa tat gtt gca gaa cat att gct gaa       400
   Gly Ser Thr Leu Gly Gly Ala Glu Tyr Val Ala Glu His Ile Ala Glu
   10                  15                  20                  25
   ata tta gaa caa caa gat tat cct gta cgt tta gaa cat gga cca aat       448
   Ile Leu Glu Gln Gln Asp Tyr Pro Val Arg Leu Glu His Gly Pro Asn
                   30                  35                  40
   ttt gaa gaa gtg atc gat gaa aaa tgt tgg ctt gtt gtc acc tct acc       496
   Phe Glu Glu Val Ile Asp Glu Lys Cys Trp Leu Val Val Thr Ser Thr
               45                  50                  55
   cat ggt gca ggt gaa tta ccg gat aat att aaa cct ctg ttt gaa aaa       544
   His Gly Ala Gly Glu Leu Pro Asp Asn Ile Lys Pro Leu Phe Glu Lys
           60                  65                  70
   tta gca ttt cac cca aaa cag tta gct gac tta cgc ttt gcg gtg atc       592
   Leu Ala Phe His Pro Lys Gln Leu Ala Asp Leu Arg Phe Ala Val Ile
       75                  80                  85
   ggg tta ggt aat tcg gat tat gat acc ttc tgt cac gca gtg gat cat       640
   Gly Leu Gly Asn Ser Asp Tyr Asp Thr Phe Cys His Ala Val Asp His
   90                  95                  100                 105
   gtg gaa caa tta ctg cta agc aaa gat gct tta caa ctg tgt gaa tcg       688
   Val Glu Gln Leu Leu Leu Ser Lys Asp Ala Leu Gln Leu Cys Glu Ser
                   110                 115                 120
   cta aga atg gat atg cta acc att act gat cct gaa cac acg gcc gaa       736
   Leu Arg Met Asp Met Leu Thr Ile Thr Asp Pro Glu His Thr Ala Glu
               125                 130                 135
   caa tgg ctc cca caa ttt ctc agt caa tta taatatttat tccctataca         786
   Gln Trp Leu Pro Gln Phe Leu Ser Gln Leu
           140                 145
   atggcatatg taaatcaaat atgccatttt tcatctcgat caagcataat atttaaccaa     846
   tcaaatcaat attttctctg tggataacta agatcaaaac tgtataaaag ctgtttttat     906
   tccctgaata agattgaatg ttttttattc tgtggataac taaagaagtt attcacagtt     966
   ttttctggtg ccaaattgag atcttaacaa cttaaaaaat gatctaagtt attcatttaa    1026
   aaaaagaaaa ggatcttaat cacagcacta taggatccta ataatcataa taataagatc    1086
   tctttatata aaaagatcct atctttatta actcacgatc tttttcacga tcatcgtaca    1146
   gtcttgatca aaaatgtttc tttcatggat ccataaattt cagtagaata gccaaccagc    1206
   aaaaaggatc aaaagatcca taaaatccga gataaattaa caaggttact atgttttata   1266
   ctgaaaatta tgatgttatt gtgatcggtg gtggacacgc aggtactgaa gctgcacttg   1326
   caccggcacg catgggactc aagaccctat tattaaccca taatgttgat acactagggc   1386
   aaatgtcttg taatcctgcg attggtggga ttggtaaagg ccatttagtc cgagaaattg   1446
   atgcgatggg cggtttaatg gcaactgctg cggaccaagc aggaatccaa tttcgtacct   1506
   taaacagcag caaaggaccg gcggtacgtg ctacacgtgc gcaagctgac cgcgttttat   1566
   atcgccaagc agtacgtatt gcattagaaa atcaagaaaa tttagatatt tttcaacaag   1626
   aagtgaccga tattatttta gatcaggatc gtgtctgcgg tgttgttact aaaatgggtt   1686
   taaaatttca cgcaaaagca gtgattttaa cagccggtac tttcctttct ggtaagatcc   1746
   acattggttt agaaaattat acaggtggac gcgcgggtga tcctgcttca gtgatgttag   1806
   ccgatcgttt aagagaactg aatttacgtg tanatcgttt aaaaacgggt acaccgcccc   1866
   gtattgatgc acgtactatt gatttctcaa tactggctaa acaacatggc gatgaaaaat   1926
   tacctgtctt ttccttcatg ggatctgttg atcaacaccc acgtcaaatt ccatgtttta   1986
   ttacccatac aaatgaacaa acgcatgaag tgatccgtaa taacttacat cgcagcccaa   2046
   tgtatgctgg gatcattgaa gggatcggtc cacgttattg cccttctatt gaagataaag   2106
   taatgcgttt ttctgagcgt aattctcatc aaatctacct tgaacctgaa gggttgacaa   2166
   acaaaacaaa gaaattgcgg atttacaaaa acaagtgcaa gcactgcaag cagatttaag   2226
   cgaaatggca aagaaaaacc gcaatcaagc gttgattgca ggtggtattg gcggtggcat   2286
   tgttgcagtc ggtattgagc tcattcgctt gcaatttggg ggctaactga tggcatttga   2346
   tgaaaaaaca cgtgcgctgg ttcgtcgcta ctatgtattt gagtttttat cgcttgagca   2406
   atcagcaagt aaagctaaag tctcatttaa caccgcgcga cgctggaaga aagaggcggc   2466
   aagcaagggc gatgactggg ataaagtgcg tgatgtacaa gtaatggcgg gcaatgagct   2526
   gactgatatc acaaaaggat tgttatcggg ctttattatt caatatcgcg caaccatgga   2586
   tgagattcaa aactcggatt taaaagcaca agataaagtc ga                      2628
<210> 40
   <211> 147
   <212> PRT
   <213> Pasteurella Multocida
<400> 40
   Met Lys Thr Lys Ile Cys Ile Ile Thr Gly Ser Thr Leu Gly Gly Ala
   1               5                   10                  15
   Glu Tyr Val Ala Glu His Ile Ala Glu Ile Leu Glu Gln Gln Asp Tyr
               20                  25                  30
   Pro Val Arg Leu Glu His Gly Pro Asn Phe Glu Glu Val Ile Asp Glu
           35                  40                  45
   Lys Cys Trp Leu Val Val Thr Ser Thr His Gly Ala Gly Glu Leu Pro
       50                  55                  60
   Asp Asn Ile Lys Pro Leu Phe Glu Lys Leu Ala Phe His Pro Lys Gln
   65                  70                  75                  80
   Leu Ala Asp Leu Arg Phe Ala Val Ile Gly Leu Gly Asn Ser Asp Tyr
                   85                  90                  95
   Asp Thr Phe Cys His Ala Val Asp His Val Glu Gln Leu Leu Leu Ser
               100                 105                 110
   Lys Asp Ala Leu Gln Leu Cys Glu Ser Leu Arg Met Asp Met Leu Thr
           115                 120                 125
   Ile Thr Asp Pro Glu His Thr Ala Glu Gln Trp Leu Pro Gln Phe Leu
       130                 135                 140
   Ser Gln Leu
   145
<210> 41
   <211> 5191
   <212> DNA
   <213> Pasteurella Multocida
<220>
   <221> misc_feature
   <223> mreB
<220>
   <221> CDS
   <222> (3203) .. (4255)
<400> 41
   ctgcagtacc aaccacccaa atgttgtgct tctgctgtaa ttcacgcaaa gtgcgcgcga      60
   ggttggttac acggattaat ggcacgactt cagcggcacc acaagctact ttacgtgcga     120
   tagcggttaa ttgggcagat ttatcttttg gtactatcac agcacacacg cccgctgcat     180
   ccgcagtacg caaacaagca cctaagttat gtggatcagt cacaccatct aacactaata     240
   gcaacggatt ggactgattt tgtaaaagcg tatctaaatc tgcttcattc aattctttcg     300
   ctggctgaac acgtgccatg atgccttggt gtacttcgcc ctctgctttt ttatcaaggg    360
   tttggcgatt aacaaattgg atagtaatac ccaaccgatg aagttcattg agcaaaggtt    420
   gtaaacgttt atcgtcgcgc cctttgagtg cataaacttc aattaaacgc tctggcgcgt    480
   tgtttaaaaa ggcacttact gcatgaatgc cataaatatt ttcactcatc tactttctct    540
   ttttagctga tttcttactg gcttttttcg tggttgttga tggtttaaca acgcttgttt    600
   ttcttaccgc acttttactg gtgcttggtt tacgtttatg gcgttgtttt tcgagacttt    660
   cagcgtagcg tgcatttttc ttgagcttgt ctttggctgt tttgccttct cgtaatggtt    720
   tacgctcact cgacactaaa gaaaaatcca cttggcgttg ttcaaggcta actgcttcca    780
   cacggatttt taccttatcg ccaatgcggt agatcatacc actattttca ccgattaaac    840
   gttgtcgagc gagatcaaat tggtagtagt cgttatctaa ggtagaaatg tggactaagc    900
   catcaataaa gaggtcatct aagcggacaa ataagccaaa acctgtcaca gatgaaatca    960
   cacctgtaaa ttcttcgcct acatgatctt gcatatattc gcattttagc cagtctgcaa   1020
   catcacgagt ggcatcatcg gcccgtcgtt ctgtcatgga gcagtggtcg cctaatacat   1080
   ccatgtcatc aagtgtatag tgataccctc ccgtatcagt ggttctccgt ttcgagcctt   1140
   ttagtttggc taacaagtat ttaatcccac gatgcaaagt caaatcagga taacggcgaa   1200
   ttggcgaagt aaaatgcgca tattcttcga gtgcaagccc aaagtgccca atattgtcag   1260
   gatgataaac ggcttgactt aacgaacgta atacatggtt tgcaataact catgatctgg   1320
   acgttcgcga atttgctcca ataatttagc gtagtctgcg gtacttggtt tactgccacc   1380
   ttcaaggctt aaaccacatt cactgaggaa ttggcggaag cccgtcactt tttcttcgct   1440
   tggacccgca tgaatacgga aaagggctgg ctcttgatgt ttttccataa agtttgccgc   1500
   cgcaatattg gcgaggatca tacattcttc aatgatttta tgcgcatcat tacgaataac   1560
   aggctcaatc cgttcgattc gccccatttc attaaacaca aacttacttt caatggtttc   1620
   aaagtcaatg gcgccacgtt gatgacgggc ttccactaag gcttggtaca ttacatggag   1680
   ttcttttaaa tggggaacca gtgcttgata acgagtacaa agttcttcat cgtcctctaa   1740
   aatacgagca actttggtat aggttaagcg agcatgagag ttcatgaccg cttcataaaa   1800
   ttcatagcct gtgattttac cttttgctga aacattcagc tcacagacca tacataagcg   1860
   atcgacttgt gggtttaatg aacaaagtcc attggagagg atttccggta gcataggaac   1920
   aacgcgattt gggaaatata ccgagtttcc acgcgcatgt gcttcggtat caagggcagt   1980
   acgtaaacga acatagtagc tgacatcggc aatggcaacc cacagtttcc aaccgcctcc   2040
   acgttttttc tggcaaaaaa ccgcatcgtc aaaatcgcga gcatcttcac catcaatggt    2100
   gactaacgga agatggcgta aatcaatacg tcctgatttg gcttcttccg gtacttcttc   2160
   actcagttta gaaacttgtt tgaggaccgc gtctggaaaa acatggggaa tatcatgatt   2220
   acggatagca atttccacct ccatcccttt tgccatattt tcaccgagaa tttcgctaat   2280
   cattccaaca ggttggctaa atgttgcggt acgtggtttt aattcaacta caaccacttg   2340
   tcccatacga gcgccttggc ggtgttcatt cggaattaaa atgtcgcgat taattcgact   2400
   atcgtcaggt actacataac caataccatc ttctaagaaa aaacgaccaa caatctgttt   2460
   tttacgctgt tgtaagacgc ggacaatccg cacttcttgg cgaccacgac ggtcaaaacc   2520
   gctaggttgg gcgaggacat agtcaccgtg cattactcgc tgcatttggc tgttgggtat   2580
   aaaccaatcg ctgtctttac cttcgacttg taaaaaacca taaccatcac gatgacctaa   2640
   tacggtccct tttaataaat ccagtttttc cggtaaagcg tagcgtttac gtttagtgaa   2700
   aaccaattgt ccgtcatttt ccatcgctct taagcgacgg cgcatggctt cttgttgttc   2760
   ttcattttga atagcaaatg tggtcagtaa ttcttctttt gagataggtg cattatgttg   2820
   acgaattgta tcaagaataa attcgcgact tgggatcggg ttctcatatt ttgcgagttc   2880
   ttcttgatag tttggatctt gcaaatgtgg attgtttttg atttttgcca taatgactcc   2940
   ctttgagaat ggaaatacgc attcgtttaa tgacctcaag tttgacactc tgatttgttg   3000
   aaagcaagta aaaaacctgt tcgtgactga aacaatctgt actcgcggga tttgtcgtgc   3060
   atgaaagcgt aaagacaaag caaatttcga caatttacag aaaattctct gttataaagg   3120
   tacatttttt gatacattta gcacaatttt tcaatactca aatcagagtg tccattattt   3180
   aatttagttc agcggaattc tt atg tta ttt aaa aaa att cga ggc tta ttt    3232
                            Met Leu Phe Lys Lys Ile Arg Gly Leu Phe
                            1               5                   10
   tca aat gat ctg tcc atc gat ctt ggc aca gcg aat acc tta att tat    3280
   Ser Asn Asp Leu Ser Ile Asp Leu Gly Thr Ala Asn Thr Leu Ile Tyr
                   15                  20                  25
   gtc aaa gga caa ggg att gtt tta gat gaa cct tct gtt gtg gcg att    3328
   Val Lys Gly Gln Gly Ile Val Leu Asp Glu Pro Ser Val Val Ala Ile
               30                  35                  40
   cgc caa gaa cgt tca ggt gca tta aaa agc att gct gcg gtt ggt cgt    3376
   Arg Gln Glu Arg Ser Gly Ala Leu Lys Ser Ile Ala Ala Val Gly Arg
           45                  50                  55
   gat gcc aaa tta atg tta ggc cgt aca ccg aaa agc att gca gcg att    3424
   Asp Ala Lys Leu Met Leu Gly Arg Thr Pro Lys Ser Ile Ala Ala Ile
       60                  65                  70
   cgt cct atg aaa gat ggg gtg atc gca gat ttc ttt gtg aca gaa aaa    3472
   Arg Pro Met Lys Asp Gly Val Ile Ala Asp Phe Phe Val Thr Glu Lys
   75                  80                  85                  90
   atg ttg caa tat ttt att aaa caa gtg cac agc agc aat ttt atg cgt    3520
   Met Leu Gln Tyr Phe Ile Lys Gln Val His Ser Ser Asn Phe Met Arg
                   95                  100                 105
   cca agt cca cgt gtc tta gtt tgt gta cct gcg gga gct acg caa gtc      3568
   Pro Ser Pro Arg Val Leu Val Cys Val Pro Ala Gly Ala Thr Gln Val
               110                 115                 120
   gaa cga cgt gca atc aaa gaa tct gcc att ggt gct ggg gca cgc gag      3616
   Glu Arg Arg Ala Ile Lys Glu Ser Ala Ile Gly Ala Gly Ala Arg Glu
           125                 130                 135
   gtg tac ttg att gag gaa ccg atg gcg gca gcg att ggt gct aaa tta      3664
   Val Tyr Leu Ile Glu Glu Pro Met Ala Ala Ala Ile Gly Ala Lys Leu
       140                 145                 150
   cct gtt tcg act gcc aca ggt tcg atg gtg atc gat atc ggt ggt ggt      3712
   Pro Val Ser Thr Ala Thr Gly Ser Met Val Ile Asp Ile Gly Gly Gly
   155                 160                 165                 170
   acg acg gaa gtt gcg gtg att tct tta aat ggc att gtg tat tcc tct      3760
   Thr Thr Glu Val Ala Val Ile Ser Leu Asn Gly Ile Val Tyr Ser Ser
                   175                 180                 185
   tca gtc cgc att ggt ggt gat cgt ttt gat gag gcg att att tct tat      3808
   Ser Val Arg Ile Gly Gly Asp Arg Phe Asp Glu Ala Ile Ile Ser Tyr
               190                 195                 200
   gta cgc aag acg ttc ggt tca att att ggg gaa ccg aca gca gag cgt      3856
   Val Arg Lys Thr Phe Gly Ser Ile Ile Gly Glu Pro Thr Ala Glu Arg
           205                 210                 215
   atc aaa caa gag att ggt agt gcg ttt att caa gaa ggc gat gaa gtc      3904
   Ile Lys Gln Glu Ile Gly Ser Ala Phe Ile Gln Glu Gly Asp Glu Val
       220                 225                 230
   cgt gaa att gaa gtg cat ggt cat aac tta gca gaa ggt gcg ccg cgt      3952
   Arg Glu Ile Glu Val His Gly His Asn Leu Ala Glu Gly Ala Pro Arg
   235                 240                 245                 250
   tct ttc aaa ctc acc tca cgt gat gtg tta gaa gct att caa gcc ccg      4000
   Ser Phe Lys Leu Thr Ser Arg Asp Val Leu Glu Ala Ile Gln Ala Pro
                   255                 260                 265
   tta aat ggc att gtt gcg gca gtg cgc acg gcc ttg gaa gag tgt caa      4048
   Leu Asn Gly Ile Val Ala Ala Val Arg Thr Ala Leu Glu Glu Cys Gln
               270                 275                 280
   cca gaa cat gct gcg gat att ttt gaa cgt ggc atg gtc tta act ggt      4096
   Pro Glu His Ala Ala Asp Ile Phe Glu Arg Gly Met Val Leu Thr Gly
           285                 290                 295
   ggc ggt gcc ctt att cgt aat att gat gtt tta ctg tca aaa gaa acc      4144
   Gly Gly Ala Leu Ile Arg Asn Ile Asp Val Leu Leu Ser Lys Glu Thr
       300                 305                 310
   ggt gtg ccg gtt atc atc gcc gat gat cct tta acc tgt gtt gcc cgt      4192
   Gly Val Pro Val Ile Ile Ala Asp Asp Pro Leu Thr Cys Val Ala Arg
   315                 320                 325                 330
   ggt ggt ggc gag gca tta gag atg atc gat atg cac ggt ggt gat att      4240
   Gly Gly Gly Glu Ala Leu Glu Met Ile Asp Met His Gly Gly Asp Ile
                   335                 340                 345
   ttt agt gac gat atc taatatgatt taaaagtgcg gtgatattag accgcacttt     4295
   Phe Ser Asp Asp Ile
               350
   tacttctctt ttattgctga caaggctagc ctaattcgta tatgaaacct atttttggaa   4355
   aagcacctcc tttaggtctt cgcttaattc tggcgatttt agcatccatt gcattgattg   4415
   tttcggacgg tcaatccaat gcgatgatta aagcacgcag tattatggaa accgcagtag   4475
   gcgggctgta ttatcttgcc aatacaccga gaacggtatt ggatggggtt tcagataatt   4535
   tggttgatac caataaattg caaattgaaa accgagtttt gcgtgatcaa ctgcgtgaaa   4595
   aaaatgcaga tttattgttg ttagatcaac tcaaagtaga aaatcaacgc ctgcgcttat   4655
   tgcttaattc ccctctacgt acagatgagt ataaaaaaat tgctgaagtt ttaacggcag   4715
   aaactgatgt gtatcgtaag caagtcgtga ttaaccaagg acaacgtgac ggtgcttatg   4775
   tcgggcagcc gattattgat gaaaagggta ttgttgggca acttatctcc gttggtgaaa   4835
   atacgagtcg cgttcttcta ttgacagatg tgactcattc tattccagta caagtactac   4895
   gtaatgatgt ccgtttgatt gctagtggaa caggacggaa tgatgaactg agtttagatc   4955
   atgtgccgcg ttcggtcgat attgtcaaag gggatttatt agtcacttct ggattaggtg   5015
   ggcgtttttt agaaggttat cctgttgcca ttgtggaatc cgtatcacgt gatgggcaaa   5075
   attattttgc tactgtaaca gcaaagccat tagcttcgat tgaacgttta cgctatgttt   5135
   tgcttttatg gccgacgaat gaagagatgc gcaaagtcca gtctatttca cctgca       5191
<210> 42
   <211> 351
   <212> PRT
   <213> Pasteurella Multocida
<400> 42
   Met Leu Phe Lys Lys Ile Arg Gly Leu Phe Ser Asn Asp Leu Ser Ile
   1               5                   10                  15
   Asp Leu Gly Thr Ala Asn Thr Leu Ile Tyr Val Lys Gly Gln Gly Ile
               20                  25                  30
   Val Leu Asp Glu Pro Ser Val Val Ala Ile Arg Gln Glu Arg Ser Gly
           35                  40                  45
   Ala Leu Lys Ser Ile Ala Ala Val Gly Arg Asp Ala Lys Leu Met Leu
       50                  55                  60
   Gly Arg Thr Pro Lys Ser Ile Ala Ala Ile Arg Pro Met Lys Asp Gly
   65                  70                  75                  80
   Val Ile Ala Asp Phe Phe Val Thr Glu Lys Met Leu Gln Tyr Phe Ile
                   85                  90                  95
   Lys Gln Val His Ser Ser Asn Phe Met Arg Pro Ser Pro Arg Val Leu
               100                 105                 110
   Val Cys Val Pro Ala Gly Ala Thr Gln Val Glu Arg Arg Ala Ile Lys
           115                 120                 125
   Glu Ser Ala Ile Gly Ala Gly Ala Arg Glu Val Tyr Leu Ile Glu Glu
       130                 135                 140
   Pro Met Ala Ala Ala Ile Gly Ala Lys Leu Pro Val Ser Thr Ala Thr
   145                 150                 155                 160
   Gly Ser Met Val Ile Asp Ile Gly Gly Gly Thr Thr Glu Val Ala Val
                   165                 170                 175
   Ile Ser Leu Asn Gly Ile Val Tyr Ser Ser Ser Val Arg Ile Gly Gly
               180                 185                 190
   Asp Arg Phe Asp Glu Ala Ile Ile Ser Tyr Val Arg Lys Thr Phe Gly
           195                 200                 205
   Ser Ile Ile Gly Glu Pro Thr Ala Glu Arg Ile Lys Gln Glu Ile Gly
       210                 215                 220
   Ser Ala Phe Ile Gln Glu Gly Asp Glu Val Arg Glu Ile Glu Val His
   225                 230                 235                 240
   Gly His Asn Leu Ala Glu Gly Ala Pro Arg Ser Phe Lys Leu Thr Ser
                   245                 250                 255
   Arg Asp Val Leu Glu Ala Ile Gln Ala Pro Leu Asn Gly Ile Val Ala
               260                 265                 270
   Ala Val Arg Thr Ala Leu Glu Glu Cys Gln Pro Glu His Ala Ala Asp
           275                 280                 285
   Ile Phe Glu Arg Gly Met Val Leu Thr Gly Gly Gly Ala Leu Ile Arg
       290                 295                 300
   Asn Ile Asp Val Leu Leu Ser Lys Glu Thr Gly Val Pro Val Ile Ile
   305                 310                 315                 320
   Ala Asp Asp Pro Leu Thr Cys Val Ala Arg Gly Gly Gly Glu Ala Leu
                   325                 330                 335
   Glu Met Ile Asp Met His Gly Gly Asp Ile Phe Ser Asp Asp Ile
               340                 345                 350
<210> 43
   <211> 2172
   <212> DNA
   <213> Pasteurella Multocida
<220>
   <221> misc_feature
   <223> pnp
<220>
   <221> CDS
   <222> (1)..(1464)
<400> 43
   acc cgc gtt ggg att ggg tgg cac ctg aac cca aat acc gcg tta att       48
   Thr Arg Val Gly Ile Gly Trp His Leu Asn Pro Asn Thr Ala Leu Ile
   1               5                   10                  15
   gaa aaa gtg aaa gcg att gca gaa gcg cgt tta ggc gaa gca tac cgt       96
   Glu Lys Val Lys Ala Ile Ala Glu Ala Arg Leu Gly Glu Ala Tyr Arg
               20                  25                  30
   atc act gaa aac aag cac gtt atg aac aaa att gat gcg att aaa gct      144
   Ile Thr Glu Asn Lys His Val Met Asn Lys Ile Asp Ala Ile Lys Ala
           35                  40                  45
   gat gtg att gca caa atc aca gct gaa gta gca gaa ggc gaa gac atc      192
   Asp Val Ile Ala Gln Ile Thr Ala Glu Val Ala Glu Gly Glu Asp Ile
       50                  55                  60
   agt gaa ggg aaa att gtc gat att ttc acc gca ctt gaa agc caa atc      240
   Ser Glu Gly Lys Ile Val Asp Ile Phe Thr Ala Leu Glu Ser Gln Ile
   65                  70                  75                  80
   gta cgt agc cgt atc att gct ggt gaa cca cgt att gat ggt cgt aca      288
   Val Arg Ser Arg Ile Ile Ala Gly Glu Pro Arg Ile Asp Gly Arg Thr
                   85                  90                  95
   gtg gat act gtt cgt gca tta gat att tgt act ggt gtt tta cca cgt      336
   Val Asp Thr Val Arg Ala Leu Asp Ile Cys Thr Gly Val Leu Pro Arg
               100                 105                 110
   aca cac ggt tct gcg att ttc acc cgt ggt gaa aca cag gcg tta gct      384
   Thr His Gly Ser Ala Ile Phe Thr Arg Gly Glu Thr Gln Ala Leu Ala
           115                 120                 125
   gtc gcg aca tta ggt aca gaa cgt gat gca caa att att gat gaa tta      432
   Val Ala Thr Leu Gly Thr Glu Arg Asp Ala Gln Ile Ile Asp Glu Leu
       130                 135                 140
   aca ggt gag cgt tca gat cac ttc tta ttc cac tac aac ttc ccg cca      480
   Thr Gly Glu Arg Ser Asp His Phe Leu Phe His Tyr Asn Phe Pro Pro
   145                 150                 155                 160
   tat tct gtg ggt gaa acc ggt atg att ggt tca cca aaa cgt cgt gaa      528
   Tyr Ser Val Gly Glu Thr Gly Met Ile Gly Ser Pro Lys Arg Arg Glu
                   165                 170                 175
   att ggt cat ggt cgt tta gcg aaa cgc ggt gta gct gca gtg atg cca      576
   Ile Gly His Gly Arg Leu Ala Lys Arg Gly Val Ala Ala Val Met Pro
               180                 185                 190
   aca ctt gcc gag ttc ccg tat gtg gta cgt gtt gtc tct gaa atc aca      624
   Thr Leu Ala Glu Phe Pro Tyr Val Val Arg Val Val Ser Glu Ile Thr
           195                 200                 205
   gaa tca aat ggt tct tct tct atg gca tcg gtt tgt ggt gcg tct tta      672
   Glu Ser Asn Gly Ser Ser Ser Met Ala Ser Val Cys Gly Ala Ser Leu
       210                 215                 220
   gca tta atg gat gcg ggt gta cca att aaa gcg gcg gtt gca ggt att      720
   Ala Leu Met Asp Ala Gly Val Pro Ile Lys Ala Ala Val Ala Gly Ile
   225                 230                 235                 240
   gca atg ggc tta gtc aaa gaa gac gaa aaa ttt gtg gtg ctt tca gac      768
   Ala Met Gly Leu Val Lys Glu Asp Glu Lys Phe Val Val Leu Ser Asp
                   245                 250                 255
   atc tta ggt gat gaa gat cac tta ggt gac atg gac ttc aaa gtc gcg      816
   Ile Leu Gly Asp Glu Asp His Leu Gly Asp Met Asp Phe Lys Val Ala
               260                 265                 270
   ggt aca cgt acg ggt gtg acg gca tta caa atg gat atc aaa atc gaa      864
   Gly Thr Arg Thr Gly Val Thr Ala Leu Gln Met Asp Ile Lys Ile Glu
           275                 280                 285
   ggt atc aca gca gaa atc atg caa att gcg tta aac caa gcg aaa agc      912
   Gly Ile Thr Ala Glu Ile Met Gln Ile Ala Leu Asn Gln Ala Lys Ser
       290                 295                 300
   gca cgt tta cac att tta ggt gtg atg gag caa gcg atc cca gcg cca      960
   Ala Arg Leu His Ile Leu Gly Val Met Glu Gln Ala Ile Pro Ala Pro
   305                 310                 315                 320
   cgt gcg gat att tct gat ttt gca ccg cgt att tac act atg aaa att     1008
   Arg Ala Asp Ile Ser Asp Phe Ala Pro Arg Ile Tyr Thr Met Lys Ile
                   325                 330                 335
   gat ccg aag aaa atc aaa gat gtg atc ggt aaa ggt ggt gca acc att     1056
   Asp Pro Lys Lys Ile Lys Asp Val Ile Gly Lys Gly Gly Ala Thr Ile
               340                 345                 350
   cgt gcc tta aca gaa gaa aca ggt acc tca att gat atc gat gat gat     1104
   Arg Ala Leu Thr Glu Glu Thr Gly Thr Ser Ile Asp Ile Asp Asp Asp
           355                 360                 365
   ggt acg gtg aag att gct gcg gtt gat ggc aat tca gca aaa gag gtg     1152
   Gly Thr Val Lys Ile Ala Ala Val Asp Gly Asn Ser Ala Lys Glu Val
       370                 375                 380
   atg gcg cgt att gaa gat att act gca gaa gtt gaa gcg ggt gca gtg     1200
   Met Ala Arg Ile Glu Asp Ile Thr Ala Glu Val Glu Ala Gly Ala Val
   385                 390                 395                 400
   tat aaa ggt aaa gtt act cgt tta gct gat ttt ggt gcc ttc gtt tct      1248
   Tyr Lys Gly Lys Val Thr Arg Leu Ala Asp Phe Gly Ala Phe Val Ser
                   405                 410                 415
   atc gta ggt aac aaa gaa ggc tta gtg cat att tct caa atc gcg gaa      1296
   Ile Val Gly Asn Lys Glu Gly Leu Val His Ile Ser Gln Ile Ala Glu
               420                 425                 430
   gag cgt gtt gag aaa gtg agt gat tat ctt gca gtg ggg caa gaa gtg      1344
   Glu Arg Val Glu Lys Val Ser Asp Tyr Leu Ala Val Gly Gln Glu Val
           435                 440                 445
   act gtt aaa gtg gtt gag att gat cgt caa ggt cgt att cgt tta acc      1392
   Thr Val Lys Val Val Glu Ile Asp Arg Gln Gly Arg Ile Arg Leu Thr
       450                 455                 460
   atg aaa gaa gtt gca cca aag caa gaa cac gtt gat tct gtt gtc gca      1440
   Met Lys Glu Val Ala Pro Lys Gln Glu His Val Asp Ser Val Val Ala
   465                 470                 475                 480
   gac gtt gcc gca gaa gaa aac gca taagcaataa acaccaacgc ccttcgtgat     1494
   Asp Val Ala Ala Glu Glu Asn Ala
                   485
   aaagggcgtt ggtgtgcatg ttgataagta caatttgtgc tttaaggcga agcgaaatga    1554
   agcaatttca tccgtggtta aagtgcctgc taattttccc attttgggta tgttgtttaa    1614
   cagcttgtgt taatcatgaa caagtttttc tttcaaaaga gaaattaatg ttagcagagc    1674
   aacatccgaa tgatcatctt gagcatgagg tgatggttgc gcaaattagc gaattgttac    1734
   ttgttaaagg gttaaaaaaa gaagaacgtg cgattttaca ttttgagcga ggcgtgctgt    1794
   acgatagctt aggattgtgg gcattggcgc gttatgattt tgaccaaaca ttagcgttgt    1854
   atccaaagtt ggcagcagcg tttaattatt taggtttata tttattgtta gaggaagatt    1914
   acagcgcatc tctagatatc tttaatgtgt tgtttgaact tgatcctcaa tatgagtatg    1974
   cattcctaaa tagagggcta aatttttatt acgtcggacg ttatgaatta gctcagcggg    2034
   attttcttca attttatcaa gccgataaat cagatccata ccgcacttta tggctttatt      2094
   taaatgaatt aaagcataat cctcaggatg cttctaaaaa tcttgctcaa cgagcaatgg    2154
   ggctttctga tgaatatt                                                  2172
<210> 44
   <211> 488
   <212> PRT
   <213> Pasteurella Multocida
<400> 44
   Thr Arg Val Gly Ile Gly Trp His Leu Asn Pro Asn Thr Ala Leu Ile
   1               5                   10                  15
   Glu Lys Val Lys Ala Ile Ala Glu Ala Arg Leu Gly Glu Ala Tyr Arg
               20                  25                  30
   Ile Thr Glu Asn Lys His Val Met Asn Lys Ile Asp Ala Ile Lys Ala
           35                  40                  45
   Asp Val Ile Ala Gln Ile Thr Ala Glu Val Ala Glu Gly Glu Asp Ile
       50                  55                  60
   Ser Glu Gly Lys Ile Val Asp Ile Phe Thr Ala Leu Glu Ser Gln Ile
   65                  70                  75                  80
   Val Arg Ser Arg Ile Ile Ala Gly Glu Pro Arg Ile Asp Gly Arg Thr
                   85                  90                  95
   Val Asp Thr Val Arg Ala Leu Asp Ile Cys Thr Gly Val Leu Pro Arg
               100                 105                 110
   Thr His Gly Ser Ala Ile Phe Thr Arg Gly Glu Thr Gln Ala Leu Ala
           115                 120                 125
   Val Ala Thr Leu Gly Thr Glu Arg Asp Ala Gln Ile Ile Asp Glu Leu
       130                 135                 140
   Thr Gly Glu Arg Ser Asp His Phe Leu Phe His Tyr Asn Phe Pro Pro
   145                 150                 155                 160
   Tyr Ser Val Gly Glu Thr Gly Met Ile Gly Ser Pro Lys Arg Arg Glu
                   165                 170                 175
   Ile Gly His Gly Arg Leu Ala Lys Arg Gly Val Ala Ala Val Met Pro
               180                 185                 190
   Thr Leu Ala Glu Phe Pro Tyr Val Val Arg Val Val Ser Glu Ile Thr
           195                 200                 205
   Glu Ser Asn Gly Ser Ser Ser Met Ala Ser Val Cys Gly Ala Ser Leu
       210                 215                 220
   Ala Leu Met Asp Ala Gly Val Pro Ile Lys Ala Ala Val Ala Gly Ile
   225                 230                 235                 240
   Ala Met Gly Leu Val Lys Glu Asp Glu Lys Phe Val Val Leu Ser Asp
                   245                 250                 255
   Ile Leu Gly Asp Glu Asp His Leu Gly Asp Met Asp Phe Lys Val Ala
   260 265 270
   Gly Thr Arg Thr Gly Val Thr Ala Leu Gln Met Asp Ile Lys Ile Glu
   275 280 285
   Gly Ile Thr Ala Glu Ile Met Gln Ile Ala Leu Asn Gln Ala Lys Ser
   290 295 300
   Ala Arg Leu His Ile Leu Gly Val Met Glu Gln Ala Ile Pro Ala Pro
   305 310 315 320
   Arg Ala Asp Ile Ser Asp Phe Ala Pro Arg Ile Tyr Thr Met Lys Ile
   325 330 335
   Asp Pro Lys Lys Ile Lys Asp Val Ile Gly Lys Gly Gly Ala Thr Ile
   340 345 350
   Arg Ala Leu Thr Glu Glu Thr Gly Thr Ser Ile Asp Ile Asp Asp Asp
   355 360 365
   Gly Thr Val Lys Ile Ala Ala Val Asp Gly Asn Ser Ala Lys Glu Val
   370 375 380
   Met Ala Arg Ile Glu Asp Ile Thr Ala Glu Val Glu Ala Gly Ala Val
   385 390 395 400
   Tyr Lys Gly Lys Val Thr Arg Leu Ala Asp Phe Gly Ala Phe Val Ser
   405 410 415
   Ile Val Gly Asn Lys Glu Gly Leu Val His Ile Ser Gln Ile Ala Glu
   420 425 430
   Glu Arg Val Glu Lys Val Ser Asp Tyr Leu Ala Val Gly Gln Glu Val
   435 440 445
   Thr Val Lys Val Val Glu Ile Asp Arg Gln Gly Arg Ile Arg Leu Thr
   450 455 460
   Met Lys Glu Val Ala Pro Lys Gln Glu His Val Asp Ser Val Val Ala
   465 470 475 480
   Asp Val Ala Ala Glu Glu Asn Ala
   485
<210> 45
   <211> 633
   <212> DNA
   <213> Pasteurella Multocida
<220>
   <221> misc_feature
   <223> purF
<220>
   <221> CDS
   <222> (2)..(631)
<400> 45
   c gat ggg gtt tct gtt tat gct gcc cgt gtt cat atg gga caa cgt tta      49
     Asp Gly Val Ser Val Tyr Ala Ala Arg Val His Met Gly Gln Arg Leu
     1               5                   10                  15
   ggt gaa aaa att gca cgg gaa tgg gcg gat gtg gat gat att gat gtg        97
   Gly Glu Lys Ile Ala Arg Glu Trp Ala Asp Val Asp Asp Ile Asp Val
               20                  25                  30
   gtc att cct gtg cct gaa acc tct aac gat att gct tta cgt att gcg       145
   Val Ile Pro Val Pro Glu Thr Ser Asn Asp Ile Ala Leu Arg Ile Ala
           35                  40                  45
   cgc gtg tta aat aaa ccg tat cgt caa ggt ttt gtg aaa aat cgc tat       193
   Arg Val Leu Asn Lys Pro Tyr Arg Gln Gly Phe Val Lys Asn Arg Tyr
       50                  55                  60
   gta gga cgt acg ttt att atg ccg ggg cag gca ttg cga gtc agt tct       241
   Val Gly Arg Thr Phe Ile Met Pro Gly Gln Ala Leu Arg Val Ser Ser
   65                  70                  75                  80
   gtt aga cgt aaa ctc aat acc att gct tca gaa ttt aaa gat aag aat       289
   Val Arg Arg Lys Leu Asn Thr Ile Ala Ser Glu Phe Lys Asp Lys Asn
                   85                  90                  95
   gtg tta tta gtt gac gac tcg att gta cgt ggt acc acg tct gaa caa       337
   Val Leu Leu Val Asp Asp Ser Ile Val Arg Gly Thr Thr Ser Glu Gln
               100                 105                 110
   att gtc gaa atg gcg aga gcg gca ggt gcg aag aaa att tat ttt gcc       385
   Ile Val Glu Met Ala Arg Ala Ala Gly Ala Lys Lys Ile Tyr Phe Ala
           115                 120                 125
   tct gct gca cca gaa att cgt tat cca aat gtg tat ggt att gat atg       433
   Ser Ala Ala Pro Glu Ile Arg Tyr Pro Asn Val Tyr Gly Ile Asp Met
       130                 135                 140
   cca acc aaa aat gaa ttg atc gct tat ggt cgt gat gta gat gaa att       481
   Pro Thr Lys Asn Glu Leu Ile Ala Tyr Gly Arg Asp Val Asp Glu Ile
   145                 150                 155                 160
   gct aac tta att ggt gtg gat aaa ttg att ttc caa gat ttg gat gcg       529
   Ala Asn Leu Ile Gly Val Asp Lys Leu Ile Phe Gln Asp Leu Asp Ala
                   165                 170                 175
   tta act ggt tct gtg caa caa gaa aat cca agt att caa gac ttt gat       577
   Leu Thr Gly Ser Val Gln Gln Glu Asn Pro Ser Ile Gln Asp Phe Asp
               180                 185                 190
   tgt tcg gtg ttt aca ggg gtt tat gtg acg ggc gat att aca cct gaa      625
   Cys Ser Val Phe Thr Gly Val Tyr Val Thr Gly Asp Ile Thr Pro Glu
           195                 200                 205
   tat ctg ga                                                           633
   Tyr Leu
       210
<210> 46
   <211> 210
   <212> PRT
   <213> Pasteurella Multocida
<400> 46
   Asp Gly Val Ser Val Tyr Ala Ala Arg Val His Met Gly Gln Arg Leu
   1               5                   10                  15
   Gly Glu Lys Ile Ala Arg Glu Trp Ala Asp Val Asp Asp Ile Asp Val
               20                  25                  30
   Val Ile Pro Val Pro Glu Thr Ser Asn Asp Ile Ala Leu Arg Ile Ala
           35                  40                  45
   Arg Val Leu Asn Lys Pro Tyr Arg Gln Gly Phe Val Lys Asn Arg Tyr
       50                  55                  60
   Val Gly Arg Thr Phe Ile Met Pro Gly Gln Ala Leu Arg Val Ser Ser
   65                  70                  75                  80
   Val Arg Arg Lys Leu Asn Thr Ile Ala Ser Glu Phe Lys Asp Lys Asn
                   85                  90                  95
   Val Leu Leu Val Asp Asp Ser Ile Val Arg Gly Thr Thr Ser Glu Gln
               100                 105                 110
   Ile Val Glu Met Ala Arg Ala Ala Gly Ala Lys Lys Ile Tyr Phe Ala
           115                 120                 125
   Ser Ala Ala Pro Glu Ile Arg Tyr Pro Asn Val Tyr Gly Ile Asp Met
       130                 135                 140
   Pro Thr Lys Asn Glu Leu Ile Ala Tyr Gly Arg Asp Val Asp Glu Ile
   145                 150                 155                 160
   Ala Asn Leu Ile Gly Val Asp Lys Leu Ile Phe Gln Asp Leu Asp Ala
                   165                 170                 175
   Leu Thr Gly Ser Val Gln Gln Glu Asn Pro Ser Ile Gln Asp Phe Asp
               180                 185                 190
   Cys Ser Val Phe Thr Gly Val Tyr Val Thr Gly Asp Ile Thr Pro Glu
           195                 200                 205
   Tyr Leu
       210
<210> 47
   <211> 4788
   <212> DNA
   <213> Pasteurella Multocida
<220>
   <221> misc_feature
   <223> rci
<220>
   <221> CDS
   <222> (1)..(876)
<220>
   <221> misc_feature
   <222> (3084)..(3084)
   <223> n is a, c, g, or t
<400> 47
   gac gag gag aga aaa tta gct gat ttg gca aaa ggt atc gct cca gat      48
   Asp Glu Glu Arg Lys Leu Ala Asp Leu Ala Lys Gly Ile Ala Pro Asp
   1               5                   10                  15
   att att ttt aga gat gta ata gaa cgc tat caa aat gaa gtg tct ata      96
   Ile Ile Phe Arg Asp Val Ile Glu Arg Tyr Gln Asn Glu Val Ser Ile
               20                  25                  30
   act aaa aaa ggc gcg cga aat gaa att ata aga tta aac cgc ttt tta     144
   Thr Lys Lys Gly Ala Arg Asn Glu Ile Ile Arg Leu Asn Arg Phe Leu
           35                  40                  45
   aga tat gat att tct aat ctg tat att cgt gat tta aga aaa gaa gat     192
   Arg Tyr Asp Ile Ser Asn Leu Tyr Ile Arg Asp Leu Arg Lys Glu Asp
       50                  55                  60
   ttt gag gag tgg atc aga att cgc cta acc gaa gta tcg gat gct agc     240
   Phe Glu Glu Trp Ile Arg Ile Arg Leu Thr Glu Val Ser Asp Ala Ser
   65                  70                  75                  80
   gtt aga cgt gag ctt gtt act ata tcg tca gtg ctg aca aca gca ata     288
   Val Arg Arg Glu Leu Val Thr Ile Ser Ser Val Leu Thr Thr Ala Ile
                   85                  90                  95
   aat aag tgg gga tat att tca agg cat cca atg act ggt att gaa aaa     336
   Asn Lys Trp Gly Tyr Ile Ser Arg His Pro Met Thr Gly Ile Glu Lys
               100                 105                 110
   cca aaa aac tcg gca gaa aga aaa gaa cga tat tca gaa cag gac att     384
   Pro Lys Asn Ser Ala Glu Arg Lys Glu Arg Tyr Ser Glu Gln Asp Ile
           115                 120                 125
   aaa aca ata tta gaa aca gct aga tat tgt gaa gat aaa cta ccc ata      432
   Lys Thr Ile Leu Glu Thr Ala Arg Tyr Cys Glu Asp Lys Leu Pro Ile
       130                 135                 140
   aca ctc aaa caa aga gta gca att gca atg tta ttt gct att gaa acc      480
   Thr Leu Lys Gln Arg Val Ala Ile Ala Met Leu Phe Ala Ile Glu Thr
   145                 150                 155                 160
   gct atg cgt gct ggt gag att gct agt ata aaa tgg gat aat gtt ttt      528
   Ala Met Arg Ala Gly Glu Ile Ala Ser Ile Lys Trp Asp Asn Val Phe
                   165                 170                 175
   ctt gaa aag aga ata gta cat tta ccg aca act aaa aac ggg cac tct      576
   Leu Glu Lys Arg Ile Val His Leu Pro Thr Thr Lys Asn Gly His Ser
               180                 185                 190
   aga gat gtg ccg ctt tcg caa aga gct gtt gcg cta att tta aaa atg      624
   Arg Asp Val Pro Leu Ser Gln Arg Ala Val Ala Leu Ile Leu Lys Met
           195                 200                 205
   aaa gag gta gaa aat gga gat ctt gtg ttt cag acc acg cct gaa tca      672
   Lys Glu Val Glu Asn Gly Asp Leu Val Phe Gln Thr Thr Pro Glu Ser
       210                 215                 220
   tta agc acc acg ttt aga gtg tta aag aaa gag tgt gga ctt gaa cat      720
   Leu Ser Thr Thr Phe Arg Val Leu Lys Lys Glu Cys Gly Leu Glu His
   225                 230                 235                 240
   ctc cat ttt cat gat acg aga agg gaa gcg ttg acg aga tta tct aag      768
   Leu His Phe His Asp Thr Arg Arg Glu Ala Leu Thr Arg Leu Ser Lys
                   245                 250                 255
   aaa gta gat gta atg act cta gcc aaa att agc gga cat aga gat tta      816
   Lys Val Asp Val Met Thr Leu Ala Lys Ile Ser Gly His Arg Asp Leu
               260                 265                 270
   aga att tta caa aac aca tat tac gca ccg aat atg agt gaa gtg gca      864
   Arg Ile Leu Gln Asn Thr Tyr Tyr Ala Pro Asn Met Ser Glu Val Ala
           275                 280                 285
   aac ttg ttg gat taattcactc ttcttaaata cgccttttgc cacttgatta          916
   Asn Leu Leu Asp
       290
   catcgccagc cttatatctt ttactttcat tacttccttt ttctaatgaa actggggatg    976
   gaaagtcttg gcgggtaata atatgacgag atgtgtaatt gtaagaacga ttaatcatga   1036
   tagaaatgtc ttcaatacta agaagaactg gactatcttc tttaagttga gctaaggctc   1096
   caaacctcac agagcgtagc acttcatctt gttgtttctt tgttagagaa atattttcca   1156
   tttttacctc acttaaataa aaaagccgtc atggacggct taataaagca tttcgctata   1216
   cagttctttt gaggtgtacc caatatcgaa cttgatttga ttgttttgtt ttgcgatttc   1276
   cagtgcttct tttcgatcaa caaatcttcc gaagttggta ataacaccct gtttcttacc   1336
   aattagcgga tattgtgtac cgttctcaat agatttaatt tgtttacgca taaacatgtc 1396
   atagtgtctc gcgccagtca ctaaatgaag ttcttttgtt gtgtaatctt caagctcgca 1456
   cgcagcgcag acgattaatt ctggacgatc tcccattttc ctatctccga gcatttttca 1516
   tagcctcaaa ccaagcttgt gcgtctcctt cactgtaaaa taatgaaccg ctttctagca 1576
   tttttctgtg cttatctcta ttagcatcaa aatagaatct aataattgtt gagttacctc 1636
   ttttaaagac ctcttgattg tggtgtgctg ttttgattgg agcgggcaaa gtaagcgtta 1696
   ctgttgggcg tggttcttcc cacattccga ctatatcaaa tggattttct aaatgcggat 1756
   atttgttatg ttcaaaaaaa gaagatccat caatcgtcca agaatttctc tcaaattgtg 1816
   tcttgctctt gccaatttga aatacacctt gatataagaa agtgttgaac ggaaagaact 1876
   ttgaataatc agcagttaca tatcctttac ttccatttct aagcacaact ggctcaccgg 1936
   ttaatgcttt ttctaagtca aatgctttca ttttttactc tccagcttgc tcatcaataa 1996
   gctcatcaat ccattctcga atttcagatt ggaaattttc taacgaatta ttttcattaa 2056
   aataatctgc ttcttttaac ttactcaccg ctgttttact aaagcattta taaaaaagac 2116
   gcttttcttc aaacaaatca tcttttgaga ttgcgcatac ttttcctacg ccctctatac 2176
   ttttgtcttg aataaaacta ttaaaaagaa cctcttcttg ctgcttgttt tctactaaaa 2236
   tagaaatcgt atattttttt ggatattcca tttttaatcc tttcttttag ataacaaaaa 2296
   accgcatttc tgcggttatt ctgtgtattt atttaaaata ttacttatag tttcagcatc 2356
   ttctaatgtt aatttagaat agcttgaggt catttttcct ttaacggcaa tcctcaattc 2416
   ttccagcttt aagctataca agtagtcttt tttatcttta taaatacgac cgtacaccat 2476
   tccggataat ttaccttctt catacccatt agataaattg atctttctat ctcgaccgcg 2536
   cactctaatt gttaaatatt ttcttccaat ttttaagact tcagcctcat gttcaatgtt 2596
   tgagcgcatg agtgtttcta aaaaatatac ggtatcgcca actttaagat ttttaatcca 2656
   atctttattc ataaataccc ttatactttc ggtggcagtg gaagtggttg ccaatgagtt 2716
   actgaagata gatggtaagt acgaagagac ataaagaaaa catcatctcg ccacgcgatt 2776
   tttatttccc ctctatctgt ataaatcaga atatcctctc tctcactggg caatctatct 2836
   tttacactaa tccagccatc atcttgcgaa aattccacaa tttctggctt ttcaagcacc 2896
   aaatcagagc cgtaatcttc tttttcctct tcgctcaact gcttttcctt gcagtcagcc 2956
   ttgccaagga cgacaccgta cacggcataa ggtaaatcat tcaacgcaaa atcctcatga 3016
   tcacacatat catctgcgaa ctcatgagct tctactgcac catctaaaca actttgctta 3076
   gcttgatnta atgtttcgtg taagttaacg atatgtatgt cattagatac atctactgag 3136
   aaaaattttt tttctgtttc tatcatagtt aaattcctta ttcttttatt aatgggcgag 3196
   gaatcggttg ccagtgtgta acgttaatag ctagatcata aattggacca ttctgattta 3256
   tagctaccca aaaatattca ccatcagcaa tttctcttga tgcaattgcg taatgctcgc 3316
   catattcacc ttcacaaaaa gcaatgactg gtgtttcaac ttctggtaac ttgtcatcaa 3376
   cactaatcca gttgctatat tcccaatgaa aattaagctt ctcatagcca ttgatgactt 3436
   gatctaacgc aggtcttaat tgggtttctc caacaataat atgtgttgtt atcttaatct 3496
   ctgggattcc atctattact tcacaccagt ttttttcaat aaagtccaat ctttttttat 3556
   ctgctaaaag actttcatat tctgcttttg tgattgttac tgtttctgtc atagttaaat 3616
   tcctcatgat aaattccatg tttatttacc ttttttctgg cacaaaaaaa cacgctattg 3676
   cgtgctgtct tgattgatat aatcaattaa tctaagccca atccatttca taactggaac 3736
   agccatactg tttccaattg ctttgtatcg tggagaatca gggcattcat ctattgattt 3796
   gttgcgatat gggattttag tgtaatcgtc tggaaatccc tgtaatcttt cacactcacg 3856
   cggcgttagt tttctaacaa ctgattctac tgataaagca acgcaaggaa cattattacc 3916
   accagttccc attcttgctt ttaaggttgg tgaaatatca tcgtgaatac gacaagcttc 3976
   ttcaccctta acctcaaata gaatattttc caatcctcca tttcttccaa tgcaatgcgc 4036
   tgtattttta gaaatgattg gatcttgtga accgtgaaca acaaaagttt cactaccgcc 4096
   agcaagaact ccaccgcttg ctcttaatgt tcccgcaaca tcggatttgc gatattgagc 4156
   aaagcttgtc tcaataaagg cggcaatatc ttttgcctgc gttccgctct gttcaatatt 4216
   ccctcgcacg cttttggact caatgagtat ttgtgcaaca cttcgttttc tagcacttgc 4276
   cacaagaaac actcttttac gacgttgggc aactccgaag tattgagcat cgagaactcg 4336
   ccagcagatt gttcggattg aatgcacata accagcgttc gtccatcttc tccctgtgtg 4396
   ctgcaatggc tcacactctt gaaccagtcc agccagaagg tgtccgaatg cgttgtccga 4456
   ggtggatagc acacccggaa cgttttccca cacgagaatg cacggtggtt tgttgtcatt 4516
   gaatctaaca tagtcgatcg cttctaaaat tttaattaaa actaacgtga gatttcctcg 4576
   ctcatcgtct aaactttgac gattgccagc aactgaaaaa gattgacaag gagtaccacc 4636
   aactaaaacg tctggtgccg gaatttcacg atctaatatt ttctgttgca attcagtcat 4696
   atcaccaaga ttgggaacgt tagggtaatg gtaagctaat actgcgcttg ggaattgctc 4756
   aatttcagaa aaccagagtg gctctgcgaa tt 4788
<210> 48
   <211> 292
   <212> PRT
   <213> Pasteurella Multocida
<400> 48
   Asp Glu Glu Arg Lys Leu Ala Asp Leu Ala Lys Gly Ile Ala Pro Asp
   1               5                   10                  15
   Ile Ile Phe Arg Asp Val Ile Glu Arg Tyr Gln Asn Glu Val Ser Ile
               20                  25                  30
   Thr Lys Lys Gly Ala Arg Asn Glu Ile Ile Arg Leu Asn Arg Phe Leu
           35                  40                  45
   Arg Tyr Asp Ile Ser Asn Leu Tyr Ile Arg Asp Leu Arg Lys Glu Asp
       50                  55                  60
   Phe Glu Glu Trp Ile Arg Ile Arg Leu Thr Glu Val Ser Asp Ala Ser
   65                  70                  75                  80
   Val Arg Arg Glu Leu Val Thr Ile Ser Ser Val Leu Thr Thr Ala Ile
                   85                  90                  95
   Asn Lys Trp Gly Tyr Ile Ser Arg His Pro Met Thr Gly Ile Glu Lys
               100                 105                 110
   Pro Lys Asn Ser Ala Glu Arg Lys Glu Arg Tyr Ser Glu Gln Asp Ile
           115                 120                 125
   Lys Thr Ile Leu Glu Thr Ala Arg Tyr Cys Glu Asp Lys Leu Pro Ile
       130                 135                 140
   Thr Leu Lys Gln Arg Val Ala Ile Ala Met Leu Phe Ala Ile Glu Thr
   145                 150                 155                 160
   Ala Met Arg Ala Gly Glu Ile Ala Ser Ile Lys Trp Asp Asn Val Phe
                   165                 170                 175
   Leu Glu Lys Arg Ile Val His Leu Pro Thr Thr Lys Asn Gly His Ser
               180                 185                 190
   Arg Asp Val Pro Leu Ser Gln Arg Ala Val Ala Leu Ile Leu Lys Met
           195                 200                 205
   Lys Glu Val Glu Asn Gly Asp Leu Val Phe Gln Thr Thr Pro Glu Ser
       210                 215                 220
   Leu Ser Thr Thr Phe Arg Val Leu Lys Lys Glu Cys Gly Leu Glu His
   225                 230                 235                 240
   Leu His Phe His Asp Thr Arg Arg Glu Ala Leu Thr Arg Leu Ser Lys
                   245                 250                 255
   Lys Val Asp Val Met Thr Leu Ala Lys Ile Ser Gly His Arg Asp Leu
               260                 265                 270
   Arg Ile Leu Gln Asn Thr Tyr Tyr Ala Pro Asn Met Ser Glu Val Ala
           275                 280                 285
   Asn Leu Leu Asp
       290
<210> 49
   <211> 1618
   <212> DNA
   <213> Pasteurella Multocida
<220>
   <221> misc_feature
   <223> sopE
<220>
   <221> CDS
   <222> (2)..(1195)
<400> 49
   g ggc gat cta tgt ctg aaa ata tct aca tgg tgt caa agt cac aga atc      49
     Gly Asp Leu Cys Leu Lys Ile Ser Thr Trp Cys Gln Ser His Arg Ile
     1               5                   10                  15
   aat caa gca att cgc aca att caa agt cta tca acc gca gtc atc ggt       97
   Asn Gln Ala Ile Arg Thr Ile Gln Ser Leu Ser Thr Ala Val Ile Gly
               20                  25                  30
   att gtc tgt act gca aat gac gca gac aat gaa aca ttc cca ctc aat      145
   Ile Val Cys Thr Ala Asn Asp Ala Asp Asn Glu Thr Phe Pro Leu Asn
           35                  40                  45
   gaa ccc gtt ctc atc aca aac gtg gca gcg gca att ggc aag gct gga      193
   Glu Pro Val Leu Ile Thr Asn Val Ala Ala Ala Ile Gly Lys Ala Gly
       50                  55                  60
   aaa caa ggc acg ctt tca cgt gcg ctt gac ggg att tct gat gta gtc      241
   Lys Gln Gly Thr Leu Ser Arg Ala Leu Asp Gly Ile Ser Asp Val Val
   65                  70                  75                  80
   aat tgc aaa gtg att gtt gtg cga gtg caa gaa agt gcg caa gaa gac      289
   Asn Cys Lys Val Ile Val Val Arg Val Gln Glu Ser Ala Gln Glu Asp
                   85                  90                  95
   gaa gaa aca aaa gca agt gaa atg aac acg gca att att ggc aca atc      337
   Glu Glu Thr Lys Ala Ser Glu Met Asn Thr Ala Ile Ile Gly Thr Ile
               100                 105                 110
   aca gaa gaa ggg cag tac aca ggc ttg aag gcg tta ttg att gcg aaa      385
   Thr Glu Glu Gly Gln Tyr Thr Gly Leu Lys Ala Leu Leu Ile Ala Lys
           115                 120                 125
   aac aaa ttc ggt atc aaa cca cgt att tta tgt gtg cca aaa ttc gac      433
   Asn Lys Phe Gly Ile Lys Pro Arg Ile Leu Cys Val Pro Lys Phe Asp
       130                 135                 140
   aca aaa gaa gtc gcc aca gag ctt gca agt atc gcc gcc aaa ctc aac      481
   Thr Lys Glu Val Ala Thr Glu Leu Ala Ser Ile Ala Ala Lys Leu Asn
   145                 150                 155                 160
   gca ttt gct tac att tca tgt caa ggg tgt aaa acg aaa gaa caa gcg      529
   Ala Phe Ala Tyr Ile Ser Cys Gln Gly Cys Lys Thr Lys Glu Gln Ala
                   165                 170                 175
   gtg caa tat aaa cgc aac ttc tca caa cgt gaa gtc atg ctg atc atg      577
   Val Gln Tyr Lys Arg Asn Phe Ser Gln Arg Glu Val Met Leu Ile Met
               180                 185                 190
   ggc gat ttt ctg tca ttt aat gtc aac aca tca aaa gtt gag att gac      625
   Gly Asp Phe Leu Ser Phe Asn Val Asn Thr Ser Lys Val Glu Ile Asp
           195                 200                 205
   tat gcc gtc act cgt gcg gcg gca atg cgt gca tat ctt gat aaa gaa      673
   Tyr Ala Val Thr Arg Ala Ala Ala Met Arg Ala Tyr Leu Asp Lys Glu
       210                 215                 220
   cag ggc tgg cat acg tct att tca aat aaa ggc att aat ggc gtg agc      721
   Gln Gly Trp His Thr Ser Ile Ser Asn Lys Gly Ile Asn Gly Val Ser
   225                 230                 235                 240
   ggt gtc aca caa cca ctc tat ttt gac att aac gac agc tcg act gat      769
   Gly Val Thr Gln Pro Leu Tyr Phe Asp Ile Asn Asp Ser Ser Thr Asp
                   245                 250                 255
   gtg aac tat ctc aat gaa caa ggc atc acg tgt tgc gtg aat cat aat      817
   Val Asn Tyr Leu Asn Glu Gln Gly Ile Thr Cys Cys Val Asn His Asn
               260                 265                 270
   ggc ttt cgt ttt tgg ggc tta cgc acg act gca gaa gat cca tta ttc      865
   Gly Phe Arg Phe Trp Gly Leu Arg Thr Thr Ala Glu Asp Pro Leu Phe
           275                 280                 285
   aag ttt gaa gtg tac acc cgc act gca caa atc tta aaa gat acg att      913
   Lys Phe Glu Val Tyr Thr Arg Thr Ala Gln Ile Leu Lys Asp Thr Ile
       290                 295                 300
   gca ggg gcg ttt gat tgg gca gtg gat aaa gat att tct gtc acg cta      961
   Ala Gly Ala Phe Asp Trp Ala Val Asp Lys Asp Ile Ser Val Thr Leu
   305                 310                 315                 320
   gtg aaa gat att att gaa gca atc aat gcg aag tgg cgt gat tac acc     1009
   Val Lys Asp Ile Ile Glu Ala Ile Asn Ala Lys Trp Arg Asp Tyr Thr
                   325                 330                 335
   aca aaa ggc tac tta att ggc ggt aaa gcg tgg ctt aat aaa gag ctt     1057
   Thr Lys Gly Tyr Leu Ile Gly Gly Lys Ala Trp Leu Asn Lys Glu Leu
               340                 345                 350
   aac agt gca acg aat tta aaa gat gcg aag ttg ttg atc tct tat gat     1105
   Asn Ser Ala Thr Asn Leu Lys Asp Ala Lys Leu Leu Ile Ser Tyr Asp
           355                 360                 365
   tat cac cca gta cca ccg ctc gaa cag cta ggc ttt aat cag tac att      1153
   Tyr His Pro Val Pro Pro Leu Glu Gln Leu Gly Phe Asn Gln Tyr Ile
       370                 375                 380
   tct gat gaa tac ctt gtt gat ttt tca aat cgt tta gca tcg              1195
   Ser Asp Glu Tyr Leu Val Asp Phe Ser Asn Arg Leu Ala Ser
   385                 390                 395
   taaggggtag aaaatggctt taccacgcaa acttaaattg atgaatttaa tcatcgacgg    1255
   taacaaatat ctcggcgaag tcacggaagt gactcaacca aaattagcaa tgaaaatcga    1315
   agaatttcgc gcgggcggta tgattggttc ggtggatgtc aatctcgggc ttgaaaagct    1375
   cgaagcggaa tttaaagccg gtggctacat ggtcgaatta attaaaaaat tcggcgggtc    1435
   aatcaacggc attccattgc gttttcttgg ctcatatcag cgtgatgaca cagaagaagt    1495
   cacatctgtt gagcttgtga tgcaaggtcg atttactgaa attgacagcg gaaacagcaa    1555
   agtgggcgat gacactgaac aaacattcaa agtgccttta acgtattaca aaatcattgt    1615
   tga                                                                  1618
<210> 50
   <211> 398
   <212> PRT
   <213> Pasteurella Multocida
<400> 50
   Gly Asp Leu Cys Leu Lys Ile Ser Thr Trp Cys Gln Ser His Arg Ile
   1               5                   10                  15
   Asn Gln Ala Ile Arg Thr Ile Gln Ser Leu Ser Thr Ala Val Ile Gly
               20                  25                  30
   Ile Val Cys Thr Ala Asn Asp Ala Asp Asn Glu Thr Phe Pro Leu Asn
           35                  40                  45
   Glu Pro Val Leu Ile Thr Asn Val Ala Ala Ala Ile Gly Lys Ala Gly
       50                  55                  60
   Lys Gln Gly Thr Leu Ser Arg Ala Leu Asp Gly Ile Ser Asp Val Val
   65                  70                  75                  80
   Asn Cys Lys Val Ile Val Val Arg Val Gln Glu Ser Ala Gln Glu Asp
                   85                  90                  95
   Glu Glu Thr Lys Ala Ser Glu Met Asn Thr Ala Ile Ile Gly Thr Ile
               100                 105                 110
   Thr Glu Glu Gly Gln Tyr Thr Gly Leu Lys Ala Leu Leu Ile Ala Lys
   115 120 125
   Asn Lys Phe Gly Ile Lys Pro Arg Ile Leu Cys Val Pro Lys Phe Asp
   130 135 140
   Thr Lys Glu Val Ala Thr Glu Leu Ala Ser Ile Ala Ala Lys Leu Asn
   145 150 155 160
   Ala Phe Ala Tyr Ile Ser Cys Gln Gly Cys Lys Thr Lys Glu Gln Ala
   165 170 175
   Val Gln Tyr Lys Arg Asn Phe Ser Gln Arg Glu Val Met Leu Ile Met
   180 185 190
   Gly Asp Phe Leu Ser Phe Asn Val Asn Thr Ser Lys Val Glu Ile Asp
   195 200 205
   Tyr Ala Val Thr Arg Ala Ala Ala Met Arg Ala Tyr Leu Asp Lys Glu
   210 215 220
   Gln Gly Trp His Thr Ser Ile Ser Asn Lys Gly Ile Asn Gly Val Ser
   225 230 235 240
   Gly Val Thr Gln Pro Leu Tyr Phe Asp Ile Asn Asp Ser Ser Thr Asp
   245 250 255
   Val Asn Tyr Leu Asn Glu Gln Gly Ile Thr Cys Cys Val Asn His Asn
   260 265 270
   Gly Phe Arg Phe Trp Gly Leu Arg Thr Thr Ala Glu Asp Pro Leu Phe
   275 280 285
   Lys Phe Glu Val Tyr Thr Arg Thr Ala Gln Ile Leu Lys Asp Thr Ile
   290 295 300
   Ala Gly Ala Phe Asp Trp Ala Val Asp Lys Asp Ile Ser Val Thr Leu
   305 310 315 320
   Val Lys Asp Ile Ile Glu Ala Ile Asn Ala Lys Trp Arg Asp Tyr Thr
   325 330 335
   Thr Lys Gly Tyr Leu Ile Gly Gly Lys Ala Trp Leu Asn Lys Glu Leu
   340 345 350
   Asn Ser Ala Thr Asn Leu Lys Asp Ala Lys Leu Leu Ile Ser Tyr Asp
           355                 360                 365
   Tyr His Pro Val Pro Pro Leu Glu Gln Leu Gly Phe Asn Gln Tyr Ile
       370                 375                 380
   Ser Asp Glu Tyr Leu Val Asp Phe Ser Asn Arg Leu Ala Ser
   385                 390                 395
<210> 51
   <211> 353
   <212> DNA
   <213> Pasteurella Multocida
<220>
   <221> misc_feature
   <223> unknown C1
<220>
   <221> CDS
   <222> (1)..(351)
<400> 51
   atg aca tta ttt gat gaa tgt aaa tta gct ctt aga gac gat ttt aat        48
   Met Thr Leu Phe Asp Glu Cys Lys Leu Ala Leu Arg Asp Asp Phe Asn
   1               5                   10                  15
   cta att tgt gat gaa gag aag gat tgt gta atg gat aag ttt tat ttc        96
   Leu Ile Cys Asp Glu Glu Lys Asp Cys Val Met Asp Lys Phe Tyr Phe
               20                  25                  30
   tat ttc ttg gaa aag aaa gag gaa ttt aat ttt caa gat tat tca ttt       144
   Tyr Phe Leu Glu Lys Lys Glu Glu Phe Asn Phe Gln Asp Tyr Ser Phe
           35                  40                  45
   gaa gaa atg tat ata ttt tca aaa atg gaa cct gtg tat gtt tta tgt       192
   Glu Glu Met Tyr Ile Phe Ser Lys Met Glu Pro Val Tyr Val Leu Cys
       50                  55                  60
   gat agc tct aat ata cct ttg ttt agg agt aat tgg gaa ttg att atc       240
   Asp Ser Ser Asn Ile Pro Leu Phe Arg Ser Asn Trp Glu Leu Ile Ile
   65                  70                  75                  80
   aat aat ata tat gat gtt gtc tgt tta tct aca aaa gta ttt ttt cta       288
   Asn Asn Ile Tyr Asp Val Val Cys Leu Ser Thr Lys Val Phe Phe Leu
                   85                  90                  95
   gat gat gaa aag tta atg atg gaa tta ttt cct gaa gat aaa gta aga       336
   Asp Asp Glu Lys Leu Met Met Glu Leu Phe Pro Glu Asp Lys Val Arg
               100                 105                 110
   gtc atc tat aaa aga ta                                                353
   Val Ile Tyr Lys Arg
           115
<210> 52
   <211> 117
   <212> PRT
   <213> Pasteurella Multocida
<400> 52
   Met Thr Leu Phe Asp Glu Cys Lys Leu Ala Leu Arg Asp Asp Phe Asn
   1               5                   10                  15
   Leu Ile Cys Asp Glu Glu Lys Asp Cys Val Met Asp Lys Phe Tyr Phe
               20                  25                  30
   Tyr Phe Leu Glu Lys Lys Glu Glu Phe Asn Phe Gln Asp Tyr Ser Phe
           35                  40                  45
   Glu Glu Met Tyr Ile Phe Ser Lys Met Glu Pro Val Tyr Val Leu Cys
       50                  55                  60
   Asp Ser Ser Asn Ile Pro Leu Phe Arg Ser Asn Trp Glu Leu Ile Ile
   65                  70                  75                  80
   Asn Asn Ile Tyr Asp Val Val Cys Leu Ser Thr Lys Val Phe Phe Leu
                   85                  90                  95
   Asp Asp Glu Lys Leu Met Met Glu Leu Phe Pro Glu Asp Lys Val Arg
               100                 105                 110
   Val Ile Tyr Lys Arg
           115
<210> 53
   <211> 509
   <212> DNA
   <213> Pasteurella Multocida
<220>
   <221> misc_feature
   <223> unknown C2
<220>
   <221> CDS
   <222> (1)..(507)
<400> 53
   atg aaa aat ttt agg aat ata aat att tat agt gat tat gga aag gtt       48
   Met Lys Asn Phe Arg Asn Ile Asn Ile Tyr Ser Asp Tyr Gly Lys Val
   1               5                   10                  15
   gat aag gaa att ata tta gaa ttc gaa aat gaa ttt aat ata aag ctt       96
   Asp Lys Glu Ile Ile Leu Glu Phe Glu Asn Glu Phe Asn Ile Lys Leu
               20                  25                  30
   cct tct tta tac ata gat tta att acg gcg cat aat gct ccg aag agt      144
   Pro Ser Leu Tyr Ile Asp Leu Ile Thr Ala His Asn Ala Pro Lys Ser
           35                  40                  45
   gaa gag aat tgc ttt gaa tat tac aat gag cgt aat gag ccc acg ttt      192
   Glu Glu Asn Cys Phe Glu Tyr Tyr Asn Glu Arg Asn Glu Pro Thr Phe
       50                  55                  60
   tct tcc ttt gga ttt gaa ggg ttt gag aca gag cgg tct agc gcc tct      240
   Ser Ser Phe Gly Phe Glu Gly Phe Glu Thr Glu Arg Ser Ser Ala Ser
   65                  70                  75                  80
   ctt gaa aat ata tat gct cag tat att tat gat gat cca atc tat ggt      288
   Leu Glu Asn Ile Tyr Ala Gln Tyr Ile Tyr Asp Asp Pro Ile Tyr Gly
                   85                  90                  95
   tat gaa cat gtg tat tct ttt ggt agt act ggc gag gga cat ttt atc      336
   Tyr Glu His Val Tyr Ser Phe Gly Ser Thr Gly Glu Gly His Phe Ile
               100                 105                 110
   tgt ttt gat tat cgt gat gat cca aaa ggt gat gaa ccc aaa atc tgt      384
   Cys Phe Asp Tyr Arg Asp Asp Pro Lys Gly Asp Glu Pro Lys Ile Cys
           115                 120                 125
   atc gtg att cac gat gaa tat gat gaa aaa aca ggg aaa atg cga ctg      432
   Ile Val Ile His Asp Glu Tyr Asp Glu Lys Thr Gly Lys Met Arg Leu
       130                 135                 140
   ttt cct ata gca gag aat ttt gaa gcg ttt tta gat agt ttg aaa tca      480
   Phe Pro Ile Ala Glu Asn Phe Glu Ala Phe Leu Asp Ser Leu Lys Ser
   145                 150                 155                 160
   ttt gat gaa atg ata gag aag tat tcg ta                               509
   Phe Asp Glu Met Ile Glu Lys Tyr Ser
                   165
<210> 54
   <211> 169
   <212> PRT
   <213> Pasteurella Multocida
<400> 54
   Met Lys Asn Phe Arg Asn Ile Asn Ile Tyr Ser Asp Tyr Gly Lys Val
   1               5                   10                  15
   Asp Lys Glu Ile Ile Leu Glu Phe Glu Asn Glu Phe Asn Ile Lys Leu
               20                  25                  30
   Pro Ser Leu Tyr Ile Asp Leu Ile Thr Ala His Asn Ala Pro Lys Ser
           35                  40                  45
   Glu Glu Asn Cys Phe Glu Tyr Tyr Asn Glu Arg Asn Glu Pro Thr Phe
       50                  55                  60
   Ser Ser Phe Gly Phe Glu Gly Phe Glu Thr Glu Arg Ser Ser Ala Ser
   65                  70                  75                  80
   Leu Glu Asn Ile Tyr Ala Gln Tyr Ile Tyr Asp Asp Pro Ile Tyr Gly
                   85                  90                  95
   Tyr Glu His Val Tyr Ser Phe Gly Ser Thr Gly Glu Gly His Phe Ile
               100                 105                 110
   Cys Phe Asp Tyr Arg Asp Asp Pro Lys Gly Asp Glu Pro Lys Ile Cys
           115                 120                 125
   Ile Val Ile His Asp Glu Tyr Asp Glu Lys Thr Gly Lys Met Arg Leu
       130                 135                 140
   Phe Pro Ile Ala Glu Asn Phe Glu Ala Phe Leu Asp Ser Leu Lys Ser
   145                 150                 155                 160
   Phe Asp Glu Met Ile Glu Lys Tyr Ser
                   165
<210> 55
   <211> 443
   <212> DNA
   <213> Pasteurella Multocida
<220>
   <221> misc_feature
   <223> unknown C3
<220>
   <221> CDS
   <222> (1)..(441)
<400> 55
   atg ata aaa tat tta gag gga aat att aac tcg ttt ata tcg gca tta      48
   Met Ile Lys Tyr Leu Glu Gly Asn Ile Asn Ser Phe Ile Ser Ala Leu
   1               5                   10                  15
   ggt aaa aac gaa agt aat aaa gat att tta aaa tta gta gaa ata gtt      96
   Gly Lys Asn Glu Ser Asn Lys Asp Ile Leu Lys Leu Val Glu Ile Val
               20                  25                  30
   tct tca gat ttt gaa gtg gat gaa cta agt cat aaa gat gaa cac gag     144
   Ser Ser Asp Phe Glu Val Asp Glu Leu Ser His Lys Asp Glu His Glu
           35                  40                  45
   ata tat tat ttg ttt tat aag agg ggt gtt gaa ttt tgt ttt aaa aga     192
   Ile Tyr Tyr Leu Phe Tyr Lys Arg Gly Val Glu Phe Cys Phe Lys Arg
       50                  55                  60
   ata gat gaa gag tat gtc tta tat tcg gtt ttc ttt ttc ttg gta gag     240
   Ile Asp Glu Glu Tyr Val Leu Tyr Ser Val Phe Phe Phe Leu Val Glu
   65                  70                  75                  80
   gtt gat aat tat ttt tca tgc cca ttt att cat gaa tta ata tgt gat      288
   Val Asp Asn Tyr Phe Ser Cys Pro Phe Ile His Glu Leu Ile Cys Asp
                   85                  90                  95
   ctt aaa cac gga ttc tca ata gag gat att ata agg ttt tta ggg gag      336
   Leu Lys His Gly Phe Ser Ile Glu Asp Ile Ile Arg Phe Leu Gly Glu
               100                 105                 110
   cca aat ttt aaa ggt agt ggc tgg gta aga tat tct tat aat gga aga      384
   Pro Asn Phe Lys Gly Ser Gly Trp Val Arg Tyr Ser Tyr Asn Gly Arg
           115                 120                 125
   aat att cat ttc gaa ttt aat gaa tct aat gaa tta tcc cag att agc      432
   Asn Ile His Phe Glu Phe Asn Glu Ser Asn Glu Leu Ser Gln Ile Ser
       130                 135                 140
   att ttt att ta                                                       443
   Ile Phe Ile
   145
<210> 56
   <211> 147
   <212> PRT
   <213> Pasteurella Multocida
<400> 56
   Met Ile Lys Tyr Leu Glu Gly Asn Ile Asn Ser Phe Ile Ser Ala Leu
   1               5                   10                  15
   Gly Lys Asn Glu Ser Asn Lys Asp Ile Leu Lys Leu Val Glu Ile Val
               20                  25                  30
   Ser Ser Asp Phe Glu Val Asp Glu Leu Ser His Lys Asp Glu His Glu
           35                  40                  45
   Ile Tyr Tyr Leu Phe Tyr Lys Arg Gly Val Glu Phe Cys Phe Lys Arg
       50                  55                  60
   Ile Asp Glu Glu Tyr Val Leu Tyr Ser Val Phe Phe Phe Leu Val Glu
   65                  70                  75                  80
   Val Asp Asn Tyr Phe Ser Cys Pro Phe Ile His Glu Leu Ile Cys Asp
                   85                  90                  95
   Leu Lys His Gly Phe Ser Ile Glu Asp Ile Ile Arg Phe Leu Gly Glu
               100                 105                 110
   Pro Asn Phe Lys Gly Ser Gly Trp Val Arg Tyr Ser Tyr Asn Gly Arg
           115                 120                 125
   Asn Ile His Phe Glu Phe Asn Glu Ser Asn Glu Leu Ser Gln Ile Ser
   130 135 140
   Ile Phe Ile
   145
<210> 57
   <211> 8498
   <212> DNA
   <213> Pasteurella Multocida
<220>
   <221> misc_feature
   <223> unknown C
<400> 57
   gaattcgaat taagcgagaa aattgctgaa acactagaac aaagtcaatt aaatattagt 60
   caattatcaa ttgttgaaat ttatcctttc aatgaagaac aagggatacg ttttcataat 120
   aaaagtgtgg tacaacttaa accagaagag gtggaatggt catcaatcca ttatcttttc 180
   tttgctggcg atattcagca agtcgctcat ctcgcgaaag ccgcagaaat gggttgcgtg 240
   gtgattgata tgaaagggat ttgtgccagc ttgcaagacg tccctgtggt gataccggga 300
   gtaaatcagg aaaaattggt agatttacgt cagcgtaata ttgtgtcctt agccgatcca 360
   caagtgacac aacttgcatt agtcatcgcc tcgttgatgt caaatcacga aatcaaagac 420
   attgccgtaa cctcgttatt acctgcatct tatactaacg gagaaacggt aggtaaatta 480
   gcgggacaaa cagcgcgatt gttaaatggc attccacttg atgaaggcga acaacgttta 540
   gcttttgatg ttttccctac gcctgcatcg catttaaata tgcaaattca caagatcttt 600
   ccacaattag ataatgtcgt atttcattct atccaagtgc ctgttttcta cgggatgggg 660
   caaatggtga gcgtattatc ggattatgca ttagatcctc aatcttgctt agcgagctgg 720
   actgacaatc cgttgatgac ttatcatgca gaaaaatatt gcaccccagt gacgaatggc 780
   gaacaggaaa tggcagaaga gcaagcagca aaattacata taagtgggtt aagtgcggtg 840
   gaaaatggtc tacaattttg gtcggttgca gatgaacagc gctttaatct tgctttattg 900
   agtgttacgc ttgcagagtt aatttactcg caaggttatt aatttaaatg tgtttttgca 960
   cgatattttt atcttgaact ttgagagcgc actcgttttt gacgagtgcg tttttgttaa 1020
   aacattcgtt tgaaagacag tgaatgaata gcggagttat tgataagaat caatttatac 1080
   aaaagcaact gaatgttatt aatcgaggca ataaacctat tgatagtttt agttggcgcc 1140
   ataatacata aactgtactt aataatatgc aatcaatacc tagaaatatt catgacgtaa 1200
   tccaacatat cggggagggg attttaagtg atggtagaaa aaacatttag aaatctaaaa 1260
   atatatgatg attatggttc tgtctctcag gaaattattt ttaattttga aaaagagttt 1320
   gatataaaac tccctttatc ctatatctca cttgtgaaaa agtataatgg cgtttggttt 1380
   aaggaaagtg attttgaata tttatctcaa aatgggaaaa gaataataag ctcattgagt 1440
   tttgatagtt ttgagacaaa agataatatc gaaccaatga ataatatatt aagacaatat 1500
   atttatgatg atgaaattta tggatataag aatgtttatt cctttggtta cactggaaat 1560
   ggtgactttg tctgttttga ttatcgtgat gacccaaaag gtgatgagcc caaaatctgt 1620
   atcgtgattc atgatgaata tgatgaaaaa acaggcaagc gtttgttatt gcctgtggca 1680
   gaaaattttg aggcattttt agatatgctt tacgattttg atgaacgcta tccgaatggt 1740
   tatgaatagg tatttgttta aataatgtgt tgtatttttt aagcattatt tacaactaac 1800
   attttaagtg cggtcaattt tgaaaaagtt ttgggctttg agaattgggc gcattttttt 1860
   tgaaatattc ttcaatgatg agcactaatt atggattaga taatgggaat tatcgagata 1920
   tggatggtaa taaaggatgg aggctagatt ttgatcctga gaaagttgtt catgtaaata 1980
   tttttgactt tactaaaggt aaaggactag gtaaagcagt taaaaagtca ttctttttga 2040
   tagtactgaa caagagtttg aaaaatttta aagcaattaa ataaggaaga taaaatgaca 2100
   ttatttgatg aatgtaaatt agctcttaga gacgatttta atctaatttg tgatgaagag 2160
   aaggattgtg taatggataa gttttatttc tatttcttgg aaaagaaaga ggaatttaat 2220
   tttcaagatt attcatttga agaaatgtat atattttcaa aaatggaacc tgtgtatgtt 2280
   ttatgtgata gctctaatat acctttgttt aggagtaatt gggaattgat tatcaataat 2340
   atatatgatg ttgtctgttt atctacaaaa gtattttttc tagatgatga aaagttaatg 2400
   atggaattat ttcctgaaga taaagtaaga gtcatctata aaagataatt accccttgat 2460
   cgcgctcgtg tcagcacgag ttcgctttca ttaaagctct cgttaaagac tagcactagc 2520
   agtgaggttg atcgtaatca atttactgaa gaacagttaa ttaaaattaa taagaggctt 2580
   gataaaatag agggatttac atggcatcat aactcacaaa gtagtcccca aaatatgtag 2640
   ttaataccta caccaattca taaggctgtt cagcatatag gtgaaggcgc tttaagtgaa 2700
   ggaaagtgat aaaatgaaaa attttaggaa tataaatatt tatagtgatt atggaaaggt 2760
   tgataaggaa attatattag aattcgaaaa tgaatttaat ataaagcttc cttctttata 2820
   catagattta attacggcgc ataatgctcc gaagagtgaa gagaattgct ttgaatatta 2880
   caatgagcgt aatgagccca cgttttcttc ctttggattt gaagggtttg agacagagcg 2940
   gtctagcgcc tctcttgaaa atatatatgc tcagtatatt tatgatgatc caatctatgg 3000
   ttatgaacat gtgtattctt ttggtagtac tggcgaggga cattttatct gttttgatta 3060
   tcgtgatgat ccaaaaggtg atgaacccaa aatctgtatc gtgattcacg atgaatatga 3120
   tgaaaaaaca gggaaaatgc gactgtttcc tatagcagag aattttgaag cgtttttaga 3180
   tagtttgaaa tcatttgatg aaatgataga gaagtattcg taatgtctcc gttcatatct 3240
   catagcgatg ctctctcccc tgattttatg gaaattttgt ttaaattagg agggcactaa 3300
   aatgaaaaaa ttttatttaa tttttttatt attcctaagt ggatgttttt atcatgatgg 3360
   atgtatctat actcctcaga tggtaaattg ttttgttgat aaaggagata tatttccatc 3420
   aatatctcgg tatcaaaagc cttatagtct aggaaaaacc aattcagaac agcgttggaa 3480
   ggatgttatg ttttgtggag gtaaatatgg tgattataaa ttagagaata taaaaacagt 3540
   ggaacaatcg gataaattac atcattgtat gacccaaaaa gggtatatcc atttaactcc 3600
   cgcagaatgt ggataccaaa atcctaaatg ggataaaggc gtttgtaatt tataagtgtt 3660
   ttggatttta ctttacgaat tttttttgaa aaaagttacc gcaggtatgt ttttaatcaa 3720
   agcccaggtg gcgcttgttg gatgtgttat tcgcatagta gttattatgc ggaagtgcta 3780
   aatgaattca ttattaaaaa tggaattaaa gagcgtaatt agcaccgttt gcggtgagtg 3840
   atttaatttc tcctgatgtt atggaaatat tatttaagtt aggaggtgat taggatgaaa 3900
   aagttgttat tgttcagttt attattactt ttaaatggat gcctatattc ttttgaagag 3960
   gagtgtttta gaccgttaat tcaggtgggg tcatctgctt gtcataagaa taaaggagat 4020
   atatttccat caatagcacg tttccaaaaa gtagagaaca tcggaaaaac tgatgccaag 4080
   caacgttgga aagatgcggt tgattgtgga agtaagtatg gcgatgaaga tttaatatat 4140
   ataaatgata ataatttata tagcattttt cactcctgta tggttaaaaa aggatataaa 4200
   aagtttcatc ccgcagaatg tggataccaa aatcctaaat gggataaggg tatttgtaat 4260
   ttataagtgt tttgaatttt actttacaaa ctttttttga aaaatgttac cgctggtatg 4320
   tctgttttta gggataaaga ttcgttcatt ttctttaaat ttaggcacgg tataaaagag 4380
   cttgattatc aaataataat atcgtgttcg tgtccacacg agtagggttt gattaaagta 4440
   ctcctcaaaa gcgagtgcta gctgtggggt aaacggtggg aaagaaagta aagagtatat 4500
   aaaccaattg accgcataac gtgcggtcaa tttctaagat ttatgagttc cagaaaaata 4560
   gaccgaaaag tgcggtaaag gggtaacgaa acacaaaaaa taaaaatatg acagcaagct 4620
   gtgtcattaa accccagaaa aaaacttgac cgattgttgc actgctgaaa atggcaaaaa 4680
   gtaagccgat ttcatagggt agcattgcaa taaaccagat taatccactt ggatgatcac 4740
   ttttcattac ggactcctta tttatggctg ataacttaac acttgtattg aaaattaaag 4800
   cagatttaaa caatgcgttg agcgatttta aagcgcttaa aacggaattg caacgcgcag 4860
   gcgtggagtc tatcagtaag taactcgcca gtttttgcaa tcatttatca tattcacaaa 4920
   gccgtaatgc atattggaga aggttaagtg taagtgaagg aagataaaat gaaatttaga 4980
   cattttggca tttattatga ttatggcgta gtttcccaag agattatttt agattttgag 5040
   gaaacgtttg gtattaaatt accaaaattg tatattgagt tgatcacaaa acataattcc 5100
   ccaagattga atagagatca tttcaattat tatgatttct ctacgagtga ggaagaagga 5160
   acggaattta tattcaaagg atttgaaaca gaaagtaata ggcatgcgcc gccagaaaat 5220
   atttatgcac aatatcttta tgatgatgaa atatatggtt atgaacatgt gtattctttt 5280
   ggcagtaccg ctagtggtga ctttgtctgt tttgattacc gagataatcc gaaaggcagt 5340
   gaacctaaaa tttgtcttgt gattcatgat gaatatgatg aagaaacagg caagcattta 5400
   ttgtttcctg tggcagaaaa ttttgaggta tttttagata tgctttacga ttttgatgaa 5460
   cgctatccga atggttatga ataggtattt aaataatgta ttgtattttt taagcgttat 5520
   ttaataacgt tttaagtgcg gtcaatttca aaaaagtttt gctttgagga attgaccgtt 5580
   tttgtatcat ggatatgatg tgagcaattg atatcctgtt tgtgttaact ggcatgtggt 5640
   aaagcattcg tcaaagacga gctctagtgg tgagggacta atcatgaatg aactattgga 5700
   cttttataga aatataactt tttttgaaga atatgatgaa aactcattca taggaagatg 5760
   gcttgattac tcggaatgga atgataaaga atattggaaa ttagagaaag atttactgaa 5820
   aattgctcaa atgtatagaa ctactaatga agtttttcca gatattttaa taggggttat 5880
   gcgtatcatt gaattattaa tgatacctaa ctggaatagt tttataatat ctaattcaga 5940
   aagtgttgat atttatgata gatatgaacg atttaaatat ataatttcaa tacttttcaa 6000
   taataaagat gttagactag atggcttttc atatacagaa aagaatgatg atgtatttta 6060
   taatgaatag aaaaattaaa agtaagctaa tttgtagaaa taataaaagt ctcgcttgta 6120
   ttttttcttt gagatcgtgc ttgagtccac acgagtgcta gctgtaggca gagtgtcttg 6180
   gtgattataa aaagttaagg atagtattgt tgatatgaaa gaaaagacta aatattcaat 6240
   ggggaatcaa agtgataaaa aataaatttt atatttcttg gttaggattt tcaatatcat 6300
   tatatgtttt gcttttatgt agagatttgt tttattctct ttatatgttt tttattatga 6360
   tgtatttgtc ttaaaatttt cttttaagga ggcgtttttt actatcttta tttttattgt 6420
   gctatgtttc attattcatt attcattatt cattattcat tattcattat tcattatttt 6480
   atatataaat atggtaaaag gtctgaatgc ccaaaaaaga aaccattaag tgaaaatgta 6540
   acatacaata aaaggtagta ggaataaata aaatattagg aataacagaa tgataaaata 6600
   tttagaggga aatattaact cgtttatatc ggcattaggt aaaaacgaaa gtaataaaga 6660
   tattttaaaa ttagtagaaa tagtttcttc agattttgaa gtggatgaac taagtcataa 6720
   agatgaacac gagatatatt atttgtttta taagaggggt gttgaatttt gttttaaaag 6780
   aatagatgaa gagtatgtct tatattcggt tttctttttc ttggtagagg ttgataatta 6840
   tttttcatgc ccatttattc atgaattaat atgtgatctt aaacacggat tctcaataga 6900
   ggatattata aggtttttag gggagccaaa ttttaaaggt agtggctggg taagatattc 6960
   ttataatgga agaaatattc atttcgaatt taatgaatct aatgaattat cccagattag 7020
   catttttatt taataattta ggattgggaa cgctctcgtg ttcacacaag tgcggtttga 7080
   ttaaagcact tgtcaaaggc gagatccagc tgtgggagat ttgatagaga aatggctctc 7140
   aggattgacg ttagtagagg gagcataaat caagaaaaaa catttaaaat aggaacatta 7200
   ggtggtgagt aatggaaatc actcgtttta aaatcctact tattgccttg attatattag 7260
   gtaatgggta tgtgtttctt ggtggaccag ctaaaaaaca attcgctata gaagcagaaa 7320
   caaggcggat ttatcgtact ttaacgaaag aaattatttt tatgaatgga gaatataaac 7380
   aatttggagg gcgagttatt caaggattta ctttagtcat ttctttttct cattcaagta 7440
   cagaaaaccg cattaagatt ttagaaaaaa ttaaagagat gggatttgat tcaaaaaata 7500
   aaacatcaaa accgagtctt catctttttt gtcaaggaga aagtggtttt ttgatcgcag 7560
   aaaaacctga atttagaata gattatgaga aaaagatgac ttattgtttg gagtagcaaa 7620
   ggaaggcaat gataagtgta ttttctaatc agatttaatt taagtattaa agtgcggtca 7680
   attttgaaaa cgttttactt tgaaaaattg accgtttttt tacatgagag agtgaaagta 7740
   caccacctta ggcaatgtgg ttgtggataa ggtgattaag gaatacccaa atgggggtta 7800
   tgaagcgagg gtgctgatcc ctaacccgaa agcgaaaacc gatcctgatg cgccgaaatt 7860
   tttggagaaa aggggaaatg aaggtgtatc cacaatgttt ccaagaacat ggacagagga 7920
   taggttgaaa gtggagttag agcatgcgtt taataataga gtaccaatgg aaaagtttga 7980
   aaataaatgg gaaggtataa caaaatcagg cgtgaaagta gaatgggttc tggatagaaa 8040
   tggtaaagtg ttgactctat tttgttaatt ttctgtgtct ttttgtaaaa aataagttgt 8100
   tgaaataaca tttttctcta taatcaaatc ttaatgataa ttaatatcat taaataagaa 8160
   tggatactta gatgaggatt atatgaaaga tttatcaaaa atgacctgcc ttgaagactt 8220
   acgccgtgtt gcgcaacgta aagtgccaaa aatgttttat gactatgtag tttcaggatc 8280
   ttggagcgaa agcacattgc atgccaaccg taatgatttt caagcaatta agctacgtcg 8340
   acctgtggcg attttgccca tttctgcatt cagctctgca attcgtcgct tgtgctgttc 8400
   tgttgcgcgc gcaagctctc ttgtcgactt gccttttcta attgggcttg ttgctcccaa 8460
   taacgtgagg tcgccacatc cactttacgt agtgttga 8498
<210> 58
   <211> 5798
   <212> DNA
   <213> Pasteurella Multocida
<220>
   <221> misc_feature
   <223> Unknown D1
<220>
   <221> CDS
   <222> (2686)..(4446)
<400> 58
   gtcgacaaga ataaaacgaa acagcaaata gaattagaat tactacttat taacaataat      60
   ccttttttaa agattctagc tgttttagat aaaaatatac gtgtgaagct tttggtcaca    120
   ttattattat caggcctata ttatttatat gcagtaaaaa acgattcaaa ttttgtcata    180
   ggtttatcta ttattttagt ttttattatt gtcattccag ggattttgac aaatgctatt    240
   ttgaaagcta aggtgaaaaa aatcatggta gatttaccag gttttattga cttagttgca    300
   gtaaatgttc aaacagggat tagtattgat gcggctttaa aacaagtggc aatcgatttt    360
   aagaaactta atccagatct tacttatgtg atgttaagga ttattagaaa atctgaactt    420
   acgggattat cacaagcgtt acaggatctt tcgatctcat tgccaacaac agaaataaga    480
   atgttttgta ctgttttaca acagagttta aattttggtt cttcaattta ttctcactta    540
   attcagttgt ctgcagatat cagggagata caattattaa tcattgagga aaagttaggt    600
   acattatcag ctaaaatgag tatcccattg attttgttta ttatgttccc aataatcatt    660
   ttaattctag caccaggtat aatgagggta tttccaaatg ttttttaaat ttaccaagaa    720
   aatcgttttt gttagtttag ctttatctgt cgttggttgt tctacccatt ctcagcaagg    780
   catgacacag aaaagtatgt catctgaaac aataacggca aaagagactt tatatgaaag    840
   tacgcaaaat tattcggcac tcatttcact gtatcgcgat gtgttgaaag ccaaagaaga    900
   tccttcaata cgctataaat tagcgaagac atactatcag cgaggtgaca gcaaatcttc    960
   tttactttat ttaacgccat tactgaatga taatacgaag cttgctacac aagcgaaaat   1020
   attacagata aaaaatctaa ttcaattaaa taatttccaa gaagcaattt ctgtcgcaaa   1080
   tgaactctta ttaaaatcac ctaatgaagg agaagtatat aatttaagag gtatcgctta   1140
   tgcgcaaaat gggaatttgg tgaatgcccg aaatgatatc aataaagcaa gagagttctt   1200
   tattaatgat aatgttgcta ttaataattt agccatgcta aatattatta atggcgattt   1260
   taataatgct gtttctttac tgttgccaca atatttaaat ggcgttaaga attctcgatt   1320
   gattcataat cttgtttttg ctttagttaa aaatggtgat cttgattatg caaaagatat   1380
   cattgttaaa gagcgtttaa atacttcacc agatgattta attaatgcat tgaaaaaaac   1440
   tacacatgta tcaaaaggtg taactcggta acactaagga tttgatatga aaaagtttct   1500
   atcaaatata aaaggaacct cgtcaattga atttgctttg acgatagcgt tctatttatt   1560
   tgttgtgatg tttatttttg aattttgtcg attagcggtt gcgacagctt attgggattt   1620
   agctataacg gaaagtgtca gaattgcgaa gaatgaacaa gcaatttctg gaaattatga   1680
   agaagcattt aggaaagctc ttacaaagca aaaaaaattc catgatgaat cgacaattgg   1740
   atatttggcg ttgttagaag ataataaatt tgatgtaaaa gtccaatatg tggattgtga   1800
   taaagaaacg gaatgtatta aaaatcttct gcttaataaa tttcgccaac cacaaaaaaa   1860
   tcataaagga gagttaatct ctcctacggg gagtcgcgcg actttagcac aatattcttt   1920
   aacttataaa tataagttta tggtgccgtt agtatttatt cctgagtctt ggtctcaagt   1980
   agtgctgaac cgtgaatttg ttgttgtaca ggaatttgag cgttctcaat ttatgttagg   2040
   agcaaaacca agttctcttg ggacgaatcc atagaaaatt tactcattat ttcgagctat   2100
   atatgaaaga gtcaggttta gttaaattca agcatttttg gaaaaataaa aagggcgcag   2160
   tgacgataga gttccttttt atgtcaatgt ttctgattgt gctatttgca tttctcttcg   2220
   atttagtaat gttacgttct acattaggca agttagataa tgcctcatat acattagtta   2280
   gtattctccg tgaacgtaca cagttgtacg atagagttgc acaaattaat attgatgatc   2340
   ataagcaatt tgaaaagctt gctaagaaac tgatttatgg tgatcagaat agtaataaaa   2400
   ggatcgatgt tgttttagaa tattgggcac aagacggttc tggacggagg attccaaata   2460
   tcattggcga ttgtacgcct tacaaaaaac tttctgattt atcctattta tctcctcgct   2520
   cagaactcaa taatgaaaga aaaataccgc tttatcaaat tactctttgt gttgaaactc   2580
   agggcttgtt tgaaacaata ttactggata agtctgagcg ttcaacgggg ctgattagat   2640
   catcgtcaat gtcagtatca cgataaatta tcgttaggga acttt atg aaa aaa ctt   2697
                                                     Met Lys Lys Leu
                                                     1
   tat tta att cgt tct tgc tat gat tca gtc aga aaa ttt tat gag aat     2745
   Tyr Leu Ile Arg Ser Cys Tyr Asp Ser Val Arg Lys Phe Tyr Glu Asn
   5                   10                  15                  20
   gag cta ggt gtt tat aca gta atg act gca tta cta gca ttt cca tta     2793
   Glu Leu Gly Val Tyr Thr Val Met Thr Ala Leu Leu Ala Phe Pro Leu
                   25                  30                  35
   tta gtt ttg att gga ttt acg gtt gat gga act ggg gtt gtg ctt gat     2841
   Leu Val Leu Ile Gly Phe Thr Val Asp Gly Thr Gly Val Val Leu Asp
               40                  45                  50
   aaa gca cgt tta gct caa gga atg gat caa gct gct tta gct ttg gtt     2889
   Lys Ala Arg Leu Ala Gln Gly Met Asp Gln Ala Ala Leu Ala Leu Val
           55                  60                  65
   gct gaa aac aat gac tac cga gaa aat aaa aaa cat ggt gat gtt aat      2937
   Ala Glu Asn Asn Asp Tyr Arg Glu Asn Lys Lys His Gly Asp Val Asn
       70                  75                  80
   cgg caa gta gta tcg cct caa gac aaa gca aaa ttt ggt ggt aat gaa      2985
   Arg Gln Val Val Ser Pro Gln Asp Lys Ala Lys Phe Gly Gly Asn Glu
   85                  90                  95                  100
   ttt atg gcg aaa caa gaa aag cgt aat caa gag ctt atc cag ggt att      3033
   Phe Met Ala Lys Gln Glu Lys Arg Asn Gln Glu Leu Ile Gln Gly Ile
                   105                 110                 115
   gcc aaa ctt tat tta cgt tca gaa aat gcg aat gct tca tct gat gca      3081
   Ala Lys Leu Tyr Leu Arg Ser Glu Asn Ala Asn Ala Ser Ser Asp Ala
               120                 125                 130
   cca atc act att gat aaa cct ttt cat tat tca tgt gag gag tta gat      3129
   Pro Ile Thr Ile Asp Lys Pro Phe His Tyr Ser Cys Glu Glu Leu Asp
           135                 140                 145
   tta cct aca gct aat gag tat gca cgt cgt aaa cct att gtt tgt gaa      3177
   Leu Pro Thr Ala Asn Glu Tyr Ala Arg Arg Lys Pro Ile Val Cys Glu
       150                 155                 160
   gtg caa ggt ggc gtc aat cgt aaa ttt tgg ctt cct gtc agt gaa tcg      3225
   Val Gln Gly Gly Val Asn Arg Lys Phe Trp Leu Pro Val Ser Glu Ser
   165                 170                 175                 180
   tta gtt agt gaa gat aaa ctg aaa aaa gat cga gtt aga ctg gaa tcc      3273
   Leu Val Ser Glu Asp Lys Leu Lys Lys Asp Arg Val Arg Leu Glu Ser
                   185                 190                 195
   gat acc agt tat gcg att aaa gaa aaa ggc atc gtg att cct gtg gag      3321
   Asp Thr Ser Tyr Ala Ile Lys Glu Lys Gly Ile Val Ile Pro Val Glu
               200                 205                 210
   cta atg ctt gtt tcg gat tat tct ggt tcg atg aat agt cat tta cag      3369
   Leu Met Leu Val Ser Asp Tyr Ser Gly Ser Met Asn Ser His Leu Gln
           215                 220                 225
   gat aaa aac ggt aga tct cta gga aaa gct aaa att act att tta aga      3417
   Asp Lys Asn Gly Arg Ser Leu Gly Lys Ala Lys Ile Thr Ile Leu Arg
       230                 235                 240
   gaa gtg gtt agt gaa att tcg aaa att tta ttg cca gaa gat gtt agc      3465
   Glu Val Val Ser Glu Ile Ser Lys Ile Leu Leu Pro Glu Asp Val Ser
   245                 250                 255                 260
   gaa ggt gtg agc cct ttc aac cgt att ggc ttt acg act ttt tct ggc      3513
   Glu Gly Val Ser Pro Phe Asn Arg Ile Gly Phe Thr Thr Phe Ser Gly
                   265                 270                 275
   ggt gtt aga caa cgg gat gtc act gaa ggc tgt gtg ctt cca tat gaa      3561
   Gly Val Arg Gln Arg Asp Val Thr Glu Gly Cys Val Leu Pro Tyr Glu
               280                 285                 290
   gga aaa ata tca caa act tct cga aaa tta act att cgt tat tgg att      3609
   Gly Lys Ile Ser Gln Thr Ser Arg Lys Leu Thr Ile Arg Tyr Trp Ile
           295                 300                 305
   acg ggt aat aat aca cct tgg aaa ttt aat gct ggg aga tgg gag aga      3657
   Thr Gly Asn Asn Thr Pro Trp Lys Phe Asn Ala Gly Arg Trp Glu Arg
       310                 315                 320
   agt aca gtg tct ttc cag gag cat tat aaa ggc tat tat gac aaa ttc      3705
   Ser Thr Val Ser Phe Gln Glu His Tyr Lys Gly Tyr Tyr Asp Lys Phe
   325                 330                 335                 340
   cat tct tca act tgt aga ggc tca ggg agc tct aga act tgt caa att      3753
   His Ser Ser Thr Cys Arg Gly Ser Gly Ser Ser Arg Thr Cys Gln Ile
                   345                 350                 355
   gat gca aat cct aag aaa att atg gat tat gca cta aaa att aat gac      3801
   Asp Ala Asn Pro Lys Lys Ile Met Asp Tyr Ala Leu Lys Ile Asn Asp
               360                 365                 370
   tgg acg aca att aga gaa tta ttt aat act tat ata gat gta agt ggg      3849
   Trp Thr Thr Ile Arg Glu Leu Phe Asn Thr Tyr Ile Asp Val Ser Gly
           375                 380                 385
   acg att gac caa att tcc cag ttt gat ggt tca aac aga cgt tat gat      3897
   Thr Ile Asp Gln Ile Ser Gln Phe Asp Gly Ser Asn Arg Arg Tyr Asp
       390                 395                 400
   atg gtg ttt act gat gaa gaa cgg tgt ctt ggc gga aat att ggt aga      3945
   Met Val Phe Thr Asp Glu Glu Arg Cys Leu Gly Gly Asn Ile Gly Arg
   405                 410                 415                 420
   aga aca act cga gct tgg ttt gat caa aaa aat aaa gat att aca aga      3993
   Arg Thr Thr Arg Ala Trp Phe Asp Gln Lys Asn Lys Asp Ile Thr Arg
                   425                 430                 435
   gag ttg aat att gtt cgt cct tct ggt tgg act tct gca tct tcg ggg      4041
   Glu Leu Asn Ile Val Arg Pro Ser Gly Trp Thr Ser Ala Ser Ser Gly
               440                 445                 450
   ctt ctt gtt gga gct aat atc atg atg gac gag aat aag aat cct gat      4089
   Leu Leu Val Gly Ala Asn Ile Met Met Asp Glu Asn Lys Asn Pro Asp
           455                 460                 465
   gcg caa cct tcg aaa ctc ggg aca aat att caa cgt gtt atc tta gta      4137
   Ala Gln Pro Ser Lys Leu Gly Thr Asn Ile Gln Arg Val Ile Leu Val
       470                 475                 480
   tta tct gat ggt gaa gat aac tgg cca act tat agt aca tta acg act      4185
   Leu Ser Asp Gly Glu Asp Asn Trp Pro Thr Tyr Ser Thr Leu Thr Thr
   485                 490                 495                 500
   ctt tta aac aat ggt atg tgt gac aaa att cga gaa caa ttg ggc aag      4233
   Leu Leu Asn Asn Gly Met Cys Asp Lys Ile Arg Glu Gln Leu Gly Lys
                   505                 510                 515
   tta caa gat cca aat tta cga gag tta cca gga aga att gcg ttt gtt      4281
   Leu Gln Asp Pro Asn Leu Arg Glu Leu Pro Gly Arg Ile Ala Phe Val
               520                 525                 530
   gcg ttt ggc tat agt cca cca gca aac caa gtt gcc gct tgg aaa aaa      4329
   Ala Phe Gly Tyr Ser Pro Pro Ala Asn Gln Val Ala Ala Trp Lys Lys
           535                 540                 545
   tgt gta ggt gat caa tat tat acg gct tat tcg aaa gaa gag ttg tta      4377
   Cys Val Gly Asp Gln Tyr Tyr Thr Ala Tyr Ser Lys Glu Glu Leu Leu
       550                 555                 560
   gat agt ttc aaa caa att att gga ttt gaa gaa gag gtg ggg cgt tct      4425
   Asp Ser Phe Lys Gln Ile Ile Gly Phe Glu Glu Glu Val Gly Arg Ser
   565                 570                 575                 580
   tca tct cat aaa ccg aaa ttt taagattgtc caaggataac gctaaaaaat         4476
   Ser Ser His Lys Pro Lys Phe
                   585
   ctcttagcac aggctaagag atttttttat gtgtttttca aattttatct actggtgatt     4536
   ttaattcatt acataacata actttttcgt gaataataca gaatagacaa caataagaat    4596
   taaaacgctc aaggcataga ggcttagtcg aacaaactaa gctattttgc gcgattgatt    4656
   gggatataga tgttatttca aataagcaat aaccatggta ctgagaaaga agatgagtgc    4716
   cgtaataaag tagaagcgat ttttcttttg gctgaaaggt ggtgttagcc tttttcggct    4776
   aaagaaaata gtagcaacgg caatataaag ggcgataaag agcattttgt aaatgaacca    4836
   tgttgttaca ttttgctgaa aaagaagaaa tagcccaatt cccgaacaga acaacagcgt    4896
   atcacttaag tgcggtaaga tttttaatac ttttctgtct cgccaatttt tgcctgttaa    4956
   ttgcatcatg ccacggataa taaatagact gaggctgaga aaggcacagg caatgtgtag    5016
   ataaataaga taatatgcca tcgatttttt tcctaagata aagaaagcag tagcgaggct    5076
   actgctttaa aataatatcg tatttagtga atgaatttaa ctttctgcgc tttcgaccgc    5136
   actttcgctc gcatcactct caatgtcttc ttcatcgaca tcacaaacga gttgtaatcc    5196
   aaccagtgtt tcactttcac ttgttcggat gagacgtact ccttgtgtat ttcgaccgat    5256
   aatattgatt tcgtttacgc gagtacgtac tagcgtaccg gcatcagtaa tgagcataat    5316
   ttgatcgctt tcctcaactt gtgttgctgc aacgacttta ccgttacgct cactcacttt    5376
   aatcgagatc acgcctttgg tgttacgtga tttagttggg tattccgcta attcagtacg    5436
   tttaccataa ccgttttgtg tggcggttaa aatggcgcct tcattttttg gaataacaag    5496
   tgacacgact ttatcaatat tgagatccaa ggcatcatta gtattctcat cagagatctc    5556
   ttccatatcg accgcacttt catcatccga caaatcattc gttaacgcca gtttaatacc    5616
   gcgtacacct gttgctgcac gccccatggc acgcacagca ttttcactaa agcgaactac    5676
   acgtccttgt gcggagaaga gcatgatttc attttgacca tcggtgatat ccacaccgat    5736
   aagttcgtct tcatcacgca agttgagtgc aataatgcct gttgaacgag gacgactgaa    5796
   tt                                                                   5798
<210> 59
   <211> 587
   <212> PRT
   <213> Pasteurella Multocida
<400> 59
   Met Lys Lys Leu Tyr Leu Ile Arg Ser Cys Tyr Asp Ser Val Arg Lys
   1 5 10 15
   Phe Tyr Glu Asn Glu Leu Gly Val Tyr Thr Val Met Thr Ala Leu Leu
   20 25 30
   Ala Phe Pro Leu Leu Val Leu Ile Gly Phe Thr Val Asp Gly Thr Gly
   35 40 45
   Val Val Leu Asp Lys Ala Arg Leu Ala Gln Gly Met Asp Gln Ala Ala
   50 55 60
   Leu Ala Leu Val Ala Glu Asn Asn Asp Tyr Arg Glu Asn Lys Lys His
   65 70 75 80
   Gly Asp Val Asn Arg Gln Val Val Ser Pro Gln Asp Lys Ala Lys Phe
   85 90 95
   Gly Gly Asn Glu Phe Met Ala Lys Gln Glu Lys Arg Asn Gln Glu Leu
   100 105 110
   Ile Gln Gly Ile Ala Lys Leu Tyr Leu Arg Ser Glu Asn Ala Asn Ala
   115 120 125
   Ser Ser Asp Ala Pro Ile Thr Ile Asp Lys Pro Phe His Tyr Ser Cys
   130 135 140
   Glu Glu Leu Asp Leu Pro Thr Ala Asn Glu Tyr Ala Arg Arg Lys Pro
   145 150 155 160
   Ile Val Cys Glu Val Gln Gly Gly Val Asn Arg Lys Phe Trp Leu Pro
   165 170 175
   Val Ser Glu Ser Leu Val Ser Glu Asp Lys Leu Lys Lys Asp Arg Val
   180 185 190
   Arg Leu Glu Ser Asp Thr Ser Tyr Ala Ile Lys Glu Lys Gly Ile Val
   195 200 205
   Ile Pro Val Glu Leu Met Leu Val Ser Asp Tyr Ser Gly Ser Met Asn
   210 215 220
   Ser His Leu Gln Asp Lys Asn Gly Arg Ser Leu Gly Lys Ala Lys Ile
   225 230 235 240
   Thr Ile Leu Arg Glu Val Val Ser Glu Ile Ser Lys Ile Leu Leu Pro
                   245                 250                 255
   Glu Asp Val Ser Glu Gly Val Ser Pro Phe Asn Arg Ile Gly Phe Thr
               260                 265                 270
   Thr Phe Ser Gly Gly Val Arg Gln Arg Asp Val Thr Glu Gly Cys Val
           275                 280                 285
   Leu Pro Tyr Glu Gly Lys Ile Ser Gln Thr Ser Arg Lys Leu Thr Ile
       290                 295                 300
   Arg Tyr Trp Ile Thr Gly Asn Asn Thr Pro Trp Lys Phe Asn Ala Gly
   305                 310                 315                 320
   Arg Trp Glu Arg Ser Thr Val Ser Phe Gln Glu His Tyr Lys Gly Tyr
                   325                 330                 335
   Tyr Asp Lys Phe His Ser Ser Thr Cys Arg Gly Ser Gly Ser Ser Arg
               340                 345                 350
   Thr Cys Gln Ile Asp Ala Asn Pro Lys Lys Ile Met Asp Tyr Ala Leu
           355                 360                 365
   Lys Ile Asn Asp Trp Thr Thr Ile Arg Glu Leu Phe Asn Thr Tyr Ile
       370                 375                 380
   Asp Val Ser Gly Thr Ile Asp Gln Ile Ser Gln Phe Asp Gly Ser Asn
   385                 390                 395                 400
   Arg Arg Tyr Asp Met Val Phe Thr Asp Glu Glu Arg Cys Leu Gly Gly
                   405                 410                 415
   Asn Ile Gly Arg Arg Thr Thr Arg Ala Trp Phe Asp Gln Lys Asn Lys
               420                 425                 430
   Asp Ile Thr Arg Glu Leu Asn Ile Val Arg Pro Ser Gly Trp Thr Ser
           435                 440                 445
   Ala Ser Ser Gly Leu Leu Val Gly Ala Asn Ile Met Met Asp Glu Asn
       450                 455                 460
   Lys Asn Pro Asp Ala Gln Pro Ser Lys Leu Gly Thr Asn Ile Gln Arg
   465                 470                 475                 480
   Val Ile Leu Val Leu Ser Asp Gly Glu Asp Asn Trp Pro Thr Tyr Ser
                   485                 490                 495
   Thr Leu Thr Thr Leu Leu Asn Asn Gly Met Cys Asp Lys Ile Arg Glu
               500                 505                 510
   Gln Leu Gly Lys Leu Gln Asp Pro Asn Leu Arg Glu Leu Pro Gly Arg
           515                 520                 525
   Ile Ala Phe Val Ala Phe Gly Tyr Ser Pro Pro Ala Asn Gln Val Ala
       530                 535                 540
   Ala Trp Lys Lys Cys Val Gly Asp Gln Tyr Tyr Thr Ala Tyr Ser Lys
   545                 550                 555                 560
   Glu Glu Leu Leu Asp Ser Phe Lys Gln Ile Ile Gly Phe Glu Glu Glu
                   565                 570                 575
   Val Gly Arg Ser Ser Ser His Lys Pro Lys Phe
               580                 585
<210> 60
   <211> 5798
   <212> DNA
   <213> Pasteurella Multocida
<220>
   <221> misc_feature
   <223> unknown D2
<220>
   <221> CDS
   <222> (698)..(1468)
<400> 60
   gtcgacaaga ataaaacgaa acagcaaata gaattagaat tactacttat taacaataat      60
   ccttttttaa agattctagc tgttttagat aaaaatatac gtgtgaagct tttggtcaca    120
   ttattattat caggcctata ttatttatat gcagtaaaaa acgattcaaa ttttgtcata    180
   ggtttatcta ttattttagt ttttattatt gtcattccag ggattttgac aaatgctatt    240
   ttgaaagcta aggtgaaaaa aatcatggta gatttaccag gttttattga cttagttgca    300
   gtaaatgttc aaacagggat tagtattgat gcggctttaa aacaagtggc aatcgatttt    360
   aagaaactta atccagatct tacttatgtg atgttaagga ttattagaaa atctgaactt    420
   acgggattat cacaagcgtt acaggatctt tcgatctcat tgccaacaac agaaataaga    480
   atgttttgta ctgttttaca acagagttta aattttggtt cttcaattta ttctcactta    540
   attcagttgt ctgcagatat cagggagata caattattaa tcattgagga aaagttaggt    600
   acattatcag ctaaaatgag tatcccattg attttgttta ttatgttccc aataatcatt    660
   ttaattctag caccaggtat aatgagggta tttccaa atg ttt ttt aaa ttt acc     715
                                            Met Phe Phe Lys Phe Thr
                                            1               5
   aag aaa atc gtt ttt gtt agt tta gct tta tct gtc gtt ggt tgt tct      763
   Lys Lys Ile Val Phe Val Ser Leu Ala Leu Ser Val Val Gly Cys Ser
               10                  15                  20
   acc cat tct cag caa ggc atg aca cag aaa agt atg tca tct gaa aca      811
   Thr His Ser Gln Gln Gly Met Thr Gln Lys Ser Met Ser Ser Glu Thr
           25                  30                  35
   ata acg gca aaa gag act tta tat gaa agt acg caa aat tat tcg gca      859
   Ile Thr Ala Lys Glu Thr Leu Tyr Glu Ser Thr Gln Asn Tyr Ser Ala
       40                  45                  50
   ctc att tca ctg tat cgc gat gtg ttg aaa gcc aaa gaa gat cct tca      907
   Leu Ile Ser Leu Tyr Arg Asp Val Leu Lys Ala Lys Glu Asp Pro Ser
   55                  60                  65                  70
   ata cgc tat aaa tta gcg aag aca tac tat cag cga ggt gac agc aaa      955
   Ile Arg Tyr Lys Leu Ala Lys Thr Tyr Tyr Gln Arg Gly Asp Ser Lys
                   75                  80                  85
   tct tct tta ctt tat tta acg cca tta ctg aat gat aat acg aag ctt     1003
   Ser Ser Leu Leu Tyr Leu Thr Pro Leu Leu Asn Asp Asn Thr Lys Leu
               90                  95                  100
   gct aca caa gcg aaa ata tta cag ata aaa aat cta att caa tta aat     1051
   Ala Thr Gln Ala Lys Ile Leu Gln Ile Lys Asn Leu Ile Gln Leu Asn
           105                 110                 115
   aat ttc caa gaa gca att tct gtc gca aat gaa ctc tta tta aaa tca     1099
   Asn Phe Gln Glu Ala Ile Ser Val Ala Asn Glu Leu Leu Leu Lys Ser
       120                 125                 130
   cct aat gaa gga gaa gta tat aat tta aga ggt atc gct tat gcg caa     1147
   Pro Asn Glu Gly Glu Val Tyr Asn Leu Arg Gly Ile Ala Tyr Ala Gln
   135                 140                 145                 150
   aat ggg aat ttg gtg aat gcc cga aat gat atc aat aaa gca aga gag     1195
   Asn Gly Asn Leu Val Asn Ala Arg Asn Asp Ile Asn Lys Ala Arg Glu
                   155                 160                 165
   ttc ttt att aat gat aat gtt gct att aat aat tta gcc atg cta aat     1243
   Phe Phe Ile Asn Asp Asn Val Ala Ile Asn Asn Leu Ala Met Leu Asn
               170                 175                 180
   att att aat ggc gat ttt aat aat gct gtt tct tta ctg ttg cca caa     1291
   Ile Ile Asn Gly Asp Phe Asn Asn Ala Val Ser Leu Leu Leu Pro Gln
           185                 190                 195
   tat tta aat ggc gtt aag aat tct cga ttg att cat aat ctt gtt ttt     1339
   Tyr Leu Asn Gly Val Lys Asn Ser Arg Leu Ile His Asn Leu Val Phe
       200                 205                 210
   gct tta gtt aaa aat ggt gat ctt gat tat gca aaa gat atc att gtt      1387
   Ala Leu Val Lys Asn Gly Asp Leu Asp Tyr Ala Lys Asp Ile Ile Val
   215                 220                 225                 230
   aaa gag cgt tta aat act tca cca gat gat tta att aat gca ttg aaa      1435
   Lys Glu Arg Leu Asn Thr Ser Pro Asp Asp Leu Ile Asn Ala Leu Lys
                   235                 240                 245
   aaa act aca cat gta tca aaa ggt gta act cgg taacactaag gatttgatat   1488
   Lys Thr Thr His Val Ser Lys Gly Val Thr Arg
               250                 255
   gaaaaagttt ctatcaaata taaaaggaac ctcgtcaatt gaatttgctt tgacgatagc   1548
   gttctattta tttgttgtga tgtttatttt tgaattttgt cgattagcgg ttgcgacagc   1608
   ttattgggat ttagctataa cggaaagtgt cagaattgcg aagaatgaac aagcaatttc   1668
   tggaaattat gaagaagcat ttaggaaagc tcttacaaag caaaaaaaat tccatgatga   1728
   atcgacaatt ggatatttgg cgttgttaga agataataaa tttgatgtaa aagtccaata   1788
   tgtggattgt gataaagaaa cggaatgtat taaaaatctt ctgcttaata aatttcgcca   1848
   accacaaaaa aatcataaag gagagttaat ctctcctacg gggagtcgcg cgactttagc   1908
   acaatattct ttaacttata aatataagtt tatggtgccg ttagtattta ttcctgagtc   1968
   ttggtctcaa gtagtgctga accgtgaatt tgttgttgta caggaatttg agcgttctca   2028
   atttatgtta ggagcaaaac caagttctct tgggacgaat ccatagaaaa tttactcatt   2088
   atttcgagct atatatgaaa gagtcaggtt tagttaaatt caagcatttt tggaaaaata   2148
   aaaagggcgc agtgacgata gagttccttt ttatgtcaat gtttctgatt gtgctatttg   2208
   catttctctt cgatttagta atgttacgtt ctacattagg caagttagat aatgcctcat   2268
   atacattagt tagtattctc cgtgaacgta cacagttgta cgatagagtt gcacaaatta   2328
   atattgatga tcataagcaa tttgaaaagc ttgctaagaa actgatttat ggtgatcaga   2388
   atagtaataa aaggatcgat gttgttttag aatattgggc acaagacggt tctggacgga   2448
   ggattccaaa tatcattggc gattgtacgc cttacaaaaa actttctgat ttatcctatt   2508
   tatctcctcg ctcagaactc aataatgaaa gaaaaatacc gctttatcaa attactcttt   2568
   gtgttgaaac tcagggcttg tttgaaacaa tattactgga taagtctgag cgttcaacgg   2628
   ggctgattag atcatcgtca atgtcagtat cacgataaat tatcgttagg gaactttatg   2688
   aaaaaacttt atttaattcg ttcttgctat gattcagtca gaaaatttta tgagaatgag   2748
   ctaggtgttt atacagtaat gactgcatta ctagcatttc cattattagt tttgattgga   2808
   tttacggttg atggaactgg ggttgtgctt gataaagcac gtttagctca aggaatggat   2868
   caagctgctt tagctttggt tgctgaaaac aatgactacc gagaaaataa aaaacatggt   2928
   gatgttaatc ggcaagtagt atcgcctcaa gacaaagcaa aatttggtgg taatgaattt   2988
   atggcgaaac aagaaaagcg taatcaagag cttatccagg gtattgccaa actttattta 3048
   cgttcagaaa atgcgaatgc ttcatctgat gcaccaatca ctattgataa accttttcat 3108
   tattcatgtg aggagttaga tttacctaca gctaatgagt atgcacgtcg taaacctatt 3168
   gtttgtgaag tgcaaggtgg cgtcaatcgt aaattttggc ttcctgtcag tgaatcgtta 3228
   gttagtgaag ataaactgaa aaaagatcga gttagactgg aatccgatac cagttatgcg 3288
   attaaagaaa aaggcatcgt gattcctgtg gagctaatgc ttgtttcgga ttattctggt 3348
   tcgatgaata gtcatttaca ggataaaaac ggtagatctc taggaaaagc taaaattact 3408
   attttaagag aagtggttag tgaaatttcg aaaattttat tgccagaaga tgttagcgaa 3468
   ggtgtgagcc ctttcaaccg tattggcttt acgacttttt ctggcggtgt tagacaacgg 3528
   gatgtcactg aaggctgtgt gcttccatat gaaggaaaaa tatcacaaac ttctcgaaaa 3588
   ttaactattc gttattggat tacgggtaat aatacacctt ggaaatttaa tgctgggaga 3648
   tgggagagaa gtacagtgtc tttccaggag cattataaag gctattatga caaattccat 3708
   tcttcaactt gtagaggctc agggagctct agaacttgtc aaattgatgc aaatcctaag 3768
   aaaattatgg attatgcact aaaaattaat gactggacga caattagaga attatttaat 3828
   acttatatag atgtaagtgg gacgattgac caaatttccc agtttgatgg ttcaaacaga 3888
   cgttatgata tggtgtttac tgatgaagaa cggtgtcttg gcggaaatat tggtagaaga 3948
   acaactcgag cttggtttga tcaaaaaaat aaagatatta caagagagtt gaatattgtt 4008
   cgtccttctg gttggacttc tgcatcttcg gggcttcttg ttggagctaa tatcatgatg 4068
   gacgagaata agaatcctga tgcgcaacct tcgaaactcg ggacaaatat tcaacgtgtt 4128
   atcttagtat tatctgatgg tgaagataac tggccaactt atagtacatt aacgactctt 4188
   ttaaacaatg gtatgtgtga caaaattcga gaacaattgg gcaagttaca agatccaaat 4248
   ttacgagagt taccaggaag aattgcgttt gttgcgtttg gctatagtcc accagcaaac 4308
   caagttgccg cttggaaaaa atgtgtaggt gatcaatatt atacggctta ttcgaaagaa 4368
   gagttgttag atagtttcaa acaaattatt ggatttgaag aagaggtggg gcgttcttca 4428
   tctcataaac cgaaatttta agattgtcca aggataacgc taaaaaatct cttagcacag 4488
   gctaagagat ttttttatgt gtttttcaaa ttttatctac tggtgatttt aattcattac 4548
   ataacataac tttttcgtga ataatacaga atagacaaca ataagaatta aaacgctcaa 4608
   ggcatagagg cttagtcgaa caaactaagc tattttgcgc gattgattgg gatatagatg 4668
   ttatttcaaa taagcaataa ccatggtact gagaaagaag atgagtgccg taataaagta 4728
   gaagcgattt ttcttttggc tgaaaggtgg tgttagcctt tttcggctaa agaaaatagt 4788
   agcaacggca atataaaggg cgataaagag cattttgtaa atgaaccatg ttgttacatt   4848
   ttgctgaaaa agaagaaata gcccaattcc cgaacagaac aacagcgtat cacttaagtg   4908
   cggtaagatt tttaatactt ttctgtctcg ccaatttttg cctgttaatt gcatcatgcc   4968
   acggataata aatagactga ggctgagaaa ggcacaggca atgtgtagat aaataagata   5028
   atatgccatc gatttttttc ctaagataaa gaaagcagta gcgaggctac tgctttaaaa   5088
   taatatcgta tttagtgaat gaatttaact ttctgcgctt tcgaccgcac tttcgctcgc   5148
   atcactctca atgtcttctt catcgacatc acaaacgagt tgtaatccaa ccagtgtttc   5208
   actttcactt gttcggatga gacgtactcc ttgtgtattt cgaccgataa tattgatttc   5268
   gtttacgcga gtacgtacta gcgtaccggc atcagtaatg agcataattt gatcgctttc   5328
   ctcaacttgt gttgctgcaa cgactttacc gttacgctca ctcactttaa tcgagatcac   5388
   gcctttggtg ttacgtgatt tagttgggta ttccgctaat tcagtacgtt taccataacc   5448
   gttttgtgtg gcggttaaaa tggcgccttc atttttggga ataacaagtg acacgacttt   5508
   atcaatattg agatccaagg catcattagt attctcatca gagatctctt ccatatcgac   5568
   cgcactttca tcatccgaca aatcattcgt taacgccagt ttaataccgc gtacacctgt   5628
   tgctgcacgc cccatggcac gcacagcatt ttcactaaag cgaactacac gtccttgtgc   5688
   ggagaagagc atgatttcat tttgaccatc ggtgatatcc acaccgataa gttcgtcttc   5748
   atcacgcaag ttgagtgcaa taatgcctgt tgaacgagga cgactgaatt              5798
<210> 61
   <211> 257
   <212> PRT
   <213> Pasteurella Multocida
<400> 61
   Met Phe Phe Lys Phe Thr Lys Lys Ile Val Phe Val Ser Leu Ala Leu
   1               5                   10                  15
   Ser Val Val Gly Cys Ser Thr His Ser Gln Gln Gly Met Thr Gln Lys
               20                  25                  30
   Ser Met Ser Ser Glu Thr Ile Thr Ala Lys Glu Thr Leu Tyr Glu Ser
           35                  40                  45
   Thr Gln Asn Tyr Ser Ala Leu Ile Ser Leu Tyr Arg Asp Val Leu Lys
       50                  55                  60
   Ala Lys Glu Asp Pro Ser Ile Arg Tyr Lys Leu Ala Lys Thr Tyr Tyr
   65                  70                  75                  80
   Gln Arg Gly Asp Ser Lys Ser Ser Leu Leu Tyr Leu Thr Pro Leu Leu
                   85                  90                  95
   Asn Asp Asn Thr Lys Leu Ala Thr Gln Ala Lys Ile Leu Gln Ile Lys
               100                 105                 110
   Asn Leu Ile Gln Leu Asn Asn Phe Gln Glu Ala Ile Ser Val Ala Asn
           115                 120                 125
   Glu Leu Leu Leu Lys Ser Pro Asn Glu Gly Glu Val Tyr Asn Leu Arg
       130                 135                 140
   Gly Ile Ala Tyr Ala Gln Asn Gly Asn Leu Val Asn Ala Arg Asn Asp
   145                 150                 155                 160
   Ile Asn Lys Ala Arg Glu Phe Phe Ile Asn Asp Asn Val Ala Ile Asn
                   165                 170                 175
   Asn Leu Ala Met Leu Asn Ile Ile Asn Gly Asp Phe Asn Asn Ala Val
               180                 185                 190
   Ser Leu Leu Leu Pro Gln Tyr Leu Asn Gly Val Lys Asn Ser Arg Leu
           195                 200                 205
   Ile His Asn Leu Val Phe Ala Leu Val Lys Asn Gly Asp Leu Asp Tyr
       210                 215                 220
   Ala Lys Asp Ile Ile Val Lys Glu Arg Leu Asn Thr Ser Pro Asp Asp
   225                 230                 235                 240
   Leu Ile Asn Ala Leu Lys Lys Thr Thr His Val Ser Lys Gly Val Thr
                   245                 250                 255
   Arg
<210> 62
   <211> 1788
   <212> DNA
   <213> Pasteurella Multocida
<220>
   <221> misc_feature
   <223> unknown k
<220>
   <221> CDS
   <222> (1)..(600)
<400> 62
   gtc aac act ggg cta att cac agt aac ggt aat gcc aag ctc act ttt      48
   Val Asn Thr Gly Leu Ile His Ser Asn Gly Asn Ala Lys Leu Thr Phe
   1               5                   10                  15
   aaa gat gac acc agt ttt gtg act gaa gga aat aac ttt atc aca gca      96
   Lys Asp Asp Thr Ser Phe Val Thr Glu Gly Asn Asn Phe Ile Thr Ala
               20                  25                  30
   aaa gac aac tta gaa atc acg gca aaa aat gtt caa att gat caa gcg     144
   Lys Asp Asn Leu Glu Ile Thr Ala Lys Asn Val Gln Ile Asp Gln Ala
           35                  40                  45
   aaa aat att caa tta aac gcg aat atc acg atc aat acc aag tct ggt     192
   Lys Asn Ile Gln Leu Asn Ala Asn Ile Thr Ile Asn Thr Lys Ser Gly
       50                  55                  60
   ttt gtg aat tac ggt acc tta gca agt gct caa aat tta acg att aat     240
   Phe Val Asn Tyr Gly Thr Leu Ala Ser Ala Gln Asn Leu Thr Ile Asn
   65                  70                  75                  80
   acc gaa caa ggc agc att tat aac ata ggc ggt atc ttg ggg gcg ggt     288
   Thr Glu Gln Gly Ser Ile Tyr Asn Ile Gly Gly Ile Leu Gly Ala Gly
                   85                  90                  95
   aaa agt ttg aat ctg agc gcg aaa aga gga gaa aac caa gga gga tat     336
   Lys Ser Leu Asn Leu Ser Ala Lys Arg Gly Glu Asn Gln Gly Gly Tyr
               100                 105                 110
   ctt att aat caa ggt aag agt cta ctc cat tct gaa ggc gcc atg aac     384
   Leu Ile Asn Gln Gly Lys Ser Leu Leu His Ser Glu Gly Ala Met Asn
           115                 120                 125
   ctc aca gcg gat cgc acg gtg tac aat tta ggg aat att ttt gct aaa     432
   Leu Thr Ala Asp Arg Thr Val Tyr Asn Leu Gly Asn Ile Phe Ala Lys
       130                 135                 140
   ggt gac gcg acg atc aat gca aac gcg tta att aat gat gtt act ctc     480
   Gly Asp Ala Thr Ile Asn Ala Asn Ala Leu Ile Asn Asp Val Thr Leu
   145                 150                 155                 160
   aca ggt cgt ctt gag tat caa gat ctg aaa aaa gat tat acg cgt tat     528
   Thr Gly Arg Leu Glu Tyr Gln Asp Leu Lys Lys Asp Tyr Thr Arg Tyr
                   165                 170                 175
   tat cgt atc aat gaa acg gca aaa cat ggt tgg cat aat aac ttc tat     576
   Tyr Arg Ile Asn Glu Thr Ala Lys His Gly Trp His Asn Asn Phe Tyr
               180                 185                 190
   gaa tta aac gtc gac aga gtt tct tgatttgtgc atcaattttg taaccaccgg   630
   Glu Leu Asn Val Asp Arg Val Ser
           195                 200
   ttaataaaac accagcaatt tcaacgccat tcatggcaga taatgccgct gcgacgatca   690
   catcaggacg atccgcggaa gtgacaagta aacttccaac gcggaaatgt tccaccatat   750
   tggtcaaatt acgtgcacag aaagtgatgc cacgaatgcg acgttcattg atcgcgcctt   810
   catgaataat ggcagcacct aaatgtttgg ctaaatcaat ggcacgagtc gcaattaatt 870
   ctgcgctcca aggaatacat gccaagattt taattgggct tttctcaaat aaatgataaa 930
   tctcagatac ttgattttgt gtgtgttgga aagaatcaaa aatttctgcc aagtcagggc 990
   gagtacgacc agattcatca atcggcgcat taaatttatt gatcacaaca ccaagtaaat 1050
   tagggttatt tttgctgcca aataatgagg ctgcggcttt gatgcgttct ttgagttctg 1110
   ccggtgtttc cgtcgccggt gctgcaacaa gaatgatttc cgcatcaagt gcttgagcaa 1170
   tttcatagtt aatgctattg gcataagaat gcttacgcgt agggattaaa ccttccacca 1230
   cgacaatttc attgtttttg gcgagttgtt gatgattttc aacaattttt tctagtacca 1290
   catcagattg attttgaccg atgagtgatt cagctacact taacataaat ggttcactgg 1350
   tttcaatggt ggtactggtg cgaataattg atgttgtgcg atcaatcata tcttcacctg 1410
   agttcggctg agaaattggt ttcataaagc cgactttcgc ccctttttgc tccagtgcat 1470
   gtgttaaacc taagctgaca ctggttaagc ctacaccagc actaatcggg ataaggataa 1530
   ttgtacgtga cataataaac cctaatttgt tgataattta tacaaaaaga aactgccgat 1590
   gaatcggcag ttaattgatc tttacgcgat gcaaaggcgc gcggtatctt gtgcaataac 1650
   aagttcttca ttcgttggga tcaccatggc aacaggcgta ttgtctgctg taatcacccc 1710
   ttcatgacca aagcgagccg ctttgttttt atctgaatcc acttgataac cgaacagttt 1770
   taaatggttt aaggttga 1788
<210> 63
   <211> 200
   <212> PRT
   <213> Pasteurella Multocida
<400> 63
   Val Asn Thr Gly Leu Ile His Ser Asn Gly Asn Ala Lys Leu Thr Phe
   1 5 10 15
   Lys Asp Asp Thr Ser Phe Val Thr Glu Gly Asn Asn Phe Ile Thr Ala
   20 25 30
   Lys Asp Asn Leu Glu Ile Thr Ala Lys Asn Val Gln Ile Asp Gln Ala
   35 40 45
   Lys Asn Ile Gln Leu Asn Ala Asn Ile Thr Ile Asn Thr Lys Ser Gly
   50 55 60
   Phe Val Asn Tyr Gly Thr Leu Ala Ser Ala Gln Asn Leu Thr Ile Asn
   65 70 75 80
   Thr Glu Gln Gly Ser Ile Tyr Asn Ile Gly Gly Ile Leu Gly Ala Gly
                   85                  90                  95
   Lys Ser Leu Asn Leu Ser Ala Lys Arg Gly Glu Asn Gln Gly Gly Tyr
               100                 105                 110
   Leu Ile Asn Gln Gly Lys Ser Leu Leu His Ser Glu Gly Ala Met Asn
           115                 120                 125
   Leu Thr Ala Asp Arg Thr Val Tyr Asn Leu Gly Asn Ile Phe Ala Lys
       130                 135                 140
   Gly Asp Ala Thr Ile Asn Ala Asn Ala Leu Ile Asn Asp Val Thr Leu
   145                 150                 155                 160
   Thr Gly Arg Leu Glu Tyr Gln Asp Leu Lys Lys Asp Tyr Thr Arg Tyr
                   165                 170                 175
   Tyr Arg Ile Asn Glu Thr Ala Lys His Gly Trp His Asn Asn Phe Tyr
               180                 185                 190
   Glu Leu Asn Val Asp Arg Val Ser
           195                 200
<210> 64
   <211> 278
   <212> DNA
   <213> Pasteurella Multocida
<220>
   <221> misc_feature
   <223> unknown o
<220>
   <221> CDS
   <222> (108)..(278)
<400> 64
   gaattccaac caaatctcac accagagcaa gaacgctaca tagtggaatg gttggcagaa      60
   cattacccaa atggaaataa accttaacca tagcaagaga gaagaaa atg aaa att       116
                                                       Met Lys Ile
                                                       1
   act att aca cga aat cat cca gaa gta ttt caa gaa tcc gct cgt tta       164
   Thr Ile Thr Arg Asn His Pro Glu Val Phe Gln Glu Ser Ala Arg Leu
       5                   10                  15
   gta gcc gaa aag ttc att aaa gcc caa tgt gta gaa gca tta aca ttg       212
   Val Ala Glu Lys Phe Ile Lys Ala Gln Cys Val Glu Ala Leu Thr Leu
   20                  25                  30                  35
   gct ttg att gag ggt gtc gag cac ttt gtg ctg gaa ggt gag gag gaa      260
   Ala Leu Ile Glu Gly Val Glu His Phe Val Leu Glu Gly Glu Glu Glu
                   40                  45                  50
   agc aaa agg gga cat agt                                              278
   Ser Lys Arg Gly His Ser
               55
<210> 65
   <211> 57
   <212> PRT
   <213> Pasteurella Multocida
<400> 65
   Met Lys Ile Thr Ile Thr Arg Asn His Pro Glu Val Phe Gln Glu Ser
   1               5                   10                  15
   Ala Arg Leu Val Ala Glu Lys Phe Ile Lys Ala Gln Cys Val Glu Ala
               20                  25                  30
   Leu Thr Leu Ala Leu Ile Glu Gly Val Glu His Phe Val Leu Glu Gly
           35                  40                  45
   Glu Glu Glu Ser Lys Arg Gly His Ser
       50                  55
<210> 66
   <211> 1020
   <212> DNA
   <213> Pasteurella Multocida
<220>
   <221> misc_feature
   <223> unknown P
<220>
   <221> CDS
   <222> (1)..(597)
   <223> unknown P
<400> 66
   gtc aac aca tca aaa gtt gag att gac tat gcc gtc act cgt gcg gcg       48
   Val Asn Thr Ser Lys Val Glu Ile Asp Tyr Ala Val Thr Arg Ala Ala
   1               5                   10                  15
   gca atg cgt gca tat ctt gat aaa gaa cag ggc tgg cat acg tct att       96
   Ala Met Arg Ala Tyr Leu Asp Lys Glu Gln Gly Trp His Thr Ser Ile
               20                  25                  30
   tca aat aaa ggc att aat ggc gtg agc ggt gtc aca caa cca ctc tat      144
   Ser Asn Lys Gly Ile Asn Gly Val Ser Gly Val Thr Gln Pro Leu Tyr
           35                  40                  45
   ttt gac att aac gac agc tcg act gat gtg aac tat ctc aat gaa caa      192
   Phe Asp Ile Asn Asp Ser Ser Thr Asp Val Asn Tyr Leu Asn Glu Gln
       50                  55                  60
   ggc atc acg tgt tgc gtg aat cat aat ggc ttt cgt ttt tgg ggc tta      240
   Gly Ile Thr Cys Cys Val Asn His Asn Gly Phe Arg Phe Trp Gly Leu
   65                  70                  75                  80
   cgc acg act gca gaa gat cca tta ttc aag ttt gaa gtg tac acc cgc      288
   Arg Thr Thr Ala Glu Asp Pro Leu Phe Lys Phe Glu Val Tyr Thr Arg
                   85                  90                  95
   act gca caa atc tta aaa gat acg att gca ggg gcg ttt gat tgg gca      336
   Thr Ala Gln Ile Leu Lys Asp Thr Ile Ala Gly Ala Phe Asp Trp Ala
               100                 105                 110
   gtg gat aaa gat att tct gtc acg cta gtg aaa gat att att gaa gca      384
   Val Asp Lys Asp Ile Ser Val Thr Leu Val Lys Asp Ile Ile Glu Ala
           115                 120                 125
   atc aat gcg aag tgg cgt gat tac acc aca aaa ggc tac tta att ggc      432
   Ile Asn Ala Lys Trp Arg Asp Tyr Thr Thr Lys Gly Tyr Leu Ile Gly
       130                 135                 140
   ggt aaa gcg tgg ctt aat aaa gag ctt aac agt gca acg aat tta aaa      480
   Gly Lys Ala Trp Leu Asn Lys Glu Leu Asn Ser Ala Thr Asn Leu Lys
   145                 150                 155                 160
   gat gcg aag ttg ttg atc tct tat gat tat cac cca gta cca ccg ctc      528
   Asp Ala Lys Leu Leu Ile Ser Tyr Asp Tyr His Pro Val Pro Pro Leu
                   165                 170                 175
   gaa cag cta ggc ttt aat cag tac att tct gat gaa tac ctt gtt gat      576
   Glu Gln Leu Gly Phe Asn Gln Tyr Ile Ser Asp Glu Tyr Leu Val Asp
               180                 185                 190
   ttt tca aat cgt tta gca tcg taaggggtag aaaatggctt taccacgcaa         627
   Phe Ser Asn Arg Leu Ala Ser
           195
   acttaaattg atgaatttaa tcatcgacgg taacaaatat ctcggcgaag tcacggaagt    687
   gactcaacca aaattagcaa tgaaaatcga agaatttcgc gcgggcggta tgattggttc    747
   ggtggatgtc aatctcgggc ttgaaaagct cgaagcggaa tttaaagccg gtggctacat    807
   ggtcgaatta attaaaaaat tcggcgggtc aatcaacggc attccattgc gttttcttgg    867
   ctcatatcag cgtgatgaca cagaagaagt cacatctgtt gagcttgtga tgcaaggtcg    927
   atttactgaa attgacagcg gaaacagcaa agtgggcgat gacactgaac aaacattcaa    987
   agtgccttta acgtattaca aaatcattgt tga                                1020
<210> 67
   <211> 199
   <212> PRT
   <213> Pasteurella Multocida
<400> 67
   Val Asn Thr Ser Lys Val Glu Ile Asp Tyr Ala Val Thr Arg Ala Ala
   1               5                   10                  15
   Ala Met Arg Ala Tyr Leu Asp Lys Glu Gln Gly Trp His Thr Ser Ile
               20                  25                  30
   Ser Asn Lys Gly Ile Asn Gly Val Ser Gly Val Thr Gln Pro Leu Tyr
           35                  40                  45
   Phe Asp Ile Asn Asp Ser Ser Thr Asp Val Asn Tyr Leu Asn Glu Gln
       50                  55                  60
   Gly Ile Thr Cys Cys Val Asn His Asn Gly Phe Arg Phe Trp Gly Leu
   65                  70                  75                  80
   Arg Thr Thr Ala Glu Asp Pro Leu Phe Lys Phe Glu Val Tyr Thr Arg
                   85                  90                  95
   Thr Ala Gln Ile Leu Lys Asp Thr Ile Ala Gly Ala Phe Asp Trp Ala
               100                 105                 110
   Val Asp Lys Asp Ile Ser Val Thr Leu Val Lys Asp Ile Ile Glu Ala
           115                 120                 125
   Ile Asn Ala Lys Trp Arg Asp Tyr Thr Thr Lys Gly Tyr Leu Ile Gly
       130                 135                 140
   Gly Lys Ala Trp Leu Asn Lys Glu Leu Asn Ser Ala Thr Asn Leu Lys
   145                 150                 155                 160
   Asp Ala Lys Leu Leu Ile Ser Tyr Asp Tyr His Pro Val Pro Pro Leu
                   165                 170                 175
   Glu Gln Leu Gly Phe Asn Gln Tyr Ile Ser Asp Glu Tyr Leu Val Asp
               180                 185                 190
   Phe Ser Asn Arg Leu Ala Ser
           195
<210> 68
   <211> 2584
   <212> DNA
   <213> Pasteurella Multocida
<220>
   <221> misc_feature
   <223> xyla
<220>
   <221> CDS
   <222> (1042)..(2286)
<400> 68
   gtcgaccagc ttagattttg cgacggggtt aatttcttct atcgtttcaa tcattgcgtt      60
   taccattatt ttatggaatc tctctggacc gatgaccatt gccaatattg aaattcctca     120
   cgcgatggtc tttttggtct ttatttacgt gctgtttagc agtattgtgg catttaaaat     180
   cggtcgcccg ttaattcagc tcaattttgc caatgaacgc ttaaacgcca actaccgtta     240
   ttcacttatc cgtctgaaag aatatgctga aagcattgct ttttatcgtg gtgaaaaaat     300
   ggaaaaacgt ctattgacca cacaatttaa tcaggtgatt gataacgttt ggcaagtaat     360
   ctaccgcacc ttgaaattat ccggttttaa cttaatcatt acgcagattt cggtggtttt     420
   tccgctggtg attcaagtga cacgttattt tcgtcgacaa taggtgcata tgagggtgtt     480
   agaatagcga tactttctgt tggaaaagta aactctttaa tataaataga aatcgcttga     540
   atgattctcg ggcaaaaaat aatgtactca tttgcgatct catactgata atggcgaagt     600
   aaatatcttc ttacaatatt atggtaatta tcaggtaata ccgtatagcc atagattcca     660
   gttctatttt gttttgctaa ataattgatg agcatttgag gcgcaggtaa atccatatct     720
   gcaacagaca ttgaaatcat atccttgccg tatttacgag taattgccca tttagcacta     780
   tgacaatctg atctatcagt aaaaacatca aacaaattat ccgtcataca tgttctccaa     840
   tattggattt atataaactt tagaacttga ggtagattgt tggaattgtt aaatctggta     900
   tttctattac gttttttctt ttttgtgata taagccacaa taaccaataa tcttaattgt     960
   taagtgaaat aacgtaattg atcctcccat tgttttacta aattatgtct ctgaaactta    1020
   tttgttcagg agaaatcatt t atg tcc act tac ttc gac aaa att gaa aaa      1071
                           Met Ser Thr Tyr Phe Asp Lys Ile Glu Lys
                           1               5                   10
   gta aat tat gaa ggt gta act tca tct aat ccg ttt gca tat aag cat      1119
   Val Asn Tyr Glu Gly Val Thr Ser Ser Asn Pro Phe Ala Tyr Lys His
                   15                  20                  25
   tat gat gct aat caa gtt att tta ggt aag acg atg gct gaa cac tta      1167
   Tyr Asp Ala Asn Gln Val Ile Leu Gly Lys Thr Met Ala Glu His Leu
               30                  35                  40
   cgt tta gcc gtc tgt tat tgg cac act ttc tgt tgg aca ggg aat gat      1215
   Arg Leu Ala Val Cys Tyr Trp His Thr Phe Cys Trp Thr Gly Asn Asp
           45                  50                  55
   atg ttc ggt gtc ggt tct ttc gat cgt tgt tgg cag aag gcg agt gat      1263
   Met Phe Gly Val Gly Ser Phe Asp Arg Cys Trp Gln Lys Ala Ser Asp
       60                  65                  70
   tca tta gca ggt gca aaa caa aaa gca gat atc gct ttt gaa ttt ttc      1311
   Ser Leu Ala Gly Ala Lys Gln Lys Ala Asp Ile Ala Phe Glu Phe Phe
   75                  80                  85                  90
   agt aaa tta ggc ata cct tat tat tgt ttt cat gat gtt gat gtt gcg      1359
   Ser Lys Leu Gly Ile Pro Tyr Tyr Cys Phe His Asp Val Asp Val Ala
                   95                  100                 105
   cca gaa ggt cat tca ttt aaa gaa tat ttg tcg aac ttt aat aca atg      1407
   Pro Glu Gly His Ser Phe Lys Glu Tyr Leu Ser Asn Phe Asn Thr Met
               110                 115                 120
   atc gat gtt tta gcg cag aaa caa gaa gaa aca ggc gtc aaa ttg ttg      1455
   Ile Asp Val Leu Ala Gln Lys Gln Glu Glu Thr Gly Val Lys Leu Leu
           125                 130                 135
   tgg ggg act gca aat tgt ttt aca cac cct cgt tat atg tct ggt gct      1503
   Trp Gly Thr Ala Asn Cys Phe Thr His Pro Arg Tyr Met Ser Gly Ala
       140                 145                 150
   gca aca aat ccg aat cca gaa att ttt gct tgg gct gct gca caa gta      1551
   Ala Thr Asn Pro Asn Pro Glu Ile Phe Ala Trp Ala Ala Ala Gln Val
   155                 160                 165                 170
   ttt act gcc atg ggg gca act cag cgt tta ggt ggt gaa aat tat gtt      1599
   Phe Thr Ala Met Gly Ala Thr Gln Arg Leu Gly Gly Glu Asn Tyr Val
                   175                 180                 185
   ttg tgg gga gga cgt gaa gga tat gaa acg tta tta aat acc aat tta      1647
   Leu Trp Gly Gly Arg Glu Gly Tyr Glu Thr Leu Leu Asn Thr Asn Leu
               190                 195                 200
   aaa cag gag cga gag caa att gga cgt ttc atg caa atg gtg gtt gag      1695
   Lys Gln Glu Arg Glu Gln Ile Gly Arg Phe Met Gln Met Val Val Glu
           205                 210                 215
   cat aaa tat aaa atc ggt ttt aac ggg act ttg ctg att gaa cca aag      1743
   His Lys Tyr Lys Ile Gly Phe Asn Gly Thr Leu Leu Ile Glu Pro Lys
       220                 225                 230
   cca caa gag cca acg aaa cat caa tat gac tat gat gtg gcg acc gtt      1791
   Pro Gln Glu Pro Thr Lys His Gln Tyr Asp Tyr Asp Val Ala Thr Val
   235                 240                 245                 250
   tat ggc ttt tta aag cag ttt ggt tta gaa aaa gaa att aaa gtg aat      1839
   Tyr Gly Phe Leu Lys Gln Phe Gly Leu Glu Lys Glu Ile Lys Val Asn
                   255                 260                 265
   att gaa gct aat cac gca aca tta gct gga cac act ttc cag cat gaa      1887
   Ile Glu Ala Asn His Ala Thr Leu Ala Gly His Thr Phe Gln His Glu
               270                 275                 280
   gtc gcc atg gct aca gcg tta gat att ttt ggt tct att gat gca aat      1935
   Val Ala Met Ala Thr Ala Leu Asp Ile Phe Gly Ser Ile Asp Ala Asn
           285                 290                 295
   cgt ggt gat cca caa tta ggt tgg gat acc gat caa ttc cct aat agc      1983
   Arg Gly Asp Pro Gln Leu Gly Trp Asp Thr Asp Gln Phe Pro Asn Ser
       300                 305                 310
   gta gaa gaa aat act ttg gtc ata tat gaa att ctc aaa gca ggg ggc      2031
   Val Glu Glu Asn Thr Leu Val Ile Tyr Glu Ile Leu Lys Ala Gly Gly
   315                 320                 325                 330
   ttt aca acc ggt ggt ttt aat ttt gat gct aaa atc cgt cgg cag agt      2079
   Phe Thr Thr Gly Gly Phe Asn Phe Asp Ala Lys Ile Arg Arg Gln Ser
                   335                 340                 345
   acg gat cct tac gat tta ttt cat gga cat att ggc gcg att gat gta      2127
   Thr Asp Pro Tyr Asp Leu Phe His Gly His Ile Gly Ala Ile Asp Val
               350                 355                 360
   ctt gcc tta tca cta aaa tgt gcg gcg aaa atg ctt gaa gag caa gct      2175
   Leu Ala Leu Ser Leu Lys Cys Ala Ala Lys Met Leu Glu Glu Gln Ala
           365                 370                 375
   tta caa aaa gtc gtc aat caa cgt tat gct ggt tgg aca tca tca ctt      2223
   Leu Gln Lys Val Val Asn Gln Arg Tyr Ala Gly Trp Thr Ser Ser Leu
       380                 385                 390
   ggt caa ctt gtt caa atc cgg tcc tac cac gcg tgt ctg caa tac aga      2271
   Gly Gln Leu Val Gln Ile Arg Ser Tyr His Ala Cys Leu Gln Tyr Arg
   395                 400                 405                 410
   cta aca aaa gtg ctt taaaacgttc cggcttacgc cagacatcta gacgattgaa      2326
   Leu Thr Lys Val Leu
                   415
   taatttcaat attgtctccg cacgtaattc aaaggctttg tgtatgtgcg aatgatattc    2386
   acaacaaagt tctgcaaaat cttgaattgc gtgaggtaat ttaaagcgct gacataagcg    2446
   tcttgtcggc atgacaccag ctttttcatg tccataatga tgtggcaata tttcttttgg    2506
   tgttaaggct tttcctaaat catgacaaat tgcagcaaaa cgtaccgcac ttttgtcact    2566
   gtccgtgttt tctgtcga                                                  2584
<210> 69
   <211> 415
   <212> PRT
   <213> Pasteurella Multocida
<400> 69
   Met Ser Thr Tyr Phe Asp Lys Ile Glu Lys Val Asn Tyr Glu Gly Val
   1               5                   10                  15
   Thr Ser Ser Asn Pro Phe Ala Tyr Lys His Tyr Asp Ala Asn Gln Val
               20                  25                  30
   Ile Leu Gly Lys Thr Met Ala Glu His Leu Arg Leu Ala Val Cys Tyr
           35                  40                  45
   Trp His Thr Phe Cys Trp Thr Gly Asn Asp Met Phe Gly Val Gly Ser
       50                  55                  60
   Phe Asp Arg Cys Trp Gln Lys Ala Ser Asp Ser Leu Ala Gly Ala Lys
   65 70 75 80
   Gln Lys Ala Asp Ile Ala Phe Glu Phe Phe Ser Lys Leu Gly Ile Pro
   85 90 95
   Tyr Tyr Cys Phe His Asp Val Asp Val Ala Pro Glu Gly His Ser Phe
   100 105 110
   Lys Glu Tyr Leu Ser Asn Phe Asn Thr Met Ile Asp Val Leu Ala Gln
   115 120 125
   Lys Gln Glu Glu Thr Gly Val Lys Leu Leu Trp Gly Thr Ala Asn Cys
   130 135 140
   Phe Thr His Pro Arg Tyr Met Ser Gly Ala Ala Thr Asn Pro Asn Pro
   145 150 155 160
   Glu Ile Phe Ala Trp Ala Ala Ala Gln Val Phe Thr Ala Met Gly Ala
   165 170 175
   Thr Gln Arg Leu Gly Gly Glu Asn Tyr Val Leu Trp Gly Gly Arg Glu
   180 185 190
   Gly Tyr Glu Thr Leu Leu Asn Thr Asn Leu Lys Gln Glu Arg Glu Gln
   195 200 205
   Ile Gly Arg Phe Met Gln Met Val Val Glu His Lys Tyr Lys Ile Gly
   210 215 220
   Phe Asn Gly Thr Leu Leu Ile Glu Pro Lys Pro Gln Glu Pro Thr Lys
   225 230 235 240
   His Gln Tyr Asp Tyr Asp Val Ala Thr Val Tyr Gly Phe Leu Lys Gln
   245 250 255
   Phe Gly Leu Glu Lys Glu Ile Lys Val Asn Ile Glu Ala Asn His Ala
   260 265 270
   Thr Leu Ala Gly His Thr Phe Gln His Glu Val Ala Met Ala Thr Ala
   275 280 285
   Leu Asp Ile Phe Gly Ser Ile Asp Ala Asn Arg Gly Asp Pro Gln Leu
   290 295 300
   Gly Trp Asp Thr Asp Gln Phe Pro Asn Ser Val Glu Glu Asn Thr Leu
   305 310 315 320
   Val Ile Tyr Glu Ile Leu Lys Ala Gly Gly Phe Thr Thr Gly Gly Phe
   325 330 335
   Asn Phe Asp Ala Lys Ile Arg Arg Gln Ser Thr Asp Pro Tyr Asp Leu
   340 345 350
   Phe His Gly His Ile Gly Ala Ile Asp Val Leu Ala Leu Ser Leu Lys
   355 360 365
   Cys Ala Ala Lys Met Leu Glu Glu Gln Ala Leu Gln Lys Val Val Asn
   370 375 380
   Gln Arg Tyr Ala Gly Trp Thr Ser Ser Leu Gly Gln Leu Val Gln Ile
   385 390 395 400
   Arg Ser Tyr His Ala Cys Leu Gln Tyr Arg Leu Thr Lys Val Leu
   405 410 415
<210> 70
   <211> 3501
   <212> DNA
   <213> Pasteurella Multocida
<220>
   <221> misc_feature
   <223> yabk
<220>
   <221> CDS
   <222> (298)..(1905)
<400> 70
   gaattcgagg aagggggcgt attacaaatt gaaacggctg cacgtgtagc acaacatgat 60
   aatgcctgtg cggatcattt ccttgccttt ttacttcatc cagaagcaca agggcattta 120
   gtcaagaata atgtgatgtt accggtgatt aataccaata ttgaaccgca ctttgatgcc 180
   cttagagcca cccaaatgaa cacgaaagtg ctcgatacct caaaagtgaa tgccgaacaa 240
   gtcaaaaaat ggattgctgt ttggcaaacg accctaaccc aataattgtt tgtcttg 297
   atg ttt aag cga ttt cgt gca ttc aca tac cgt ccc gcc agt tat ctt 345
   Met Phe Lys Arg Phe Arg Ala Phe Thr Tyr Arg Pro Ala Ser Tyr Leu
   1 5 10 15
   ggc ggg atg ttg gtg att gtt ttt ctg agc gct ttt tat gcg ttc gcc 393
   Gly Gly Met Leu Val Ile Val Phe Leu Ser Ala Phe Tyr Ala Phe Ala
   20 25 30
   tta ggg gcg gtt ttt tcg ctc cct ttt gcg cgc agt tgg aca gcg ttg 441
   Leu Gly Ala Val Phe Ser Leu Pro Phe Ala Arg Ser Trp Thr Ala Leu
           35                  40                  45
   ttg agt gat cag tat tta caa cac gtg atc atc ttt agc ttt tgg caa      489
   Leu Ser Asp Gln Tyr Leu Gln His Val Ile Ile Phe Ser Phe Trp Gln
       50                  55                  60
   gcc ttt ctg tcg gcg gta ctt gcg gtc ctc ttt ggt ggc att gta gca      537
   Ala Phe Leu Ser Ala Val Leu Ala Val Leu Phe Gly Gly Ile Val Ala
   65                  70                  75                  80
   cga gcc ttt ttt tat caa ccg ttt gtg ggc aag aaa ctg atc ctc aaa      585
   Arg Ala Phe Phe Tyr Gln Pro Phe Val Gly Lys Lys Leu Ile Leu Lys
                   85                  90                  95
   tta ttt tca ctg act ttt gtg tta cct gcc tta gtg gcg att ttt ggt      633
   Leu Phe Ser Leu Thr Phe Val Leu Pro Ala Leu Val Ala Ile Phe Gly
               100                 105                 110
   tta tta ggc gtg tat ggc gct tct ggc tgg tta gcg atg tta agc cag      681
   Leu Leu Gly Val Tyr Gly Ala Ser Gly Trp Leu Ala Met Leu Ser Gln
           115                 120                 125
   ttt ttc gct tgg gat tgg act cct aat att tac ggc tta aca ggt att      729
   Phe Phe Ala Trp Asp Trp Thr Pro Asn Ile Tyr Gly Leu Thr Gly Ile
       130                 135                 140
   tta ctg gcg cat ctt ttt ttt aat gtc cca tta gct tgt cgc ctg ttt      777
   Leu Leu Ala His Leu Phe Phe Asn Val Pro Leu Ala Cys Arg Leu Phe
   145                 150                 155                 160
   tta caa ggt ttg caa gca att ccg gtg caa caa cgt cag ctc gcg gca      825
   Leu Gln Gly Leu Gln Ala Ile Pro Val Gln Gln Arg Gln Leu Ala Ala
                   165                 170                 175
   caa ctc aat tta cgt ggt tgg cat ttt ata cgt ctg att gag tgg ccc      873
   Gln Leu Asn Leu Arg Gly Trp His Phe Ile Arg Leu Ile Glu Trp Pro
               180                 185                 190
   tat tta cgc cag caa ttg tta cct gca ttt act ttg att ttc atg ctg      921
   Tyr Leu Arg Gln Gln Leu Leu Pro Ala Phe Thr Leu Ile Phe Met Leu
           195                 200                 205
   tgt ttt acc agt ttt gcg att gtg ctc act tta ggt ggc gga ccg aaa      969
   Cys Phe Thr Ser Phe Ala Ile Val Leu Thr Leu Gly Gly Gly Pro Lys
       210                 215                 220
   tat acc acg ttg gaa gtg gct atc tat caa gcg att tta ttt gag ttt     1017
   Tyr Thr Thr Leu Glu Val Ala Ile Tyr Gln Ala Ile Leu Phe Glu Phe
   225                 230                 235                 240
   gat gta ccg aaa gcc ggc tta ttt gcg tta tta caa ttt gtt ttt tgt     1065
   Asp Val Pro Lys Ala Gly Leu Phe Ala Leu Leu Gln Phe Val Phe Cys
                   245                 250                 255
   ttt ctg tta ttc acg ctg agt agc ttt ttt tct cca gcc ccc gcc acg     1113
   Phe Leu Leu Phe Thr Leu Ser Ser Phe Phe Ser Pro Ala Pro Ala Thr
               260                 265                 270
   aca tta cac agt caa cct act tgg ttt gcg ccc caa tcg tat tgg gtt     1161
   Thr Leu His Ser Gln Pro Thr Trp Phe Ala Pro Gln Ser Tyr Trp Val
           275                 280                 285
   aaa tta tgg caa cgt atg atc att gtg tgt gcg aca gta ttt atc tta      1209
   Lys Leu Trp Gln Arg Met Ile Ile Val Cys Ala Thr Val Phe Ile Leu
       290                 295                 300
   tta ccg cta ctc aat acg cta gtt tct gct ttg ctt tcg tct cag ttt      1257
   Leu Pro Leu Leu Asn Thr Leu Val Ser Ala Leu Leu Ser Ser Gln Phe
   305                 310                 315                 320
   ttt acc ttg tgg tta caa cct caa tta tgg aaa gca tta ggt tac tcg      1305
   Phe Thr Leu Trp Leu Gln Pro Gln Leu Trp Lys Ala Leu Gly Tyr Ser
                   325                 330                 335
   ctc acc atc gcc ccc act tct gca ttg ctc gct tta gta ctg tct ttt      1353
   Leu Thr Ile Ala Pro Thr Ser Ala Leu Leu Ala Leu Val Leu Ser Phe
               340                 345                 350
   gcc tta tta ttg ctt gcc aga gaa tta cat tgg cga cat tat cgc agc      1401
   Ala Leu Leu Leu Leu Ala Arg Glu Leu His Trp Arg His Tyr Arg Ser
           355                 360                 365
   tta tcc cat gtg att tta aat atc ggt gcg acc att tta gcc att cca      1449
   Leu Ser His Val Ile Leu Asn Ile Gly Ala Thr Ile Leu Ala Ile Pro
       370                 375                 380
   acg tta gtg tta gct att ggt tta ttc att tta tta cgt gag atc gat      1497
   Thr Leu Val Leu Ala Ile Gly Leu Phe Ile Leu Leu Arg Glu Ile Asp
   385                 390                 395                 400
   ttt tct cca tac cat ctt ttt ggg gtt gtg gta tgc tgt aac gcg tta      1545
   Phe Ser Pro Tyr His Leu Phe Gly Val Val Val Cys Cys Asn Ala Leu
                   405                 410                 415
   gct gct atg cct ttt gtg ttg cgt att ttg gct tta ccg atg cat aac      1593
   Ala Ala Met Pro Phe Val Leu Arg Ile Leu Ala Leu Pro Met His Asn
               420                 425                 430
   aat atg att tat tat gaa aaa tta tgc caa tca ctt aac ctg cgt ggt      1641
   Asn Met Ile Tyr Tyr Glu Lys Leu Cys Gln Ser Leu Asn Leu Arg Gly
           435                 440                 445
   tgg caa cgt ttt cga ttg att gaa tgg cac aag ctt cgt gcg cca atg      1689
   Trp Gln Arg Phe Arg Leu Ile Glu Trp His Lys Leu Arg Ala Pro Met
       450                 455                 460
   aaa tac gcc ttt gca ctg gct tgt gcg tta tca tta ggc gat ttc acc      1737
   Lys Tyr Ala Phe Ala Leu Ala Cys Ala Leu Ser Leu Gly Asp Phe Thr
   465                 470                 475                 480
   gca atc gcg tta ttt ggt cag gct gac ttc aca tcg tta ccg cat ttg      1785
   Ala Ile Ala Leu Phe Gly Gln Ala Asp Phe Thr Ser Leu Pro His Leu
                   485                 490                 495
   ttg tat caa caa ttg ggg cat tat cgt agt cag gaa gcg gca gta aca      1833
   Leu Tyr Gln Gln Leu Gly His Tyr Arg Ser Gln Glu Ala Ala Val Thr
               500                 505                 510
   gcg ttt att tta ttg gtt ttt tgt ttg agt gtt ttt atg att att gaa      1881
   Ala Phe Ile Leu Leu Val Phe Cys Leu Ser Val Phe Met Ile Ile Glu
           515                 520                 525
   cga cat cag gaa ccg cgt gat gat taatttaaac ggtgttcagt tttcctataa    1935
   Arg His Gln Glu Pro Arg Asp Asp
       530                 535
   tacctttact tttgagctgg atttgcagat tcctgctcaa caaaaagttg ctattattgg   1995
   cgccagtggc gcagggaaga gtaccttatt aaatttgatt gcgggttttg cattgccaca   2055
   gcaaggggaa atttggttga atggtgaaaa tcatagccaa actcaacctt atgaacgtcc   2115
   ggtatctatt ttgtttcaag aaaacaactt gtttacgcat ttgactgtgg cagagaatat   2175
   ggcattaggg ctgaaaccaa gcctaaaact gaccgcactt gaacaacaac gcgtacaaca   2235
   agtggcaagt gcagtgggtt tgcagggttt tcttaatcaa ttacccaccc agttatcggg   2295
   tgggcaaaaa cagcgtgtgg cgttggcgcg ttgtttatta cgcgataagc caattttgtt   2355
   attggatgaa cctttttctg ccttagatcc cgatttacgg gcagaaatgt tgcatttatt   2415
   gttacagttg tgtgatgaaa aaaaattaac actcctgatc gtgacacatc aagtgaacga   2475
   attacagcag aaaatggatc gtatgattcg ttttgaacat ggtaggatga gtgagtccac   2535
   cattttgaag gataatttta acgaaaaaca gaccgcactt taggcggctt attaattaga   2595
   taaggaaaga gaaaaatatg acaacggcaa cggcatttga gattcgtaca cttactcctc   2655
   acccgacctt agaatattgg tctgtgtgta aaattgaagc cctgtttgag accccttttt   2715
   tagatttggt ttatcgggct gcgcaagtgc accgagaaaa ctttaatcct aaagccattc   2775
   aattatccac tttaatgtcg attaaaacgg ggggatgtcc agaggattgt ggctactgtc   2835
   cgcagtcagc acgttatcat actggagtag aaaagcagca attactcgat gtggaagaaa   2895
   ttgttgaaaa agccaaaatt gccaaagcac gtggtgcagg gcgcttttgt atgggggctg   2955
   catggcgtgg accgaaaccg aaagacattg aaaaagttac cgcaatcatt aaagcggtga   3015
   aagaactggg cttagaaacc tgtggtacct ttggtttatt gcaagatggg atggcagaag   3075
   atttaaaaga agcgggtttg gattattata accataatct cgatacagcc ccagaacact   3135
   acggtaatgt gattggtacc cgtcaatttg atgatcgtat taatacgtta ggtaaagtgc   3195
   gtaaagctgg cttaaaagtg tgctgtggcg ggattattgg catgaatgaa acccgtaaag   3255
   aaagagcagg attaattgct agcttagcta atttagaccc gcaacccgaa tcggtgccga   3315
   ttaatcaatt agtgaaagtg gaaggtaccc ctttagccga tgcggcagaa ttagactgga   3375
   cagaatttgt gcgcactatt gcggtggcgc gtattaccat gccgaaaagc tatgtacgtt   3435
   tatcagcagg gcgtcaaggc atgtcggaag aaatgcaagc catgtgcttt atggctggcg   3495
   cgaatt                                                              3501
<210> 71
   <211> 536
   <212> PRT
   <213> Pasteurella Multocida
<400> 71
   Met Phe Lys Arg Phe Arg Ala Phe Thr Tyr Arg Pro Ala Ser Tyr Leu
   1               5                   10                  15
   Gly Gly Met Leu Val Ile Val Phe Leu Ser Ala Phe Tyr Ala Phe Ala
               20                  25                  30
   Leu Gly Ala Val Phe Ser Leu Pro Phe Ala Arg Ser Trp Thr Ala Leu
           35                  40                  45
   Leu Ser Asp Gln Tyr Leu Gln His Val Ile Ile Phe Ser Phe Trp Gln
       50                  55                  60
   Ala Phe Leu Ser Ala Val Leu Ala Val Leu Phe Gly Gly Ile Val Ala
   65                  70                  75                  80
   Arg Ala Phe Phe Tyr Gln Pro Phe Val Gly Lys Lys Leu Ile Leu Lys
                   85                  90                  95
   Leu Phe Ser Leu Thr Phe Val Leu Pro Ala Leu Val Ala Ile Phe Gly
               100                 105                 110
   Leu Leu Gly Val Tyr Gly Ala Ser Gly Trp Leu Ala Met Leu Ser Gln
           115                 120                 125
   Phe Phe Ala Trp Asp Trp Thr Pro Asn Ile Tyr Gly Leu Thr Gly Ile
       130                 135                 140
   Leu Leu Ala His Leu Phe Phe Asn Val Pro Leu Ala Cys Arg Leu Phe
   145                 150                 155                 160
   Leu Gln Gly Leu Gln Ala Ile Pro Val Gln Gln Arg Gln Leu Ala Ala
                   165                 170                 175
   Gln Leu Asn Leu Arg Gly Trp His Phe Ile Arg Leu Ile Glu Trp Pro
               180                 185                 190
   Tyr Leu Arg Gln Gln Leu Leu Pro Ala Phe Thr Leu Ile Phe Met Leu
           195                 200                 205
   Cys Phe Thr Ser Phe Ala Ile Val Leu Thr Leu Gly Gly Gly Pro Lys
       210                 215                 220
   Tyr Thr Thr Leu Glu Val Ala Ile Tyr Gln Ala Ile Leu Phe Glu Phe
   225                 230                 235                 240
   Asp Val Pro Lys Ala Gly Leu Phe Ala Leu Leu Gln Phe Val Phe Cys
                   245                 250                 255
   Phe Leu Leu Phe Thr Leu Ser Ser Phe Phe Ser Pro Ala Pro Ala Thr
               260                 265                 270
   Thr Leu His Ser Gln Pro Thr Trp Phe Ala Pro Gln Ser Tyr Trp Val
           275                 280                 285
   Lys Leu Trp Gln Arg Met Ile Ile Val Cys Ala Thr Val Phe Ile Leu
       290                 295                 300
   Leu Pro Leu Leu Asn Thr Leu Val Ser Ala Leu Leu Ser Ser Gln Phe
   305                 310                 315                 320
   Phe Thr Leu Trp Leu Gln Pro Gln Leu Trp Lys Ala Leu Gly Tyr Ser
                   325                 330                 335
   Leu Thr Ile Ala Pro Thr Ser Ala Leu Leu Ala Leu Val Leu Ser Phe
               340                 345                 350
   Ala Leu Leu Leu Leu Ala Arg Glu Leu His Trp Arg His Tyr Arg Ser
           355                 360                 365
   Leu Ser His Val Ile Leu Asn Ile Gly Ala Thr Ile Leu Ala Ile Pro
       370                 375                 380
   Thr Leu Val Leu Ala Ile Gly Leu Phe Ile Leu Leu Arg Glu Ile Asp
   385                 390                 395                 400
   Phe Ser Pro Tyr His Leu Phe Gly Val Val Val Cys Cys Asn Ala Leu
                   405                 410                 415
   Ala Ala Met Pro Phe Val Leu Arg Ile Leu Ala Leu Pro Met His Asn
               420                 425                 430
   Asn Met Ile Tyr Tyr Glu Lys Leu Cys Gln Ser Leu Asn Leu Arg Gly
           435                 440                 445
   Trp Gln Arg Phe Arg Leu Ile Glu Trp His Lys Leu Arg Ala Pro Met
       450                 455                 460
   Lys Tyr Ala Phe Ala Leu Ala Cys Ala Leu Ser Leu Gly Asp Phe Thr
   465 470 475 480
   Ala Ile Ala Leu Phe Gly Gln Ala Asp Phe Thr Ser Leu Pro His Leu
   485 490 495
   Leu Tyr Gln Gln Leu Gly His Tyr Arg Ser Gln Glu Ala Ala Val Thr
   500 505 510
   Ala Phe Ile Leu Leu Val Phe Cys Leu Ser Val Phe Met Ile Ile Glu
   515 520 525
   Arg His Gln Glu Pro Arg Asp Asp
   530 535
<210> 72
   <211> 3182
   <212> DNA
   <213> Pasteurella Multocida
<220>
   <221> misc_feature
   <223> ygiK
<220>
   <221> misc_feature
   <222> (452)..(452)
   <223> n is a, c, g, or t
<220>
   <221> CDS
   <222> (1544)..(2809)
<400> 72
   aactactaag gagttatgta tgaatattag aaaatcatta ctattaatat ccctagcaag 60
   cttcatgtca ctttcagttt cagctgcaga aattaatttg aaatttgaaa gttcgaattt 120
   tgcaggagaa aaagtttatg aaatccaaaa agaatggact gacaatattg aaaaagcttc 180
   caatgggaga ataagtatag agttattacc tctcgactca gtcttaaaat ctagtgacat 240
   gctttctggt gttcgaaata aaattattga tggagcggtt gcaacagcgg caatgtatgc 300
   aggcactgac cctggattcg gattaattgg tgatactatt tctgcttgga accatgacga 360
   agatatttta aatttttact ataatggagg tggttttgaa gttgttgata atattttcca 420
   acaatatggt gccaaactca ttggtgtatc anttacggga gcagaatcat taccatcgaa 480
   agtaaaaata gctaatactg aagattttaa aggtataaaa attcgggctc cctctggtcc 540
   tatccaaaaa ttgtttgcaa gattaggagc cgctcctgtt ggtcttcctg gttcagaaat 600
   ctatactagt ttagaaaaag gtattattga tgctgccgat ttctcaacgt ttgcaaataa 660
   tcaagcacaa ggagtccatg atattgcaaa atatccaatc tatccgggaa ttcattcttc    720
   accagccgtt catatgatta tgaatcataa aacttggagt agcttaactc catcggatca    780
   agcattctta attgcttact ttaaagggat ggctctcgat actctgactc gtgctcatta    840
   tgaagataaa ctagcatata aagaagcact tgagaaagga gtacaaccag tttcttggaa    900
   tcaacaagaa attacaaaag ttcgttctat cgctaaagaa atttggcaag agatagctca    960
   acaatctgaa ataggtaatc aagtattatc aagtattaat gaaattccta gaatctcaag   1020
   gaatgctgca ataatcgtca agatggataa gacgatatcg tcttatccat taaggagtaa   1080
   aacatgctta tttctaaata tttattatgg ctctgtaata agctagatca aatattcatt   1140
   aaagtaggtt attacgtttc ttatattttt ctattagttg ttatcattgg ttttacgagg   1200
   ttgttgctcg gtatttattc tctagcccaa cactttgggt tcatgaagta acaacatttt   1260
   taataagtct atcattactt tatggtggag tagcttgtta cgccagtaat aaacatattg   1320
   ccatgacatt tattagacaa aaattaccta ataagatcaa atggttacta gaactcttag   1380
   ttgaaatact tatttttatc ttctttattt tgcttagtta cggagcatac ttatcagcta   1440
   gagaagcatt atttactcca tcaggaaaat tcaaaatgca aacttctgga agtgtattag   1500
   acatgccatt tccagcaatt gaaaaaagtt tcttctttat ttc atg cct cat cat     1555
                                                   Met Pro His His
                                                   1
   tgt tgt tct ttc agt act aca tat att ccg tca cat cta tac aaa ata     1603
   Cys Cys Ser Phe Ser Thr Thr Tyr Ile Pro Ser His Leu Tyr Lys Ile
   5                   10                  15                  20
   tca gga gga att atc atg ata agt gca ttt ggg ata ggt att gga act     1651
   Ser Gly Gly Ile Ile Met Ile Ser Ala Phe Gly Ile Gly Ile Gly Thr
                   25                  30                  35
   ctt att atc ttt tta atg atg att tcc ctt tta ttt att gga atg cca     1699
   Leu Ile Ile Phe Leu Met Met Ile Ser Leu Leu Phe Ile Gly Met Pro
               40                  45                  50
   tta ggt ttt ctc act ggg tta atc gct tta gtt att tca tat ctt tgg     1747
   Leu Gly Phe Leu Thr Gly Leu Ile Ala Leu Val Ile Ser Tyr Leu Trp
           55                  60                  65
   ttt gat act acc gca ata atg caa atg ata gct tca cgt gtc act gat     1795
   Phe Asp Thr Thr Ala Ile Met Gln Met Ile Ala Ser Arg Val Thr Asp
       70                  75                  80
   ttc aca tca tct tac act ttt gta gct gtg cct atg ttt gtt ctt atg     1843
   Phe Thr Ser Ser Tyr Thr Phe Val Ala Val Pro Met Phe Val Leu Met
   85                  90                  95                  100
   gca aca tta ctt gat aag act gga att gct aga gat ctc tac aac gca     1891
   Ala Thr Leu Leu Asp Lys Thr Gly Ile Ala Arg Asp Leu Tyr Asn Ala
                   105                 110                 115
   atg cga gtc att ggc ggt cga tta cga ggt gga att gca att caa tcg      1939
   Met Arg Val Ile Gly Gly Arg Leu Arg Gly Gly Ile Ala Ile Gln Ser
               120                 125                 130
   atg ttt gtt gca gtt cta ctt gct acg atg tca ggt att atc ggt gga      1987
   Met Phe Val Ala Val Leu Leu Ala Thr Met Ser Gly Ile Ile Gly Gly
           135                 140                 145
   gaa act gtt tta tta ggc atg ttg gca tta cca caa atg tta cgc tta      2035
   Glu Thr Val Leu Leu Gly Met Leu Ala Leu Pro Gln Met Leu Arg Leu
       150                 155                 160
   ggc tat aat aaa aac tta gct ata gga act gtt gta gca gga gga gca      2083
   Gly Tyr Asn Lys Asn Leu Ala Ile Gly Thr Val Val Ala Gly Gly Ala
   165                 170                 175                 180
   ttg ggt aca atg gtt cct cca agt atc gtg ttg att att tac gga atg      2131
   Leu Gly Thr Met Val Pro Pro Ser Ile Val Leu Ile Ile Tyr Gly Met
                   185                 190                 195
   acc gca aat gtt tct att gga gaa cta ttt ctt gca gca att cca gcc      2179
   Thr Ala Asn Val Ser Ile Gly Glu Leu Phe Leu Ala Ala Ile Pro Ala
               200                 205                 210
   tcc tta cta ctt tct aca ttc tat att tta tat att cta gta ctt tgc      2227
   Ser Leu Leu Leu Ser Thr Phe Tyr Ile Leu Tyr Ile Leu Val Leu Cys
           215                 220                 225
   tac ttc aaa cct agc tat ggc cct gca atg cct agc tca gaa aat cat      2275
   Tyr Phe Lys Pro Ser Tyr Gly Pro Ala Met Pro Ser Ser Glu Asn His
       230                 235                 240
   aca tta acg aaa gaa gat att aaa aaa att att cat gat att gca att      2323
   Thr Leu Thr Lys Glu Asp Ile Lys Lys Ile Ile His Asp Ile Ala Ile
   245                 250                 255                 260
   cca gta gct atc gcc aca tgg att tta gga agt att tat ggc ggg ata      2371
   Pro Val Ala Ile Ala Thr Trp Ile Leu Gly Ser Ile Tyr Gly Gly Ile
                   265                 270                 275
   gca tca atc act gaa tct gcc tgt gtt ggt gta gtt ggg gta ata tta      2419
   Ala Ser Ile Thr Glu Ser Ala Cys Val Gly Val Val Gly Val Ile Leu
               280                 285                 290
   gca gca ttc tat cga aaa gaa tta aat ttc aaa ata gta caa gaa tca      2467
   Ala Ala Phe Tyr Arg Lys Glu Leu Asn Phe Lys Ile Val Gln Glu Ser
           295                 300                 305
   cta aaa cat aca atc aat act gtt ggt atg ata atc tgg gtc ggc att      2515
   Leu Lys His Thr Ile Asn Thr Val Gly Met Ile Ile Trp Val Gly Ile
       310                 315                 320
   ggc gca aca atg att ata ggt att tat aat cta atg ggt ggg gac cga      2563
   Gly Ala Thr Met Ile Ile Gly Ile Tyr Asn Leu Met Gly Gly Asp Arg
   325                 330                 335                 340
   ttt ata gct aac tta ttc gct agc tta gat gcc tct cca att tat act      2611
   Phe Ile Ala Asn Leu Phe Ala Ser Leu Asp Ala Ser Pro Ile Tyr Thr
                   345                 350                 355
   atc att att atg atg gtt att tta tta ata ctt ggt atg ttc tta gat      2659
   Ile Ile Ile Met Met Val Ile Leu Leu Ile Leu Gly Met Phe Leu Asp
               360                 365                 370
   tgg att ggt gtt gcc atg ttg act ttc ctc aag aca agt aaa gcg aca      2707
   Trp Ile Gly Val Ala Met Leu Thr Phe Leu Lys Thr Ser Lys Ala Thr
           375                 380                 385
   atc aat ttg tgt ttt gac ata gtc agg tac agt att tgg cgt ggt ccc      2755
   Ile Asn Leu Cys Phe Asp Ile Val Arg Tyr Ser Ile Trp Arg Gly Pro
       390                 395                 400
   tcc ttc cac agt acc aat gtt cat cgt ggt acc ttt gtc ggg cgc ggt      2803
   Ser Phe His Ser Thr Asn Val His Arg Gly Thr Phe Val Gly Arg Gly
   405                 410                 415                 420
   act ttt tagtaaatct tgcgcgatac gaataaacgc attgatggca tttgctccgt       2859
   Thr Phe
   tttgtggatc gactgccgca tgagcagatt tgccaaaaaa ttcaattaca tacttcccaa    2919
   tcccttttct ttcgttaacg tttccactta gattgcccag aagccgatct gtctgaatgg   2979
   gaacaagtgt tataccaaga agcgaatcca acaggtgaag tggtgatcgg tatggtgggt    3039
   aaatacactg aattaccgga tgcctacaaa tcggttaatg aagccttgaa acacgcaggc    3099
   ttaaaaaacc gtcttagcgt gcaaatcaaa tatattgatt cacaagatgt ggaaaccaaa    3159
   ggcacagaag tgttagaagg cgt                                            3182
<210> 73
   <211> 422
   <212> PRT
   <213> Pasteurella Multocida
<400> 73
   Met Pro His His Cys Cys Ser Phe Ser Thr Thr Tyr Ile Pro Ser His
   1               5                   10                  15
   Leu Tyr Lys Ile Ser Gly Gly Ile Ile Met Ile Ser Ala Phe Gly Ile
               20                  25                  30
   Gly Ile Gly Thr Leu Ile Ile Phe Leu Met Met Ile Ser Leu Leu Phe
           35                  40                  45
   Ile Gly Met Pro Leu Gly Phe Leu Thr Gly Leu Ile Ala Leu Val Ile
       50                  55                  60
   Ser Tyr Leu Trp Phe Asp Thr Thr Ala Ile Met Gln Met Ile Ala Ser
   65                  70                  75                  80
   Arg Val Thr Asp Phe Thr Ser Ser Tyr Thr Phe Val Ala Val Pro Met
                   85                  90                  95
   Phe Val Leu Met Ala Thr Leu Leu Asp Lys Thr Gly Ile Ala Arg Asp
               100                 105                 110
   Leu Tyr Asn Ala Met Arg Val Ile Gly Gly Arg Leu Arg Gly Gly Ile
           115                 120                 125
   Ala Ile Gln Ser Met Phe Val Ala Val Leu Leu Ala Thr Met Ser Gly
       130                 135                 140
   Ile Ile Gly Gly Glu Thr Val Leu Leu Gly Met Leu Ala Leu Pro Gln
   145                 150                 155                 160
   Met Leu Arg Leu Gly Tyr Asn Lys Asn Leu Ala Ile Gly Thr Val Val
                   165                 170                 175
   Ala Gly Gly Ala Leu Gly Thr Met Val Pro Pro Ser Ile Val Leu Ile
               180                 185                 190
   Ile Tyr Gly Met Thr Ala Asn Val Ser Ile Gly Glu Leu Phe Leu Ala
           195                 200                 205
   Ala Ile Pro Ala Ser Leu Leu Leu Ser Thr Phe Tyr Ile Leu Tyr Ile
       210                 215                 220
   Leu Val Leu Cys Tyr Phe Lys Pro Ser Tyr Gly Pro Ala Met Pro Ser
   225                 230                 235                 240
   Ser Glu Asn His Thr Leu Thr Lys Glu Asp Ile Lys Lys Ile Ile His
                   245                 250                 255
   Asp Ile Ala Ile Pro Val Ala Ile Ala Thr Trp Ile Leu Gly Ser Ile
               260                 265                 270
   Tyr Gly Gly Ile Ala Ser Ile Thr Glu Ser Ala Cys Val Gly Val Val
           275                 280                 285
   Gly Val Ile Leu Ala Ala Phe Tyr Arg Lys Glu Leu Asn Phe Lys Ile
       290                 295                 300
   Val Gln Glu Ser Leu Lys His Thr Ile Asn Thr Val Gly Met Ile Ile
   305                 310                 315                 320
   Trp Val Gly Ile Gly Ala Thr Met Ile Ile Gly Ile Tyr Asn Leu Met
                   325                 330                 335
   Gly Gly Asp Arg Phe Ile Ala Asn Leu Phe Ala Ser Leu Asp Ala Ser
               340                 345                 350
   Pro Ile Tyr Thr Ile Ile Ile Met Met Val Ile Leu Leu Ile Leu Gly
           355                 360                 365
   Met Phe Leu Asp Trp Ile Gly Val Ala Met Leu Thr Phe Leu Lys Thr
       370                 375                 380
   Ser Lys Ala Thr Ile Asn Leu Cys Phe Asp Ile Val Arg Tyr Ser Ile
   385                 390                 395                 400
   Trp Arg Gly Pro Ser Phe His Ser Thr Asn Val His Arg Gly Thr Phe
                   405                 410                 415
   Val Gly Arg Gly Thr Phe
               420
<210> 74
   <211> 2787
   <212> DNA
   <213> Pasteurella Multocida
<220>
   <221> misc_feature
   <223> yhcJ
<220>
   <221> CDS
   <222> (463)..(936)
<400> 74
   gttaacacac catgattaat gatgccggtt gaagccactg caacgtaatc gaattgtccg      60
   gcatacaaag caagaatgtt ggccagtgtg tcatgcatcg cattggcagc atcagcttgt     120
   ggcgttgcaa tctgttggcg ttgttctatt ttgccgtctg ttacaatagc cgaggcaatt     180
   tttgttccac caatatctaa tgctaaacag cgcataggct ctccttctgt gatgacttat     240
   tttgccgatt tgacggcatc ggcaaaccag cttacgatat gttcgaggcg agtcagcgca     300
   gatcctacgg tgacagagta agcaccaatc tcaattgcgg ttttcgccaa ttctggggtg     360
   ttatagcgcc cttctgccat cactcggcag ccagcagcat tcaaatcttt gactaactga     420
   taatccggtt cagctggaat ttcaccgcca gtataaccag ac atg gtg cta cca        474
                                                  Met Val Leu Pro
                                                  1
   ata att tct acc cct aag ttg tgg caa tac atc cct tct tca aaa tta       522
   Ile Ile Ser Thr Pro Lys Leu Trp Gln Tyr Ile Pro Ser Ser Lys Leu
   5                   10                  15                  20
   gaa caa tcc gcc atg gct aaa caa cct aat tct ttg att cgt tta ata      570
   Glu Gln Ser Ala Met Ala Lys Gln Pro Asn Ser Leu Ile Arg Leu Ile
                   25                  30                  35
   atg gct tca cgt gta gtt gga cgg acg cga tcg gta cca tca aaa gca      618
   Met Ala Ser Arg Val Val Gly Arg Thr Arg Ser Val Pro Ser Lys Ala
               40                  45                  50
   ata ata tcg gcg cct gct gcg gct aac tct tca atg tct tgt aaa aat      666
   Ile Ile Ser Ala Pro Ala Ala Ala Asn Ser Ser Met Ser Cys Lys Asn
           55                  60                  65
   ggg cta ata cga acg gga ctg tca ggt aaa tcg cgt tta acg ata cca      714
   Gly Leu Ile Arg Thr Gly Leu Ser Gly Lys Ser Arg Leu Thr Ile Pro
       70                  75                  80
   ata atc ggt aca ttg acg acg tta cgc gtg gct ttt aaa ttt tcg atc      762
   Ile Ile Gly Thr Leu Thr Thr Leu Arg Val Ala Phe Lys Phe Ser Ile
   85                  90                  95                  100
   cct tca ata cgt aac ccg gca gca cca ccg ata acg gat gct tgc gcc      810
   Pro Ser Ile Arg Asn Pro Ala Ala Pro Pro Ile Thr Asp Ala Cys Ala
                   105                 110                 115
   atg gcg gca aca att tct ggc gag tcc att ggc cca tta tct acg ggc      858
   Met Ala Ala Thr Ile Ser Gly Glu Ser Ile Gly Pro Leu Ser Thr Gly
               120                 125                 130
   tgg caa gat gcg att aag cca tat tta att tgt tct aaa act tgc gga      906
   Trp Gln Asp Ala Ile Lys Pro Tyr Leu Ile Cys Ser Lys Thr Cys Gly
           135                 140                 145
   tgt gat agt ttt gac ata tta act cca gtc taaatttatc aaaagaagat        956
   Cys Asp Ser Phe Asp Ile Leu Thr Pro Val
       150                 155
   tgactccaat ttgcataggt taatcttaga attaaaaaat aacaaccaaa ataataaaaa   1016
   tttgagatct ttgtcgcata tttattcata gggaatagac agcttaattt tagttatgat   1076
   ttgtcaatcc ttgctatttt ttgtgtttgc tggtttgcga tacactgttc taatattgct   1136
   ttgagcactt gataaccttg ctcattaaaa tgtaatccgt cggtacaaag gcgtaaatcc   1196
   agttcaccgt tagaatcaca aaagtatttt tgtgtttcaa cgtaagtcac gtctgacgga   1256
   caatgttgtt ttaaataggt attgagcctg tgaatttgtg cgttagtgac cgtattaatc   1316
   tgattgaccg gtgtggcttc taataaaaag tagtgggacg taggagaaat ggtgtgtagg   1376
   tgagtcagaa tgtcatttaa ctatcgcatg acttgcgccg gtgaatacgt ttcttcctta   1436
   caaatatcat tgacgcctaa aaaaagaaaa acagattgtc caagttgttg aatccgttta   1496
   ggtttaacga taacatccaa atattgtcgc gtactgacgc cagaaagtcc taaattggcg   1556
   acggtttgtc ccgctaattg aggtgtgcct gctacctgtt cgtcccacat gtcaaaaagt   1616
   gaatgaccaa ttaagctgat attggcaggt ttggaaaatt ccgccatttt gctctgatag   1676
   cgttgataaa tatcctgatc acttagcatg tgtggacctc tattttgaaa taaaacgcta   1736
   agtattatat aaaacctgat atgccggtaa acagtaaact tatcttccgt aggggtaaat   1796
   attcaatttt gtgacgaacc tatcatttat gaaataaaac ttcattttct atataaaaaa   1856
   tagttttttc actttagaat gccaaacgtg tgaaatttat ttcatcatca ttttaacgta    1916
   atcccaacgt aaccaataga ggagaactca taatgaaatt taaaaaacta ctacttgcat   1976
   ctttatgttt aggtgtttca gcttctgtat ttgcagcaga ttacgatctt aaattcggta   2036
   tggttgcggg tccaagctca aacgaatata aagcagtaga attctttgcg aaagaagtga   2096
   aagaaaaatc caatggcaaa attgatgtgg ctattttccc tagctcacag ttaggtgatg   2156
   accgtgtgat gattaaacaa ttaaaagacg gtgcattaga ctttacgtta ggtgaatcag   2216
   cacgtttcca aatttacttc ccagaagcag aagtatttgc gttgccttat atgattccta   2276
   attttgaaac ctctaaaaaa gcgttgctcg acacaaaatt tggtcaaggt ttattgaaaa   2336
   aaattgataa agagttaaac gtacaagtgt tatctgtggc gtataacggt acacgtcaaa   2396
   caacttctaa ccgtgcaatc aacagcattg aagacatgaa agggttaaaa ttacgtgtac   2456
   ctaacgcggc aaccaacctt gcttatgcaa aatacgtggg tgcagcgcca acaccaatgg   2516
   cattctctga agtttacctt gcgcttcaaa caaactctgt ggatggtcaa gaaaacccat   2576
   taccgacaat ccaagcacaa aaattctatg aagtacaaaa atacttagcg ttaactaacc   2636
   acatcttaaa tgaccaactt tacttaatca gtaacgatac gttggcagat ttaccagaag   2696
   atttacaaaa agtggttaaa gatgcagcag cgaaagccgc tgaatatcac actaaactct   2756
   tcgttgacgg tgagaacagc ttagttgaat t                                  2787
<210> 75
   <211> 158
   <212> PRT
   <213> Pasteurella Multocida
<400> 75
   Met Val Leu Pro Ile Ile Ser Thr Pro Lys Leu Trp Gln Tyr Ile Pro
   1               5                   10                  15
   Ser Ser Lys Leu Glu Gln Ser Ala Met Ala Lys Gln Pro Asn Ser Leu
               20                  25                  30
   Ile Arg Leu Ile Met Ala Ser Arg Val Val Gly Arg Thr Arg Ser Val
           35                  40                  45
   Pro Ser Lys Ala Ile Ile Ser Ala Pro Ala Ala Ala Asn Ser Ser Met
       50                  55                  60
   Ser Cys Lys Asn Gly Leu Ile Arg Thr Gly Leu Ser Gly Lys Ser Arg
   65 70 75 80
   Leu Thr Ile Pro Ile Ile Gly Thr Leu Thr Thr Leu Arg Val Ala Phe
   85 90 95
   Lys Phe Ser Ile Pro Ser Ile Arg Asn Pro Ala Ala Pro Pro Ile Thr
   100 105 110
   Asp Ala Cys Ala Met Ala Ala Thr Ile Ser Gly Glu Ser Ile Gly Pro
   115 120 125
   Leu Ser Thr Gly Trp Gln Asp Ala Ile Lys Pro Tyr Leu Ile Cys Ser
   130 135 140
   Lys Thr Cys Gly Cys Asp Ser Phe Asp Ile Leu Thr Pro Val
   145 150 155
<210> 76
   <211> 2787
   <212> DNA
   <213> Pasteurella Multocida
<220>
   <221> misc_feature
   <223> yiaO
<220>
   <221> CDS
   <222> (1949)..(2785)
<400> 76
   gttaacacac catgattaat gatgccggtt gaagccactg caacgtaatc gaattgtccg 60
   gcatacaaag caagaatgtt ggccagtgtg tcatgcatcg cattggcagc atcagcttgt 120
   ggcgttgcaa tctgttggcg ttgttctatt ttgccgtctg ttacaatagc cgaggcaatt 180
   tttgttccac caatatctaa tgctaaacag cgcataggct ctccttctgt gatgacttat 240
   tttgccgatt tgacggcatc ggcaaaccag cttacgatat gttcgaggcg agtcagcgca 300
   gatcctacgg tgacagagta agcaccaatc tcaattgcgg ttttcgccaa ttctggggtg 360
   ttatagcgcc cttctgccat cactcggcag ccagcagcat tcaaatcttt gactaactga 420
   taatccggtt cagctggaat ttcaccgcca gtataaccag acatggtgct accaataatt 480
   tctaccccta agttgtggca atacatccct tcttcaaaat tagaacaatc cgccatggct 540
   aaacaaccta attctttgat tcgtttaata atggcttcac gtgtagttgg acggacgcga 600
   tcggtaccat caaaagcaat aatatcggcg cctgctgcgg ctaactcttc aatgtcttgt 660
   aaaaatgggc taatacgaac gggactgtca ggtaaatcgc gtttaacgat accaataatc     720
   ggtacattga cgacgttacg cgtggctttt aaattttcga tcccttcaat acgtaacccg     780
   gcagcaccac cgataacgga tgcttgcgcc atggcggcaa caatttctgg cgagtccatt     840
   ggcccattat ctacgggctg gcaagatgcg attaagccat atttaatttg ttctaaaact     900
   tgcggatgtg atagttttga catattaact ccagtctaaa tttatcaaaa gaagattgac     960
   tccaatttgc ataggttaat cttagaatta aaaaataaca accaaaataa taaaaatttg    1020
   agatctttgt cgcatattta ttcataggga atagacagct taattttagt tatgatttgt    1080
   caatccttgc tattttttgt gtttgctggt ttgcgataca ctgttctaat attgctttga     1140
   gcacttgata accttgctca ttaaaatgta atccgtcggt acaaaggcgt aaatccagtt    1200
   caccgttaga atcacaaaag tatttttgtg tttcaacgta agtcacgtct gacggacaat    1260
   gttgttttaa ataggtattg agcctgtgaa tttgtgcgtt agtgaccgta ttaatctgat    1320
   tgaccggtgt ggcttctaat aaaaagtagt gggacgtagg agaaatggtg tgtaggtgag    1380
   tcagaatgtc atttaactat cgcatgactt gcgccggtga atacgtttct tccttacaaa    1440
   tatcattgac gcctaaaaaa agaaaaacag attgtccaag ttgttgaatc cgtttaggtt    1500
   taacgataac atccaaatat tgtcgcgtac tgacgccaga aagtcctaaa ttggcgacgg    1560
   tttgtcccgc taattgaggt gtgcctgcta cctgttcgtc ccacatgtca aaaagtgaat    1620
   gaccaattaa gctgatattg gcaggtttgg aaaattccgc cattttgctc tgatagcgtt    1680
   gataaatatc ctgatcactt agcatgtgtg gacctctatt ttgaaataaa acgctaagta    1740
   ttatataaaa cctgatatgc cggtaaacag taaacttatc ttccgtaggg gtaaatattc    1800
   aattttgtga cgaacctatc atttatgaaa taaaacttca ttttctatat aaaaaatagt    1860
   tttttcactt tagaatgcca aacgtgtgaa atttatttca tcatcatttt aacgtaatcc    1920
   caacgtaacc aatagaggag aactcata atg aaa ttt aaa aaa cta cta ctt     1972
                                   Met Lys Phe Lys Lys Leu Leu Leu
                                   1               5
   gca tct tta tgt tta ggt gtt tca gct tct gta ttt gca gca gat tac    2020
   Ala Ser Leu Cys Leu Gly Val Ser Ala Ser Val Phe Ala Ala Asp Tyr
       10                  15                  20
   gat ctt aaa ttc ggt atg gtt gcg ggt cca agc tca aac gaa tat aaa    2068
   Asp Leu Lys Phe Gly Met Val Ala Gly Pro Ser Ser Asn Glu Tyr Lys
   25                  30                  35                  40
   gca gta gaa ttc ttt gcg aaa gaa gtg aaa gaa aaa tcc aat ggc aaa    2116
   Ala Val Glu Phe Phe Ala Lys Glu Val Lys Glu Lys Ser Asn Gly Lys
                   45                  50                  55
   att gat gtg gct att ttc cct agc tca cag tta ggt gat gac cgt gtg    2164
   Ile Asp Val Ala Ile Phe Pro Ser Ser Gln Leu Gly Asp Asp Arg Val
               60                  65                  70
   atg att aaa caa tta aaa gac ggt gca tta gac ttt acg tta ggt gaa      2212
   Met Ile Lys Gln Leu Lys Asp Gly Ala Leu Asp Phe Thr Leu Gly Glu
           75                  80                  85
   tca gca cgt ttc caa att tac ttc cca gaa gca gaa gta ttt gcg ttg      2260
   Ser Ala Arg Phe Gln Ile Tyr Phe Pro Glu Ala Glu Val Phe Ala Leu
       90                  95                  100
   cct tat atg att cct aat ttt gaa acc tct aaa aaa gcg ttg ctc gac      2308
   Pro Tyr Met Ile Pro Asn Phe Glu Thr Ser Lys Lys Ala Leu Leu Asp
   105                 110                 115                 120
   aca aaa ttt ggt caa ggt tta ttg aaa aaa att gat aaa gag tta aac      2356
   Thr Lys Phe Gly Gln Gly Leu Leu Lys Lys Ile Asp Lys Glu Leu Asn
                   125                 130                 135
   gta caa gtg tta tct gtg gcg tat aac ggt aca cgt caa aca act tct      2404
   Val Gln Val Leu Ser Val Ala Tyr Asn Gly Thr Arg Gln Thr Thr Ser
               140                 145                 150
   aac cgt gca atc aac agc att gaa gac atg aaa ggg tta aaa tta cgt      2452
   Asn Arg Ala Ile Asn Ser Ile Glu Asp Met Lys Gly Leu Lys Leu Arg
           155                 160                 165
   gta cct aac gcg gca acc aac ctt gct tat gca aaa tac gtg ggt gca      2500
   Val Pro Asn Ala Ala Thr Asn Leu Ala Tyr Ala Lys Tyr Val Gly Ala
       170                 175                 180
   gcg cca aca cca atg gca ttc tct gaa gtt tac ctt gcg ctt caa aca      2548
   Ala Pro Thr Pro Met Ala Phe Ser Glu Val Tyr Leu Ala Leu Gln Thr
   185                 190                 195                 200
   aac tct gtg gat ggt caa gaa aac cca tta ccg aca atc caa gca caa      2596
   Asn Ser Val Asp Gly Gln Glu Asn Pro Leu Pro Thr Ile Gln Ala Gln
                   205                 210                 215
   aaa ttc tat gaa gta caa aaa tac tta gcg tta act aac cac atc tta      2644
   Lys Phe Tyr Glu Val Gln Lys Tyr Leu Ala Leu Thr Asn His Ile Leu
               220                 225                 230
   aat gac caa ctt tac tta atc agt aac gat acg ttg gca gat tta cca      2692
   Asn Asp Gln Leu Tyr Leu Ile Ser Asn Asp Thr Leu Ala Asp Leu Pro
           235                 240                 245
   gaa gat tta caa aaa gtg gtt aaa gat gca gca gcg aaa gcc gct gaa      2740
   Glu Asp Leu Gln Lys Val Val Lys Asp Ala Ala Ala Lys Ala Ala Glu
       250                 255                 260
   tat cac act aaa ctc ttc gtt gac ggt gag aac agc tta gtt gaa tt       2787
   Tyr His Thr Lys Leu Phe Val Asp Gly Glu Asn Ser Leu Val Glu
   265                 270                 275
<210> 77
   <211> 279
   <212> PRT
   <213> Pasteurella Multocida
<400> 77
   Met Lys Phe Lys Lys Leu Leu Leu Ala Ser Leu Cys Leu Gly Val Ser
   1               5                   10                  15
   Ala Ser Val Phe Ala Ala Asp Tyr Asp Leu Lys Phe Gly Met Val Ala
               20                  25                  30
   Gly Pro Ser Ser Asn Glu Tyr Lys Ala Val Glu Phe Phe Ala Lys Glu
           35                  40                  45
   Val Lys Glu Lys Ser Asn Gly Lys Ile Asp Val Ala Ile Phe Pro Ser
       50                  55                  60
   Ser Gln Leu Gly Asp Asp Arg Val Met Ile Lys Gln Leu Lys Asp Gly
   65                  70                  75                  80
   Ala Leu Asp Phe Thr Leu Gly Glu Ser Ala Arg Phe Gln Ile Tyr Phe
                   85                  90                  95
   Pro Glu Ala Glu Val Phe Ala Leu Pro Tyr Met Ile Pro Asn Phe Glu
               100                 105                 110
   Thr Ser Lys Lys Ala Leu Leu Asp Thr Lys Phe Gly Gln Gly Leu Leu
           115                 120                 125
   Lys Lys Ile Asp Lys Glu Leu Asn Val Gln Val Leu Ser Val Ala Tyr
       130                 135                 140
   Asn Gly Thr Arg Gln Thr Thr Ser Asn Arg Ala Ile Asn Ser Ile Glu
   145                 150                 155                 160
   Asp Met Lys Gly Leu Lys Leu Arg Val Pro Asn Ala Ala Thr Asn Leu
                   165                 170                 175
   Ala Tyr Ala Lys Tyr Val Gly Ala Ala Pro Thr Pro Met Ala Phe Ser
               180                 185                 190
   Glu Val Tyr Leu Ala Leu Gln Thr Asn Ser Val Asp Gly Gln Glu Asn
           195                 200                 205
   Pro Leu Pro Thr Ile Gln Ala Gln Lys Phe Tyr Glu Val Gln Lys Tyr
       210                 215                 220
   Leu Ala Leu Thr Asn His Ile Leu Asn Asp Gln Leu Tyr Leu Ile Ser
   225                 230                 235                 240
   Asn Asp Thr Leu Ala Asp Leu Pro Glu Asp Leu Gln Lys Val Val Lys
                   245                 250                 255
   Asp Ala Ala Ala Lys Ala Ala Glu Tyr His Thr Lys Leu Phe Val Asp
               260                 265                 270
   Gly Glu Asn Ser Leu Val Glu
           275
<210> 78
   <211> 2590
   <212> DNA
   <213> Pasteurella Multocida
<220>
   <221> misc_feature
   <223> yigF
<220>
   <221> CDS
   <222> (908)..(1294)
<400> 78
   ctgcaggctc gattagtggg gcaccgaaag aaaaaaccgt gcaaattatt cacgccgcag      60
   aacagcagcc acgcggttat tacacgggga tttttggtct gttcgatggt gagtcgttac     120
   aaagtgcggt ggcaattcgt tttattgagc aagtggacga gaaattgatt ttccgcagcg     180
   gtggcgggat tacgatctta agcgagctag aagacgagta ccaagaattg atccaaaaag     240
   tgtatgtacc agtaggataa gcgatgacat ttcctttatt tgagacgatc gctattgtga     300
   acggtgaaat tcagcacctt gccctgcatc aacaacgtta tgcggcaagt ttggcgacct     360
   tttacggcga gaaaggagcg aaagtacagg atcttgcgaa aattattcag attccgaccg     420
   cacttgaaca cactcaacat gcgccgataa tccgttgtcg gattgattac aatcagcaag     480
   actgtgacgt gcattatttt ccctatcaac gcaaaattta ccgcactttt cagcctgtca     540
   tttgcgatga aattaactat gatctgaaat atgctgatcg ggcattatta aatcagttat     600
   ttgctcagcg tagggattgt gatgagatta tgattatcaa acacggcaag gtgacggatt     660
   gcagtattgg taatctggtg tttcgccaag gtgagcaatg gttcacgcca gatagcccgt     720
   tattttacgg cacacaacga gcctggttat tacaacaagg caaaattcaa gcccgttcca     780
   tcttattgca agagatcgca caatttgaag aaattcggtt aattaatgca ctaaatccgc     840
   tgtaaatttt ccttgaacag cgtaaaataa aacaactttt tcagtcagat aaaaggagat     900
   aaacgac atg acg aaa gta att cat act gac aat gca cca gcc gcc att       949
           Met Thr Lys Val Ile His Thr Asp Asn Ala Pro Ala Ala Ile
           1               5                   10
   ggt cct tat gta caa gcg gta gat tta ggt aat atg ctg tta acc tct      997
   Gly Pro Tyr Val Gln Ala Val Asp Leu Gly Asn Met Leu Leu Thr Ser
   15                  20                  25                  30
   ggg caa att cca gtg aat cca aaa acc ggt gaa gtg cca gcg gat atc     1045
   Gly Gln Ile Pro Val Asn Pro Lys Thr Gly Glu Val Pro Ala Asp Ile
                   35                  40                  45
   gta gca caa gca cgt caa tcg tta gaa aac gtg aaa gcg att gtg gaa     1093
   Val Ala Gln Ala Arg Gln Ser Leu Glu Asn Val Lys Ala Ile Val Glu
               50                  55                  60
   caa gcg gga tta caa gtc gca aat atc gtg aaa acc acg gtg ttt gtg     1141
   Gln Ala Gly Leu Gln Val Ala Asn Ile Val Lys Thr Thr Val Phe Val
           65                  70                  75
   aaa gat tta aat gac ttt gca gcg gtc aat gcg gag tat gaa cgt ttc     1189
   Lys Asp Leu Asn Asp Phe Ala Ala Val Asn Ala Glu Tyr Glu Arg Phe
       80                  85                  90
   ttt aaa gag aac aat cac cct agc ttc cct gct cgt tca tgt gtg gaa     1237
   Phe Lys Glu Asn Asn His Pro Ser Phe Pro Ala Arg Ser Cys Val Glu
   95                  100                 105                 110
   gtg gca cgt ttg ccg aaa gat gtg ggg att gaa atc gag gca atc gct     1285
   Val Ala Arg Leu Pro Lys Asp Val Gly Ile Glu Ile Glu Ala Ile Ala
                   115                 120                 125
   gta aaa gcc taatgaatag cttgcattta tcttagtcgt agcaaaacaa             1334
   Val Lys Ala
   tctcttttca cttgctctct tcaaagcaag ttgataagtg atttttattg ggcgtttttc   1394
   tattgatagc caaaaacgcc ctttactgat agagaataaa ctatgcaaaa tcaagtcatc   1454
   gagattctac aataccgttt aaaaccacaa tcaggacaaa cgtttcacca aattatgcgt   1514
   gagatcagtg ttccactcca taaacaacat gggattgatg tcattgcgta tggaaattca   1574
   ttacatgata ttgacagcta ttatttaatc cgtgcatttg agacagaaac caaattgcaa   1634
   cagcagctcg atgcttttta tgccagtgat gattggcgtg atggaccaag agaaagtatc   1694
   attcgcctga ttgaaagcag tttaaaatcg gtgatcatgc tcccgacaca ggcaatccat   1754
   gcactacgca accattatcc tcaataaaat caacaaccgc acccaatcag tgcggtcatt   1814
   ttttcttact ttttcagtgc taagggaaaa acaacgatag tggacgttgt ttaatcaatt   1874
   tccaaacaca ttgcgcgata tcacaccaac tctcaatttc tgtttctaaa gaacgcagcg   1934
   caacccataa cgcgataaag aaactgacaa tcaaattcac cataccaatc aataacacga   1994
   acactaaacc ttgtaagaac atctgccaag taaacgcgcc actgatcgcc atatagccca   2054
   aattcgcaga agaaaacgcc acatggcgaa tatctaacgg taaattaagc aaatacccga   2114
   ctaaccccgt taaaccaagc aataaaccaa aacacagatt tcccataatc gaaccgtaat   2174
   tatcatgcca gtattctgca aatttgtagc gcatattacg ggtcaacaga cggcgtaaaa   2234
   tagggtgatt tcttagtcgc atttttaagt tcaaataatt actacgatta tcaaaataac   2294
   cagaaataat cccagaaaag aataaccaga aacccgcaat ggcggcaaac cataaggacc   2354
   ctttcatcgg atcaagggat ttttgttggt aggcaatctc cgcgtcactc aataaaggtg   2414
   taccaacata atgttgatag cctagcgcaa gcaaacaagc cacagaaatc gctaaagtga   2474
   cattacccaa gactgccact gtttgcgagc gaaacacatc gatcagcagt tgagctaatt   2534
   gcagattaac ggatttgcct tgtccattat ccaccgtttc tgcaaaacgt gctgca       2590
<210> 79
   <211> 129
   <212> PRT
   <213> Pasteurella Multocida
<400> 79
   Met Thr Lys Val Ile His Thr Asp Asn Ala Pro Ala Ala Ile Gly Pro
   1               5                   10                  15
   Tyr Val Gln Ala Val Asp Leu Gly Asn Met Leu Leu Thr Ser Gly Gln
               20                  25                  30
   Ile Pro Val Asn Pro Lys Thr Gly Glu Val Pro Ala Asp Ile Val Ala
           35                  40                  45
   Gln Ala Arg Gln Ser Leu Glu Asn Val Lys Ala Ile Val Glu Gln Ala
       50                  55                  60
   Gly Leu Gln Val Ala Asn Ile Val Lys Thr Thr Val Phe Val Lys Asp
   65                  70                  75                  80
   Leu Asn Asp Phe Ala Ala Val Asn Ala Glu Tyr Glu Arg Phe Phe Lys
                   85                  90                  95
   Glu Asn Asn His Pro Ser Phe Pro Ala Arg Ser Cys Val Glu Val Ala
               100                 105                 110
   Arg Leu Pro Lys Asp Val Gly Ile Glu Ile Glu Ala Ile Ala Val Lys
           115                 120                 125
   Ala
<210> 80
   <211> 6642
   <212> DNA
   <213> Pasteurella Multocida
<220>
   <221> misc_feature
   <223> yleA
<220>
   <221> CDS
   <222> (463)..(1884)
<400> 80
   aacccagctc agataagttt aattctggct atggaaagtg gaccaaatcc ttcgatctcc      60
   caggtttcta aggcgcgata cgggcaaata cgagtatttt cgatatactg ccaagcacct     120
   agttgtttta agagattaac ggaagcaaca ctgatcgcag aaatgcgaag atcgtaaggc     180
   gaattaggtt gaaactgtgg gagtggggcg tgttcgataa gctggacttg atgtccttgt     240
   ttacgtaagc caagcgcaca ggctgcacca accatgccac cgccaaccac gatcatgtct     300
   ttttgtatga cgtccatagg attttccttt tctttttgtt acgtattcta ccgtcaatga     360
   gggaatttca aaagaaatct cttttttagc tagccagcat aggttcaaga ctgtaaaata     420
   gtcagtcaca tttttatagg ttaactgaat tttttaaacg at atg acg caa aaa       474
                                                  Met Thr Gln Lys
                                                  1
   tta cat att aaa acg tgg ggt tgt cag atg aat gaa tat gat tca tct       522
   Leu His Ile Lys Thr Trp Gly Cys Gln Met Asn Glu Tyr Asp Ser Ser
   5                   10                  15                  20
   aaa atg gca gat ctc tta aac agt act cac ggc tta gag tta aca gaa       570
   Lys Met Ala Asp Leu Leu Asn Ser Thr His Gly Leu Glu Leu Thr Glu
                   25                  30                  35
   att ccg gaa gaa gcg gat gtg tta ttg tta aac act tgc tca att cgt       618
   Ile Pro Glu Glu Ala Asp Val Leu Leu Leu Asn Thr Cys Ser Ile Arg
               40                  45                  50
   gaa aaa gca caa gaa aaa gtt ttc cat caa tta gga cgt tgg aaa gaa       666
   Glu Lys Ala Gln Glu Lys Val Phe His Gln Leu Gly Arg Trp Lys Glu
           55                  60                  65
   tta aag aaa cat aag ccg gga ctc gtt atc ggt gtt ggg ggc tgt gtt       714
   Leu Lys Lys His Lys Pro Gly Leu Val Ile Gly Val Gly Gly Cys Val
       70                  75                  80
   gcc tca caa gaa gga gaa cac att cgt act cgt gct cct tat gtc gat       762
   Ala Ser Gln Glu Gly Glu His Ile Arg Thr Arg Ala Pro Tyr Val Asp
   85                  90                  95                  100
   att att ttt gga cca caa acc tta cat cgt tta cct gaa atg atc aat       810
   Ile Ile Phe Gly Pro Gln Thr Leu His Arg Leu Pro Glu Met Ile Asn
                   105                 110                 115
   cag atc aga ggt ggt aaa agc tca gta gtc gat gtc agt ttt cca gaa       858
   Gln Ile Arg Gly Gly Lys Ser Ser Val Val Asp Val Ser Phe Pro Glu
               120                 125                 130
   att gaa aaa ttc gac cgt tta cca gaa ccg cgt gca gaa ggt cca act      906
   Ile Glu Lys Phe Asp Arg Leu Pro Glu Pro Arg Ala Glu Gly Pro Thr
           135                 140                 145
   gct ttc gta tcc att atg gaa ggc tgt aat aaa tat tgc tca ttc tgt      954
   Ala Phe Val Ser Ile Met Glu Gly Cys Asn Lys Tyr Cys Ser Phe Cys
       150                 155                 160
   gtc gtg cct tat acg cgt ggt gaa gaa gtc agt cgt cca gtg gat gat      1002
   Val Val Pro Tyr Thr Arg Gly Glu Glu Val Ser Arg Pro Val Asp Asp
   165                 170                 175                 180
   gta tta ttt gaa att gca cag ttg gca gag caa ggc gtg cgt gaa gtg      1050
   Val Leu Phe Glu Ile Ala Gln Leu Ala Glu Gln Gly Val Arg Glu Val
                   185                 190                 195
   aat tta tta gga caa aac gtg aac gct tat cgt ggt gca act cat gat      1098
   Asn Leu Leu Gly Gln Asn Val Asn Ala Tyr Arg Gly Ala Thr His Asp
               200                 205                 210
   gac ggt att tgt act ttt gcg gaa ttg tta cgt tta gta gcc gct att      1146
   Asp Gly Ile Cys Thr Phe Ala Glu Leu Leu Arg Leu Val Ala Ala Ile
           215                 220                 225
   gat ggt att gac cgt tta cgt ttt acc acc agt cac cca att gag ttc      1194
   Asp Gly Ile Asp Arg Leu Arg Phe Thr Thr Ser His Pro Ile Glu Phe
       230                 235                 240
   act gat gac att att gat gtg tac cgt gat acg cca gag ttg gtg agt      1242
   Thr Asp Asp Ile Ile Asp Val Tyr Arg Asp Thr Pro Glu Leu Val Ser
   245                 250                 255                 260
   ttc tta cac tta cct gta caa agt ggt tct gat cgt gtg tta tct atg      1290
   Phe Leu His Leu Pro Val Gln Ser Gly Ser Asp Arg Val Leu Ser Met
                   265                 270                 275
   atg aaa cgc aat cat acg gca tta gaa tat aaa tcg att att cgg aag      1338
   Met Lys Arg Asn His Thr Ala Leu Glu Tyr Lys Ser Ile Ile Arg Lys
               280                 285                 290
   tta aga gcg gtg cgt cca gag att caa att agc tca gat ttt att gtc      1386
   Leu Arg Ala Val Arg Pro Glu Ile Gln Ile Ser Ser Asp Phe Ile Val
           295                 300                 305
   ggt ttc ccg ggc gaa aca gca gaa gat ttc gag caa acc atg aat tta      1434
   Gly Phe Pro Gly Glu Thr Ala Glu Asp Phe Glu Gln Thr Met Asn Leu
       310                 315                 320
   att gca caa gta aat ttt gat atg agt ttc agc ttc att tat tca gca      1482
   Ile Ala Gln Val Asn Phe Asp Met Ser Phe Ser Phe Ile Tyr Ser Ala
   325                 330                 335                 340
   cgt cca ggc acg cca gca gca gat atg cct gat gat gtg aca gaa gaa      1530
   Arg Pro Gly Thr Pro Ala Ala Asp Met Pro Asp Asp Val Thr Glu Glu
                   345                 350                 355
   gag aag aaa caa cgt tta tat gtg ttg caa caa cgc att aac aat caa      1578
   Glu Lys Lys Gln Arg Leu Tyr Val Leu Gln Gln Arg Ile Asn Asn Gln
               360                 365                 370
   gcc gcg caa ttt agt cga gca atg tta ggc aca gaa cag cgc gtg tta      1626
   Ala Ala Gln Phe Ser Arg Ala Met Leu Gly Thr Glu Gln Arg Val Leu
           375                 380                 385
   gtg gaa gga ccc tcg aaa aaa gat tta atg gaa ctc aca ggg cgt aca      1674
   Val Glu Gly Pro Ser Lys Lys Asp Leu Met Glu Leu Thr Gly Arg Thr
       390                 395                 400
   gaa act aat cgt atc gtc aat ttt gtg ggc acg cct gat atg att ggg      1722
   Glu Thr Asn Arg Ile Val Asn Phe Val Gly Thr Pro Asp Met Ile Gly
   405                 410                 415                 420
   aag ttt gtt gat atc aag atc acg gat gtg ttt act aac tca ctg cgt      1770
   Lys Phe Val Asp Ile Lys Ile Thr Asp Val Phe Thr Asn Ser Leu Arg
                   425                 430                 435
   ggt gaa gtc gtt aga act gaa gaa caa atg gga ctt cgc gtt gtt caa      1818
   Gly Glu Val Val Arg Thr Glu Glu Gln Met Gly Leu Arg Val Val Gln
               440                 445                 450
   tcg cca caa atg gtg att aat cgt act cgt aaa gaa gat gaa ctc ggc      1866
   Ser Pro Gln Met Val Ile Asn Arg Thr Arg Lys Glu Asp Glu Leu Gly
           455                 460                 465
   gtg gga cgt tat cac gcg tagtcgtgct atcccttcaa atatttaacc             1914
   Val Gly Arg Tyr His Ala
       470
   gctctcgagt ttctcaagag cggttatttt ttatgaaaaa tttttgataa attgaccgct   1974
   ctttttattg cttcatttta tgatagacag cgtgttttct gttattcatc gtatttcttt  2034
   ttatttcact tcattaataa attattaaat ttcaattgct tatcaaaatt gttgtttttt   2094
   gctttttttc tatttatagc atggttattt tttatacaca catggcgtat ttctccatat   2154
   ttttacaaaa aactgtgact tcactctaac tattgttctc tcgtgcttta ctccatttta   2214
   taaggcggtt agtttagatc atgttgtttt tacaacatat tttgaggttg tttgaaggtt   2274
   gagcttattt tatagttgag gtgatgatga aaaaatttaa tcctagtata ttggcgttgt   2334
   ccatatcaag tctgctactc acatccacat tgacctttgg tcaaatccag caacaagata   2394
   aagcactctt tggtgtgaaa gaacatcaag aaagcctact ctttcatcag agcttagtag   2454
   aacaaggtag tgataatgtg ccaatttggc gcattccgtc tttattaaga acaaaagacg   2514
   gtgtattaat tgccgccgca gataagcgtt ggcaacaccg tggtgactgg ggcgatattg   2574
   ataccgcaat ccgaattagt catgatgatg ggaaaacatg gggaaatatt acaacgattt   2634
   tggatttgcc atcaaagaat ggggaaaaat ccccatcagc accagatcct gtaacattca   2694
   acgcgtgggg agatagacaa aattgtacaa cttactgtaa ttctgctttt ttgatcgatg   2754
   ctcagatggt acaagataaa cgtaatggta gaattttttt agcagttgat atgtttgccg   2814
   atggagcagg tttttttggt gtaaaagaca gtggtaatgg gcgcattaat attgatggta   2874
   aacagtatcc tattttaaat gaaaagcaat cgggtcgaca ttgaaccctg tgttcttaac   2934
   tctttcactt gctcaacgtt caccggaatc acgcggtcaa taaaagtcgg tttccagttt 2994
   aaacccggtt gaacaatagc gtgtaattca ccaaaacgca tcaccacgcc acgttctgct 3054
   tctttgacag tataaaaacc gctaacgccc cagacaattg caccaatcac cgctgcaatt 3114
   ggcaataatt taccaagatt taacgatcca ccagaatgcg agttattgcg tttaccgcca 3174
   ttccctgaac cgcctaattt tttcagcaga ttattaaaaa tctcttcaat atcaggtggt 3234
   gattgctctt gattgttttg cttacgacct gactggttat cattccaatt cggctgaccg 3294
   ccttcattat caggacgctg actctcaggt tttttctgtc cgggtttgcc ccaaggatct 3354
   tgatctgaac cgttcaatga cattacgttc tccatttgtc aaaaaattgt tacttgttta 3414
   aacaaattcc cacagtctaa acaaaatgtt ggattaagtc taacgaaact cgttactaaa 3474
   tggggatatt ttttatgatt taaagtgccg ccaataaaaa cgcacaatgg tctcattttt 3534
   acggcttact acctgacttt tgtcctatcg ctttgtcggt catgtttttt cattcacctt 3594
   ataataaatg gcgtcgtgta aggtggtttc cgctaatatt cccctctgcc aagtaaaata 3654
   gtgcacatct aagcactgtg tttggttaat aaaaaaagcc aagtaaaacg gcaagcggtg 3714
   actaaagagg agttaactaa gtttctgtat gctagcgtac tattgactct tcttgcactg 3774
   tgtttgggtt ttattgcggt gaatgaaacc ctagcgtgta tttctttgtt attgttggct 3834
   tatttaagcg ttagccgagt attatttgcg ctcgcgttgt tgggcgtgat catgaatttg 3894
   agctattact attatctctt gagtatccct ttattacata aatctttctt actcatggga 3954
   gtaggcatcg ttctggcact agttacgttt gttttatcac gttataacaa ggcaccgcag 4014
   gcaacattac aagctgaatc gcacaatacg tttcaaccac aaacaatgtt acgtaaaaaa 4074
   ctaggattca ctttgttggc gacatgtttg attgcctttg ccacgaatta tactattcat 4134
   aaatatgaag atattttaac gaatggtgag tcaattattt taaaaacggc acccgttgat 4194
   ccgcgttcct tgatgcaagg ggattatatg acgttgaact atgaaatctt ggcagacatt 4254
   agtgaggaat ggggaaaaaa tttagaagag gaaaaaacac agtattttgt ttatgcgttg 4314
   ttaaaacgag acagcctagg gattgctacc ttgtgtcgtc ttgaaaccaa agcacctaca 4374
   acatttgacg ggtgtacacc aaatatttac ttaccggtga atgtcgcgat gtggtggccg 4434
   cgtttaccaa gtcaagacta cttttttgcc gagggaaaag gtgaatatta tgcacaagct 4494
   gaatatgccg aatatcgctt taaaggggga aaagcgttgt tgttccgctt actagataaa 4554
   aatttaaaag cattataaaa caaaaggcgg tagtgaaccg cctttatgtt gtatgccact 4614
   tacttcaccg caatcattga gccaaaatta aagcattgaa accataattc cacttgttga 4674
   aaaccgactt cagctaaacg cattttatgg gcttgaatgc tgtctgtacg catcacattt 4734
   tcaagtgcgg tgcgtttttg gctgacttca agttcactgt atccattcgc gcgtttaaat 4794
   tgatgatgta aatcaatcaa caaggcattc atgttttgat cttcaaaatg aaatttctcg 4854
   gatagtacca agatcccatt cggttgtaag ccttggtaaa tcttacttaa taaggctcga 4914
   cgatcttcag gtggcagaaa ttgcaaagtg aaatttaaaa ttaccatgaa ggcattttcg 4974
   atttcgatat ggcgtatatc atcacaaata atgtccacag gtattgtact ttgataggca 5034
   ctaacatgct ggcgacaacg atcgaccatg ggttgtgagt tatcaacacc aatgatcttg 5094
   acgccgggtt gattaatatg acgacgtgca gataatgttg ccgcaccgcg cgagcacccc 5154
   agatcataga cgttgctatt cgccgtaaca aagcgtgatg ccaacatccc aatagcggta 5214
   atgatgttgg catagccggg aatagagcgt tggatcatgt cgggaaagac ttcagctacg 5274
   ctttcatcaa aagtaaaatc ccccaattta gcaatgggga gtcgaaaata gagtatcttt 5334
   tgtcataatg tattaaagac cgagaaaaag tgcggtcatt ttagaagaag tttcgaccta 5394
   tcacaaataa attatgattt cgggaataaa tgcgccatgg cactttcttc ttgtttaata 5454
   cgcacgccta ggataagcaa ataaatgggt aatccaatca atgcggtata ttttgcttgg 5514
   cagaaaagcg ataaaccaat taactcggga ataatgttta agaaataatt cgggtgacgt 5574
   acgtatttaa acaagaaaga gcggttaatg tgatgttccg gtaaaatgta aagttttact 5634
   gtccaaatct ctttcagttc ataaatcacg taaaatagca tggcgatcgc aaagactaaa 5694
   atcgccagcc ctatttgtga ggtgctatta aatgctgtgt tttggttatt agcttcaatg 5754
   atcgcggcga gataaaatac aacatgggca atggataaca gcgtagaatt gcgtttacca 5814
   tattgtattg caccttttgc aatcaatgct ttttcatggc ggattgaaat agacagacta 5874
   taaaaacgga tcgctaaaat acaggcaaaa gtgatattga taaataacat gtggttatcc 5934
   ttaacataat tttattccaa taaagtggcg taatataaca aaaaatccga tgatatggca 5994
   tggaaatacg cctttatttt gaacgttcat cgcttttctt tttctttatt ggttgatttg 6054
   taagcataaa taaccttttt atctctcttg gttttccgct ataattgagt gaatttttga 6114
   gcataattaa gagtacaaag gattttgaaa tgatgcgtac acattattgc ggtgcattaa 6174
   accgtgacaa tatcggacaa gaagtaacat tgagtggttg ggtgcatcgt cgccgtgatt 6234
   taggtgggtt aatttttatt gatatgcgtg atcgtgaagg gattgtacaa gtgtgtttcg 6294
   atccaatata tcaagaagca ctcaccacag cagcaagttt acgtaatgag ttttgtattc 6354
   aaattaaagg cgaagtgatt gcccgcccag ataatcaaat caacaaaaat atggcaacag 6414
   gcgaagtgga agtgttagca aaatccctgt ctatttataa cagcgcagag ccattacctc 6474
   tcgactttaa ccaaaataat acggaagaac agcgtttaaa ataccgttat ttagacttac 6534
   gtcgcccaga aatggcgcaa cgtttaaaaa cacgagccaa aattaccagc tttgtgcgtc 6594
   gctttatgga cgaacatggt ttcttagata ttgaaacacc gatgttga 6642
<210> 81
   <211> 474
   <212> PRT
   <213> Pasteurella Multocida
<400> 81
   Met Thr Gln Lys Leu His Ile Lys Thr Trp Gly Cys Gln Met Asn Glu
   1               5                   10                  15
   Tyr Asp Ser Ser Lys Met Ala Asp Leu Leu Asn Ser Thr His Gly Leu
               20                  25                  30
   Glu Leu Thr Glu Ile Pro Glu Glu Ala Asp Val Leu Leu Leu Asn Thr
           35                  40                  45
   Cys Ser Ile Arg Glu Lys Ala Gln Glu Lys Val Phe His Gln Leu Gly
       50                  55                  60
   Arg Trp Lys Glu Leu Lys Lys His Lys Pro Gly Leu Val Ile Gly Val
   65                  70                  75                  80
   Gly Gly Cys Val Ala Ser Gln Glu Gly Glu His Ile Arg Thr Arg Ala
                   85                  90                  95
   Pro Tyr Val Asp Ile Ile Phe Gly Pro Gln Thr Leu His Arg Leu Pro
               100                 105                 110
   Glu Met Ile Asn Gln Ile Arg Gly Gly Lys Ser Ser Val Val Asp Val
           115                 120                 125
   Ser Phe Pro Glu Ile Glu Lys Phe Asp Arg Leu Pro Glu Pro Arg Ala
       130                 135                 140
   Glu Gly Pro Thr Ala Phe Val Ser Ile Met Glu Gly Cys Asn Lys Tyr
   145                 150                 155                 160
   Cys Ser Phe Cys Val Val Pro Tyr Thr Arg Gly Glu Glu Val Ser Arg
                   165                 170                 175
   Pro Val Asp Asp Val Leu Phe Glu Ile Ala Gln Leu Ala Glu Gln Gly
               180                 185                 190
   Val Arg Glu Val Asn Leu Leu Gly Gln Asn Val Asn Ala Tyr Arg Gly
           195                 200                 205
   Ala Thr His Asp Asp Gly Ile Cys Thr Phe Ala Glu Leu Leu Arg Leu
       210                 215                 220
   Val Ala Ala Ile Asp Gly Ile Asp Arg Leu Arg Phe Thr Thr Ser His
   225                 230                 235                 240
   Pro Ile Glu Phe Thr Asp Asp Ile Ile Asp Val Tyr Arg Asp Thr Pro
                   245                 250                 255
   Glu Leu Val Ser Phe Leu His Leu Pro Val Gln Ser Gly Ser Asp Arg
               260                 265                 270
   Val Leu Ser Met Met Lys Arg Asn His Thr Ala Leu Glu Tyr Lys Ser
           275                 280                 285
   Ile Ile Arg Lys Leu Arg Ala Val Arg Pro Glu Ile Gln Ile Ser Ser
       290                 295                 300
   Asp Phe Ile Val Gly Phe Pro Gly Glu Thr Ala Glu Asp Phe Glu Gln
   305                 310                 315                 320
   Thr Met Asn Leu Ile Ala Gln Val Asn Phe Asp Met Ser Phe Ser Phe
                   325                 330                 335
   Ile Tyr Ser Ala Arg Pro Gly Thr Pro Ala Ala Asp Met Pro Asp Asp
               340                 345                 350
   Val Thr Glu Glu Glu Lys Lys Gln Arg Leu Tyr Val Leu Gln Gln Arg
           355                 360                 365
   Ile Asn Asn Gln Ala Ala Gln Phe Ser Arg Ala Met Leu Gly Thr Glu
       370                 375                 380
   Gln Arg Val Leu Val Glu Gly Pro Ser Lys Lys Asp Leu Met Glu Leu
   385                 390                 395                 400
   Thr Gly Arg Thr Glu Thr Asn Arg Ile Val Asn Phe Val Gly Thr Pro
                   405                 410                 415
   Asp Met Ile Gly Lys Phe Val Asp Ile Lys Ile Thr Asp Val Phe Thr
               420                 425                 430
   Asn Ser Leu Arg Gly Glu Val Val Arg Thr Glu Glu Gln Met Gly Leu
           435                 440                 445
   Arg Val Val Gln Ser Pro Gln Met Val Ile Asn Arg Thr Arg Lys Glu
       450                 455                 460
   Asp Glu Leu Gly Val Gly Arg Tyr His Ala
   465                 470
<210> 82
   <211> 4835
   <212> DNA
   <213> Pasteurella Multocida
<220>
   <221> misc_feature
   <223> yojB
<220>
   <221> CDS
   <222> (407)..(1156)
<400> 82
   gtcaacgacg gggcgggtct tagaacattg gcatacgggt acgatgacac gccgtgtccc      60
   agagctccat cgctccttcc caaataactt ggtttggatg cacccattag atgcgaaaaa     120
   acgtggttta cgtcatggcg ataaagtgaa gatcagctca cgtcgtggcg aaatgatttc     180
   tcacttagat acccgtggac gtaataaagt cccacaaggc ttagtttaca ccactttctt     240
   tgatgcaggt cagttagcaa actatctcac tttagatgcg acagacccaa tttcaaaaga     300
   aacggacttc aaaaaatgtg cggtcaaagt ggaaaaagcg taacacgtta aatttaatga     360
   ggaacgaccg cactttgctt tcagtaaagt gcggttggaa agtcga atg aaa aaa        415
                                                      Met Lys Lys
                                                      1
   aca gtt gtg aat cct gaa cgt cgt cga ttt ttt aaa gag gct acg cgc       463
   Thr Val Val Asn Pro Glu Arg Arg Arg Phe Phe Lys Glu Ala Thr Arg
       5                   10                  15
   act gca ggc ggg ttg gca ggg gtg act ttg ctc ctt ggt ttg caa caa       511
   Thr Ala Gly Gly Leu Ala Gly Val Thr Leu Leu Leu Gly Leu Gln Gln
   20                  25                  30                  35
   aag cag agt ctt gcg cgc gaa ggc gtg gcg tta cgc cca cct ttt gcc       559
   Lys Gln Ser Leu Ala Arg Glu Gly Val Ala Leu Arg Pro Pro Phe Ala
                   40                  45                  50
   ctt gag aat gag aaa gcg ttt tct gct gcg tgc att cgt tgt ggt cag       607
   Leu Glu Asn Glu Lys Ala Phe Ser Ala Ala Cys Ile Arg Cys Gly Gln
               55                  60                  65
   tgt gta caa gcc tgt cca cat gag atg ttg cat ctt gcc tca ctg att       655
   Cys Val Gln Ala Cys Pro His Glu Met Leu His Leu Ala Ser Leu Ile
           70                  75                  80
   tca ccg atg gaa gca ggt aca ccg tat ttc att gcg cgc gat aag ccc       703
   Ser Pro Met Glu Ala Gly Thr Pro Tyr Phe Ile Ala Arg Asp Lys Pro
           85                  90                  95
   tgt gaa atg tgt gtg gat att cct tgt gca aaa gcc tgc cca acc ggt      751
   Cys Glu Met Cys Val Asp Ile Pro Cys Ala Lys Ala Cys Pro Thr Gly
   100                 105                 110                 115
   gca ttg gat aat caa gca aca gaa atc gat gat gcg cgt atg ggg tta      799
   Ala Leu Asp Asn Gln Ala Thr Glu Ile Asp Asp Ala Arg Met Gly Leu
                   120                 125                 130
   gct gtc ctg cta gat cat gaa act tgt ctg aac tgg caa ggt tta cgc      847
   Ala Val Leu Leu Asp His Glu Thr Cys Leu Asn Trp Gln Gly Leu Arg
               135                 140                 145
   tgt gat gtg tgt tat cgc gtc tgt ccg ctg att aat aaa gcg att acg      895
   Cys Asp Val Cys Tyr Arg Val Cys Pro Leu Ile Asn Lys Ala Ile Thr
           150                 155                 160
   tta gtg atg cat cgt aat gag cgt acg ggt aag cac gcc gtc ttt atc      943
   Leu Val Met His Arg Asn Glu Arg Thr Gly Lys His Ala Val Phe Ile
       165                 170                 175
   cca aca gtg cat tcc gaa gcc tgt aca gga tgt ggc aaa tgt gaa gaa      991
   Pro Thr Val His Ser Glu Ala Cys Thr Gly Cys Gly Lys Cys Glu Glu
   180                 185                 190                 195
   gct tgc gtt cta gaa gaa gcg gca atc aaa gtg tta ccg atg gca tta     1039
   Ala Cys Val Leu Glu Glu Ala Ala Ile Lys Val Leu Pro Met Ala Leu
                   200                 205                 210
   gcg aaa ggc atg tta ggt aaa cat tac cgt tta ggt tgg gaa gag aaa     1087
   Ala Lys Gly Met Leu Gly Lys His Tyr Arg Leu Gly Trp Glu Glu Lys
               215                 220                 225
   gaa aaa gcc ggg cat tcc ctt gcg cca gaa ggc att att tct ctc ccg     1135
   Glu Lys Ala Gly His Ser Leu Ala Pro Glu Gly Ile Ile Ser Leu Pro
           230                 235                 240
   act cgg tta ccg gag agc ttg taatggcaaa ttcaccaaaa tatgcgggta        1186
   Thr Arg Leu Pro Glu Ser Leu
       245                 250
   aagaagcacg agaaaagtta ggctggtggt acgccaatcg ctttttgttc tggcgacgtt   1246
   taacccagct gagtattctt gccatgtttt taagcggacc ttattttggg gtgtggatct   1306
   tgaaaggcaa ttacagtagt agtcttttgc ttgatttgat cccgatgact gatcctttga   1366
   ttatggcaga aagtctcgcg accggtttta tgcctaccat gaccgcgttg ttgggtgccc   1426
   tgattgtggt ggtgctttat gccattttag ggagtcgcgt tttctgtgct tgggtttgtc   1486
   cgttaaacat cgtgacagat gcgtccgctt ggctcagacg taaattagaa attcgtcaat   1546
   cggcaaaact cccacgaagt ttacgctatg cgatcttagt gatgattttg ttaggcagtg   1606
   cgctaagcgg gttattactt tgggaatggc tcaatccggt tgcagcacta ggtcgtgcgt   1666
   taatttacgg tttcggtgcg acagtttggc ttgttcttgc ggtgttttta tttgatttat   1726
   ttattgtcga gcatggttgg tgtgggcatt tatgcccaat aggtgcagcc tatggtgtga   1786
   ttggtgcgaa aggacttttt cgtatcaaag ttgagcatcg ccaacaatgt gataattgca 1846
   tggattgcta taacgtctgt ccagaaccac aagtgttacg cgatccatta catgcaaaaa 1906
   agagtgaaag cccacttgtg ctttcaaaag attgtatcag ctgtggacgt tgtatcgacg 1966
   tttgccctga aaaagtattt atttttacaa cacgatttaa tcattcagtt aatcattcgg 2026
   gggagtgatc aaaatgaaaa aaacaatgct aattttgacc gcactttttg ccttcacggt 2086
   caatgccaat gaagtcaaag tgggcaaaag tttacaagat tcacctgaaa atatcgcgcc 2146
   agccttccac aatacaccaa aagaaagtgg cttggcgccg ttaaactatg tgaaccaacc 2206
   accgatggtg ccacacgcga cgaaaaacta tcaggtgacg aaaaatatca accaatgttt 2266
   aacttgtcat agcccagaag cctcacgtgt tactggtgca acacgcatca gtccaaccca 2326
   ctttatggat cgtgatggta acattgtcgg aggcacctca cctcgccgtt acttctgttt 2386
   acaatgccat gtctctcaat ctgatgttga gccgattatt caaaatgaat ttaaaccgat 2446
   ggctggtttt ggtaaataag ttaaggacgg aactatgttg aacttaatta aacgcttttg 2506
   gaaatggttt cgctcgccca gtcgtattgc cgttggtacc ttgattaccc ttgcttttat 2566
   tgcaggtatt gtctcttggg ttgggtttaa ttatggctta gagcaaacca atacagagga 2626
   attctgtgtt agttgtcaca gtaatgatgt gtatccagaa tatttacata cggcgcatta 2686
   tttaaatcgc agtggagtaa aagccacttg tcctgattgc catgtaccgc acgaatttat 2746
   tccaaaaatg atccgtaaag tccaagccag tcgagaagtg tatgcgcatc ttatgggtta 2806
   tacggatacc attgataaat ttaactcccg tcgtttgcat atggcagaac gtgaatgggc 2866
   acgattaaaa gccaataact cgcaggaatg ccgtaactgc cataactttg agaatatgga 2926
   ttttagtcag caaaaaacgg tggcggaaaa aatgcatgca cttgctataa aagaagaaaa 2986
   aacctgtatt gactgtcaca aagggattgc gcaccaatta ccggatatga gcggtgttga 3046
   gtcgggtttt agtacggaac aaaaataaca cttttcttgt tctcccacag gaaatctgca 3106
   tcgtattcag gtgcagattt ttttattttt acgatcatcc tcacaacctt ttcgctgata 3166
   aataggatac aacgctagct acacgctatc ttgctgcttt ttgtgttgtt gtgagggatt 3226
   atgtcatctt atctgcctct tttattacgt ttgttacaaa ttccgcgttt gggttcgctg 3286
   gcgatccagc gtttattgga gcacatcagt ccagcggaat taatggaata tgatacaaaa 3346
   gcctttcaac agataggctg gacggcacag cagattcagc gttggtttac gcctgagaac 3406
   cgttatattg atcccgcgtt agcctgggta aacgaacaac aacatattgt ggattggttt 3466
   gatcctcatt atcctccctt gttaaagcaa acagaagagg caccacttgt cttgtttgtg 3526
   aaaggggaag tggctacgct atctgcacag caagtggcga tcgtgggcag tcgtcattgt 3586
   tcacgttatg gggaatactg ggcaaattat ttcgccactc aactggctta tgccgatatt   3646
   gtggtgacca gtggtttagc gttaggtatt gatggtttct cacatcaagc ggtagtggat   3706
   atccacggga agacgattgc agtattaggc agtggtttag aggtcattta tccgaaaaaa   3766
   caccgaggtt tagccgaaaa aattattgaa catcaaggtg cgttagtgtc tgaattttta   3826
   cctttccaac cccctgtggc agaaaatttt ccacgtcgta atcgcattat tagtggttta   3886
   tctttgggga cattagtcat tgaagcctca gaaaacagtg gttctttaat tactgcccgc   3946
   tatgctttag agcaaaatcg agatgttttt gcgttaccgg ggcagattca gcatggattt   4006
   agccaaggtt gtcataaact gattaaacag ggggcaatat tagtcgaaag tattcaggat    4066
   attttagaac atctctcgcc ttattcgcat tgtgccatgc cagcgttgcc tcgtacggag   4126
   aacgcctttt ctcaacaagt aacagacaca tcaaccatca atactgcgcg gataacgcca   4186
   gaacatccgg aattatatgc caaaattggc tatatgcccg tcagtattga tgtattagcc   4246
   caacaagtca atctacctat tgatacctta ttagtacagc ttttaacctt ggaattgcaa   4306
   gacttgattg ttgctgaaaa tggattatat cagcgcaaat aacggaaaga gtaaccacaa   4366
   atgagaaagg actgtgttga aacggctatc aacatcagtc cttgcagtat attatctcat   4426
   atgacacgag acggcagttt catgagatta atagagacta aacgcttgta aatacggtag   4486
   tttgcctcgt tgtagcattt tctcaatatt gcctttgtga tcatcacgac ctaagcctcg   4546
   tagctcaata ttgataccaa tacttttgtc ataaaaaatt tgattcgccg tttggttttc   4606
   tttgctggtc acactacgtc gggcaccaac attaatcgcc caacagcaag tgctgtattc   4666
   tagtcccaaa tattgctcaa caggctttct caacgcaaga tcttgataat gacgtgctac   4726
   caccgcccat tgatccgtga gtgcccatgc cacagttaga ccaagctgtt ttatgtcttg   4786
   tccgtagcgg ttcgccgctg aggtgaggtt ttggtcaata tattgttga               4835
<210> 83
   <211> 250
   <212> PRT
   <213> Pasteurella Multocida
<400> 83
   Met Lys Lys Thr Val Val Asn Pro Glu Arg Arg Arg Phe Phe Lys Glu
   1               5                   10                  15
   Ala Thr Arg Thr Ala Gly Gly Leu Ala Gly Val Thr Leu Leu Leu Gly
               20                  25                  30
   Leu Gln Gln Lys Gln Ser Leu Ala Arg Glu Gly Val Ala Leu Arg Pro
           35                  40                  45
   Pro Phe Ala Leu Glu Asn Glu Lys Ala Phe Ser Ala Ala Cys Ile Arg
       50                  55                  60
   Cys Gly Gln Cys Val Gln Ala Cys Pro His Glu Met Leu His Leu Ala
   65                  70                  75                  80
   Ser Leu Ile Ser Pro Met Glu Ala Gly Thr Pro Tyr Phe Ile Ala Arg
                   85                  90                  95
   Asp Lys Pro Cys Glu Met Cys Val Asp Ile Pro Cys Ala Lys Ala Cys
               100                 105                 110
   Pro Thr Gly Ala Leu Asp Asn Gln Ala Thr Glu Ile Asp Asp Ala Arg
           115                 120                 125
   Met Gly Leu Ala Val Leu Leu Asp His Glu Thr Cys Leu Asn Trp Gln
       130                 135                 140
   Gly Leu Arg Cys Asp Val Cys Tyr Arg Val Cys Pro Leu Ile Asn Lys
   145                 150                 155                 160
   Ala Ile Thr Leu Val Met His Arg Asn Glu Arg Thr Gly Lys His Ala
                   165                 170                 175
   Val Phe Ile Pro Thr Val His Ser Glu Ala Cys Thr Gly Cys Gly Lys
               180                 185                 190
   Cys Glu Glu Ala Cys Val Leu Glu Glu Ala Ala Ile Lys Val Leu Pro
           195                 200                 205
   Met Ala Leu Ala Lys Gly Met Leu Gly Lys His Tyr Arg Leu Gly Trp
       210                 215                 220
   Glu Glu Lys Glu Lys Ala Gly His Ser Leu Ala Pro Glu Gly Ile Ile
   225                 230                 235                 240
   Ser Leu Pro Thr Arg Leu Pro Glu Ser Leu
                   245                 250
<210> 84
   <211> 3494
   <212> DNA
   <213> Pasteurella Multocida
<220>
   <221> misc_feature
   <223> yyaM
<220>
   <221> CDS
   <222> (2411)..(2719)
<400> 84
   gtcaacgatc cgaatacgac ggaagaaaac tatatgcaag tcatttatgg caagactgcg 60
   cgtctattcg aagctgcgac gcattgcgcc gcgatacttg ctcatgcgac ggaagcccaa 120
   gaaaatgctt tacgcgaata tggttgttat ctaggcacag cttttcaatt agtcgatgat 180
   attttagatt atagtgcaga tgcaaaagca ctcggcaaaa atattggtga tgatttagcg 240
   gaaggtaaac caacattacc gttattacac gccatgcatc acggcaatcc agcacaagca 300
   aaattgattc gcgaagcgat tgagcaaggg ggtaagcgtg atattttaga ggatgtactg 360
   acaattatga cagaacataa atccctcgac tatgcgatga tgcgcgctaa acaagaagca 420
   caaaaagccg ttgatgcgat tgcattattg cctgaaaatg aatataaaca agcgttaatt 480
   tcattagctt acttatccgt cgatcgcgct tattaaccac ttaataaggc gagacatgtt 540
   agcgtaacga ccgcctaaag tgcggtcatt tatttagtaa ttttaaacac gacaatgaca 600
   gaacaaaaca tccctacgaa aaaaacacgc aaaggcaaag atcctcacgc gccttttgta 660
   cgcgaaaaat tatccctacc aaatgggcat aacaaattgt tattgcattc ttgttgtgcg 720
   ccttgctcgg gagaagtaat ggaggcaatt catgcttcag gtattgaatt tactatttac 780
   ttctacaacc caaacattca cccattaaaa gaatatctca ttcgtaaaga agaaaatatt 840
   cgttttgcgg aaaaatgggg cattccgttt attgatgccg attatgatcg tcaagaatgg 900
   tttgaccgtg ccaaaggcat ggaagatgag ccagagcgtg gtattcgttg cactatgtgc 960
   tttgatatgc gttttgaaaa agccgcagaa tatgcacaca acaatggttt ccccgtattt 1020
   actagctgct taggtatttc acgttggaaa gacatgaacc aaatcaacgg ttgtggacac 1080
   cgtgccgccg aaaaatatga tgatgtagtg tattgggatt ataactggcg taaaggtggt 1140
   ggctcacaac gtatgattga aatcagtaag cgtgaacgtt tttaccagca agaatattgc 1200
   ggttgtgtgt attctttgcg tgacacgaat aaatggcgtg aagcaaacgg acgccaaaaa 1260
   attgaaattg gcaaattgta ttattccgcc gattaaaaca accaagtaaa ccatcgtgcc 1320
   gtcatataac atggcggcac ttttttaggc tttatatatg caacgttttc gccaatcttt 1380
   cgaaacctaa tgctaacgcc tcttcactaa tatttaatgc tggggcaaaa cgcaatacat 1440
   taggacccgc cactaaaatc attaagccat tatctgctgc ttttttgaca aattcgctcg 1500
   ctcttccgtg atattgctcg ataagttctg caccaattaa caaaccttcg ccacggattt 1560
   ctttaaataa gccacattgc tgattaattt cttgcagctt ttgcataaat ttttcagaag 1620
   tgcgctgaat tttttgcaag aatggtggtg ctgaaatgat atcaatcact ttttctgcca   1680
   cggcacaagc gagaggattg ccaccaaagg tggtgccatg cacgccaggt gcaaaacttt   1740
   tggcgatctc atgcgttgtc aacgaatgag aaaattgtga cagattgggc tccaacggga   1800
   cgtgcggatg agattgggga tacgcaagta cgttatgaaa ttgagcaggt gagttctagc   1860
   aattatagtg cactgtttgt ctccatttta cagatgaaac gtaatgaggt ggtatttagt   1920
   ccacatttag cggataaaca acgttatagt tctgatcgct tgaaccaact tgttggtgaa   1980
   ttagatgcgt cttatcgtaa acaagtccgt gatttgaata acagcggatt gatgccaatt   2040
   cagtctgtat tcggtacgga cagtaatggg cgtactgctc tcgtgttagg cgcacccttt   2100
   aatcatgcgt ggacaaaact aggacaagtt ttaccgcagc ttgaatttga tattaaagat   2160
   gaaattattg gtcgtggggt aagggagtta aaataccgtc cagctggagc aaaaagttgg   2220
   tggtggccat ttggtcgtgc tgaaggcagt agcggactga aaacaggtac ctattttatg   2280
   cagttaagcg ccttagggaa gcaaagtgcg gtggtgatga ccgatgatga tggcaatgcg   2340
   ttatctgggg agcaagctca ggcgctttat caagcattac aaaatctctt agcgaaataa   2400
   tacagtcaag atg act aaa ctc agt atc cag cga gat aac ttg att tgt      2449
              Met Thr Lys Leu Ser Ile Gln Arg Asp Asn Leu Ile Cys
              1               5                   10
   ttg agt tat gtc gca tta atg gga ttc ggc ttt ccc att atg cgt tat     2497
   Leu Ser Tyr Val Ala Leu Met Gly Phe Gly Phe Pro Ile Met Arg Tyr
       15                  20                  25
   atg agt att cat ttt gat aca tta aat aat aac gct gtt cgc ttt ctc     2545
   Met Ser Ile His Phe Asp Thr Leu Asn Asn Asn Ala Val Arg Phe Leu
   30                  35                  40                  45
   tca ggg ggc agc gtt ttt att tta gcc tgt ttt ttt tat tat cgc gct     2593
   Ser Gly Gly Ser Val Phe Ile Leu Ala Cys Phe Phe Tyr Tyr Arg Ala
                   50                  55                  60
   gag tta aca tct tcg ggg gct ggc gtc cag tcc gtt gcg atg ttg ccg     2641
   Glu Leu Thr Ser Ser Gly Ala Gly Val Gln Ser Val Ala Met Leu Pro
               65                  70                  75
   agt tca agt tta ggt ttc tta ata ttg aaa act gta cca tct ttt tca     2689
   Ser Ser Ser Leu Gly Phe Leu Ile Leu Lys Thr Val Pro Ser Phe Ser
           80                  85                  90
   tac gtt aca atc tca aca ctt aat cgc gtt tgaccttccg atttttgata       2739
   Tyr Val Thr Ile Ser Thr Leu Asn Arg Val
       95                  100
   gtcaaagact actgagtaac gcttgtagtc gcgtgaatcg actgttacat aagccgatat   2799
   gtcagaataa gtactgccgg tatatcgtct taatctaaga ttaagcttgc cacttttgtt   2859
   cgataaagcg tcaaacgaaa gcacgacttt accgtccttg acttccacct gatcttcaat   2919
   gagcacttga cttagtgcga ccaatcgacc gttggcagtc agtgtcgcaa tgccgtgatc   2979
   cgtatcaagc gttacaccgc tattttttcc ccagttttta ttgagctttt cactatgttt   3039
   cagtaagttt ctgccaccaa tctgcaactg attaaactta gcttcaagcg tttcactctt   3099
   gactgcaagc gacttgtttt cattgctaac cgtctgctca agtgctgtga ttttggatgt   3159
   taaatctaac ttagttgcat tgacttcatc agtccattct gactttaact cttttctcgc   3219
   aagtgacgcc acttcatctt tgctagcttt cgtttctttt aagtcagaaa tgccactagt   3279
   attttgcgcc actttagaat cgagcgtttc tagttttgta gagaaagatt tgtctttttc   3339
   gctagccgtt ttttgaatta gctgtatttc actttcgctc aatccaactc tagcagttag   3399
   actgtctagc ttgtcagcag tagatttatt cacagtcgct tgtgattgct tgtgttgaat   3459
   aatatccgcg cttacttccg agatagccac gtcga                              3494
<210> 85
   <211> 103
   <212> PRT
   <213> Pasteurella Multocida
<400> 85
   Met Thr Lys Leu Ser Ile Gln Arg Asp Asn Leu Ile Cys Leu Ser Tyr
   1               5                   10                  15
   Val Ala Leu Met Gly Phe Gly Phe Pro Ile Met Arg Tyr Met Ser Ile
               20                  25                  30
   His Phe Asp Thr Leu Asn Asn Asn Ala Val Arg Phe Leu Ser Gly Gly
           35                  40                  45
   Ser Val Phe Ile Leu Ala Cys Phe Phe Tyr Tyr Arg Ala Glu Leu Thr
       50                  55                  60
   Ser Ser Gly Ala Gly Val Gln Ser Val Ala Met Leu Pro Ser Ser Ser
   65                  70                  75                  80
   Leu Gly Phe Leu Ile Leu Lys Thr Val Pro Ser Phe Ser Tyr Val Thr
                   85                  90                  95
   Ile Ser Thr Leu Asn Arg Val
               100
<210> 86
   <211> 19
   <212> DNA
   <213> Artificial Sequence
<220>
   <223> Description of Artificial Sequence: primer
<400> 86
   aggccggtac cggccgcct 19
<210> 87
   <211> 19
   <212> DNA
   <213> Artificial Sequence
<220>
   <223> Description of Artificial Sequence: primer
<400> 87
   cggccggtac cggcctagg 19
<210> 88
   <211> 18
   <212> DNA
   <213> Artificial Sequence
<220>
   <223> Description of Artificial Sequence: primer
<400> 88
   catggtaccc attctaac 18
<210> 89
   <211> 18
   <212> DNA
   <213> Artificial Sequence
<220>
   <223> Description of Artificial Sequence: primer
<400> 89
   ctaggtacct acaacctc 18
<210> 90
   <211> 119
   <212> DNA
   <213> Artificial Sequence
<220>
   <223> Description of Artificial Sequence: transposon insert
<220>
   <221> misc_feature
   <222> (25)..(25)
   <223> n is a, c, g, or t
<220>
   <221> misc_feature
   <222> (27)..(27)
   <223> n is a, c, g, or t
<220>
   <221> misc_feature
   <222> (29) .. (29)
   <223> n is a, c, g, or t
<220>
   <221> misc_feature
   <222> (31) .. (31)
   <223> n is a, c, g, or t
<220>
   <221> misc_feature
   <222> (33) .. (33)
   <223> n is a, c, g, or t
<220>
   <221> misc_feature
   <222> (35) .. (35)
   <223> n is a, c, g, or t
<220>
   <221> misc_feature
   <222> (37) .. (37)
   <223> n is a, c, g, or t
<220>
   <221> misc_feature
   <222> (39) .. (39)
   <223> n is a, c, g, or t
<220>
   <221> misc_feature
   <222> (41) .. (41)
   <223> n is a, c, g, or t
<220>
   <221> misc_feature
   <222> (43) .. (43)
   <223> n is a, c, g, or t
<220>
   <221> misc_feature
   <222> (45) .. (45)
   <223> n is a, c, g, or t
<220>
   <221> misc_feature
   <222> (47) .. (47)
   <223> n is a, c, g, or t
<220>
   <221> misc_feature
   <222> (49) .. (49)
   <223> n is a, c, g, or t
<220>
   <221> misc_feature
   <222> (51) .. (51)
   <223> n is a, c, g, or t
<220>
   <221> misc_feature
   <222> (53) .. (53)
   <223> n is a, c, g, or t
<220>
   <221> misc_feature
   <222> (55) .. (55)
   <223> n is a, c, g, or t
<220>
   <221> misc_feature
   <222> (57) .. (57)
   <223> n is a, c, g, or t
<220>
   <221> misc_feature
   <222> (59) .. (59)
   <223> n is a, c, g, or t
<220>
   <221> misc_feature
   <222> (61) .. (61)
   <223> n is a, c, g, or t
<220>
   <221> misc_feature
   <222> (63) .. (63)
   <223> n is a, c, g, or t
<220>
   <221> misc_feature
   <222> (65) .. (65)
   <223> n is a, c, g, or t
<220>
   <221> misc_feature
   <222> (67) .. (67)
   <223> n is a, c, g, or t
<220>
   <221> misc_feature
   <222> (69) .. (69)
   <223> n is a, c, g, or t
<220>
   <221> misc_feature
   <222> (71) .. (71)
   <223> n is a, c, g, or t
<220>
   <221> misc_feature
   <222> (73) .. (73)
   <223> n is a, c, g, or t
<220>
   <221> misc_feature
   <222> (75) .. (75)
   <223> n is a, c, g, or t
<220>
   <221> misc_feature
   <222> (77) .. (77)
   <223> n is a, c, g, or t
<220>
   <221> misc_feature
   <222> (79) .. (79)
   <223> n is a, c, g, or t
<220>
   <221> misc_feature
   <222> (81) .. (81)
   <223> n is a, c, g, or t
<220>
   <221> misc_feature
   <222> (83) .. (83)
   <223> n is a, c, g, or t
<220>
   <221> misc_feature
   <222> (85) .. (85)
   <223> n is a, c, g, or t
<220>
   <221> misc_feature
   <222> (87) .. (87)
   <223> n is a, c, g, or t
<220>
   <221> misc_feature
   <222> (89) .. (89)
   <223> n is a, c, g, or t
<220>
   <221> misc_feature
   <222> (91) .. (91)
   <223> n is a, c, g, or t
<220>
   <221> misc_feature
   <222> (93) .. (93)
   <223> n is a, c, g, or t
<400> 90
   ctaggtacct acaacctcaa gcttnknknk nknknknknk nknknknknk nknknknknk     60
   nknknknknk nknknknknk nknknknknk nknkaagctt ggttagaatg ggtaccatg     119
<210> 91
   <211> 18
   <212> DNA
   <213> Artificial Sequence
<220>
   <223> Description of Artificial Sequence: primer
<400> 91
   tacctacaac ctcaagct 18
<210> 92
   <211> 18
   <212> DNA
   <213> Artificial Sequence
<220>
   <223> Description of Artificial Sequence: primer
<400> 92
   tacccattct aaccaagc 18
<210> 93
   <211> 19
   <212> DNA
   <213> Artificial Sequence
<220>
   <223> Description of Artificial Sequence: primer
<400> 93
   tacctacaac ctcaagctt 19
<210> 94
   <211> 20
   <212> DNA
   <213> Artificial Sequence
<220>
   <223> Description of Artificial Sequence: primer
<400> 94
   tacccattct aaccaagctt 20
<210> 95
   <211> 19
   <212> DNA
   <213> Artificial Sequence
<220>
   <223> Description of Artificial Sequence: primer
<400> 95
   ggcagagcat tacgctgac 19
<210> 96
   <211> 27
   <212> DNA
   <213> Artificial Sequence
<220>
   <223> Description of Artificial Sequence: primer
<400> 96
   gtaccggcca ggcggccacg cgtattc 27
<210> 97
   <211> 531
   <212> DNA
   <213> Actinobacillus pleuropneumoniae
<220>
   <221> misc_feature
   <223> atpG
<400> 97
   tgttgagctt ggtttagtag ggtcgaaagg cgtaagcttt taccaaaatc taggcttaaa     60
   cgtgagatct caagtaacgg gattaggcga taatccggaa atggaacgta tcgtgggcgc    120
   agttaatgaa atgattaatg cgttccgaaa cggagaagtg gatgcggttt acgtcgctta    180
   caaccgtttt gaaaatacga tgtcacaaaa acctgttatc gcacagttac ttccgttacc    240
   taaactagat gacgatgaat tagatacgaa aggttcatgg gattatattt atgaaccgaa    300
   tccacaagtt ttattggata gtttacttgt tcgttattta gaaactcagg tataccaagc    360
   agttgtagat aacctagctt ctgaacaagc cgctcgaatg gtagcgatga aagccgcaac    420
   agataatgcg ggtacattaa tcgatgaatt acaattagtg tataacaaag ctcgccaagc    480
   aagcattaca aatgaattaa acgaaattgt tgcgggtgcc gcagcaattt a             531
<210> 98
   <211> 25
   <212> DNA
   <213> Artificial Sequence
<220>
   <223> Description of Artificial Sequence: primer
<400> 98
   tctccattcc cttgctgcgg caccc 25
<210> 99
   <211> 20
   <212> DNA
   <213> Artificial Sequence
<220>
   <223> Description of Artificial Sequence: primer
<400> 99
   ggattacagc cggatccggg 20
<210> 100
   <211> 1034
   <212> DNA
   <213> Pasteurella multocida
<220>
   <221> misc_feature
   <223> cap5E
<220>
   <221> CDS
   <222> (1)..(1032)
<400> 100
   atg ttt aaa aat aaa aca ctt tta att aca ggt gga acg ggt tct ttt      48
   Met Phe Lys Asn Lys Thr Leu Leu Ile Thr Gly Gly Thr Gly Ser Phe
   1               5                   10                  15
   ggt aat gct gta ctc aaa cgt ttc tta gaa aca gat att cga gaa att      96
   Gly Asn Ala Val Leu Lys Arg Phe Leu Glu Thr Asp Ile Arg Glu Ile
               20                  25                  30
   cgt gtt ttt tcg cgt gat gag aag aaa caa gat gac atg cgg aaa aaa     144
   Arg Val Phe Ser Arg Asp Glu Lys Lys Gln Asp Asp Met Arg Lys Lys
           35                  40                  45
   tat aat gat gca aaa tta aaa ttt tat att ggc gat gtt cgt gac tac     192
   Tyr Asn Asp Ala Lys Leu Lys Phe Tyr Ile Gly Asp Val Arg Asp Tyr
       50                  55                  60
   gat agt att tta aat gcc tcg cga ggt gtt gac tat att tat cat gct     240
   Asp Ser Ile Leu Asn Ala Ser Arg Gly Val Asp Tyr Ile Tyr His Ala
   65                  70                  75                  80
   gcc gca tta aag caa gtg cct tca tgc gag ttt tat ccg tta gag gca     288
   Ala Ala Leu Lys Gln Val Pro Ser Cys Glu Phe Tyr Pro Leu Glu Ala
                   85                  90                  95
   gtg aaa acc aat att tta ggt acg gca aat gtc tta gaa gcc gcc atc     336
   Val Lys Thr Asn Ile Leu Gly Thr Ala Asn Val Leu Glu Ala Ala Ile
               100                 105                 110
   caa aac cag ata aaa cgc gtc gtc tgt ctt agc aca gat aaa gcg gtg     384
   Gln Asn Gln Ile Lys Arg Val Val Cys Leu Ser Thr Asp Lys Ala Val
           115                 120                 125
   tac cca att aat gcg atg ggc att tct aaa gca atg atg gaa aaa gtc     432
   Tyr Pro Ile Asn Ala Met Gly Ile Ser Lys Ala Met Met Glu Lys Val
       130                 135                 140
   atc atc gca aaa tcg cgt aac cta gaa ggc aca cca acg aca atc tgt     480
   Ile Ile Ala Lys Ser Arg Asn Leu Glu Gly Thr Pro Thr Thr Ile Cys
   145                 150                 155                 160
   tgt act cgc tat ggc aat gtc atg gca tcg cgt ggt tcg gtt atc cca     528
   Cys Thr Arg Tyr Gly Asn Val Met Ala Ser Arg Gly Ser Val Ile Pro
                   165                 170                 175
   tta ttt gtc gat caa ata cgt caa ggc aag cct ttt act att act gat     576
   Leu Phe Val Asp Gln Ile Arg Gln Gly Lys Pro Phe Thr Ile Thr Asp
               180                 185                 190
   cct gag atg aca cgc ttt atg atg aca ttg gaa gat gct gtg gat tta     624
   Pro Glu Met Thr Arg Phe Met Met Thr Leu Glu Asp Ala Val Asp Leu
           195                 200                 205
   gtc cta tat gca ttt aaa aat ggt caa aat ggt gat gtt ttt gta caa     672
   Val Leu Tyr Ala Phe Lys Asn Gly Gln Asn Gly Asp Val Phe Val Gln
       210                 215                 220
   aaa gcc ccc gca gca acc att ggt acc ctt gcc aaa gca att acc gaa      720
   Lys Ala Pro Ala Ala Thr Ile Gly Thr Leu Ala Lys Ala Ile Thr Glu
   225                 230                 235                 240
   tta tta tct gtc cca aat cac cct att tcc att ata ggt acg cgt cat      768
   Leu Leu Ser Val Pro Asn His Pro Ile Ser Ile Ile Gly Thr Arg His
                   245                 250                 255
   gga gag aaa gca ttc gaa gct tta tta agc cgt gaa gaa atg gtt cat      816
   Gly Glu Lys Ala Phe Glu Ala Leu Leu Ser Arg Glu Glu Met Val His
               260                 265                 270
   gca att aat gaa ggt aat tat tat cgc atc cca gcc gat caa cgc agt      864
   Ala Ile Asn Glu Gly Asn Tyr Tyr Arg Ile Pro Ala Asp Gln Arg Ser
           275                 280                 285
   tta aat tac agt aaa tat gtc gaa aaa ggg gaa cca aaa att acc gaa      912
   Leu Asn Tyr Ser Lys Tyr Val Glu Lys Gly Glu Pro Lys Ile Thr Glu
       290                 295                 300
   gtc acc gac tac aac tca cat aat act gag cgt ttg act gtc aag gaa      960
   Val Thr Asp Tyr Asn Ser His Asn Thr Glu Arg Leu Thr Val Lys Glu
   305                 310                 315                 320
   atg aag cag tta ctg ctt aaa ctt gaa ttc ata cag aaa atg att gag     1008
   Met Lys Gln Leu Leu Leu Lys Leu Glu Phe Ile Gln Lys Met Ile Glu
                   325                 330                 335
   ggt gaa tac atc tca ccg gag gta ta                                  1034
   Gly Glu Tyr Ile Ser Pro Glu Val
               340
<210> 101
   <211> 344
   <212> PRT
   <213> Pasteurella multocida
<400> 101
   Met Phe Lys Asn Lys Thr Leu Leu Ile Thr Gly Gly Thr Gly Ser Phe
   1               5                   10                  15
   Gly Asn Ala Val Leu Lys Arg Phe Leu Glu Thr Asp Ile Arg Glu Ile
               20                  25                  30
   Arg Val Phe Ser Arg Asp Glu Lys Lys Gln Asp Asp Met Arg Lys Lys
           35                  40                  45
   Tyr Asn Asp Ala Lys Leu Lys Phe Tyr Ile Gly Asp Val Arg Asp Tyr
       50                  55                  60
   Asp Ser Ile Leu Asn Ala Ser Arg Gly Val Asp Tyr Ile Tyr His Ala
   65                  70                  75                  80
   Ala Ala Leu Lys Gln Val Pro Ser Cys Glu Phe Tyr Pro Leu Glu Ala
                   85                  90                  95
   Val Lys Thr Asn Ile Leu Gly Thr Ala Asn Val Leu Glu Ala Ala Ile
               100                 105                 110
   Gln Asn Gln Ile Lys Arg Val Val Cys Leu Ser Thr Asp Lys Ala Val
           115                 120                 125
   Tyr Pro Ile Asn Ala Met Gly Ile Ser Lys Ala Met Met Glu Lys Val
       130                 135                 140
   Ile Ile Ala Lys Ser Arg Asn Leu Glu Gly Thr Pro Thr Thr Ile Cys
   145                 150                 155                 160
   Cys Thr Arg Tyr Gly Asn Val Met Ala Ser Arg Gly Ser Val Ile Pro
                   165                 170                 175
   Leu Phe Val Asp Gln Ile Arg Gln Gly Lys Pro Phe Thr Ile Thr Asp
               180                 185                 190
   Pro Glu Met Thr Arg Phe Met Met Thr Leu Glu Asp Ala Val Asp Leu
           195                 200                 205
   Val Leu Tyr Ala Phe Lys Asn Gly Gln Asn Gly Asp Val Phe Val Gln
       210                 215                 220
   Lys Ala Pro Ala Ala Thr Ile Gly Thr Leu Ala Lys Ala Ile Thr Glu
   225                 230                 235                 240
   Leu Leu Ser Val Pro Asn His Pro Ile Ser Ile Ile Gly Thr Arg His
                   245                 250                 255
   Gly Glu Lys Ala Phe Glu Ala Leu Leu Ser Arg Glu Glu Met Val His
               260                 265                 270
   Ala Ile Asn Glu Gly Asn Tyr Tyr Arg Ile Pro Ala Asp Gln Arg Ser
           275                 280                 285
   Leu Asn Tyr Ser Lys Tyr Val Glu Lys Gly Glu Pro Lys Ile Thr Glu
       290                 295                 300
   Val Thr Asp Tyr Asn Ser His Asn Thr Glu Arg Leu Thr Val Lys Glu
   305                 310                 315                 320
   Met Lys Gln Leu Leu Leu Lys Leu Glu Phe Ile Gln Lys Met Ile Glu
                   325                 330                 335
   Gly Glu Tyr Ile Ser Pro Glu Val
               340
<210> 102
   <211> 4931
   <212> DNA
   <213> Pasteurella Multocida
<220>
   <221> misc_feature
   <223> fhaB2
<220>
   <221> CDS
   <222> (1)..(4929)
<220>
   <221> misc_feature
   <222> (4894)..(4894)
   <223> n is a, c, g, or t
<400> 102
   atg aac aaa aat cgt tac aaa ctc att ttt agt caa gtc aaa ggt tgt       48
   Met Asn Lys Asn Arg Tyr Lys Leu Ile Phe Ser Gln Val Lys Gly Cys
   1               5                   10                  15
   ctc gtt cct gtg gca gaa tgt att aac tca gct att agc aat ggt tca       96
   Leu Val Pro Val Ala Glu Cys Ile Asn Ser Ala Ile Ser Asn Gly Ser
               20                  25                  30
   tct gat tca aca tcc aca tca gaa caa gtt gaa gag gaa cct ttc ctt      144
   Ser Asp Ser Thr Ser Thr Ser Glu Gln Val Glu Glu Glu Pro Phe Leu
           35                  40                  45
   cta gaa caa tat tca ctt tcc tcc gtg tct tta tta gta aaa agc acg      192
   Leu Glu Gln Tyr Ser Leu Ser Ser Val Ser Leu Leu Val Lys Ser Thr
       50                  55                  60
   ttc aat cct gtt tcg tat gca atg caa ttg act tgg aaa cag ctt tct      240
   Phe Asn Pro Val Ser Tyr Ala Met Gln Leu Thr Trp Lys Gln Leu Ser
   65                  70                  75                  80
   att tta ttt tta act gtg att tct gtt cct gtt ttg gct gag gga aaa      288
   Ile Leu Phe Leu Thr Val Ile Ser Val Pro Val Leu Ala Glu Gly Lys
                   85                  90                  95
   ggg gat gaa aga aat caa tta aca gtg att gat aat agc gat cat att      336
   Gly Asp Glu Arg Asn Gln Leu Thr Val Ile Asp Asn Ser Asp His Ile
               100                 105                 110
   aaa tta gat gca tct aat ctt gct ggt aat gat aaa aca aaa atc tat      384
   Lys Leu Asp Ala Ser Asn Leu Ala Gly Asn Asp Lys Thr Lys Ile Tyr
           115                 120                 125
   caa gca gaa aat aaa gtt ctg gtt att gat att gct aaa cca aat ggg      432
   Gln Ala Glu Asn Lys Val Leu Val Ile Asp Ile Ala Lys Pro Asn Gly
       130                 135                 140
   aaa ggg att tca gat aac cgt ttt gaa aaa ttt aat att cca aat agc      480
   Lys Gly Ile Ser Asp Asn Arg Phe Glu Lys Phe Asn Ile Pro Asn Ser
   145                 150                 155                 160
   gcg gtg ttt aat aat aat ggg act gaa gcg cag gca aga tca aca tta      528
   Ala Val Phe Asn Asn Asn Gly Thr Glu Ala Gln Ala Arg Ser Thr Leu
                   165                 170                 175
   att ggt tac att ccg caa aat caa aat tta agg gga ggg aaa gaa gct      576
   Ile Gly Tyr Ile Pro Gln Asn Gln Asn Leu Arg Gly Gly Lys Glu Ala
               180                 185                 190
   gat gtt ata tta aat caa gtg aca ggt cct caa gaa agt aaa att gtt      624
   Asp Val Ile Leu Asn Gln Val Thr Gly Pro Gln Glu Ser Lys Ile Val
           195                 200                 205
   ggc gcg ctt gaa gta tta ggt aaa aaa gct gat atc gtc att gca aac      672
   Gly Ala Leu Glu Val Leu Gly Lys Lys Ala Asp Ile Val Ile Ala Asn
       210                 215                 220
   caa aat ggt att acc tta aat ggt gta aga aca ata aat tca gat cgt      720
   Gln Asn Gly Ile Thr Leu Asn Gly Val Arg Thr Ile Asn Ser Asp Arg
   225                 230                 235                 240
   ttt gtt gcc act acg agt gag ctt ata gat ccg aat cag atg atg tta      768
   Phe Val Ala Thr Thr Ser Glu Leu Ile Asp Pro Asn Gln Met Met Leu
                   245                 250                 255
   aag gtt aca aaa gga aat gtg atc att gat att gat ggt ttt tcg aca      816
   Lys Val Thr Lys Gly Asn Val Ile Ile Asp Ile Asp Gly Phe Ser Thr
               260                 265                 270
   gat gga tta aag tat tta gat att att gct aaa aaa att gaa caa aag      864
   Asp Gly Leu Lys Tyr Leu Asp Ile Ile Ala Lys Lys Ile Glu Gln Lys
           275                 280                 285
   caa tca att aca tca ggg gat aat tca gaa gca aaa aca gat gtc act      912
   Gln Ser Ile Thr Ser Gly Asp Asn Ser Glu Ala Lys Thr Asp Val Thr
       290                 295                 300
   ctt att gcg ggt tcc agt gaa tat gat tta agc aaa cat gag ctg aaa      960
   Leu Ile Ala Gly Ser Ser Glu Tyr Asp Leu Ser Lys His Glu Leu Lys
   305                 310                 315                 320
   aaa acg agc ggt gaa aat gta tct aat gat gtt att gct atc acg gga     1008
   Lys Thr Ser Gly Glu Asn Val Ser Asn Asp Val Ile Ala Ile Thr Gly
                   325                 330                 335
   tct agt aca ggc gca atg cat ggt aaa aat att aag ttg att gtg aca     1056
   Ser Ser Thr Gly Ala Met His Gly Lys Asn Ile Lys Leu Ile Val Thr
               340                 345                 350
   gat aaa ggt gca ggc gta aaa cat gat gga att att ttg tct gaa aat     1104
   Asp Lys Gly Ala Gly Val Lys His Asp Gly Ile Ile Leu Ser Glu Asn
           355                 360                 365
   gat att cag att gaa atg aat gaa ggt gac tta gaa ctt ggc aat acg     1152
   Asp Ile Gln Ile Glu Met Asn Glu Gly Asp Leu Glu Leu Gly Asn Thr
       370                 375                 380
   att cag caa aca gtg gta aaa aaa gac cga aat att cga gcc aag aaa      1200
   Ile Gln Gln Thr Val Val Lys Lys Asp Arg Asn Ile Arg Ala Lys Lys
   385                 390                 395                 400
   aaa att gaa gtg aaa aac gct aat cgt gtt ttt gtt ggt agt caa acg      1248
   Lys Ile Glu Val Lys Asn Ala Asn Arg Val Phe Val Gly Ser Gln Thr
                   405                 410                 415
   aaa tca gat gaa att tcg tta gag gcg aaa caa gtt aaa atc aga aaa      1296
   Lys Ser Asp Glu Ile Ser Leu Glu Ala Lys Gln Val Lys Ile Arg Lys
               420                 425                 430
   aac gca gag att agg agt acg aca caa gcc aaa atc gta gca aag ggt      1344
   Asn Ala Glu Ile Arg Ser Thr Thr Gln Ala Lys Ile Val Ala Lys Gly
           435                 440                 445
   gcc ctg tct att gag caa aat gcg aag ctc gtc gct aaa aag ata gat      1392
   Ala Leu Ser Ile Glu Gln Asn Ala Lys Leu Val Ala Lys Lys Ile Asp
       450                 455                 460
   gtg gca aca gaa act cta act aat gct ggg cgt att tat ggt cga gag      1440
   Val Ala Thr Glu Thr Leu Thr Asn Ala Gly Arg Ile Tyr Gly Arg Glu
   465                 470                 475                 480
   gtt aag ctt gac act aat aat ttg att aat gat aaa gaa att tat gct      1488
   Val Lys Leu Asp Thr Asn Asn Leu Ile Asn Asp Lys Glu Ile Tyr Ala
                   485                 490                 495
   gaa cgg aaa ttg agt att ttg acg aaa gga aaa gat ctt gaa att att      1536
   Glu Arg Lys Leu Ser Ile Leu Thr Lys Gly Lys Asp Leu Glu Ile Ile
               500                 505                 510
   caa gat aga tat ttg tct cca ctg atg cgc gta aaa agt agt gtc cgc      1584
   Gln Asp Arg Tyr Leu Ser Pro Leu Met Arg Val Lys Ser Ser Val Arg
           515                 520                 525
   ttt tta ggc tct ccg ttt ttc tca ata tct ccg tcg atg ctc gca agc      1632
   Phe Leu Gly Ser Pro Phe Phe Ser Ile Ser Pro Ser Met Leu Ala Ser
       530                 535                 540
   ctt agt gca cag ttt aag cct ggt ttt gtg aat aag gga ctc att gaa      1680
   Leu Ser Ala Gln Phe Lys Pro Gly Phe Val Asn Lys Gly Leu Ile Glu
   545                 550                 555                 560
   agt gcg ggg agt gca gaa tta act ttt aaa gaa aaa acc agt ttt tta      1728
   Ser Ala Gly Ser Ala Glu Leu Thr Phe Lys Glu Lys Thr Ser Phe Leu
                   565                 570                 575
   aca gag ggc aat aat ttt att aga gct aaa gat gcg tta act att aac      1776
   Thr Glu Gly Asn Asn Phe Ile Arg Ala Lys Asp Ala Leu Thr Ile Asn
               580                 585                 590
   gcc caa aat att gaa att gat aaa aat caa gat att caa ttg ggt gct      1824
   Ala Gln Asn Ile Glu Ile Asp Lys Asn Gln Asp Ile Gln Leu Gly Ala
           595                 600                 605
   aat ata acg ttg aat gtg gaa gaa aac ttt gtt aat cgt gca gga aca      1872
   Asn Ile Thr Leu Asn Val Glu Glu Asn Phe Val Asn Arg Ala Gly Thr
       610                 615                 620
   ctg gca act ggt aaa aca ctg aca att aat acc gaa agt ggc agt att      1920
   Leu Ala Thr Gly Lys Thr Leu Thr Ile Asn Thr Glu Ser Gly Ser Ile
   625                 630                 635                 640
   tac aat ctt ggt ggg aca tta ggt gct gga aaa tca tta aaa ctg act      1968
   Tyr Asn Leu Gly Gly Thr Leu Gly Ala Gly Lys Ser Leu Lys Leu Thr
                   645                 650                 655
   gct aaa tca acg gaa gaa ggt atg gga aat att gtt aac caa gaa aac      2016
   Ala Lys Ser Thr Glu Glu Gly Met Gly Asn Ile Val Asn Gln Glu Asn
               660                 665                 670
   ggt tta ttc cat aca ctc ggt aat atg atg tta gaa gca gag cgt tct      2064
   Gly Leu Phe His Thr Leu Gly Asn Met Met Leu Glu Ala Glu Arg Ser
           675                 680                 685
   gtt tat aat att ggc gat att tat gcg agt aaa aaa tta aca gtt cat      2112
   Val Tyr Asn Ile Gly Asp Ile Tyr Ala Ser Lys Lys Leu Thr Val His
       690                 695                 700
   act cat aat ttg att aat gat gtg cgt tta tct ggc aat gtg agt tat      2160
   Thr His Asn Leu Ile Asn Asp Val Arg Leu Ser Gly Asn Val Ser Tyr
   705                 710                 715                 720
   aag cct atc ggt tca agt cgt gat tat gat atc agt cgt gtt gcg gta      2208
   Lys Pro Ile Gly Ser Ser Arg Asp Tyr Asp Ile Ser Arg Val Ala Val
                   725                 730                 735
   cat ggt tgg cac aat aat gtt tat aag ctc aac tta aat ctg caa gaa      2256
   His Gly Trp His Asn Asn Val Tyr Lys Leu Asn Leu Asn Leu Gln Glu
               740                 745                 750
   caa gat aaa acc gat att aaa gtt gtg aaa atg ggg gct atc cgt tct      2304
   Gln Asp Lys Thr Asp Ile Lys Val Val Lys Met Gly Ala Ile Arg Ser
           755                 760                 765
   gat ggt gat ttt gac ttt aag gga ata aag gcg aca tca tca gaa tca      2352
   Asp Gly Asp Phe Asp Phe Lys Gly Ile Lys Ala Thr Ser Ser Glu Ser
       770                 775                 780
   aaa ccg cag tta att aat cat gga tta att aat gtc aaa gga aca ttt      2400
   Lys Pro Gln Leu Ile Asn His Gly Leu Ile Asn Val Lys Gly Thr Phe
   785                 790                 795                 800
   aat gcg gaa gct gat caa gtg gtg aac caa atg aaa gcg ttt aac caa      2448
   Asn Ala Glu Ala Asp Gln Val Val Asn Gln Met Lys Ala Phe Asn Gln
                   805                 810                 815
   aat gca tta gca agc gtg ttt aag aat cca gcg aaa atc acg atg tac      2496
   Asn Ala Leu Ala Ser Val Phe Lys Asn Pro Ala Lys Ile Thr Met Tyr
               820                 825                 830
   tat caa cca ctt act cgt tat att tgg aca cca tta tcg ggt aat gca      2544
   Tyr Gln Pro Leu Thr Arg Tyr Ile Trp Thr Pro Leu Ser Gly Asn Ala
           835                 840                 845
   tcg cgt gaa ttt aac aat tta gag tct ttc ctc gat gcc ttg ttt ggc      2592
   Ser Arg Glu Phe Asn Asn Leu Glu Ser Phe Leu Asp Ala Leu Phe Gly
       850                 855                 860
   tca aca aca atc tta aaa tca agt ttc tat agt acg gaa aat ttt agt      2640
   Ser Thr Thr Ile Leu Lys Ser Ser Phe Tyr Ser Thr Glu Asn Phe Ser
   865                 870                 875                 880
   gct tat cag ctt cta tct cat att cag cat tca cca atg tac caa aaa      2688
   Ala Tyr Gln Leu Leu Ser His Ile Gln His Ser Pro Met Tyr Gln Lys
                   885                 890                 895
   gcg atg gca caa gtg ttt ggt gca gag tgg cat agt aaa tcc tat gat      2736
   Ala Met Ala Gln Val Phe Gly Ala Glu Trp His Ser Lys Ser Tyr Asp
               900                 905                 910
   gag atg cga aac aaa tgg aaa agc ttt aaa gaa aat cca aca gat ttc      2784
   Glu Met Arg Asn Lys Trp Lys Ser Phe Lys Glu Asn Pro Thr Asp Phe
           915                 920                 925
   att tat tac cca tca gaa aaa gca aaa atc cta gcg gga aaa cta gaa      2832
   Ile Tyr Tyr Pro Ser Glu Lys Ala Lys Ile Leu Ala Gly Lys Leu Glu
       930                 935                 940
   ggt aag ctt aca acg cta caa aat ggt gaa tat gcc gaa cgt ggt aag      2880
   Gly Lys Leu Thr Thr Leu Gln Asn Gly Glu Tyr Ala Glu Arg Gly Lys
   945                 950                 955                 960
   ttt gat gag agt atc caa att ggt aaa cac caa tta tcg cta cca tca      2928
   Phe Asp Glu Ser Ile Gln Ile Gly Lys His Gln Leu Ser Leu Pro Ser
                   965                 970                 975
   gta gag ctt aaa gcg gag ttt agt gat aaa gaa cgt ttg gaa gag gac      2976
   Val Glu Leu Lys Ala Glu Phe Ser Asp Lys Glu Arg Leu Glu Glu Asp
               980                 985                 990
   ggg gta gat tta tcc tcg atc gcc  gaa ctc tta gaa atg  cca aac tta    3024
   Gly Val Asp Leu Ser Ser Ile Ala  Glu Leu Leu Glu Met  Pro Asn Leu
           995                  1000                 1005
   ttt att  gat aat agt atc caa  tta gaa aag aaa aag  ttg tct cct       3069
   Phe Ile  Asp Asn Ser Ile Gln  Leu Glu Lys Lys Lys  Leu Ser Pro
       1010                 1015                 1020
   att gag  gat cta gat gaa gaa  cca cgt aaa aat ctg  gat ata gaa       3114
   Ile Glu  Asp Leu Asp Glu Glu  Pro Arg Lys Asn Leu  Asp Ile Glu
       1025                 1030                 1035
   gaa agc  cat tct aat tca tcg  gat gac gtg ctt agc  atg aat gat       3159
   Glu Ser  His Ser Asn Ser Ser  Asp Asp Val Leu Ser  Met Asn Asp
       1040                 1045                 1050
   gat gag  tct gat aca gac gat  agc aag tgg agt atg  ggc aat gat       3204
   Asp Glu  Ser Asp Thr Asp Asp  Ser Lys Trp Ser Met  Gly Asn Asp
       1055                 1060                 1065
   gag aaa  gag atg ccc gat gat  aag ctg ggt ata agt  cgt gat gat       3249
   Glu Lys  Glu Met Pro Asp Asp  Lys Leu Gly Ile Ser  Arg Asp Asp
       1070                 1075                 1080
   cgt gga  aat aaa cca cct cgt  act gat cct aca gtt  gat tat ctt       3294
   Arg Gly  Asn Lys Pro Pro Arg  Thr Asp Pro Thr Val  Asp Tyr Leu
       1085                 1090                 1095
   aac cct  gat gaa ttc ttt gaa  aat ggt tat ctc ttg  aat gag cta       3339
   Asn Pro  Asp Glu Phe Phe Glu  Asn Gly Tyr Leu Leu  Asn Glu Leu
       1100                 1105                 1110
   cta cag  gag ctt gga gaa gag  ccg tta cta aaa gaa  ggg gaa gat        3384
   Leu Gln  Glu Leu Gly Glu Glu  Pro Leu Leu Lys Glu  Gly Glu Asp
       1115                 1120                 1125
   cat ttt  aaa cgt tct acc aat  cta gtc cgt cta ggc  gag aga gat        3429
   His Phe  Lys Arg Ser Thr Asn  Leu Val Arg Leu Gly  Glu Arg Asp
       1130                 1135                 1140
   agg caa  aat aga gaa aag aga  gaa aaa gag ggg tat  ttt gat ctg        3474
   Arg Gln  Asn Arg Glu Lys Arg  Glu Lys Glu Gly Tyr  Phe Asp Leu
       1145                 1150                 1155
   cct ggt  aca tta gat atg aaa  ctg cag gag tta ttc  gaa aaa aga        3519
   Pro Gly  Thr Leu Asp Met Lys  Leu Gln Glu Leu Phe  Glu Lys Arg
       1160                 1165                 1170
   aaa caa  aaa cac gaa gca gaa  cag aaa gca aga ata  gaa aaa gca        3564
   Lys Gln  Lys His Glu Ala Glu  Gln Lys Ala Arg Ile  Glu Lys Ala
       1175                 1180                 1185
   ctt cta  caa aaa tca gaa caa  caa gaa aaa cgt gtt  gaa gaa cgt        3609
   Leu Leu  Gln Lys Ser Glu Gln  Gln Glu Lys Arg Val  Glu Glu Arg
       1190                 1195                 1200
   aag caa  gag gaa aaa cgt caa  gcg caa gat aaa att  gct aag caa        3654
   Lys Gln  Glu Glu Lys Arg Gln  Ala Gln Asp Lys Ile  Ala Lys Gln
       1205                 1210                 1215
   gta gaa  att gca aaa gaa atg  caa cgg gta gaa gaa  att cgc cag        3699
   Val Glu  Ile Ala Lys Glu Met  Gln Arg Val Glu Glu  Ile Arg Gln
       1220                 1225                 1230
   aga gaa  aaa caa ctt gcg atc  caa ctg caa gaa gaa  gag aag aaa        3744
   Arg Glu  Lys Gln Leu Ala Ile  Gln Leu Gln Glu Glu  Glu Lys Lys
       1235                 1240                 1245
   caa caa  gaa gaa aaa cat tta  tcc gag gag aaa aaa  caa gct gaa        3789
   Gln Gln  Glu Glu Lys His Leu  Ser Glu Glu Lys Lys  Gln Ala Glu
       1250                 1255                 1260
   cag aaa  caa aaa gct gag gag  aaa gtt gca caa gaa  aga tta gac        3834
   Gln Lys  Gln Lys Ala Glu Glu  Lys Val Ala Gln Glu  Arg Leu Asp
       1265                 1270                 1275
   att gaa  caa cag aaa gcg tat  gaa gaa atg gcg aag  cga gag gca        3879
   Ile Glu  Gln Gln Lys Ala Tyr  Glu Glu Met Ala Lys  Arg Glu Ala
       1280                 1285                 1290
   gag gca  tca aaa aat gtt tta  ttg aaa gcg att gat  gaa gaa cgt        3924
   Glu Ala  Ser Lys Asn Val Leu  Leu Lys Ala Ile Asp  Glu Glu Arg
       1295                 1300                 1305
   cca aaa  gtg gaa act gat cca  ctt ttc cgt aca aaa  ttg aaa tat        3969
   Pro Lys  Val Glu Thr Asp Pro  Leu Phe Arg Thr Lys  Leu Lys Tyr
       1310                 1315                 1320
   atc aat  caa gat gac tat gct  ggt gca aat tat ttc  ttc aat aaa        4014
   Ile Asn  Gln Asp Asp Tyr Ala  Gly Ala Asn Tyr Phe  Phe Asn Lys
           1325                1330                1335
   gtt ggt  tta aat aca aaa ggt  cat caa aaa gta aat  gtg tta ggg      4059
   Val Gly  Leu Asn Thr Lys Gly  His Gln Lys Val Asn  Val Leu Gly
       1340                1345                1350
   gat aac  tat ttt gat cat caa  gtg att act cgc tcg  att gag aaa      4104
   Asp Asn  Tyr Phe Asp His Gln  Val Ile Thr Arg Ser  Ile Glu Lys
       1355                1360                1365
   aaa gta  gat aac cac ctt aac  caa aaa tac aat ctc  agc gat gtg      4149
   Lys Val  Asp Asn His Leu Asn  Gln Lys Tyr Asn Leu  Ser Asp Val
       1370                1375                1380
   gaa tta  gtt aaa cag ctg atg  gac aat tcc aca aca  caa gcg cag      4194
   Glu Leu  Val Lys Gln Leu Met  Asp Asn Ser Thr Thr  Gln Ala Gln
       1385                1390                1395
   gag ttg  gat ttg aaa cta ggt  gcg gca tta act aaa  gaa caa caa      4239
   Glu Leu  Asp Leu Lys Leu Gly  Ala Ala Leu Thr Lys  Glu Gln Gln
       1400                1405                1410
   gct aac  ttg acc caa gat atc  gtt tgg tat gtc aaa  acg aag gta      4284
   Ala Asn  Leu Thr Gln Asp Ile  Val Trp Tyr Val Lys  Thr Lys Val
       1415                1420                1425
   aag ggc  aaa gat gtg ttt gtt  cca aag gtt tat ttc  gct tct gaa      4329
   Lys Gly  Lys Asp Val Phe Val  Pro Lys Val Tyr Phe  Ala Ser Glu
       1430                1435                1440
   acg ctc  gta gaa gcc caa aaa  tta caa ggt tta ggc  act ggg act      4374
   Thr Leu  Val Glu Ala Gln Lys  Leu Gln Gly Leu Gly  Thr Gly Thr
       1445                1450                1455
   atc aga  gtt ggt gaa gct aag  att aaa gcc aaa gat  gtg gtg aat      4419
   Ile Arg  Val Gly Glu Ala Lys  Ile Lys Ala Lys Asp  Val Val Asn
       1460                1465                1470
   acc ggg  aca tta gct ggg aga  aaa ctc aat gtt gaa  gcg agt aat      4464
   Thr Gly  Thr Leu Ala Gly Arg  Lys Leu Asn Val Glu  Ala Ser Asn
       1475                1480                1485
   aaa atc  aaa aat caa ggg agt  atc tta agt act caa  gaa aca cgt      4509
   Lys Ile  Lys Asn Gln Gly Ser  Ile Leu Ser Thr Gln  Glu Thr Arg
       1490                1495                1500
   tta gtc  ggg cgt aaa ggt att  gaa aac gta tct cgt  tca ttt gca      4554
   Leu Val  Gly Arg Lys Gly Ile  Glu Asn Val Ser Arg  Ser Phe Ala
       1505                1510                1515
   aat gat  gaa tta gga gtc act  gca caa cgc tca gaa  atc aaa acg      4599
   Asn Asp  Glu Leu Gly Val Thr  Ala Gln Arg Ser Glu  Ile Lys Thr
       1520                1525                1530
   gaa ggt  cat tta cat ctt gaa  aca gat aag gat tca  act att gat      4644
   Glu Gly  His Leu His Leu Glu  Thr Asp Lys Asp Ser  Thr Ile Asp
       1535                1540                1545
   gta caa  gca tcg gat att aaa  gca aaa aca agc ttt  gtg aag act      4689
   Val Gln  Ala Ser Asp Ile Lys  Ala Lys Thr Ser Phe  Val Lys Thr
       1550                1555                1560
   ggt gat  gtg aat ctc aaa aat  aca tac aat act aaa  cat gcc tac      4734
   Gly Asp  Val Asn Leu Lys Asn  Thr Tyr Asn Thr Lys  His Ala Tyr
       1565                 1570                 1575
   cgt gag  aaa ttc tcg ccg agt  gca cta caa gtt gca  gaa ctt gat      4779
   Arg Glu  Lys Phe Ser Pro Ser  Ala Leu Gln Val Ala  Glu Leu Asp
       1580                 1585                 1590
   gtg gca  ggg ctt aaa gtc cca  ctt tta ggc gtg tcc  gtc tcc atc      4824
   Val Ala  Gly Leu Lys Val Pro  Leu Leu Gly Val Ser  Val Ser Ile
       1595                 1600                 1605
   cag ttt  att cag agc ata cta  gtg agg caa ctt caa  gag gga tca      4869
   Gln Phe  Ile Gln Ser Ile Leu  Val Arg Gln Leu Gln  Glu Gly Ser
       1610                 1615                 1620
   atc ttc  gaa gta ggg cac tta  cat ntt gcg gta gac  aga aga tgt      4914
   Ile Phe  Glu Val Gly His Leu  His Xaa Ala Val Asp  Arg Arg Cys
       1625                 1630                 1635
   gaa cca  agc ggg gag ta                                             4931
   Glu Pro  Ser Gly Glu
       1640
<210> 103
   <211> 1643
   <212> PRT
   <213> Pasteurella Multocida
<220>
   <221> misc_feature
   <222> (1632) .. (1632)
   <223> The 'Xaa' at location 1632 stands for Ile, Val, Leu, or Phe.
<400> 103
   Met Asn Lys Asn Arg Tyr Lys Leu Ile Phe Ser Gln Val Lys Gly Cys
   1               5                   10                  15
   Leu Val Pro Val Ala Glu Cys Ile Asn Ser Ala Ile Ser Asn Gly Ser
               20                  25                  30
   Ser Asp Ser Thr Ser Thr Ser Glu Gln Val Glu Glu Glu Pro Phe Leu
           35                  40                  45
   Leu Glu Gln Tyr Ser Leu Ser Ser Val Ser Leu Leu Val Lys Ser Thr
       50                  55                  60
   Phe Asn Pro Val Ser Tyr Ala Met Gln Leu Thr Trp Lys Gln Leu Ser
   65                  70                  75                  80
   Ile Leu Phe Leu Thr Val Ile Ser Val Pro Val Leu Ala Glu Gly Lys
                   85                  90                  95
   Gly Asp Glu Arg Asn Gln Leu Thr Val Ile Asp Asn Ser Asp His Ile
               100                 105                 110
   Lys Leu Asp Ala Ser Asn Leu Ala Gly Asn Asp Lys Thr Lys Ile Tyr
           115                 120                 125
   Gln Ala Glu Asn Lys Val Leu Val Ile Asp Ile Ala Lys Pro Asn Gly
       130                 135                 140
   Lys Gly Ile Ser Asp Asn Arg Phe Glu Lys Phe Asn Ile Pro Asn Ser
   145                 150                 155                 160
   Ala Val Phe Asn Asn Asn Gly Thr Glu Ala Gln Ala Arg Ser Thr Leu
                   165                 170                 175
   Ile Gly Tyr Ile Pro Gln Asn Gln Asn Leu Arg Gly Gly Lys Glu Ala
               180                 185                 190
   Asp Val Ile Leu Asn Gln Val Thr Gly Pro Gln Glu Ser Lys Ile Val
           195                 200                 205
   Gly Ala Leu Glu Val Leu Gly Lys Lys Ala Asp Ile Val Ile Ala Asn
       210                 215                 220
   Gln Asn Gly Ile Thr Leu Asn Gly Val Arg Thr Ile Asn Ser Asp Arg
   225                 230                 235                 240
   Phe Val Ala Thr Thr Ser Glu Leu Ile Asp Pro Asn Gln Met Met Leu
                   245                 250                 255
   Lys Val Thr Lys Gly Asn Val Ile Ile Asp Ile Asp Gly Phe Ser Thr
               260                 265                 270
   Asp Gly Leu Lys Tyr Leu Asp Ile Ile Ala Lys Lys Ile Glu Gln Lys
           275                 280                 285
   Gln Ser Ile Thr Ser Gly Asp Asn Ser Glu Ala Lys Thr Asp Val Thr
       290                 295                 300
   Leu Ile Ala Gly Ser Ser Glu Tyr Asp Leu Ser Lys His Glu Leu Lys
   305                 310                 315                 320
   Lys Thr Ser Gly Glu Asn Val Ser Asn Asp Val Ile Ala Ile Thr Gly
                   325                 330                 335
   Ser Ser Thr Gly Ala Met His Gly Lys Asn Ile Lys Leu Ile Val Thr
               340                 345                 350
   Asp Lys Gly Ala Gly Val Lys His Asp Gly Ile Ile Leu Ser Glu Asn
           355                 360                 365
   Asp Ile Gln Ile Glu Met Asn Glu Gly Asp Leu Glu Leu Gly Asn Thr
       370                 375                 380
   Ile Gln Gln Thr Val Val Lys Lys Asp Arg Asn Ile Arg Ala Lys Lys
   385                 390                 395                 400
   Lys Ile Glu Val Lys Asn Ala Asn Arg Val Phe Val Gly Ser Gln Thr
                   405                 410                 415
   Lys Ser Asp Glu Ile Ser Leu Glu Ala Lys Gln Val Lys Ile Arg Lys
               420                 425                 430
   Asn Ala Glu Ile Arg Ser Thr Thr Gln Ala Lys Ile Val Ala Lys Gly
           435                 440                 445
   Ala Leu Ser Ile Glu Gln Asn Ala Lys Leu Val Ala Lys Lys Ile Asp
       450                 455                 460
   Val Ala Thr Glu Thr Leu Thr Asn Ala Gly Arg Ile Tyr Gly Arg Glu
   465                 470                 475                 480
   Val Lys Leu Asp Thr Asn Asn Leu Ile Asn Asp Lys Glu Ile Tyr Ala
                   485                 490                 495
   Glu Arg Lys Leu Ser Ile Leu Thr Lys Gly Lys Asp Leu Glu Ile Ile
               500                 505                 510
   Gln Asp Arg Tyr Leu Ser Pro Leu Met Arg Val Lys Ser Ser Val Arg
           515                 520                 525
   Phe Leu Gly Ser Pro Phe Phe Ser Ile Ser Pro Ser Met Leu Ala Ser
       530                 535                 540
   Leu Ser Ala Gln Phe Lys Pro Gly Phe Val Asn Lys Gly Leu Ile Glu
   545                 550                 555                 560
   Ser Ala Gly Ser Ala Glu Leu Thr Phe Lys Glu Lys Thr Ser Phe Leu
                   565                 570                 575
   Thr Glu Gly Asn Asn Phe Ile Arg Ala Lys Asp Ala Leu Thr Ile Asn
   580 585 590
   Ala Gln Asn Ile Glu Ile Asp Lys Asn Gln Asp Ile Gln Leu Gly Ala
   595 600 605
   Asn Ile Thr Leu Asn Val Glu Glu Asn Phe Val Asn Arg Ala Gly Thr
   610 615 620
   Leu Ala Thr Gly Lys Thr Leu Thr Ile Asn Thr Glu Ser Gly Ser Ile
   625 630 635 640
   Tyr Asn Leu Gly Gly Thr Leu Gly Ala Gly Lys Ser Leu Lys Leu Thr
   645 650 655
   Ala Lys Ser Thr Glu Glu Gly Met Gly Asn Ile Val Asn Gln Glu Asn
   660 665 670
   Gly Leu Phe His Thr Leu Gly Asn Met Met Leu Glu Ala Glu Arg Ser
   675 680 685
   Val Tyr Asn Ile Gly Asp Ile Tyr Ala Ser Lys Lys Leu Thr Val His
   690 695 700
   Thr His Asn Leu Ile Asn Asp Val Arg Leu Ser Gly Asn Val Ser Tyr
   705 710 715 720
   Lys Pro Ile Gly Ser Ser Arg Asp Tyr Asp Ile Ser Arg Val Ala Val
   725 730 735
   His Gly Trp His Asn Asn Val Tyr Lys Leu Asn Leu Asn Leu Gln Glu
   740 745 750
   Gln Asp Lys Thr Asp Ile Lys Val Val Lys Met Gly Ala Ile Arg Ser
   755 760 765
   Asp Gly Asp Phe Asp Phe Lys Gly Ile Lys Ala Thr Ser Ser Glu Ser
   770 775 780
   Lys Pro Gln Leu Ile Asn His Gly Leu Ile Asn Val Lys Gly Thr Phe
   785 790 795 800
   Asn Ala Glu Ala Asp Gln Val Val Asn Gln Met Lys Ala Phe Asn Gln
   805 810 815
   Asn Ala Leu Ala Ser Val Phe Lys Asn Pro Ala Lys Ile Thr Met Tyr
   820 825 830
   Tyr Gln Pro Leu Thr Arg Tyr Ile Trp Thr Pro Leu Ser Gly Asn Ala
           835                 840                 845
   Ser Arg Glu Phe Asn Asn Leu Glu Ser Phe Leu Asp Ala Leu Phe Gly
       850                 855                 860
   Ser Thr Thr Ile Leu Lys Ser Ser Phe Tyr Ser Thr Glu Asn Phe Ser
   865                 870                 875                 880
   Ala Tyr Gln Leu Leu Ser His Ile Gln His Ser Pro Met Tyr Gln Lys
                   885                 890                 895
   Ala Met Ala Gln Val Phe Gly Ala Glu Trp His Ser Lys Ser Tyr Asp
               900                 905                 910
   Glu Met Arg Asn Lys Trp Lys Ser Phe Lys Glu Asn Pro Thr Asp Phe
           915                 920                 925
   Ile Tyr Tyr Pro Ser Glu Lys Ala Lys Ile Leu Ala Gly Lys Leu Glu
       930                 935                 940
   Gly Lys Leu Thr Thr Leu Gln Asn Gly Glu Tyr Ala Glu Arg Gly Lys
   945                 950                 955                 960
   Phe Asp Glu Ser Ile Gln Ile Gly Lys His Gln Leu Ser Leu Pro Ser
                   965                 970                 975
   Val Glu Leu Lys Ala Glu Phe Ser Asp Lys Glu Arg Leu Glu Glu Asp
               980                 985                 990
   Gly Val Asp Leu Ser Ser Ile Ala  Glu Leu Leu Glu Met  Pro Asn Leu
           995                 1000                 1005
   Phe Ile  Asp Asn Ser Ile Gln  Leu Glu Lys Lys Lys  Leu Ser Pro
       1010                 1015                 1020
   Ile Glu  Asp Leu Asp Glu Glu  Pro Arg Lys Asn Leu  Asp Ile Glu
       1025                 1030                 1035
   Glu Ser  His Ser Asn Ser Ser  Asp Asp Val Leu Ser  Met Asn Asp
       1040                 1045                 1050
   Asp Glu  Ser Asp Thr Asp Asp  Ser Lys Trp Ser Met  Gly Asn Asp
       1055                 1060                 1065
   Glu Lys Glu Met Pro Asp Asp Lys Leu Gly Ile Ser Arg Asp Asp
   1070 1075 1080
   Arg Gly Asn Lys Pro Pro Arg Thr Asp Pro Thr Val Asp Tyr Leu
   1085 1090 1095
   Asn Pro Asp Glu Phe Phe Glu Asn Gly Tyr Leu Leu Asn Glu Leu
   1100 1105 1110
   Leu Gln Glu Leu Gly Glu Glu Pro Leu Leu Lys Glu Gly Glu Asp
   1115 1120 1125
   His Phe Lys Arg Ser Thr Asn Leu Val Arg Leu Gly Glu Arg Asp
   1130 1135 1140
   Arg Gln Asn Arg Glu Lys Arg Glu Lys Glu Gly Tyr Phe Asp Leu
   1145 1150 1155
   Pro Gly Thr Leu Asp Met Lys Leu Gln Glu Leu Phe Glu Lys Arg
   1160 1165 1170
   Lys Gln Lys His Glu Ala Glu Gln Lys Ala Arg Ile Glu Lys Ala
   1175 1180 1185
   Leu Leu Gln Lys Ser Glu Gln Gln Glu Lys Arg Val Glu Glu Arg
   1190 1195 1200
   Lys Gln Glu Glu Lys Arg Gln Ala Gln Asp Lys Ile Ala Lys Gln
   1205 1210 1215
   Val Glu Ile Ala Lys Glu Met Gln Arg Val Glu Glu Ile Arg Gln
   1220 1225 1230
   Arg Glu Lys Gln Leu Ala Ile Gln Leu Gln Glu Glu Glu Lys Lys
   1235 1240 1245
   Gln Gln Glu Glu Lys His Leu Ser Glu Glu Lys Lys Gln Ala Glu
   1250 1255 1260
   Gln Lys Gln Lys Ala Glu Glu Lys Val Ala Gln Glu Arg Leu Asp
   1265 1270 1275
   Ile Glu Gln Gln Lys Ala Tyr Glu Glu Met Ala Lys Arg Glu Ala
   1280 1285 1290
   Glu Ala  Ser Lys Asn Val Leu  Leu Lys Ala Ile Asp  Glu Glu Arg
       1295                 1300                 1305
   Pro Lys  Val Glu Thr Asp Pro  Leu Phe Arg Thr Lys  Leu Lys Tyr
       1310                 1315                 1320
   Ile Asn  Gln Asp Asp Tyr Ala  Gly Ala Asn Tyr Phe  Phe Asn Lys
       1325                 1330                 1335
   Val Gly  Leu Asn Thr Lys Gly  His Gln Lys Val Asn  Val Leu Gly
       1340                 1345                 1350
   Asp Asn  Tyr Phe Asp His Gln  Val Ile Thr Arg Ser  Ile Glu Lys
       1355                 1360                 1365
   Lys Val  Asp Asn His Leu Asn  Gln Lys Tyr Asn Leu  Ser Asp Val
       1370                 1375                 1380
   Glu Leu  Val Lys Gln Leu Met  Asp Asn Ser Thr Thr  Gln Ala Gln
       1385                 1390                 1395
   Glu Leu  Asp Leu Lys Leu Gly  Ala Ala Leu Thr Lys  Glu Gln Gln
       1400                 1405                 1410
   Ala Asn  Leu Thr Gln Asp Ile  Val Trp Tyr Val Lys  Thr Lys Val
       1415                 1420                 1425
   Lys Gly  Lys Asp Val Phe Val  Pro Lys Val Tyr Phe  Ala Ser Glu
       1430                 1435                 1440
   Thr Leu  Val Glu Ala Gln Lys  Leu Gln Gly Leu Gly  Thr Gly Thr
       1445                 1450                 1455
   Ile Arg  Val Gly Glu Ala Lys  Ile Lys Ala Lys Asp  Val Val Asn
       1460                 1465                 1470
   Thr Gly  Thr Leu Ala Gly Arg  Lys Leu Asn Val Glu  Ala Ser Asn
       1475                 1480                 1485
   Lys Ile  Lys Asn Gln Gly Ser  Ile Leu Ser Thr Gln  Glu Thr Arg
       1490                 1495                 1500
   Leu Val  Gly Arg Lys Gly Ile  Glu Asn Val Ser Arg  Ser Phe Ala
       1505                 1510                 1515
   Asn Asp  Glu Leu Gly Val Thr  Ala Gln Arg Ser Glu  Ile Lys Thr
   1520 1525 1530
   Glu Gly His Leu His Leu Glu Thr Asp Lys Asp Ser Thr Ile Asp
   1535 1540 1545
   Val Gln Ala Ser Asp Ile Lys Ala Lys Thr Ser Phe Val Lys Thr
   1550 1555 1560
   Gly Asp Val Asn Leu Lys Asn Thr Tyr Asn Thr Lys His Ala Tyr
   1565 1570 1575
   Arg Glu Lys Phe Ser Pro Ser Ala Leu Gln Val Ala Glu Leu Asp
   1580 1585 1590
   Val Ala Gly Leu Lys Val Pro Leu Leu Gly Val Ser Val Ser Ile
   1595 1600 1605
   Gln Phe Ile Gln Ser Ile Leu Val Arg Gln Leu Gln Glu Gly Ser
   1610 1615 1620
   Ile Phe Glu Val Gly His Leu His Xaa Ala Val Asp Arg Arg Cys
   1625 1630 1635
   Glu Pro Ser Gly Glu
   1640
<210> 104
   <211> 2009
   <212> DNA
   <213> Pasteurella Multocida
<220>
   <221> misc_feature
   <223> hmbR
<220>
   <221> CDS
   <222> (1) .. (2007)
<400> 104
   atc cgt ggc gtt gat aaa gat cgt gtc gct gtt att gtt gat gga ata 48
   Ile Arg Gly Val Asp Lys Asp Arg Val Ala Val Ile Val Asp Gly Ile
   1 5 10 15
   ccg cag gct gaa tcg act ata tct act tcc gca cgt tat tcg act gaa 96
   Pro Gln Ala Glu Ser Thr Ile Ser Thr Ser Ala Arg Tyr Ser Thr Glu
   20 25 30
   cgt cat aat ggt aat att aat aat att gaa tac gaa aat gtt agt tcg 144
   Arg His Asn Gly Asn Ile Asn Asn Ile Glu Tyr Glu Asn Val Ser Ser
   35 40 45
   ttg aaa gtt caa aaa ggg gca gct tct gta atg tat ggt agc ggt gcg      192
   Leu Lys Val Gln Lys Gly Ala Ala Ser Val Met Tyr Gly Ser Gly Ala
       50                  55                  60
   tta ggt gga acc gtg gag ttt acc aca aaa gat att gag gac ttt gtc      240
   Leu Gly Gly Thr Val Glu Phe Thr Thr Lys Asp Ile Glu Asp Phe Val
   65                  70                  75                  80
   gaa cct ggt cgc cat ttg ggc ttt ttg tct aaa acc ggc tat act tca      288
   Glu Pro Gly Arg His Leu Gly Phe Leu Ser Lys Thr Gly Tyr Thr Ser
                   85                  90                  95
   aaa aac aga gaa tat cgt caa gtc atc gga gtt gga ggg aaa ggg gaa      336
   Lys Asn Arg Glu Tyr Arg Gln Val Ile Gly Val Gly Gly Lys Gly Glu
               100                 105                 110
   cac ttt ttt ggt ttt gta caa tta acc aaa cgt tgg ggg cat gaa aca      384
   His Phe Phe Gly Phe Val Gln Leu Thr Lys Arg Trp Gly His Glu Thr
           115                 120                 125
   atc aac aac ggc aaa ggt aca gac att ctc ggc gaa cat cga ggt aaa      432
   Ile Asn Asn Gly Lys Gly Thr Asp Ile Leu Gly Glu His Arg Gly Lys
       130                 135                 140
   ccc aat ccg ctc aac tac tat act aca tca tgg tta acg aaa gtc ggt      480
   Pro Asn Pro Leu Asn Tyr Tyr Thr Thr Ser Trp Leu Thr Lys Val Gly
   145                 150                 155                 160
   tac gat att aat aac act cat cgt ttt aca ctg ttt tta gaa gat cgc      528
   Tyr Asp Ile Asn Asn Thr His Arg Phe Thr Leu Phe Leu Glu Asp Arg
                   165                 170                 175
   cgt gaa aag aag ctt acc gaa gaa aaa aca tta ggg ctt agt gat gca      576
   Arg Glu Lys Lys Leu Thr Glu Glu Lys Thr Leu Gly Leu Ser Asp Ala
               180                 185                 190
   gtg cgt ttt gct aat gat caa acc cct tat ctc cgt tat ggt att gaa      624
   Val Arg Phe Ala Asn Asp Gln Thr Pro Tyr Leu Arg Tyr Gly Ile Glu
           195                 200                 205
   tat cga tat aac ggc ttg tct tgg ttg gaa acg gta aag ctt ttt ttg      672
   Tyr Arg Tyr Asn Gly Leu Ser Trp Leu Glu Thr Val Lys Leu Phe Leu
       210                 215                 220
   gca aag cag aaa atc gaa caa cgt tct gct ctc caa gag ttt gat att      720
   Ala Lys Gln Lys Ile Glu Gln Arg Ser Ala Leu Gln Glu Phe Asp Ile
   225                 230                 235                 240
   aat aat agg aat aaa ttg gat tcg act atg tcg ttt gta tat tta caa      768
   Asn Asn Arg Asn Lys Leu Asp Ser Thr Met Ser Phe Val Tyr Leu Gln
                   245                 250                 255
   aga cag aat ata gct cgg gga gaa ttt tca acg agt cct tta tat tgg      816
   Arg Gln Asn Ile Ala Arg Gly Glu Phe Ser Thr Ser Pro Leu Tyr Trp
               260                 265                 270
   ggg ccg agt cgc cat cgt tta tct gcg aaa ttc gaa ttt cgt gat aag      864
   Gly Pro Ser Arg His Arg Leu Ser Ala Lys Phe Glu Phe Arg Asp Lys
           275                 280                 285
   ttt tta gaa aat atg aat aag cat ttt acg ttt cgg ccg tgg caa atc      912
   Phe Leu Glu Asn Met Asn Lys His Phe Thr Phe Arg Pro Trp Gln Ile
       290                 295                 300
   aat aga ttc aga caa caa ggt cga aat aac tat aca gaa gtg ttt ccc      960
   Asn Arg Phe Arg Gln Gln Gly Arg Asn Asn Tyr Thr Glu Val Phe Pro
   305                 310                 315                 320
   gtt aaa tcc cga gag ttt tct ttt tct ctt atg gac gac att aag att     1008
   Val Lys Ser Arg Glu Phe Ser Phe Ser Leu Met Asp Asp Ile Lys Ile
                   325                 330                 335
   ggc gaa ttg cta cat ctc gga ttg ggc ggt cgg tgg gat cac tat aac     1056
   Gly Glu Leu Leu His Leu Gly Leu Gly Gly Arg Trp Asp His Tyr Asn
               340                 345                 350
   tat aag cca tta tta aat tct cag cat aat atc aac agg aca cag aga     1104
   Tyr Lys Pro Leu Leu Asn Ser Gln His Asn Ile Asn Arg Thr Gln Arg
           355                 360                 365
   tta cct tat cca aaa aca tca tcc aaa ttt tcg tat caa ttg agt tta     1152
   Leu Pro Tyr Pro Lys Thr Ser Ser Lys Phe Ser Tyr Gln Leu Ser Leu
       370                 375                 380
   gag tat caa tta cat cca tca cat caa att gca tac cgt tta agt acc     1200
   Glu Tyr Gln Leu His Pro Ser His Gln Ile Ala Tyr Arg Leu Ser Thr
   385                 390                 395                 400
   ggt ttt agg gtt ccc cgt gtt gaa gat ctt tat ttt gaa gac cga gga     1248
   Gly Phe Arg Val Pro Arg Val Glu Asp Leu Tyr Phe Glu Asp Arg Gly
                   405                 410                 415
   aaa agt tct tca caa ttt ctt cct aac ccc gat cta caa ccg gaa act     1296
   Lys Ser Ser Ser Gln Phe Leu Pro Asn Pro Asp Leu Gln Pro Glu Thr
               420                 425                 430
   gca ctg aat cat gaa ata agt tac cgt ttc caa aat caa tat gcc cat     1344
   Ala Leu Asn His Glu Ile Ser Tyr Arg Phe Gln Asn Gln Tyr Ala His
           435                 440                 445
   ttc agc gtc ggg ctt ttc cgt aca cgt tat cat aac ttt att caa gaa     1392
   Phe Ser Val Gly Leu Phe Arg Thr Arg Tyr His Asn Phe Ile Gln Glu
       450                 455                 460
   cgt gag atg acc tgt gat aaa att cca tat gag tat aat agg act tat     1440
   Arg Glu Met Thr Cys Asp Lys Ile Pro Tyr Glu Tyr Asn Arg Thr Tyr
   465                 470                 475                 480
   gga tat tgc acg cat aat act tat gta atg ttt gtt aat gaa cct gaa     1488
   Gly Tyr Cys Thr His Asn Thr Tyr Val Met Phe Val Asn Glu Pro Glu
                   485                 490                 495
   gcc gtg att aaa ggg gtt gaa gta agc ggt gct tta aat ggg tcg gca     1536
   Ala Val Ile Lys Gly Val Glu Val Ser Gly Ala Leu Asn Gly Ser Ala
               500                 505                 510
   ttc gga ctt tcc gac ggt tta act ttc cgt ctc aaa ggg agc tac agc     1584
   Phe Gly Leu Ser Asp Gly Leu Thr Phe Arg Leu Lys Gly Ser Tyr Ser
           515                 520                 525
   aaa ggt caa aat cat gac ggc gat ccg tta aaa tct att caa cca tgg     1632
   Lys Gly Gln Asn His Asp Gly Asp Pro Leu Lys Ser Ile Gln Pro Trp
       530                 535                 540
   aca gtg gta acc ggt att gat tac gaa act gaa ggg tgg agc gtg agt      1680
   Thr Val Val Thr Gly Ile Asp Tyr Glu Thr Glu Gly Trp Ser Val Ser
   545                 550                 555                 560
   ttg agc ggg cgt tat agt gcg gct aaa aaa gcc aaa gat gcg ata gaa      1728
   Leu Ser Gly Arg Tyr Ser Ala Ala Lys Lys Ala Lys Asp Ala Ile Glu
                   565                 570                 575
   acg gaa tac aca cat gat aaa aag gtt gtc aaa caa tgg ccg cat tta      1776
   Thr Glu Tyr Thr His Asp Lys Lys Val Val Lys Gln Trp Pro His Leu
               580                 585                 590
   agt cca tcc tac ttt gtt gtt gat ttt acg ggg caa gtt aac ctc agt      1824
   Ser Pro Ser Tyr Phe Val Val Asp Phe Thr Gly Gln Val Asn Leu Ser
           595                 600                 605
   aaa aat gtc att ttg aat atg ggg gta ttt aac ttg ttc aat cgt gat      1872
   Lys Asn Val Ile Leu Asn Met Gly Val Phe Asn Leu Phe Asn Arg Asp
       610                 615                 620
   tat atg acg tgg gac agt gca tat aac ttg ttt act agg ggg tat act      1920
   Tyr Met Thr Trp Asp Ser Ala Tyr Asn Leu Phe Thr Arg Gly Tyr Thr
   625                 630                 635                 640
   tcc cgt tct gtc cgt gct aac agc cca ggc att aat cgg ttt acc gca      1968
   Ser Arg Ser Val Arg Ala Asn Ser Pro Gly Ile Asn Arg Phe Thr Ala
                   645                 650                 655
   cca aaa cgt aat ttt gct gcc tcg gtg gaa att cgt ttt ta               2009
   Pro Lys Arg Asn Phe Ala Ala Ser Val Glu Ile Arg Phe
               660                 665
<210> 105
   <211> 669
   <212> PRT
   <213> Pasteurella Multocida
<400> 105
   Ile Arg Gly Val Asp Lys Asp Arg Val Ala Val Ile Val Asp Gly Ile
   1               5                   10                  15
   Pro Gln Ala Glu Ser Thr Ile Ser Thr Ser Ala Arg Tyr Ser Thr Glu
               20                  25                  30
   Arg His Asn Gly Asn Ile Asn Asn Ile Glu Tyr Glu Asn Val Ser Ser
           35                  40                  45
   Leu Lys Val Gln Lys Gly Ala Ala Ser Val Met Tyr Gly Ser Gly Ala
       50                  55                  60
   Leu Gly Gly Thr Val Glu Phe Thr Thr Lys Asp Ile Glu Asp Phe Val
   65                  70                  75                  80
   Glu Pro Gly Arg His Leu Gly Phe Leu Ser Lys Thr Gly Tyr Thr Ser
                   85                  90                  95
   Lys Asn Arg Glu Tyr Arg Gln Val Ile Gly Val Gly Gly Lys Gly Glu
               100                 105                 110
   His Phe Phe Gly Phe Val Gln Leu Thr Lys Arg Trp Gly His Glu Thr
           115                 120                 125
   Ile Asn Asn Gly Lys Gly Thr Asp Ile Leu Gly Glu His Arg Gly Lys
       130                 135                 140
   Pro Asn Pro Leu Asn Tyr Tyr Thr Thr Ser Trp Leu Thr Lys Val Gly
   145                 150                 155                 160
   Tyr Asp Ile Asn Asn Thr His Arg Phe Thr Leu Phe Leu Glu Asp Arg
                   165                 170                 175
   Arg Glu Lys Lys Leu Thr Glu Glu Lys Thr Leu Gly Leu Ser Asp Ala
               180                 185                 190
   Val Arg Phe Ala Asn Asp Gln Thr Pro Tyr Leu Arg Tyr Gly Ile Glu
           195                 200                 205
   Tyr Arg Tyr Asn Gly Leu Ser Trp Leu Glu Thr Val Lys Leu Phe Leu
       210                 215                 220
   Ala Lys Gln Lys Ile Glu Gln Arg Ser Ala Leu Gln Glu Phe Asp Ile
   225                 230                 235                 240
   Asn Asn Arg Asn Lys Leu Asp Ser Thr Met Ser Phe Val Tyr Leu Gln
                   245                 250                 255
   Arg Gln Asn Ile Ala Arg Gly Glu Phe Ser Thr Ser Pro Leu Tyr Trp
               260                 265                 270
   Gly Pro Ser Arg His Arg Leu Ser Ala Lys Phe Glu Phe Arg Asp Lys
           275                 280                 285
   Phe Leu Glu Asn Met Asn Lys His Phe Thr Phe Arg Pro Trp Gln Ile
       290                 295                 300
   Asn Arg Phe Arg Gln Gln Gly Arg Asn Asn Tyr Thr Glu Val Phe Pro
   305                 310                 315                 320
   Val Lys Ser Arg Glu Phe Ser Phe Ser Leu Met Asp Asp Ile Lys Ile
   325 330 335
   Gly Glu Leu Leu His Leu Gly Leu Gly Gly Arg Trp Asp His Tyr Asn
   340 345 350
   Tyr Lys Pro Leu Leu Asn Ser Gln His Asn Ile Asn Arg Thr Gln Arg
   355 360 365
   Leu Pro Tyr Pro Lys Thr Ser Ser Lys Phe Ser Tyr Gln Leu Ser Leu
   370 375 380
   Glu Tyr Gln Leu His Pro Ser His Gln Ile Ala Tyr Arg Leu Ser Thr
   385 390 395 400
   Gly Phe Arg Val Pro Arg Val Glu Asp Leu Tyr Phe Glu Asp Arg Gly
   405 410 415
   Lys Ser Ser Ser Gln Phe Leu Pro Asn Pro Asp Leu Gln Pro Glu Thr
   420 425 430
   Ala Leu Asn His Glu Ile Ser Tyr Arg Phe Gln Asn Gln Tyr Ala His
   435 440 445
   Phe Ser Val Gly Leu Phe Arg Thr Arg Tyr His Asn Phe Ile Gln Glu
   450 455 460
   Arg Glu Met Thr Cys Asp Lys Ile Pro Tyr Glu Tyr Asn Arg Thr Tyr
   465 470 475 480
   Gly Tyr Cys Thr His Asn Thr Tyr Val Met Phe Val Asn Glu Pro Glu
   485 490 495
   Ala Val Ile Lys Gly Val Glu Val Ser Gly Ala Leu Asn Gly Ser Ala
   500 505 510
   Phe Gly Leu Ser Asp Gly Leu Thr Phe Arg Leu Lys Gly Ser Tyr Ser
   515 520 525
   Lys Gly Gln Asn His Asp Gly Asp Pro Leu Lys Ser Ile Gln Pro Trp
   530 535 540
   Thr Val Val Thr Gly Ile Asp Tyr Glu Thr Glu Gly Trp Ser Val Ser
   545 550 555 560
   Leu Ser Gly Arg Tyr Ser Ala Ala Lys Lys Ala Lys Asp Ala Ile Glu
                   565                 570                 575
   Thr Glu Tyr Thr His Asp Lys Lys Val Val Lys Gln Trp Pro His Leu
               580                 585                 590
   Ser Pro Ser Tyr Phe Val Val Asp Phe Thr Gly Gln Val Asn Leu Ser
           595                 600                 605
   Lys Asn Val Ile Leu Asn Met Gly Val Phe Asn Leu Phe Asn Arg Asp
       610                 615                 620
   Tyr Met Thr Trp Asp Ser Ala Tyr Asn Leu Phe Thr Arg Gly Tyr Thr
   625                 630                 635                 640
   Ser Arg Ser Val Arg Ala Asn Ser Pro Gly Ile Asn Arg Phe Thr Ala
                   645                 650                 655
   Pro Lys Arg Asn Phe Ala Ala Ser Val Glu Ile Arg Phe
               660                 665
<210> 106
   <211> 908
   <212> DNA
   <213> Pasteurella Multocida
<220>
   <221> misc_feature
   <223> lgtC
<220>
   <221> CDS
   <222> (1) .. (906)
<400> 106
   atg aat att tta ttt gtt tct gat gat gtt tat gct aaa cat ctg gtg       48
   Met Asn Ile Leu Phe Val Ser Asp Asp Val Tyr Ala Lys His Leu Val
   1               5                   10                  15
   gtt gcg att aaa agc att ata aat cat aat gaa aaa ggt att tca ttt       96
   Val Ala Ile Lys Ser Ile Ile Asn His Asn Glu Lys Gly Ile Ser Phe
               20                  25                  30
   tat att ttt gat ttg ggt ata aag gat gaa aat aag aga aat att aat      144
   Tyr Ile Phe Asp Leu Gly Ile Lys Asp Glu Asn Lys Arg Asn Ile Asn
           35                  40                  45
   gat att gtt tct tct tat gga agt gaa gtc aac ttt att gct gtg aat      192
   Asp Ile Val Ser Ser Tyr Gly Ser Glu Val Asn Phe Ile Ala Val Asn
       50                  55                  60
   gag aaa gaa ttt gag agt ttt cct gtt caa att agt tat att tct tta      240
   Glu Lys Glu Phe Glu Ser Phe Pro Val Gln Ile Ser Tyr Ile Ser Leu
   65                  70                  75                  80
   gca aca tat gca agg cta aaa gcg gca gag tat ttg ccg gat aat tta         288
   Ala Thr Tyr Ala Arg Leu Lys Ala Ala Glu Tyr Leu Pro Asp Asn Leu
                   85                  90                  95
   aat aaa att att tat tta gat gtt gat gtt ttg gtt ttt aac tca tta         336
   Asn Lys Ile Ile Tyr Leu Asp Val Asp Val Leu Val Phe Asn Ser Leu
               100                 105                 110
   gaa atg tta tgg aat gtt gat gtt aat aat ttt ctt acc gca gcc tgt         384
   Glu Met Leu Trp Asn Val Asp Val Asn Asn Phe Leu Thr Ala Ala Cys
           115                 120                 125
   tat gat tct ttc atc gaa aat gaa aag tct gag cat aaa aaa tcg att         432
   Tyr Asp Ser Phe Ile Glu Asn Glu Lys Ser Glu His Lys Lys Ser Ile
       130                 135                 140
   tca atg tca gat aag gaa tat tat ttt aat gca gga gta atg cta ttt         480
   Ser Met Ser Asp Lys Glu Tyr Tyr Phe Asn Ala Gly Val Met Leu Phe
   145                 150                 155                 160
   aat tta gat gaa tgg cgg aag atg gat gta ttc tca aga gct tta gac         528
   Asn Leu Asp Glu Trp Arg Lys Met Asp Val Phe Ser Arg Ala Leu Asp
                   165                 170                 175
   ctg tta gct atg tat cct aat caa atg att tat cag gat caa gat ata         576
   Leu Leu Ala Met Tyr Pro Asn Gln Met Ile Tyr Gln Asp Gln Asp Ile
               180                 185                 190
   ttg aat atc ctt ttt agg aat aaa gtc tgt tat tta gat tgc aga ttt         624
   Leu Asn Ile Leu Phe Arg Asn Lys Val Cys Tyr Leu Asp Cys Arg Phe
           195                 200                 205
   aat ttc atg cca aat caa ctt gaa aga ata aaa caa tac cat aaa gga         672
   Asn Phe Met Pro Asn Gln Leu Glu Arg Ile Lys Gln Tyr His Lys Gly
       210                 215                 220
   aaa ttg agc aac tta cat tct tta gaa aaa aca acg atg cct gtc gtt         720
   Lys Leu Ser Asn Leu His Ser Leu Glu Lys Thr Thr Met Pro Val Val
   225                 230                 235                 240
   att tca cat tat tgt ggt cca gaa aaa gcg tgg cat gcg gat tgt aaa         768
   Ile Ser His Tyr Cys Gly Pro Glu Lys Ala Trp His Ala Asp Cys Lys
                   245                 250                 255
   cat ttt aat gta tat ttc tat cag aaa ata tta gca gaa ata acg aga         816
   His Phe Asn Val Tyr Phe Tyr Gln Lys Ile Leu Ala Glu Ile Thr Arg
               260                 265                 270
   ggc acg gat aaa gaa cgc gta tta tct ata aaa act tat ctc aag gcc         864
   Gly Thr Asp Lys Glu Arg Val Leu Ser Ile Lys Thr Tyr Leu Lys Ala
           275                 280                 285
   ttg att aga agg att aga tat aaa ttc aaa tat caa gtc tat ta              908
   Leu Ile Arg Arg Ile Arg Tyr Lys Phe Lys Tyr Gln Val Tyr
       290                 295                 300
<210> 107
   <211> 302
   <212> PRT
   <213> Pasteurella Multocida
<400> 107
   Met Asn Ile Leu Phe Val Ser Asp Asp Val Tyr Ala Lys His Leu Val
   1               5                   10                  15
   Val Ala Ile Lys Ser Ile Ile Asn His Asn Glu Lys Gly Ile Ser Phe
               20                  25                  30
   Tyr Ile Phe Asp Leu Gly Ile Lys Asp Glu Asn Lys Arg Asn Ile Asn
           35                  40                  45
   Asp Ile Val Ser Ser Tyr Gly Ser Glu Val Asn Phe Ile Ala Val Asn
       50                  55                  60
   Glu Lys Glu Phe Glu Ser Phe Pro Val Gln Ile Ser Tyr Ile Ser Leu
   65                  70                  75                  80
   Ala Thr Tyr Ala Arg Leu Lys Ala Ala Glu Tyr Leu Pro Asp Asn Leu
                   85                  90                  95
   Asn Lys Ile Ile Tyr Leu Asp Val Asp Val Leu Val Phe Asn Ser Leu
               100                 105                 110
   Glu Met Leu Trp Asn Val Asp Val Asn Asn Phe Leu Thr Ala Ala Cys
           115                 120                 125
   Tyr Asp Ser Phe Ile Glu Asn Glu Lys Ser Glu His Lys Lys Ser Ile
       130                 135                 140
   Ser Met Ser Asp Lys Glu Tyr Tyr Phe Asn Ala Gly Val Met Leu Phe
   145                 150                 155                 160
   Asn Leu Asp Glu Trp Arg Lys Met Asp Val Phe Ser Arg Ala Leu Asp
                   165                 170                 175
   Leu Leu Ala Met Tyr Pro Asn Gln Met Ile Tyr Gln Asp Gln Asp Ile
               180                 185                 190
   Leu Asn Ile Leu Phe Arg Asn Lys Val Cys Tyr Leu Asp Cys Arg Phe
           195                 200                 205
   Asn Phe Met Pro Asn Gln Leu Glu Arg Ile Lys Gln Tyr His Lys Gly
       210                 215                 220
   Lys Leu Ser Asn Leu His Ser Leu Glu Lys Thr Thr Met Pro Val Val
   225                 230                 235                 240
   Ile Ser His Tyr Cys Gly Pro Glu Lys Ala Trp His Ala Asp Cys Lys
                   245                 250                 255
   His Phe Asn Val Tyr Phe Tyr Gln Lys Ile Leu Ala Glu Ile Thr Arg
               260                 265                 270
   Gly Thr Asp Lys Glu Arg Val Leu Ser Ile Lys Thr Tyr Leu Lys Ala
           275                 280                 285
   Leu Ile Arg Arg Ile Arg Tyr Lys Phe Lys Tyr Gln Val Tyr
       290                 295                 300
<210> 108
   <211> 2054
   <212> DNA
   <213> Pasteurella Multocida
<220>
   <221> misc_feature
   <223> pnp
<220>
   <221> CDS
   <222> (1) .. (2052)
<400> 108
   atg gca agt atg gat gat act act gtg ttt gtc aca gtg gtt gcc aaa       48
   Met Ala Ser Met Asp Asp Thr Thr Val Phe Val Thr Val Val Ala Lys
   1               5                   10                  15
   aaa gat gtg aaa gaa ggt caa gac ttc ttc cca tta act gtt aac tat       96
   Lys Asp Val Lys Glu Gly Gln Asp Phe Phe Pro Leu Thr Val Asn Tyr
               20                  25                  30
   caa gag cgt act tat gct gca ggc cgt att cct ggt ggc ttt ttc aaa      144
   Gln Glu Arg Thr Tyr Ala Ala Gly Arg Ile Pro Gly Gly Phe Phe Lys
           35                  40                  45
   cgt gaa ggt cgt cct tct gaa ggc gaa act tta att gct cgt tta att      192
   Arg Glu Gly Arg Pro Ser Glu Gly Glu Thr Leu Ile Ala Arg Leu Ile
       50                  55                  60
   gac cgt cca att cgt cct ctt ttc cca gaa ggt ttt tat aac gaa atc      240
   Asp Arg Pro Ile Arg Pro Leu Phe Pro Glu Gly Phe Tyr Asn Glu Ile
   65                  70                  75                  80
   caa atc gtg gcg aca gtg gtg tct gtt aat ccg caa att tgt cca gat      288
   Gln Ile Val Ala Thr Val Val Ser Val Asn Pro Gln Ile Cys Pro Asp
                   85                  90                  95
   tta gtg gca atg atc ggt gca tct gcg gca ctt tct tta tca ggt gtg      336
   Leu Val Ala Met Ile Gly Ala Ser Ala Ala Leu Ser Leu Ser Gly Val
                   100                 105                 110
   cca ttt aat ggc cct atc ggt gcg gca cgt gtt ggt ttt att gat gat        384
   Pro Phe Asn Gly Pro Ile Gly Ala Ala Arg Val Gly Phe Ile Asp Asp
               115                 120                 125
   caa ttt gtg tta aac cca acc atg aac gag caa aaa caa agc cgt tta        432
   Gln Phe Val Leu Asn Pro Thr Met Asn Glu Gln Lys Gln Ser Arg Leu
       130                 135                 140
   gac ttg gtt gtc gcg gga aca gat aaa gcg gtg tta atg gtg gaa tct        480
   Asp Leu Val Val Ala Gly Thr Asp Lys Ala Val Leu Met Val Glu Ser
   145                 150                 155                 160
   gaa gcc gat gta tta acc gaa gaa caa atg tta gct gcg gtg gtg ttt        528
   Glu Ala Asp Val Leu Thr Glu Glu Gln Met Leu Ala Ala Val Val Phe
                   165                 170                 175
   ggt cat cag caa caa caa gtg gtg att gac gcg atc aaa gaa ttt acc        576
   Gly His Gln Gln Gln Gln Val Val Ile Asp Ala Ile Lys Glu Phe Thr
                   180                 185                 190
   gca gaa gcc ggt aaa ccg cgt tgg gat tgg gtg gca cct gaa cca aat        624
   Ala Glu Ala Gly Lys Pro Arg Trp Asp Trp Val Ala Pro Glu Pro Asn
               195                 200                 205
   acc gcg tta att gaa aaa gtg aaa gcg att gca gaa gcg cgt tta ggc        672
   Thr Ala Leu Ile Glu Lys Val Lys Ala Ile Ala Glu Ala Arg Leu Gly
       210                 215                 220
   gaa gca tac cgt atc act gaa aaa caa gca cgt tat gaa caa att gat        720
   Glu Ala Tyr Arg Ile Thr Glu Lys Gln Ala Arg Tyr Glu Gln Ile Asp
   225                 230                 235                 240
   gcg att aaa gct gat gtg att gca caa atc aca gct gaa gta gca gaa        768
   Ala Ile Lys Ala Asp Val Ile Ala Gln Ile Thr Ala Glu Val Ala Glu
                   245                 250                 255
   ggc gaa gac atc agt gaa ggg aaa att gtc gat att ttc acc gca ctt        816
   Gly Glu Asp Ile Ser Glu Gly Lys Ile Val Asp Ile Phe Thr Ala Leu
                   260                 265                 270
   gaa agc caa atc gta cgt agc cgt atc att gct ggt gaa cca cgt att        864
   Glu Ser Gln Ile Val Arg Ser Arg Ile Ile Ala Gly Glu Pro Arg Ile
               275                 280                 285
   gat ggt cgt aca gtg gat act gtt cgt gca tta gat att tgt act ggt        912
   Asp Gly Arg Thr Val Asp Thr Val Arg Ala Leu Asp Ile Cys Thr Gly
       290                 295                 300
   gtt tta cca cgt aca cac ggt tct gcg att ttc acc cgt ggt gaa aca        960
   Val Leu Pro Arg Thr His Gly Ser Ala Ile Phe Thr Arg Gly Glu Thr
   305                 310                 315                 320
   cag gcg tta gct gtc gcg aca tta ggt aca gaa cgt gat gca caa att       1008
   Gln Ala Leu Ala Val Ala Thr Leu Gly Thr Glu Arg Asp Ala Gln Ile
                   325                 330                 335
   att gat gaa tta aca ggt gag cgt tca gat cac ttc tta ttc cac tac       1056
   Ile Asp Glu Leu Thr Gly Glu Arg Ser Asp His Phe Leu Phe His Tyr
                   340                 345                 350
   aac ttc ccg cca tat tct gtg ggt gaa acc ggt atg att ggt tca cca      1104
   Asn Phe Pro Pro Tyr Ser Val Gly Glu Thr Gly Met Ile Gly Ser Pro
           355                 360                 365
   aaa cgt cgt gaa att ggt cat ggt cgt tta gcg aaa cgc ggt gta gct      1152
   Lys Arg Arg Glu Ile Gly His Gly Arg Leu Ala Lys Arg Gly Val Ala
       370                 375                 380
   gca gtg atg cca aca ctt gcc gag ttc ccg tat gtg gta cgt gtt gtc      1200
   Ala Val Met Pro Thr Leu Ala Glu Phe Pro Tyr Val Val Arg Val Val
   385                 390                 395                 400
   tct gaa atc aca gaa tca aat ggt tct tct tct atg gca tcg gtt tgt      1248
   Ser Glu Ile Thr Glu Ser Asn Gly Ser Ser Ser Met Ala Ser Val Cys
                   405                 410                 415
   ggt gcg tct tta gca tta atg gat gcg ggt gta cca att aaa gcg gcg      1296
   Gly Ala Ser Leu Ala Leu Met Asp Ala Gly Val Pro Ile Lys Ala Ala
               420                 425                 430
   gtt gca ggt att gca atg ggc tta gtc aaa gaa gac gaa aaa ttt gtg      1344
   Val Ala Gly Ile Ala Met Gly Leu Val Lys Glu Asp Glu Lys Phe Val
           435                 440                 445
   gtg ctt tca gac atc tta ggt gat gaa gat cac tta ggt gac atg gac      1392
   Val Leu Ser Asp Ile Leu Gly Asp Glu Asp His Leu Gly Asp Met Asp
       450                 455                 460
   ttc aaa gtc gcg ggt aca cgt acg ggt gtg acg gca tta caa atg gat      1440
   Phe Lys Val Ala Gly Thr Arg Thr Gly Val Thr Ala Leu Gln Met Asp
   465                 470                 475                 480
   atc aaa atc gaa ggt atc aca gca gaa atc atg caa att gcg tta aac      1488
   Ile Lys Ile Glu Gly Ile Thr Ala Glu Ile Met Gln Ile Ala Leu Asn
                   485                 490                 495
   caa gcg aaa agc gca cgt tta cac att tta ggt gtg atg gag caa gcg      1536
   Gln Ala Lys Ser Ala Arg Leu His Ile Leu Gly Val Met Glu Gln Ala
               500                 505                 510
   atc cca gcg cca cgt gcg gat att tct gat ttt gca ccg cgt att tac      1584
   Ile Pro Ala Pro Arg Ala Asp Ile Ser Asp Phe Ala Pro Arg Ile Tyr
           515                 520                 525
   act atg aaa att gat ccg aag aaa atc aaa gat gtg atc ggt aaa ggt      1632
   Thr Met Lys Ile Asp Pro Lys Lys Ile Lys Asp Val Ile Gly Lys Gly
       530                 535                 540
   ggt gca acc att cgt gcc tta aca gaa gaa aca ggt acc tca att gat      1680
   Gly Ala Thr Ile Arg Ala Leu Thr Glu Glu Thr Gly Thr Ser Ile Asp
   545                 550                 555                 560
   atc gat gat gat ggt acg gtg aag att gct gcg gtt gat ggc aat tca      1728
   Ile Asp Asp Asp Gly Thr Val Lys Ile Ala Ala Val Asp Gly Asn Ser
                   565                 570                 575
   gca aaa gag gtg atg gcg cgt att gaa gat att act gca gaa gtt gaa      1776
   Ala Lys Glu Val Met Ala Arg Ile Glu Asp Ile Thr Ala Glu Val Glu
               580                 585                 590
   gcg ggt gca gtg tat aaa ggt aaa gtt act cgt tta gct gat ttt ggt      1824
   Ala Gly Ala Val Tyr Lys Gly Lys Val Thr Arg Leu Ala Asp Phe Gly
           595                 600                 605
   gcc ttc gtt tct atc gta ggt aac aaa gaa ggc tta gtg cat att tct      1872
   Ala Phe Val Ser Ile Val Gly Asn Lys Glu Gly Leu Val His Ile Ser
       610                 615                 620
   caa atc gcg gaa gag cgt gtt gag aaa gtg agt gat tat ctt gca gtg      1920
   Gln Ile Ala Glu Glu Arg Val Glu Lys Val Ser Asp Tyr Leu Ala Val
   625                 630                 635                 640
   ggg caa gaa gtg act gtt aaa gtg gtt gag att gat cgt caa ggt cgt      1968
   Gly Gln Glu Val Thr Val Lys Val Val Glu Ile Asp Arg Gln Gly Arg
                   645                 650                 655
   att cgt tta acc atg aaa gaa gtt gca cca aag caa gaa cac gtt gat      2016
   Ile Arg Leu Thr Met Lys Glu Val Ala Pro Lys Gln Glu His Val Asp
               660                 665                 670
   tct gtt gtc gca gac gtt gcc gca gaa gaa aac gca ta                   2054
   Ser Val Val Ala Asp Val Ala Ala Glu Glu Asn Ala
           675                 680
<210> 109
   <211> 684
   <212> PRT
   <213> Pasteurella Multocida
<400> 109
   Met Ala Ser Met Asp Asp Thr Thr Val Phe Val Thr Val Val Ala Lys
   1               5                   10                  15
   Lys Asp Val Lys Glu Gly Gln Asp Phe Phe Pro Leu Thr Val Asn Tyr
               20                  25                  30
   Gln Glu Arg Thr Tyr Ala Ala Gly Arg Ile Pro Gly Gly Phe Phe Lys
           35                  40                  45
   Arg Glu Gly Arg Pro Ser Glu Gly Glu Thr Leu Ile Ala Arg Leu Ile
       50                  55                  60
   Asp Arg Pro Ile Arg Pro Leu Phe Pro Glu Gly Phe Tyr Asn Glu Ile
   65                  70                  75                  80
   Gln Ile Val Ala Thr Val Val Ser Val Asn Pro Gln Ile Cys Pro Asp
                   85                  90                  95
   Leu Val Ala Met Ile Gly Ala Ser Ala Ala Leu Ser Leu Ser Gly Val
               100                 105                 110
   Pro Phe Asn Gly Pro Ile Gly Ala Ala Arg Val Gly Phe Ile Asp Asp
   115 120 125
   Gln Phe Val Leu Asn Pro Thr Met Asn Glu Gln Lys Gln Ser Arg Leu
   130 135 140
   Asp Leu Val Val Ala Gly Thr Asp Lys Ala Val Leu Met Val Glu Ser
   145 150 155 160
   Glu Ala Asp Val Leu Thr Glu Glu Gln Met Leu Ala Ala Val Val Phe
   165 170 175
   Gly His Gln Gln Gln Gln Val Val Ile Asp Ala Ile Lys Glu Phe Thr
   180 185 190
   Ala Glu Ala Gly Lys Pro Arg Trp Asp Trp Val Ala Pro Glu Pro Asn
   195 200 205
   Thr Ala Leu Ile Glu Lys Val Lys Ala Ile Ala Glu Ala Arg Leu Gly
   210 215 220
   Glu Ala Tyr Arg Ile Thr Glu Lys Gln Ala Arg Tyr Glu Gln Ile Asp
   225 230 235 240
   Ala Ile Lys Ala Asp Val Ile Ala Gln Ile Thr Ala Glu Val Ala Glu
   245 250 255
   Gly Glu Asp Ile Ser Glu Gly Lys Ile Val Asp Ile Phe Thr Ala Leu
   260 265 270
   Glu Ser Gln Ile Val Arg Ser Arg Ile Ile Ala Gly Glu Pro Arg Ile
   275 280 285
   Asp Gly Arg Thr Val Asp Thr Val Arg Ala Leu Asp Ile Cys Thr Gly
   290 295 300
   Val Leu Pro Arg Thr His Gly Ser Ala Ile Phe Thr Arg Gly Glu Thr
   305 310 315 320
   Gln Ala Leu Ala Val Ala Thr Leu Gly Thr Glu Arg Asp Ala Gln Ile
   325 330 335
   Ile Asp Glu Leu Thr Gly Glu Arg Ser Asp His Phe Leu Phe His Tyr
   340 345 350
   Asn Phe Pro Pro Tyr Ser Val Gly Glu Thr Gly Met Ile Gly Ser Pro
   355 360 365
   Lys Arg Arg Glu Ile Gly His Gly Arg Leu Ala Lys Arg Gly Val Ala
   370 375 380
   Ala Val Met Pro Thr Leu Ala Glu Phe Pro Tyr Val Val Arg Val Val
   385 390 395 400
   Ser Glu Ile Thr Glu Ser Asn Gly Ser Ser Ser Met Ala Ser Val Cys
   405 410 415
   Gly Ala Ser Leu Ala Leu Met Asp Ala Gly Val Pro Ile Lys Ala Ala
   420 425 430
   Val Ala Gly Ile Ala Met Gly Leu Val Lys Glu Asp Glu Lys Phe Val
   435 440 445
   Val Leu Ser Asp Ile Leu Gly Asp Glu Asp His Leu Gly Asp Met Asp
   450 455 460
   Phe Lys Val Ala Gly Thr Arg Thr Gly Val Thr Ala Leu Gln Met Asp
   465 470 475 480
   Ile Lys Ile Glu Gly Ile Thr Ala Glu Ile Met Gln Ile Ala Leu Asn
   485 490 495
   Gln Ala Lys Ser Ala Arg Leu His Ile Leu Gly Val Met Glu Gln Ala
   500 505 510
   Ile Pro Ala Pro Arg Ala Asp Ile Ser Asp Phe Ala Pro Arg Ile Tyr
   515 520 525
   Thr Met Lys Ile Asp Pro Lys Lys Ile Lys Asp Val Ile Gly Lys Gly
   530 535 540
   Gly Ala Thr Ile Arg Ala Leu Thr Glu Glu Thr Gly Thr Ser Ile Asp
   545 550 555 560
   Ile Asp Asp Asp Gly Thr Val Lys Ile Ala Ala Val Asp Gly Asn Ser
   565 570 575
   Ala Lys Glu Val Met Ala Arg Ile Glu Asp Ile Thr Ala Glu Val Glu
   580 585 590
   Ala Gly Ala Val Tyr Lys Gly Lys Val Thr Arg Leu Ala Asp Phe Gly
   595 600 605
   Ala Phe Val Ser Ile Val Gly Asn Lys Glu Gly Leu Val His Ile Ser
       610                 615                 620
   Gln Ile Ala Glu Glu Arg Val Glu Lys Val Ser Asp Tyr Leu Ala Val
   625                 630                 635                 640
   Gly Gln Glu Val Thr Val Lys Val Val Glu Ile Asp Arg Gln Gly Arg
                   645                 650                 655
   Ile Arg Leu Thr Met Lys Glu Val Ala Pro Lys Gln Glu His Val Asp
               660                 665                 670
   Ser Val Val Ala Asp Val Ala Ala Glu Glu Asn Ala
           675                 680
<210> 110
   <211> 1514
   <212> DNA
   <213> Pasteurella Multocida
<220>
   <221> misc_feature
   <223> purF
<220>
   <221> CDS
   <222> (1) .. (1512)
<400> 110
   atg tgt ggt att gtt ggt att gtt agc caa agc ccc gtt aac caa tca       48
   Met Cys Gly Ile Val Gly Ile Val Ser Gln Ser Pro Val Asn Gln Ser
   1               5                   10                  15
   att tat gat gcg tta acc tta ttg caa cac cgc ggg caa gac gcc gcc       96
   Ile Tyr Asp Ala Leu Thr Leu Leu Gln His Arg Gly Gln Asp Ala Ala
               20                  25                  30
   ggg att gta acc gta gat gat gaa aac cga ttc cgc ttg cgt aaa gcg      144
   Gly Ile Val Thr Val Asp Asp Glu Asn Arg Phe Arg Leu Arg Lys Ala
           35                  40                  45
   aac ggg tta gtc agc gat gta ttt gaa caa gtt cat atg tta cgt tta      192
   Asn Gly Leu Val Ser Asp Val Phe Glu Gln Val His Met Leu Arg Leu
       50                  55                  60
   caa ggc aat gct ggc att gga cat gtt cgt tat cct acg gct ggg agc      240
   Gln Gly Asn Ala Gly Ile Gly His Val Arg Tyr Pro Thr Ala Gly Ser
   65                  70                  75                  80
   tca agt gtc tct gaa gcg caa cct ttt tat gta aat tcg cct tat ggc      288
   Ser Ser Val Ser Glu Ala Gln Pro Phe Tyr Val Asn Ser Pro Tyr Gly
                   85                  90                  95
   tta acc tta gtg cat aat ggt aac ttg acc aat tca agt gaa tta aaa      336
   Leu Thr Leu Val His Asn Gly Asn Leu Thr Asn Ser Ser Glu Leu Lys
               100                 105                 110
   gaa aag tta ttt cgt ctc gca cgt cgc cat gta aat acc aat tca gat      384
   Glu Lys Leu Phe Arg Leu Ala Arg Arg His Val Asn Thr Asn Ser Asp
           115                 120                 125
   tct gaa tta tta ctc aat atc tta gcc aat cac ctt gat cac ttc gaa      432
   Ser Glu Leu Leu Leu Asn Ile Leu Ala Asn His Leu Asp His Phe Glu
       130                 135                 140
   aaa tac caa tta gat ccg caa gat gta ttc agt gct gtc aaa caa acg      480
   Lys Tyr Gln Leu Asp Pro Gln Asp Val Phe Ser Ala Val Lys Gln Thr
   145                 150                 155                 160
   cat cag gat att cgt ggt gct tat gct tgt atc gcc atg att att ggt      528
   His Gln Asp Ile Arg Gly Ala Tyr Ala Cys Ile Ala Met Ile Ile Gly
                   165                 170                 175
   cat ggt atg gtc gcg ttt cgt gat ccg aac ggt atc cgt ccg tta gtg      576
   His Gly Met Val Ala Phe Arg Asp Pro Asn Gly Ile Arg Pro Leu Val
               180                 185                 190
   tta ggg aaa cgc gag gaa aat ggc aaa aca gag tat atg ttt gcc tcc      624
   Leu Gly Lys Arg Glu Glu Asn Gly Lys Thr Glu Tyr Met Phe Ala Ser
           195                 200                 205
   gaa agt atc gca tta gat aca gtg ggt ttt gag ttt gta cga gat gta      672
   Glu Ser Ile Ala Leu Asp Thr Val Gly Phe Glu Phe Val Arg Asp Val
       210                 215                 220
   caa ccc ggc gaa gcg att tat gtc acg ttt gaa ggg gaa atg tat gct      720
   Gln Pro Gly Glu Ala Ile Tyr Val Thr Phe Glu Gly Glu Met Tyr Ala
   225                 230                 235                 240
   cag caa tgc gca gac aaa cca aca tta aca cct tgt att ttt gaa tac      768
   Gln Gln Cys Ala Asp Lys Pro Thr Leu Thr Pro Cys Ile Phe Glu Tyr
                   245                 250                 255
   gtc tat ttt gca cgt cca gac tct tgc atc gat ggg gtt tct gtt tat      816
   Val Tyr Phe Ala Arg Pro Asp Ser Cys Ile Asp Gly Val Ser Val Tyr
               260                 265                 270
   gct gcc cgt gtt cat atg gga caa cgt tta ggt gaa aaa att gca cgg      864
   Ala Ala Arg Val His Met Gly Gln Arg Leu Gly Glu Lys Ile Ala Arg
           275                 280                 285
   gaa tgg gcg gat gtg gat gat att gat gtg gtc att cct gtg cct gaa      912
   Glu Trp Ala Asp Val Asp Asp Ile Asp Val Val Ile Pro Val Pro Glu
       290                 295                 300
   acc tct aac gat att gct tta cgt att gcg cgc gtg tta aat aaa ccg      960
   Thr Ser Asn Asp Ile Ala Leu Arg Ile Ala Arg Val Leu Asn Lys Pro
   305                 310                 315                 320
   tat cgt caa ggt ttt gtg aaa aat cgc tat gta gga cgt acg ttt att     1008
   Tyr Arg Gln Gly Phe Val Lys Asn Arg Tyr Val Gly Arg Thr Phe Ile
                   325                 330                 335
   atg ccg ggg cag gca ttg cga gtc agt tct gtt aga cgt aaa ctc aat     1056
   Met Pro Gly Gln Ala Leu Arg Val Ser Ser Val Arg Arg Lys Leu Asn
                   340                 345                 350
   acc att gct tca gaa ttt aaa gat aag aat gtg tta tta gtt gac gac      1104
   Thr Ile Ala Ser Glu Phe Lys Asp Lys Asn Val Leu Leu Val Asp Asp
               355                 360                 365
   tcg att gta cgt ggt acc acg tct gaa caa att gtc gaa atg gcg aga      1152
   Ser Ile Val Arg Gly Thr Thr Ser Glu Gln Ile Val Glu Met Ala Arg
           370                 375                 380
   gcg gca ggt gcg aag aaa att tat ttt gcc tct gct gca cca gaa att      1200
   Ala Ala Gly Ala Lys Lys Ile Tyr Phe Ala Ser Ala Ala Pro Glu Ile
   385                 390                 395                     400
   cgt tat cca aat gtg tat ggt att gat atg cca acc aaa aat gaa ttg      1248
   Arg Tyr Pro Asn Val Tyr Gly Ile Asp Met Pro Thr Lys Asn Glu Leu
                   405                 410                 415
   atc gct tat ggt cgt gat gta gat gaa att gct aac tta att ggt gtg      1296
   Ile Ala Tyr Gly Arg Asp Val Asp Glu Ile Ala Asn Leu Ile Gly Val
               420                 425                 430
   gat aaa ttg att ttc caa gat ttg gat gcg tta act ggt tct gtg caa      1344
   Asp Lys Leu Ile Phe Gln Asp Leu Asp Ala Leu Thr Gly Ser Val Gln
           435                 440                 445
   caa gaa aat cca agt att caa gac ttt gat tgt tcg gtg ttt aca ggg      1392
   Gln Glu Asn Pro Ser Ile Gln Asp Phe Asp Cys Ser Val Phe Thr Gly
       450                 455                 460
   gtt tat gtg acg ggc gat att aca cct gaa tat ctg gat aat att gca      1440
   Val Tyr Val Thr Gly Asp Ile Thr Pro Glu Tyr Leu Asp Asn Ile Ala
   465                 470                 475                     480
   gaa cag cgt aat gat atc gcc aag aaa aag cgt gaa aaa gat gct acc      1488
   Glu Gln Arg Asn Asp Ile Ala Lys Lys Lys Arg Glu Lys Asp Ala Thr
                   485                 490                 495
   aat ctt gaa atg cac aat gaa aaa ta                                   1514
   Asn Leu Glu Met His Asn Glu Lys
               500
<210> 111
   <211> 504
   <212> PRT
   <213> Pasteurella Multocida
<400> 111
   Met Cys Gly Ile Val Gly Ile Val Ser Gln Ser Pro Val Asn Gln Ser
   1               5                   10                  15
   Ile Tyr Asp Ala Leu Thr Leu Leu Gln His Arg Gly Gln Asp Ala Ala
               20                  25                  30
   Gly Ile Val Thr Val Asp Asp Glu Asn Arg Phe Arg Leu Arg Lys Ala
           35                  40                  45
   Asn Gly Leu Val Ser Asp Val Phe Glu Gln Val His Met Leu Arg Leu
       50                  55                  60
   Gln Gly Asn Ala Gly Ile Gly His Val Arg Tyr Pro Thr Ala Gly Ser
   65                  70                  75                  80
   Ser Ser Val Ser Glu Ala Gln Pro Phe Tyr Val Asn Ser Pro Tyr Gly
                   85                  90                  95
   Leu Thr Leu Val His Asn Gly Asn Leu Thr Asn Ser Ser Glu Leu Lys
               100                 105                 110
   Glu Lys Leu Phe Arg Leu Ala Arg Arg His Val Asn Thr Asn Ser Asp
           115                 120                 125
   Ser Glu Leu Leu Leu Asn Ile Leu Ala Asn His Leu Asp His Phe Glu
       130                 135                 140
   Lys Tyr Gln Leu Asp Pro Gln Asp Val Phe Ser Ala Val Lys Gln Thr
   145                 150                 155                 160
   His Gln Asp Ile Arg Gly Ala Tyr Ala Cys Ile Ala Met Ile Ile Gly
                   165                 170                 175
   His Gly Met Val Ala Phe Arg Asp Pro Asn Gly Ile Arg Pro Leu Val
               180                 185                 190
   Leu Gly Lys Arg Glu Glu Asn Gly Lys Thr Glu Tyr Met Phe Ala Ser
           195                 200                 205
   Glu Ser Ile Ala Leu Asp Thr Val Gly Phe Glu Phe Val Arg Asp Val
       210                 215                 220
   Gln Pro Gly Glu Ala Ile Tyr Val Thr Phe Glu Gly Glu Met Tyr Ala
   225                 230                 235                 240
   Gln Gln Cys Ala Asp Lys Pro Thr Leu Thr Pro Cys Ile Phe Glu Tyr
                   245                 250                 255
   Val Tyr Phe Ala Arg Pro Asp Ser Cys Ile Asp Gly Val Ser Val Tyr
               260                 265                 270
   Ala Ala Arg Val His Met Gly Gln Arg Leu Gly Glu Lys Ile Ala Arg
           275                 280                 285
   Glu Trp Ala Asp Val Asp Asp Ile Asp Val Val Ile Pro Val Pro Glu
       290                 295                 300
   Thr Ser Asn Asp Ile Ala Leu Arg Ile Ala Arg Val Leu Asn Lys Pro
   305                 310                 315                 320
   Tyr Arg Gln Gly Phe Val Lys Asn Arg Tyr Val Gly Arg Thr Phe Ile
                   325                 330                 335
   Met Pro Gly Gln Ala Leu Arg Val Ser Ser Val Arg Arg Lys Leu Asn
               340                 345                 350
   Thr Ile Ala Ser Glu Phe Lys Asp Lys Asn Val Leu Leu Val Asp Asp
           355                 360                 365
   Ser Ile Val Arg Gly Thr Thr Ser Glu Gln Ile Val Glu Met Ala Arg
       370                 375                 380
   Ala Ala Gly Ala Lys Lys Ile Tyr Phe Ala Ser Ala Ala Pro Glu Ile
   385                 390                 395                 400
   Arg Tyr Pro Asn Val Tyr Gly Ile Asp Met Pro Thr Lys Asn Glu Leu
                   405                 410                 415
   Ile Ala Tyr Gly Arg Asp Val Asp Glu Ile Ala Asn Leu Ile Gly Val
               420                 425                 430
   Asp Lys Leu Ile Phe Gln Asp Leu Asp Ala Leu Thr Gly Ser Val Gln
           435                 440                 445
   Gln Glu Asn Pro Ser Ile Gln Asp Phe Asp Cys Ser Val Phe Thr Gly
       450                 455                 460
   Val Tyr Val Thr Gly Asp Ile Thr Pro Glu Tyr Leu Asp Asn Ile Ala
   465                 470                 475                 480
   Glu Gln Arg Asn Asp Ile Ala Lys Lys Lys Arg Glu Lys Asp Ala Thr
                   485                 490                 495
   Asn Leu Glu Met His Asn Glu Lys
               500
<210> 112
   <211> 989
   <212> DNA
   <213> Pasteurella Multocida
<220>
   <221> misc_feature
   <223> rci
<220>
   <221> CDS
   <222> (1) .. (987)
<400> 112
   atg gca aca ata aga aaa cgt ggt aac tca tat cgt gct gag ata agc        48
   Met Ala Thr Ile Arg Lys Arg Gly Asn Ser Tyr Arg Ala Glu Ile Ser
   1               5                   10                  15
   aaa aac gga gta agg aaa tca gca aca ttt aag act aaa tca gaa gct        96
   Lys Asn Gly Val Arg Lys Ser Ala Thr Phe Lys Thr Lys Ser Glu Ala
               20                  25                  30
   aat gcg tgg gct gtt gac gag gag aga aaa tta gct gat ttg gca aaa       144
   Asn Ala Trp Ala Val Asp Glu Glu Arg Lys Leu Ala Asp Leu Ala Lys
           35                  40                  45
   ggt atc gct cca gat att att ttt aga gat gta ata gaa cgc tat caa       192
   Gly Ile Ala Pro Asp Ile Ile Phe Arg Asp Val Ile Glu Arg Tyr Gln
       50                  55                  60
   aat gaa gtg tct ata act aaa aaa ggc gcg cga aat gaa att ata aga       240
   Asn Glu Val Ser Ile Thr Lys Lys Gly Ala Arg Asn Glu Ile Ile Arg
   65                  70                  75                  80
   tta aac cgc ttt tta aga tat gat att tct aat ctg tat att cgt gat       288
   Leu Asn Arg Phe Leu Arg Tyr Asp Ile Ser Asn Leu Tyr Ile Arg Asp
                   85                  90                  95
   tta aga aaa gaa gat ttt gag gag tgg atc aga att cgc cta acc gaa       336
   Leu Arg Lys Glu Asp Phe Glu Glu Trp Ile Arg Ile Arg Leu Thr Glu
               100                 105                 110
   gta tcg gat gct agc gtt aga cgt gag ctt gtt act ata tcg tca gtg       384
   Val Ser Asp Ala Ser Val Arg Arg Glu Leu Val Thr Ile Ser Ser Val
           115                 120                 125
   ctg aca aca gca ata aat aag tgg gga tat att tca agg cat cca atg       432
   Leu Thr Thr Ala Ile Asn Lys Trp Gly Tyr Ile Ser Arg His Pro Met
       130                 135                 140
   act ggt att gaa aaa cca aaa aac tcg gca gaa aga aaa gaa cga tat       480
   Thr Gly Ile Glu Lys Pro Lys Asn Ser Ala Glu Arg Lys Glu Arg Tyr
   145                 150                 155                 160
   tca gaa cag gac att aaa aca ata tta gaa aca gct aga tat tgt gaa       528
   Ser Glu Gln Asp Ile Lys Thr Ile Leu Glu Thr Ala Arg Tyr Cys Glu
                   165                 170                 175
   gat aaa cta ccc ata aca ctc aaa caa aga gta gca att gca atg tta       576
   Asp Lys Leu Pro Ile Thr Leu Lys Gln Arg Val Ala Ile Ala Met Leu
               180                 185                 190
   ttt gct att gaa acc gct atg cgt gct ggt gag att gct agt ata aaa       624
   Phe Ala Ile Glu Thr Ala Met Arg Ala Gly Glu Ile Ala Ser Ile Lys
           195                 200                 205
   tgg gat aat gtt ttt ctt gaa aag aga ata gta cat tta ccg aca act      672
   Trp Asp Asn Val Phe Leu Glu Lys Arg Ile Val His Leu Pro Thr Thr
       210                 215                 220
   aaa aac ggg cac tct aga gat gtg ccg ctt tcg caa aga gct gtt gcg      720
   Lys Asn Gly His Ser Arg Asp Val Pro Leu Ser Gln Arg Ala Val Ala
   225                 230                 235                 240
   cta att tta aaa atg aaa gag gta gaa aat gga gat ctt gtg ttt cag      768
   Leu Ile Leu Lys Met Lys Glu Val Glu Asn Gly Asp Leu Val Phe Gln
                   245                 250                 255
   acc acg cct gaa tca tta agc acc acg ttt aga gtg tta aag aaa gag      816
   Thr Thr Pro Glu Ser Leu Ser Thr Thr Phe Arg Val Leu Lys Lys Glu
               260                 265                 270
   tgt gga ctt gaa cat ctc cat ttt cat gat acg aga agg gaa gcg ttg      864
   Cys Gly Leu Glu His Leu His Phe His Asp Thr Arg Arg Glu Ala Leu
           275                 280                 285
   acg aga tta tct aag aaa gta gat gta atg act cta gcc aaa att agc      912
   Thr Arg Leu Ser Lys Lys Val Asp Val Met Thr Leu Ala Lys Ile Ser
       290                 295                 300
   gga cat aga gat tta aga att tta caa aac aca tat tac gca ccg aat      960
   Gly His Arg Asp Leu Arg Ile Leu Gln Asn Thr Tyr Tyr Ala Pro Asn
   305                 310                 315                 320
   atg agt gaa gtg gca aac ttg ttg gat ta                               989
   Met Ser Glu Val Ala Asn Leu Leu Asp
                   325
<210> 113
   <211> 329
   <212> PRT
   <213> Pasteurella Multocida
<400> 113
   Met Ala Thr Ile Arg Lys Arg Gly Asn Ser Tyr Arg Ala Glu Ile Ser
   1               5                   10                  15
   Lys Asn Gly Val Arg Lys Ser Ala Thr Phe Lys Thr Lys Ser Glu Ala
               20                  25                  30
   Asn Ala Trp Ala Val Asp Glu Glu Arg Lys Leu Ala Asp Leu Ala Lys
           35                  40                  45
   Gly Ile Ala Pro Asp Ile Ile Phe Arg Asp Val Ile Glu Arg Tyr Gln
       50                  55                  60
   Asn Glu Val Ser Ile Thr Lys Lys Gly Ala Arg Asn Glu Ile Ile Arg
   65                  70                  75                  80
   Leu Asn Arg Phe Leu Arg Tyr Asp Ile Ser Asn Leu Tyr Ile Arg Asp
                   85                  90                  95
   Leu Arg Lys Glu Asp Phe Glu Glu Trp Ile Arg Ile Arg Leu Thr Glu
               100                 105                 110
   Val Ser Asp Ala Ser Val Arg Arg Glu Leu Val Thr Ile Ser Ser Val
           115                 120                 125
   Leu Thr Thr Ala Ile Asn Lys Trp Gly Tyr Ile Ser Arg His Pro Met
       130                 135                 140
   Thr Gly Ile Glu Lys Pro Lys Asn Ser Ala Glu Arg Lys Glu Arg Tyr
   145                 150                 155                 160
   Ser Glu Gln Asp Ile Lys Thr Ile Leu Glu Thr Ala Arg Tyr Cys Glu
                   165                 170                 175
   Asp Lys Leu Pro Ile Thr Leu Lys Gln Arg Val Ala Ile Ala Met Leu
               180                 185                 190
   Phe Ala Ile Glu Thr Ala Met Arg Ala Gly Glu Ile Ala Ser Ile Lys
           195                 200                 205
   Trp Asp Asn Val Phe Leu Glu Lys Arg Ile Val His Leu Pro Thr Thr
       210                 215                 220
   Lys Asn Gly His Ser Arg Asp Val Pro Leu Ser Gln Arg Ala Val Ala
   225                 230                 235                 240
   Leu Ile Leu Lys Met Lys Glu Val Glu Asn Gly Asp Leu Val Phe Gln
                   245                 250                 255
   Thr Thr Pro Glu Ser Leu Ser Thr Thr Phe Arg Val Leu Lys Lys Glu
               260                 265                 270
   Cys Gly Leu Glu His Leu His Phe His Asp Thr Arg Arg Glu Ala Leu
           275                 280                 285
   Thr Arg Leu Ser Lys Lys Val Asp Val Met Thr Leu Ala Lys Ile Ser
       290                 295                 300
   Gly His Arg Asp Leu Arg Ile Leu Gln Asn Thr Tyr Tyr Ala Pro Asn
   305                 310                 315                 320
   Met Ser Glu Val Ala Asn Leu Leu Asp
                           325
<210> 114
   <211> 1190
   <212> DNA
   <213> Pasteurella Multocida
<220>
   <221> misc_feature
   <223> sopE
<220>
   <221> CDS
   <222> (1) .. (1188)
<400> 114
   atg tct gaa gaa tat cta cat ggt gtc aaa gtc aca gaa atc aat caa       48
   Met Ser Glu Glu Tyr Leu His Gly Val Lys Val Thr Glu Ile Asn Gln
   1               5                   10                  15
   gca att cgc aca att caa agt cta tca acc gca gtc atc ggt att gtc       96
   Ala Ile Arg Thr Ile Gln Ser Leu Ser Thr Ala Val Ile Gly Ile Val
               20                  25                  30
   tgt act gca aat gac gca gac aat gaa aca ttc cca ctc aat gaa ccc      144
   Cys Thr Ala Asn Asp Ala Asp Asn Glu Thr Phe Pro Leu Asn Glu Pro
           35                  40                  45
   gtt ctc atc aca aac gtg gca gcg gca att ggc aag gct gga aaa caa      192
   Val Leu Ile Thr Asn Val Ala Ala Ala Ile Gly Lys Ala Gly Lys Gln
       50                  55                  60
   ggc acg ctt tca cgt gcg ctt gac ggg att tct gat gta gtc aat tgc      240
   Gly Thr Leu Ser Arg Ala Leu Asp Gly Ile Ser Asp Val Val Asn Cys
   65                  70                  75                  80
   aaa gtg att gtt gtg cga gtg caa gaa agt gcg caa gaa gac gaa gaa      288
   Lys Val Ile Val Val Arg Val Gln Glu Ser Ala Gln Glu Asp Glu Glu
                   85                  90                  95
   aca aaa gca agt gaa atg aac acg gca att att ggc aca atc aca gaa      336
   Thr Lys Ala Ser Glu Met Asn Thr Ala Ile Ile Gly Thr Ile Thr Glu
               100                 105                 110
   gaa ggg cag tac aca ggc ttg aag gcg tta ttg att gcg aaa aac aaa      384
   Glu Gly Gln Tyr Thr Gly Leu Lys Ala Leu Leu Ile Ala Lys Asn Lys
           115                 120                 125
   ttc ggt atc aaa cca cgt att tta tgt gtg cca aaa ttc gac aca aaa      432
   Phe Gly Ile Lys Pro Arg Ile Leu Cys Val Pro Lys Phe Asp Thr Lys
       130                 135                 140
   gaa gtc gcc aca gag ctt gca agt atc gcc gcc aaa ctc aac gca ttt      480
   Glu Val Ala Thr Glu Leu Ala Ser Ile Ala Ala Lys Leu Asn Ala Phe
   145                 150                 155                 160
   gct tac att tca tgt caa ggg tgt aaa acg aaa gaa caa gcg gtg caa      528
   Ala Tyr Ile Ser Cys Gln Gly Cys Lys Thr Lys Glu Gln Ala Val Gln
                   165                 170                 175
   tat aaa cgc aac ttc tca caa cgt gaa gtc atg ctg atc atg ggc gat       576
   Tyr Lys Arg Asn Phe Ser Gln Arg Glu Val Met Leu Ile Met Gly Asp
                   180                 185                 190
   ttt ctg tca ttt aat gtc aac aca tca aaa gtt gag att gac tat gcc       624
   Phe Leu Ser Phe Asn Val Asn Thr Ser Lys Val Glu Ile Asp Tyr Ala
               195                 200                 205
   gtc act cgt gcg gcg gca atg cgt gca tat ctt gat aaa gaa cag ggc       672
   Val Thr Arg Ala Ala Ala Met Arg Ala Tyr Leu Asp Lys Glu Gln Gly
       210                 215                 220
   tgg cat acg tct att tca aat aaa ggc att aat ggc gtg agc ggt gtc       720
   Trp His Thr Ser Ile Ser Asn Lys Gly Ile Asn Gly Val Ser Gly Val
   225                 230                 235                 240
   aca caa cca ctc tat ttt gac att aac gac agc tcg act gat gtg aac       768
   Thr Gln Pro Leu Tyr Phe Asp Ile Asn Asp Ser Ser Thr Asp Val Asn
                   245                 250                 255
   tat ctc aat gaa caa ggc atc acg tgt tgc gtg aat cat aat ggc ttt       816
   Tyr Leu Asn Glu Gln Gly Ile Thr Cys Cys Val Asn His Asn Gly Phe
               260                 265                 270
   cgt ttt tgg ggc tta cgc acg act gca gaa gat cca tta ttc aag ttt       864
   Arg Phe Trp Gly Leu Arg Thr Thr Ala Glu Asp Pro Leu Phe Lys Phe
           275                 280                 285
   gaa gtg tac acc cgc act gca caa atc tta aaa gat acg att gca ggg       912
   Glu Val Tyr Thr Arg Thr Ala Gln Ile Leu Lys Asp Thr Ile Ala Gly
       290                 295                 300
   gcg ttt gat tgg gca gtg gat aaa gat att tct gtc acg cta gtg aaa       960
   Ala Phe Asp Trp Ala Val Asp Lys Asp Ile Ser Val Thr Leu Val Lys
   305                 310                 315                 320
   gat att att gaa gca atc aat gcg aag tgg cgt gat tac acc aca aaa      1008
   Asp Ile Ile Glu Ala Ile Asn Ala Lys Trp Arg Asp Tyr Thr Thr Lys
                   325                 330                 335
   ggc tac tta att ggc ggt aaa gcg tgg ctt aat aaa gag ctt aac agt      1056
   Gly Tyr Leu Ile Gly Gly Lys Ala Trp Leu Asn Lys Glu Leu Asn Ser
               340                 345                 350
   gca acg aat tta aaa gat gcg aag ttg ttg atc tct tat gat tat cac      1104
   Ala Thr Asn Leu Lys Asp Ala Lys Leu Leu Ile Ser Tyr Asp Tyr His
           355                 360                 365
   cca gta cca ccg ctc gaa cag cta ggc ttt aat cag tac att tct gat      1152
   Pro Val Pro Pro Leu Glu Gln Leu Gly Phe Asn Gln Tyr Ile Ser Asp
       370                 375                 380
   gaa tac ctt gtt gat ttt tca aat cgt tta gca tcg ta                   1190
   Glu Tyr Leu Val Asp Phe Ser Asn Arg Leu Ala Ser
   385                 390                 395
<210> 115
   <211> 396
   <212> PRT
   <213> Pasteurella Multocida
<400> 115
   Met Ser Glu Glu Tyr Leu His Gly Val Lys Val Thr Glu Ile Asn Gln
   1               5                   10                  15
   Ala Ile Arg Thr Ile Gln Ser Leu Ser Thr Ala Val Ile Gly Ile Val
               20                  25                  30
   Cys Thr Ala Asn Asp Ala Asp Asn Glu Thr Phe Pro Leu Asn Glu Pro
           35                  40                  45
   Val Leu Ile Thr Asn Val Ala Ala Ala Ile Gly Lys Ala Gly Lys Gln
       50                  55                  60
   Gly Thr Leu Ser Arg Ala Leu Asp Gly Ile Ser Asp Val Val Asn Cys
   65                  70                  75                  80
   Lys Val Ile Val Val Arg Val Gln Glu Ser Ala Gln Glu Asp Glu Glu
                   85                  90                  95
   Thr Lys Ala Ser Glu Met Asn Thr Ala Ile Ile Gly Thr Ile Thr Glu
               100                 105                 110
   Glu Gly Gln Tyr Thr Gly Leu Lys Ala Leu Leu Ile Ala Lys Asn Lys
           115                 120                 125
   Phe Gly Ile Lys Pro Arg Ile Leu Cys Val Pro Lys Phe Asp Thr Lys
       130                 135                 140
   Glu Val Ala Thr Glu Leu Ala Ser Ile Ala Ala Lys Leu Asn Ala Phe
   145                 150                 155                 160
   Ala Tyr Ile Ser Cys Gln Gly Cys Lys Thr Lys Glu Gln Ala Val Gln
                   165                 170                 175
   Tyr Lys Arg Asn Phe Ser Gln Arg Glu Val Met Leu Ile Met Gly Asp
               180                 185                 190
   Phe Leu Ser Phe Asn Val Asn Thr Ser Lys Val Glu Ile Asp Tyr Ala
           195                 200                 205
   Val Thr Arg Ala Ala Ala Met Arg Ala Tyr Leu Asp Lys Glu Gln Gly
       210                 215                 220
   Trp His Thr Ser Ile Ser Asn Lys Gly Ile Asn Gly Val Ser Gly Val
   225                 230                 235                 240
   Thr Gln Pro Leu Tyr Phe Asp Ile Asn Asp Ser Ser Thr Asp Val Asn
                   245                 250                 255
   Tyr Leu Asn Glu Gln Gly Ile Thr Cys Cys Val Asn His Asn Gly Phe
               260                 265                 270
   Arg Phe Trp Gly Leu Arg Thr Thr Ala Glu Asp Pro Leu Phe Lys Phe
           275                 280                 285
   Glu Val Tyr Thr Arg Thr Ala Gln Ile Leu Lys Asp Thr Ile Ala Gly
       290                 295                 300
   Ala Phe Asp Trp Ala Val Asp Lys Asp Ile Ser Val Thr Leu Val Lys
   305                 310                 315                 320
   Asp Ile Ile Glu Ala Ile Asn Ala Lys Trp Arg Asp Tyr Thr Thr Lys
                   325                 330                 335
   Gly Tyr Leu Ile Gly Gly Lys Ala Trp Leu Asn Lys Glu Leu Asn Ser
               340                 345                 350
   Ala Thr Asn Leu Lys Asp Ala Lys Leu Leu Ile Ser Tyr Asp Tyr His
           355                 360                 365
   Pro Val Pro Pro Leu Glu Gln Leu Gly Phe Asn Gln Tyr Ile Ser Asp
       370                 375                 380
   Glu Tyr Leu Val Asp Phe Ser Asn Arg Leu Ala Ser
   385                 390                 395
<210> 116
   <211> 2204
   <212> DNA
   <213> Pasteurella Multocida
<220>
   <221> misc_feature
   <223> unkK
<220>
   <221> CDS
   <222> (1) .. (2202)
<400> 116
   atg aat aaa aat cgc tat aaa ctc att ttt agt aaa act aaa ggc tgt        48
   Met Asn Lys Asn Arg Tyr Lys Leu Ile Phe Ser Lys Thr Lys Gly Cys
   1               5                   10                  15
   ctt gta cct gtt gct gaa acg att aat tct gca gta gga aat gcc tca      96
   Leu Val Pro Val Ala Glu Thr Ile Asn Ser Ala Val Gly Asn Ala Ser
               20                  25                  30
   tca aaa gac gtt tct gac acc gag ata agt gct tct caa cca gcg ctc      144
   Ser Lys Asp Val Ser Asp Thr Glu Ile Ser Ala Ser Gln Pro Ala Leu
           35                  40                  45
   aac tcg ccg ctt tcg acc ctt tct gta tta gtc aaa acc gca ttt aat      192
   Asn Ser Pro Leu Ser Thr Leu Ser Val Leu Val Lys Thr Ala Phe Asn
       50                  55                  60
   ccg gtt tca aca ttg atg tcg ttg act tgg aaa gaa tac gcc gtt tta      240
   Pro Val Ser Thr Leu Met Ser Leu Thr Trp Lys Glu Tyr Ala Val Leu
   65                  70                  75                  80
   tta tta agt gtg gtg tct ttt cct ctt atg gca caa gcc tct gat aca      288
   Leu Leu Ser Val Val Ser Phe Pro Leu Met Ala Gln Ala Ser Asp Thr
                   85                  90                  95
   gat tca gtg gta caa aga aaa cct gaa tta act gat gtg acg aat agc      336
   Asp Ser Val Val Gln Arg Lys Pro Glu Leu Thr Asp Val Thr Asn Ser
               100                 105                 110
   aac agc tat cat gtg gaa tta gat aga gag cat cat aaa ggg gag cat      384
   Asn Ser Tyr His Val Glu Leu Asp Arg Glu His His Lys Gly Glu His
           115                 120                 125
   caa aca aaa atc aaa cat act gag aat aat gtc atc att gtt gat att      432
   Gln Thr Lys Ile Lys His Thr Glu Asn Asn Val Ile Ile Val Asp Ile
       130                 135                 140
   gca aaa cca aac caa aag ggc att tca gat aac cgt ttt aaa cac ttc      480
   Ala Lys Pro Asn Gln Lys Gly Ile Ser Asp Asn Arg Phe Lys His Phe
   145                 150                 155                 160
   aac atc cca aat ggg gcg gta ttt aac aat agc gcc aag gaa aaa cgc      528
   Asn Ile Pro Asn Gly Ala Val Phe Asn Asn Ser Ala Lys Glu Lys Arg
                   165                 170                 175
   tca cag tta gtg ggg tat ttg cca ggt aac cag aat tta acg gaa ggt      576
   Ser Gln Leu Val Gly Tyr Leu Pro Gly Asn Gln Asn Leu Thr Glu Gly
               180                 185                 190
   agt gaa gca aaa gcg atc tta aat cag gtg act gga ccg gat gcc agt      624
   Ser Glu Ala Lys Ala Ile Leu Asn Gln Val Thr Gly Pro Asp Ala Ser
           195                 200                 205
   aaa att gaa ggc gcc ctt gaa att tta ggg caa aaa gcc gat ttg gtg      672
   Lys Ile Glu Gly Ala Leu Glu Ile Leu Gly Gln Lys Ala Asp Leu Val
       210                 215                 220
   att gcg aac caa aat ggc att gtg ctt aat ggg gta aaa acc att aat      720
   Ile Ala Asn Gln Asn Gly Ile Val Leu Asn Gly Val Lys Thr Ile Asn
   225                 230                 235                 240
   gcc aat cgt ttt gtg gca aca acc agt agt acc att gat cct gag caa      768
   Ala Asn Arg Phe Val Ala Thr Thr Ser Ser Thr Ile Asp Pro Glu Gln
                   245                 250                 255
   atg cag tta aat gtc acg caa ggt aca gtg aca att ggg gtg gat gga      816
   Met Gln Leu Asn Val Thr Gln Gly Thr Val Thr Ile Gly Val Asp Gly
               260                 265                 270
   ttt gcc aca gat ggc tta cct tat ttg gat atc att gcc aaa aag att      864
   Phe Ala Thr Asp Gly Leu Pro Tyr Leu Asp Ile Ile Ala Lys Lys Ile
           275                 280                 285
   gaa caa aaa caa gcg att aca aaa gaa aga aca gga aat tcc gaa acc      912
   Glu Gln Lys Gln Ala Ile Thr Lys Glu Arg Thr Gly Asn Ser Glu Thr
       290                 295                 300
   gat atc act ttt gtc gca ggt aac agt aaa tat gat tta aag aca cat      960
   Asp Ile Thr Phe Val Ala Gly Asn Ser Lys Tyr Asp Leu Lys Thr His
   305                 310                 315                 320
   caa gtg aca gaa aag cat acc gct gag gca caa ggt gaa att gcg att     1008
   Gln Val Thr Glu Lys His Thr Ala Glu Ala Gln Gly Glu Ile Ala Ile
                   325                 330                 335
   agc ggt gcg agt acc ggt gca atg tac ggt aaa aat atc aaa tta atc     1056
   Ser Gly Ala Ser Thr Gly Ala Met Tyr Gly Lys Asn Ile Lys Leu Ile
               340                 345                 350
   gta acg gat aaa ggc gct ggg gta aaa cat gat ggc att att tta tct     1104
   Val Thr Asp Lys Gly Ala Gly Val Lys His Asp Gly Ile Ile Leu Ser
           355                 360                 365
   gag gcg gat att caa att gaa acc cat gag ggc gat gtt gaa tta ggc     1152
   Glu Ala Asp Ile Gln Ile Glu Thr His Glu Gly Asp Val Glu Leu Gly
       370                 375                 380
   aat aca aaa aat aat cag aat gag aat tat gcc aaa gct cat gcg gaa     1200
   Asn Thr Lys Asn Asn Gln Asn Glu Asn Tyr Ala Lys Ala His Ala Glu
   385                 390                 395                 400
   ggg aat ttt acg gtt aaa ggc ggt aag cac gtt att att ggt aag gaa     1248
   Gly Asn Phe Thr Val Lys Gly Gly Lys His Val Ile Ile Gly Lys Glu
                   405                 410                 415
   gtt aaa gcc aac aaa gcg gtc gat att caa gca caa gaa aca aca gta     1296
   Val Lys Ala Asn Lys Ala Val Asp Ile Gln Ala Gln Glu Thr Thr Val
               420                 425                 430
   aga caa aat gcg aaa tta act gcc aaa acg agt gcc aaa att aca gca     1344
   Arg Gln Asn Ala Lys Leu Thr Ala Lys Thr Ser Ala Lys Ile Thr Ala
           435                 440                 445
   agt aag agt gtg aat ctt gaa gat aac gcg aaa ctt att gct aat gag     1392
   Ser Lys Ser Val Asn Leu Glu Asp Asn Ala Lys Leu Ile Ala Asn Glu
       450                 455                 460
   ctg agc aca aca acc aat aaa tta acc aat aaa ggt agc att tac ggc     1440
   Leu Ser Thr Thr Thr Asn Lys Leu Thr Asn Lys Gly Ser Ile Tyr Gly
   465                 470                 475                 480
   aag aaa gtg acg cta gat gct gat aat tta gtc aat agt aaa gaa atc     1488
   Lys Lys Val Thr Leu Asp Ala Asp Asn Leu Val Asn Ser Lys Glu Ile
                   485                 490                 495
   tat gcg tct agc gaa ctt gat att caa acc aaa ggt cgt gat ctt tta     1536
   Tyr Ala Ser Ser Glu Leu Asp Ile Gln Thr Lys Gly Arg Asp Leu Leu
               500                 505                 510
   ctt gag gat ggg gtt aat caa cca ctg agt ttc tta aaa ggc gct tca      1584
   Leu Glu Asp Gly Val Asn Gln Pro Leu Ser Phe Leu Lys Gly Ala Ser
           515                 520                 525
   ttg tta gcg ccg ggg ttt gtc aac act ggg cta att cac agt aac ggt      1632
   Leu Leu Ala Pro Gly Phe Val Asn Thr Gly Leu Ile His Ser Asn Gly
       530                 535                 540
   aat gcc aag ctc act ttt aaa gat gac acc agt ttt gtg act gaa gga      1680
   Asn Ala Lys Leu Thr Phe Lys Asp Asp Thr Ser Phe Val Thr Glu Gly
   545                 550                 555                 560
   aat aac ttt atc aca gca aaa gac aac tta gaa atc acg gca aaa aat      1728
   Asn Asn Phe Ile Thr Ala Lys Asp Asn Leu Glu Ile Thr Ala Lys Asn
                   565                 570                 575
   gtt caa att gat caa gcg aaa aat att caa tta aac gcg aat atc acg      1776
   Val Gln Ile Asp Gln Ala Lys Asn Ile Gln Leu Asn Ala Asn Ile Thr
               580                 585                 590
   atc aat acc aag tct ggt ttt gtg aat tac ggt acc tta gca agt gct      1824
   Ile Asn Thr Lys Ser Gly Phe Val Asn Tyr Gly Thr Leu Ala Ser Ala
           595                 600                 605
   caa aat tta acg att aat acc gaa caa ggc agc att tat aac ata ggc      1872
   Gln Asn Leu Thr Ile Asn Thr Glu Gln Gly Ser Ile Tyr Asn Ile Gly
       610                 615                 620
   ggt atc ttg ggg gcg ggt aaa agt ttg aat ctg agc gcg aaa aga gga      1920
   Gly Ile Leu Gly Ala Gly Lys Ser Leu Asn Leu Ser Ala Lys Arg Gly
   625                 630                 635                 640
   gaa aac caa gga gga tat ctt att aat caa ggt aag agt cta ctc cat      1968
   Glu Asn Gln Gly Gly Tyr Leu Ile Asn Gln Gly Lys Ser Leu Leu His
                   645                 650                 655
   tct gaa ggc gcc atg aac ctc aca gcg gat cgc acg gtg tac aat tta      2016
   Ser Glu Gly Ala Met Asn Leu Thr Ala Asp Arg Thr Val Tyr Asn Leu
               660                 665                 670
   ggg aat att ttt gct aaa ggt gac gcg acg atc aat gca aac gcg tta      2064
   Gly Asn Ile Phe Ala Lys Gly Asp Ala Thr Ile Asn Ala Asn Ala Leu
           675                 680                 685
   att aat gat gtt act ctc aca ggt cgt ctt gag tat caa gat ctg aaa      2112
   Ile Asn Asp Val Thr Leu Thr Gly Arg Leu Glu Tyr Gln Asp Leu Lys
       690                 695                 700
   aaa gat tat acg cgt tat tat cgt atc aat gaa acg gca aaa cat ggt      2160
   Lys Asp Tyr Thr Arg Tyr Tyr Arg Ile Asn Glu Thr Ala Lys His Gly
   705                 710                 715                 720
   tgg cat aat aac ttc tat gaa tta aac gtc gac aga gtt tct tg           2204
   Trp His Asn Asn Phe Tyr Glu Leu Asn Val Asp Arg Val Ser
                   725                 730
<210> 117
   <211> 734
   <212> PRT
   <213> Pasteurella Multocida
<400> 117
   Met Asn Lys Asn Arg Tyr Lys Leu Ile Phe Ser Lys Thr Lys Gly Cys
   1 5 10 15
   Leu Val Pro Val Ala Glu Thr Ile Asn Ser Ala Val Gly Asn Ala Ser
   20 25 30
   Ser Lys Asp Val Ser Asp Thr Glu Ile Ser Ala Ser Gln Pro Ala Leu
   35 40 45
   Asn Ser Pro Leu Ser Thr Leu Ser Val Leu Val Lys Thr Ala Phe Asn
   50 55 60
   Pro Val Ser Thr Leu Met Ser Leu Thr Trp Lys Glu Tyr Ala Val Leu
   65 70 75 80
   Leu Leu Ser Val Val Ser Phe Pro Leu Met Ala Gln Ala Ser Asp Thr
   85 90 95
   Asp Ser Val Val Gln Arg Lys Pro Glu Leu Thr Asp Val Thr Asn Ser
   100 105 110
   Asn Ser Tyr His Val Glu Leu Asp Arg Glu His His Lys Gly Glu His
   115 120 125
   Gln Thr Lys Ile Lys His Thr Glu Asn Asn Val Ile Ile Val Asp Ile
   130 135 140
   Ala Lys Pro Asn Gln Lys Gly Ile Ser Asp Asn Arg Phe Lys His Phe
   145 150 155 160
   Asn Ile Pro Asn Gly Ala Val Phe Asn Asn Ser Ala Lys Glu Lys Arg
   165 170 175
   Ser Gln Leu Val Gly Tyr Leu Pro Gly Asn Gln Asn Leu Thr Glu Gly
   180 185 190
   Ser Glu Ala Lys Ala Ile Leu Asn Gln Val Thr Gly Pro Asp Ala Ser
   195 200 205
   Lys Ile Glu Gly Ala Leu Glu Ile Leu Gly Gln Lys Ala Asp Leu Val
   210 215 220
   Ile Ala Asn Gln Asn Gly Ile Val Leu Asn Gly Val Lys Thr Ile Asn
   225                 230                 235                 240
   Ala Asn Arg Phe Val Ala Thr Thr Ser Ser Thr Ile Asp Pro Glu Gln
                   245                 250                 255
   Met Gln Leu Asn Val Thr Gln Gly Thr Val Thr Ile Gly Val Asp Gly
               260                 265                 270
   Phe Ala Thr Asp Gly Leu Pro Tyr Leu Asp Ile Ile Ala Lys Lys Ile
           275                 280                 285
   Glu Gln Lys Gln Ala Ile Thr Lys Glu Arg Thr Gly Asn Ser Glu Thr
       290                 295                 300
   Asp Ile Thr Phe Val Ala Gly Asn Ser Lys Tyr Asp Leu Lys Thr His
   305                 310                 315                 320
   Gln Val Thr Glu Lys His Thr Ala Glu Ala Gln Gly Glu Ile Ala Ile
                   325                 330                 335
   Ser Gly Ala Ser Thr Gly Ala Met Tyr Gly Lys Asn Ile Lys Leu Ile
               340                 345                 350
   Val Thr Asp Lys Gly Ala Gly Val Lys His Asp Gly Ile Ile Leu Ser
           355                 360                 365
   Glu Ala Asp Ile Gln Ile Glu Thr His Glu Gly Asp Val Glu Leu Gly
       370                 375                 380
   Asn Thr Lys Asn Asn Gln Asn Glu Asn Tyr Ala Lys Ala His Ala Glu
   385                 390                 395                 400
   Gly Asn Phe Thr Val Lys Gly Gly Lys His Val Ile Ile Gly Lys Glu
                   405                 410                 415
   Val Lys Ala Asn Lys Ala Val Asp Ile Gln Ala Gln Glu Thr Thr Val
               420                 425                 430
   Arg Gln Asn Ala Lys Leu Thr Ala Lys Thr Ser Ala Lys Ile Thr Ala
           435                 440                 445
   Ser Lys Ser Val Asn Leu Glu Asp Asn Ala Lys Leu Ile Ala Asn Glu
       450                 455                 460
   Leu Ser Thr Thr Thr Asn Lys Leu Thr Asn Lys Gly Ser Ile Tyr Gly
   465                 470                 475                 480
   Lys Lys Val Thr Leu Asp Ala Asp Asn Leu Val Asn Ser Lys Glu Ile
                   485                 490                 495
   Tyr Ala Ser Ser Glu Leu Asp Ile Gln Thr Lys Gly Arg Asp Leu Leu
               500                 505                 510
   Leu Glu Asp Gly Val Asn Gln Pro Leu Ser Phe Leu Lys Gly Ala Ser
           515                 520                 525
   Leu Leu Ala Pro Gly Phe Val Asn Thr Gly Leu Ile His Ser Asn Gly
       530                 535                 540
   Asn Ala Lys Leu Thr Phe Lys Asp Asp Thr Ser Phe Val Thr Glu Gly
   545                 550                 555                 560
   Asn Asn Phe Ile Thr Ala Lys Asp Asn Leu Glu Ile Thr Ala Lys Asn
                   565                 570                 575
   Val Gln Ile Asp Gln Ala Lys Asn Ile Gln Leu Asn Ala Asn Ile Thr
               580                 585                 590
   Ile Asn Thr Lys Ser Gly Phe Val Asn Tyr Gly Thr Leu Ala Ser Ala
           595                 600                 605
   Gln Asn Leu Thr Ile Asn Thr Glu Gln Gly Ser Ile Tyr Asn Ile Gly
       610                 615                 620
   Gly Ile Leu Gly Ala Gly Lys Ser Leu Asn Leu Ser Ala Lys Arg Gly
   625                 630                 635                 640
   Glu Asn Gln Gly Gly Tyr Leu Ile Asn Gln Gly Lys Ser Leu Leu His
                   645                 650                 655
   Ser Glu Gly Ala Met Asn Leu Thr Ala Asp Arg Thr Val Tyr Asn Leu
               660                 665                 670
   Gly Asn Ile Phe Ala Lys Gly Asp Ala Thr Ile Asn Ala Asn Ala Leu
           675                 680                 685
   Ile Asn Asp Val Thr Leu Thr Gly Arg Leu Glu Tyr Gln Asp Leu Lys
       690                 695                 700
   Lys Asp Tyr Thr Arg Tyr Tyr Arg Ile Asn Glu Thr Ala Lys His Gly
   705 710 715 720
   Trp His Asn Asn Phe Tyr Glu Leu Asn Val Asp Arg Val Ser
   725 730
<210> 118
   <211> 251
   <212> DNA
   <213> Pasteurella Multocida
<220>
   <221> misc_feature
   <223> unkO
<220>
   <221> CDS
   <222> (1) .. (249)
<400> 118
   atg aaa att act att aca cga aat cat cca gaa gta ttt caa gaa tcc       48
   Met Lys Ile Thr Ile Thr Arg Asn His Pro Glu Val Phe Gln Glu Ser
   1               5                   10                  15
   gct cgt tta gta gcc gaa aag ttc att aaa gcc caa tgt gta gaa gca       96
   Ala Arg Leu Val Ala Glu Lys Phe Ile Lys Ala Gln Cys Val Glu Ala
               20                  25                  30
   tta aca ttg gct ttg att gag ggt gtc gag cac ttt gtg ctg gaa ggt      144
   Leu Thr Leu Ala Leu Ile Glu Gly Val Glu His Phe Val Leu Glu Gly
           35                  40                  45
   gag gag gaa agc aaa agg gga cat agt att aag gtt gta tta aaa gga      192
   Glu Glu Glu Ser Lys Arg Gly His Ser Ile Lys Val Val Leu Lys Gly
       50                  55                  60
   agt cac gaa gtt att aag tca gag gtg aat aca aat gaa aaa aat cat      240
   Ser His Glu Val Ile Lys Ser Glu Val Asn Thr Asn Glu Lys Asn His
   65                  70                  75                  80
   tgt aat cat ta                                                       251
   Cys Asn His
<210> 119
   <211> 83
   <212> PRT
   <213> Pasteurella Multocida
<400> 119
   Met Lys Ile Thr Ile Thr Arg Asn His Pro Glu Val Phe Gln Glu Ser
   1               5                   10                  15
   Ala Arg Leu Val Ala Glu Lys Phe Ile Lys Ala Gln Cys Val Glu Ala
               20                  25                  30
   Leu Thr Leu Ala Leu Ile Glu Gly Val Glu His Phe Val Leu Glu Gly
           35                  40                  45
   Glu Glu Glu Ser Lys Arg Gly His Ser Ile Lys Val Val Leu Lys Gly
       50                  55                  60
   Ser His Glu Val Ile Lys Ser Glu Val Asn Thr Asn Glu Lys Asn His
   65                  70                  75                  80
   Cys Asn His
<210> 120
   <211> 548
   <212> DNA
   <213> Pasteurella Multocida
<220>
   <221> CDS
   <222> (1) .. (546)
   <223> unkP
<400> 120
   atg cgt gca tat ctt gat aaa gaa cag ggc tgg cat acg tct att tca       48
   Met Arg Ala Tyr Leu Asp Lys Glu Gln Gly Trp His Thr Ser Ile Ser
   1               5                   10                  15
   aat aaa ggc att aat ggc gtg agc ggt gtc aca caa cca ctc tat ttt       96
   Asn Lys Gly Ile Asn Gly Val Ser Gly Val Thr Gln Pro Leu Tyr Phe
               20                  25                  30
   gac att aac gac agc tcg act gat gtg aac tat ctc aat gaa caa ggc      144
   Asp Ile Asn Asp Ser Ser Thr Asp Val Asn Tyr Leu Asn Glu Gln Gly
           35                  40                  45
   atc acg tgt tgc gtg aat cat aat ggc ttt cgt ttt tgg ggc tta cgc      192
   Ile Thr Cys Cys Val Asn His Asn Gly Phe Arg Phe Trp Gly Leu Arg
       50                  55                  60
   acg act gca gaa gat cca tta ttc aag ttt gaa gtg tac acc cgc act      240
   Thr Thr Ala Glu Asp Pro Leu Phe Lys Phe Glu Val Tyr Thr Arg Thr
   65                  70                  75                  80
   gca caa atc tta aaa gat acg att gca ggg gcg ttt gat tgg gca gtg      288
   Ala Gln Ile Leu Lys Asp Thr Ile Ala Gly Ala Phe Asp Trp Ala Val
                   85                  90                  95
   gat aaa gat att tct gtc acg cta gtg aaa gat att att gaa gca atc      336
   Asp Lys Asp Ile Ser Val Thr Leu Val Lys Asp Ile Ile Glu Ala Ile
               100                 105                 110
   aat gcg aag tgg cgt gat tac acc aca aaa ggc tac tta att ggc ggt      384
   Asn Ala Lys Trp Arg Asp Tyr Thr Thr Lys Gly Tyr Leu Ile Gly Gly
           115                 120                 125
   aaa gcg tgg ctt aat aaa gag ctt aac agt gca acg aat tta aaa gat      432
   Lys Ala Trp Leu Asn Lys Glu Leu Asn Ser Ala Thr Asn Leu Lys Asp
       130                 135                 140
   gcg aag ttg ttg atc tct tat gat tat cac cca gta cca ccg ctc gaa      480
   Ala Lys Leu Leu Ile Ser Tyr Asp Tyr His Pro Val Pro Pro Leu Glu
   145                 150                 155                 160
   cag cta ggc ttt aat cag tac att tct gat gaa tac ctt gtt gat ttt      528
   Gln Leu Gly Phe Asn Gln Tyr Ile Ser Asp Glu Tyr Leu Val Asp Phe
                   165                 170                 175
   tca aat cgt tta gca tcg ta                                           548
   Ser Asn Arg Leu Ala Ser
               180
<210> 121
   <211> 182
   <212> PRT
   <213> Pasteurella Multocida
<400> 121
   Met Arg Ala Tyr Leu Asp Lys Glu Gln Gly Trp His Thr Ser Ile Ser
   1               5                   10                  15
   Asn Lys Gly Ile Asn Gly Val Ser Gly Val Thr Gln Pro Leu Tyr Phe
               20                  25                  30
   Asp Ile Asn Asp Ser Ser Thr Asp Val Asn Tyr Leu Asn Glu Gln Gly
           35                  40                  45
   Ile Thr Cys Cys Val Asn His Asn Gly Phe Arg Phe Trp Gly Leu Arg
       50                  55                  60
   Thr Thr Ala Glu Asp Pro Leu Phe Lys Phe Glu Val Tyr Thr Arg Thr
   65                  70                  75                  80
   Ala Gln Ile Leu Lys Asp Thr Ile Ala Gly Ala Phe Asp Trp Ala Val
                   85                  90                  95
   Asp Lys Asp Ile Ser Val Thr Leu Val Lys Asp Ile Ile Glu Ala Ile
               100                 105                 110
   Asn Ala Lys Trp Arg Asp Tyr Thr Thr Lys Gly Tyr Leu Ile Gly Gly
           115                 120                 125
   Lys Ala Trp Leu Asn Lys Glu Leu Asn Ser Ala Thr Asn Leu Lys Asp
       130                 135                 140
   Ala Lys Leu Leu Ile Ser Tyr Asp Tyr His Pro Val Pro Pro Leu Glu
           145                 150                 155                 160
   Gln Leu Gly Phe Asn Gln Tyr Ile Ser Asp Glu Tyr Leu Val Asp Phe
                   165                 170                 175
   Ser Asn Arg Leu Ala Ser
               180
<210> 122
   <211> 69
   <212> DNA
   <213> Actinobacillus pleuropneumoniae
<220>
   <221> misc_feature
   <223> apvA-or1
<220>
   <221> CDS
   <222> (1) .. (69)
<400> 122
   atg ttt tat gtc atg ctt gcc aat agg acg tct ata att tca tca atc        48
   Met Phe Tyr Val Met Leu Ala Asn Arg Thr Ser Ile Ile Ser Ser Ile
   1               5                   10                  15
   gat aag ttt aag ata ctt agc                                            69
   Asp Lys Phe Lys Ile Leu Ser
               20
<210> 123
   <211> 23
   <212> PRT
   <213> Actinobacillus pleuropneumoniae
<400> 123
   Met Phe Tyr Val Met Leu Ala Asn Arg Thr Ser Ile Ile Ser Ser Ile
   1               5                   10                  15
   Asp Lys Phe Lys Ile Leu Ser
               20
<210> 124
   <211> 64
   <212> DNA
   <213> Actinobacillus pleuropneumoniae
<220>
   <221> misc_feature
   <223> apvA-or2
<220>
   <221> CDS
   <222> (3) .. (62)
<400> 124
   ag cta agt atc tta aac tta tcg att gat gaa att ata gac gtc cta          47
      Leu Ser Ile Leu Asn Leu Ser Ile Asp Glu Ile Ile Asp Val Leu
      1               5                   10                  15
   ttg gca agc atg aca ta                                                  64
   Leu Ala Ser Met Thr
                   20
<210> 125
   <211> 20
   <212> PRT
   <213> Actinobacillus pleuropneumoniae
<400> 125
   Leu Ser Ile Leu Asn Leu Ser Ile Asp Glu Ile Ile Asp Val Leu Leu
   1               5                   10                  15
   Ala Ser Met Thr
               20
<210> 126
   <211> 653
   <212> DNA
   <213> Actinobacillus pleuropneumoniae
<220>
   <221> misc_feature
   <223> apvB
<220>
   <221> CDS
   <222> (1)..(651)
<400> 126
   tta att agc ttt cct ttt att act ttt gca agt aat gtt aat gga gcc      48
   Leu Ile Ser Phe Pro Phe Ile Thr Phe Ala Ser Asn Val Asn Gly Ala
   1               5                   10                  15
   gaa att gga ttg gga gga gcc cgt gag agt agt att tac tat tct aaa      96
   Glu Ile Gly Leu Gly Gly Ala Arg Glu Ser Ser Ile Tyr Tyr Ser Lys
               20                  25                  30
   cat aaa gta gca aca aat ccc ttt tta gca ctt gat ctt tct tta ggt     144
   His Lys Val Ala Thr Asn Pro Phe Leu Ala Leu Asp Leu Ser Leu Gly
           35                  40                  45
   aat ttt tat atg aga ggg act gca gga att agc gaa ata gga tat gaa     192
   Asn Phe Tyr Met Arg Gly Thr Ala Gly Ile Ser Glu Ile Gly Tyr Glu
       50                  55                  60
   caa tct ttc act gac aat ttc agc gta tca ctg ttt gtt aac cca ttt     240
   Gln Ser Phe Thr Asp Asn Phe Ser Val Ser Leu Phe Val Asn Pro Phe
   65                  70                  75                  80
   gat ggt ttt tca att aaa gga aaa gac ttg tta cct gga tat caa agt      288
   Asp Gly Phe Ser Ile Lys Gly Lys Asp Leu Leu Pro Gly Tyr Gln Ser
                   85                  90                  95
   att caa act cgc aaa act caa ttt gcc ttt ggt tgg gga tta aat tat      336
   Ile Gln Thr Arg Lys Thr Gln Phe Ala Phe Gly Trp Gly Leu Asn Tyr
               100                 105                 110
   aat ttg gga ggt tta ttc ggc tta aat gat act ttt ata tcc ttg gaa      384
   Asn Leu Gly Gly Leu Phe Gly Leu Asn Asp Thr Phe Ile Ser Leu Glu
           115                 120                 125
   gga aaa agc gga aaa cgt ggt gcg agt agt aat gtc agc tta ctt aaa      432
   Gly Lys Ser Gly Lys Arg Gly Ala Ser Ser Asn Val Ser Leu Leu Lys
       130                 135                 140
   tcg ttt aat atg acg aaa aat tgg aaa gtt tca cca tat att ggc tca      480
   Ser Phe Asn Met Thr Lys Asn Trp Lys Val Ser Pro Tyr Ile Gly Ser
   145                 150                 155                 160
   agt tat tat tca tct aaa tat aca gat tat tac ttt ggt att aaa caa      528
   Ser Tyr Tyr Ser Ser Lys Tyr Thr Asp Tyr Tyr Phe Gly Ile Lys Gln
                   165                 170                 175
   tcc gaa tta ggt aat aaa att aca tcc gta tat aaa cct aaa gca gct      576
   Ser Glu Leu Gly Asn Lys Ile Thr Ser Val Tyr Lys Pro Lys Ala Ala
               180                 185                 190
   tat gca aca cac ata ggt att aat act gat tat gct ttc acg aac aat      624
   Tyr Ala Thr His Ile Gly Ile Asn Thr Asp Tyr Ala Phe Thr Asn Asn
           195                 200                 205
   ctt ggc atg ggt tta tct gtc ggt tgg at                               653
   Leu Gly Met Gly Leu Ser Val Gly Trp
       210                 215
<210> 127
   <211> 217
   <212> PRT
   <213> Actinobacillus pleuropneumoniae
<400> 127
   Leu Ile Ser Phe Pro Phe Ile Thr Phe Ala Ser Asn Val Asn Gly Ala
   1               5                   10                  15
   Glu Ile Gly Leu Gly Gly Ala Arg Glu Ser Ser Ile Tyr Tyr Ser Lys
               20                  25                  30
   His Lys Val Ala Thr Asn Pro Phe Leu Ala Leu Asp Leu Ser Leu Gly
           35                  40                  45
   Asn Phe Tyr Met Arg Gly Thr Ala Gly Ile Ser Glu Ile Gly Tyr Glu
       50                  55                  60
   Gln Ser Phe Thr Asp Asn Phe Ser Val Ser Leu Phe Val Asn Pro Phe
   65                  70                  75                  80
   Asp Gly Phe Ser Ile Lys Gly Lys Asp Leu Leu Pro Gly Tyr Gln Ser
                   85                  90                  95
   Ile Gln Thr Arg Lys Thr Gln Phe Ala Phe Gly Trp Gly Leu Asn Tyr
               100                 105                 110
   Asn Leu Gly Gly Leu Phe Gly Leu Asn Asp Thr Phe Ile Ser Leu Glu
           115                 120                 125
   Gly Lys Ser Gly Lys Arg Gly Ala Ser Ser Asn Val Ser Leu Leu Lys
       130                 135                 140
   Ser Phe Asn Met Thr Lys Asn Trp Lys Val Ser Pro Tyr Ile Gly Ser
   145                 150                 155                 160
   Ser Tyr Tyr Ser Ser Lys Tyr Thr Asp Tyr Tyr Phe Gly Ile Lys Gln
                   165                 170                 175
   Ser Glu Leu Gly Asn Lys Ile Thr Ser Val Tyr Lys Pro Lys Ala Ala
               180                 185                 190
   Tyr Ala Thr His Ile Gly Ile Asn Thr Asp Tyr Ala Phe Thr Asn Asn
           195                 200                 205
   Leu Gly Met Gly Leu Ser Val Gly Trp
       210                 215
<210> 128
   <211> 242
   <212> DNA
   <213> Actinobacillus pleuropneumoniae
<220>
   <221> misc_feature
   <223> apvC
<220>
   <221> CDS
   <222> (1) .. (240)
<400> 128
   atg tgg cgg atg gga gat ttt atg tct aaa aaa gag agg ctg aat gat      48
   Met Trp Arg Met Gly Asp Phe Met Ser Lys Lys Glu Arg Leu Asn Asp
   1               5                   10                  15
   atg gct cgc cag att tta tca gcg gcg gag ttg ctc att gca aag gaa      96
   Met Ala Arg Gln Ile Leu Ser Ala Ala Glu Leu Leu Ile Ala Lys Glu
               20                  25                  30
   ggt ttg caa aat tta tcg atg agg aaa atc gca agt gaa gcc ggt atc      144
   Gly Leu Gln Asn Leu Ser Met Arg Lys Ile Ala Ser Glu Ala Gly Ile
           35                  40                  45
   gca aca ggc acg ctt tat ctc tat ttc aaa acg aaa gac gag tta ctg      192
   Ala Thr Gly Thr Leu Tyr Leu Tyr Phe Lys Thr Lys Asp Glu Leu Leu
       50                  55                  60
   gat tgt ttg gcg gaa caa tta cat gaa cga tat tat cgt tat ctg aat      240
   Asp Cys Leu Ala Glu Gln Leu His Glu Arg Tyr Tyr Arg Tyr Leu Asn
   65                  70                  75                  80
   at                                                                    242
<210> 129
   <211> 80
   <212> PRT
   <213> Actinobacillus pleuropneumoniae
<400> 129
   Met Trp Arg Met Gly Asp Phe Met Ser Lys Lys Glu Arg Leu Asn Asp
   1               5                   10                  15
   Met Ala Arg Gln Ile Leu Ser Ala Ala Glu Leu Leu Ile Ala Lys Glu
               20                  25                  30
   Gly Leu Gln Asn Leu Ser Met Arg Lys Ile Ala Ser Glu Ala Gly Ile
           35                  40                  45
   Ala Thr Gly Thr Leu Tyr Leu Tyr Phe Lys Thr Lys Asp Glu Leu Leu
       50                  55                  60
   Asp Cys Leu Ala Glu Gln Leu His Glu Arg Tyr Tyr Arg Tyr Leu Asn
   65                  70                  75                  80
<210> 130
   <211> 527
   <212> DNA
   <213> Actinobacillus pleuropneumoniae
<220>
   <221> misc_feature
   <223> apvD
<220>
   <221> CDS
   <222> (1)..(525)
<400> 130
   aat att caa aaa aca gtt att gct agc ggc aca ttg caa gcg act gaa       48
   Asn Ile Gln Lys Thr Val Ile Ala Ser Gly Thr Leu Gln Ala Thr Glu
   1               5                   10                  15
   caa gta gat att ggt gca caa gta tct ggg cag att aag cat att tta       96
   Gln Val Asp Ile Gly Ala Gln Val Ser Gly Gln Ile Lys His Ile Leu
               20                  25                  30
   gta caa gaa gga cag aag gtt aaa aaa ggt gag cta tta gct gta att      144
   Val Gln Glu Gly Gln Lys Val Lys Lys Gly Glu Leu Leu Ala Val Ile
           35                  40                  45
   gat cca cgt ctg gct gaa acg gaa tta aaa cta gca aaa gct gag cta      192
   Asp Pro Arg Leu Ala Glu Thr Glu Leu Lys Leu Ala Lys Ala Glu Leu
       50                  55                  60
   gca aat gct tct gct aat ttg gat aca aaa aaa att aat ctt aag caa      240
   Ala Asn Ala Ser Ala Asn Leu Asp Thr Lys Lys Ile Asn Leu Lys Gln
   65                  70                  75                  80
   ctg caa tca gat tgg gaa cgt cat caa cgt ttg ata cga acc aat gcg      288
   Leu Gln Ser Asp Trp Glu Arg His Gln Arg Leu Ile Arg Thr Asn Ala
                   85                  90                  95
   aca agc caa aag gaa aca gaa gaa gca aaa agt aga tta aat acg gcc      336
   Thr Ser Gln Lys Glu Thr Glu Glu Ala Lys Ser Arg Leu Asn Thr Ala
               100                 105                 110
   aaa gca gaa ctt caa att gcg caa aat aat cta gat atc gct aaa atc      384
   Lys Ala Glu Leu Gln Ile Ala Gln Asn Asn Leu Asp Ile Ala Lys Ile
           115                 120                 125
   aga gtg gaa aaa gct gaa acc gaa cta gga tat aca gaa att cgt tct      432
   Arg Val Glu Lys Ala Glu Thr Glu Leu Gly Tyr Thr Glu Ile Arg Ser
       130                 135                 140
   cca ctt gat gca aca gta att tca gta ttt gcg caa aat ggt caa act      480
   Pro Leu Asp Ala Thr Val Ile Ser Val Phe Ala Gln Asn Gly Gln Thr
   145                 150                 155                 160
   tta gtc acc acc caa caa gta cca gtg ctg atg aaa tta gct aat at       527
   Leu Val Thr Thr Gln Gln Val Pro Val Leu Met Lys Leu Ala Asn
                   165                 170                 175
<210> 131
   <211> 175
   <212> PRT
   <213> Actinobacillus pleuropneumoniae
<400> 131
   Asn Ile Gln Lys Thr Val Ile Ala Ser Gly Thr Leu Gln Ala Thr Glu
   1               5                   10                  15
   Gln Val Asp Ile Gly Ala Gln Val Ser Gly Gln Ile Lys His Ile Leu
               20                  25                  30
   Val Gln Glu Gly Gln Lys Val Lys Lys Gly Glu Leu Leu Ala Val Ile
           35                  40                  45
   Asp Pro Arg Leu Ala Glu Thr Glu Leu Lys Leu Ala Lys Ala Glu Leu
       50                  55                  60
   Ala Asn Ala Ser Ala Asn Leu Asp Thr Lys Lys Ile Asn Leu Lys Gln
   65                  70                  75                  80
   Leu Gln Ser Asp Trp Glu Arg His Gln Arg Leu Ile Arg Thr Asn Ala
                   85                  90                  95
   Thr Ser Gln Lys Glu Thr Glu Glu Ala Lys Ser Arg Leu Asn Thr Ala
               100                 105                 110
   Lys Ala Glu Leu Gln Ile Ala Gln Asn Asn Leu Asp Ile Ala Lys Ile
           115                 120                 125
   Arg Val Glu Lys Ala Glu Thr Glu Leu Gly Tyr Thr Glu Ile Arg Ser
       130                 135                 140
   Pro Leu Asp Ala Thr Val Ile Ser Val Phe Ala Gln Asn Gly Gln Thr
   145                 150                 155                 160
   Leu Val Thr Thr Gln Gln Val Pro Val Leu Met Lys Leu Ala Asn
                   165                 170                 175
<210> 132
   <211> 867
   <212> DNA
   <213> Actinobacillus pleuropneumoniae
<220>
   <221> misc_feature
   <223> atpG
<220>
   <221> CDS
   <222> (1) .. (864)
<400> 132
   atg gca ggt gcg aaa gag ata aga acc aaa att gca agt gtg aaa aat       48
   Met Ala Gly Ala Lys Glu Ile Arg Thr Lys Ile Ala Ser Val Lys Asn
   1               5                   10                  15
   act caa aaa atc acc aaa gca atg gaa atg gtt gct acc tct aaa atg       96
   Thr Gln Lys Ile Thr Lys Ala Met Glu Met Val Ala Thr Ser Lys Met
               20                  25                  30
   cgt aaa acg caa gag cgt atg gct gcc agt cgt cct tat tcg gaa aca      144
   Arg Lys Thr Gln Glu Arg Met Ala Ala Ser Arg Pro Tyr Ser Glu Thr
           35                  40                  45
   atc cgt aag gtg att agc cat att gcg aaa gga agc att ggt tat aag      192
   Ile Arg Lys Val Ile Ser His Ile Ala Lys Gly Ser Ile Gly Tyr Lys
       50                  55                  60
   cac ccg ttt tta act gaa cgt gat att aaa aaa gta ggc tat ctt gtc      240
   His Pro Phe Leu Thr Glu Arg Asp Ile Lys Lys Val Gly Tyr Leu Val
   65                  70                  75                  80
   gtt tcg acc gat cgc ggt tta tgc ggt ggc ctt aat atc aat tta ttc      288
   Val Ser Thr Asp Arg Gly Leu Cys Gly Gly Leu Asn Ile Asn Leu Phe
                   85                  90                  95
   aaa gcg act ttg aat gaa ttt aaa acg tgg aaa gat aaa gac gtt agt      336
   Lys Ala Thr Leu Asn Glu Phe Lys Thr Trp Lys Asp Lys Asp Val Ser
               100                 105                 110
   gtt gag ctt ggt tta gta ggg tcg aaa ggc gta agc ttt tac caa aat      384
   Val Glu Leu Gly Leu Val Gly Ser Lys Gly Val Ser Phe Tyr Gln Asn
           115                 120                 125
   cta ggc tta aac gtg aga tct caa gta acg gga tta ggc gat aat ccg      432
   Leu Gly Leu Asn Val Arg Ser Gln Val Thr Gly Leu Gly Asp Asn Pro
       130                 135                 140
   gaa atg gaa cgt atc gtg ggc gca gtt aat gaa atg att aat gcg ttc      480
   Glu Met Glu Arg Ile Val Gly Ala Val Asn Glu Met Ile Asn Ala Phe
   145                 150                 155                 160
   cga aac gga gaa gtg gat gcg gtt tac gtc gct tac aac cgt ttt gaa      528
   Arg Asn Gly Glu Val Asp Ala Val Tyr Val Ala Tyr Asn Arg Phe Glu
                   165                 170                 175
   aat acg atg tca caa aaa cct gtt atc gca cag tta ctt ccg tta cct      576
   Asn Thr Met Ser Gln Lys Pro Val Ile Ala Gln Leu Leu Pro Leu Pro
               180                 185                 190
   aaa cta gat gac gat gaa tta gat acg aaa ggt tca tgg gat tat att      624
   Lys Leu Asp Asp Asp Glu Leu Asp Thr Lys Gly Ser Trp Asp Tyr Ile
           195                 200                 205
   tat gaa ccg aat cca caa gtt tta ttg gat agt tta ctt gtt cgt tat      672
   Tyr Glu Pro Asn Pro Gln Val Leu Leu Asp Ser Leu Leu Val Arg Tyr
       210                 215                 220
   tta gaa act cag gta tac caa gca gtt gta gat aac cta gct tct gaa      720
   Leu Glu Thr Gln Val Tyr Gln Ala Val Val Asp Asn Leu Ala Ser Glu
   225                 230                 235                 240
   caa gcc gct cga atg gta gcg atg aaa gcc gca aca gat aat gcg ggt      768
   Gln Ala Ala Arg Met Val Ala Met Lys Ala Ala Thr Asp Asn Ala Gly
                   245                 250                 255
   aca tta atc gat gaa tta caa tta gtg tat aac aaa gct cgc caa gca      816
   Thr Leu Ile Asp Glu Leu Gln Leu Val Tyr Asn Lys Ala Arg Gln Ala
               260                 265                 270
   agc att aca aat gaa tta aac gaa att gtt gcg ggt gcc gca gca att      864
   Ser Ile Thr Asn Glu Leu Asn Glu Ile Val Ala Gly Ala Ala Ala Ile
           275                 280                 285
   taa                                                                  867
<210> 133
   <211> 288
   <212> PRT
   <213> Actinobacillus pleuropneumoniae
<400> 133
   Met Ala Gly Ala Lys Glu Ile Arg Thr Lys Ile Ala Ser Val Lys Asn
   1               5                   10                  15
   Thr Gln Lys Ile Thr Lys Ala Met Glu Met Val Ala Thr Ser Lys Met
               20                  25                  30
   Arg Lys Thr Gln Glu Arg Met Ala Ala Ser Arg Pro Tyr Ser Glu Thr
           35                  40                  45
   Ile Arg Lys Val Ile Ser His Ile Ala Lys Gly Ser Ile Gly Tyr Lys
       50                  55                  60
   His Pro Phe Leu Thr Glu Arg Asp Ile Lys Lys Val Gly Tyr Leu Val
   65                  70                  75                  80
   Val Ser Thr Asp Arg Gly Leu Cys Gly Gly Leu Asn Ile Asn Leu Phe
                   85                  90                  95
   Lys Ala Thr Leu Asn Glu Phe Lys Thr Trp Lys Asp Lys Asp Val Ser
               100                 105                 110
   Val Glu Leu Gly Leu Val Gly Ser Lys Gly Val Ser Phe Tyr Gln Asn
           115                 120                 125
   Leu Gly Leu Asn Val Arg Ser Gln Val Thr Gly Leu Gly Asp Asn Pro
       130                 135                 140
   Glu Met Glu Arg Ile Val Gly Ala Val Asn Glu Met Ile Asn Ala Phe
   145                 150                 155                 160
   Arg Asn Gly Glu Val Asp Ala Val Tyr Val Ala Tyr Asn Arg Phe Glu
                   165                 170                 175
   Asn Thr Met Ser Gln Lys Pro Val Ile Ala Gln Leu Leu Pro Leu Pro
               180                 185                 190
   Lys Leu Asp Asp Asp Glu Leu Asp Thr Lys Gly Ser Trp Asp Tyr Ile
           195                 200                 205
   Tyr Glu Pro Asn Pro Gln Val Leu Leu Asp Ser Leu Leu Val Arg Tyr
       210                 215                 220
   Leu Glu Thr Gln Val Tyr Gln Ala Val Val Asp Asn Leu Ala Ser Glu
   225                 230                 235                 240
   Gln Ala Ala Arg Met Val Ala Met Lys Ala Ala Thr Asp Asn Ala Gly
                   245                 250                 255
   Thr Leu Ile Asp Glu Leu Gln Leu Val Tyr Asn Lys Ala Arg Gln Ala
               260                 265                 270
   Ser Ile Thr Asn Glu Leu Asn Glu Ile Val Ala Gly Ala Ala Ala Ile
           275                 280                 285
<210> 134
   <211> 534
   <212> DNA
   <213> Actinobacillus pleuropneumoniae
<220>
   <221> misc_feature
   <223> atpH
<220>
   <221> CDS
   <222> (1)..(531)
<400> 134
   atg tca gaa tta agt aca gta gct cgc ccc tac gct aaa gca gct ttt       48
   Met Ser Glu Leu Ser Thr Val Ala Arg Pro Tyr Ala Lys Ala Ala Phe
   1               5                   10                  15
   gat ttt gct tta gaa caa ggt cag ttg gac aaa tgg caa gaa atg tta       96
   Asp Phe Ala Leu Glu Gln Gly Gln Leu Asp Lys Trp Gln Glu Met Leu
               20                  25                  30
   cag ttt tcg gca ttc gtt gct gaa aac gaa caa gtg gcg gaa tat att      144
   Gln Phe Ser Ala Phe Val Ala Glu Asn Glu Gln Val Ala Glu Tyr Ile
           35                  40                  45
   aat tct tcc ctt gca agc ggt cag att tct gaa act ttt atc aaa atc      192
   Asn Ser Ser Leu Ala Ser Gly Gln Ile Ser Glu Thr Phe Ile Lys Ile
       50                  55                  60
   tgc ggc gac caa ctt gat caa tat ggg caa aat ttt att cgt gta atg      240
   Cys Gly Asp Gln Leu Asp Gln Tyr Gly Gln Asn Phe Ile Arg Val Met
   65                  70                  75                  80
   gct gaa aat aaa cgt ctg gct gtg ttg cct atg gtt ttt gat act ttc      288
   Ala Glu Asn Lys Arg Leu Ala Val Leu Pro Met Val Phe Asp Thr Phe
                   85                  90                  95
   gta tca tta cga gcg gaa cat gaa gcg gta aaa gat gta aca att gtt      336
   Val Ser Leu Arg Ala Glu His Glu Ala Val Lys Asp Val Thr Ile Val
                   100                 105                 110
   tcg gca aac gaa tta agt caa gca caa gaa gat aaa atc gca aaa gcg       384
   Ser Ala Asn Glu Leu Ser Gln Ala Gln Glu Asp Lys Ile Ala Lys Ala
           115                 120                 125
   atg gaa aaa cgc tta ggt caa aaa gtt cgt tta acc aac caa atc gat       432
   Met Glu Lys Arg Leu Gly Gln Lys Val Arg Leu Thr Asn Gln Ile Asp
       130                 135                 140
   aac agc ctg att gca ggc gta att att aaa tac gat gat gtt gtt att       480
   Asn Ser Leu Ile Ala Gly Val Ile Ile Lys Tyr Asp Asp Val Val Ile
   145                 150                 155                 160
   gat ggt agt agc cgc ggt cag tta aat cgc tta gcg tca gcg ttg agc       528
   Asp Gly Ser Ser Arg Gly Gln Leu Asn Arg Leu Ala Ser Ala Leu Ser
                   165                 170                 175
   ttg taa                                                               534
   Leu
<210> 135
   <211> 177
   <212> PRT
   <213> Actinobacillus pleuropneumoniae
<400> 135
   Met Ser Glu Leu Ser Thr Val Ala Arg Pro Tyr Ala Lys Ala Ala Phe
   1               5                   10                  15
   Asp Phe Ala Leu Glu Gln Gly Gln Leu Asp Lys Trp Gln Glu Met Leu
               20                  25                  30
   Gln Phe Ser Ala Phe Val Ala Glu Asn Glu Gln Val Ala Glu Tyr Ile
           35                  40                  45
   Asn Ser Ser Leu Ala Ser Gly Gln Ile Ser Glu Thr Phe Ile Lys Ile
       50                  55                  60
   Cys Gly Asp Gln Leu Asp Gln Tyr Gly Gln Asn Phe Ile Arg Val Met
   65                  70                  75                  80
   Ala Glu Asn Lys Arg Leu Ala Val Leu Pro Met Val Phe Asp Thr Phe
                   85                  90                  95
   Val Ser Leu Arg Ala Glu His Glu Ala Val Lys Asp Val Thr Ile Val
               100                 105                 110
   Ser Ala Asn Glu Leu Ser Gln Ala Gln Glu Asp Lys Ile Ala Lys Ala
           115                 120                 125
   Met Glu Lys Arg Leu Gly Gln Lys Val Arg Leu Thr Asn Gln Ile Asp
       130                 135                 140
   Asn Ser Leu Ile Ala Gly Val Ile Ile Lys Tyr Asp Asp Val Val Ile
   145                 150                 155                 160
   Asp Gly Ser Ser Arg Gly Gln Leu Asn Arg Leu Ala Ser Ala Leu Ser
                   165                 170                 175
   Leu
<210> 136
   <211> 321
   <212> DNA
   <213> Actinobacillus pleuropneumoniae
<220>
   <221> misc_feature
   <223> dksA
<220>
   <221> CDS
   <222> (1) .. (318)
<400> 136
   gca tgg cat gtg caa att atg gac gaa gct gag cgt aca aaa aac caa       48
   Ala Trp His Val Gln Ile Met Asp Glu Ala Glu Arg Thr Lys Asn Gln
   1               5                   10                  15
   atg cag gaa gaa gtc gct aat ttc gcc gat cct gcg gac cgc gcc act       96
   Met Gln Glu Glu Val Ala Asn Phe Ala Asp Pro Ala Asp Arg Ala Thr
               20                  25                  30
   cag gaa gaa gaa ttc agt ctt gaa tta aga aac cgt gac cgt gag cgt      144
   Gln Glu Glu Glu Phe Ser Leu Glu Leu Arg Asn Arg Asp Arg Glu Arg
           35                  40                  45
   aaa ttg ctt aag aag att gag caa acg tta aat agc att gcc gaa gac      192
   Lys Leu Leu Lys Lys Ile Glu Gln Thr Leu Asn Ser Ile Ala Glu Asp
       50                  55                  60
   gaa tac ggc tat tgc gaa act tgc ggt gtt gaa atc ggt tta cgt cgt      240
   Glu Tyr Gly Tyr Cys Glu Thr Cys Gly Val Glu Ile Gly Leu Arg Arg
   65                  70                  75                  80
   tta gaa gcg cgc ccg acc gcg gat atg tgt atc gat tgc aaa aca ctt      288
   Leu Glu Ala Arg Pro Thr Ala Asp Met Cys Ile Asp Cys Lys Thr Leu
                   85                  90                  95
   gcg gaa atc cgt gaa aag caa atg ggc tta taa                          321
   Ala Glu Ile Arg Glu Lys Gln Met Gly Leu
               100                 105
<210> 137
   <211> 106
   <212> PRT
   <213> Actinobacillus pleuropneumoniae
<400> 137
   Ala Trp His Val Gln Ile Met Asp Glu Ala Glu Arg Thr Lys Asn Gln
   1               5                   10                  15
   Met Gln Glu Glu Val Ala Asn Phe Ala Asp Pro Ala Asp Arg Ala Thr
               20                  25                  30
   Gln Glu Glu Glu Phe Ser Leu Glu Leu Arg Asn Arg Asp Arg Glu Arg
           35                  40                  45
   Lys Leu Leu Lys Lys Ile Glu Gln Thr Leu Asn Ser Ile Ala Glu Asp
       50                  55                  60
   Glu Tyr Gly Tyr Cys Glu Thr Cys Gly Val Glu Ile Gly Leu Arg Arg
   65                  70                  75                  80
   Leu Glu Ala Arg Pro Thr Ala Asp Met Cys Ile Asp Cys Lys Thr Leu
                   85                  90                  95
   Ala Glu Ile Arg Glu Lys Gln Met Gly Leu
               100                 105
<210> 138
   <211> 33
   <212> DNA
   <213> Actinobacillus pleuropneumoniae
<220>
   <221> misc_feature
   <223> dnaK
<220>
   <221> CDS
   <222> (1)..(30)
<400> 138
   gct gag ttt gaa gaa gtg aaa gat aat aaa taa                      33
   Ala Glu Phe Glu Glu Val Lys Asp Asn Lys
   1               5                   10
<210> 139
   <211> 10
   <212> PRT
   <213> Actinobacillus pleuropneumoniae
<400> 139
   Ala Glu Phe Glu Glu Val Lys Asp Asn Lys
   1               5                   10
<210> 140
   <211> 453
   <212> DNA
   <213> Actinobacillus pleuropneumoniae
<220>
   <221> misc_feature
   <223> exbB
<220>
   <221> CDS
   <222> (1) .. (450)
<400> 140
   atg gaa caa atg ctt gaa ctt tta caa ggt cat gtt gat tat att att        48
   Met Glu Gln Met Leu Glu Leu Leu Gln Gly His Val Asp Tyr Ile Ile
   1               5                   10                  15
   tta ggc tta tta cta tta atg agt gtt gtg ttg gta tgg aaa att att        96
   Leu Gly Leu Leu Leu Leu Met Ser Val Val Leu Val Trp Lys Ile Ile
               20                  25                  30
   gaa cgc gta ctt ttc tac aaa caa ttg gat gtg acc aaa tat gac acg       144
   Glu Arg Val Leu Phe Tyr Lys Gln Leu Asp Val Thr Lys Tyr Asp Thr
           35                  40                  45
   cta caa gat ttg gaa att gat acc act cgc aat tta acc acc att tcc       192
   Leu Gln Asp Leu Glu Ile Asp Thr Thr Arg Asn Leu Thr Thr Ile Ser
       50                  55                  60
   act atc ggt gcc aac gcc cct tat atc ggt tta tta gga acc gta tta       240
   Thr Ile Gly Ala Asn Ala Pro Tyr Ile Gly Leu Leu Gly Thr Val Leu
   65                  70                  75                  80
   ggg atc tta ctt acc ttc tat cat tta ggg cat tcc ggc ggt gat att       288
   Gly Ile Leu Leu Thr Phe Tyr His Leu Gly His Ser Gly Gly Asp Ile
                   85                  90                  95
   gac gcc gca tcc att atg gtt cac ctt tcg ctt gca tta aaa gca acc       336
   Asp Ala Ala Ser Ile Met Val His Leu Ser Leu Ala Leu Lys Ala Thr
               100                 105                 110
   gca gcc ggt atc tta gtc gct att ccg gca atg atg ttc tac agc ggt       384
   Ala Ala Gly Ile Leu Val Ala Ile Pro Ala Met Met Phe Tyr Ser Gly
           115                 120                 125
   ttt aac cgt aaa gtg gat gaa agc aaa ctt aaa tgg caa gcg att caa       432
   Phe Asn Arg Lys Val Asp Glu Ser Lys Leu Lys Trp Gln Ala Ile Gln
       130                 135                 140
   gct cgt aaa gcc aat caa taa                                           453
   Ala Arg Lys Ala Asn Gln
   145                 150
<210> 141
   <211> 150
   <212> PRT
   <213> Actinobacillus pleuropneumoniae
<400> 141
   Met Glu Gln Met Leu Glu Leu Leu Gln Gly His Val Asp Tyr Ile Ile
   1               5                   10                  15
   Leu Gly Leu Leu Leu Leu Met Ser Val Val Leu Val Trp Lys Ile Ile
               20                  25                  30
   Glu Arg Val Leu Phe Tyr Lys Gln Leu Asp Val Thr Lys Tyr Asp Thr
           35                  40                  45
   Leu Gln Asp Leu Glu Ile Asp Thr Thr Arg Asn Leu Thr Thr Ile Ser
       50                  55                  60
   Thr Ile Gly Ala Asn Ala Pro Tyr Ile Gly Leu Leu Gly Thr Val Leu
   65                  70                  75                  80
   Gly Ile Leu Leu Thr Phe Tyr His Leu Gly His Ser Gly Gly Asp Ile
                   85                  90                  95
   Asp Ala Ala Ser Ile Met Val His Leu Ser Leu Ala Leu Lys Ala Thr
               100                 105                 110
   Ala Ala Gly Ile Leu Val Ala Ile Pro Ala Met Met Phe Tyr Ser Gly
           115                 120                 125
   Phe Asn Arg Lys Val Asp Glu Ser Lys Leu Lys Trp Gln Ala Ile Gln
       130                 135                 140
   Ala Arg Lys Ala Asn Gln
   145                 150
<210> 142
   <211> 720
   <212> DNA
   <213> Actinobacillus pleuropneumoniae
<220>
   <221> misc_feature
   <223> fkpA
<220>
   <221> CDS
   <222> (1) .. (717)
<400> 142
   atg tta aaa aat aaa ctt tct gtt ctt gca atc gta gcc ggt acg ttc      48
   Met Leu Lys Asn Lys Leu Ser Val Leu Ala Ile Val Ala Gly Thr Phe
   1               5                   10                  15
   gtt tca gct caa act gca ttt gca gcg gat caa aaa ttc att gac gat      96
   Val Ser Ala Gln Thr Ala Phe Ala Ala Asp Gln Lys Phe Ile Asp Asp
               20                  25                  30
   tca tca tat gca gtc ggc gta ttg atg ggt aaa aat atc gaa ggc gtc      144
   Ser Ser Tyr Ala Val Gly Val Leu Met Gly Lys Asn Ile Glu Gly Val
           35                  40                  45
   gtt gaa tca caa aaa gaa att ttt tct tat aac caa gat aaa atc ttg      192
   Val Glu Ser Gln Lys Glu Ile Phe Ser Tyr Asn Gln Asp Lys Ile Leu
       50                  55                  60
   gcg ggt gtc caa gat acc atc aaa aaa acc ggt aaa tta acc gat gaa      240
   Ala Gly Val Gln Asp Thr Ile Lys Lys Thr Gly Lys Leu Thr Asp Glu
   65                  70                  75                  80
   gat cta caa aaa caa tta aaa tcg ctt gat act tat ctt gca agt caa      288
   Asp Leu Gln Lys Gln Leu Lys Ser Leu Asp Thr Tyr Leu Ala Ser Gln
                   85                  90                  95
   gaa agc aaa att gcg gcg gag aaa agc aaa gca acc gta gaa gcc ggt      336
   Glu Ser Lys Ile Ala Ala Glu Lys Ser Lys Ala Thr Val Glu Ala Gly
               100                 105                 110
   aat aaa ttt cgt acc gac tac gaa aaa caa agc ggc gtg aaa aaa acc      384
   Asn Lys Phe Arg Thr Asp Tyr Glu Lys Gln Ser Gly Val Lys Lys Thr
           115                 120                 125
   gct tcc ggt tta ctt tat aaa att gaa aaa gcc ggc acg ggc gaa tcg      432
   Ala Ser Gly Leu Leu Tyr Lys Ile Glu Lys Ala Gly Thr Gly Glu Ser
       130                 135                 140
   cct aaa gcg gaa gat acc gtt aaa gtt cac tat aaa ggg aca tta acc      480
   Pro Lys Ala Glu Asp Thr Val Lys Val His Tyr Lys Gly Thr Leu Thr
   145                 150                 155                 160
   gat ggt acg gta ttc gat agc tca tac gat cgc ggt gag ccg att gaa      528
   Asp Gly Thr Val Phe Asp Ser Ser Tyr Asp Arg Gly Glu Pro Ile Glu
                   165                 170                 175
   ttc caa tta aac caa tta att ccg ggt tgg att gaa gcg att cca atg      576
   Phe Gln Leu Asn Gln Leu Ile Pro Gly Trp Ile Glu Ala Ile Pro Met
               180                 185                 190
   ttg aaa aaa ggc gga aaa atg gaa atc gtc gtt ccg cct gaa ctt ggt      624
   Leu Lys Lys Gly Gly Lys Met Glu Ile Val Val Pro Pro Glu Leu Gly
           195                 200                 205
   tac ggc gaa cgc caa gca ggt aag att ccg gca agt tca acc tta aaa      672
   Tyr Gly Glu Arg Gln Ala Gly Lys Ile Pro Ala Ser Ser Thr Leu Lys
       210                 215                 220
   ttc gag att gaa ttg tta gat ttc aaa gcg gcc gaa gcg aaa aaa taa      720
   Phe Glu Ile Glu Leu Leu Asp Phe Lys Ala Ala Glu Ala Lys Lys
   225                 230                 235
<210> 143
   <211> 239
   <212> PRT
   <213> Actinobacillus pleuropneumoniae
<400> 143
   Met Leu Lys Asn Lys Leu Ser Val Leu Ala Ile Val Ala Gly Thr Phe
   1               5                   10                  15
   Val Ser Ala Gln Thr Ala Phe Ala Ala Asp Gln Lys Phe Ile Asp Asp
               20                  25                  30
   Ser Ser Tyr Ala Val Gly Val Leu Met Gly Lys Asn Ile Glu Gly Val
           35                  40                  45
   Val Glu Ser Gln Lys Glu Ile Phe Ser Tyr Asn Gln Asp Lys Ile Leu
       50                  55                  60
   Ala Gly Val Gln Asp Thr Ile Lys Lys Thr Gly Lys Leu Thr Asp Glu
   65                  70                  75                  80
   Asp Leu Gln Lys Gln Leu Lys Ser Leu Asp Thr Tyr Leu Ala Ser Gln
                   85                  90                  95
   Glu Ser Lys Ile Ala Ala Glu Lys Ser Lys Ala Thr Val Glu Ala Gly
               100                 105                 110
   Asn Lys Phe Arg Thr Asp Tyr Glu Lys Gln Ser Gly Val Lys Lys Thr
           115                 120                 125
   Ala Ser Gly Leu Leu Tyr Lys Ile Glu Lys Ala Gly Thr Gly Glu Ser
       130                 135                 140
   Pro Lys Ala Glu Asp Thr Val Lys Val His Tyr Lys Gly Thr Leu Thr
   145                 150                 155                 160
   Asp Gly Thr Val Phe Asp Ser Ser Tyr Asp Arg Gly Glu Pro Ile Glu
                   165                 170                 175
   Phe Gln Leu Asn Gln Leu Ile Pro Gly Trp Ile Glu Ala Ile Pro Met
               180                 185                 190
   Leu Lys Lys Gly Gly Lys Met Glu Ile Val Val Pro Pro Glu Leu Gly
           195                 200                 205
   Tyr Gly Glu Arg Gln Ala Gly Lys Ile Pro Ala Ser Ser Thr Leu Lys
       210                 215                 220
   Phe Glu Ile Glu Leu Leu Asp Phe Lys Ala Ala Glu Ala Lys Lys
   225                 230                 235
<210> 144
   <211> 290
   <212> DNA
   <213> Actinobacillus pleuropneumoniae
<220>
   <221> misc_feature
   <223> HI0379
<220>
   <221> CDS
   <222> (3) .. (287)
<400> 144
   tg cat agc gtg aga ggt ccg ggc ggc ggt tat caa ctc ggt aag caa        47
      His Ser Val Arg Gly Pro Gly Gly Gly Tyr Gln Leu Gly Lys Gln
      1               5                   10                  15
   cct gaa gag att agt gtg ggg atg att att gcg gcg gtg aat gaa aat        95
   Pro Glu Glu Ile Ser Val Gly Met Ile Ile Ala Ala Val Asn Glu Asn
                   20                  25                  30
   ctc gac gta acc aaa tgt aaa ggt agc ggc aac tgt agc aaa aac tct       143
   Leu Asp Val Thr Lys Cys Lys Gly Ser Gly Asn Cys Ser Lys Asn Ser
               35                  40                  45
   cag tgc tta acc cat cat tta tgg gaa cgt tta gaa gaa caa atc ggt       191
   Gln Cys Leu Thr His His Leu Trp Glu Arg Leu Glu Glu Gln Ile Gly
           50                  55                  60
   gtg ttt tta aat acg att act tta gcg gaa ctt gtt gaa gaa cat tcg       239
   Val Phe Leu Asn Thr Ile Thr Leu Ala Glu Leu Val Glu Glu His Ser
       65                  70                  75
   gat cac gat tgt gaa aaa gaa cat tgc cac gat cat tca cac aaa cat       287
   Asp His Asp Cys Glu Lys Glu His Cys His Asp His Ser His Lys His
   80                  85                  90                  95
   taa                                                                   290
<210> 145
   <211> 95
   <212> PRT
   <213> Actinobacillus pleuropneumoniae
<400> 145
   His Ser Val Arg Gly Pro Gly Gly Gly Tyr Gln Leu Gly Lys Gln Pro
   1               5                   10                  15
   Glu Glu Ile Ser Val Gly Met Ile Ile Ala Ala Val Asn Glu Asn Leu
                   20                  25                  30
   Asp Val Thr Lys Cys Lys Gly Ser Gly Asn Cys Ser Lys Asn Ser Gln
           35                  40                  45
   Cys Leu Thr His His Leu Trp Glu Arg Leu Glu Glu Gln Ile Gly Val
       50                  55                  60
   Phe Leu Asn Thr Ile Thr Leu Ala Glu Leu Val Glu Glu His Ser Asp
   65                  70                  75                  80
   His Asp Cys Glu Lys Glu His Cys His Asp His Ser His Lys His
                   85                  90                  95
<210> 146
   <211> 273
   <212> DNA
   <213> Actinobacillus pleuropneumoniae
<220>
   <221> misc_feature
   <223> hupA
<220>
   <221> CDS
   <222> (1) .. (270)
<400> 146
   atg aac aaa act gag tta atc gat gca atc gca gct ggt gca gag tta        48
   Met Asn Lys Thr Glu Leu Ile Asp Ala Ile Ala Ala Gly Ala Glu Leu
   1               5                   10                  15
   agc aag aaa gac gcg aaa gcg gca tta gaa gcg act tta aat gcg atc        96
   Ser Lys Lys Asp Ala Lys Ala Ala Leu Glu Ala Thr Leu Asn Ala Ile
               20                  25                  30
   tct gaa agc cta aaa aat ggc gac acc gtt cag tta atc ggc ttc ggt       144
   Ser Glu Ser Leu Lys Asn Gly Asp Thr Val Gln Leu Ile Gly Phe Gly
           35                  40                  45
   act ttt aaa gta aac gag cgt aat gca cgt acg ggt cgt aac ccg cgt       192
   Thr Phe Lys Val Asn Glu Arg Asn Ala Arg Thr Gly Arg Asn Pro Arg
       50                  55                  60
   acc ggc gaa gaa atc aaa atc gca gca tct aaa gtg ccg gcg ttt gtt       240
   Thr Gly Glu Glu Ile Lys Ile Ala Ala Ser Lys Val Pro Ala Phe Val
   65                  70                  75                  80
   gca ggt aaa gca tta aaa gat tta gta aaa taa                           273
   Ala Gly Lys Ala Leu Lys Asp Leu Val Lys
                   85                  90
<210> 147
   <211> 90
   <212> PRT
   <213> Actinobacillus pleuropneumoniae
<400> 147
   Met Asn Lys Thr Glu Leu Ile Asp Ala Ile Ala Ala Gly Ala Glu Leu
   1               5                   10                  15
   Ser Lys Lys Asp Ala Lys Ala Ala Leu Glu Ala Thr Leu Asn Ala Ile
               20                  25                  30
   Ser Glu Ser Leu Lys Asn Gly Asp Thr Val Gln Leu Ile Gly Phe Gly
           35                  40                  45
   Thr Phe Lys Val Asn Glu Arg Asn Ala Arg Thr Gly Arg Asn Pro Arg
       50                  55                  60
   Thr Gly Glu Glu Ile Lys Ile Ala Ala Ser Lys Val Pro Ala Phe Val
   65                  70                  75                  80
   Ala Gly Lys Ala Leu Lys Asp Leu Val Lys
                   85                  90
<210> 148
   <211> 551
   <212> DNA
   <213> Actinobacillus pleuropneumoniae
<220>
   <221> misc_feature
   <223> lpdA
<220>
   <221> CDS
   <222> (1) .. (549)
<400> 148
   atg agc aaa gaa atc aaa acg caa gtc gtg gta ctt ggt gcg ggt cct       48
   Met Ser Lys Glu Ile Lys Thr Gln Val Val Val Leu Gly Ala Gly Pro
   1               5                   10                  15
   gcc ggt tat tca gcg gca ttc cgt tgt gcc gac tta ggc tta gaa aca       96
   Ala Gly Tyr Ser Ala Ala Phe Arg Cys Ala Asp Leu Gly Leu Glu Thr
               20                  25                  30
   gta att gtc gaa cgt tat tca act ttg ggc ggt gta tgc tta aac gta      144
   Val Ile Val Glu Arg Tyr Ser Thr Leu Gly Gly Val Cys Leu Asn Val
           35                  40                  45
   ggt tgt att ccg tct aaa gca tta tta cac gtt gca aaa gtt atc gaa      192
   Gly Cys Ile Pro Ser Lys Ala Leu Leu His Val Ala Lys Val Ile Glu
       50                  55                  60
   gaa gca aaa cac gca gag aaa aac ggt att act ttc ggt gag ccc aac      240
   Glu Ala Lys His Ala Glu Lys Asn Gly Ile Thr Phe Gly Glu Pro Asn
   65                  70                  75                  80
   att gat tta gat aaa gtg cgt gcg ggt aaa gaa gcg gtt gtt tct aaa      288
   Ile Asp Leu Asp Lys Val Arg Ala Gly Lys Glu Ala Val Val Ser Lys
                   85                  90                  95
   tta acc ggc ggt tta gcg ggt atg gct aaa gca cgt aaa gta aca gta      336
   Leu Thr Gly Gly Leu Ala Gly Met Ala Lys Ala Arg Lys Val Thr Val
               100                 105                 110
   gtg gaa ggt tta gcg gcg ttt acc gat ccg aat act tta gta gct cgt      384
   Val Glu Gly Leu Ala Ala Phe Thr Asp Pro Asn Thr Leu Val Ala Arg
           115                 120                 125
   gac cgt gac ggt aat ccg aca acg att aaa ttt gat tat gca att att      432
   Asp Arg Asp Gly Asn Pro Thr Thr Ile Lys Phe Asp Tyr Ala Ile Ile
       130                 135                 140
   gca gcc ggt tct cgt ccg att cag ctt ccg ttc att cca cac gaa gat      480
   Ala Ala Gly Ser Arg Pro Ile Gln Leu Pro Phe Ile Pro His Glu Asp
   145                 150                 155                 160
   ccg cgt gtg tgg gat tct acg gat gca ctt aaa tta aaa gaa gta ccc      528
   Pro Arg Val Trp Asp Ser Thr Asp Ala Leu Lys Leu Lys Glu Val Pro
                   165                 170                 175
   gaa aaa att act cat tat ggg cc                                       551
   Glu Lys Ile Thr His Tyr Gly
               180
<210> 149
   <211> 183
   <212> PRT
   <213> Actinobacillus pleuropneumoniae
<400> 149
   Met Ser Lys Glu Ile Lys Thr Gln Val Val Val Leu Gly Ala Gly Pro
   1               5                   10                  15
   Ala Gly Tyr Ser Ala Ala Phe Arg Cys Ala Asp Leu Gly Leu Glu Thr
               20                  25                  30
   Val Ile Val Glu Arg Tyr Ser Thr Leu Gly Gly Val Cys Leu Asn Val
           35                  40                  45
   Gly Cys Ile Pro Ser Lys Ala Leu Leu His Val Ala Lys Val Ile Glu
       50                  55                  60
   Glu Ala Lys His Ala Glu Lys Asn Gly Ile Thr Phe Gly Glu Pro Asn
   65                  70                  75                  80
   Ile Asp Leu Asp Lys Val Arg Ala Gly Lys Glu Ala Val Val Ser Lys
                   85                  90                  95
   Leu Thr Gly Gly Leu Ala Gly Met Ala Lys Ala Arg Lys Val Thr Val
               100                 105                 110
   Val Glu Gly Leu Ala Ala Phe Thr Asp Pro Asn Thr Leu Val Ala Arg
           115                 120                 125
   Asp Arg Asp Gly Asn Pro Thr Thr Ile Lys Phe Asp Tyr Ala Ile Ile
       130                 135                 140
   Ala Ala Gly Ser Arg Pro Ile Gln Leu Pro Phe Ile Pro His Glu Asp
   145                 150                 155                 160
   Pro Arg Val Trp Asp Ser Thr Asp Ala Leu Lys Leu Lys Glu Val Pro
                   165                 170                 175
   Glu Lys Ile Thr His Tyr Gly
               180
<210> 150
   <211> 1095
   <212> DNA
   <213> Actinobacillus pleuropneumoniae
<220>
   <221> misc_feature
   <223> Omp5-2
<220>
   <221> CDS
   <222> (1) .. (1092)
<400> 150
   atg aaa aaa tca tta gtt gct tta aca gta tta tcg gct gca gcg gta      48
   Met Lys Lys Ser Leu Val Ala Leu Thr Val Leu Ser Ala Ala Ala Val
   1               5                   10                  15
   gct caa gca gcg cca caa caa aat act ttc tac gca ggt gcg aaa gca      96
   Ala Gln Ala Ala Pro Gln Gln Asn Thr Phe Tyr Ala Gly Ala Lys Ala
               20                  25                  30
   ggt tgg gcg tca ttc cat gat ggt atc gaa caa tta gat tca gct aaa     144
   Gly Trp Ala Ser Phe His Asp Gly Ile Glu Gln Leu Asp Ser Ala Lys
           35                  40                  45
   aac aca gat cgc ggt aca aaa tac ggt atc aac cgt aat tca gta act     192
   Asn Thr Asp Arg Gly Thr Lys Tyr Gly Ile Asn Arg Asn Ser Val Thr
       50                  55                  60
   tac ggc gta ttc ggc ggt tac caa att tta aac caa gac aaa tta ggt     240
   Tyr Gly Val Phe Gly Gly Tyr Gln Ile Leu Asn Gln Asp Lys Leu Gly
   65                  70                  75                  80
   tta gcg gct gaa tta ggt tat gac tat ttc ggt cgt gtg cgc ggt tct      288
   Leu Ala Ala Glu Leu Gly Tyr Asp Tyr Phe Gly Arg Val Arg Gly Ser
                   85                  90                  95
   gaa aaa cca aac ggt aaa gcg gac aag aaa act ttc cgt cac gct gca      336
   Glu Lys Pro Asn Gly Lys Ala Asp Lys Lys Thr Phe Arg His Ala Ala
               100                 105                 110
   cac ggt gcg aca atc gca tta aaa cct agc tac gaa gta tta cct gac      384
   His Gly Ala Thr Ile Ala Leu Lys Pro Ser Tyr Glu Val Leu Pro Asp
           115                 120                 125
   tta gac gtt tac ggt aaa gta ggt atc gca tta gta aac aat aca tat      432
   Leu Asp Val Tyr Gly Lys Val Gly Ile Ala Leu Val Asn Asn Thr Tyr
       130                 135                 140
   aaa aca ttc aat gca gca caa gag aaa gtg aaa act cgt cgt ttc caa      480
   Lys Thr Phe Asn Ala Ala Gln Glu Lys Val Lys Thr Arg Arg Phe Gln
   145                 150                 155                 160
   agt tct tta att tta ggt gcg ggt gtt gag tac gca att ctt cct gaa      528
   Ser Ser Leu Ile Leu Gly Ala Gly Val Glu Tyr Ala Ile Leu Pro Glu
                   165                 170                 175
   tta gcg gca cgt gtt gaa tac caa tgg tta aac aac gca ggt aaa gca      576
   Leu Ala Ala Arg Val Glu Tyr Gln Trp Leu Asn Asn Ala Gly Lys Ala
               180                 185                 190
   agc tac tct act tta aat cgt atg ggt gca act gac tac cgt tcg gat      624
   Ser Tyr Ser Thr Leu Asn Arg Met Gly Ala Thr Asp Tyr Arg Ser Asp
           195                 200                 205
   atc agt tcc gta tct gca ggt tta agc tac cgt ttc ggt caa ggt gcg      672
   Ile Ser Ser Val Ser Ala Gly Leu Ser Tyr Arg Phe Gly Gln Gly Ala
       210                 215                 220
   gca ccg gtt gca gct ccg gca gtt gaa act aaa aac ttc gca ttc agc      720
   Ala Pro Val Ala Ala Pro Ala Val Glu Thr Lys Asn Phe Ala Phe Ser
   225                 230                 235                 240
   tct gac gta tta ttc gca ttc ggt aaa tca aac tta aaa ccg gct gcg      768
   Ser Asp Val Leu Phe Ala Phe Gly Lys Ser Asn Leu Lys Pro Ala Ala
                   245                 250                 255
   gca aca gca tta gat gca atg caa acc gaa atc aat aac gca ggt tta      816
   Ala Thr Ala Leu Asp Ala Met Gln Thr Glu Ile Asn Asn Ala Gly Leu
               260                 265                 270
   tca aat gct gcg atc caa gta aac ggt tac acg gac cgt atc ggt aaa      864
   Ser Asn Ala Ala Ile Gln Val Asn Gly Tyr Thr Asp Arg Ile Gly Lys
           275                 280                 285
   gaa gct tca aac tta aaa ctt tca caa cgt cgt gcg gaa aca gta gct      912
   Glu Ala Ser Asn Leu Lys Leu Ser Gln Arg Arg Ala Glu Thr Val Ala
       290                 295                 300
   aac tac atc gtt tct aaa ggt gct ccg gca gct aac gta act gca gta      960
   Asn Tyr Ile Val Ser Lys Gly Ala Pro Ala Ala Asn Val Thr Ala Val
   305                 310                 315                 320
   ggt tac ggt gaa gca aac cct gta acc ggc gca aca tgt gac aaa gtt     1008
   Gly Tyr Gly Glu Ala Asn Pro Val Thr Gly Ala Thr Cys Asp Lys Val
                   325                 330                 335
   aaa ggt cgt aaa gca tta atc gct tgc tta gca ccg gat cgt cgt gtt      1056
   Lys Gly Arg Lys Ala Leu Ile Ala Cys Leu Ala Pro Asp Arg Arg Val
               340                 345                 350
   gaa gtt caa gtt caa ggt act aaa gaa gta act atg taa                  1095
   Glu Val Gln Val Gln Gly Thr Lys Glu Val Thr Met
           355                 360
<210> 151
   <211> 364
   <212> PRT
   <213> Actinobacillus pleuropneumoniae
<400> 151
   Met Lys Lys Ser Leu Val Ala Leu Thr Val Leu Ser Ala Ala Ala Val
   1               5                   10                  15
   Ala Gln Ala Ala Pro Gln Gln Asn Thr Phe Tyr Ala Gly Ala Lys Ala
               20                  25                  30
   Gly Trp Ala Ser Phe His Asp Gly Ile Glu Gln Leu Asp Ser Ala Lys
           35                  40                  45
   Asn Thr Asp Arg Gly Thr Lys Tyr Gly Ile Asn Arg Asn Ser Val Thr
       50                  55                  60
   Tyr Gly Val Phe Gly Gly Tyr Gln Ile Leu Asn Gln Asp Lys Leu Gly
   65                  70                  75                  80
   Leu Ala Ala Glu Leu Gly Tyr Asp Tyr Phe Gly Arg Val Arg Gly Ser
                   85                  90                  95
   Glu Lys Pro Asn Gly Lys Ala Asp Lys Lys Thr Phe Arg His Ala Ala
               100                 105                 110
   His Gly Ala Thr Ile Ala Leu Lys Pro Ser Tyr Glu Val Leu Pro Asp
           115                 120                 125
   Leu Asp Val Tyr Gly Lys Val Gly Ile Ala Leu Val Asn Asn Thr Tyr
       130                 135                 140
   Lys Thr Phe Asn Ala Ala Gln Glu Lys Val Lys Thr Arg Arg Phe Gln
   145                 150                 155                 160
   Ser Ser Leu Ile Leu Gly Ala Gly Val Glu Tyr Ala Ile Leu Pro Glu
                   165                 170                 175
   Leu Ala Ala Arg Val Glu Tyr Gln Trp Leu Asn Asn Ala Gly Lys Ala
               180                 185                 190
   Ser Tyr Ser Thr Leu Asn Arg Met Gly Ala Thr Asp Tyr Arg Ser Asp
           195                 200                 205
   Ile Ser Ser Val Ser Ala Gly Leu Ser Tyr Arg Phe Gly Gln Gly Ala
       210                 215                 220
   Ala Pro Val Ala Ala Pro Ala Val Glu Thr Lys Asn Phe Ala Phe Ser
   225                 230                 235                 240
   Ser Asp Val Leu Phe Ala Phe Gly Lys Ser Asn Leu Lys Pro Ala Ala
                   245                 250                 255
   Ala Thr Ala Leu Asp Ala Met Gln Thr Glu Ile Asn Asn Ala Gly Leu
               260                 265                 270
   Ser Asn Ala Ala Ile Gln Val Asn Gly Tyr Thr Asp Arg Ile Gly Lys
           275                 280                 285
   Glu Ala Ser Asn Leu Lys Leu Ser Gln Arg Arg Ala Glu Thr Val Ala
       290                 295                 300
   Asn Tyr Ile Val Ser Lys Gly Ala Pro Ala Ala Asn Val Thr Ala Val
   305                 310                 315                 320
   Gly Tyr Gly Glu Ala Asn Pro Val Thr Gly Ala Thr Cys Asp Lys Val
                   325                 330                 335
   Lys Gly Arg Lys Ala Leu Ile Ala Cys Leu Ala Pro Asp Arg Arg Val
               340                 345                 350
   Glu Val Gln Val Gln Gly Thr Lys Glu Val Thr Met
           355                 360
<210> 152
   <211> 1110
   <212> DNA
   <213> Actinobacillus pleuropneumoniae
<220>
   <221> misc_feature
   <223> Omp5
<220>
   <221> CDS
   <222> (1)..(1107)
<400> 152
   atg aaa aaa tca tta gtt gct tta gca gta tta tcg gct gca gca gta      48
   Met Lys Lys Ser Leu Val Ala Leu Ala Val Leu Ser Ala Ala Ala Val
   1               5                   10                  15
   gct caa gca gct cca caa caa aat act ttc tac gca ggt gcg aaa gtt      96
   Ala Gln Ala Ala Pro Gln Gln Asn Thr Phe Tyr Ala Gly Ala Lys Val
               20                  25                  30
   ggt caa tca tca ttt cac cac ggt gtt aac caa tta aaa tct ggt cac      144
   Gly Gln Ser Ser Phe His His Gly Val Asn Gln Leu Lys Ser Gly His
           35                  40                  45
   gat gat cgt tat aat gat aaa aca cgt aag tat ggt atc aac cgt aac      192
   Asp Asp Arg Tyr Asn Asp Lys Thr Arg Lys Tyr Gly Ile Asn Arg Asn
       50                  55                  60
   tct gta act tac ggt gta ttc ggc ggt tac caa atc tta aac caa aat      240
   Ser Val Thr Tyr Gly Val Phe Gly Gly Tyr Gln Ile Leu Asn Gln Asn
   65                  70                  75                  80
   aac ttc ggt tta gca gct gaa tta ggc tat gac tac tac ggt cgc gta      288
   Asn Phe Gly Leu Ala Ala Glu Leu Gly Tyr Asp Tyr Tyr Gly Arg Val
                   85                  90                  95
   cgt ggt aac gta gat gaa ttc cgt aca gtt aaa cac tct gct cac ggt      336
   Arg Gly Asn Val Asp Glu Phe Arg Thr Val Lys His Ser Ala His Gly
               100                 105                 110
   tta aac tta gcg tta aaa cca agc tac gaa gta tta cct gac tta gac      384
   Leu Asn Leu Ala Leu Lys Pro Ser Tyr Glu Val Leu Pro Asp Leu Asp
           115                 120                 125
   gtt tac ggt aaa gta ggt att gcg gtt gtt cgt aat gac tat aaa aaa      432
   Val Tyr Gly Lys Val Gly Ile Ala Val Val Arg Asn Asp Tyr Lys Lys
       130                 135                 140
   tat ggt gcg gaa aac act aac gaa tca aca aca aaa ttc cac aaa tta      480
   Tyr Gly Ala Glu Asn Thr Asn Glu Ser Thr Thr Lys Phe His Lys Leu
   145                 150                 155                 160
   aaa gca tca act att tta ggt gca ggt gtt gag tac gca att ctt cct      528
   Lys Ala Ser Thr Ile Leu Gly Ala Gly Val Glu Tyr Ala Ile Leu Pro
                   165                 170                 175
   gaa tta gcg gca cgt gtt gaa tac caa tac tta aac aaa gcg ggt aac      576
   Glu Leu Ala Ala Arg Val Glu Tyr Gln Tyr Leu Asn Lys Ala Gly Asn
               180                 185                 190
   tta aat aaa gca tta gtt cgt tca ggc aca caa gat gtg gac ttc caa      624
   Leu Asn Lys Ala Leu Val Arg Ser Gly Thr Gln Asp Val Asp Phe Gln
           195                 200                 205
   tat gct cct gat atc cac tct gta aca gca ggt tta tca tac cgt ttc      672
   Tyr Ala Pro Asp Ile His Ser Val Thr Ala Gly Leu Ser Tyr Arg Phe
       210                 215                 220
   ggt caa ggc gct gta gca cca gtt gtt gag cca gaa gtt gta act aaa      720
   Gly Gln Gly Ala Val Ala Pro Val Val Glu Pro Glu Val Val Thr Lys
   225                 230                 235                 240
   aac ttc gca ttc agc tca gac gtt tta ttt gat ttc ggt aaa tca agc      768
   Asn Phe Ala Phe Ser Ser Asp Val Leu Phe Asp Phe Gly Lys Ser Ser
                   245                 250                 255
   tta aaa cca gca gca gca aca gct tta gac gca gct aac act gaa atc      816
   Leu Lys Pro Ala Ala Ala Thr Ala Leu Asp Ala Ala Asn Thr Glu Ile
               260                 265                 270
   gct aac tta ggt tta gca act cca gct atc caa gtt aac ggt tat aca      864
   Ala Asn Leu Gly Leu Ala Thr Pro Ala Ile Gln Val Asn Gly Tyr Thr
           275                 280                 285
   gac cgt atc ggt aaa gaa gct tca aac tta aaa ctt tca caa cgc cgt      912
   Asp Arg Ile Gly Lys Glu Ala Ser Asn Leu Lys Leu Ser Gln Arg Arg
       290                 295                 300
   gca gaa act gta gct aac tac tta gtt tct aaa ggt caa aac cct gca      960
   Ala Glu Thr Val Ala Asn Tyr Leu Val Ser Lys Gly Gln Asn Pro Ala
   305                 310                 315                 320
   aac gta act gca gta ggt tac ggt gaa gca aac cca gta acc ggc gca     1008
   Asn Val Thr Ala Val Gly Tyr Gly Glu Ala Asn Pro Val Thr Gly Ala
                   325                 330                 335
   aca tgt gat gca gtt aaa ggt cgt aaa gca tta atc gct tgc tta gca     1056
   Thr Cys Asp Ala Val Lys Gly Arg Lys Ala Leu Ile Ala Cys Leu Ala
               340                 345                 350
   ccg gat cgt cgt gtt gaa gtt caa gta caa ggt gct aaa aac gta gct     1104
   Pro Asp Arg Arg Val Glu Val Gln Val Gln Gly Ala Lys Asn Val Ala
           355                 360                 365
   atg taa                                                             1110
   Met
<210> 153
   <211> 369
   <212> PRT
   <213> Actinobacillus pleuropneumoniae
<400> 153
   Met Lys Lys Ser Leu Val Ala Leu Ala Val Leu Ser Ala Ala Ala Val
   1               5                   10                  15
   Ala Gln Ala Ala Pro Gln Gln Asn Thr Phe Tyr Ala Gly Ala Lys Val
               20                  25                  30
   Gly Gln Ser Ser Phe His His Gly Val Asn Gln Leu Lys Ser Gly His
           35                  40                  45
   Asp Asp Arg Tyr Asn Asp Lys Thr Arg Lys Tyr Gly Ile Asn Arg Asn
       50                  55                  60
   Ser Val Thr Tyr Gly Val Phe Gly Gly Tyr Gln Ile Leu Asn Gln Asn
   65                  70                  75                  80
   Asn Phe Gly Leu Ala Ala Glu Leu Gly Tyr Asp Tyr Tyr Gly Arg Val
                   85                  90                  95
   Arg Gly Asn Val Asp Glu Phe Arg Thr Val Lys His Ser Ala His Gly
               100                 105                 110
   Leu Asn Leu Ala Leu Lys Pro Ser Tyr Glu Val Leu Pro Asp Leu Asp
           115                 120                 125
   Val Tyr Gly Lys Val Gly Ile Ala Val Val Arg Asn Asp Tyr Lys Lys
       130                 135                 140
   Tyr Gly Ala Glu Asn Thr Asn Glu Ser Thr Thr Lys Phe His Lys Leu
   145                 150                 155                 160
   Lys Ala Ser Thr Ile Leu Gly Ala Gly Val Glu Tyr Ala Ile Leu Pro
                   165                 170                 175
   Glu Leu Ala Ala Arg Val Glu Tyr Gln Tyr Leu Asn Lys Ala Gly Asn
               180                 185                 190
   Leu Asn Lys Ala Leu Val Arg Ser Gly Thr Gln Asp Val Asp Phe Gln
           195                 200                 205
   Tyr Ala Pro Asp Ile His Ser Val Thr Ala Gly Leu Ser Tyr Arg Phe
       210                 215                 220
   Gly Gln Gly Ala Val Ala Pro Val Val Glu Pro Glu Val Val Thr Lys
   225                 230                 235                 240
   Asn Phe Ala Phe Ser Ser Asp Val Leu Phe Asp Phe Gly Lys Ser Ser
                   245                 250                 255
   Leu Lys Pro Ala Ala Ala Thr Ala Leu Asp Ala Ala Asn Thr Glu Ile
               260                 265                 270
   Ala Asn Leu Gly Leu Ala Thr Pro Ala Ile Gln Val Asn Gly Tyr Thr
           275                 280                 285
   Asp Arg Ile Gly Lys Glu Ala Ser Asn Leu Lys Leu Ser Gln Arg Arg
       290                 295                 300
   Ala Glu Thr Val Ala Asn Tyr Leu Val Ser Lys Gly Gln Asn Pro Ala
   305                 310                 315                 320
   Asn Val Thr Ala Val Gly Tyr Gly Glu Ala Asn Pro Val Thr Gly Ala
                   325                 330                 335
   Thr Cys Asp Ala Val Lys Gly Arg Lys Ala Leu Ile Ala Cys Leu Ala
               340                 345                 350
   Pro Asp Arg Arg Val Glu Val Gln Val Gln Gly Ala Lys Asn Val Ala
           355                 360                 365
   Met
<210> 154
   <211> 1076
   <212> DNA
   <213> Actinobacillus pleuropneumoniae
<220>
   <221> misc_feature
   <223> pnp new
<220>
   <221> CDS
   <222> (1) .. (1074)
<400> 154
   aat att aaa gaa ttc gta aaa gaa gcg ggt aaa ccg cgt tgg gat tgg       48
   Asn Ile Lys Glu Phe Val Lys Glu Ala Gly Lys Pro Arg Trp Asp Trp
   1               5                   10                  15
   gtt gcg ccg gaa ccg aat acc gca tta atc aac caa gtt aaa gcg tta       96
   Val Ala Pro Glu Pro Asn Thr Ala Leu Ile Asn Gln Val Lys Ala Leu
               20                  25                  30
   gcg gaa gcg cgt atc ggc gat gcg tat cgt att aca gaa aaa caa gcg      144
   Ala Glu Ala Arg Ile Gly Asp Ala Tyr Arg Ile Thr Glu Lys Gln Ala
           35                  40                  45
   cgt tac gaa caa atc gat gca att aaa gcg gat gtt atc gca caa tta      192
   Arg Tyr Glu Gln Ile Asp Ala Ile Lys Ala Asp Val Ile Ala Gln Leu
       50                  55                  60
   acc gca caa gac gaa acc gtt tct gaa ggt gcg att att gat att att      240
   Thr Ala Gln Asp Glu Thr Val Ser Glu Gly Ala Ile Ile Asp Ile Ile
   65                  70                  75                  80
   acc gca tta gaa agt tct att gtt cgc ggt cgt att att gcc ggc gaa      288
   Thr Ala Leu Glu Ser Ser Ile Val Arg Gly Arg Ile Ile Ala Gly Glu
                   85                  90                  95
   ccg cgt att gac ggt cgt acg gta gat acg gtt cgt gca tta gac att      336
   Pro Arg Ile Asp Gly Arg Thr Val Asp Thr Val Arg Ala Leu Asp Ile
               100                 105                 110
   tgc acc ggc gta tta cct cgt acg cac ggt tct gca atc ttt act cgc      384
   Cys Thr Gly Val Leu Pro Arg Thr His Gly Ser Ala Ile Phe Thr Arg
           115                 120                 125
   ggt gaa aca caa gca tta gcg gtt gca acc tta ggt act gag cgc gat      432
   Gly Glu Thr Gln Ala Leu Ala Val Ala Thr Leu Gly Thr Glu Arg Asp
       130                 135                 140
   gca caa att gtt gac gaa tta acc ggc gag aaa tca gac cgt ttc tta      480
   Ala Gln Ile Val Asp Glu Leu Thr Gly Glu Lys Ser Asp Arg Phe Leu
   145                 150                 155                 160
   ttc cac tat aac ttc cct ccg tac tct gtc ggt gaa acc ggt cgt atc      528
   Phe His Tyr Asn Phe Pro Pro Tyr Ser Val Gly Glu Thr Gly Arg Ile
                   165                 170                 175
   ggt tcg ccg aaa cgt cgt gaa atc ggc cac ggt cgt tta gcg aaa cgc      576
   Gly Ser Pro Lys Arg Arg Glu Ile Gly His Gly Arg Leu Ala Lys Arg
               180                 185                 190
   ggt gta tta gcg gta atg ccg act gct gaa gaa ttc ccg tat gta gtg      624
   Gly Val Leu Ala Val Met Pro Thr Ala Glu Glu Phe Pro Tyr Val Val
           195                 200                 205
   cgc gta gta tct gaa att acc gaa tca aac ggt tct tct tca atg gct      672
   Arg Val Val Ser Glu Ile Thr Glu Ser Asn Gly Ser Ser Ser Met Ala
       210                 215                 220
   tcc gta tgc ggc gca tct tta gcg tta atg gac gca ggc gta ccg att      720
   Ser Val Cys Gly Ala Ser Leu Ala Leu Met Asp Ala Gly Val Pro Ile
   225                 230                 235                 240
   aaa gcg gcg gtt gcg ggt atc gca atg ggc tta gtg aaa gaa gaa gaa      768
   Lys Ala Ala Val Ala Gly Ile Ala Met Gly Leu Val Lys Glu Glu Glu
                   245                 250                 255
   aaa ttt gtg gtg ctt tca gac atc tta ggt gac gaa gac cat tta ggc      816
   Lys Phe Val Val Leu Ser Asp Ile Leu Gly Asp Glu Asp His Leu Gly
               260                 265                 270
   gat atg gac ttc aaa gta gcc ggt acg cgt gaa ggt gta acc gca ctt      864
   Asp Met Asp Phe Lys Val Ala Gly Thr Arg Glu Gly Val Thr Ala Leu
           275                 280                 285
   caa atg gat att aaa atc gaa ggt atc acg cct gaa att atg caa atc      912
   Gln Met Asp Ile Lys Ile Glu Gly Ile Thr Pro Glu Ile Met Gln Ile
       290                 295                 300
   gca tta aat caa gcg aaa ggt gcg cgt atg cac atc tta agc gtg atg      960
   Ala Leu Asn Gln Ala Lys Gly Ala Arg Met His Ile Leu Ser Val Met
   305                 310                 315                 320
   gaa caa gcg att cct gca cct cgt gcc gat att tcc gat ttt gcg cct     1008
   Glu Gln Ala Ile Pro Ala Pro Arg Ala Asp Ile Ser Asp Phe Ala Pro
                   325                 330                 335
   cgt att cat acg atg aag atc gat ccg aag aaa atc aaa gac gtg atc     1056
   Arg Ile His Thr Met Lys Ile Asp Pro Lys Lys Ile Lys Asp Val Ile
   340 345 350
   ggt aaa ggc ggt gcg gtt at 1076
   Gly Lys Gly Gly Ala Val
   355
<210> 155
   <211> 358
   <212> PRT
   <213> Actinobacillus pleuropneumoniae
<400> 155
   Asn Ile Lys Glu Phe Val Lys Glu Ala Gly Lys Pro Arg Trp Asp Trp
   1 5 10 15
   Val Ala Pro Glu Pro Asn Thr Ala Leu Ile Asn Gln Val Lys Ala Leu
   20 25 30
   Ala Glu Ala Arg Ile Gly Asp Ala Tyr Arg Ile Thr Glu Lys Gln Ala
   35 40 45
   Arg Tyr Glu Gln Ile Asp Ala Ile Lys Ala Asp Val Ile Ala Gln Leu
   50 55 60
   Thr Ala Gln Asp Glu Thr Val Ser Glu Gly Ala Ile Ile Asp Ile Ile
   65 70 75 80
   Thr Ala Leu Glu Ser Ser Ile Val Arg Gly Arg Ile Ile Ala Gly Glu
   85 90 95
   Pro Arg Ile Asp Gly Arg Thr Val Asp Thr Val Arg Ala Leu Asp Ile
   100 105 110
   Cys Thr Gly Val Leu Pro Arg Thr His Gly Ser Ala Ile Phe Thr Arg
   115 120 125
   Gly Glu Thr Gln Ala Leu Ala Val Ala Thr Leu Gly Thr Glu Arg Asp
   130 135 140
   Ala Gln Ile Val Asp Glu Leu Thr Gly Glu Lys Ser Asp Arg Phe Leu
   145 150 155 160
   Phe His Tyr Asn Phe Pro Pro Tyr Ser Val Gly Glu Thr Gly Arg Ile
   165 170 175
   Gly Ser Pro Lys Arg Arg Glu Ile Gly His Gly Arg Leu Ala Lys Arg
   180 185 190
   Gly Val Leu Ala Val Met Pro Thr Ala Glu Glu Phe Pro Tyr Val Val
           195                 200                 205
   Arg Val Val Ser Glu Ile Thr Glu Ser Asn Gly Ser Ser Ser Met Ala
       210                 215                 220
   Ser Val Cys Gly Ala Ser Leu Ala Leu Met Asp Ala Gly Val Pro Ile
   225                 230                 235                 240
   Lys Ala Ala Val Ala Gly Ile Ala Met Gly Leu Val Lys Glu Glu Glu
                   245                 250                 255
   Lys Phe Val Val Leu Ser Asp Ile Leu Gly Asp Glu Asp His Leu Gly
               260                 265                 270
   Asp Met Asp Phe Lys Val Ala Gly Thr Arg Glu Gly Val Thr Ala Leu
           275                 280                 285
   Gln Met Asp Ile Lys Ile Glu Gly Ile Thr Pro Glu Ile Met Gln Ile
       290                 295                 300
   Ala Leu Asn Gln Ala Lys Gly Ala Arg Met His Ile Leu Ser Val Met
   305                 310                 315                 320
   Glu Gln Ala Ile Pro Ala Pro Arg Ala Asp Ile Ser Asp Phe Ala Pro
                   325                 330                 335
   Arg Ile His Thr Met Lys Ile Asp Pro Lys Lys Ile Lys Asp Val Ile
               340                 345                 350
   Gly Lys Gly Gly Ala Val
           355
<210> 156
   <211> 1055
   <212> DNA
   <213> Actinobacillus pleuropneumoniae
<220>
   <221> misc_feature
   <223> potD
<220>
   <221> CDS
   <222> (1)..(1053)
<400> 156
   atg aaa aaa tta gcg ggt tta ttt gca gca ggt tta gcg aca gtt gca      48
   Met Lys Lys Leu Ala Gly Leu Phe Ala Ala Gly Leu Ala Thr Val Ala
   1               5                   10                  15
   tta aca gcg tgt aat gaa gaa aag cca aaa gcg gct gaa gca gcg gct      96
   Leu Thr Ala Cys Asn Glu Glu Lys Pro Lys Ala Ala Glu Ala Ala Ala
               20                  25                  30
   caa ccg gca gca gcg gga aca gtt cac ctt tat act tgg act gaa tat     144
   Gln Pro Ala Ala Ala Gly Thr Val His Leu Tyr Thr Trp Thr Glu Tyr
           35                  40                  45
   gtg cct gaa ggc ttg tta gat gaa ttt aca aag caa acc ggt atc aaa     192
   Val Pro Glu Gly Leu Leu Asp Glu Phe Thr Lys Gln Thr Gly Ile Lys
       50                  55                  60
   gta gag gtt tca agc ctt gaa tct aac gaa acc atg tat gcg aaa tta     240
   Val Glu Val Ser Ser Leu Glu Ser Asn Glu Thr Met Tyr Ala Lys Leu
   65                  70                  75                  80
   aaa tta caa ggt aaa gac ggc ggt tac gat gtt atc gca cct tct aac     288
   Lys Leu Gln Gly Lys Asp Gly Gly Tyr Asp Val Ile Ala Pro Ser Asn
                   85                  90                  95
   tac ttc gtt tca aaa atg gcg aaa gaa ggt atg tta gcg gaa tta gat     336
   Tyr Phe Val Ser Lys Met Ala Lys Glu Gly Met Leu Ala Glu Leu Asp
               100                 105                 110
   cac gca aaa ctt cct gta atc aaa gag tta aac caa gat tgg tta aac     384
   His Ala Lys Leu Pro Val Ile Lys Glu Leu Asn Gln Asp Trp Leu Asn
           115                 120                 125
   aaa cct tat gac caa ggt aac aaa tac tct tta ccg caa tta tta ggt     432
   Lys Pro Tyr Asp Gln Gly Asn Lys Tyr Ser Leu Pro Gln Leu Leu Gly
       130                 135                 140
   gca ccg ggt atc gca ttt aac tca aat gac tat aag ggc gat gcg ttc     480
   Ala Pro Gly Ile Ala Phe Asn Ser Asn Asp Tyr Lys Gly Asp Ala Phe
   145                 150                 155                 160
   act tct tgg ggt gat tta tgg aaa cct gag ttt gcg aat aaa gta caa     528
   Thr Ser Trp Gly Asp Leu Trp Lys Pro Glu Phe Ala Asn Lys Val Gln
                   165                 170                 175
   tta tta gat gac gca cgt gaa gta ttt aac att gcg tta tta aaa tta     576
   Leu Leu Asp Asp Ala Arg Glu Val Phe Asn Ile Ala Leu Leu Lys Leu
               180                 185                 190
   ggt aaa aac cct aat aca acc aat ccg gaa gag att aaa gcg gct tac     624
   Gly Lys Asn Pro Asn Thr Thr Asn Pro Glu Glu Ile Lys Ala Ala Tyr
           195                 200                 205
   gaa gag tta aga aaa tta cgt cca aac gta ctt tct ttc act tca gac     672
   Glu Glu Leu Arg Lys Leu Arg Pro Asn Val Leu Ser Phe Thr Ser Asp
       210                 215                 220
   aac cca gcg aac tca ttt atc gca ggt gaa gta tct gta ggt caa tta     720
   Asn Pro Ala Asn Ser Phe Ile Ala Gly Glu Val Ser Val Gly Gln Leu
   225                 230                 235                 240
   tgg aac ggt tct gta cgt att gcg aaa aaa gaa caa gcg ccg gta aac     768
   Trp Asn Gly Ser Val Arg Ile Ala Lys Lys Glu Gln Ala Pro Val Asn
                   245                 250                 255
   atg gtg ttc cca aaa gaa ggt cct gta ctt tgg gtt gat acg tta gcc      816
   Met Val Phe Pro Lys Glu Gly Pro Val Leu Trp Val Asp Thr Leu Ala
               260                 265                 270
   att ccg gcg aat gcg aaa aac aaa gaa aat gcg cat aag tta atc aac      864
   Ile Pro Ala Asn Ala Lys Asn Lys Glu Asn Ala His Lys Leu Ile Asn
           275                 280                 285
   tac tta tta agc gca ccg gtt gcg gaa aaa tta acg tta gaa atc ggt      912
   Tyr Leu Leu Ser Ala Pro Val Ala Glu Lys Leu Thr Leu Glu Ile Gly
       290                 295                 300
   tat ccg act tca aac gta gaa gcg tta aaa aca tta cca aaa gag att      960
   Tyr Pro Thr Ser Asn Val Glu Ala Leu Lys Thr Leu Pro Lys Glu Ile
   305                 310                 315                 320
   acc gaa gat ccg gca atc tat ccg aca gct gat gtg tta aaa gcg gca     1008
   Thr Glu Asp Pro Ala Ile Tyr Pro Thr Ala Asp Val Leu Lys Ala Ala
                   325                 330                 335
   caa tgg caa gac gat gta ggt aat gca atc gaa ctt tac gaa aaa ta      1055
   Gln Trp Gln Asp Asp Val Gly Asn Ala Ile Glu Leu Tyr Glu Lys
               340                 345                 350
<210> 157
   <211> 351
   <212> PRT
   <213> Actinobacillus pleuropneumoniae
<400> 157
   Met Lys Lys Leu Ala Gly Leu Phe Ala Ala Gly Leu Ala Thr Val Ala
   1               5                   10                  15
   Leu Thr Ala Cys Asn Glu Glu Lys Pro Lys Ala Ala Glu Ala Ala Ala
               20                  25                  30
   Gln Pro Ala Ala Ala Gly Thr Val His Leu Tyr Thr Trp Thr Glu Tyr
           35                  40                  45
   Val Pro Glu Gly Leu Leu Asp Glu Phe Thr Lys Gln Thr Gly Ile Lys
       50                  55                  60
   Val Glu Val Ser Ser Leu Glu Ser Asn Glu Thr Met Tyr Ala Lys Leu
   65                  70                  75                  80
   Lys Leu Gln Gly Lys Asp Gly Gly Tyr Asp Val Ile Ala Pro Ser Asn
                   85                  90                  95
   Tyr Phe Val Ser Lys Met Ala Lys Glu Gly Met Leu Ala Glu Leu Asp
               100                 105                 110
   His Ala Lys Leu Pro Val Ile Lys Glu Leu Asn Gln Asp Trp Leu Asn
           115                 120                 125
   Lys Pro Tyr Asp Gln Gly Asn Lys Tyr Ser Leu Pro Gln Leu Leu Gly
       130                 135                 140
   Ala Pro Gly Ile Ala Phe Asn Ser Asn Asp Tyr Lys Gly Asp Ala Phe
   145                 150                 155                 160
   Thr Ser Trp Gly Asp Leu Trp Lys Pro Glu Phe Ala Asn Lys Val Gln
                   165                 170                 175
   Leu Leu Asp Asp Ala Arg Glu Val Phe Asn Ile Ala Leu Leu Lys Leu
               180                 185                 190
   Gly Lys Asn Pro Asn Thr Thr Asn Pro Glu Glu Ile Lys Ala Ala Tyr
           195                 200                 205
   Glu Glu Leu Arg Lys Leu Arg Pro Asn Val Leu Ser Phe Thr Ser Asp
       210                 215                 220
   Asn Pro Ala Asn Ser Phe Ile Ala Gly Glu Val Ser Val Gly Gln Leu
   225                 230                 235                 240
   Trp Asn Gly Ser Val Arg Ile Ala Lys Lys Glu Gln Ala Pro Val Asn
                   245                 250                 255
   Met Val Phe Pro Lys Glu Gly Pro Val Leu Trp Val Asp Thr Leu Ala
               260                 265                 270
   Ile Pro Ala Asn Ala Lys Asn Lys Glu Asn Ala His Lys Leu Ile Asn
           275                 280                 285
   Tyr Leu Leu Ser Ala Pro Val Ala Glu Lys Leu Thr Leu Glu Ile Gly
       290                 295                 300
   Tyr Pro Thr Ser Asn Val Glu Ala Leu Lys Thr Leu Pro Lys Glu Ile
   305                 310                 315                 320
   Thr Glu Asp Pro Ala Ile Tyr Pro Thr Ala Asp Val Leu Lys Ala Ala
                   325                 330                 335
   Gln Trp Gln Asp Asp Val Gly Asn Ala Ile Glu Leu Tyr Glu Lys
               340                 345                 350
<210> 158
   <211> 525
   <212> DNA
   <213> Actinobacillus pleuropneumoniae
<220>
   <221> misc_feature
   <223> rpmF
<220>
   <221> CDS
   <222> (1)..(522)
<400> 158
   atg caa aag gta aaa cta ccc ctc acc att gac cca tat aaa gac gct        48
   Met Gln Lys Val Lys Leu Pro Leu Thr Ile Asp Pro Tyr Lys Asp Ala
   1               5                   10                  15
   cag cgt cga atg gat tac gaa ggc tac atc tca cgt agt ctg ctt aat        96
   Gln Arg Arg Met Asp Tyr Glu Gly Tyr Ile Ser Arg Ser Leu Leu Asn
               20                  25                  30
   cgt ttg ggt gaa tct gtg agc aat gtg cta agc gat gca caa gtt act       144
   Arg Leu Gly Glu Ser Val Ser Asn Val Leu Ser Asp Ala Gln Val Thr
           35                  40                  45
   ctc tcg tta tat atc gat ccg caa cgc tta acc gtt att aaa ggt acg       192
   Leu Ser Leu Tyr Ile Asp Pro Gln Arg Leu Thr Val Ile Lys Gly Thr
       50                  55                  60
   gcg aca gtg gaa gtg gaa ttc gat tgc caa cga tgc ggt aac ccg ttt       240
   Ala Thr Val Glu Val Glu Phe Asp Cys Gln Arg Cys Gly Asn Pro Phe
   65                  70                  75                  80
   aca caa acg ctt gac tgt tcg ttt tgt ttc agt ccg gtg tcc aat atg       288
   Thr Gln Thr Leu Asp Cys Ser Phe Cys Phe Ser Pro Val Ser Asn Met
                   85                  90                  95
   gat cag gcg gac aat ttg ccc gaa att tat gaa cca atc gaa gta aac       336
   Asp Gln Ala Asp Asn Leu Pro Glu Ile Tyr Glu Pro Ile Glu Val Asn
               100                 105                 110
   gag ttc ggt gaa gta aat tta cta gat atg atc gaa gat gga ttt atc       384
   Glu Phe Gly Glu Val Asn Leu Leu Asp Met Ile Glu Asp Gly Phe Ile
           115                 120                 125
   atc gaa ttg cct cta gtc ccg atg cat agt gaa gaa cac tgt gaa gtg       432
   Ile Glu Leu Pro Leu Val Pro Met His Ser Glu Glu His Cys Glu Val
       130                 135                 140
   tcc gtg agt gaa cag gtg ttt ggc gaa ttg cct gaa gaa ttg gcg aaa       480
   Ser Val Ser Glu Gln Val Phe Gly Glu Leu Pro Glu Glu Leu Ala Lys
   145                 150                 155                 160
   aaa cct aac ccg ttc gct gta tta gct aat tta aag aaa aac tag           525
   Lys Pro Asn Pro Phe Ala Val Leu Ala Asn Leu Lys Lys Asn
                   165                 170
<210> 159
   <211> 174
   <212> PRT
   <213> Actinobacillus pleuropneumoniae
<400> 159
   Met Gln Lys Val Lys Leu Pro Leu Thr Ile Asp Pro Tyr Lys Asp Ala
   1               5                   10                  15
   Gln Arg Arg Met Asp Tyr Glu Gly Tyr Ile Ser Arg Ser Leu Leu Asn
               20                  25                  30
   Arg Leu Gly Glu Ser Val Ser Asn Val Leu Ser Asp Ala Gln Val Thr
           35                  40                  45
   Leu Ser Leu Tyr Ile Asp Pro Gln Arg Leu Thr Val Ile Lys Gly Thr
       50                  55                  60
   Ala Thr Val Glu Val Glu Phe Asp Cys Gln Arg Cys Gly Asn Pro Phe
   65                  70                  75                  80
   Thr Gln Thr Leu Asp Cys Ser Phe Cys Phe Ser Pro Val Ser Asn Met
                   85                  90                  95
   Asp Gln Ala Asp Asn Leu Pro Glu Ile Tyr Glu Pro Ile Glu Val Asn
               100                 105                 110
   Glu Phe Gly Glu Val Asn Leu Leu Asp Met Ile Glu Asp Gly Phe Ile
           115                 120                 125
   Ile Glu Leu Pro Leu Val Pro Met His Ser Glu Glu His Cys Glu Val
       130                 135                 140
   Ser Val Ser Glu Gln Val Phe Gly Glu Leu Pro Glu Glu Leu Ala Lys
   145                 150                 155                 160
   Lys Pro Asn Pro Phe Ala Val Leu Ala Asn Leu Lys Lys Asn
                   165                 170
<210> 160
   <211> 1302
   <212> DNA
   <213> Actinobacillus pleuropneumoniae
<220>
   <221> misc_feature
   <223> tig
<220>
   <221> CDS
   <222> (1) .. (1299)
<400> 160
   atg tca att tct att gaa act tta gaa ggc tta caa cgc cgc gta act       48
   Met Ser Ile Ser Ile Glu Thr Leu Glu Gly Leu Gln Arg Arg Val Thr
   1               5                   10                  15
   att acc gta gct gct gat aaa atc gaa gcg gct tac aaa gag caa tta       96
   Ile Thr Val Ala Ala Asp Lys Ile Glu Ala Ala Tyr Lys Glu Gln Leu
               20                  25                  30
   aaa ggc tat gcg aaa aac gct cgt gta gac ggt ttc cgt aaa ggt aaa      144
   Lys Gly Tyr Ala Lys Asn Ala Arg Val Asp Gly Phe Arg Lys Gly Lys
           35                  40                  45
   gta ccg cac gca att atc gaa caa cgt ttc ggt tta gcg gct cgc caa      192
   Val Pro His Ala Ile Ile Glu Gln Arg Phe Gly Leu Ala Ala Arg Gln
       50                  55                  60
   gac gta tta tcc gat gaa atg caa cgt gcg ttc ttt gat gcg gta atc      240
   Asp Val Leu Ser Asp Glu Met Gln Arg Ala Phe Phe Asp Ala Val Ile
   65                  70                  75                  80
   gct gag aaa att aac ctt gcc ggt cgt cct acc ttc aca ccg aac aac      288
   Ala Glu Lys Ile Asn Leu Ala Gly Arg Pro Thr Phe Thr Pro Asn Asn
                   85                  90                  95
   tac caa ccg agt caa gaa ttc agc ttc act gca act ttt gaa gta ttc      336
   Tyr Gln Pro Ser Gln Glu Phe Ser Phe Thr Ala Thr Phe Glu Val Phe
               100                 105                 110
   ccg gaa gtt gaa tta aaa ggc tta gaa aat atc gaa gtt gaa aaa ccg      384
   Pro Glu Val Glu Leu Lys Gly Leu Glu Asn Ile Glu Val Glu Lys Pro
           115                 120                 125
   gtt gta gaa atc aca gaa gct gat tta gac aaa atg atc gat gtg tta      432
   Val Val Glu Ile Thr Glu Ala Asp Leu Asp Lys Met Ile Asp Val Leu
       130                 135                 140
   cgt aaa caa caa gcg act tgg gct gaa tct caa gca gcg gca caa gcg      480
   Arg Lys Gln Gln Ala Thr Trp Ala Glu Ser Gln Ala Ala Ala Gln Ala
   145                 150                 155                 160
   gaa gac cgt gtt gta atc gac ttc gta ggt tct gta gac ggt gaa gag      528
   Glu Asp Arg Val Val Ile Asp Phe Val Gly Ser Val Asp Gly Glu Glu
                   165                 170                 175
   ttt gaa ggc ggt aaa gcg aca gac ttc act tta gca atg ggt caa agt      576
   Phe Glu Gly Gly Lys Ala Thr Asp Phe Thr Leu Ala Met Gly Gln Ser
               180                 185                 190
   cgt atg atc cct ggt ttt gaa gaa ggt atc gtt ggt cac aaa gcc ggc      624
   Arg Met Ile Pro Gly Phe Glu Glu Gly Ile Val Gly His Lys Ala Gly
           195                 200                 205
   gaa caa ttc gat atc gat gtt act ttc cct gaa gaa tac cac gct gaa      672
   Glu Gln Phe Asp Ile Asp Val Thr Phe Pro Glu Glu Tyr His Ala Glu
       210                 215                 220
   aac tta aaa ggt aaa gcg gcg aaa ttc gca att aca ctt aag aaa gta      720
   Asn Leu Lys Gly Lys Ala Ala Lys Phe Ala Ile Thr Leu Lys Lys Val
   225                 230                 235                 240
   gaa aat atc gta tta cct gaa tta acc gaa gaa ttc gtg aaa aaa ttc       768
   Glu Asn Ile Val Leu Pro Glu Leu Thr Glu Glu Phe Val Lys Lys Phe
                   245                 250                 255
   ggt tca gca aaa act gta gaa gat tta cgt gcg gaa att aag aaa aat       816
   Gly Ser Ala Lys Thr Val Glu Asp Leu Arg Ala Glu Ile Lys Lys Asn
               260                 265                 270
   atg caa cgt gaa ctt aaa aac gca gta acc gca cgc gtt aaa aac caa       864
   Met Gln Arg Glu Leu Lys Asn Ala Val Thr Ala Arg Val Lys Asn Gln
           275                 280                 285
   gta atc aac ggt tta atc gca caa aat gaa att gaa gtg ccg gct gca       912
   Val Ile Asn Gly Leu Ile Ala Gln Asn Glu Ile Glu Val Pro Ala Ala
       290                 295                 300
   gcg gta gcg gaa gaa gtg gac gta tta cgt cgt caa gcg gtt caa cgt       960
   Ala Val Ala Glu Glu Val Asp Val Leu Arg Arg Gln Ala Val Gln Arg
   305                 310                 315                 320
   ttc ggt ggt aaa ccg gaa atg gct gca caa tta ccg gcg gaa tta ttc      1008
   Phe Gly Gly Lys Pro Glu Met Ala Ala Gln Leu Pro Ala Glu Leu Phe
                   325                 330                 335
   gaa gcg gat gca aaa cgt cgt gtt caa gta ggt tta tta ctt tca acc      1056
   Glu Ala Asp Ala Lys Arg Arg Val Gln Val Gly Leu Leu Leu Ser Thr
               340                 345                 350
   gta atc ggt act aac gaa tta aaa gtt gat gaa aaa cgt gtt gaa gaa      1104
   Val Ile Gly Thr Asn Glu Leu Lys Val Asp Glu Lys Arg Val Glu Glu
           355                 360                 365
   acg att gca gaa atc gct tca gct tac gaa caa ccg gcg gaa gtt gtt      1152
   Thr Ile Ala Glu Ile Ala Ser Ala Tyr Glu Gln Pro Ala Glu Val Val
       370                 375                 380
   gct cat tat gcg aaa aac cgt caa tta acc gaa aat atc cgt aac gta      1200
   Ala His Tyr Ala Lys Asn Arg Gln Leu Thr Glu Asn Ile Arg Asn Val
   385                 390                 395                 400
   gtg tta gaa gag caa gcg gtt gaa gtt gta ctt gcg aaa gca aaa gta      1248
   Val Leu Glu Glu Gln Ala Val Glu Val Val Leu Ala Lys Ala Lys Val
                   405                 410                 415
   act gaa aaa gcg act tct ttt gat gaa gta atg gct caa caa gct caa      1296
   Thr Glu Lys Ala Thr Ser Phe Asp Glu Val Met Ala Gln Gln Ala Gln
               420                 425                 430
   ggc taa                                                              1302
   Gly
<210> 161
   <211> 433
   <212> PRT
   <213> Actinobacillus pleuropneumoniae
<400> 161
   Met Ser Ile Ser Ile Glu Thr Leu Glu Gly Leu Gln Arg Arg Val Thr
   1 5 10 15
   Ile Thr Val Ala Ala Asp Lys Ile Glu Ala Ala Tyr Lys Glu Gln Leu
   20 25 30
   Lys Gly Tyr Ala Lys Asn Ala Arg Val Asp Gly Phe Arg Lys Gly Lys
   35 40 45
   Val Pro His Ala Ile Ile Glu Gln Arg Phe Gly Leu Ala
   Ala Arg Gln
   50 55 60
   Asp Val Leu Ser Asp Glu Met Gln Arg Ala Phe Phe Asp Ala Val Ile
   65 70 75 80
   Ala Glu Lys Ile Asn Leu Ala Gly Arg Pro Thr Phe Thr Pro Asn Asn
   85 90 95
   Tyr Gln Pro Ser Gln Glu Phe Ser Phe Thr Ala Thr Phe Glu Val Phe
   100 105 110
   Pro Glu Val Glu Leu Lys Gly Leu Glu Asn Ile Glu Val Glu Lys Pro
   115 120 125
   Val Val Glu Ile Thr Glu Ala Asp Leu Asp Lys Met Ile Asp Val Leu
   130 135 140
   Arg Lys Gln Gln Ala Thr Trp Ala Glu Ser Gln Ala Ala Ala Gln Ala
   145 150 155 160
   Glu Asp Arg Val Val Ile Asp Phe Val Gly Ser Val Asp Gly Glu Glu
   165 170 175
   Phe Glu Gly Gly Lys Ala Thr Asp Phe Thr Leu Ala Met Gly Gln Ser
   180 185 190
   Arg Met Ile Pro Gly Phe Glu Glu Gly Ile Val Gly His Lys Ala Gly
   195 200 205
   Glu Gln Phe Asp Ile Asp Val Thr Phe Pro Glu Glu Tyr His Ala Glu
   210 215 220
   Asn Leu Lys Gly Lys Ala Ala Lys Phe Ala Ile Thr Leu Lys Lys Val
   225 230 235 240
   Glu Asn Ile Val Leu Pro Glu Leu Thr Glu Glu Phe Val Lys Lys Phe
   245 250 255
   Gly Ser Ala Lys Thr Val Glu Asp Leu Arg Ala Glu Ile Lys Lys Asn
   260 265 270
   Met Gln Arg Glu Leu Lys Asn Ala Val Thr Ala Arg Val Lys Asn Gln
   275 280 285
   Val Ile Asn Gly Leu Ile Ala Gln Asn Glu Ile Glu Val Pro Ala Ala
   290 295 300
   Ala Val Ala Glu Glu Val Asp Val Leu Arg Arg Gln Ala Val Gln Arg
   305 310 315 320
   Phe Gly Gly Lys Pro Glu Met Ala Ala Gln Leu Pro Ala Glu Leu Phe
   325 330 335
   Glu Ala Asp Ala Lys Arg Arg Val Gln Val Gly Leu Leu Leu Ser Thr
   340 345 350
   Val Ile Gly Thr Asn Glu Leu Lys Val Asp Glu Lys Arg Val Glu Glu
   355 360 365
   Thr Ile Ala Glu Ile Ala Ser Ala Tyr Glu Gln Pro Ala Glu Val Val
   370 375 380
   Ala His Tyr Ala Lys Asn Arg Gln Leu Thr Glu Asn Ile Arg Asn Val
   385 390 395 400
   Val Leu Glu Glu Gln Ala Val Glu Val Val Leu Ala Lys Ala Lys Val
   405 410 415
   Thr Glu Lys Ala Thr Ser Phe Asp Glu Val Met Ala Gln Gln Ala Gln
   420 425 430
   Gly
<210> 162
   <211> 316
   <212> DNA
   <213> Actinobacillus pleuropneumoniae
<220>
   <221> misc_feature
   <223> tRNA-glu
<400> 162
   aatattgcgc tcaaatggca aagcggagag catctttaaa tgttgtcccc atcgtctaga 60
   ggcctaggac atcgcccttt cacggcggta accggggttc gaatccccgt ggggacgcca 120
   tttaaagatg acttttgttg tctgaattgt tctttaaaaa attggaaaca agctgaaaac 180
   tgagagattt tcgaaagaaa gtctgagtag taaaagataa gtaattatct tgaaaatctt 240
   agctgaacaa aagcagctaa gtgtttagtt gaataaagta tcgcgttgaa tgcgttcaaa 300
   taaaatttga aaatat 316
<210> 163
   <211> 85
   <212> DNA
   <213> Actinobacillus pleuropneumoniae
<220>
   <221> misc_feature
   <223> tRNA-leu
<400> 163
   gctctggtgg tggaattggt agacacgcta tcttgagggg gtagtgtcca taggatgtgc 60
   gagttcgagt ctcgcccaga gcacc 85
<210> 164
   <211> 623
   <212> DNA
   <213> Actinobacillus pleuropneumoniae
<220>
   <221> misc_feature
   <223> yaeE
<220>
   <221> CDS
   <222> (1)..(621)
<400> 164
   atg caa gaa ctc aca cct caa atg tgg ggc tta gtc ggc act tca acg      48
   Met Gln Glu Leu Thr Pro Gln Met Trp Gly Leu Val Gly Thr Ser Thr
   1               5                   10                  15
   ctt gaa acg ctc tat atg ggc ttt gcg gcg act tta ctt gct gtg gta      96
   Leu Glu Thr Leu Tyr Met Gly Phe Ala Ala Thr Leu Leu Ala Val Val
               20                  25                  30
   gtc ggt ttg ccg atc ggt ttt ctg gca ttt tta acc ggt aaa gga gag      144
   Val Gly Leu Pro Ile Gly Phe Leu Ala Phe Leu Thr Gly Lys Gly Glu
           35                  40                  45
   att tta gag aat ccg cgt tta cat caa gta tta gat gtg att att aat      192
   Ile Leu Glu Asn Pro Arg Leu His Gln Val Leu Asp Val Ile Ile Asn
       50                  55                  60
   atc ggt cgt tcc gta ccg ttt att att ttg tta gtc gtg ttg tta cct      240
   Ile Gly Arg Ser Val Pro Phe Ile Ile Leu Leu Val Val Leu Leu Pro
   65                  70                  75                  80
   ttt acg cgt tta ttg gtc ggg aca acg ctc ggt act acg gcg gcg att      288
   Phe Thr Arg Leu Leu Val Gly Thr Thr Leu Gly Thr Thr Ala Ala Ile
                   85                  90                  95
   gtg ccg tta agc gtt tcg gca att ccg ttt ttt gcg cgt tta act tca      336
   Val Pro Leu Ser Val Ser Ala Ile Pro Phe Phe Ala Arg Leu Thr Ser
               100                 105                 110
   aat gcg tta tta gaa atc cca gca ggt tta acc gaa gcg gcg aaa tcg      384
   Asn Ala Leu Leu Glu Ile Pro Ala Gly Leu Thr Glu Ala Ala Lys Ser
           115                 120                 125
   atg ggc gca acg aat tgg caa gtg gtc agt aaa ttt tat tta ccg gaa      432
   Met Gly Ala Thr Asn Trp Gln Val Val Ser Lys Phe Tyr Leu Pro Glu
       130                 135                 140
   tca ctg ccg att tta atc aat ggt atc aca tta act tta gtc gct tta      480
   Ser Leu Pro Ile Leu Ile Asn Gly Ile Thr Leu Thr Leu Val Ala Leu
   145                 150                 155                 160
   atc ggt tat tcg gca atg gcg ggt gcg gtc ggc ggc ggc ggt ttg ggt      528
   Ile Gly Tyr Ser Ala Met Ala Gly Ala Val Gly Gly Gly Gly Leu Gly
                   165                 170                 175
   aac ctt gcc atc agt tac ggt gaa cac cga aat atg gtc tat gta aaa      576
   Asn Leu Ala Ile Ser Tyr Gly Glu His Arg Asn Met Val Tyr Val Lys
               180                 185                 190
   tgg atc tca aca att att atc gta gcg att gtg atg atc agt caa aa       623
   Trp Ile Ser Thr Ile Ile Ile Val Ala Ile Val Met Ile Ser Gln
           195                 200                 205
<210> 165
   <211> 207
   <212> PRT
   <213> Actinobacillus pleuropneumoniae
<400> 165
   Met Gln Glu Leu Thr Pro Gln Met Trp Gly Leu Val Gly Thr Ser Thr
   1               5                   10                  15
   Leu Glu Thr Leu Tyr Met Gly Phe Ala Ala Thr Leu Leu Ala Val Val
               20                  25                  30
   Val Gly Leu Pro Ile Gly Phe Leu Ala Phe Leu Thr Gly Lys Gly Glu
           35                  40                  45
   Ile Leu Glu Asn Pro Arg Leu His Gln Val Leu Asp Val Ile Ile Asn
       50                  55                  60
   Ile Gly Arg Ser Val Pro Phe Ile Ile Leu Leu Val Val Leu Leu Pro
   65                  70                  75                  80
   Phe Thr Arg Leu Leu Val Gly Thr Thr Leu Gly Thr Thr Ala Ala Ile
                   85                  90                  95
   Val Pro Leu Ser Val Ser Ala Ile Pro Phe Phe Ala Arg Leu Thr Ser
               100                 105                 110
   Asn Ala Leu Leu Glu Ile Pro Ala Gly Leu Thr Glu Ala Ala Lys Ser
           115                 120                 125
   Met Gly Ala Thr Asn Trp Gln Val Val Ser Lys Phe Tyr Leu Pro Glu
       130                 135                 140
   Ser Leu Pro Ile Leu Ile Asn Gly Ile Thr Leu Thr Leu Val Ala Leu
   145                 150                 155                 160
   Ile Gly Tyr Ser Ala Met Ala Gly Ala Val Gly Gly Gly Gly Leu Gly
                   165                 170                 175
   Asn Leu Ala Ile Ser Tyr Gly Glu His Arg Asn Met Val Tyr Val Lys
               180                 185                 190
   Trp Ile Ser Thr Ile Ile Ile Val Ala Ile Val Met Ile Ser Gln
           195                 200                 205
<210> 166
   <211> 24
   <212> DNA
   <213> Artificial
<220>
   <223> Primer
<400> 166
   gacgtttccc gttgaatatg gctc 24
<210> 167
   <211> 26
   <212> DNA
   <213> Artificial
<220>
   <223> Primer
<400> 167
   gccggatccg ggatcatatg acaaga 26
<210> 168
   <211> 24
   <212> DNA
   <213> Artificial
<220>
   <223> Primer
<400> 168
   gacaagatgt gtatccacct taac 24